(19) Europäisches Patentamt
European Patent Office
Office européen des brevets

(11) EP 1 426 439 A1

(12) # EUROPEAN PATENT APPLICATION
published in accordance with Art. 158(3) EPC

(43) Date of publication:
**09.06.2004 Bulletin 2004/24**

(21) Application number: **02760723.3**

(22) Date of filing: **23.08.2002**

(51) Int Cl.[7]: **C12N 15/00**, C12Q 1/68

(86) International application number:
**PCT/JP2002/008495**

(87) International publication number:
**WO 2003/018792 (06.03.2003 Gazette 2003/10)**

(84) Designated Contracting States:
**AT BE BG CH CY CZ DE DK EE ES FI FR GB GR IE IT LI LU MC NL PT SE SK TR**

(30) Priority: **24.08.2001 JP 2001255379**

(71) Applicants:
- **National Institute of Advanced Industrial Science and Technology**
  **Tokyo 100-8921 (JP)**
- **Daikin Industries, Ltd.**
  **Osaka-shi, Osaka 530-8323 (JP)**

(72) Inventors:
- **IWAHASHI, Hitoshi**
  **Tsukuba-shi, Ibaraki 305-8561 (JP)**
- **MOMOSE, Yuko**
  **Tsukuba-shi, Ibaraki 305-8561 (JP)**
- **KITAGAWA, Emiko**
  **Tsukuba-shi, Ibaraki 305-8561 (JP)**
- **TAKAHASHI, Junko**
  **Tsukuba-shi, Ibaraki 305-0841 (JP)**

(74) Representative: **HOFFMANN - EITLE**
**Patent- und Rechtsanwälte**
**Arabellastrasse 4**
**81925 München (DE)**

(54) **METHOD OF DETECTING TOXIC SUBSTANCE**

(57) Biology-based processes for detecting toxic substances are provided. The processes comprise transforming cells with a vector comprising a polynucleotide which comprises a promoter of certain yeast gene operably linked to a polynucleotide encoding a marker protein; contacting the transformed cells to test sample; and detecting the expression of mRNA encoding the marker protein, thus detecting a toxic compound in the test sample.

EP 1 426 439 A1

## Description

FILED OF THE INVENTION

This invention relates to biology-based processes for detecting toxic substances. It also relates to polynucleotides, vectors, and cells as used for the processes.

BACKGROUND ART

Environmental chemical fate search has been conducted every year for 24 years from 1974 through 1998 by Environment Agency, and revealed that about 40% of 775 chemical substances that have been searched so far are emitted into the environment. Chemical substances that are industrially produced at the present in Japan are estimated about 50,000, and the production scale and the kinds of chemical substances are increasing year by year. It is known that chemical substances that are accidentally produced by water treatment with chlorine and incineration pollute the environment. Although such facts allow us to predict that there are a large number of chemical substances that have been accumulated in the environment, it is extremely difficult to search and examine individually the all chemical substances.

Conventional bioassays (approaches to evaluate the harmful effects on biological materials on the basis of their responses) wherein inhibited growth and particular biological responses in individuals and cells of fishes, daphnia and shellfish are used as indicators make it possible to determine the presence or absence of the toxicity of chemical substances in the environment, but neither possible to evaluate the characters nor origins of the toxicity. The evaluation methods based on the activity of nitrite-forming bacteria or nitrate-forming bacteria (Japanese Patent Publication (kokai) No. 123705/ 1994, Japanese Patent Publication (kokai) No. 2000-206087) and the activity of iron bacteria have been proposed, and devices such as Acute toxiciants monitor (Fuji Electric Corporate Research and Development, Ltd. Japan) are marketed. In foreign countries, the devices for evaluation based on emission intensity of luminous bacteria are commercially available (MICROTOX, azur, Co., USA; LUMIS, drlange, Co. Germany). However, those devices still involve conventional bioassays, and never provide any detailed information of toxic chemical substances.

In Japan, the risk control of chemical substances is reconsidered every time a chemical substance pollution is newly found, and official regulations and self-imposed regulations are combined to organize the system for risk control. However, any system has not been yet organized that could quickly respond to the present complicated and diversified conditions including accidental productions and environmental emission of toxic chemical substance as typified by trihalomethane and dioxin. Animal experiments as used in the method for evaluation of toxic substance of "Law Concerning Examination and Manufacture, etc. of Chemical Substances" are expensive and time-consuming, and are not accepted across the world. Although, as such, the control system has been continuously discussed, it has not been successfully accomplished because there is no way to dissolve the problem. Thus, a method for detect readily chemical substances occurring in the environment is desired.

DISCLOSURE OF THE INVENTION

The inventors of the present application found that a toxic substance activates the promoter of a particular yeast gene to induce the transcription of mRNA from the polynucleotide encoding a marker protein operably linked to the promoter, and accomplished the present invention.

Specifically, the invention of the present application relates, as the first embodiment, to a polynucleotide which comprises a polynucleotide sequence operably linked to a polynucleotide encoding a marker protein, wherein the polynucleotide sequence comprises a promoter from yeast genes that is selected from a group consisting of the following, or a promoter from a gene that is homologous to the yeast genes and is derived from other species; YBR072W, YCR102C, YCR107W, YDL218W, YDL243C, YDR453C, YDR533C, YFL014W, YFL056C, YFL057C, YGR110W, YJR155W, YKL071W, YKR076W, YLL060C, YLR460C, YMR090W, YNL331C, YNL332W, YNL335W, YOL150C, YOL165C, YPL171C, YPR167C, YBL048W, YBL064C, YBL107C, YBR008C, YBR173C, YBR256C, YBR296C, YDL021W, YFL022C, YFL024C, YFL061W, YGL121C, YGL158W, YGR043C, YHR029C, YHR112C, YHR139C, YHR179W, YHR209W, YIR030C, YJR010W, YJR048W, YKL001C, YKL107W, YKR075C, YKR097W, YLL056C, YLR297W, YLR303W, YML087C, YMR096W, YNL274C, YOL151W, YOR226C, YOR338W, YOR391C, YPL280W, YDR406W, YJL153C, YLR346C, YOR049C, YOR153W, YPL088W, YAL034C, YDL124W, YDL174C, YDR476C, YGL156W, YGR035C, YGR157W, YGR213C, YGR281W, YGR284C, YHL047C, YHR043C, YHR044C, YHR054C, YJR073C, YKL165C, YLR008C, YMR315W, YNL211C, YOL031C, YOL101C, YOR303W, YAL005C, YAR031W, YBL005W-A, YBL022C, YBL041W, YBL049W, YBL075C, YBL078C, YBR062C, YBR169C, YBR294W, YCL020W, YCL035C, YCL043C, YCL050C, YCL057W, YCR012W, YCR013C, YCR060W, YDL007W, YDL027C, YDL097C, YDL110C, YDL126C, YDL169C, YDR070C, YDR155C, YDR158W, YDR204W, YDR210W, YDR214W, YDR258C, YDR313C, YDR368W, YDR435C, YER012W, YER037W, YER091C, YER103W, YFL044C, YFR003C, YFR010W, YFR020W, YFR024C, YFR044C, YFR053C, YGL006W, YGL048C, YGL062W, YGL141W, YGL157W, YGL163C, YGL180W, YGL184C, YGR010W, YGR028W, YGR032W, YGR048W, YGR124W, YGR135W, YGR142W, YGR161C, YGR192C, YGR197C, YGR201C, YGR212W, YGR231C, YGR244C, YGR254W, YGR268C, YHL030W, YHR016C, YHR018C, YHR055C, YHR087W, YHR166C, YIL160C, YIR017C, YJL034W, YJL048C, YJL052W, YJL144W, YJL163C, YJR009C, YJR069C, YJR074W, YJR130C, YJR149W, YKL065C, YKL073W, YKL103C, YKL142W, YKL210W, YKL218C, YKR011C, YKR018C, YKR046C, YKR049C, YLL024C, YLL026W, YLR027C, YLR080W, YLR107W, YLR121C, YLR132C, YLR133W, YLR155C, YLR158C, YLR161W, YLR195C, YLR217W, YLR328W, YLR336C, YLR345W, YLR370C, YLR423C, YML004C, YML092C, YML128C, YML130C, YML131W, YMR040W, YMR118C, YMR214W, YMR251W, YMR297W, YMR322C, YNL036W, YNL055C, YNL071W, YNL094W, YNL134C, YNL155W, YNL160W, YNL239W, YNL241C, YOL005C, YOR020C, YOR027W, YOR037W, YOR059C, YOR120W, YOR134W, YOR152C, YOR173W, YOR289W, YOR362C, YPL240C, YPR030W, YAL008W, YAL023C, YAL060W, YAL062W, YAR009C, YBL101C, YBR006W, YBR046C, YBR052C, YBR053C, YBR056W, YBR099C, YBR137W, YBR139W, YBR149W, YBR170C, YBR177C, YBR203W, YBR207W, YBR212W, YBR239C, YBR284W, YBR293W, YCL018W, YCL033C, YCL040W, YCL049C, YCR062W, YCR067C, YCR082W, YDL010W, YDL020C, YDL024C, YDL054C, YDL095W, YDL100C, YDL115C, YDL144C, YDL198C, YDL223C, YDL245C, YDL246C, YDR001C, YDR032C, YDR058C, YDR072C, YDR127W, YDR168W, YDR169C, YDR188W, YDR231C, YDR261C, YDR264C, YDR272W, YDR293C, YDR304C, YDR330W, YDR403W, YDR411C, YDR427W, YDR436W, YDR497C, YDR511W, YDR516C, YDR519W, YDR545W, YEL012W, YEL030W, YER004W, YER009W, YER021W, YER035W, YER053C, YER079W, YER094C, YER096W, YER125W, YER158C, YER163C, YER175C, YER177W, YER178W, YER185W, YFL006W, YFL010C, YFL016C, YFL029C, YFL030W, YFL031W, YFL032W, YFL038C, YFL041W, YFR004W, YFR047C, YFR050C, YFR052W, YGL011C, YGL013C, YGL037C, YGL047W, YGL053W, YGL091C, YGL094C, YGL127C, YGL150C, YGL199C, YGL207W, YGL248W, YGR008C, YGR037C, YGR055W, YGR101W, YGR130C, YGR154C, YGR194C, YGR221C, YGR232W, YGR248W, YGR253C, YGR256W, YHL008C, YHR027C, YHR053C, YHR057C, YHR111W, YHR138C, YHR161C, YHR164C, YHR169W, YHR174W, YHR176W, YHR199C, YIL010W, YIL034C, YIL041W, YIL045W, YIL087C, YIL107C, YIL142W, YIL155C, YIR034C, YIR036C, YIR037W, YIR038C, YIR039C, YJL001W, YJL031C, YJL035C, YJL053W, YJL057C, YJL066C, YJL068C, YJL082W, YJL099W, YJL102W, YJL151C, YJL152W, YJL161W, YJL164C, YJL171C, YJL172W, YJL210W, YJL219W, YJR008W, YJR045C, YJR046W, YJR106W, YJR117W, YJR137C, YKL007W, YKL026C, YKL035W, YKL091C, YKL104C, YKL117W, YKL145W, YKL146W, YKL151C, YKL152C, YKL153W, YKL193C, YKL195W, YKL213C, YKL215C, YLL028W, YLL039C, YLL058W, YLR054C, YLR103C, YLR120C, YLR136C, YLR149C, YLR152C, YLR178C, YLR259C, YLR299W, YLR324W, YLR327C, YLR348C, YLR350W, YLR356W, YLR362W, YLR387C, YLR429W, YML054C, YML070W, YML100W, YML117W, YML125C, YMR004W, YMR008C, YMR009W, YMR020W, YMR067C, YMR089C, YMR097C, YMR102C, YMR105C, YMR107W, YMR152W, YMR180C, YMR184W, YMR191W, YMR219W, YMR271C, YMR275C, YMR295C, YMR314W, YMR316W, YNL006W, YNL007C, YNL012W, YNL044W, YNL045W, YNL074C, YNL092W, YNL093W, YNL104C, YNL115C, YNL156C, YNL231C, YNL234W, YNL237W, YNL281W, YNL305C, YNL333W, YNR010W, YNR019W, YNR033W, YNR059W, YNR068C, YNR069C, YOL032W, YOL036W, YOL047C, YOL071W, YOL082W, YOL083W, YOL117W, YOL119C, YOL126C, YOL131W, YOL153C, YOL162W, YOL163W, YOL164W, YOR019W, YOR035C, YOR036W, YOR099W, YOR117W, YOR124C, YOR130C, YOR132W, YOR157C, YOR185C, YOR197W, YOR259C, YOR261C, YOR273C, YOR288C, YOR332W, YOR336W, YOR347C, YPL017C, YPL087W, YPL106C, YPL109C, YPL149W, YPL154C, YPL196W, YPL206C, YPL222W, YPR023C, YPR024W, YPR026W, YPR067W, YPR103W, YPR108W, YPR151C, YAL012W, YBR029C, YBR222C, YCL009C, YCL027W, YCL064C, YCR098C, YDL222C, YDR055W, YDR077W, YDR502C, YEL001C, YEL042W, YER026C, YER106W, YGR136W, YGR138C, YHR137W, YHR142W, YIL023C, YIL153W, YJL073W, YJR004C, YJR054W, YKL039W, YKL086W, YKL163W, YKR091W, YLR109W, YLR194C, YLR250W, YMR095C, YMR189W, YNL106C, YNL169C, YNL322C, YOR181W, YOR198C, YOR208W, YOR247W, YPL089C, YAL038W, YAL053W, YBR023C, YBR214W, YBR295W, YCR048W, YDL072C, YDL204W, YDR085C, YDR098C, YDR259C, YDR380W, YDR388W, YDR391C, YDR432W, YDR481C, YDR510W, YER069W, YGL022W, YGL126W, YGL209W, YGL255W, YGR189C, YGR282C, YHL035C, YHR030C, YIL022W, YIL024C, YIL117C, YIL123W, YIL140W, YIL154C, YJL088W, YJL108C, YJL149W, YJL159W, YJL186W, YJR148W, YKL096W, YLR180W, YLR273C, YLR300W, YLR307W, YLR378C, YLR391W, YMR094W, YMR104C, YMR276W, YMR296C, YNL190W, YNL208W, YNL300W, YNR064C, YOL013C, YOL058W, YOR248W, YOR355W, YPL052W, YPL163C, YPR079W, YAR028W, YBR146W, YBR183W, YCL038C, YCR071C, YDL008W, YDR019C, YDR031W, YDR115W, YDR486C, YER038C, YER130C, YFL054C, YGL136C, YGR146C, YGR207C, YHL040C, YIL167W, YJL020C, YKR039W, YLR031W, YLR205C, YMR072W, YMR140W, YMR173W, YMR195W, YMR226C, YNL037C, YNR002C, YOL143C, YOR136W, YOR215C, YOR382W, YOR383C, YPL054W, YPL271W, YPR127W, YAL044C, YAL054C, YAR010C, YAR027W, YAR071W, YBL001C, YBL043W, YBL057C, YBR014C, YBR024W, YBR035C, YBR068C, YBR111C, YBR116C, YBR147W, YBR168W, YBR246W, YBR273C, YCR004C, YCR021C, YCR037C, YCR088W, YDL022W, YDL128W, YDL238C, YDR003W, YDR009W, YDR033W, YDR084C, YDR104C, YDR270W, YDR315C, YDR340W, YDR357C, YDR358W, YDR396W, YDR405W, YDR410C, YDR434W, YDR482C, YDR487C, YDR520C, YDR534C, YDR539W, YEL011W, YEL065W, YEL066W, YER039C, YER044C, YER067W, YER080W, YER107C, YFL020C, YFL028C, YFL043C, YFR015C, YGL001C, YGL008C, YGL068W, YGL073W, YGL104C, YGL113W, YGL154C, YGL167C, YGL229C, YGL242C, YGL249W, YGL253W, YGR052W, YGR060W, YGR065C, YGR106C, YGR111W, YGR220C, YGR257C, YHL023C, YHL048W, YHR004C, YHR037W, YHR071W, YHR092C, YHR190W, YIL007C, YIL033C, YIL070C, YIL088C, YIL111W, YIR002C, YIR016W, YIR035C, YIR043C, YJL012C, YJL083W, YJL089W, YJL116C, YJL131C, YJL132W, YJL196C, YJR061W, YJR086W, YJR142W, YJR161C, YKL008C, YKL013C, YKL041W, YKL067W, YKL138C, YKL139W, YKL150W, YKL175W, YKR006C, YKR014C, YKR070W, YLL023C, YLR023C, YLR093C, YLR118C, YLR142W, YLR225C, YLR241W, YLR251W, YLR252W, YLR270W, YML030W, YML110C, YMR021C, YMR027W, YMR148W, YMR181C, YMR262W, YMR272C, YMR298W, YNL011C, YNL130C, YNL214W, YNL259C, YOL129W, YOR042W, YOR052C, YOR137C, YOR149C, YOR165W, YOR270C, YOR285W, YOR367W, YPL018W, YPL156C, YPL186C, YPL203W, YPL216W, YPL255W, YPR006C, YPR073C, YPR098C, YBR050C, YBR145W, YBR299W, YDR518W, YEL020C, YFL062W, YGL039W, YGL134W, YJL217W, YJR159W, YLR126C, YNL249C, YNL284C, YNL336W, YOL157C, YOR344C, YOR381W, YPL265W, YPR124W, YBR074W, YBR109C, YBR126C, YBR201W, YCR005C, YDL248W, YDR041W, YDR105C, YDR268W, YDR452W, YEL075C, YER046W, YER050C, YER136W, YER159C, YGL250W, YGR019W, YGR042W, YGR053C, YGR066C, YGR247W, YGR255C, YGR295C, YHL044W, YHR145C, YIL058W, YIL065C, YIL083C, YIL098C, YIL172C, YJL030W, YJL185C, YJL213W, YJR029W, YJR099W, YJR122W, YJR125C, YKL190W, YKR020W, YLL025W, YLL051C, YLR043C, YLR090W, YLR100W, YLR108C, YLR290C, YML068W, YMR051C, YMR139W, YMR178W, YMR193W, YNL015W, YNL079C, YNL122C, YNL223W, YNL285W, YNL293W, YNR007C, YNR035C, YNR061C, YOL016C, YOL104C, YOR220W, YOR221C, YOR374W, YPL123C, YPR077C, YPR107C, YPR147C, YBR093C, YBR196C, YEL041W, YEL047C, YER023W, YER119C, YFL055W, YGR209C, YIL124W, YKL187C, YLL055W, YMR318C, YOL152W, YAL007C, YBR067C, YBR115C, YBR285W, YBR292C, YDL043C, YDL123W, YDL131W, YDL168W, YDL212W, YDR056C, YDR132C, YDR154C, YDR183W, YDR216W, YDR253C, YDR295C, YDR494W, YDR513W, YEL072W, YER045C, YER061C, YER181C, YFL052W, YFL058W, YFR030W, YGL089C, YGL096W, YGL114W, YGL193C, YGL202W, YGL204C, YGL259W, YGR006W, YGR070W, YGR088W, YHL034C, YHL036W, YHR048W, YHR104W, YHR163W, YIL060W, YIL136W, YIR024C, YJL036W, YJL045W, YJL060W, YJL101C, YJL155C, YJR085C, YJR109C, YJR156C, YKL070W, YKL161C, YKL221W, YKR071C, YLL009C, YLL050C, YLR092W, YLR145W, YLR156W, YLR163C, YLR220W, YLR280C, YLR311C, YLR390W, YML116W, YMR034C, YMR038C, YMR081C, YMR250W, YNL240C, YNL260C, YNL277W, YNR074C, YOL044W, YOL084W, YOL147C, YOL159C, YOR184W, YOR228C, YOR255W, YPL223C, YPR160W, YDL182W, YBR047W, YBR054W, YBR291C, YDR069C, YER124C, YER131W, YGR044C, YIL094C, YKR007W, YMR240C, YNR050C, YOR007C, YAL015C, YBL065W, YBR105C, YBR182C, YBR186W, YBR244W, YBR272C, YCL069W, YDL025C, YDL059C, YDL085W, YDL113C, YDL244W, YDR018C, YDR054C, YDR202C, YDR223W, YDR350C, YDR353W, YDR374C, YDR512C, YEL052W, YEL070W, YER098W, YFR017C, YGL046W, YGL067W, YGL098W, YGL117W, YGL146C, YGL240W, YGR011W, YGR067C, YGR133W, YGR153W, YGR223C, YHR116W, YHR124W, YIL097W, YIL168W, YJL103C, YJL221C, YJR036C, YJR095W, YKL085W, YKL133C, YKL162C, YKL188C, YKL217W, YKR061W, YKR105C, YLL062C, YLR174W, YLR216C, YLR247C, YLR260W, YLR267W, YLR389C, YML007W, YMR041C, YMR177W, YMR253C, YNL009W, YNL117W, YNL128W, YNL183C, YNR073C, YOL133W, YOL158C, YOR133W, YOR225W, YOR227W, YPL161C, YPL166W, YPL202C, YPL224C, YPR015C, YPR086W, YPR201W, YAL061W, YAL067C, YAR007C, YBL033C, YBL056W, YBL086C, YBR026C, YBR073W, YBR101C, YBR117C, YBR123C, YBR213W, YBR269C, YBR280C, YCR036W, YDL132W, YDL149W, YDL200C, YDL234C, YDL242W, YDR099W, YDR177W, YDR256C, YDR392W, YDR394W, YDR531W, YEL071W, YER014W, YER042W, YER090W, YER184C, YFL059W, YFR042W, YFR046C, YFR049W, YGL026C, YGL058W, YGL185C, YGL227W, YGL252C, YGL254W, YGR089W, YGR112W, YGR134W, YGR276C, YHL019C, YHR012W, YHR017W, YHR028C, YHR106W, YHR109W, YHR156C, YIL036W, YIL046W, YIL143C, YIL152W, YIL159W, YIL164C, YJL071W, YJL094C, YJL154C, YJR056C, YJR072C, YJR110W, YJR139C, YKL025C, YKL034W, YKL064W, YKL171W, YKL196C, YKR012C, YKR068C, YKR069W, YLL001W, YLL057C, YLL061W, YLR064W, YLR070C, YLR099C, YLR144C, YLR157C, YLR160C, YLR164W, YLR364W, YLR421C, YML032C, YML042W, YML112W, YML118W, YMR114C, YMR115W, YMR258C, YNL181W, YNL191W, YNL212W, YNL213C, YNL250W, YNL265C, YNL312W, YNR032W, YOL038W, YOL049W, YOL064C, YOR088W, YOR155C, YOR257W, YOR265W, YOR377W, YOR386W, YPL031C, YPL113C, YPL124W, YPL151C, YPL249C, YPL260W, YPL274W, YPR048W, YPR061C, YPR093C, YPR125W, YPR158W, YPR168W, YPR169W, YPR174C, YPR180W, YPR193C, YPR200C, YAL014C, YAL017W, YAL049C, YBL019W, YBL058W, YBR001C, YBR013C, YBR018C, YBR037C, YBR045C, YBR051W, YBR063C, YBR128C, YBR129C, YBR204C, YBR241C, YBR255W, YBR281C, YCL044C, YCL055W, YCR014C, YCR019W, YCR024C, YCR105W, YDL065C, YDL089W, YDL143W, YDL173W, YDL193W, YDL197C, YDL206W, YDL230W, YDL233W, YDR040C, YDR071C, YDR078C, YDR109C, YDR140W, YDR194C, YDR212W, YDR221W, YDR257C, YDR271C, YDR287W, YDR294C, YDR316W, YDR329C, YDR338C, YDR369C, YDR421W, YDR425W, YDR485C, YDR488C, YDR504C, YDR505C, YDR506C, YDR515W, YEL005C, YEL037C, YEL044W, YER017C, YER048C, YER052C, YER078C, YER089C, YER092W, YER100W, YER162C, YER182W, YFL021W, YFL042C, YFR045W, YFR051C, YFR056C, YGL040C, YGL041C, YGL045W, YGL057C, YGL093W, YGL105W, YGL125W, YGL166W, YGL181W, YGL183C, YGL215W, YGL216W, YGL221C, YGL223C, YGR007W, YGR029W, YGR155W, YGR156W, YGR186W, YGR198W, YGR210C, YGR211W, YGR237C, YGR250C, YGR258C, YGR266W, YGR270W, YGR274C, YGR277C, YHL021C, YHL037C, YHL038C, YHR082C, YHR083W, YHR134W, YHR160C, YHR171W, YHR180W, YHR205W, YIL062C, YIL072W, YIL075C, YIL099W, YIL108W, YIL165C, YIL170W, YIR009W, YIR018W, YIR031C, YIR032C, YJL032W, YJL049W, YJL128C, YJL165C, YJR044C, YJR052W, YJR090C, YJR091C, YJR103W, YJR104C, YJR153W, YKL059C, YKL079W, YKL090W, YKL094W, YKL192C, YKL209C, YKR052C, YKR102W, YKR106W, YLL054C, YLR025W, YLR097C, YLR200W, YLR226W, YLR248W, YLR266C, YLR392C, YLR427W, YML013W, YML029W, YML041C, YML051W, YML078W, YML079W, YML088W, YML099C, YMR056C, YMR068W, YMR091C, YMR110C, YMR160W, YMR186W, YMR255W, YNL005C, YNL026W, YNL039W, YNL063W, YNL064C, YNL077W, YNL083W, YNL147W, YNL176C, YNL194C, YNL253W, YNL257C, YNL261W, YNL264C, YNL276C, YNR006W, YNR034W, YNR047W, YNR051C, YNR071C, YOL065C, YOL067C, YOR005C, YOR008C, YOR022C, YOR023C, YOR058C, YOR069W, YOR087W, YOR138C, YOR229W, YOR256C, YOR267C, YPL005W, YPL020C, YPL022W, YPL105C, YPL147W, YPL150W, YPL152W, YPL164C, YPL168W, YPL180W, YPL188W, YPL194W, YPR025C, YPR047W, YPR049C, YPR066W, YPR081C, YPR134W, YPR140W, YPR148C, YPR155C, YPR172W, YPR185W, YAL018C, YAR064W, YBR012C, YBR076W, YBR287W, YDR043C, YDR250C, YDR373W, YFR014C, YGL191W, YGR180C, YHR136C, YJL026W, YJL037W, YLR038C, YNL058C, YOR031W, YGR087C, YIL166C, YHR008C, YIL129C, YGL256W, YJR030C, YMR077C, YBR264C, YPL177C, YKR040C, YGL056C, YDR128W, YGR139W, YBL101W-A, YOR253W, YOL026C, YDR278C, YHR095W, YCL042W, YNL200C, YPL221W, YLR415C, YMR058W, YPR037C, YER072W, YML028W, YOR325W, YAL039C, YMR112C, YJR107W, YGL088W, YJR058C, YNL142W, YDR090C, YMR071C, YBL093C, YGR293C, YML055W, YDL017W, YDL210W, YGL055W, YCL025C, YDR080W, YDL181W, YNR030W, YJL017W, YIL127C, YDR281C, YDR366C, YFR026C, YJL212C, YPL215W, YEL019C, YBR132C, YHL018W, YNL196C, YPL038W, YAR047C, YPL262W, YHL006C, YPL225W, YBR124W, YOR148C, YKR053C, YBL044W, YER029C, YLR360W, YCL056C, YCR007C, YGR239C, YNL256W, YPR146C, YLR377C, YKL097C, YBR066C, YLR338W, YDL229W, YBR253W, YJR027W, YKL198C, YBL030C, YBR031W, YBR118W, YBR162C, YBR221C, YCR024C-A, YCR106W, YDL046W, YDR012W, YDR133C, YDR134C, YDR276C, YDR342C, YDR343C, YEL027W, YEL034W, YGR038W, YGR243W, YGR279C, YHR094C, YHR105W, YHR175W, YHR181W, YIL056W, YIL162W, YJL059W, YJL097W, YJL158C, YJR105W, YKL051W, YKL056C, YKL097W-A, YKL100C, YKL141W, YKR066C, YLR134W, YLR258W, YLR339C, YML058W, YMR083W, YMR203W, YNL209W, YNL307C, YOL030W, YOR178C, YPL028W, YPR028W, YPR113W, YPR149W, YPR150W, YPR183W, YAL016W, YBL099W, YBL100C, YBR011C, YBR096W, YBR100W, YBR127C, YBR283C, YBR286W, YCL008C, YCL058C, YCR030C, YCR034W, YCR069W, YDL015C, YDL023C, YDL061C, YDL086W, YDR038C, YDR039C, YDR050C, YDR151C, YDR178W, YDR233C, YDR284C, YDR298C, YDR345C, YDR359C, YDR382W, YDR385W, YDR400W, YDR407C, YDR538W, YEL024W, YEL033W, YEL063C, YER057C, YER081W, YER120W, YFL011W, YGL012W, YGL206C, YGR022C, YGR026W, YGR082W, YGR107W, YGR172C, YGR191W, YGR204W, YGR260W, YHL005C, YHL046C, YHR025W, YHR026W, YHR123W, YHR126C, YHR143W, YIL011W, YIL015W, YIL018W, YIL157C, YIR041W, YJL016W, YJL121C, YJL133W, YJL138C, YJL191W, YJR018W, YJR047C, YJR077C, YJR119C, YJR121W, YJR123W, YJR143C, YJR145C, YKL060C, YKL147C, YKL148C, YKL157W, YKL164C, YKL169C, YKR033C, YLL041C, YLL064C, YLR041W, YLR044C, YLR056W, YLR058C, YLR081W, YLR089C, YLR110C, YLR177W, YLR264W, YLR284C, YLR304C, YLR340W, YLR354C, YLR372W, YLR388W, YML022W, YMR007W, YMI2011W, YMR015C, YMR092C, YMR101C, YMR156C, YMR205C, YMR215W, YMR261C, YMR323W, YNL069C, YNL135C, YNL195C, YNR076W, YOL039W, YOL073C, YOL086C, YOL120C, YOL156W, YOL161C, YOR002W, YOR009W, YOR010C, YOR085W, YOR108W, YOR128C, YOR129C, YOR142W, YOR161C, YOR176W, YOR230W, YOR298W, YPL004C, YPL036W, YPL048W, YPL057C, YPL059W, YPL061W, YPL135W, YPL179W, YPL218W, YPL220W, YPL246C, YPL272C, YPR063C, YPR080W, YPR181C, YBR290W, YCR010C, YCR091W, YDL107W, YDL129W, YDR066C, YDR529C, YFL026W, YGL018C, YGL059W, YNL144C, YOR003W, YAL037W, YAR023C, YBR003W, YBR020W, YBR044C, YBR091C, YBR185C, YBR282W, YCR015C, YCR038C, YCR043C, YDL119C, YDL146W, YDL220C, YDR057W, YDR123C, YDR125C, YDR222W, YDR225W, YDR277C, YDR286C, YDR347W, YDR408C, YDR438W, YDR479C, YDR483W, YEL039C, YEL057C, YEL073C, YER066W, YER076C, YER084W, YER121W, YER189W, YFL017C, YFL046W, YFR006W, YFR008W, YGL115W, YGL208W, YGL214W, YGL218W, YGR021W, YGR023W, YGR024C, YGR064W, YGR076C, YGR096W, YGR108W, YGR174C, YGR182C, YGR236C, YGR288W, YHL042W, YHR195W, YHR210C, YIL006W, YIL012W, YIL028W, YIL050W, YIL057C, YIL089W, YIL102C, YIL113W, YIL122W, YJL100W, YJL169W, YJL199C, YJR039W, YJR050W, YJR101W, YKL003C, YKL016C, YKL061W, YKL093W, YKL121W, YKL160W, YKL170W, YKL194C, YKR034W, YKR067W, YLR006C, YLR016C, YLR030W, YLR036C, YLR112W, YLR125W, YLR128W, YLR204W, YLR211C, YLR233C, YLR257W, YLR288C, YLR326W, YLR334C, YLR395C, YLR408C, YLR414C, YLR444C, YML050W, YML107C, YML120C, YMR031C, YMR053C, YMR073C, YMR162C, YMR204C, YMR206W, YMR284W, YNL010W, YNL025C, YNL127W, YNL139C, YNL217W, YOL116W, YOL118C, YOR053W, YOR100C, YOR103C, YOR122C, YOR150W, YOR187W, YOR251C, YOR312C, YOR327C, YOR348C, YOR352W, YOR388C, YOR394W, YPL001W, YPL033C, YPL066W, YPL148C, YPL230W, YPL275W, YPL276W, YPR005C, YPR014C, YPR192W, YPR194C, YBR005W, YER025W, YFL027C, YGL080W, YGL205W, YHL028W, YHR185C, YIL076W, YJL166W, YLR046C, YMR035W, YMR238W, YMR252C, YNL192W, YNL202W, YOL108C, YOR385W, YPR165W, YAR033W, YBL038W, YBR009C, YBR010W, YBR151W, YCL067C, YCR096C, YDL137W, YDL192W, YDR073W, YDR086C, YDR224C, YDR377W, YDR378C, YER015W, YGL187C, YHR162W, YJL167W, YJL216C, YKR009C, YLR165C, YMR197C, YNL157W, YOL002C, YOL109W, YOR180C, YPL010W, YPL233W, YBR036C, YDR297W, YGR149W, YGR224W, YNL043C, YPL067C, YPL170W, YCR046C, YDR387C, YFL050C, YGL051W, YHR132C, YIL112W, YJL141C, YKR098C, YLR052W, YLR206W, YML129C, YNL203C, YNR014W, YOL043C, YOL096C, YPR184W, YAL028W, YAL055W, YAR062W, YBL095W, YBL102W, YBR122C, YBR157C, YBR161W, YBR251W, YBR298C, YCR039C, YCR083W, YDL018C, YDL067C, YDL078C, YDL091C, YDL215C, YDL216C, YDR022C, YDR067C, YDR079W, YDR181C, YDR186C, YDR196C, YDR262W, YDR306C, YDR319C, YER188W, YGL004C, YGL035C, YGR036C, YGR062C, YGR120C, YGR131W, YGR141W, YGR167W, YGR287C, YHL024W, YHR080C, YHR097C, YIL077C, YJL046W, YJL070C, YJL096W, YJL113W, YJL146W, YJL180C, YJR019C, YJR049C, YKR058W, YLL005C, YLR078C, YLR151C, YLR271W, YLR295C, YLR351C, YLR375W, YMR023C, YMR025W, YMR135C, YMR210W, YMR267W, YMR278W, YMR293C, YNL073W, YNR037C, YNR040W, YNR072W, YOR028C, YOR316C, YOR328W, YOR363C, YPL039W, YPL040C, YPL099C, YPL107W, YPL134C, YPL138C, YPL140C.

The base sequences and the amino acid sequences of the yeast genes are disclosed in public databases such as MIPS in Germany: Munich Information Center for Protein Sequence, and SGD in USA: Saccharomyces Genome Database, and are known via the internet. The sequences of promoters are also disclosed in a public database of SCPD: The Promoter Database of *Saccharomyces cerevisiae.*

In addition to the promoters from yeast genes as described above, promoters from a gene that is homologous to the yeast genes and is derived from other species may be used in the invention. In this context, "a gene that is homologous to the yeast genes" means a gene that comprises a base sequence having a 50% or more, preferably 80% or more of the base sequences of yeast genes, wherein the base sequence encodes a protein having the same functions as the proteins encoded by the yeast genes.

A polynucleotide encoding a marker protein is operably linked to a polynucleotide of a promoter from the gene as described above so as to provide a polynucleotide construct. Processes to link a polynucleotide encoding a protein operably to a promoter is well known in the art. See, for example, R. W. Old, S. B. Primrose Principles of Gene Manipulation 5th Ed., BAIFUKAN CO., LTD, pp 138-165, pp.234-263,2000.

Examples of marker proteins include GFP (Green Fluorescence Protein) (Heim, R., Cubitt, A. B. and Tsien, R. Y. (1995) Nature 373, 663-664; Heim, R., Prasher DC. and Tsien, R. Y. (1994) Proc. Natl. Acad. Sci., 91, 12501-12504; Warg, S. and Hazerigg, T. (1994) Nature 639, 400-403; Youvan, D.C. and Michel-Beyerle, M.E. (1996) Nature Biotechnology 14 1219-1220; Chalfie, M., Tu, Y., Euskirchen, G., Ward, W. W. and Prasher, D.C. (1994) Science 263, 802-805), β-galactosidase (Canestro C, Albalat R, Escriva H, Gonzalez-Duarte R. Endogenous beta-galactosidase activity in amphioxus: a useful histochemical marker for the digestive system.Dev Genes Evol 2001 Mar 211(3): 154-6), luciferases (Arch Toxicol 2002 Jun;76(5-6):257-61, Estrogenic activity of UV filters determined by an in vitro reporter gene assay and an in vivo transgenic zebrafish assay.Schreurs R, Lanser P, Seinen W, Van Der Burg B.), and acetyltransferase (J Recept Signal Transduct Res 2001 Feb;21(1):71-84, A simplified method for large scale quantification of ranscriptional activity and its use in studies of steroids and steroid receptors. Zhang S, Lu J, Iyama K, Lo SC, Danielsen M.).

The present invention also relates to a vector that comprises a polynucleotide construct as described above.

A polynucleotide comprising a promoter sequence from yeast genes is obtained by preparing a primer that transcribes a likely necessary portion on the basis of the base sequence of the yeast genes as known in the public database, and amplifying the same by PCR using the genomic DNA of yeast as a template. Further, a plasmid that is capable of replicating in an intended cell is selected, and a base sequence of a marker protein is introduced into the plasmid. The polynucleotide comprising a promoter sequence as shown above is inserted upstream the marker gene to obtain an intended vector.

The present invention also relates to a host cell that is transformed with the vector as described above. It is natural to use preferably human cells as a host cell, and cells from other mammalian such as mouse may be used. Also, cells from fishes, nematode or the like as used in bioassay so far may be used in view of environmental toxic evaluation. Further, it is preferred to use microbiological cells since cultivation of them are easy. It is more preferred to use yeast cells because the present method is based on the use of genes from yeast cells, and because yeast is grown irrespective of variable environmental conditions such as salt concentration. Transformation of cells are well known. For example, see Kaiser C, Michaelis S, Mitchell A: Lithium acetate yeast transformation, Methods in Yeast Genetics, A Cold Spring Harbor Laboratory Course Manual 1994 edition (Cold Spring Harbor Laboratory Press) pp.

133-134,1994. The purpose may be attained without vector when the encoding region of the yeast gene is replaced with a polynucleotide sequence encoding a marker protein. The polynucleotide construct can be directly introduced into cells, and the method therefore is also well known.

The invention also relates to a process for detecting a toxic compound in a test material, which comprises:

(1) contacting the test material to the transformed cells as described above, and
(2) detecting the expression of mRNA encoding a marker protein.

The step to contact a test material to a cell comprises for example culturing the transformed cell in liquid at an appropriate condition for the cultivation of the cell, and adding the test material directly to the culture liquid.

Then, the expression level of a marker protein or a mRNA encoding the protein is determined.

The determination of the expression level of a marker protein may be conducted by destroying the cells, obtaining an extract containing the protein, and determining the level of marker protein in the extract. For example, when a marker protein is GFP, the fluorescence level in the protein extract is determined with a spectrofluorometer. Also, even when the cells are not destroyed, it is possible to determine them by observation and image processing with fluorescence microscopes and laser microscopes, determination with flow cytometry, and detection with evanescent lights.

The level of an expressed mRNA may be detected by 1) northern blotting (OGATA Nobukuni, NOJIMA Hiroshi: Genetic Engineering Keywords Book, revised 2nd ed., Yodosha, co.jp, 2000, pp299-301), 2) reverse transcription-PCR (RT-PCR) (NAKABEPPU Yusaku,et al.: Cell Technology, suppl. Tips series, Modified PCR Tips, Shujunsha Co.Ltd., 1999, pp25-43) and the like.

Procedures of northern blotting comprises electrophoresing the RNA, transferring the pattern to a filter, and hybridizing it with a specific probe labeled with an isotope, thereby analyzing the presences and the amount of mRNA in the sample as well as the length of the same. RT-PCR is a procedure for detection and quantitative determination of an intended RNA in a form of the amplified cDNA, which comprises forming cDNA from the RNA by reverse-transcription with reverse transcriptase, and conducting PCR using the cDNA as a starting material as well as specific primer sets and a thermostable DNA polymerase.

Toxic substances to be detected according to the present invention include, but are not limited to, $Na_2As$, $CdCl_2$, $HgCl_2$, $PbCl_2$, 4-nitroquinolin-N-oxide, 2,4,5-trichlorophenol, $\gamma$-hexachlorocyclohexane, manganese ethylenebis (dithiocarbamate), 2,4,5,6-tetrachloro-1,3-benzenedicarbonitrile, tetramethylthiuram disulfide, zinc N,N'-ethylenebis (dithiocarbamate), 8-methyl-N-vanillyl-6-nonenamide, gingerol, acrolein, dimethylsulfoxide, Roundup (trademark, herbicide) (N-(phosphomethyl) glycinate ammonium 41.0%, surfactant 59.0%), sodium dodecylbenzosulfonate, sodium lauryl sulfate , 2,4-dichlorophenoxyacetic acid, potassium cyanide, benzo(a)pyrene, formaldehyde, bisphenol-A, 2,5-dichlorophenol, methylmercury chloride, p-nonylphenol, pentachlorophenol, nickel (II) chloride, potassium bichromate , triphenyltin chloride, phenol, S-4-chlorobenzyl-N,N-diethylcarbamate, hexachlorophene, triclosan, and copper sulfate.

When the method as described above is conducted using two or more cells, both of which are transformed with a vector comprising a polynucleotide of a promoter from different yeast genes operably linked to a polynucleotide encoding a marker protein, then toxic substances can be further examined. For example, as shown in the working examples hereinafter, YLL057C can be used as a yeast gene promoter to detect 2,4-dichlorophenoxyacetic acid, arsenious acid or a salt thereof, cadmium salt, and cyanide or a salt thereof, and YLR303W can be used as a yeast gene promoter to detect 2,4-dichlorophenoxyacetic acid, arsenious acid or a salt thereof, cadmium salt, cyanide or a salt thereof, benzo(a)pyrene, formaldehyde, manganese ethylenebis (dithiocarbamate), and a mercuric salt. Accordingly, when for example the expression of a marker protein is induced by using YLR303W as yeast gene, and not induced by YLL057C, then toxic substances are identified as benzo(a)pyrene, mercuric salt, manganese ethylenebis(dithiocarbamate) or formaldehyde. When the expression of a marker protein is induced by using both yeast genes, then toxic substances are identified as 2,4-dichlorophenoxyacetic acid, arsenious acid or a salt thereof, cadmium salt, or cyanide or a salt thereof.

The following examples are presented for purpose of further illustration of the invention, and such examples are not intended to be limiting the invention in any respect.

EXAMPLES

Example 1

The following experiment was conducted to examine which yeast gene is useful for the detection of a toxic substance.

Yeast (*Saccharomyces cerevisiae* S288C (a SUC2ma1 me1 gap2 CUP1)) were cultured at 25°C on YPD medium (yeast extract 1%, polypepton 2%, glucose 2%). One of toxic chemical substances was added to the cell at logarithmic growth phase, and the cell was further cultured for two hours. Cell was cultured without any chemical substance in the same condition, and was used as control. Concentrations of the chemical substances were defined to inhibit the growth of the yeast but not lead to death.

| Chemical Substances | | Concentrations |
|---|---|---|
| (1) | $Na_2As$ | 0.3mM |
| (2) | $CdCl_2$ | 0.3mM |
| (3) | $HgCl_2$ | 0.7mM |
| (4) | $PbCl_2$ | 2mM |
| (5) | 4-nitroquinolin-N-oxide | 0.2μM |
| (6) | 2,4,5-trichlorophenol | 16μM |
| (7) | γ-hexachlorocyclohexane | 1.3mM |
| (8) | manganese ethylenebis(dithiocarbamate) | 2ppm |
| (9) | 2,4,5,6-tetrachloro-1,3- benzenedicarbonitrile | 10μM |
| (10) | Tetramethylthiuram disulfide | 75μM |
| (11) | zinc N,N'-ethylenebis(dithiocarbamate) | 2ppm |
| (12) | 8-methyl-N-vanillyl-6-nonenamide 0.82mM | |
| ( 13) | gingerol | 1.36mM |
| (14) | acrolein | 0.20mM |
| (15) | dimethylsulfoxide | 1.41 M |
| (16) | Roundup (trademark, herbicide) 1) | 1500-fold dilution |
| (17) | sodium dodecylbenzosulfonate | 0.02% |
| (18) | sodium lauryl sulfate | 0.01% |

1) N- (phosphomethyl) glycinate ammonium41.0%, surfactant 59.0%

After the completion of cultivation, the culture was centrifuged to collect the cells. To the cells, sodium acetate buffer (50mM sodium acetate, 10mM EDTA, 1% SDS) was added, and the mixture was shaken at 65°C for five minutes, followed by returning to room temperature, and obtaining the supernatant, which procedure was repeated two times. To the supernatant, 1/2 amount of a solution of phenol/chloroform was added, and the mixture was centrifuged to give a supernatant, which was added with an equal amount of chloroform, and the mixture was centrifuged to give a supernatant. To the supernatant, an equal amount of isopropanol containing 0.3 M sodium acetate was added, and the mixture was allowed to stand at room temperature for 30 minutes, after which the mixture was centrifuged to give a sediment of the whole RNAs. Seventy % ethanol was added to the sediment, and the mixture was again centrifuged to give a sediment, which was then dried and dissolved in water. mRNA was isolated from the whole RNAs as followings. In view of the fact that a poly A chain is attached to the 3' terminus of mRNA, a polynucleotide having a poly T structure which was immobilized on the surface of latex beads was used to trap the mRNA, and then the mRNA was washed and eluted with spine column (Oligotex-dT30<Super>mRNA Purification Kit, Takara). Reverse transcription of the mRNA was conducted with a reverse transcriptase (Super Script II Reverse Transcriptase; catalogue No. 18064-014, GibcoBRL) using fluorescence-labeled nucleotides to give cDNAs that were introduced with Cy3-dUTP or Cy5-dUTP during the reverse transcription.

The labeled cDNAs were dissolved in TE buffer (10mM Tris-HCl/1mM EDTA, pH8.0), and the solution was dropped on the DNA chip containing the whole genes of yeast (DNA Chip Research Inc. Japan) so that the cDNAs were hybridized on the DNA chip at 65°C for over 12 hours. The fluoresces intensity of the DNA chip was read with a scanner, and the ratio relative to the fluorescence intensity resulting from the absence of chemical substance was estimated as the following, which is shown in Tables 1 to 9:

$$\frac{\text{The level of expressed mRNA in the presence of chemical substance}}{\text{The level of expressed mRNA in the absence of chemical substance}}$$

In the Tables, "Intensity" indicated in the rightmost column is a value of the level of expressed mRNA of each gene in control cells as divided by the average level of the expressions of the whole genes. A gene of which mRNA level is lower in control, and is higher in cases of addition of chemical substance, is especially useful in the detection of toxic substances.

**Table 1 Unknown protein yeast genes**

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YCR102C | 3.4 | 3.2 | 2.4 | 1.1 | 0.7 | 0.4 | 1.1 | 4.9 | 9.8 | 61.5 | 13.5 | 0.5 | 1.1 | 4.1 | 0.9 | 1.2 | 1.1 | 1.2 | 0.53 |
| YDL218W | 3.5 | 1.2 | 2.6 | 1.4 | 0.7 | 0.9 | 1.1 | 1.5 | 72.5 | 12.6 | 5.7 | 0.9 | 1.1 | 4.8 | 1.1 | 3.5 | 1.1 | 0.9 | 0.42 |
| YDR533C | 3.4 | 4.9 | 3.5 | 1.8 | 1.3 | 2.2 | 1.7 | 5.4 | 7.3 | 12.7 | 7.7 | 1.4 | 3.0 | 2.9 | 2.5 | 6.7 | 2.3 | 2.7 | 1.82 |
| YGR110W | 1.6 | 3.1 | 2.2 | 5.9 | 0.9 | 0.9 | 1.7 | 1.5 | 6.4 | 4.8 | 0.5 | 1.4 | 3.6 | 2.8 | 1.2 | 17.1 | 0.9 | 2.1 | 0.19 |
| YKL071W | 8.3 | 3.8 | 5.4 | 2.8 | 1.1 | 18.6 | 3.7 | 24.7 | 162.2 | 109.2 | 40.3 | 1.0 | 8.4 | 21.9 | 1.7 | 2.2 | 1.0 | 0.9 | 0.31 |
| YLR460C | 2.1 | 2.1 | 2.0 | 1.1 | 1.3 | 0.6 | 1.1 | 8.1 | 13.9 | 31.6 | 14.3 | 0.4 | 0.7 | 3.5 | 0.7 | 1.8 | 1.0 | 1.1 | 0.48 |
| YMR090W | 6.9 | 9.5 | 2.3 | 3.5 | 0.7 | 3.8 | 2.7 | 3.9 | 16.7 | 6.1 | 8.8 | 2.2 | 6.9 | 6.5 | 1.9 | 5.1 | 1.8 | 2.8 | 1.30 |
| YOL150C | 4.1 | 3.7 | 9.9 | 0.9 | 1.0 | 5.2 | 2.4 | 3.7 | 10.8 | 8.3 | 9.4 | 2.9 | 8.9 | 5.7 | 1.8 | 3.1 | 2.7 | 2.2 | 0.47 |
| YBL048W | 2.0 | 7.9 | 2.3 | 2.8 | 1.3 | 2.0 | 1.9 | 1.2 | 7.2 | 4.4 | 2.0 | 1.2 | 2.7 | 2.0 | 4.1 | 5.7 | 1.3 | 1.0 | 0.29 |
| YBL107C | 1.4 | 1.1 | 0.4 | 1.5 | 1.2 | 1.1 | 2.1 | 1.9 | 1.0 | 3.1 | 2.9 | 1.4 | 2.6 | 2.5 | 1.4 | 1.5 | 0.9 | 1.4 | 1.03 |
| YFL024C | 1.0 | 1.1 | 1.1 | 0.7 | 1.1 | 2.0 | 3.3 | 0.9 | 0.5 | 0.5 | 0.6 | 0.8 | 0.9 | 3.3 | 0.9 | 1.1 | 0.9 | 0.8 | 0.37 |
| YGL121C | 2.6 | 5.0 | 1.4 | 4.2 | 0.9 | 9.8 | 4.7 | 3.0 | 7.0 | 15.0 | 6.1 | 1.6 | 7.8 | 5.8 | 1.6 | 8.5 | 2.7 | 3.5 | 0.60 |
| YHR029C | 2.8 | 1.8 | 1.8 | 1.8 | 1.0 | 2.7 | 2.0 | 2.7 | 13.6 | 8.6 | 3.8 | 1.5 | 4.7 | 3.5 | 1.0 | 1.0 | 2.1 | 2.1 | 0.87 |
| YHR209W | 2.8 | 1.1 | 0.8 | 4.5 | 1.8 | 3.2 | 5.0 | 2.0 | 2.9 | 6.4 | 3.3 | 1.0 | 2.0 | 2.2 | 5.0 | 3.5 | 2.6 | 2.6 | 0.56 |
| YKL107W | 2.4 | 2.5 | 0.5 | 0.8 | 1.1 | 2.0 | 1.1 | 0.9 | 28.2 | 8.3 | 2.0 | 1.0 | 1.9 | 3.4 | 1.7 | 3.5 | 1.6 | 0.9 | 0.17 |
| YKR075C | 1.5 | 0.7 | 2.7 | 4.5 | 1.2 | 1.5 | 1.4 | 2.0 | 0.2 | 0.6 | 1.3 | 2.3 | 3.8 | 2.7 | 0.4 | 4.3 | 2.4 | 1.9 | 1.15 |
| YLL056C | 2.1 | 2.3 | 3.0 | 2.0 | 1.1 | 0.6 | 1.0 | 1.3 | 58.8 | 8.8 | 1.7 | 1.3 | 6.8 | 11.0 | 1.5 | 4.8 | 5.3 | 2.5 | 0.32 |
| YLR297W | 2.4 | 2.1 | 3.5 | 1.7 | 1.3 | 0.8 | 1.1 | 1.3 | 2.1 | 5.5 | 3.5 | 0.8 | 0.8 | 2.3 | 0.7 | 1.8 | 0.7 | 1.0 | 0.81 |
| YOR338W | 1.8 | 1.8 | 0.5 | 1.7 | 1.6 | 2.6 | 1.3 | 2.2 | 0.6 | 3.5 | 0.8 | 1.2 | 1.4 | 2.5 | 1.8 | 1.0 | 1.1 | 0.5 | 0.52 |
| YOR391C | 2.1 | 2.0 | 0.9 | 1.2 | 1.3 | 1.6 | 2.0 | 2.8 | 18.8 | 18.1 | 3.2 | 1.1 | 2.3 | 2.4 | 5.8 | 2.2 | 1.0 | 0.9 | 0.22 |
| YPL280W | 1.7 | 1.9 | 1.9 | 2.0 | 1.4 | 1.9 | 1.8 | 2.3 | 49.8 | 14.0 | 1.9 | 0.9 | 2.4 | 2.4 | 4.3 | 1.5 | 1.2 | 0.9 | 0.22 |
| YLR346C | 4.4 | 2.0 | 2.1 | 4.5 | 1.4 | 5.5 | 5.6 | 1.3 | 8.6 | 6.6 | 0.5 | 4.2 | 6.2 | 2.2 | 1.0 | 2.4 | 10.6 | 7.8 | 0.53 |
| YOR049C | 2.5 | 0.9 | 12.8 | 1.4 | 1.3 | 8.4 | 4.2 | 0.9 | 1.1 | 1.9 | 1.1 | 6.7 | 7.1 | 1.5 | 1.3 | 4.3 | 2.6 | 2.9 | 0.22 |
| YAL034C | 1.1 | 1.8 | 2.8 | 1.1 | 1.0 | 1.5 | 1.8 | 1.0 | 3.0 | 3.3 | 1.5 | 2.8 | 4.9 | 1.2 | 1.4 | 1.0 | 1.2 | 1.3 | 0.52 |
| YDR476C | 0.7 | 1.8 | 6.0 | 1.2 | 0.5 | 3.1 | 1.5 | 1.3 | 1.7 | 3.4 | 1.6 | 2.1 | 2.8 | 1.1 | 2.2 | 2.0 | 2.4 | 1.5 | 0.58 |
| YGR035C | 1.6 | 0.6 | 1.2 | 0.6 | 1.3 | 3.3 | 1.1 | 0.9 | 1.1 | 0.5 | 0.4 | 3.3 | 2.1 | 0.8 | 0.3 | 1.9 | 2.5 | 4.3 | 1.04 |
| YGR284C | 1.0 | 2.0 | 4.0 | 1.4 | 1.2 | 1.4 | 2.2 | 1.1 | 4.3 | 1.8 | 1.7 | 2.1 | 3.6 | 1.2 | 3.9 | 1.9 | 1.3 | 2.1 | 2.57 |
| YHR054C | 1.7 | 0.9 | 2.2 | 1.0 | 1.0 | 2.9 | 1.6 | 1.8 | 2.6 | 7.7 | 0.9 | 3.6 | 5.8 | 1.0 | 0.8 | 2.2 | 5.1 | 3.4 | 0.80 |
| YLR008C | 0.9 | 0.5 | 0.7 | 0.9 | 1.3 | 2.7 | 2.0 | 1.0 | 0.4 | 0.7 | 0.8 | 3.5 | 6.2 | 1.6 | 0.7 | 1.7 | 5.4 | 4.9 | 2.00 |

| | | | | | | | | | | | | | | | | | | | |
|---|---|---|---|---|---|---|---|---|---|---|---|---|---|---|---|---|---|---|---|
| YMR315W | | 4.3 | 1.8 | 1.7 | | 4.2 | 2.9 | 2.0 | 2.4 | 2.6 | 3.2 | 2.0 | 2.5 | 1.3 | 2.5 | 1.6 | 2.1 | 2.8 | 1.76 |
| YNL211C | 1.2 | 1.0 | 0.8 | 1.0 | 1.0 | 7.4 | 3.1 | 1.1 | 0.8 | 1.1 | 1.1 | 3.2 | 9.9 | 1.1 | 0.8 | 9.3 | 4.2 | 6.8 | 0.52 |
| YOL031C | 0.9 | 1.9 | 1.0 | 1.7 | 1.2 | 1.0 | 2.5 | 2.1 | 2.3 | 1.4 | 1.3 | 2.1 | 2.4 | 1.1 | 4.4 | 1.5 | 1.2 | 1.9 | 1.20 |
| YOL101C | 0.9 | 0.8 | 0.4 | 1.7 | 1.6 | 0.5 | 0.7 | 0.9 | 0.1 | 0.3 | 0.9 | 2.4 | 0.2 | 0.8 | 0.6 | 3.5 | 0.6 | 0.6 | 1.37 |
| YAR031W | 1.5 | 1.1 | 1.1 | 1.7 | 1.1 | 2.9 | 1.6 | 1.2 | 2.7 | 1.6 | 1.4 | 1.2 | 3.3 | 1.4 | 0.9 | 3.5 | 1.6 | 2.5 | 1.00 |
| YBL049W | 2.9 | 11.8 | 2.1 | 2.9 | 1.1 | | 1.7 | 1.9 | 5.2 | 5.2 | 2.2 | 0.7 | 3.9 | 1.5 | 7.2 | 6.4 | 1.1 | 1.1 | 0.47 |
| YBR062C | 1.3 | 1.7 | 0.4 | 1.2 | 1.4 | 1.9 | 1.5 | 1.9 | 5.4 | 2.2 | 2.3 | 1.2 | 2.8 | 2.6 | 0.7 | 2.2 | 1.4 | 1.6 | 0.93 |
| YCR013C | 1.3 | 1.4 | 5.0 | 1.1 | 1.1 | 1.8 | 1.6 | 0.7 | 0.8 | 1.3 | 2.4 | 1.4 | 3.9 | 1.5 | 1.6 | 1.6 | 1.1 | 1.3 | 2.80 |
| YDL027C | 1.2 | 2.0 | 1.7 | 1.7 | 1.2 | 2.1 | 2.9 | 1.5 | 2.0 | 2.3 | 1.7 | 1.7 | 3.2 | 1.1 | 1.4 | 1.8 | 1.4 | 2.0 | 0.69 |
| YDL110C | 1.5 | 1.4 | 1.1 | 2.1 | 0.8 | 1.7 | 2.5 | 1.8 | 3.0 | 2.3 | 1.9 | 1.5 | 2.7 | 2.7 | 1.3 | 3.6 | 2.0 | 4.4 | 1.27 |
| YDL169C | 1.7 | 1.7 | 1.1 | 1.3 | 1.1 | 2.6 | 2.1 | 1.3 | 7.3 | 4.7 | 1.2 | 1.2 | 3.7 | 2.7 | 2.2 | 3.8 | 1.5 | 1.6 | 0.42 |
| YDR070C | 2.2 | 6.4 | 3.6 | 3.5 | 0.9 | 6.2 | 1.4 | 1.9 | 13.9 | 6.0 | 4.1 | 4.3 | 6.6 | 2.0 | 2.1 | 15.1 | 1.2 | 3.1 | 0.44 |
| YDR210W | 1.1 | 1.0 | 0.9 | 1.0 | 2.0 | 1.2 | 1.5 | 1.4 | 0.5 | 1.0 | 1.0 | 1.0 | 2.3 | 1.1 | 1.0 | 1.5 | 1.9 | 1.9 | 1.82 |
| YDR214W | 1.3 | 4.0 | 4.5 | 1.3 | 1.1 | 1.7 | 1.5 | 1.5 | 5.5 | 2.4 | 1.9 | 1.1 | 5.3 | 1.2 | 1.1 | 0.7 | 1.2 | 1.0 | 1.07 |
| YDR435C | 1.2 | 1.1 | 1.2 | 1.4 | 1.2 | 2.5 | 2.1 | 1.2 | 0.8 | 2.1 | 1.3 | 1.1 | 2.5 | 1.3 | 0.6 | 2.0 | 1.3 | 2.0 | 1.30 |
| YER037W | 2.0 | 2.1 | 0.8 | 1.1 | 1.3 | 3.2 | 2.6 | 1.0 | 1.0 | 5.5 | 1.5 | 1.2 | 3.0 | 1.7 | 0.8 | 1.8 | 1.7 | 2.6 | 1.17 |
| YFL044C | 1.0 | 1.5 | 0.8 | 2.3 | 1.6 | 1.3 | 1.6 | 1.4 | 5.2 | 3.7 | 2.2 | 1.2 | 4.7 | 1.2 | 1.0 | 1.8 | 1.3 | 1.4 | 0.50 |
| YFR003C | 1.2 | 1.5 | 0.8 | 1.6 | 1.0 | 2.2 | 1.4 | 1.4 | 8.3 | 3.1 | 1.4 | 0.9 | 2.5 | 2.1 | 1.0 | 1.4 | 1.3 | 1.8 | 1.01 |
| YFR020W | 1.0 | 0.9 | 1.2 | 1.0 | 0.9 | 1.4 | 1.1 | 0.9 | 3.7 | 3.5 | 1.4 | 0.9 | 2.3 | 1.8 | 2.6 | 1.0 | 1.2 | 0.9 | 0.61 |
| YFR044C | 0.9 | 1.3 | 3.8 | 1.1 | 0.9 | 2.1 | 1.2 | 0.8 | 2.1 | 2.2 | 1.9 | 1.2 | 3.1 | 0.8 | 1.5 | 1.2 | 1.0 | 1.0 | 1.99 |
| YGR010W | 1.2 | 1.6 | 0.9 | 1.2 | 0.8 | 1.2 | 1.7 | 1.6 | 13.6 | 8.9 | 1.5 | 0.9 | 5.2 | 1.9 | 1.3 | 1.4 | 0.8 | 1.3 | 0.55 |
| YGR142W | 2.7 | 6.2 | 15.3 | 1.3 | 1.3 | 0.9 | 1.4 | 1.5 | 36.8 | 12.2 | 2.3 | 2.0 | 8.9 | 0.9 | 1.3 | 0.3 | 1.9 | 1.5 | 1.28 |
| YGR161C | 2.0 | 2.2 | 5.4 | 3.8 | 1.0 | 1.4 | 1.4 | 1.3 | 2.0 | 3.8 | 0.8 | 1.7 | 2.8 | 0.9 | 1.7 | 1.1 | 1.5 | 1.8 | 0.65 |
| YGR212W | 1.5 | 1.7 | 1.5 | 1.3 | 1.2 | | | 1.0 | 1.1 | 3.8 | 0.9 | 1.5 | 10.0 | 1.1 | 1.1 | 2.1 | 3.9 | 2.2 | 0.28 |
| YGR268C | 1.1 | 2.3 | 2.0 | 2.5 | 1.1 | 1.1 | 1.0 | 1.1 | 4.1 | 1.6 | 0.8 | 1.0 | 2.8 | 1.0 | 1.5 | 1.2 | 1.5 | 1.4 | 0.48 |
| YHR016C | 0.8 | 1.5 | 1.7 | 1.5 | | 1.8 | 3.7 | 0.8 | 2.8 | 2.0 | 2.2 | 1.3 | 3.3 | 0.9 | 0.6 | 2.1 | 1.2 | 1.4 | 0.51 |
| YHR087W | 1.2 | 2.7 | 8.6 | 2.8 | 0.7 | 4.2 | 3.4 | 1.2 | 4.1 | 3.9 | 3.7 | 1.4 | 4.0 | 1.5 | 1.9 | 3.8 | 1.2 | 2.9 | 1.34 |
| YJL048C | 1.7 | 2.5 | 2.0 | 2.9 | 0.9 | 1.5 | 0.7 | 1.5 | 1.2 | 3.8 | 3.2 | 1.1 | 2.4 | 1.7 | 0.6 | 2.3 | 1.6 | 2.5 | 0.82 |
| YJL144W | 1.9 | 1.6 | 2.2 | 1.6 | 1.2 | 1.7 | 1.7 | 1.3 | 14.6 | 8.9 | 3.0 | 0.8 | 3.9 | 1.6 | 1.0 | 2.5 | 2.2 | 2.4 | 0.60 |
| YJL163C | 1.2 | 1.9 | 1.7 | 1.5 | 0.9 | 2.8 | 2.3 | 0.7 | 14.5 | 2.5 | 3.1 | 2.0 | 4.5 | 1.0 | 2.0 | 2.8 | 1.5 | 1.7 | 0.28 |
| YJR074W | 1.0 | 1.4 | 2.0 | 2.0 | 0.7 | 1.2 | 1.8 | 1.3 | 3.7 | 3.1 | 1.2 | 2.0 | 2.8 | 1.7 | 3.4 | 1.5 | 1.8 | 1.9 | 0.68 |
| YKL065C | 1.9 | 2.0 | 1.3 | 1.7 | 1.0 | 2.3 | 3.8 | 1.8 | 3.1 | 3.9 | 1.3 | 1.3 | 3.0 | 2.1 | 1.7 | 2.8 | 2.8 | 4.6 | 2.29 |

| | | | | | | | | | | | | | | | | | | |
|---|---|---|---|---|---|---|---|---|---|---|---|---|---|---|---|---|---|---|
| YKR011C | 1.1 | 1.6 | 0.9 | 1.6 | 1.2 | 2.3 | 2.7 | 2.0 | 4.7 | 4.4 | 1.5 | 1.1 | 3.5 | 1.4 | 1.8 | 1.3 | 1.3 | 1.9 | 0.66 |
| YKR018C | 0.7 | 1.1 | 1.7 | 0.7 | 0.9 | 1.7 | 1.0 | 0.7 | 3.9 | 1.7 | 1.4 | 1.0 | 3.0 | 1.1 | 1.0 | 1.8 | 1.0 | 1.2 | 0.98 |
| YKR046C | 1.9 | 7.8 | 2.8 | 1.8 | 0.6 | 1.9 | 1.3 | 0.8 | 3.9 | 2.0 | 2.6 | 1.3 | 2.8 | 1.9 | 2.9 | 3.5 | 1.2 | 1.9 | 0.73 |
| YKR049C | 1.6 | 4.2 | 1.0 | 3.2 | 0.9 | 3.0 | 2.4 | 1.3 | 2.2 | 3.2 | 2.2 | 1.2 | 4.1 | 2.1 | 0.9 | 4.2 | 1.6 | 3.3 | 1.17 |
| YLR132C | 1.0 | 1.1 | 0.3 | 1.1 | 1.2 | 1.1 | 1.1 | 0.7 | 4.5 | 2.5 | 0.5 | 1.0 | 2.7 | 1.3 | 2.3 | 1.6 | 1.2 | 1.6 | 0.36 |
| YLR161W | 0.9 | 0.8 | 0.8 | 0.7 | 0.9 | 1.2 | 1.3 | 0.8 | 1.0 | 1.5 | 1.5 | 0.8 | 2.1 | 1.0 | 0.6 | 1.1 | 1.1 | 0.9 | 0.33 |
| YLR217W | 0.9 | 2.0 | 2.1 | 0.7 | 1.4 | 1.4 | 1.5 | 1.0 | 3.2 | 8.2 | | 0.9 | 3.8 | 0.9 | 0.9 | 0.8 | 1.0 | 1.2 | 0.35 |
| YLR328W | 1.3 | 1.2 | 1.3 | 0.9 | 0.9 | 4.8 | 5.4 | 1.1 | 0.9 | 1.2 | 1.4 | 1.2 | 4.3 | 0.5 | 0.8 | 1.4 | 1.1 | 0.8 | 1.04 |
| YML128C | 1.5 | 3.3 | 4.1 | 4.1 | 0.5 | 5.1 | 2.8 | 1.1 | 5.1 | 3.1 | 2.9 | 1.4 | 4.9 | 1.4 | 2.6 | 5.6 | 2.0 | 5.5 | 1.66 |
| YMR040W | 3.1 | 2.9 | 0.9 | 2.8 | 1.7 | 2.8 | 5.1 | 1.7 | 1.4 | 1.4 | 1.5 | 2.1 | 5.2 | 1.8 | 2.0 | 2.7 | 3.1 | 3.7 | 0.62 |
| YMR251W | 1.3 | 1.3 | 1.8 | 0.6 | 2.2 | 1.6 | 3.1 | 1.7 | 3.1 | 11.6 | 2.8 | 1.2 | 2.5 | 1.5 | 0.4 | 1.3 | 1.5 | 0.9 | 0.20 |
| YMR322C | 2.0 | 4.3 | 1.5 | 2.9 | 1.4 | 1.8 | 1.7 | 2.1 | 21.3 | 10.3 | 3.2 | 1.1 | 2.1 | 2.7 | 3.6 | 1.8 | 1.4 | 0.8 | 0.18 |
| YNL094W | 0.9 | 1.4 | 1.1 | 1.0 | 1.4 | 2.9 | 1.2 | 2.0 | 3.5 | 2.6 | 1.7 | 1.2 | 3.4 | 1.0 | 1.5 | 1.5 | 1.1 | 1.3 | 0.66 |
| YNL134C | 2.3 | 6.0 | 4.5 | 0.9 | 1.7 | 1.4 | 1.5 | 3.2 | 6.1 | 5.9 | 5.0 | 1.1 | 3.1 | 1.4 | 0.8 | 2.1 | 1.3 | 2.0 | 2.51 |
| YNL155W | 1.1 | 1.2 | 1.1 | 0.9 | 1.4 | 3.0 | 1.3 | 1.7 | 5.8 | 2.7 | 2.2 | 1.1 | 4.4 | 2.1 | 2.0 | 2.0 | 1.1 | 1.6 | 1.89 |
| YOR059C | 1.2 | 1.2 | 0.4 | 0.9 | 1.4 | 1.1 | 2.2 | 1.3 | 7.6 | 4.6 | 1.0 | 1.0 | 3.3 | 2.2 | 3.5 | 1.8 | 1.3 | 1.7 | 0.71 |
| YOR152C | 4.5 | 2.5 | 3.4 | 2.9 | 0.8 | 4.5 | 3.2 | 1.2 | 10.0 | 5.0 | 2.7 | 2.9 | 7.4 | 1.7 | 3.6 | 2.1 | 4.0 | 2.2 | 0.42 |
| YOR173W | 0.7 | 2.3 | 6.3 | 2.0 | 0.9 | 3.5 | 4.9 | 1.4 | 5.2 | 3.7 | 2.3 | 1.9 | 3.8 | 2.8 | 0.7 | 5.7 | 1.7 | 3.8 | 0.54 |
| YOR289W | 1.6 | 1.5 | 1.0 | 2.0 | 1.2 | 4.2 | 6.1 | 2.4 | 2.4 | 1.7 | 2.6 | 1.5 | 4.3 | 2.0 | 1.6 | 3.0 | 2.3 | 3.9 | 0.74 |
| YPR030W | 1.7 | 1.4 | 1.4 | 1.0 | 1.2 | 1.3 | 1.2 | 0.9 | 2.5 | 2.4 | 1.2 | 1.4 | 3.2 | 1.5 | 1.5 | 3.1 | 1.4 | 2.1 | 0.30 |
| YAL008W | 1.4 | 2.4 | 2.0 | 2.4 | 0.7 | 1.6 | 1.9 | 1.3 | 1.8 | 1.5 | 2.2 | 1.3 | 2.2 | 2.6 | 0.9 | 4.2 | 1.4 | 1.9 | 1.04 |
| YBR053C | 1.0 | 1.6 | 2.4 | 2.4 | 1.6 | 2.7 | 4.1 | 1.2 | 2.9 | 2.1 | 2.3 | 1.1 | 2.4 | 1.0 | 0.7 | 2.8 | 1.9 | 2.4 | 1.25 |
| YBR099C | 1.0 | 1.4 | 7.2 | 1.8 | 1.0 | 0.5 | 0.9 | 1.5 | 3.4 | 12.9 | 0.4 | 1.0 | 2.1 | 1.6 | 0.8 | 0.5 | 0.8 | 0.9 | 0.47 |
| YBR137W | 1.1 | 2.2 | 1.4 | 3.5 | 0.9 | 2.4 | 2.7 | 2.0 | 2.2 | 1.8 | 2.4 | 1.4 | 2.1 | 1.8 | 0.6 | 2.8 | 1.4 | 2.6 | 1.40 |
| YBR203W | 1.4 | 1.4 | 1.6 | 2.4 | 0.9 | 0.6 | 0.9 | 1.3 | 1.1 | 1.8 | 1.3 | 1.6 | 2.4 | 1.3 | 1.0 | 18.0 | 1.4 | 2.2 | 0.38 |
| YCL049C | 1.9 | 1.3 | 1.4 | 1.2 | 1.6 | 2.3 | 2.1 | 1.0 | 2.0 | 1.9 | 1.6 | 1.4 | 2.4 | 1.8 | 1.4 | 2.1 | 1.4 | 1.4 | 0.86 |
| YCR082W | 1.4 | 1.2 | 0.7 | 1.2 | 1.3 | 1.3 | 1.5 | 1.5 | 4.3 | 2.3 | 2.0 | 1.0 | 2.0 | 2.4 | 1.6 | 2.2 | 1.2 | 1.7 | 1.62 |
| YDL054C | 1.3 | 2.2 | 2.7 | 1.1 | 1.2 | 1.2 | 0.9 | 0.7 | 3.5 | 2.1 | 2.1 | 1.1 | 2.7 | 1.2 | 1.0 | 1.0 | 1.0 | 1.0 | 0.92 |
| YDL115C | 1.4 | 1.0 | 0.5 | 1.8 | 0.8 | 1.5 | 1.8 | 1.6 | 6.6 | 3.6 | 1.8 | 1.1 | 1.9 | 2.3 | 1.2 | 1.6 | 1.3 | 1.8 | 0.83 |
| YDL144C | 0.8 | 0.9 | 1.2 | 1.5 | 1.0 | 1.8 | 1.0 | 0.9 | 3.6 | 1.2 | 1.9 | 1.0 | 2.1 | 1.2 | 1.0 | 1.1 | 0.9 | 0.9 | 0.52 |
| YDL223C | 0.7 | 1.1 | 5.0 | 2.5 | 0.9 | 0.8 | 1.0 | 0.3 | 3.8 | 1.5 | 5.2 | 1.0 | 3.1 | 1.3 | 3.1 | 7.2 | 0.9 | 1.0 | 0.32 |
| YDR032C | 1.7 | 2.2 | 3.5 | 2.5 | 1.4 | 2.7 | 2.8 | 2.4 | 3.1 | 2.9 | 3.3 | 1.8 | 2.9 | 2.8 | 1.3 | 3.8 | 1.9 | 3.5 | 3.50 |

| | | | | | | | | | | | | | | | | | | | |
|---|---|---|---|---|---|---|---|---|---|---|---|---|---|---|---|---|---|---|---|
| YDR330W | 0.8 | 1.5 | 1.2 | 0.8 | 1.2 | 2.3 | 1.3 | 1.1 | 3.9 | 2.6 | 0.9 | 1.0 | 2.2 | 1.1 | 1.6 | 1.4 | 1.5 | 1.3 | 0.53 |
| YDR411C | 0.9 | 2.8 | 1.8 | 1.2 | 1.6 | 0.8 | 2.0 | 0.9 | 0.7 | 1.0 | 1.3 | 1.5 | 1.9 | 0.8 | 4.1 | 1.5 | 1.5 | 1.3 | 0.56 |
| YDR511W | 1.3 | 1.7 | 0.9 | 2.3 | 1.0 | 2.0 | 1.5 | 1.7 | 2.5 | 2.2 | 1.2 | 0.8 | 3.1 | 2.2 | 0.8 | 2.2 | 1.3 | 1.5 | 0.66 |
| YDR545W | 0.6 | 1.2 | 0.6 | 0.7 | 1.4 | 1.0 | 1.2 | 0.6 | 1.8 | 0.7 | 0.7 | 0.9 | 2.0 | 0.7 | 0.9 | 0.9 | 0.9 | 0.9 | 1.06 |
| YER004W | 1.0 | 1.5 | 1.9 | 1.4 | 1.5 | 1.2 | 1.4 | 1.1 | 4.5 | 2.9 | 2.2 | 1.7 | 4.6 | 1.8 | 1.4 | 2.3 | 1.4 | 1.2 | 1.48 |
| YER035W | 1.4 | 1.6 | 1.6 | 1.9 | 1.5 | 0.9 | 1.9 | 1.2 | 1.2 | 1.4 | 1.4 | 1.1 | 1.9 | 1.0 | 0.9 | 2.1 | 2.2 | 2.1 | 0.85 |
| YER079W | 1.1 | 1.6 | 1.9 | 2.0 | 0.8 | 1.5 | 1.9 | 1.4 | 5.3 | 3.4 | 2.1 | 0.9 | 2.1 | 1.6 | 0.5 | 1.4 | 1.0 | 1.6 | 0.69 |
| YER158C | 1.2 | 0.9 | 0.9 | 3.1 | 0.8 | 0.7 | 1.2 | 0.9 | 2.1 | 1.6 | 2.3 | 1.0 | 3.1 | 0.9 | 0.6 | 1.6 | 1.6 | 1.2 | 0.61 |
| YER163C | 1.1 | 2.4 | 1.2 | 1.6 | 0.8 | 2.0 | 1.5 | 1.4 | 5.4 | 3.4 | 1.6 | 1.0 | 2.9 | 1.9 | 1.6 | 2.4 | 1.2 | 1.2 | 0.62 |
| YER175C | 0.9 | 1.2 | 1.3 | 2.2 | 0.9 | 0.8 | 2.3 | 1.1 | 1.9 | 11.5 | 1.1 | 0.9 | 4.3 | 1.1 | 1.3 | 2.8 | 0.9 | 1.2 | 0.53 |
| YFL006W | 0.8 | 2.8 | 1.6 | 1.0 | 1.4 | 1.2 | 1.0 | 1.0 | 3.2 | 1.7 | 1.2 | 1.2 | 3.1 | 1.7 | 2.4 | 1.1 | 1.0 | 0.8 | 0.82 |
| YFL010C | 1.0 | 4.8 | 1.3 | 1.0 | 1.3 | 1.9 | 1.5 | 1.0 | 3.1 | 1.4 | 1.4 | 1.5 | 3.3 | 1.4 | 1.7 | 1.4 | 1.9 | 1.6 | 0.77 |
| YFL032W | 0.4 | 1.5 | 1.6 | 0.8 | 0.9 | 0.6 | 1.1 | 0.4 | 0.5 | 0.6 | 1.1 | 0.7 | 2.4 | 0.6 | 2.0 | 0.7 | 1.7 | 0.8 | 1.62 |
| YGL037C | 1.5 | 2.7 | 6.5 | 2.1 | 0.9 | 1.3 | 2.9 | 1.2 | 2.1 | 1.6 | 3.9 | 0.8 | 2.3 | 1.0 | 0.8 | 1.5 | 1.3 | 2.6 | 4.58 |
| YGL047W | 1.0 | 1.9 | 1.6 | 2.3 | 1.3 | 1.5 | 1.7 | 1.1 | 4.5 | 5.7 | 1.9 | 1.1 | 2.6 | 2.3 | 1.7 | 2.9 | 1.1 | 1.2 | 0.97 |
| YGL053W | 1.5 | 1.5 | 1.2 | 2.5 | 2.0 | 2.7 | 3.7 | 1.2 | 3.3 | 2.4 | 1.3 | 1.3 | 3.3 | 1.1 | 1.1 | 3.9 | 2.2 | 3.7 | 1.26 |
| YGL199C | 1.1 | 1.7 | 1.7 | 1.0 | 0.7 | 0.7 | 1.3 | 0.7 | | 0.1 | | 0.9 | 2.0 | 0.8 | 1.6 | 1.6 | 1.2 | 1.0 | 0.36 |
| YGR101W | 1.0 | 1.3 | 1.2 | 1.1 | 1.2 | 0.9 | 1.5 | 0.8 | 4.0 | 1.4 | 2.0 | 1.0 | 2.4 | 0.9 | 1.1 | 2.0 | 1.0 | 1.1 | 1.04 |
| YGR130C | 0.9 | 0.7 | 0.6 | 1.6 | 1.4 | 0.9 | 1.3 | 0.9 | 2.0 | 1.5 | 0.8 | 0.7 | 1.9 | 0.9 | 1.2 | 2.2 | 1.3 | 1.6 | 0.78 |
| YGR154C | 1.8 | 2.9 | 2.2 | 0.5 | 1.2 | 1.4 | 2.0 | 1.7 | 25.2 | 9.1 | 1.5 | 0.9 | 2.5 | 2.2 | 0.7 | 2.0 | 1.0 | 1.2 | 0.36 |
| YGR221C | 0.9 | 2.2 | 0.9 | 1.4 | 0.9 | 3.0 | 1.2 | 1.1 | | 0.7 | 0.5 | 1.6 | 2.3 | 1.4 | 1.5 | 2.7 | 1.3 | 1.3 | 0.29 |
| YHR138C | 2.8 | 1.8 | 2.2 | 2.8 | 1.5 | 3.8 | 3.0 | 1.4 | 5.3 | 4.6 | 1.6 | 1.8 | 4.0 | 3.4 | 2.2 | 3.3 | 5.0 | 7.1 | 1.89 |
| YHR199C | 1.9 | 2.0 | 3.2 | 2.0 | 1.2 | 1.1 | 1.1 | 1.0 | 8.6 | 6.0 | 1.9 | 1.1 | 2.1 | 2.0 | 2.8 | 3.3 | 1.1 | 0.9 | 0.63 |
| YIL041W | 1.0 | 1.2 | 2.1 | 1.3 | 0.5 | 1.0 | 0.8 | 0.9 | 1.8 | 1.4 | 1.4 | 1.0 | 2.3 | 1.3 | 1.3 | 0.7 | 0.8 | 1.1 | 1.33 |
| YIL087C | 1.3 | 1.9 | 3.4 | 1.6 | 1.4 | 4.1 | 1.8 | 1.1 | 2.4 | 1.8 | 2.8 | 1.1 | 2.4 | 1.3 | 0.4 | 5.6 | 1.7 | 1.6 | 0.68 |
| YJL057C | 1.5 | 1.2 | 1.6 | 1.7 | 1.5 | 1.4 | 2.7 | 1.5 | 7.3 | 6.1 | 2.0 | 1.0 | 2.5 | 1.6 | 1.4 | 1.9 | 1.4 | 1.5 | 0.51 |
| YJL066C | 0.9 | 1.2 | 1.2 | 2.5 | 0.8 | 1.4 | 1.6 | 0.8 | 2.0 | 2.1 | 0.8 | 1.0 | 2.2 | 1.0 | 1.3 | 2.8 | 1.4 | 1.5 | 0.43 |
| YJL082W | 1.4 | 0.8 | 2.4 | 1.1 | 1.7 | 0.2 | 0.4 | 0.9 | 1.4 | 2.9 | 1.0 | 1.3 | 2.1 | 0.5 | 3.2 | 0.2 | 0.6 | 0.3 | 2.07 |
| YJL151C | 1.0 | 2.4 | 3.2 | 1.2 | 1.3 | 2.2 | 1.4 | 0.8 | 0.5 | 1.6 | 1.0 | 1.3 | 2.4 | 1.3 | 1.0 | 2.5 | 3.5 | 2.4 | 1.69 |
| YJL152W | 0.9 | 1.5 | 1.8 | 1.3 | 1.6 | 1.5 | 1.2 | 0.7 | 0.3 | 1.0 | 1.0 | 1.0 | 1.8 | 1.4 | 0.8 | 1.6 | 2.5 | 1.7 | 0.80 |
| YJL161W | 1.7 | 2.2 | 1.5 | 4.6 | 1.1 | 8.0 | 3.6 | 1.4 | 3.1 | 2.1 | 3.7 | 1.0 | 2.5 | 2.2 | 0.6 | 4.4 | 1.9 | 2.1 | 0.44 |
| YJL171C | 2.3 | 0.7 | 2.8 | 1.4 | 1.0 | 2.7 | 1.4 | 0.9 | 1.0 | 1.7 | 2.2 | 1.0 | 2.9 | 1.0 | 1.8 | 1.0 | 3.6 | 2.9 | 1.94 |

| | | | | | | | | | | | | | | | | | | | |
|---|---|---|---|---|---|---|---|---|---|---|---|---|---|---|---|---|---|---|---|
| YJR008W | 1.6 | 1.5 | 1.2 | 1.7 | 1.4 | 2.2 | 2.9 | 1.1 | 1.8 | 1.9 | 1.4 | 1.3 | 2.0 | 1.2 | 0.9 | 3.3 | 1.5 | 2.7 | 0.87 |
| YKL151C | 1.2 | 3.1 | 1.7 | 2.5 | 1.0 | 2.0 | 5.6 | 1.0 | 3.3 | 3.5 | 2.3 | 1.0 | 2.0 | 1.3 | 1.4 | 3.7 | 1.4 | 1.8 | 1.02 |
| YKL153W | 1.1 | 1.4 | 2.0 | 0.7 | 1.0 | 2.9 | 1.1 | 0.7 | 0.8 | 1.4 | 2.1 | 1.1 | 2.4 | 1.1 | 1.5 | 1.1 | 0.9 | 1.3 | 1.60 |
| YKL195W | 0.8 | 0.5 | 0.4 | 1.1 | 1.9 | 2.5 | 2.1 | 1.2 | 3.6 | 2.1 | 1.0 | 1.1 | 2.4 | 1.6 | 0.9 | 1.7 | 1.4 | 1.6 | 1.58 |
| YLR054C | 2.0 | 1.5 | 0.4 | 2.4 | 1.3 | 1.2 | 1.1 | 1.1 | 2.7 | 1.1 | 0.7 | 1.1 | 3.2 | 1.1 | 0.7 | 0.9 | 1.6 | 1.1 | 0.14 |
| YLR149C | 1.4 | 1.9 | 3.4 | 2.3 | 0.8 | 1.4 | 1.6 | 1.1 | 6.2 | 5.2 | 2.8 | 2.0 | 3.0 | 1.2 | 1.3 | 2.8 | 1.3 | 3.0 | 0.33 |
| YLR152C | 0.7 | 1.3 | 1.1 | 0.9 | 1.1 | 2.1 | 1.4 | 1.2 | 0.6 | 1.1 | 3.6 | 1.0 | 2.8 | 1.2 | 0.8 | 2.8 | 1.5 | 1.8 | 0.46 |
| YLR324W | 0.7 | 1.2 | 0.9 | 1.0 | 1.3 | 1.2 | 1.0 | 1.0 | 3.1 | 1.4 | 0.9 | 0.8 | 2.3 | 1.4 | 1.6 | 1.7 | 1.2 | 1.2 | 0.49 |
| YLR350W | 1.2 | 1.9 | 3.2 | 2.3 | 0.8 | 1.6 | 1.2 | 1.0 | 0.6 | 1.4 | 0.9 | 1.8 | 4.5 | 2.8 | 1.6 | 2.9 | 1.6 | 1.1 | 1.52 |
| YLR387C | 0.9 | 1.6 | 0.8 | 0.6 | 1.3 | 1.9 | 1.1 | 1.4 | 7.8 | 3.0 | 0.9 | 1.1 | 3.6 | 1.7 | 1.9 | 1.0 | 1.0 | 1.4 | 1.20 |
| YML117W | 0.9 | 2.2 | 0.9 | 1.6 | 0.8 | 1.0 | 0.7 | 0.7 | 1.0 | 1.4 | 0.8 | 0.8 | 2.0 | 0.8 | 2.8 | 0.7 | 1.1 | 1.3 | 0.70 |
| YMR009W | 2.3 | 1.9 | 0.5 | 1.9 | 1.9 | 1.2 | 1.9 | 1.4 | 3.4 | 2.2 | 2.1 | 0.6 | 2.3 | 1.9 | 0.6 | 2.3 | 2.7 | 2.5 | 0.75 |
| YMR067C | 0.9 | 0.9 | 1.1 | 0.4 | 1.1 | 1.4 | 1.2 | 1.1 | 4.0 | 3.5 | 1.1 | 1.1 | 2.2 | 1.5 | 1.4 | 1.0 | 1.0 | 1.0 | 0.49 |
| YMR097C | 1.5 | 1.0 | 0.7 | 1.6 | 0.7 | 1.2 | 1.5 | 1.5 | 2.1 | 2.1 | 1.5 | 1.2 | 2.1 | 2.1 | 1.7 | 1.7 | 1.4 | 1.8 | 0.59 |
| YMR102C | 1.0 | 2.9 | 1.1 | 0.8 | 1.2 | 6.3 | 1.8 | 1.0 | 1.7 | 2.6 | 1.5 | 2.4 | 4.4 | 0.7 | 0.7 | 1.1 | 2.4 | 2.8 | 0.94 |
| YMR107W | 5.0 | 3.9 | 1.7 | 18.4 | 1.1 | 1.0 | 1.2 | 1.3 | 6.0 | 17.1 | 3.0 | 0.8 | 1.8 | 8.1 | 1.9 | 12.0 | 1.3 | 8.1 | 0.49 |
| YMR180C | 1.2 | 0.9 | 1.0 | 1.3 | 1.0 | 2.4 | 1.7 | 1.8 | 1.8 | 2.2 | 0.7 | 1.2 | 3.1 | 1.7 | 1.0 | 1.8 | 1.4 | 1.7 | 0.57 |
| YMR184W | 1.3 | 5.4 | 1.0 | 1.1 | 0.8 | 0.9 | 1.6 | 1.9 | 2.1 | 0.9 | 1.5 | 1.7 | 2.3 | 1.5 | 1.1 | 1.2 | 1.4 | 2.0 | 0.68 |
| YMR191W | 1.3 | 2.4 | 1.9 | 1.6 | 1.5 | 1.0 | 2.0 | 1.1 | 2.2 | 3.5 | 2.3 | 1.8 | 3.4 | 1.5 | 2.7 | 1.7 | 1.2 | 2.1 | 0.99 |
| YMR295C | 1.7 | 1.0 | 1.2 | 1.6 | 1.4 | 1.9 | 1.3 | 1.0 | 1.0 | 0.8 | 1.2 | 1.1 | 2.3 | 1.3 | 0.9 | 1.9 | 2.1 | 3.4 | 3.43 |
| YMR316W | 3.2 | 2.9 | 1.1 | 1.9 | 1.1 | 0.7 | 2.0 | 1.6 | 2.1 | 3.0 | 1.5 | 1.9 | 2.9 | 1.3 | 0.5 | 1.3 | 6.9 | 6.4 | 1.25 |
| YNL044W | 1.3 | 0.9 | 2.3 | 1.3 | 1.2 | 0.9 | 0.9 | 1.8 | 0.8 | 0.8 | 1.1 | 1.0 | 3.1 | 1.2 | 2.4 | 1.0 | 3.1 | 3.4 | 2.56 |
| YNL074C | 0.9 | 1.3 | 1.8 | 1.2 | 1.3 | 1.7 | 1.1 | 0.6 | 1.5 | 2.0 | 1.3 | 0.9 | 2.3 | 0.6 | 1.0 | 0.7 | 1.1 | 1.3 | 0.68 |
| YNL092W | 1.3 | 1.4 | 2.4 | 1.8 | 0.9 | 1.2 | 1.6 | 0.9 | 2.3 | 1.5 | 0.7 | 1.1 | 2.3 | 1.2 | 2.7 | 1.3 | 1.7 | 1.6 | 0.16 |
| YNL115C | 0.7 | 1.8 | 1.6 | 1.4 | 0.8 | 9.1 | 2.4 | 0.8 | 2.6 | 1.9 | 1.1 | 0.8 | 2.5 | 1.0 | 1.6 | 3.1 | 1.6 | 2.6 | 0.80 |
| YNL156C | 1.1 | 1.2 | 0.9 | 1.3 | 1.6 | 1.3 | 1.9 | 1.2 | 2.4 | 1.9 | 1.4 | 0.9 | 2.0 | 1.4 | 1.8 | 2.1 | 1.8 | 1.9 | 1.85 |
| YNL234W | 1.1 | 1.1 | 0.6 | 1.8 | 1.7 | 1.3 | 1.2 | 1.3 | 2.6 | 3.3 | 1.3 | 1.1 | 2.5 | 1.5 | 1.4 | 2.6 | 1.0 | 1.0 | 0.49 |
| YNL281W | 1.0 | 1.3 | 2.0 | 1.4 | 1.7 | 0.9 | 1.2 | 1.2 | 2.8 | 1.6 | 1.6 | 0.7 | 2.0 | 1.2 | 1.2 | 0.5 | 0.9 | 0.9 | 1.79 |
| YNL305C | 0.9 | 2.2 | 2.7 | 1.4 | 1.3 | 1.1 | 1.7 | 0.7 | 2.9 | 2.1 | 1.8 | 1.1 | 2.6 | 1.3 | 1.6 | 2.5 | 1.7 | 1.6 | 1.35 |
| YNR068C | 1.2 | 1.5 | 2.3 | 2.6 | 1.7 | 0.4 | 1.8 | 1.1 | 6.7 | 14.4 | 1.1 | 1.3 | 3.7 | 1.8 | 1.0 | 1.4 | 1.0 | 1.0 | 0.24 |
| YOL032W | 1.3 | 0.8 | 2.0 | 1.3 | 1.4 | 2.1 | 1.4 | 1.5 | 4.5 | 3.0 | 1.4 | 1.0 | 2.3 | 1.3 | 0.9 | 2.4 | 1.7 | 1.8 | 0.77 |
| YOL036W | 0.8 | 0.9 | 1.3 | 0.8 | 1.2 | 1.2 | 1.0 | 0.8 | 3.1 | 2.8 | 2.2 | 1.0 | 2.5 | 1.0 | 2.0 | 1.0 | 1.1 | 0.9 | 0.48 |

| | | | | | | | | | | | | | | | | | | | |
|---|---|---|---|---|---|---|---|---|---|---|---|---|---|---|---|---|---|---|---|
| YOL047C | 1.4 | 1.1 | 1.1 | 2.5 | 1.4 | 1.5 | 1.3 | 1.1 | 0.6 | 2.3 | 0.9 | 0.8 | 2.0 | 1.2 | 12.3 | 1.3 | 1.5 | 1.0 | 0.19 |
| YOL071W | 1.3 | 2.0 | 1.7 | 2.0 | 0.9 | 1.7 | 2.0 | 1.6 | 4.4 | 2.3 | 4.3 | 1.0 | 2.5 | 2.5 | 0.7 | 4.6 | 1.3 | 2.0 | 1.08 |
| YOL082W | 1.0 | 1.6 | 1.6 | 2.1 | 1.5 | 1.8 | 2.5 | 1.1 | 3.4 | 2.0 | 1.6 | 1.0 | 2.4 | 1.4 | 1.0 | 2.4 | 1.6 | 2.5 | 0.60 |
| YOL083W | 1.1 | 2.3 | 1.2 | 2.8 | 1.4 | 4.5 | 3.1 | 1.3 | 2.7 | 2.3 | 1.3 | 1.2 | 2.3 | 1.4 | 1.0 | 3.8 | 1.4 | 3.2 | 0.41 |
| YOL117W | 1.0 | 1.1 | 0.5 | 1.4 | 1.7 | 2.0 | 1.6 | 1.1 | 1.3 | 4.1 | 1.0 | 0.9 | 2.2 | 1.7 | 1.1 | 2.2 | 1.3 | 1.4 | 0.21 |
| YOL131W | 1.0 | 1.5 | 0.6 | 1.0 | 1.5 | 1.1 | 1.3 | 1.1 | 8.4 | 8.1 | 1.4 | 0.9 | 3.1 | 2.3 | 1.4 | 0.7 | 1.1 | 0.9 | 0.15 |
| YOL162W | 2.0 | 6.9 | 1.7 | 1.4 | 0.9 | 1.2 | 2.0 | 1.6 | 12.0 | 6.8 | 4.3 | 1.4 | 3.8 | 1.4 | 0.6 | 0.9 | 1.2 | 0.9 | 0.26 |
| YOR019W | 2.9 | 1.2 | 0.8 | 4.7 | 1.4 | 1.3 | 2.8 | 1.1 | 3.5 | 7.1 | 1.7 | 1.0 | 2.2 | 1.4 | 0.7 | 2.6 | 1.7 | 2.8 | 0.40 |
| YOR197W | 0.8 | 1.0 | 1.1 | 1.4 | 0.6 | 1.3 | 0.9 | 0.9 | 0.8 | 0.7 | 1.0 | 1.0 | 2.1 | 0.7 | 1.7 | 1.0 | 1.5 | 1.5 | 1.30 |
| YPL087W | 1.0 | 5.1 | 3.0 | 7.3 | 0.6 | 4.3 | 4.2 | 1.0 | 1.0 | 2.2 | 2.5 | 1.8 | 4.4 | 1.2 | 1.3 | 1.8 | 2.1 | 2.7 | 1.49 |
| YPL196W | 1.3 | 1.6 | 1.3 | 1.7 | 1.5 | 2.0 | 2.4 | 1.2 | 10.9 | 2.8 | 1.7 | 0.9 | 2.1 | 1.6 | 0.7 | 4.3 | 1.4 | 2.3 | 0.92 |
| YPL206C | 1.0 | 2.9 | 1.4 | 1.9 | 0.7 | 1.8 | 1.2 | 1.0 | 1.7 | 1.1 | 1.5 | 0.8 | 2.5 | 1.1 | 0.8 | 1.2 | 1.4 | 1.9 | 1.22 |
| YPL222W | 0.9 | 3.5 | 0.8 | 1.3 | 1.2 | 2.2 | 1.1 | 0.7 | 7.4 | 3.4 | 1.2 | 1.3 | 3.0 | 1.1 | 0.9 | 2.2 | 1.1 | 0.9 | 0.26 |
| YPR023C | 1.2 | 1.4 | 1.2 | 0.8 | 1.1 | 1.4 | 1.2 | 0.9 | 4.4 | 2.8 | 1.7 | 0.9 | 2.0 | 0.9 | 0.8 | 1.4 | 1.0 | 1.1 | 0.81 |
| YPR151C | 2.3 | 2.0 | 1.5 | 4.1 | 1.3 | 1.6 | 4.4 | 1.4 | 3.0 | 9.5 | 1.4 | 0.9 | 2.0 | 1.4 | 0.7 | 2.8 | 1.3 | 1.4 | 0.16 |
| YDL222C | 0.9 | 1.1 | 1.5 | 3.9 | 1.0 | 1.4 | 1.0 | 0.7 | 1.9 | 1.0 | 2.6 | 1.0 | 2.2 | 1.3 | 7.1 | 6.5 | 0.9 | 1.3 | 0.50 |
| YEL001C | 1.0 | 1.0 | 1.4 | 0.7 | 0.9 | 1.3 | 1.1 | 1.2 | 2.0 | 1.2 | 1.2 | 1.1 | 0.9 | 1.3 | 3.0 | 1.2 | 0.7 | 1.3 | 2.81 |
| YER106W | 1.3 | 0.7 | 1.3 | 1.2 | 1.6 | 1.1 | 1.0 | 1.2 | 0.4 | 1.8 | 1.0 | 0.9 | 1.0 | 1.5 | 6.8 | 0.9 | 0.8 | 0.8 | 0.25 |
| YIL023C | 1.1 | 1.2 | 2.1 | 1.8 | 1.3 | 0.9 | 1.2 | 0.7 | 3.5 | 1.6 | 1.1 | 0.9 | 1.1 | 0.7 | 5.6 | 1.1 | 1.2 | 1.0 | 0.93 |
| YJR054W | 0.8 | 0.9 | 0.3 | 0.9 | 1.0 | 0.7 | 0.9 | 1.6 | | 0.6 | 0.7 | 0.9 | 0.4 | 1.2 | 3.1 | 0.6 | 0.9 | 0.9 | 0.52 |
| YKL086W | 1.0 | 1.1 | 2.4 | 2.6 | 0.8 | 1.0 | 0.7 | 1.1 | 8.2 | 20.5 | 0.7 | 0.6 | 1.3 | 2.9 | 5.0 | 0.6 | 0.9 | 1.0 | 0.28 |
| YKR091W | 1.5 | 2.3 | 0.9 | 3.5 | 1.6 | 1.0 | 1.3 | 1.3 | 1.0 | 1.6 | 1.1 | 0.9 | 1.0 | 1.9 | 6.3 | 1.6 | 1.6 | 1.3 | 0.45 |
| YLR194C | 2.1 | 2.2 | 1.3 | 3.6 | 1.0 | 0.9 | 1.0 | 1.0 | 0.9 | 1.1 | 1.4 | 1.6 | 4.0 | 1.2 | 3.6 | 0.9 | 1.0 | 0.9 | 0.60 |
| YMR095C | 1.1 | 2.0 | 1.6 | 1.0 | 0.9 | 0.7 | 1.2 | 1.2 | | 1.4 | 2.1 | 0.8 | 3.4 | 2.2 | 41.7 | 1.0 | 1.0 | 1.0 | 0.23 |
| YAL053W | 0.9 | 0.9 | 1.3 | 2.1 | 0.8 | 1.1 | 0.7 | 0.8 | 1.4 | 0.9 | 2.3 | 0.6 | 1.1 | 0.7 | 2.9 | 1.1 | 1.2 | 1.6 | 1.60 |
| YDL072C | 1.3 | 1.1 | 3.5 | 2.1 | 1.4 | 1.5 | 1.8 | 0.8 | 1.1 | 1.5 | 1.4 | 1.2 | 1.6 | 1.8 | 2.6 | 2.6 | 1.6 | 2.8 | 3.25 |
| YDL204W | 1.4 | 1.9 | 4.8 | 3.4 | 1.0 | 5.6 | 3.2 | 1.1 | 2.3 | 2.0 | 3.2 | 0.9 | 3.2 | 2.4 | 3.6 | 8.6 | 1.8 | 2.7 | 0.73 |
| YDR391C | 1.2 | 2.0 | 1.3 | 1.6 | 1.2 | 2.8 | 2.0 | 1.4 | 1.5 | 2.7 | 1.9 | 1.4 | 1.8 | 2.3 | 3.5 | 3.0 | 1.8 | 2.4 | 1.05 |
| YIL024C | 1.4 | 1.3 | 1.1 | 2.1 | 1.3 | 1.0 | 1.3 | 1.1 | 2.2 | 1.8 | 0.6 | 0.7 | 1.0 | 1.5 | 3.1 | 1.0 | 1.1 | 1.0 | 0.35 |
| YIL117C | 2.5 | 1.2 | 1.0 | 3.3 | 0.8 | 1.2 | 1.5 | 1.8 | 2.3 | 3.5 | 1.8 | 1.4 | 1.3 | 2.6 | 7.5 | 1.0 | 2.6 | 2.6 | 1.22 |
| YJL108C | 1.2 | 1.2 | 1.0 | 2.2 | 1.5 | 0.6 | 0.6 | 1.0 | 0.3 | 0.7 | 1.0 | 0.4 | 0.5 | 1.0 | 4.0 | 0.6 | 1.0 | 0.9 | 0.40 |
| YJL149W | 1.2 | 1.8 | 1.5 | 6.8 | 0.8 | 0.7 | 1.3 | 1.0 | 1.5 | 3.8 | 1.5 | 1.1 | 1.9 | 1.1 | 9.0 | 1.4 | 0.8 | 1.2 | 0.28 |

| | | | | | | | | | | | | | | | | | | | |
|---|---|---|---|---|---|---|---|---|---|---|---|---|---|---|---|---|---|---|---|
| YJL186W | 0.6 | 0.9 | 1.2 | 0.8 | 0.7 | 0.7 | 0.6 | 0.6 | 0.2 | 0.5 | 0.6 | 0.6 | 0.4 | 0.8 | 2.5 | 1.0 | 0.7 | 0.8 | 1.07 |
| YNL190W | 1.0 | 1.4 | 2.6 | 0.9 | 1.0 | 0.5 | 0.8 | 0.8 | 1.1 | 1.1 | 1.0 | 1.0 | 1.3 | 0.6 | 2.6 | 1.3 | 0.7 | 0.8 | 4.72 |
| YNL208W | 1.5 | 3.2 | 2.9 | 2.1 | 1.2 | 1.4 | 0.9 | 1.5 | 2.3 | 3.1 | 2.5 | 0.8 | 1.7 | 1.0 | 2.6 | 0.9 | 1.0 | 1.0 | 1.55 |
| YNL300W | 0.7 | 0.3 | 2.9 | 1.5 | 0.9 | 0.4 | 0.4 | 0.9 | | 0.2 | 0.9 | 0.8 | 0.7 | 0.8 | 3.3 | 1.0 | 1.0 | 0.8 | 0.39 |
| YNR064C | 0.9 | 0.6 | 0.9 | 1.8 | 1.5 | 2.1 | 0.8 | 0.9 | | 2.1 | 0.7 | 0.7 | 1.6 | 0.8 | 6.1 | 1.4 | 1.1 | 1.9 | 2.17 |
| YOR248W | 2.4 | 0.7 | 2.9 | 1.3 | 0.7 | 0.2 | 0.3 | 0.7 | 0.1 | 0.4 | 2.3 | 0.5 | 0.4 | 0.5 | 8.8 | 0.3 | 1.0 | 0.8 | 2.47 |
| YPL052W | 1.8 | 1.7 | 0.4 | 1.0 | 1.7 | 1.9 | 1.4 | 1.7 | 2.0 | 4.8 | 1.2 | 1.1 | 1.6 | 3.2 | 3.3 | 1.1 | 1.3 | 1.5 | 0.88 |
| YPR079W | 1.0 | 1.8 | 1.3 | 1.5 | 0.9 | 1.0 | 2.3 | 1.1 | 1.7 | 2.8 | 0.8 | 1.0 | 1.3 | 1.3 | 2.9 | 1.9 | 1.4 | 1.5 | 0.42 |
| YAR028W | 1.2 | 1.0 | 0.7 | 1.2 | 0.9 | 3.3 | 1.8 | 1.3 | 1.0 | 0.9 | 0.9 | 0.8 | 1.4 | 1.2 | 0.9 | 1.5 | 1.9 | 2.1 | 1.40 |
| YDR031W | 1.5 | 1.1 | 1.7 | 1.5 | 1.2 | 3.6 | 1.9 | 1.4 | 1.1 | 2.7 | 1.2 | 0.9 | 1.3 | 2.5 | 0.8 | 2.4 | 1.3 | 2.5 | 1.23 |
| YDR486C | 1.1 | 1.5 | 1.2 | 0.9 | 1.2 | 2.7 | 1.8 | 1.8 | 2.5 | 5.8 | 1.4 | 1.3 | 1.7 | 2.4 | 1.1 | 2.6 | 1.1 | 1.7 | 1.18 |
| YER038C | 1.2 | 1.4 | 0.6 | 1.2 | 1.4 | 2.8 | 1.5 | 1.1 | 0.7 | 2.5 | 0.6 | 0.9 | 2.2 | 1.3 | 0.8 | 1.6 | 1.2 | 1.4 | 0.51 |
| YGL136C | 1.1 | 1.0 | 1.2 | 1.1 | 1.0 | 3.1 | 0.7 | 1.1 | 1.3 | 1.0 | 1.4 | 1.1 | 1.1 | 2.3 | 0.9 | 1.4 | 1.3 | 1.1 | 0.64 |
| YGR146C | 1.6 | 2.0 | 2.0 | 2.0 | 0.9 | 4.1 | 2.7 | 1.4 | 0.8 | 3.8 | 0.9 | 1.4 | 1.7 | 1.3 | 1.4 | 1.8 | 1.3 | 0.9 | 0.75 |
| YJL020C | 0.7 | 1.2 | 0.8 | 0.8 | 1.0 | 2.1 | 1.3 | 0.6 | 1.3 | 1.0 | 0.6 | 0.7 | 1.6 | 1.2 | 1.4 | 0.8 | 2.0 | 1.3 | 0.95 |
| YLR031W | 1.3 | 1.0 | 0.7 | 1.3 | 1.3 | 3.0 | 1.5 | 0.9 | 0.9 | 1.4 | 0.7 | 1.0 | 1.6 | 1.5 | 0.7 | 1.6 | 1.2 | 1.2 | 0.30 |
| YLR205C | 1.2 | 5.1 | 0.7 | 1.3 | 1.3 | 4.4 | 9.0 | 1.7 | 1.5 | 5.7 | 0.4 | 1.3 | 1.4 | 2.4 | 2.5 | 2.3 | 2.0 | 1.4 | 0.29 |
| YMR140W | 1.0 | 0.9 | 1.2 | 0.7 | 1.1 | 17.1 | 4.3 | 0.8 | 1.8 | 2.3 | 0.7 | 0.9 | 1.6 | 1.2 | 1.4 | 2.8 | 1.4 | 2.0 | 0.54 |
| YMR195W | 1.7 | 3.1 | 1.2 | 2.2 | 1.1 | 2.6 | 1.1 | 1.0 | 0.2 | 0.7 | 1.1 | 0.9 | 0.7 | 1.3 | 0.6 | 2.5 | 1.6 | 2.2 | 1.28 |
| YOR215C | 1.5 | 1.3 | 0.7 | 1.8 | 1.8 | 2.6 | 2.6 | 1.7 | 2.1 | 1.3 | 1.6 | 1.1 | 1.3 | 1.7 | 0.8 | 3.3 | 1.3 | 2.9 | 2.08 |
| YOR382W | 0.7 | 7.6 | 3.3 | 0.5 | 1.2 | 13.1 | 28.4 | 2.0 | 1.1 | 12.8 | 2.5 | 2.8 | 1.7 | 0.4 | 2.1 | 4.3 | 1.9 | 1.9 | 0.79 |
| YPL054W | 4.2 | 5.3 | 4.9 | 3.1 | 1.1 | 2.3 | 1.8 | 1.3 | 2.5 | 9.2 | 1.9 | 1.3 | 1.5 | 1.4 | 1.0 | 1.3 | 1.5 | 1.4 | 0.25 |
| YPR127W | 1.1 | 1.4 | 1.0 | 1.6 | 1.1 | 3.2 | 1.7 | 1.2 | 1.9 | 2.4 | 1.6 | 0.9 | 1.3 | 1.4 | 0.4 | 2.4 | 1.3 | 1.2 | 0.65 |
| YAR027W | 1.8 | 1.0 | 1.3 | 1.4 | 1.2 | 2.3 | 2.2 | 1.3 | 2.3 | 1.7 | 1.7 | 1.2 | 2.6 | 1.3 | 1.2 | 2.8 | 2.0 | 4.7 | 2.15 |
| YBL057C | 1.1 | 0.9 | 0.6 | 1.0 | 1.3 | 2.2 | 1.1 | 1.4 | 1.6 | 1.2 | 0.9 | 1.0 | 1.0 | 1.6 | 1.0 | 1.1 | 1.2 | 1.2 | 1.88 |
| YBR116C | 0.9 | 4.8 | 2.4 | 2.3 | 1.1 | 2.8 | 1.5 | 1.1 | 9.0 | 8.7 | 3.1 | 1.1 | 2.0 | 2.7 | 1.7 | 9.9 | 0.9 | 1.0 | 0.45 |
| YBR147W | 2.2 | 1.1 | 1.5 | 3.2 | 1.2 | 2.2 | 1.2 | 1.9 | 0.9 | 3.6 | 4.0 | 1.3 | 1.2 | 1.1 | 2.1 | 6.0 | 1.3 | 1.9 | 0.63 |
| YBR168W | 0.9 | 0.8 | 1.0 | 1.3 | 1.6 | 3.5 | 1.6 | 1.0 | 1.1 | 1.7 | 0.8 | 0.9 | 1.9 | 1.8 | 0.9 | 1.0 | 1.1 | 1.5 | 0.56 |
| YBR246W | 1.0 | 0.8 | 1.7 | 0.6 | 1.1 | 3.1 | 1.1 | 0.9 | 0.5 | 0.9 | 0.8 | 0.9 | 1.1 | 1.3 | 0.9 | 1.8 | 1.4 | 1.7 | 0.82 |
| YBR273C | 0.8 | 0.6 | 0.6 | 1.3 | 1.9 | 2.2 | 1.4 | 1.4 | 2.2 | 2.0 | 0.6 | 0.8 | 2.1 | 0.9 | 0.8 | 0.9 | 1.4 | 1.4 | 1.33 |
| YDR003W | 2.0 | 2.4 | 1.6 | 1.5 | 1.8 | 1.8 | 1.8 | 1.1 | 6.6 | 3.6 | 1.8 | 0.8 | 1.5 | 1.2 | 1.0 | 1.8 | 1.4 | 2.0 | 0.95 |
| YDR340W | 1.2 | 1.4 | 0.7 | 0.8 | 1.1 | 1.9 | 2.3 | 1.3 | 1.4 | 2.4 | 1.4 | 0.9 | 1.3 | 2.0 | 1.4 | 1.0 | 1.1 | 1.6 | 1.90 |

| | | | | | | | | | | | | | | | | | | | |
|---|---|---|---|---|---|---|---|---|---|---|---|---|---|---|---|---|---|---|---|
| YDR357C | 1.3 | 1.5 | 0.3 | 1.3 | 2.0 | 1.9 | 2.2 | 1.7 | 1.0 | 0.9 | 1.1 | 0.6 | 1.0 | 1.4 | 0.5 | 2.2 | 1.3 | 1.7 | 0.73 |
| YDR396W | 0.7 | 1.3 | 0.6 | 0.7 | 1.4 | 2.0 | 1.5 | 1.4 | 0.3 | 1.2 | 1.0 | 1.2 | 0.6 | 1.4 | 0.9 | 1.3 | 1.1 | 1.3 | 0.63 |
| YDR434W | 0.8 | 0.8 | 1.5 | 1.2 | 1.0 | 2.2 | 1.4 | 0.9 | 0.8 | 1.2 | 0.8 | 0.9 | 1.2 | 0.9 | 1.3 | 0.8 | 1.2 | 1.5 | 1.53 |
| YDR482C | 1.1 | 0.9 | 0.7 | 1.1 | 1.5 | 1.7 | 1.2 | 1.0 | 0.9 | 0.8 | 0.8 | 0.8 | 1.1 | 1.0 | 0.5 | 1.7 | 1.6 | 1.2 | 0.85 |
| YDR520C | 0.9 | 2.1 | 0.9 | 0.6 | 1.3 | 2.3 | 1.2 | 1.0 | 0.8 | 0.7 | 0.6 | 1.2 | 1.4 | 1.0 | 1.3 | 0.9 | 1.1 | 1.0 | 0.46 |
| YDR534C | 0.8 | 4.8 | 1.8 | 0.9 | 1.0 | 2.1 | 9.7 | 1.0 | 0.6 | 7.5 | 1.7 | 1.5 | 1.8 | 0.7 | 4.2 | 1.0 | 0.6 | 1.0 | 0.34 |
| YDR539W | 0.7 | 2.0 | 1.1 | 1.0 | 1.8 | 2.1 | 2.3 | 1.3 | 0.7 | 1.2 | 1.3 | 1.2 | 1.3 | 1.1 | 1.5 | 1.6 | 1.2 | 1.4 | 0.61 |
| YER044C | 1.2 | 1.0 | 2.1 | 1.3 | 1.8 | 3.0 | 1.8 | 0.9 | 0.3 | 0.7 | 1.7 | 0.7 | 0.9 | 1.4 | 0.7 | 3.0 | 1.3 | 2.1 | 1.62 |
| YER067W | 4.0 | 1.9 | 5.2 | 6.0 | 1.1 | 2.1 | 4.9 | 2.1 | 1.8 | 3.8 | 2.2 | 1.1 | 1.2 | 1.7 | 0.5 | 3.5 | 1.5 | 3.0 | 1.57 |
| YER080W | 0.8 | 0.8 | 1.5 | 0.5 | 1.1 | 5.3 | 3.0 | 1.4 | 4.0 | 2.7 | 1.2 | 0.9 | 1.8 | 1.1 | 0.8 | 1.9 | 1.2 | 1.3 | 0.55 |
| YGL113W | 0.7 | 0.9 | 0.9 | 0.4 | 1.5 | 2.1 | 0.9 | 0.9 | 0.7 | 0.8 | 0.7 | 0.8 | 1.2 | 0.9 | 1.0 | 1.0 | 1.5 | 0.9 | 0.35 |
| YGL242C | 0.8 | 0.9 | 0.6 | 0.8 | 1.4 | 1.9 | 2.0 | 1.5 | 0.9 | 1.3 | 1.4 | 0.9 | 1.6 | 1.6 | 1.2 | 1.1 | 1.5 | 1.4 | 1.32 |
| YGR052W | 1.6 | 0.8 | 3.9 | 4.6 | 1.4 | 2.6 | 0.8 | 1.1 | 0.6 | 1.7 | 0.6 | 0.7 | 1.5 | 1.3 | 0.6 | 7.6 | 1.8 | 4.0 | 0.48 |
| YGR106C | 1.2 | 0.8 | 1.9 | 1.3 | 0.9 | 2.0 | 1.1 | 1.0 | 0.7 | 1.0 | 1.2 | 1.1 | 1.0 | 1.2 | 0.9 | 1.1 | 1.0 | 1.7 | 4.60 |
| YGR111W | 1.1 | 2.0 | 1.0 | 1.7 | 1.0 | 2.4 | 1.8 | 1.8 | 3.3 | 2.5 | 1.6 | 0.9 | 2.2 | 1.5 | 0.9 | 2.2 | 1.1 | 2.1 | 0.79 |
| YHL023C | 0.7 | 0.6 | 1.3 | 0.7 | 1.1 | 1.8 | 1.0 | 1.0 | 1.3 | 1.1 | 1.0 | 1.1 | 1.9 | 1.0 | 1.0 | 1.1 | 0.9 | 0.9 | 0.32 |
| YHL048W | 1.3 | 1.6 | 2.8 | 1.3 | 1.1 | 3.7 | 2.3 | 1.2 | 0.8 | 1.2 | 0.9 | 1.1 | 1.7 | 1.0 | 2.3 | 2.5 | 1.9 | 3.2 | 4.10 |
| YIL007C | 1.2 | 1.0 | 1.1 | 1.0 | 1.4 | 2.0 | 1.3 | 1.2 | 1.1 | 1.4 | 1.2 | 1.0 | 1.1 | 1.7 | 1.1 | 1.9 | 1.2 | 1.5 | 0.69 |
| YIR016W | 1.1 | 1.1 | 1.4 | 1.6 | 1.2 | 2.3 | 1.7 | 0.8 | 1.4 | 1.3 | 1.4 | 1.3 | 1.6 | 1.3 | 1.0 | 2.7 | 1.9 | 2.0 | 0.67 |
| YIR043C | 1.2 | 1.6 | 2.2 | 1.8 | 1.3 | 2.4 | 2.2 | 1.0 | 0.8 | 1.3 | 1.4 | 1.3 | 2.0 | 1.4 | 2.5 | 2.6 | 1.4 | 2.5 | 3.90 |
| YJL012C | 0.7 | 0.6 | 1.6 | 0.7 | 1.5 | 1.9 | 0.5 | 1.0 | 0.6 | 1.0 | 0.7 | 1.2 | 1.3 | 1.0 | 1.1 | 0.3 | 0.9 | 0.8 | 1.77 |
| YJL083W | 0.9 | 1.3 | 0.8 | 0.9 | 1.1 | 2.8 | 0.7 | 0.7 | 0.0 | 0.4 | 0.3 | 0.9 | 0.8 | 1.1 | 0.9 | 0.6 | 1.4 | 1.0 | 0.34 |
| YJL131C | 0.9 | 0.8 | 0.5 | 1.1 | 1.7 | 2.2 | 1.9 | 1.2 | 0.5 | 1.0 | 0.3 | 0.8 | 1.8 | 1.5 | 1.0 | 2.2 | 1.3 | 1.2 | 0.54 |
| YJR061W | 1.0 | 2.1 | 1.0 | 0.7 | 1.1 | 2.0 | 0.9 | 1.9 | 1.6 | 1.4 | 0.8 | 0.9 | 0.8 | 1.5 | 1.6 | 1.1 | 1.3 | 1.1 | 0.34 |
| YJR161C | 1.2 | 2.0 | 2.8 | 1.8 | 0.8 | 2.2 | 2.6 | 1.0 | 0.5 | 1.2 | 1.7 | 1.2 | 2.1 | 1.0 | 2.9 | 2.6 | 2.1 | 3.4 | 3.62 |
| YKL175W | 0.7 | 1.1 | 2.1 | 0.9 | 1.4 | 2.2 | 1.3 | 0.9 | 1.1 | 1.9 | 0.8 | 0.9 | 1.7 | 1.0 | 1.6 | 1.1 | 0.9 | 1.0 | 1.71 |
| YKR070W | 1.3 | 0.8 | 1.4 | 1.4 | 1.1 | 2.2 | 1.4 | 1.3 | 3.4 | 2.0 | 1.6 | 0.9 | 1.7 | 1.2 | 0.8 | 2.1 | 1.0 | 1.4 | 0.99 |
| YLL023C | 0.7 | 1.3 | 5.9 | 0.9 | 1.0 | 2.6 | 1.5 | 0.5 | 1.1 | 1.5 | 1.6 | 1.2 | 1.6 | 1.1 | 2.5 | 2.8 | 1.6 | 1.4 | 1.66 |
| YLR023C | 1.3 | 1.5 | 0.9 | 1.2 | 1.1 | 2.6 | 1.2 | 0.9 | 0.7 | 1.7 | 1.1 | 0.6 | 1.0 | 1.1 | 0.9 | 0.8 | 0.7 | 0.8 | 0.92 |
| YLR225C | 0.8 | 0.7 | 0.4 | 0.7 | 1.6 | 2.4 | 1.6 | 1.2 | 1.0 | 1.2 | 0.8 | 1.2 | 1.3 | 1.4 | 1.6 | 0.8 | 1.2 | 1.2 | 1.41 |
| YLR241W | 0.9 | 1.7 | 1.6 | 2.1 | 0.8 | 1.7 | 0.9 | 1.0 | 1.8 | 1.5 | 0.7 | 0.9 | 2.0 | 0.9 | 2.4 | 1.1 | 1.4 | 1.6 | 1.03 |
| YLR252W | 1.3 | 1.3 | 4.6 | 3.0 | 1.0 | 2.8 | 1.6 | 1.1 | 3.1 | 1.4 | 2.5 | 0.7 | 1.3 | 1.3 | 0.9 | 2.7 | 2.2 | 2.9 | 0.96 |

| | | | | | | | | | | | | | | | | | | | |
|---|---|---|---|---|---|---|---|---|---|---|---|---|---|---|---|---|---|---|---|
| YLR270W | 1.2 | 1.1 | 2.3 | 1.2 | 1.1 | 3.5 | 3.9 | 1.1 | 3.3 | 2.1 | 2.8 | 1.1 | 2.1 | 1.3 | 1.4 | 2.6 | 1.4 | 2.3 | 1.00 |
| YML030W | 1.3 | 0.8 | 0.8 | 1.4 | 1.9 | 3.0 | 2.1 | 1.4 | 0.8 | 0.8 | 1.2 | 0.7 | 1.0 | 1.3 | 0.6 | 2.7 | 1.2 | 1.3 | 1.35 |
| YMR148W | 1.0 | 1.0 | 1.6 | 0.9 | 1.2 | 2.9 | 0.8 | 0.6 | 1.0 | 0.8 | 1.0 | 0.5 | 1.0 | 1.0 | 0.7 | 1.4 | 1.1 | 0.9 | 0.28 |
| YMR181C | 1.3 | 1.6 | 1.8 | 1.1 | 1.2 | 2.1 | 2.0 | 0.9 | 1.9 | 1.4 | 1.7 | 1.4 | 2.8 | 1.0 | 1.4 | 2.8 | 1.1 | 2.0 | 0.79 |
| YMR298W | 1.3 | 1.3 | 2.9 | 0.8 | 1.5 | 2.3 | 1.3 | 1.1 | 2.4 | 2.6 | 1.7 | 0.9 | 1.0 | 2.0 | 2.0 | 1.7 | 1.4 | 1.6 | 1.37 |
| YNL011C | 1.1 | 1.5 | 0.8 | 0.9 | 0.9 | 2.9 | 2.9 | 1.5 | 3.8 | 1.6 | 1.4 | 0.6 | 1.2 | 1.2 | 0.8 | 3.1 | 1.2 | 1.8 | 0.68 |
| YOL129W | 1.0 | 1.1 | 2.5 | 1.2 | 1.6 | 2.2 | 1.6 | 0.7 | 1.2 | 1.2 | 0.7 | 0.8 | 1.8 | 1.5 | 1.0 | 1.9 | 2.6 | 2.4 | 2.61 |
| YOR042W | 0.6 | 1.4 | 0.7 | 0.7 | 1.3 | 2.1 | 1.1 | 0.9 | 2.1 | 1.2 | 0.9 | 0.8 | 1.3 | 1.2 | 1.1 | 1.1 | 1.4 | 1.1 | 0.54 |
| YOR052C | 1.5 | 1.3 | 0.5 | 2.4 | 2.3 | 2.4 | 1.4 | 1.4 | 1.4 | 1.3 | 0.8 | 1.0 | 2.6 | 1.6 | 1.2 | 1.3 | 1.2 | 2.3 | 2.64 |
| YOR137C | 1.1 | 1.0 | 0.9 | 1.2 | 1.3 | 2.1 | 1.6 | 0.6 | 0.7 | 0.7 | 0.9 | 0.8 | 1.6 | 0.9 | 1.4 | 1.0 | 1.6 | 1.5 | 0.78 |
| YPL156C | 1.4 | 1.4 | 0.9 | 2.9 | 1.2 | 2.4 | 3.2 | 1.2 | 0.5 | 2.8 | 0.9 | 1.5 | 1.7 | 1.6 | 0.8 | 2.4 | 1.6 | 1.9 | 0.70 |
| YPL186C | 1.6 | 1.7 | 1.4 | 4.5 | 1.3 | 3.2 | 3.2 | 1.9 | 2.4 | 1.5 | 3.7 | 1.0 | 1.4 | 2.0 | 0.7 | 6.2 | 1.2 | 2.9 | 0.60 |
| YPL216W | 0.7 | 0.8 | 1.3 | 0.9 | 0.9 | 1.8 | 0.8 | 0.9 | 1.8 | 1.9 | 0.9 | 0.9 | 1.1 | 1.2 | 1.0 | 1.1 | 1.1 | 0.9 | 0.47 |
| YPR098C | 1.6 | 2.6 | 1.7 | 1.9 | 2.0 | 3.0 | 2.9 | 1.1 | 1.4 | 1.7 | 1.9 | 0.9 | 1.2 | 1.8 | 0.9 | 2.9 | 2.2 | 2.2 | 1.10 |
| YFL062W | 1.3 | 2.7 | 1.7 | 2.4 | 1.6 | 1.8 | 3.5 | 1.1 | 0.6 | 1.2 | 2.2 | 1.1 | 1.6 | 1.1 | 1.6 | 2.7 | 2.1 | 3.0 | 3.43 |
| YJL217W | 0.6 | 0.7 | 3.1 | 2.5 | 0.7 | 1.7 | 3.2 | 4.5 | 0.7 | 1.7 | 4.6 | 0.7 | 0.5 | 0.7 | 0.8 | 5.1 | 1.4 | 2.5 | 2.19 |
| YLR126C | 0.9 | 1.5 | 0.4 | 0.8 | 1.3 | 1.7 | 3.4 | 1.8 | 1.3 | 3.4 | 1.3 | 1.4 | 1.6 | 1.4 | 1.4 | 1.4 | 1.3 | 1.2 | 0.61 |
| YNL249C | 1.3 | 1.7 | 0.3 | 1.5 | 1.2 | 0.7 | 2.3 | 1.7 | 2.6 | 1.7 | 1.4 | 1.0 | 1.4 | 1.3 | 0.9 | 1.3 | 1.1 | 1.1 | 0.56 |
| YNL336W | 1.2 | 1.0 | 3.4 | 1.3 | 1.3 | 1.4 | 2.5 | 0.9 | 0.7 | 1.9 | 0.8 | 1.1 | 1.3 | 1.1 | 1.9 | 2.8 | 2.0 | 3.1 | 3.74 |
| YBR074W | 0.7 | 1.3 | 0.9 | 0.8 | 1.3 | 1.5 | 2.2 | 0.7 | 0.4 | 0.5 | 0.5 | 1.0 | 1.2 | 1.5 | 0.8 | 1.3 | 1.4 | 1.0 | 0.52 |
| YDL248W | 1.1 | 1.7 | 2.8 | 1.7 | 1.5 | 2.0 | 2.5 | 1.1 | 0.9 | 1.4 | 1.4 | 1.2 | 2.0 | 0.9 | 1.8 | 1.9 | 1.6 | 2.4 | 4.35 |
| YDR105C | 0.7 | 1.1 | 2.3 | 1.0 | 0.8 | 1.4 | 2.1 | 0.8 | 2.3 | 1.7 | 1.4 | 0.9 | 1.8 | 1.1 | 0.9 | 1.4 | 1.1 | 1.4 | 1.09 |
| YEL075C | 1.1 | 1.1 | 0.7 | 1.0 | 1.1 | 1.4 | 1.9 | 0.7 | 0.6 | 0.7 | 0.7 | 1.1 | 1.7 | 1.9 | 0.8 | 1.2 | 1.4 | 1.1 | 1.19 |
| YER046W | 1.3 | 1.9 | 0.5 | 1.1 | 1.6 | 1.8 | 2.6 | 1.5 | 1.5 | 1.7 | 1.1 | 1.0 | 1.0 | 1.4 | 0.7 | 2.7 | 2.0 | 1.9 | 0.53 |
| YER050C | 1.3 | 0.9 | 0.5 | 2.3 | 2.2 | 1.0 | 2.2 | 1.5 | 1.4 | 1.6 | 1.4 | 0.7 | 1.0 | 2.1 | 1.0 | 2.1 | 1.0 | 1.8 | 1.40 |
| YGL250W | 1.1 | 1.4 | 1.9 | 1.8 | 1.6 | 1.4 | 2.0 | 1.1 | 1.5 | 1.2 | 2.4 | 0.7 | 1.2 | 1.0 | 1.1 | 2.3 | 1.2 | 1.9 | 0.45 |
| YGR042W | 1.5 | 1.1 | 1.2 | 2.0 | 1.5 | 2.2 | 2.2 | 1.8 | 1.3 | 3.2 | 1.6 | 1.1 | 1.6 | 2.2 | 1.4 | 2.6 | 1.3 | 1.9 | 0.79 |
| YGR053C | 1.3 | 0.7 | 0.6 | 2.7 | 2.1 | 1.9 | 3.4 | 1.5 | 4.5 | 2.2 | 1.6 | 0.7 | 1.2 | 1.7 | 0.7 | 2.7 | 1.5 | 2.4 | 0.39 |
| YGR066C | 1.6 | 2.5 | 1.3 | 2.5 | 1.2 | 1.7 | 2.1 | 1.3 | 2.3 | 5.0 | 2.6 | 0.8 | 1.2 | 1.9 | 0.7 | 2.6 | 1.0 | 1.1 | 0.17 |
| YGR247W | 0.8 | 1.1 | 0.8 | 1.0 | 1.0 | 0.9 | 1.8 | 0.9 | 1.1 | 1.1 | 1.1 | 0.6 | 0.8 | 1.0 | 0.4 | 1.2 | 1.0 | 1.0 | 0.45 |
| YGR295C | 1.0 | 1.6 | 2.8 | 2.0 | 0.9 | 1.9 | 2.8 | 1.1 | 0.5 | 1.2 | 1.1 | 1.1 | 1.9 | 1.0 | 2.4 | 2.4 | 1.6 | 2.8 | 4.78 |
| YHL044W | 0.9 | 1.3 | 1.5 | 1.4 | 0.7 | 1.9 | 2.0 | 1.4 | 1.1 | 1.9 | 0.7 | 1.2 | 1.6 | 1.7 | 1.1 | 3.9 | 0.8 | 1.2 | 0.45 |

| | | | | | | | | | | | | | | | | | | | |
|---|---|---|---|---|---|---|---|---|---|---|---|---|---|---|---|---|---|---|---|
| YHR145C | 1.5 | 1.6 | 0.6 | 0.9 | 1.4 | 1.3 | 2.1 | 1.6 | 0.8 | 2.4 | 2.0 | 0.9 | 1.5 | 1.8 | 1.3 | 1.1 | 1.3 | 1.1 | 1.70 |
| YIL058W | 2.0 | 0.9 | 1.4 | 0.5 | 1.3 | 1.4 | 2.2 | 0.8 | 1.1 | 1.0 | 0.0 | 0.6 | 1.0 | 2.0 | 0.9 | 2.1 | 1.4 | 1.3 | 0.39 |
| YIL065C | 1.0 | 1.3 | 0.8 | 1.6 | 1.9 | 1.6 | 2.0 | 1.4 | 3.8 | 2.1 | 1.1 | 0.9 | 1.8 | 1.7 | 0.8 | 2.4 | 2.1 | 1.4 | 0.94 |
| YIL083C | 0.8 | 1.0 | 0.9 | 0.8 | 1.2 | 1.4 | 2.2 | 1.0 | 1.0 | 1.1 | 1.2 | 1.0 | 1.0 | 1.0 | 0.9 | 1.4 | 0.9 | 1.1 | 0.76 |
| YJL185C | 0.9 | 1.0 | 1.9 | 1.6 | 1.0 | 1.4 | 2.3 | 1.0 | 0.9 | 1.2 | 1.2 | 0.8 | 1.0 | 1.4 | 1.6 | 2.3 | 1.0 | 1.1 | 0.30 |
| YJL213W | 0.8 | 1.1 | 0.2 | 1.2 | 1.5 | 2.6 | 2.1 | 1.2 | 1.3 | 1.5 | 0.7 | 0.7 | 1.0 | 0.8 | 2.8 | 3.6 | 2.1 | 2.8 | 0.33 |
| YKR020W | 1.1 | 1.8 | 0.9 | 1.6 | 1.0 | 4.5 | 2.2 | 1.5 | 1.9 | 2.8 | 1.0 | 0.8 | 1.7 | 1.4 | 1.2 | 2.5 | 1.3 | 2.0 | 0.44 |
| YLL025W | 0.8 | 1.3 | 1.8 | 0.8 | 1.1 | 1.5 | 1.9 | 1.0 | 1.5 | 2.0 | 1.4 | 1.0 | 1.3 | 1.0 | 1.2 | 1.7 | 1.0 | 0.9 | 0.40 |
| YLR108C | 3.2 | 1.8 | 4.7 | 1.8 | 1.9 | 1.3 | 2.6 | 2.0 | 7.4 | 10.4 | 2.6 | 0.8 | 1.9 | 3.4 | 1.2 | 2.3 | 1.0 | 1.5 | 0.39 |
| YLR290C | 1.3 | 1.2 | 0.8 | 1.9 | 2.1 | 1.7 | 2.3 | 1.1 | 1.2 | 1.1 | 1.5 | 0.8 | 1.0 | 1.3 | 0.8 | 3.2 | 1.3 | 2.4 | 1.49 |
| YML068W | 1.0 | 3.7 | 1.0 | 1.2 | 1.0 | 0.9 | 4.1 | 1.3 | 1.3 | 1.4 | 0.6 | 0.4 | 0.8 | 1.1 | 1.1 | 1.9 | 1.0 | 1.3 | 0.41 |
| YMR178W | 1.6 | 1.0 | 0.8 | 0.9 | 1.1 | 1.6 | 2.1 | 1.6 | 1.6 | 1.6 | 1.0 | 0.9 | 1.0 | 1.2 | 0.9 | 2.1 | 1.1 | 2.3 | 1.36 |
| YNL122C | 1.2 | 0.9 | 1.2 | 1.3 | 1.6 | 1.8 | 2.1 | 1.4 | 1.0 | 1.2 | 1.3 | 0.7 | 1.0 | 1.2 | 0.6 | 1.8 | 1.0 | 1.4 | 1.07 |
| YNL285W | 1.1 | 1.2 | 0.6 | 1.1 | 1.4 | 0.8 | 2.3 | 1.2 | 1.4 | 1.7 | 1.2 | 0.6 | 1.1 | 1.8 | 0.9 | 1.3 | 1.2 | 1.3 | 0.43 |
| YNL293W | 1.3 | 0.8 | 0.8 | 1.8 | 1.3 | 0.9 | 2.1 | 1.0 | 1.6 | 2.4 | 0.8 | 0.7 | 1.4 | 1.1 | 1.2 | 2.1 | 1.0 | 1.4 | 0.58 |
| YNR061C | 0.8 | 1.1 | 2.5 | 0.8 | 1.0 | 1.3 | 2.6 | 0.8 | 0.8 | 0.9 | 1.2 | 0.9 | 1.1 | 1.3 | 0.9 | 1.1 | 1.6 | 1.1 | 1.52 |
| YOR220W | 1.5 | 1.4 | 1.7 | 2.4 | 1.3 | 1.2 | 2.1 | 0.9 | 1.8 | 2.5 | 1.2 | 1.2 | 2.9 | 0.8 | 0.7 | 2.0 | 3.5 | 3.4 | 1.44 |
| YPR077C | 1.4 | 1.4 | 1.2 | 2.7 | 1.4 | 1.2 | 2.0 | 1.5 | 0.6 | 2.5 | 0.8 | 1.0 | 0.8 | 1.6 | 2.9 | 0.6 | 1.4 | 1.2 | 0.24 |
| YPR147C | 0.9 | 0.8 | 1.1 | 1.4 | 1.6 | 2.1 | 1.8 | 1.1 | 1.2 | 2.4 | 1.3 | 0.9 | 1.6 | 1.1 | 1.4 | 1.9 | 1.1 | 1.3 | 1.18 |
| YEL041W | 1.4 | 1.4 | 0.8 | 2.5 | 0.8 | 1.2 | 1.9 | 1.9 | 2.4 | 3.6 | 3.5 | 0.9 | 1.9 | 2.0 | 1.4 | 1.9 | 1.5 | 1.9 | 0.39 |
| YKL187C | 0.9 | 1.0 | 1.4 | 1.5 | 0.8 | 0.8 | 0.7 | 1.1 | 5.2 | 8.6 | 3.3 | 0.9 | 1.4 | 1.6 | 1.2 | 4.8 | 0.7 | 1.2 | 0.36 |
| YBR285W | 1.6 | 1.6 | 0.9 | 6.2 | 1.3 | 2.2 | 1.5 | 1.0 | 4.0 | 3.1 | 4.6 | 0.5 | 0.9 | 1.6 | 0.4 | 7.8 | 2.5 | 2.3 | 0.27 |
| YBR292C | 0.8 | 4.3 | 1.2 | 0.6 | 1.0 | 0.8 | 0.7 | 0.8 | 2.1 | 1.3 | 1.8 | 0.4 | 0.7 | 1.1 | 1.0 | 1.0 | 0.9 | 0.9 | 0.30 |
| YDL123W | 1.0 | 1.3 | 3.7 | 1.4 | 0.8 | 0.7 | 1.0 | 0.8 | 2.0 | 2.0 | 4.6 | 1.1 | 1.5 | 0.9 | 3.3 | 1.5 | 1.1 | 1.0 | 0.52 |
| YDR056C | 1.4 | 1.3 | 2.0 | 2.0 | 0.9 | 1.0 | 1.4 | 1.4 | 1.3 | 2.3 | 2.3 | 1.2 | 1.0 | 2.3 | 1.1 | 2.5 | 1.2 | 2.0 | 1.83 |
| YDR132C | 3.7 | 1.6 | 1.4 | 2.1 | 1.2 | 1.9 | 1.7 | 2.3 | 5.9 | 7.3 | 3.3 | 0.9 | 1.5 | 1.4 | 1.2 | 1.2 | 1.0 | 1.3 | 0.40 |
| YDR154C | 1.0 | 2.1 | 2.5 | 1.7 | 0.8 | 1.5 | 0.9 | 1.1 | 1.5 | 1.2 | 3.2 | 1.0 | 2.6 | 1.0 | 1.8 | 1.3 | 1.5 | 2.0 | 2.69 |
| YDR295C | 0.8 | 1.0 | 0.6 | 1.0 | 0.9 | 1.0 | 0.8 | 1.3 | 2.6 | 1.3 | 1.9 | 0.7 | 1.8 | 1.1 | 1.4 | 0.6 | 0.8 | 1.0 | 0.49 |
| YDR494W | 0.9 | 0.9 | 0.2 | 1.3 | 1.3 | 1.4 | 1.7 | 1.2 | 1.2 | 0.9 | 1.6 | 0.8 | 1.0 | 1.0 | 0.6 | 1.9 | 1.1 | 1.3 | 1.39 |
| YEL072W | 3.2 | 3.5 | 1.6 | 1.8 | 1.5 | 0.6 | 1.3 | 1.6 | 4.7 | 4.3 | 1.7 | 0.9 | 1.0 | 2.0 | 0.5 | 1.5 | 0.8 | 1.1 | 0.39 |
| YER045C | 1.6 | 2.1 | 1.2 | 1.0 | 0.9 | 0.8 | 1.1 | 1.4 | 2.0 | 1.5 | 2.3 | 0.8 | 1.2 | 1.3 | 1.4 | 1.6 | 1.1 | 1.1 | 0.34 |
| YER181C | 1.1 | 0.4 | 1.7 | 2.2 | 1.0 | 0.7 | 1.0 | 0.9 | | 0.8 | 0.1 | 0.6 | 1.3 | 1.0 | 0.7 | 0.7 | 0.7 | 1.1 | 0.42 |

| | | | | | | | | | | | | | | | | | | | |
|---|---|---|---|---|---|---|---|---|---|---|---|---|---|---|---|---|---|---|---|
| YGL114W | 1.5 | 0.8 | 3.6 | 1.3 | 1.3 | 0.7 | 1.0 | 1.1 | 2.7 | 5.6 | 3.2 | 0.6 | 1.2 | 1.1 | 1.2 | 1.4 | 0.9 | 0.8 | 0.60 |
| YGL193C | 1.1 | 0.9 | 0.9 | 1.4 | 0.7 | 0.7 | 1.0 | 0.7 | 0.8 | 1.0 | 2.1 | 0.7 | 1.1 | 1.0 | 1.0 | 1.7 | 1.1 | 0.9 | 0.62 |
| YGL204C | 0.9 | 2.2 | 1.2 | 1.2 | 0.7 | 0.3 | 1.3 | 0.8 | 1.0 | 0.9 | 1.9 | 0.8 | 1.4 | 1.8 | 0.8 | 1.1 | 0.9 | 0.9 | 0.38 |
| YGL259W | 1.1 | 1.6 | 1.4 | 1.5 | 1.1 | 0.8 | 1.6 | 1.3 | 2.6 | 2.2 | 2.7 | 1.1 | 1.9 | 1.1 | 1.3 | 4.1 | 0.9 | 0.9 | 0.49 |
| YIL060W | 1.0 | 2.3 | 0.8 | 1.0 | 1.2 | 0.7 | 0.9 | 1.8 | 0.9 | 3.0 | 1.9 | 0.7 | 0.6 | 0.9 | 1.3 | 1.1 | 1.2 | 0.9 | 1.22 |
| YJL036W | 1.3 | 1.0 | 0.6 | 1.6 | 0.7 | 1.1 | 1.5 | 1.5 | 6.6 | 3.5 | 1.7 | 1.2 | 2.2 | 1.5 | 1.1 | 2.1 | 1.4 | 1.6 | 0.88 |
| YJR085C | 1.0 | 1.9 | 2.8 | 4.5 | 0.6 | 1.5 | 1.4 | 0.9 | 3.7 | 2.6 | 2.9 | 1.1 | 1.9 | 1.9 | 0.5 | 2.0 | 1.4 | 1.9 | 2.19 |
| YKR071C | 3.6 | 1.6 | 1.0 | 1.1 | 1.9 | 0.6 | 0.9 | 2.1 | 4.7 | 4.9 | 2.3 | 0.8 | 1.5 | 1.6 | 0.7 | 0.8 | 0.9 | 1.0 | 1.05 |
| YLR145W | 0.9 | 1.1 | 0.6 | 1.5 | 1.1 | 0.6 | 0.9 | 1.1 | 1.9 | 1.2 | 2.4 | 0.8 | 0.7 | 1.6 | 1.0 | 2.5 | 1.0 | 0.8 | 0.62 |
| YLR156W | 1.3 | 1.0 | 0.7 | 0.8 | 0.8 | 0.6 | 1.3 | 1.0 | 1.1 | 2.2 | 1.9 | 0.8 | 1.9 | 1.0 | 1.0 | 1.7 | 1.1 | 0.9 | 0.31 |
| YLR280C | 0.6 | 1.4 | 0.4 | 0.6 | 1.3 | 0.9 | 1.5 | 1.0 | 0.5 | 1.3 | 2.1 | 0.8 | 1.6 | 0.5 | 0.6 | 1.2 | 1.2 | 0.9 | 0.19 |
| YLR311C | 1.5 | 1.9 | 2.8 | 2.9 | 0.9 | 0.4 | 1.1 | 1.0 | 0.9 | 3.0 | 7.8 | 0.9 | 0.9 | 1.6 | 0.5 | 1.9 | 1.3 | 1.1 | 0.33 |
| YMR034C | 1.5 | 1.3 | 1.0 | 6.5 | 1.3 | 1.1 | 1.9 | 1.2 | 0.7 | 2.7 | 2.1 | 0.9 | 1.8 | 1.4 | 1.7 | 1.3 | 1.8 | 1.6 | 0.43 |
| YNL240C | 0.8 | 0.8 | 0.8 | 0.6 | 0.8 | 0.7 | 0.7 | 0.9 | 5.4 | 3.1 | 3.4 | 0.7 | 1.1 | 0.8 | 1.4 | 0.8 | 0.8 | 0.7 | 0.37 |
| YNL260C | 1.7 | 1.8 | 0.5 | 1.0 | 1.6 | 0.8 | 1.1 | 1.2 | 3.8 | 2.0 | 2.5 | 0.6 | 0.7 | 1.4 | 0.7 | 1.2 | 0.9 | 1.0 | 0.75 |
| YNR074C | 1.3 | 1.7 | 3.1 | 1.4 | 1.2 | 0.7 | 1.4 | 1.5 | 3.9 | 3.0 | 2.7 | 0.6 | 1.5 | 1.7 | 0.8 | 0.9 | 0.9 | 0.8 | 0.66 |
| YOL084W | 1.0 | 1.4 | 1.6 | 3.6 | 0.6 | 1.6 | 0.8 | 1.0 | 2.6 | 3.4 | 6.1 | 1.7 | 6.4 | 0.9 | 1.6 | 6.6 | 1.3 | 1.6 | 0.28 |
| YOL159C | 1.9 | 2.4 | 1.7 | 2.0 | 0.9 | 1.0 | 2.1 | 1.2 | 1.1 | 2.4 | 6.6 | 1.2 | 1.4 | 1.8 | 1.1 | 2.3 | 2.0 | 1.6 | 0.61 |
| YOR228C | 1.3 | 1.1 | 1.8 | 1.4 | 1.2 | 1.0 | 1.2 | 0.7 | 1.4 | 1.1 | 2.8 | 0.9 | 1.1 | 1.0 | 0.6 | 3.4 | 1.0 | 1.4 | 0.44 |
| YOR255W | 2.5 | 2.7 | 1.0 | 1.5 | 1.0 | 0.5 | 0.9 | 1.2 | 0.3 | 0.8 | 2.0 | 0.7 | 1.6 | 1.3 | 4.9 | 1.2 | 1.0 | 1.0 | 0.19 |
| YBR047W | 2.7 | 2.4 | 1.1 | 1.5 | 0.9 | 1.1 | | 2.2 | 11.0 | 10.0 | 3.4 | 0.8 | 1.7 | 1.0 | 0.8 | 1.1 | 1.3 | 1.0 | 0.25 |
| YER124C | 0.5 | 17.3 | 0.7 | 1.2 | 1.4 | 2.1 | 1.0 | 1.8 | 0.4 | 1.4 | 1.5 | 0.6 | 0.7 | 0.5 | 0.5 | 1.5 | 0.9 | 1.1 | 2.22 |
| YKR007W | 0.8 | 0.9 | 0.8 | 0.9 | 1.1 | 1.6 | 1.5 | 1.9 | 1.1 | 1.8 | 1.3 | 0.8 | 0.9 | 1.2 | 1.2 | 1.1 | 0.8 | 0.9 | 0.73 |
| YOR007C | 1.1 | 3.3 | 1.4 | 0.9 | 1.1 | 1.2 | 0.4 | 2.1 | 5.6 | 2.5 | 1.8 | 0.7 | 0.7 | 0.8 | 1.3 | 1.0 | 1.2 | 0.8 | 2.26 |
| YBL065W | 1.2 | 2.9 | 1.0 | 3.5 | 1.1 | 0.7 | 1.0 | 1.9 | 20.8 | 4.2 | 1.3 | 0.7 | 1.8 | 1.9 | 3.1 | 0.9 | 1.1 | 0.9 | 0.15 |
| YDL113C | 1.0 | 1.4 | 1.2 | 1.2 | 1.0 | 1.2 | 2.2 | 1.7 | 3.8 | 3.0 | 1.1 | 1.3 | 1.5 | 1.2 | 1.2 | 1.7 | 1.2 | 1.5 | 0.58 |
| YDR018C | 1.5 | 1.3 | 3.3 | 2.6 | 1.3 | 1.5 | 1.4 | 1.4 | 2.3 | 4.4 | 1.4 | 1.1 | 1.8 | 1.7 | 1.0 | 2.6 | 0.9 | 1.2 | 0.22 |
| YDR202C | 1.1 | 1.2 | 1.1 | 1.3 | 1.3 | 1.7 | 1.6 | 1.4 | 4.8 | 2.4 | 1.3 | 0.8 | 1.2 | 2.0 | 0.5 | 2.8 | 1.0 | 1.6 | 0.79 |
| YDR223W | 1.3 | 4.6 | 2.0 | 1.9 | 1.1 | 1.4 | 1.0 | 1.0 | 2.7 | 4.6 | 1.4 | 1.0 | 1.5 | 1.2 | 1.2 | 3.5 | 0.9 | 1.0 | 0.29 |
| YDR350C | 0.9 | 1.5 | 0.4 | 1.3 | 1.3 | 1.3 | 1.5 | 0.9 | 2.8 | 2.5 | 0.9 | 0.9 | 1.0 | 1.1 | 1.6 | 1.3 | 1.4 | 1.2 | 0.53 |
| YDR374C | 1.9 | 2.9 | 1.5 | 0.8 | 1.3 | 1.1 | 0.9 | 1.8 | 10.4 | 3.9 | 1.4 | 1.0 | 2.5 | 4.0 | 2.0 | 1.3 | 1.0 | 1.0 | 0.36 |
| YDR512C | 1.8 | 4.3 | 2.5 | 2.3 | 0.7 | 2.1 | 2.0 | 1.6 | 3.6 | 4.0 | 1.8 | 1.1 | 1.9 | 2.7 | 1.1 | 3.0 | 1.3 | 2.0 | 0.82 |

| | | | | | | | | | | | | | | | | | | | |
|---|---|---|---|---|---|---|---|---|---|---|---|---|---|---|---|---|---|---|---|
| YFR017C | 1.1 | 1.4 | 4.4 | 4.4 | 1.2 | 1.2 | 1.3 | 0.8 | 1.0 | 7.1 | 3.1 | 1.8 | 1.7 | 1.3 | 1.4 | 3.7 | 0.9 | 1.1 | 0.49 |
| YGL046W | 1.6 | 1.2 | 1.5 | 1.4 | 0.9 | 1.2 | 1.0 | 1.2 | 0.8 | 4.5 | 1.5 | 1.1 | 1.6 | 0.9 | 2.0 | 1.2 | 1.5 | 1.3 | 0.42 |
| YGL067W | 1.6 | 1.3 | 1.7 | 1.1 | 0.6 | 1.0 | 1.1 | 1.2 | 1.0 | 2.3 | 1.7 | 0.9 | 3.7 | 1.6 | 0.7 | 1.0 | 0.9 | 1.1 | 0.63 |
| YGL098W | 1.2 | 0.8 | 0.5 | 1.7 | 0.8 | 0.7 | 1.2 | 1.3 | 1.2 | 2.4 | 1.3 | 0.8 | 0.8 | 1.1 | 0.7 | 1.3 | 1.1 | 1.5 | 0.77 |
| YGL117W | 2.3 | 0.6 | 0.5 | 2.0 | 0.9 | 0.9 | 1.3 | 1.9 | 0.5 | 5.8 | 1.4 | 1.5 | 1.4 | 2.0 | 1.4 | 0.6 | 2.1 | 1.1 | 1.05 |
| YGL146C | 0.9 | 1.0 | 1.1 | 3.3 | 1.3 | 1.2 | 1.3 | 1.2 | 0.9 | 3.2 | 1.3 | 0.9 | 1.1 | 1.2 | 1.1 | 2.2 | 1.0 | 1.0 | 0.43 |
| YGR011W | 1.1 | 1.5 | 1.0 | 1.3 | 0.9 | 1.1 | 1.8 | 1.4 | 7.4 | 9.4 | 2.3 | 0.8 | 1.6 | 3.9 | 1.8 | 1.6 | 0.9 | 1.0 | 0.40 |
| YGR153W | 1.5 | 0.9 | 1.0 | 1.1 | 1.6 | 0.9 | 1.4 | 1.8 | 2.7 | 2.5 | 1.0 | 0.7 | 1.1 | 1.9 | 1.3 | 1.1 | 1.3 | 1.7 | 0.39 |
| YGR223C | 1.8 | 1.2 | 1.5 | 1.3 | 1.3 | 1.2 | 1.2 | 1.4 | 6.2 | 3.8 | 1.9 | 0.8 | 1.8 | 1.6 | 1.2 | 1.8 | 1.1 | 1.8 | 0.66 |
| YHR116W | 0.9 | 1.2 | 0.9 | 2.1 | 1.6 | 1.5 | 1.8 | 1.1 | 1.5 | 2.7 | 0.9 | 0.8 | 1.9 | 1.4 | 1.2 | 1.9 | 0.9 | 1.5 | 0.60 |
| YIL097W | 1.2 | 1.0 | 1.3 | 1.2 | 1.2 | 2.0 | 1.6 | 1.7 | 4.9 | 3.4 | 1.4 | 1.1 | 1.4 | 1.4 | 1.2 | 2.1 | 1.1 | 1.7 | 0.52 |
| YKL133C | 1.3 | 12.7 | 2.4 | 2.0 | 1.0 | 1.7 | 3.2 | 1.5 | 4.1 | 5.9 | 1.4 | 1.3 | 1.4 | 1.6 | 1.7 | 3.8 | 1.2 | 2.4 | 0.35 |
| YKL162C | 1.3 | 1.0 | 1.9 | 0.9 | 1.1 | 1.7 | 1.4 | 1.5 | 6.7 | 4.7 | 1.6 | 1.1 | 1.1 | 1.9 | 1.6 | 2.0 | 0.8 | 1.1 | 0.25 |
| YLL062C | 6.5 | 6.7 | 4.6 | 1.1 | 0.8 | 0.9 | 0.7 | 1.8 | 14.9 | 5.4 | 1.7 | 0.6 | 1.4 | 1.7 | 1.6 | 1.0 | 0.9 | 0.9 | 0.29 |
| YLR247C | 0.8 | 1.5 | 1.0 | 1.0 | 1.2 | 1.2 | 1.2 | 1.3 | 2.5 | 2.9 | 1.6 | 1.0 | 1.8 | 1.0 | 1.4 | 1.4 | 1.2 | 1.3 | 0.48 |
| YLR267W | 0.9 | 1.7 | 1.3 | 1.4 | 1.2 | 1.3 | 1.4 | 1.4 | 1.1 | 4.6 | 1.7 | 0.8 | 1.5 | 2.3 | 0.7 | 3.6 | 1.2 | 2.8 | 0.24 |
| YMR041C | 1.2 | 2.8 | 1.4 | 1.2 | 1.1 | 0.5 | 0.5 | 1.4 | 6.5 | 6.0 | 1.6 | 1.0 | 1.1 | 0.6 | 0.5 | 0.3 | 0.8 | 0.4 | 0.84 |
| YMR253C | 0.9 | 1.7 | 2.0 | 1.3 | 0.8 | 1.7 | 2.3 | 1.0 | 1.5 | 2.8 | 1.0 | 0.5 | 1.2 | 1.3 | 0.9 | 1.3 | 1.2 | 1.4 | 0.42 |
| YOR225W | 1.1 | 2.8 | 1.4 | 1.0 | 1.3 | 0.7 | 0.8 | 0.8 | 0.8 | 3.3 | 1.2 | 0.5 | 0.6 | 2.2 | 0.7 | 0.6 | 0.9 | 0.9 | 0.26 |
| YPL166W | 1.0 | 1.4 | 1.4 | 1.4 | 1.2 | 0.7 | 1.8 | 1.2 | 1.5 | 3.1 | 1.4 | 0.8 | 1.4 | 0.8 | 1.2 | 2.3 | 1.1 | 1.6 | 0.39 |
| YPL202C | 1.1 | 1.6 | 1.0 | 0.7 | 1.9 | 1.4 | 1.6 | 1.1 | 1.8 | 3.5 | 1.0 | 0.7 | 1.1 | 0.9 | 1.0 | 1.2 | 1.1 | 1.0 | 0.56 |
| YBR101C | 1.3 | 2.2 | 1.7 | 1.1 | 1.2 | 0.8 | 0.6 | 1.0 | 6.9 | 3.0 | 1.3 | 0.7 | 1.6 | 0.7 | 0.5 | 0.3 | 0.9 | 1.0 | 1.83 |
| YBR269C | 1.2 | 5.7 | 2.2 | 1.1 | 1.3 | 1.8 | 1.3 | 0.8 | 2.7 | 2.6 | 1.2 | 1.0 | 1.3 | 1.4 | 0.4 | 1.6 | 1.5 | 1.6 | 0.58 |
| YBR280C | 1.2 | 1.0 | 2.7 | 2.0 | 1.3 | 1.6 | 1.7 | 1.0 | 5.3 | 1.9 | 1.3 | 1.3 | 2.8 | 1.3 | 1.0 | 3.2 | 1.1 | 1.7 | 0.33 |
| YDL234C | 1.9 | 0.9 | 0.5 | 1.4 | 1.0 | 1.1 | 1.9 | 1.3 | 4.6 | 2.1 | 1.5 | 0.9 | 3.5 | 0.7 | 1.0 | 2.5 | 2.3 | 3.6 | 0.94 |
| YDL242W | 1.3 | 1.3 | 1.6 | 1.5 | 1.3 | 0.7 | 1.1 | 0.5 | 4.0 | 4.1 | 1.3 | 1.0 | 1.0 | 1.0 | 1.2 | 1.3 | 0.9 | 1.0 | 0.38 |
| YDR531W | 0.8 | 0.9 | 0.7 | 1.0 | 2.0 | 1.2 | 1.6 | 1.4 | 2.9 | 1.5 | 1.3 | 0.9 | 1.4 | 1.2 | 0.9 | 1.2 | 1.5 | 1.2 | 1.32 |
| YFR042W | 1.5 | 1.2 | 1.9 | 1.9 | 1.8 | 0.8 | 1.6 | 1.0 | 2.8 | 2.6 | 1.7 | 1.1 | 1.9 | 1.7 | 1.4 | 1.4 | 1.7 | 1.7 | 1.17 |
| YFR046C | 1.1 | 2.3 | 1.1 | 1.6 | 1.2 | 1.0 | 1.0 | 1.5 | 5.8 | 1.5 | 1.0 | 0.9 | 1.1 | 1.4 | 0.9 | 1.3 | 1.2 | 1.2 | 0.29 |
| YGL227W | 1.0 | 0.8 | 0.9 | 0.7 | 1.2 | 1.1 | 1.2 | 1.0 | 2.4 | 1.9 | 1.1 | 0.8 | 1.0 | 1.3 | 0.9 | 1.5 | 1.1 | 1.3 | 0.59 |
| YGR089W | 0.8 | 0.8 | 0.9 | 0.5 | 1.1 | 1.2 | 0.9 | 0.8 | 4.0 | 1.4 | 0.9 | 0.7 | 0.8 | 1.1 | 0.8 | 0.9 | 0.9 | 1.1 | 0.50 |
| YGR134W | 1.1 | 1.5 | 0.5 | 1.3 | 1.3 | 1.0 | 1.0 | 1.5 | 2.4 | 1.9 | 0.9 | 0.7 | 1.5 | 1.1 | 1.1 | 0.7 | 0.9 | 0.8 | 0.33 |

| | | | | | | | | | | | | | | | | | | | |
|---|---|---|---|---|---|---|---|---|---|---|---|---|---|---|---|---|---|---|---|
| YHR017W | 0.8 | 1.0 | 1.3 | 1.3 | 1.4 | 1.1 | 1.5 | 1.5 | 2.9 | 1.7 | 1.5 | 1.0 | 1.2 | 1.2 | 0.8 | 2.5 | 1.1 | 1.7 | 0.90 |
| YIL152W | 1.1 | 1.3 | 1.0 | 1.7 | 1.0 | 0.8 | 1.2 | 1.0 | 3.1 | 1.8 | 0.9 | 1.1 | 1.4 | 1.7 | 0.9 | 1.5 | 1.1 | 1.0 | 0.81 |
| YIL164C | 1.2 | 1.2 | 1.0 | 1.2 | 1.0 | 1.1 | 2.0 | 1.2 | 4.2 | 3.0 | 1.0 | 1.0 | 1.8 | 2.0 | 0.9 | 2.5 | 1.4 | 1.4 | 0.54 |
| YJR056C | 1.0 | 2.4 | 0.8 | 1.2 | 0.9 | 0.5 | 0.9 | 1.2 | 3.5 | 1.4 | 0.8 | 0.8 | 0.9 | 1.4 | 1.6 | 1.3 | 0.9 | 1.1 | 0.44 |
| YJR072C | 0.7 | 2.5 | 1.0 | 0.8 | 1.1 | 0.7 | 0.8 | 1.3 | 3.1 | 1.6 | 0.9 | 1.0 | 1.1 | 1.2 | 0.6 | 0.9 | 0.8 | 0.8 | 0.78 |
| YKL034W | 0.8 | 1.4 | 1.0 | 0.9 | 1.1 | 1.9 | 1.4 | 0.7 | 2.6 | 3.7 | 1.2 | 1.0 | 1.9 | 1.0 | 1.6 | 1.5 | 1.3 | 1.2 | 0.41 |
| YKR012C | 0.7 | 1.4 | 1.0 | 1.0 | 1.2 | 1.4 | 1.0 | 1.0 | 3.9 | 1.2 | 0.8 | 0.7 | 1.5 | 1.4 | 1.2 | 0.9 | 1.0 | 0.9 | 0.61 |
| YLR064W | 1.1 | 1.1 | 2.9 | 1.3 | 0.8 | 1.2 | 0.8 | 1.0 | 3.3 | 2.1 | 1.6 | 0.7 | 1.6 | 1.4 | 1.5 | 1.0 | 0.9 | 1.0 | 1.49 |
| YLR364W | 3.3 | 8.0 | 1.2 | 1.5 | 1.3 | 1.0 | 1.0 | 1.1 | 8.3 | 1.3 | 1.5 | 0.7 | 1.0 | 3.0 | 1.9 | 1.0 | 1.0 | 0.9 | 0.37 |
| YLR421C | 1.1 | 1.3 | 0.9 | 1.2 | 0.9 | 2.6 | 1.5 | 1.5 | 4.5 | 3.3 | 1.2 | 0.9 | 1.6 | 1.6 | 1.6 | 1.9 | 1.2 | 1.8 | 2.07 |
| YML118W | 1.5 | 0.5 | 0.7 | 2.7 | 0.7 | 0.8 | 1.5 | 0.9 | 6.6 | 1.6 | 1.3 | 0.9 | 1.7 | 1.0 | 2.2 | 1.6 | 1.5 | 1.5 | 0.26 |
| YMR114C | 1.2 | 1.4 | 0.5 | 2.4 | 1.2 | 1.2 | 1.7 | 1.1 | 2.5 | 1.7 | 1.0 | 0.9 | 1.1 | 1.5 | 1.2 | 3.0 | 1.0 | 1.3 | 0.62 |
| YMR115W | 1.0 | 0.6 | 1.3 | 1.1 | 0.9 | 1.5 | 1.0 | 1.2 | 5.8 | 2.1 | 1.1 | 1.0 | 1.3 | 1.3 | 1.2 | 1.1 | 1.0 | 1.2 | 0.61 |
| YMR258C | 0.9 | 1.4 | 1.2 | 1.1 | 0.7 | 1.2 | 1.5 | 0.9 | 2.3 | 1.8 | 1.4 | 0.9 | 1.6 | 1.0 | 0.6 | 1.2 | 1.1 | 1.3 | 0.60 |
| YNL181W | 1.2 | 1.2 | 0.6 | 1.2 | 1.4 | 1.6 | 0.9 | 1.9 | 5.3 | 2.8 | 1.2 | 0.8 | 1.8 | 1.9 | 1.0 | 1.0 | 0.8 | 1.3 | 0.86 |
| YNL191W | 1.5 | 5.1 | 4.7 | 0.7 | 0.9 | 0.6 | 3.0 | 1.2 | 3.9 | 3.2 | 1.0 | 0.5 | 0.6 | 1.0 | 1.0 | 0.6 | 0.5 | 0.6 | 0.45 |
| YNL212W | 1.1 | 1.1 | 1.1 | 0.5 | 0.9 | 0.8 | 1.3 | 1.3 | 4.6 | 2.3 | 1.3 | 0.8 | 1.3 | 1.0 | 0.9 | 1.3 | 0.9 | 1.2 | 0.67 |
| YNL265C | 1.0 | 1.3 | 0.5 | 1.3 | 0.9 | 1.9 | 1.5 | 1.4 | 5.0 | 2.6 | 0.9 | 0.8 | 2.0 | 1.9 | 0.6 | 1.7 | 0.9 | 1.8 | 0.85 |
| YOR088W | 0.7 | 0.9 | 1.7 | 0.9 | 1.2 | 0.7 | 0.3 | 0.7 | 2.5 | 1.3 | 0.8 | 0.6 | 0.4 | 0.4 | 0.8 | 0.4 | 0.7 | 0.7 | 3.56 |
| YOR155C | 0.8 | 1.2 | 1.7 | 1.0 | 1.4 | | | 0.8 | 3.6 | 1.7 | 1.6 | 0.5 | | 1.5 | 0.9 | | 0.8 | 0.9 | 0.46 |
| YPL151C | 1.0 | 0.8 | 1.2 | 1.2 | 1.0 | 0.7 | 0.7 | 1.0 | 4.2 | 2.0 | 0.9 | 0.8 | 1.4 | 0.9 | 1.8 | 0.9 | 0.8 | 1.0 | 0.51 |
| YPL249C | 0.8 | 0.9 | 1.1 | 0.6 | 0.8 | 2.5 | 1.0 | 1.0 | 3.0 | 1.2 | 0.9 | 0.9 | 1.8 | 1.0 | 1.0 | 1.0 | 0.8 | 1.2 | 0.36 |
| YPL260W | 0.9 | 3.9 | 1.4 | 0.8 | 0.8 | 1.4 | 1.2 | 1.1 | 2.6 | 2.1 | 1.0 | 0.8 | 1.4 | 1.1 | 1.3 | 1.2 | 0.8 | 1.3 | 0.82 |
| YPR061C | 1.3 | 3.2 | 1.2 | 4.9 | 1.4 | 0.5 | 1.8 | 1.2 | 3.2 | 1.9 | 1.6 | 0.8 | 1.1 | 1.7 | 0.4 | 2.7 | 1.1 | 0.9 | 0.40 |
| YPR093C | 1.1 | 1.1 | 0.7 | 1.3 | 1.0 | 0.7 | 0.8 | 1.1 | 6.6 | 2.3 | 1.2 | 0.9 | 1.4 | 1.1 | 0.8 | 1.4 | 1.4 | 1.1 | 0.39 |
| YPR158W | 1.5 | 1.5 | 3.7 | 0.9 | 1.6 | 0.7 | 1.2 | 1.2 | 5.0 | 3.5 | 1.5 | 0.6 | 1.6 | 1.1 | 0.9 | 0.6 | 0.9 | 0.7 | 0.96 |
| YPR169W | 0.9 | 0.9 | 0.5 | 0.6 | 1.0 | 1.5 | 0.9 | 1.0 | 3.5 | 1.4 | 1.0 | 0.5 | 1.0 | 1.0 | 0.9 | 1.4 | 1.1 | 1.1 | 0.86 |
| YPR174C | 0.8 | 0.9 | 0.8 | 0.6 | 1.3 | 1.1 | 0.8 | 1.2 | 3.2 | 1.0 | 0.7 | 1.1 | 1.4 | 0.9 | 1.2 | 0.4 | 1.4 | 0.9 | 0.55 |
| YAL014C | 1.0 | 1.6 | 1.4 | 1.2 | 0.8 | 0.6 | 1.2 | 1.2 | 3.1 | 2.0 | 1.7 | 1.2 | 1.5 | 1.8 | 2.3 | 0.9 | 1.1 | 1.1 | 0.56 |
| YAL017W | 0.6 | 1.4 | 2.3 | 1.2 | 1.0 | 1.1 | 1.3 | 0.6 | 1.9 | 1.6 | 0.7 | 1.0 | 2.2 | 1.1 | 1.0 | 1.3 | 1.0 | 1.2 | 0.79 |
| YAL049C | 1.0 | 2.0 | 1.7 | 1.0 | 0.7 | 1.5 | 3.4 | 1.4 | 3.0 | 1.4 | 1.0 | 1.1 | 1.6 | 2.0 | 1.4 | 2.9 | 1.6 | 1.8 | 1.13 |
| YBR013C | 1.1 | 2.7 | 1.8 | 1.4 | 0.9 | 1.8 | 1.0 | 1.4 | 2.2 | 2.2 | 1.2 | 1.2 | 2.0 | 2.5 | 1.1 | 1.5 | 1.4 | 1.1 | 0.68 |

| | | | | | | | | | | | | | | | | | | | |
|---|---|---|---|---|---|---|---|---|---|---|---|---|---|---|---|---|---|---|---|
| YBR051W | 1.2 | 0.8 | 1.5 | 0.3 | 1.1 | | 1.1 | 1.1 | 1.5 | 1.5 | 0.1 | 0.7 | 1.2 | 1.0 | 0.8 | 1.2 | 1.1 | 0.8 | 0.40 |
| YBR063C | 0.8 | 0.8 | 0.4 | 0.6 | 1.2 | 1.6 | 1.6 | 1.3 | 2.2 | 1.6 | 0.6 | 1.1 | 1.7 | 1.7 | 1.6 | 1.0 | 1.2 | 1.2 | 0.38 |
| YBR129C | 1.1 | 0.8 | 0.6 | 1.0 | 1.2 | 1.9 | 1.0 | 1.4 | 2.3 | 1.4 | 1.4 | 1.0 | 1.3 | 1.6 | 0.9 | 1.7 | 1.0 | 1.5 | 1.25 |
| YBR255W | 1.1 | 1.6 | 0.4 | 0.7 | 1.2 | 1.0 | 1.1 | 1.8 | 1.8 | 1.5 | 1.5 | 0.7 | 1.4 | 1.1 | 1.0 | 1.5 | 1.0 | 1.0 | 0.27 |
| YBR281C | 0.8 | 1.1 | 3.0 | 0.5 | 0.8 | | 0.6 | 1.2 | 1.8 | 1.4 | 1.4 | 0.5 | 1.2 | 0.9 | 1.0 | 0.8 | 0.7 | 0.8 | 0.44 |
| YCL044C | 0.8 | 1.4 | 2.4 | 0.6 | 1.1 | 1.0 | 1.0 | 1.0 | 3.3 | 4.1 | 1.1 | 0.9 | 1.7 | 1.0 | 4.4 | 0.8 | 0.9 | 0.6 | 0.21 |
| YDL089W | 1.1 | 1.3 | 1.4 | 0.9 | 1.2 | 2.0 | 2.0 | 1.1 | 2.3 | 2.5 | 2.0 | 0.9 | 1.9 | 1.2 | 1.6 | 1.3 | 1.0 | 1.1 | 0.45 |
| YDL173W | 1.0 | 1.2 | 1.0 | 0.8 | 1.3 | 1.1 | 1.6 | 1.5 | 1.8 | 1.6 | 1.2 | 1.1 | 1.7 | 2.1 | 1.1 | 1.3 | 1.3 | 1.8 | 1.19 |
| YDL193W | 0.9 | 1.5 | 1.4 | 0.8 | 1.1 | 2.0 | 1.5 | 1.6 | 2.5 | 1.4 | 1.1 | 1.1 | 1.4 | 1.1 | 1.2 | 1.8 | 0.9 | 1.4 | 0.93 |
| YDL233W | 0.8 | 2.0 | 1.0 | 3.8 | 0.9 | 1.0 | 0.8 | 1.0 | 2.1 | 1.1 | 0.7 | 0.9 | 1.8 | 1.1 | 1.3 | 0.9 | 1.1 | 1.4 | 0.33 |
| YDR071C | 1.4 | 1.1 | 0.7 | 1.3 | 1.1 | 1.5 | 1.3 | 1.6 | 2.6 | 1.6 | 1.4 | 0.9 | 1.3 | 2.0 | 0.9 | 1.2 | 0.9 | 1.6 | 3.08 |
| YDR078C | 1.1 | 1.6 | 3.5 | 0.8 | 0.9 | 0.8 | 2.8 | 1.4 | 2.1 | 1.4 | 1.1 | 0.7 | 1.1 | 1.8 | 0.5 | 1.3 | 0.9 | 1.2 | 0.53 |
| YDR109C | 0.8 | 1.0 | 1.0 | 1.3 | 1.0 | 0.9 | 1.2 | 1.5 | 1.9 | 1.3 | 0.9 | 1.0 | 1.4 | 1.0 | 1.7 | 1.5 | 1.2 | 0.9 | 0.38 |
| YDR140W | 1.7 | 1.4 | 0.9 | 1.8 | 0.7 | 0.9 | 1.2 | 1.5 | 2.2 | 2.5 | 1.7 | 1.1 | 2.0 | 3.2 | 0.9 | 1.9 | 1.3 | 1.3 | 0.78 |
| YDR221W | 0.8 | 1.3 | 0.3 | 0.8 | 1.1 | 1.5 | 0.9 | 1.0 | 2.3 | 1.4 | 0.7 | 0.6 | 1.0 | 1.0 | 1.0 | 1.0 | 1.1 | 1.0 | 0.34 |
| YDR271C | 1.0 | 0.8 | 0.7 | 1.7 | 0.8 | 1.4 | 1.3 | 0.8 | 5.4 | 0.9 | 0.6 | 0.7 | 1.5 | 1.4 | 1.3 | 0.6 | 1.2 | 0.9 | 0.32 |
| YDR316W | 1.0 | 0.8 | 0.6 | 0.9 | 1.5 | 0.7 | 0.3 | 1.4 | 2.5 | 1.0 | 1.3 | 0.6 | 0.6 | 0.7 | 1.3 | 0.3 | 0.6 | 0.5 | 0.69 |
| YDR338C | 1.1 | 1.3 | 1.4 | 1.0 | 0.7 | 0.9 | 1.1 | 0.9 | 2.7 | 1.7 | 1.9 | 0.8 | 1.3 | 1.0 | 0.8 | 0.8 | 0.9 | 1.0 | 0.40 |
| YDR421W | 1.1 | 1.0 | 1.0 | 1.1 | 0.9 | 0.5 | 0.2 | 0.9 | 3.8 | 1.2 | 1.0 | 0.7 | 0.6 | 1.2 | 1.9 | 1.0 | 0.7 | 0.8 | 0.42 |
| YDR425W | 1.3 | 1.7 | 1.0 | 2.2 | 1.4 | 1.7 | 1.8 | 1.2 | 2.6 | 2.2 | 0.9 | 1.1 | 1.4 | 1.3 | 1.3 | 1.1 | 1.2 | 1.2 | 0.31 |
| YDR485C | 0.8 | 0.9 | 0.7 | 0.9 | 1.6 | 1.2 | 1.2 | 1.1 | 2.7 | 2.0 | 0.9 | 0.9 | 1.2 | 1.1 | 1.3 | 1.5 | 0.8 | 1.0 | 0.59 |
| YDR504C | 1.0 | 1.0 | 0.9 | 0.8 | 1.1 | 1.2 | 1.1 | 0.8 | 2.3 | 1.1 | 1.2 | 0.7 | 2.2 | 1.3 | 1.2 | 1.2 | 1.2 | 1.2 | 0.70 |
| YEL044W | 0.8 | 1.5 | 0.7 | 0.9 | 1.2 | 0.8 | 0.8 | 0.8 | 2.1 | 1.2 | 1.6 | 0.6 | 1.3 | 0.6 | 0.6 | 0.7 | 1.4 | 1.0 | 0.93 |
| YER092W | 1.3 | 1.3 | 1.2 | 1.1 | 1.2 | 1.5 | 1.3 | 1.1 | 1.9 | 1.6 | 0.9 | 0.9 | 1.2 | 1.2 | 1.0 | 1.9 | 1.4 | 1.7 | 1.13 |
| YER182W | 1.2 | 0.8 | 1.1 | 1.9 | 1.2 | 0.6 | 0.8 | 1.4 | 2.2 | 1.2 | 1.5 | 1.0 | 0.9 | 1.9 | 0.5 | 3.6 | 0.8 | 1.2 | 0.87 |
| YFL042C | 0.8 | 0.9 | 0.6 | 1.1 | 1.4 | 1.5 | 1.1 | 0.8 | 2.0 | 2.0 | 5.4 | 0.9 | 1.7 | 1.2 | 1.1 | 1.0 | 1.1 | 1.2 | 0.43 |
| YFR056C | 0.9 | 1.5 | 0.7 | 0.5 | 1.2 | 0.3 | 0.9 | 1.0 | 2.4 | 2.7 | 1.3 | 0.5 | 0.3 | 0.6 | 0.9 | 0.5 | 1.1 | 0.8 | 0.59 |
| YGL041C | 0.9 | 0.6 | 1.3 | 1.0 | 0.6 | 1.4 | 1.0 | 0.8 | 1.3 | 0.4 | 0.6 | 0.6 | 1.0 | 1.2 | 0.7 | 0.8 | 1.0 | 1.1 | 0.51 |
| YGL045W | 1.4 | 1.1 | 1.9 | 3.4 | 1.0 | 0.8 | 1.7 | 1.2 | 2.8 | 4.2 | 1.2 | 1.1 | 1.3 | 1.1 | 1.5 | 3.4 | 1.3 | 1.2 | 0.47 |
| YGL057C | 1.3 | 1.1 | 1.4 | 1.4 | 1.3 | 1.3 | 1.1 | 1.1 | 2.1 | 1.1 | 1.3 | 1.1 | 1.3 | 1.5 | 0.7 | 1.2 | 0.9 | 1.1 | 0.56 |
| YGL183C | 1.1 | 1.4 | 0.8 | 0.5 | 1.6 | 1.2 | 1.2 | 0.8 | 3.4 | 2.5 | 0.8 | 0.9 | 1.5 | 1.7 | 1.7 | 0.6 | 1.4 | 1.0 | 0.18 |
| YGL223C | 1.1 | 1.7 | 1.0 | 0.7 | 1.4 | 1.1 | 1.2 | 1.0 | 2.6 | 2.0 | 1.3 | 0.9 | 1.7 | 0.9 | 0.7 | 1.1 | 0.8 | 1.0 | 0.60 |

| | | | | | | | | | | | | | | | | | | | |
|---|---|---|---|---|---|---|---|---|---|---|---|---|---|---|---|---|---|---|---|
| YGR156W | 0.9 | 1.3 | 1.7 | 1.2 | 1.0 | 1.2 | 1.3 | 1.3 | 4.0 | 2.4 | 0.5 | 1.2 | 1.8 | 1.6 | 1.3 | 0.5 | 0.8 | 0.8 | 0.26 |
| YGR198W | 0.7 | 0.9 | 0.7 | 1.0 | 0.8 | 0.8 | 0.8 | 1.0 | 2.9 | 1.8 | 1.0 | 0.7 | 1.3 | 0.8 | 0.9 | 1.2 | 1.1 | 1.0 | 0.87 |
| YGR210C | 0.9 | 1.4 | 1.1 | 0.9 | 0.8 | 0.8 | 0.8 | 1.0 | 3.5 | 1.8 | 1.0 | 0.6 | 1.0 | 0.8 | 1.3 | 0.7 | 1.1 | 0.7 | 0.56 |
| YGR211W | 0.7 | 1.0 | 2.0 | 1.0 | 0.9 | 0.6 | 0.5 | 1.0 | 2.9 | 2.5 | 0.8 | 0.6 | 0.8 | 0.4 | 0.5 | 0.3 | 0.5 | 0.5 | 1.99 |
| YGR237C | 0.8 | 1.3 | 2.5 | 0.8 | 1.2 | 0.7 | 1.4 | 1.0 | 1.8 | 1.4 | 1.6 | 0.8 | 1.6 | 0.6 | 1.1 | 1.2 | 0.9 | 1.0 | 0.50 |
| YGR250C | 1.3 | 1.4 | 1.4 | 1.5 | 1.4 | 1.6 | 1.2 | 1.1 | 2.4 | 2.8 | 1.6 | 0.6 | 1.7 | 1.3 | 1.2 | 1.2 | 1.3 | 2.1 | 1.21 |
| YGR266W | 0.7 | 0.5 | 1.0 | 0.9 | 0.8 | 1.2 | 0.9 | 0.9 | 2.0 | 1.1 | 1.2 | 0.6 | 1.1 | 0.8 | 1.1 | 1.3 | 0.7 | 1.0 | 0.59 |
| YGR277C | 1.0 | 1.9 | 1.1 | 0.8 | 0.8 | 0.8 | 1.9 | 1.3 | 2.8 | 1.5 | 1.2 | 0.9 | 1.6 | 1.5 | 0.9 | 1.0 | 1.3 | 1.1 | 0.81 |
| YHL021C | 1.6 | 3.9 | 5.9 | 3.2 | 0.9 | 1.4 | 2.7 | 1.0 | 2.7 | 1.1 | 1.4 | 1.1 | 2.6 | 1.1 | 1.2 | 2.4 | 1.3 | 3.6 | 1.27 |
| YHL037C | 1.2 | 1.4 | 0.9 | | 1.0 | 0.7 | 1.0 | 1.1 | 0.4 | 1.1 | 1.0 | 0.6 | 1.0 | 0.9 | 0.8 | 0.7 | 0.9 | 0.9 | 0.28 |
| YHR083W | 1.0 | 0.9 | 1.4 | 1.3 | 0.9 | 0.9 | 1.0 | 1.0 | 2.0 | 2.1 | 1.3 | 0.9 | 1.1 | 1.6 | 1.3 | 2.4 | 0.8 | 1.0 | 0.90 |
| YHR134W | 1.1 | 0.7 | 0.4 | 1.1 | 1.1 | 2.0 | 1.3 | 1.2 | 3.5 | 1.5 | 0.7 | 1.0 | 1.2 | 1.6 | 1.0 | 1.2 | 1.3 | 1.3 | 0.81 |
| YHR180W | 1.5 | 0.7 | 1.1 | 1.5 | 0.9 | 1.1 | 1.1 | 1.2 | 4.8 | 3.5 | 1.4 | 0.7 | 1.3 | 1.5 | 0.8 | 1.3 | 0.8 | 1.0 | 0.36 |
| YIL108W | 1.0 | 1.7 | 2.2 | 0.7 | 1.3 | 0.6 | 0.9 | 0.8 | 2.4 | 2.0 | 1.2 | 0.7 | 1.3 | 1.0 | 1.8 | 0.7 | 1.2 | 0.7 | 0.51 |
| YIL165C | 1.2 | 2.3 | 0.9 | 1.6 | 1.6 | 0.8 | 1.5 | 1.1 | 3.2 | 3.4 | 1.1 | 0.9 | 1.5 | 1.3 | 0.8 | 1.9 | 1.4 | 1.3 | 0.74 |
| YJL032W | 1.1 | 1.0 | 0.8 | 0.9 | 0.9 | 0.6 | 0.9 | 1.3 | 3.3 | 2.0 | 1.8 | 0.5 | 1.4 | 1.6 | 1.0 | 1.2 | 1.1 | 1.1 | 0.33 |
| YJL049W | 1.3 | 0.8 | 0.5 | 1.8 | 1.6 | 1.5 | 1.2 | 1.5 | 2.0 | 1.9 | 1.5 | 0.8 | 0.9 | 1.8 | 0.8 | 1.6 | 0.9 | 1.1 | 0.75 |
| YJR044C | 1.1 | 1.2 | 5.1 | 1.0 | 1.0 | 1.5 | 2.0 | 1.0 | 2.2 | 1.6 | 1.9 | 0.7 | 2.0 | 1.3 | 1.6 | 2.7 | 1.8 | 1.6 | 1.18 |
| YKL059C | 0.8 | 1.0 | 0.9 | 0.7 | 1.3 | 0.9 | 1.0 | 1.0 | 2.6 | 1.2 | 0.9 | 0.9 | 1.3 | 1.1 | 1.1 | 0.8 | 1.1 | 0.9 | 0.53 |
| YKL090W | 1.0 | 0.9 | 1.0 | 1.2 | 1.1 | 1.0 | 1.1 | 1.2 | 3.9 | 1.6 | 2.3 | 0.6 | 0.8 | 1.4 | 0.8 | 1.6 | 0.7 | 1.0 | 0.37 |
| YKL094W | 1.2 | 1.6 | 1.2 | 1.9 | 0.9 | 1.0 | 1.1 | 1.3 | 3.1 | 1.8 | 1.3 | 0.9 | 1.7 | 1.3 | 1.0 | 2.0 | 1.2 | 1.9 | 1.60 |
| YLR097C | 1.1 | 1.5 | 1.0 | 1.6 | 1.6 | 1.7 | 1.8 | 1.4 | 2.2 | 1.1 | 1.7 | 0.8 | 1.1 | 1.7 | 0.9 | 2.5 | 1.1 | 1.7 | 0.76 |
| YLR226W | 1.0 | 1.8 | 0.3 | 1.0 | 1.7 | 1.1 | 1.9 | 1.3 | 2.1 | 1.4 | 0.8 | 0.7 | 0.7 | 1.1 | 1.3 | 0.9 | 1.1 | 1.0 | 0.56 |
| YLR392C | 1.1 | 0.9 | 0.8 | 1.9 | 1.0 | 1.2 | 1.0 | 1.3 | 2.4 | 2.1 | 1.4 | 0.6 | 1.4 | 1.1 | 0.5 | 2.1 | 0.8 | 1.6 | 0.46 |
| YLR427W | 0.6 | 1.0 | 0.2 | 0.5 | 1.6 | 1.0 | 1.2 | 1.1 | 2.1 | 1.3 | 0.8 | 0.9 | 2.5 | 0.8 | 0.8 | 1.3 | 1.0 | 1.0 | 0.49 |
| YML013W | 0.8 | 1.0 | 0.4 | 0.6 | 1.3 | 0.6 | 1.3 | 0.6 | 2.1 | 1.2 | 1.1 | 0.9 | 1.2 | 1.1 | 2.2 | 1.3 | 1.1 | 0.8 | 0.27 |
| YML029W | 0.6 | 1.5 | 1.3 | 1.0 | 1.2 | 1.6 | 1.1 | 0.9 | 2.3 | 1.8 | 1.2 | 1.1 | 2.0 | 1.6 | 0.8 | 1.1 | 1.2 | 1.0 | 0.46 |
| YML041C | 1.2 | 1.0 | 0.6 | 1.0 | 1.3 | 1.9 | 1.4 | 1.8 | 3.0 | 1.5 | 0.9 | 0.8 | 1.2 | 2.2 | 1.0 | 1.4 | 1.3 | 1.3 | 0.59 |
| YML079W | 1.0 | 1.7 | 1.1 | 1.5 | 0.8 | 1.4 | 1.2 | 1.2 | 1.9 | 1.4 | 1.2 | 1.1 | 1.6 | 1.4 | 0.3 | 2.0 | 1.4 | 1.4 | 1.18 |
| YMR068W | 0.9 | 0.7 | 0.8 | 1.5 | 1.6 | 1.1 | 1.5 | 0.8 | 2.4 | 1.4 | 1.1 | 0.7 | 1.4 | 1.1 | 2.2 | 0.8 | 1.3 | 1.1 | 0.24 |
| YMR160W | 0.8 | 1.3 | 1.2 | 1.2 | 1.4 | 2.2 | 1.0 | 0.9 | 2.2 | 1.8 | 1.0 | 1.0 | 1.6 | 1.1 | 1.5 | 1.4 | 0.9 | 1.3 | 0.37 |
| YNL026W | 0.7 | 1.0 | 1.3 | 0.8 | 1.4 | 1.1 | 1.5 | 1.0 | 2.7 | 1.8 | 1.1 | 0.9 | 1.7 | 0.8 | 0.8 | 1.5 | 1.0 | 1.4 | 0.86 |

| | | | | | | | | | | | | | | | | | | | |
|---|---|---|---|---|---|---|---|---|---|---|---|---|---|---|---|---|---|---|---|
| YNL063W | 0.9 | 1.9 | 0.9 | 1.4 | 1.1 | 1.1 | 1.4 | 1.5 | 3.0 | 1.8 | 1.1 | 1.0 | 1.5 | 1.1 | 0.7 | 1.8 | 0.9 | 1.0 | 0.54 |
| YNL176C | 1.1 | 1.3 | 2.9 | 1.0 | 0.8 | 0.7 | 0.7 | 0.9 | 2.4 | 2.1 | 1.2 | 0.6 | 0.6 | 0.9 | 0.8 | 1.3 | 0.6 | 0.8 | 0.66 |
| YNL194C | 1.6 | 0.7 | 2.0 | 15.2 | 0.7 | 0.7 | 1.5 | 0.9 | 2.2 | 6.4 | 17.1 | 0.5 | 1.5 | 3.0 | 0.5 | 4.3 | 0.8 | 2.4 | 0.34 |
| YNL253W | 1.3 | 1.3 | 0.6 | 1.0 | 1.5 | 1.3 | 1.3 | 1.1 | 2.8 | 2.6 | 0.8 | 0.8 | 1.1 | 1.4 | 0.5 | 1.1 | 1.1 | 1.2 | 0.54 |
| YNL276C | 1.3 | 13.1 | 1.9 | 0.3 | 1.1 | 1.3 | 0.9 | 0.7 | 3.1 | 1.3 | 0.7 | 0.9 | 1.5 | 1.5 | 0.5 | 0.8 | 1.1 | 0.8 | 0.22 |
| YNR051C | 0.7 | 0.5 | 1.1 | 0.6 | 1.3 | 1.8 | 1.3 | 0.7 | 2.4 | 1.4 | 1.5 | 0.9 | 2.1 | 1.0 | 1.2 | 0.6 | 0.7 | 0.8 | 1.58 |
| YOR022C | 1.3 | 1.1 | 1.1 | 1.2 | 1.4 | 1.2 | 1.6 | 1.2 | 2.0 | 1.4 | 1.7 | 0.8 | 1.7 | 1.0 | 0.8 | 1.8 | 1.1 | 1.1 | 0.36 |
| YOR087W | 0.7 | 1.0 | 1.8 | 1.3 | 0.8 | 0.3 | 0.9 | 0.8 | 2.8 | 1.3 | 1.3 | 0.8 | 1.2 | 0.9 | 0.8 | 1.2 | 0.8 | 1.1 | 0.61 |
| YOR138C | 0.8 | 1.5 | 0.7 | 0.8 | 0.8 | 1.0 | 1.1 | 0.8 | 2.1 | 3.7 | 0.7 | 0.9 | 1.8 | 0.9 | 1.0 | 1.1 | 1.1 | 1.1 | 0.47 |
| YOR267C | 0.8 | 0.7 | 1.0 | 0.9 | 1.2 | 1.6 | 0.9 | 0.7 | 2.4 | 2.2 | 0.4 | 0.9 | 1.4 | 0.9 | 1.3 | 0.8 | 1.3 | 1.0 | 0.58 |
| YPL005W | 1.0 | 1.0 | 1.0 | 1.0 | 1.4 | 0.8 | 2.0 | 1.0 | 2.6 | 2.8 | 1.1 | 0.7 | 1.2 | 1.0 | 1.2 | 1.6 | 1.2 | 1.1 | 0.32 |
| YPL150W | 0.7 | 0.9 | 1.6 | 0.9 | 1.0 | 0.6 | 1.2 | 1.1 | 3.1 | 2.0 | 1.1 | 1.0 | 2.1 | 1.0 | 1.1 | 1.2 | 1.4 | 1.1 | 0.47 |
| YPL152W | 1.4 | 1.1 | 1.4 | 1.8 | 1.4 | 1.2 | 1.3 | 1.1 | 3.6 | 2.7 | 1.4 | 0.9 | 1.4 | 1.2 | 2.3 | 1.4 | 1.2 | 1.9 | 0.42 |
| YPL168W | 1.1 | 1.5 | 0.5 | 1.4 | 1.0 | 0.8 | 1.2 | 1.2 | 2.1 | 1.6 | 0.7 | 0.7 | 1.0 | 1.0 | 0.8 | 1.3 | 1.1 | 1.2 | 0.41 |
| YPL180W | 0.9 | 2.0 | 0.7 | 0.6 | 1.2 | 1.5 | 1.0 | 1.0 | 1.9 | 1.8 | 1.0 | 0.8 | 1.5 | 1.0 | 0.7 | 1.0 | 1.0 | 0.9 | 0.30 |
| YPL188W | 1.3 | 1.2 | 2.9 | 1.0 | 0.9 | 0.6 | 1.3 | 1.6 | 2.5 | 2.6 | 1.0 | 0.6 | 0.9 | 1.4 | 0.8 | 1.7 | 0.7 | 0.9 | 0.55 |
| YPR049C | 1.0 | 2.5 | 1.5 | 1.0 | 0.7 | 1.2 | 1.1 | 1.1 | 2.4 | 2.1 | 0.9 | 0.9 | 1.6 | 1.4 | 1.5 | 1.7 | 1.0 | 1.1 | 0.34 |
| YPR148C | 1.1 | 1.2 | 0.8 | 1.3 | 0.9 | 1.0 | 1.6 | 1.5 | 2.7 | 1.7 | 0.9 | 1.2 | 1.2 | 1.1 | 1.7 | 1.7 | 0.9 | 2.0 | 1.25 |
| YPR172W | 1.1 | 1.6 | 2.2 | 1.2 | 1.0 | 1.7 | 1.2 | 1.0 | 2.1 | 1.3 | 1.9 | 0.8 | 1.3 | 1.6 | 1.5 | 1.9 | 1.1 | 1.4 | 0.45 |
| YAL018C | 2.0 | 1.9 | 1.1 | 11.3 | 0.8 | 0.7 | 1.0 | 1.6 | | 0.4 | 0.1 | 0.9 | 1.3 | 1.0 | 6.9 | 1.1 | 0.9 | 0.9 | 0.21 |
| YAR064W | 2.1 | 0.9 | 0.9 | 2.8 | 0.8 | 0.5 | 1.1 | 1.1 | 0.0 | 0.7 | 0.7 | 0.8 | 1.1 | 1.1 | 1.9 | 1.0 | 0.9 | 0.9 | 0.30 |
| YBR012C | 2.5 | 1.7 | 1.3 | 0.8 | 0.9 | 1.8 | 1.5 | 1.1 | 1.4 | 2.6 | 0.7 | 1.0 | 2.1 | 0.9 | 1.1 | 1.3 | 1.4 | 1.2 | 0.46 |
| YBR287W | 1.7 | 1.9 | 3.1 | 2.4 | 1.3 | 1.4 | 1.1 | 1.0 | 1.2 | 1.6 | 2.0 | 1.2 | 3.4 | 0.8 | 1.3 | 1.6 | 3.2 | 1.9 | 2.73 |
| YDR250C | 1.9 | 1.4 | 0.9 | 3.0 | 1.0 | 0.4 | 1.0 | 1.1 | | 0.5 | 0.6 | 0.8 | 1.2 | 0.9 | 0.7 | 1.7 | 0.9 | 0.9 | 0.32 |
| YJL037W | 1.8 | 1.4 | 1.4 | 2.4 | 1.5 | 1.8 | 1.2 | 1.2 | 1.8 | 2.4 | 1.6 | 0.8 | 1.1 | 2.0 | 1.6 | 1.0 | 1.3 | 0.9 | 0.26 |
| YNL058C | 2.0 | 1.0 | 2.1 | 1.7 | 1.0 | 0.4 | 0.7 | | 0.3 | 0.7 | 1.0 | 1.0 | 0.6 | 0.8 | 2.2 | 0.9 | 0.8 | 1.0 | 0.60 |
| YJR030C | 0.7 | 4.4 | 0.9 | 1.2 | 0.7 | 0.6 | 0.7 | 0.9 | 0.4 | 0.5 | 0.8 | 0.9 | 0.7 | 1.0 | 0.8 | 0.9 | 0.7 | 0.9 | 0.27 |
| YKR040C | 1.2 | 3.7 | 2.4 | 2.9 | 0.9 | 0.9 | 0.9 | 1.0 | 1.3 | 1.2 | 0.8 | 0.8 | 0.6 | 3.8 | 1.1 | 1.2 | 0.7 | 0.8 | 0.53 |
| YDR128W | 0.6 | 3.9 | 1.4 | 0.9 | 0.8 | 0.9 | 0.8 | 1.1 | 0.9 | 1.5 | 0.3 | 0.6 | 1.1 | 1.0 | 1.4 | 1.3 | 0.9 | 0.9 | 0.40 |
| YGR139W | 1.3 | 3.7 | 0.8 | 0.7 | 1.3 | 0.6 | 0.8 | 0.6 | | 0.6 | 0.9 | 0.5 | 0.4 | 0.9 | 2.3 | 0.8 | 1.0 | 0.8 | 0.22 |
| YOR253W | 0.9 | 2.8 | 1.0 | 1.4 | 0.8 | 0.5 | 1.3 | 1.2 | 0.4 | 1.0 | 0.9 | 0.7 | 0.5 | 1.3 | 1.1 | 0.9 | 0.9 | 0.9 | 1.01 |
| YOL026C | 1.2 | 2.7 | 0.7 | 1.4 | 1.9 | 1.0 | 1.8 | 1.1 | 1.2 | 1.1 | 1.2 | 0.9 | 1.2 | 1.3 | 0.9 | 2.4 | 2.1 | 1.4 | 0.65 |

| | | | | | | | | | | | | | | | | | | | |
|---|---|---|---|---|---|---|---|---|---|---|---|---|---|---|---|---|---|---|---|
| YDR278C | 1.0 | 3.2 | 0.9 | 1.0 | 1.1 | 1.0 | 0.7 | 0.5 | 0.6 | 0.7 | 0.4 | 0.6 | 0.7 | 0.9 | 0.7 | 0.5 | 0.7 | 0.8 | 0.78 |
| YHR095W | 1.1 | 2.6 | 1.5 | 2.1 | 1.1 | 1.0 | 0.8 | 1.0 | 1.5 | 1.2 | 1.3 | 0.6 | 0.7 | 0.9 | 0.8 | 0.9 | 0.8 | 0.7 | 0.67 |
| YCL042W | | 2.5 | 7.2 | | 1.1 | 0.6 | | 0.6 | 0.7 | 2.2 | 3.2 | | 7.5 | | | | 0.8 | | 1.94 |
| YNL200C | 1.0 | 3.0 | 3.6 | 2.0 | 1.0 | 0.4 | 1.2 | 0.7 | 1.9 | 1.2 | 3.1 | 0.9 | 1.1 | 0.6 | 0.8 | 0.9 | 1.2 | 0.9 | 1.40 |
| YPL221W | 0.9 | 3.0 | 1.0 | 1.8 | 1.4 | 1.1 | 1.3 | 0.9 | 0.3 | 1.3 | 1.0 | 0.8 | 1.0 | 0.8 | 1.0 | 1.2 | 1.5 | 1.2 | 1.51 |
| YLR415C | 1.1 | 2.4 | 6.7 | | 0.9 | 0.3 | 1.2 | 0.9 | | 0.3 | 0.8 | 0.8 | 1.0 | 0.8 | 1.0 | 1.9 | 0.8 | 1.0 | 0.19 |
| YOR325W | 1.0 | 2.6 | 1.5 | 0.3 | 0.9 | 0.3 | 1.2 | 0.9 | | | 0.6 | 0.9 | 0.6 | 1.3 | 0.7 | 1.4 | 0.8 | 1.0 | 0.19 |
| YGL088W | 0.9 | 2.1 | 0.9 | 1.1 | 0.6 | 0.8 | 1.0 | 0.5 | 0.5 | 0.8 | 0.8 | 0.6 | 0.7 | 1.4 | 0.4 | 0.3 | 0.5 | 0.7 | 1.80 |
| YDR090C | 0.9 | 3.0 | 0.8 | 1.2 | 1.8 | 1.4 | 1.5 | 0.8 | 1.0 | 1.5 | 0.7 | 0.6 | 0.8 | 0.9 | 1.1 | 0.9 | 1.1 | 1.1 | 0.79 |
| YMR071C | 1.0 | 2.0 | 1.1 | 1.9 | 1.4 | 1.2 | 1.8 | 1.1 | 1.6 | 1.8 | 1.2 | 0.7 | 1.3 | 1.2 | 0.8 | 1.4 | 1.2 | 1.9 | 2.39 |
| YGR293C | 0.5 | 1.9 | 1.6 | 0.0 | 1.1 | 0.7 | 1.3 | 1.3 | | 1.1 | 0.6 | 1.0 | 0.7 | 1.0 | 0.7 | 0.9 | 0.8 | 0.9 | 0.23 |
| YJL017W | 1.1 | 1.7 | 3.5 | 1.1 | 1.3 | 1.2 | 0.2 | 1.0 | 0.8 | 1.4 | 1.4 | 1.3 | 1.5 | 1.0 | 1.1 | 0.6 | 1.0 | 1.0 | 0.85 |
| YIL127C | 1.1 | 4.8 | 0.2 | 1.2 | 1.0 | 0.4 | 0.6 | 1.2 | 0.3 | 0.5 | 0.9 | 0.7 | 0.4 | 1.3 | 0.7 | 0.2 | 0.7 | 0.6 | 1.57 |
| YDR281C | 2.1 | 2.6 | 0.4 | 2.1 | 1.3 | 1.5 | 0.6 | 1.2 | 0.2 | 0.5 | 0.8 | 0.9 | 0.7 | 1.2 | 0.3 | 0.6 | 1.5 | 1.2 | 1.56 |
| YDR366C | 0.9 | 2.0 | 0.8 | 0.9 | 1.0 | 1.5 | 1.5 | 1.1 | 1.0 | 1.5 | 1.7 | 0.6 | 0.8 | 1.4 | 1.4 | 1.0 | 1.2 | 0.9 | 1.14 |
| YFR026C | 0.9 | 2.0 | 1.0 | 0.8 | 1.0 | 1.2 | 1.3 | 1.3 | 1.8 | 0.6 | 0.8 | 0.6 | 1.1 | 1.0 | 2.3 | 2.2 | 1.1 | 1.1 | 0.39 |
| YAR047C | 1.1 | 1.7 | 1.5 | 0.7 | 1.1 | 1.3 | 1.2 | 0.8 | 0.5 | 2.3 | 1.1 | 0.9 | 1.4 | 1.3 | 0.5 | 1.0 | 1.7 | 1.0 | 0.35 |
| YHL006C | 0.7 | 1.4 | 0.9 | 0.6 | 1.1 | 1.1 | 1.2 | 1.1 | 0.9 | 0.9 | 0.7 | 1.0 | 1.1 | 1.1 | 0.6 | 0.9 | 1.1 | 1.0 | 0.37 |
| YPL225W | 1.3 | 1.7 | 1.0 | 1.6 | 1.1 | 1.8 | 1.7 | 1.7 | 1.0 | 1.6 | 0.9 | 0.7 | 0.8 | 1.4 | 0.9 | 1.8 | 1.0 | 1.7 | 2.87 |
| YBR124W | 0.9 | 1.4 | 1.5 | 0.4 | 1.0 | 0.6 | 1.1 | 1.2 | 1.1 | 0.7 | 0.4 | 0.9 | 0.9 | 1.0 | 1.0 | 0.8 | 0.9 | 0.9 | 0.29 |
| YBL044W | 0.9 | 2.7 | 2.1 | 0.8 | 1.2 | 0.7 | 1.1 | 0.7 | 0.2 | 0.5 | 0.6 | 0.9 | 0.7 | 0.9 | 0.7 | 0.6 | 0.8 | 0.8 | 0.39 |
| YCL056C | 1.3 | 1.4 | 0.7 | 1.0 | 1.5 | 1.4 | 1.6 | 1.0 | 1.5 | 1.6 | 1.5 | 1.0 | 1.3 | 2.2 | 1.7 | 2.3 | 1.2 | 1.5 | 0.82 |
| YCR007C | 2.2 | 1.6 | 0.9 | 2.9 | 1.5 | 1.4 | 2.0 | 1.0 | 1.4 | 2.6 | 1.1 | 0.8 | 1.5 | 1.7 | 0.8 | 1.7 | 2.6 | 2.1 | 0.39 |
| YPR146C | 0.8 | 1.6 | 1.3 | 0.9 | 1.2 | 1.1 | 1.0 | 0.8 | 0.9 | 1.2 | 1.4 | 0.8 | 1.3 | 0.9 | 0.9 | 1.3 | 1.0 | 1.1 | 0.80 |
| YKL097C | 0.9 | 1.6 | 0.6 | 1.4 | 1.8 | 0.7 | 0.8 | 0.4 | 0.1 | | 0.6 | 0.6 | 0.9 | 1.0 | 0.8 | 0.5 | 1.1 | 0.5 | 0.21 |
| YBR066C | 1.9 | 1.3 | 1.6 | 1.6 | 1.3 | 1.3 | 1.3 | 1.2 | 0.3 | 0.9 | 1.2 | 0.7 | 0.6 | 1.1 | 0.4 | 3.2 | 1.2 | 1.2 | 0.83 |
| YLR338W | 1.1 | 1.6 | 1.4 | 1.1 | 0.9 | 1.1 | 0.9 | 0.8 | 7.9 | 0.5 | 0.9 | 0.6 | 1.2 | 0.8 | 1.5 | 0.9 | 0.8 | 0.6 | 0.33 |
| YBR162C | 0.6 | 0.5 | 3.1 | 0.8 | 1.3 | 0.6 | 0.3 | 0.8 | 0.1 | 0.4 | 0.8 | 0.7 | 0.7 | 0.5 | 1.9 | 0.4 | 1.2 | 1.3 | 3.96 |
| YDL046W | 1.2 | 1.4 | 5.1 | 1.3 | 0.9 | 1.8 | 1.2 | 0.7 | 1.6 | 2.3 | 2.6 | 1.0 | 1.5 | 1.2 | 2.3 | 2.6 | 1.2 | 1.6 | 1.94 |
| YDR133C | 0.9 | 1.1 | 2.9 | 2.0 | 1.2 | 0.7 | 0.2 | 0.5 | 0.1 | 0.4 | 1.3 | 0.3 | 0.1 | 0.6 | 0.6 | 1.1 | 0.6 | 0.9 | 4.02 |
| YGR038W | 0.9 | 1.2 | 3.5 | 0.8 | 1.3 | 1.3 | 1.1 | 0.7 | 1.4 | 1.0 | 1.4 | 1.0 | 1.2 | 1.4 | 1.1 | 1.3 | 1.1 | 1.1 | 1.27 |
| YGR243W | 1.4 | 2.2 | 9.4 | 5.0 | 1.0 | 1.5 | 1.9 | 1.3 | 0.7 | 3.0 | 1.7 | 1.2 | 1.1 | 1.9 | 0.8 | 2.8 | 2.1 | 4.4 | 1.11 |

| | | | | | | | | | | | | | | | | | | | |
|---|---|---|---|---|---|---|---|---|---|---|---|---|---|---|---|---|---|---|---|
| YHR105W | 0.9 | 0.8 | 2.4 | 6.3 | 1.5 | 0.2 | 0.9 | 1.0 | 1.1 | 3.4 | 0.7 | 0.7 | 1.4 | 1.2 | 0.9 | 1.8 | 1.0 | 0.9 | 0.32 |
| YHR181W | 0.9 | 0.7 | 2.4 | 1.4 | 1.0 | 0.9 | 0.8 | 0.9 | 0.5 | 1.3 | 1.3 | 0.8 | 0.7 | 1.6 | 1.4 | 1.5 | 0.9 | 0.9 | 1.40 |
| YJL097W | 1.0 | 1.1 | 3.7 | 1.6 | 0.9 | 1.6 | 1.0 | 0.8 | 0.3 | 0.7 | 1.5 | 1.0 | 0.7 | 1.0 | 1.1 | 1.2 | 1.2 | 1.1 | 1.69 |
| YKL051W | 1.0 | 1.1 | 4.7 | 0.8 | 0.8 | 1.0 | 0.6 | 0.8 | 1.3 | 1.1 | 1.8 | 0.8 | 1.2 | 0.7 | 1.5 | 0.9 | 1.5 | 1.4 | 1.07 |
| YKL100C | 0.7 | 1.1 | 5.0 | 1.6 | 0.6 | 1.8 | 1.2 | 0.7 | 1.3 | 1.3 | 1.6 | 0.9 | 1.3 | 1.0 | 1.7 | 2.0 | 0.7 | 1.5 | 1.14 |
| YLR339C | 0.7 | 0.8 | 3.1 | 0.8 | 1.3 | 0.4 | 0.4 | 0.5 | | 0.4 | 1.6 | 0.6 | 0.1 | 0.5 | 1.0 | 0.5 | 0.7 | 0.6 | 1.37 |
| YOL030W | 1.0 | 0.8 | 4.4 | 1.0 | 0.9 | 0.9 | 1.3 | 0.6 | 1.2 | 0.6 | 1.3 | 0.9 | 1.7 | 0.6 | 0.9 | 0.7 | 0.9 | 1.0 | 1.93 |
| YPR150W | 1.3 | 1.1 | 4.3 | 3.0 | 1.3 | 0.9 | 0.9 | 0.8 | 1.2 | 3.3 | 0.9 | 0.6 | 1.4 | 1.4 | 0.9 | 1.8 | 0.9 | 1.0 | 0.31 |
| YBL100C | 0.8 | 1.8 | 1.6 | 0.5 | 0.6 | 0.4 | 1.1 | 0.6 | 0.2 | 0.8 | 0.9 | 0.7 | 0.9 | 0.9 | 1.0 | 0.5 | 0.8 | 0.8 | 0.58 |
| YBR096W | 1.1 | 1.5 | 2.8 | 1.6 | 0.9 | 1.7 | 1.9 | 1.0 | 1.2 | 1.6 | 1.2 | 0.9 | 1.9 | 1.7 | 1.1 | 1.1 | 1.1 | 1.5 | 1.43 |
| YBR100W | 1.0 | 1.5 | 4.8 | 1.1 | 0.9 | 0.5 | 1.0 | 0.9 | 8.5 | 3.0 | 0.2 | 0.7 | 1.2 | 1.2 | 1.3 | 1.2 | 0.8 | 0.9 | 0.35 |
| YCL058C | 1.1 | 1.6 | 2.2 | 1.2 | 1.7 | 1.1 | 0.8 | 0.9 | 0.6 | 1.0 | 1.0 | 1.0 | 0.9 | 1.9 | 1.2 | 0.8 | 1.4 | 0.9 | 0.72 |
| YCR030C | 1.1 | 0.9 | 3.3 | 1.0 | 1.0 | 1.2 | 1.0 | 0.7 | 0.9 | 2.0 | 1.1 | 0.9 | 1.7 | 0.8 | 1.3 | 1.0 | 1.2 | 1.0 | 0.63 |
| YDL015C | 0.9 | 0.7 | 3.6 | 1.1 | 1.2 | 2.5 | 1.1 | 0.9 | 0.7 | 1.1 | 0.8 | 1.0 | 0.9 | 1.1 | 1.1 | 2.1 | 1.0 | 1.5 | 2.99 |
| YDL023C | 0.8 | 1.6 | 2.4 | 1.1 | 0.6 | 0.3 | 1.5 | 0.7 | 0.7 | 1.5 | 3.5 | 0.9 | 1.4 | 0.7 | 0.6 | 0.9 | 0.8 | 1.1 | 0.78 |
| YDL086W | 0.9 | 1.2 | 1.9 | 0.9 | 1.3 | 0.9 | 0.8 | 0.8 | 1.3 | 1.1 | 1.4 | 0.8 | 1.7 | 0.9 | 0.9 | 1.1 | 0.8 | 0.8 | 0.80 |
| YDR233C | 1.1 | 1.1 | 3.2 | 1.9 | 0.7 | 0.8 | 1.2 | 1.1 | 0.2 | 0.7 | 3.7 | 1.3 | 0.5 | 0.9 | 1.5 | 2.8 | 0.9 | 0.9 | 3.47 |
| YDR359C | 0.8 | 0.7 | 1.7 | 1.9 | 1.3 | 0.8 | 1.0 | 0.8 | 0.7 | 1.0 | 0.5 | 1.0 | 1.1 | 1.3 | 1.4 | 0.9 | 0.8 | 0.9 | 0.34 |
| YEL033W | 0.9 | 1.2 | 2.7 | 1.1 | 1.0 | 0.7 | 0.3 | 0.7 | 0.6 | 0.4 | 1.2 | 0.6 | 0.4 | 1.0 | 0.6 | 0.9 | 0.9 | 0.9 | 2.96 |
| YGR022C | 1.3 | 1.1 | 1.7 | 2.2 | 1.1 | 1.3 | 0.9 | 0.9 | 0.2 | | 0.4 | 0.7 | 1.2 | 1.1 | 0.7 | 0.6 | 0.9 | 0.8 | 0.50 |
| YGR026W | 0.8 | 1.1 | 2.9 | 0.9 | 0.9 | 1.5 | 0.9 | 0.9 | 0.8 | 0.7 | 1.3 | 0.5 | 0.6 | 1.0 | 1.2 | 1.4 | 1.0 | 1.2 | 2.19 |
| YGR107W | 1.0 | 0.8 | 1.7 | 0.9 | 1.1 | 0.8 | 1.0 | 0.9 | 0.1 | 0.0 | 0.6 | 0.6 | 0.9 | 1.0 | 1.1 | 0.8 | 1.2 | 0.8 | 0.43 |
| YHL005C | 0.9 | 2.7 | 2.7 | 0.2 | 1.2 | 1.1 | 0.8 | 0.9 | | 0.9 | | 0.7 | 0.7 | 1.0 | 0.7 | 0.7 | 1.0 | 0.8 | 0.33 |
| YHR126C | 1.1 | 0.9 | 2.8 | 0.6 | 1.0 | 1.2 | 1.1 | 0.6 | 0.4 | 0.7 | 1.9 | 1.0 | 0.8 | 0.8 | 1.4 | 1.7 | 1.1 | 1.3 | 1.31 |
| YHR143W | 0.5 | 0.9 | 4.9 | 1.0 | 1.2 | 2.1 | 0.5 | 1.5 | 0.2 | 0.7 | 1.2 | 0.5 | 0.5 | 0.8 | 0.3 | 0.9 | 0.9 | 0.7 | 3.58 |
| YIL157C | 1.1 | 1.3 | 2.1 | 1.7 | 0.8 | 1.3 | 1.2 | 1.0 | 0.9 | 0.9 | 1.3 | 0.8 | 1.0 | 1.0 | 0.5 | 1.8 | 1.1 | 1.5 | 1.46 |
| YIR041W | 1.6 | 1.0 | 2.4 | 1.4 | 1.3 | 1.0 | 1.6 | 0.9 | 1.5 | 2.5 | 1.2 | 1.3 | 1.1 | 1.1 | 1.3 | 1.3 | 1.1 | 0.9 | 0.66 |
| YJL016W | 1.3 | 1.6 | 2.7 | 1.3 | 1.4 | 1.5 | 1.4 | 0.9 | 0.6 | 0.9 | 1.3 | 0.9 | 1.3 | 1.6 | 1.1 | 2.5 | 1.2 | 1.5 | 0.88 |
| YJR018W | 0.8 | 1.2 | 1.8 | 0.7 | 1.1 | 1.0 | 0.9 | 0.7 | 0.4 | 0.7 | 0.9 | 0.8 | 1.5 | 0.9 | 1.4 | 0.8 | 1.0 | 0.7 | 0.35 |
| YKL147C | 1.0 | 1.3 | 1.9 | 0.9 | 0.8 | 0.7 | 0.8 | 0.7 | 1.0 | 0.5 | 0.3 | 0.9 | 1.0 | 1.0 | 0.7 | 1.1 | 0.9 | 0.9 | 0.28 |
| YKL169C | 1.0 | 1.0 | 1.6 | 1.8 | 0.8 | 1.2 | 1.5 | 1.2 | 0.3 | 0.5 | | 0.7 | 1.0 | 1.3 | 0.8 | 2.6 | 0.9 | 1.1 | 0.45 |
| YKR033C | 0.9 | 1.2 | 2.1 | 1.0 | 1.0 | 0.6 | 1.0 | 1.0 | 0.1 | 1.4 | 0.3 | 0.4 | 0.9 | 0.9 | 1.1 | 0.6 | 1.0 | 0.9 | 0.28 |

| | | | | | | | | | | | | | | | | | | | |
|---|---|---|---|---|---|---|---|---|---|---|---|---|---|---|---|---|---|---|---|
| YLL064C | 0.8 | 1.7 | 1.7 | 1.9 | 0.9 | 1.7 | 1.1 | 0.9 | 1.1 | 2.5 | 1.0 | 1.1 | 1.3 | 1.1 | 1.2 | 1.6 | 1.2 | 1.0 | 0.68 |
| YLR041W | 0.8 | 1.2 | 2.2 | 1.5 | 1.6 | 0.5 | 1.2 | 0.6 | | 0.4 | 1.1 | 0.4 | 0.3 | 0.5 | 1.3 | 0.5 | 0.7 | 0.9 | 0.69 |
| YLR177W | 0.8 | 1.2 | 1.8 | 2.2 | 0.8 | 1.1 | 1.3 | 0.9 | 1.0 | 2.3 | 0.9 | 1.1 | 1.5 | 1.0 | 0.5 | 1.3 | 1.1 | 1.5 | 1.25 |
| YMR007W | 1.1 | 0.9 | 2.0 | 0.7 | 1.3 | 1.7 | 3.1 | 0.7 | | 0.1 | 0.9 | 0.8 | 1.3 | 1.2 | 1.8 | 1.1 | 2.5 | 1.0 | 0.30 |
| YMR156C | 1.0 | 0.9 | 2.7 | 1.7 | 1.2 | 1.4 | 1.4 | 0.9 | 0.6 | 1.3 | 1.1 | 0.7 | 1.0 | 1.2 | 0.7 | 1.3 | 1.2 | 1.1 | 0.40 |
| YMR215W | 0.8 | 0.5 | 2.1 | 1.2 | 1.1 | 0.3 | 0.3 | 0.5 | 0.1 | 0.2 | 1.1 | 0.4 | 0.2 | 0.5 | 0.7 | 0.2 | 0.7 | 0.7 | 1.59 |
| YNL195C | 0.9 | 1.1 | 3.5 | 4.7 | 0.9 | 0.8 | 1.1 | 0.9 | 2.6 | 11.7 | 2.9 | 0.6 | 1.6 | 1.5 | 1.4 | 6.4 | 0.8 | 0.9 | 0.32 |
| YOL073C | 0.7 | 0.9 | 2.5 | 0.6 | 1.1 | 1.2 | 1.3 | 0.7 | 1.9 | 1.1 | 1.7 | 0.8 | 1.6 | 0.8 | 1.6 | 1.7 | 1.2 | 1.2 | 0.54 |
| YOR129C | 0.6 | 0.8 | 3.3 | 0.8 | 1.3 | 0.4 | 0.4 | 0.6 | 0.2 | 0.4 | 1.1 | 0.6 | 0.2 | 0.5 | 0.6 | 0.3 | 0.6 | 0.4 | 5.22 |
| YOR161C | 0.6 | 1.0 | 6.0 | 2.2 | 1.1 | 0.9 | 1.2 | 0.8 | 1.0 | 1.7 | 4.6 | 0.5 | 0.7 | 0.9 | 1.4 | 2.0 | 0.7 | 1.2 | 0.82 |
| YPL004C | 0.7 | 1.2 | 3.7 | 1.9 | 1.4 | 1.3 | 2.3 | 0.9 | 1.1 | 1.6 | 1.7 | 0.9 | 1.3 | 1.0 | 1.1 | 2.0 | 1.2 | 2.2 | 4.52 |
| YPL246C | 0.7 | 0.8 | 2.6 | 0.9 | 1.4 | 1.3 | 0.8 | 0.6 | 0.4 | 0.5 | 1.1 | 0.8 | 0.9 | 0.7 | 1.2 | 1.1 | 1.2 | 0.9 | 0.98 |
| YPL272C | 1.1 | 0.9 | 2.2 | 0.6 | 1.3 | 1.1 | 0.7 | 0.8 | 1.2 | 2.5 | 1.1 | 0.7 | 1.1 | 1.1 | 0.6 | 2.5 | 0.9 | 1.2 | 0.25 |
| YPR063C | 1.0 | 0.8 | 2.6 | 0.8 | 1.2 | 1.0 | 0.6 | 0.7 | 0.5 | 0.6 | 1.0 | 0.7 | 0.8 | 1.0 | 1.0 | 1.2 | 1.0 | 0.9 | 1.41 |
| YDL129W | 1.0 | 0.7 | 1.0 | 2.3 | 1.3 | 0.9 | 0.9 | 1.0 | 0.5 | 0.9 | 0.6 | 0.9 | 0.5 | 0.8 | 0.5 | 1.4 | 0.6 | 0.8 | 0.69 |
| YDR066C | 1.3 | 1.5 | 0.9 | 2.2 | 1.0 | 1.4 | 1.1 | 1.3 | 1.5 | 1.1 | 0.6 | 0.8 | 1.4 | 1.3 | 0.9 | 1.4 | 1.1 | 1.1 | 0.57 |
| YGL059W | 1.3 | 1.5 | 0.9 | 3.1 | 0.7 | 0.7 | 1.0 | 1.4 | 1.6 | 1.7 | 1.4 | 1.0 | 2.0 | 1.2 | 1.6 | 1.7 | 1.4 | 1.4 | 0.49 |
| YNL144C | 1.8 | 0.8 | 1.2 | 4.5 | 0.9 | 1.1 | 1.1 | 1.1 | 0.7 | 1.9 | 1.7 | 1.4 | 1.0 | 1.1 | 0.1 | 1.7 | 2.4 | 2.8 | 0.51 |
| YAL037W | 1.6 | 0.9 | 1.1 | 3.5 | 1.2 | 1.1 | 1.0 | 1.3 | 0.6 | 1.9 | 0.5 | 0.8 | 1.1 | 1.2 | 0.8 | 0.6 | 0.9 | 0.9 | 0.55 |
| YAR023C | 1.1 | 1.0 | 1.7 | 2.1 | 1.3 | 1.0 | 1.1 | 0.8 | 0.9 | 1.4 | 1.0 | 1.2 | 1.1 | 1.0 | 3.1 | 1.6 | 0.9 | 1.1 | 0.62 |
| YCR015C | 1.1 | 0.9 | 0.5 | 2.1 | 1.7 | 1.0 | 1.5 | 1.2 | 0.5 | 1.0 | 1.1 | 0.9 | 0.6 | 1.2 | 0.9 | 1.1 | 1.1 | 1.2 | 0.58 |
| YCR043C | 1.4 | 0.5 | 0.8 | 2.3 | 0.9 | 0.8 | 0.7 | 1.2 | 0.3 | 0.7 | 1.0 | 0.6 | 0.8 | 1.6 | 1.2 | 0.8 | 1.0 | 1.1 | 1.25 |
| YDL146W | 1.0 | 1.2 | 1.2 | 2.0 | 0.7 | 0.7 | 1.4 | 0.9 | 0.8 | 2.3 | 1.4 | 1.0 | 1.4 | 1.0 | 1.7 | 1.8 | 1.0 | 1.2 | 0.62 |
| YDR057W | 0.9 | 1.4 | 1.6 | 2.6 | 0.8 | 0.7 | 1.4 | 1.2 | 1.5 | 1.1 | 1.0 | 0.7 | 0.9 | 1.2 | 1.1 | 1.7 | 0.7 | 1.1 | 0.70 |
| YDR222W | 1.0 | 1.1 | 1.7 | 1.7 | 1.4 | 1.3 | 0.5 | 1.0 | 0.6 | 1.0 | 1.0 | 0.5 | 0.8 | 1.3 | 0.5 | 0.6 | 0.7 | 0.8 | 0.52 |
| YDR286C | 1.6 | 0.9 | 1.1 | 2.1 | 1.0 | 1.7 | 1.3 | 1.9 | 0.8 | 1.2 | 1.1 | 0.9 | 1.4 | 1.3 | 0.8 | 2.1 | 1.1 | 1.4 | 0.90 |
| YDR438W | 1.2 | 1.8 | 0.8 | 2.1 | 1.6 | 1.3 | 1.4 | 1.0 | 1.1 | 0.8 | 1.1 | 0.8 | 1.2 | 1.3 | 1.0 | 1.2 | 1.6 | 1.2 | 0.48 |
| YDR479C | 1.1 | 1.8 | 1.0 | 2.3 | 0.9 | 1.6 | 1.2 | 0.9 | 1.2 | 1.6 | 1.0 | 1.0 | 1.2 | 1.4 | 1.6 | 2.1 | 1.1 | 1.5 | 0.51 |
| YEL057C | 1.1 | 0.6 | 1.3 | 2.4 | 1.0 | 0.9 | 1.1 | 1.2 | 1.2 | 1.9 | 0.7 | 0.8 | 1.5 | 1.5 | 1.0 | 1.8 | 0.9 | 1.0 | 0.44 |
| YEL073C | 1.6 | 0.8 | 1.0 | 2.8 | 1.2 | 1.5 | 0.8 | 0.9 | 0.7 | 1.2 | 1.1 | 0.5 | 1.0 | 1.1 | 2.8 | 1.0 | 1.3 | 0.8 | 0.38 |
| YER084W | 0.8 | 1.1 | 1.0 | 3.9 | 0.9 | 0.6 | 0.8 | 0.9 | | 0.8 | | 0.8 | 0.7 | 0.9 | 2.6 | 1.5 | 0.8 | 0.9 | 0.30 |
| YER121W | 1.1 | 1.2 | 0.7 | 7.7 | 1.3 | 2.5 | 1.3 | 1.3 | 0.6 | 0.5 | 1.2 | 0.7 | 0.6 | 1.3 | 0.9 | 4.6 | 1.2 | 1.8 | 0.73 |

| | | | | | | | | | | | | | | | | | | | |
|---|---|---|---|---|---|---|---|---|---|---|---|---|---|---|---|---|---|---|---|
| YER189W | 0.9 | 0.8 | 0.9 | 1.9 | 1.3 | 0.5 | 0.9 | 0.7 | 0.4 | 0.5 | 0.7 | 0.8 | 0.7 | 0.9 | 0.9 | 0.8 | 0.8 | 0.8 | 0.43 |
| YFL017C | 1.3 | 1.2 | 0.9 | 2.3 | 1.5 | 1.3 | 1.5 | 1.4 | 0.8 | 0.6 | 1.1 | 1.0 | 0.7 | 1.1 | 0.6 | 1.8 | 1.5 | 1.8 | 1.21 |
| YFL046W | 1.3 | 0.9 | 0.7 | 2.1 | 1.7 | 0.7 | 1.3 | 1.3 | 0.7 | 0.7 | 1.3 | 0.8 | 0.7 | 1.3 | 1.0 | 1.1 | 1.4 | 1.6 | 0.68 |
| YFR008W | 1.1 | 1.0 | 0.8 | 2.2 | 1.1 | 1.0 | 1.1 | 0.9 | 1.5 | 1.4 | 0.7 | 0.9 | 0.8 | 1.1 | 0.8 | 1.5 | 1.0 | 1.0 | 1.26 |
| YGL214W | 0.9 | 1.0 | 1.2 | 2.6 | 1.2 | 0.9 | 0.8 | 0.9 | 0.4 | 0.7 | 0.4 | 0.8 | 0.6 | 1.1 | 0.9 | 0.8 | 1.0 | 0.8 | 0.61 |
| YGL218W | 1.4 | 0.9 | 1.4 | 3.4 | 1.2 | 1.1 | 0.8 | 1.1 | 1.3 | 0.4 | 0.1 | 0.8 | 1.3 | 0.9 | 1.1 | 0.7 | 1.0 | 1.0 | 0.53 |
| YGR021W | 0.9 | 1.5 | 1.0 | 8.6 | 1.0 | 0.9 | 1.3 | 1.4 | 0.7 | 0.6 | 0.6 | 0.9 | 0.9 | 1.2 | 0.8 | 2.1 | 1.0 | 1.0 | 0.58 |
| YGR024C | 1.2 | 0.9 | 0.7 | 2.6 | 1.0 | 0.7 | 1.3 | 1.4 | 0.4 | 1.2 | 1.0 | 0.7 | 0.5 | 1.4 | 1.0 | 1.2 | 0.8 | 1.3 | 1.61 |
| YGR064W | 1.1 | 1.2 | 1.6 | 2.3 | 0.7 | 0.6 | 1.1 | 1.0 | 0.1 | 0.8 | 1.7 | 0.8 | 0.9 | 2.1 | 1.0 | 1.3 | 0.8 | 0.9 | 0.50 |
| YGR182C | 1.6 | 1.2 | 1.2 | 2.3 | 1.1 | 0.9 | 0.7 | 1.1 | 0.5 | 0.7 | 0.9 | 0.7 | 0.7 | 2.2 | 0.4 | 2.0 | 1.0 | 1.7 | 1.95 |
| YGR236C | 1.6 | 1.6 | 0.4 | 4.1 | 1.2 | 1.2 | 1.0 | 1.2 | 1.5 | 0.7 | 1.0 | 0.9 | 1.2 | 1.2 | 0.7 | 7.9 | 1.5 | 1.9 | 0.30 |
| YHL042W | 1.2 | 0.8 | 1.3 | 51.1 | 1.0 | 0.7 | 1.2 | 1.0 | 0.8 | 1.8 | 0.7 | 0.6 | 1.3 | 1.2 | 1.1 | 1.9 | 1.1 | 0.9 | 0.37 |
| YIL012W | 1.2 | 5.8 | 1.5 | 2.8 | 1.5 | 1.5 | 0.9 | 0.9 | 1.7 | 2.8 | 0.9 | 0.6 | 0.4 | 1.1 | 1.1 | 0.9 | 1.0 | 0.9 | 0.28 |
| YIL028W | 1.0 | 1.1 | 0.8 | 5.4 | 1.4 | 0.7 | 1.1 | 0.8 | 0.8 | 1.7 | 1.1 | 0.5 | 1.0 | 1.1 | 0.9 | 1.1 | 1.3 | 1.0 | 0.27 |
| YIL057C | 1.3 | 1.8 | 1.1 | 4.6 | 1.3 | 1.2 | 1.2 | 1.4 | 0.1 | 5.0 | 0.8 | 0.6 | 0.9 | 1.5 | 0.8 | 11.1 | 1.1 | 3.6 | 0.26 |
| YIL089W | 1.3 | 1.3 | 0.7 | 3.9 | 1.6 | 1.6 | 1.6 | 1.1 | | 0.6 | 0.7 | 0.6 | 0.7 | 1.4 | 0.6 | 1.3 | 1.4 | 1.3 | 0.31 |
| YIL102C | 1.4 | 1.6 | 1.0 | 6.1 | 1.5 | 0.8 | 1.1 | 0.7 | | 0.3 | | 0.6 | 0.7 | 1.0 | 0.8 | 0.6 | 1.2 | 1.0 | 0.18 |
| YIL113W | 0.9 | 1.0 | 0.9 | 2.6 | 0.6 | 1.0 | 1.9 | 0.7 | 1.9 | 2.2 | 2.5 | 0.8 | 1.3 | 1.2 | 0.9 | 1.6 | 1.4 | 1.3 | 0.48 |
| YIL122W | 1.2 | 1.1 | 1.1 | 2.1 | 0.9 | 0.2 | 0.8 | 0.7 | 0.4 | 0.8 | 2.1 | 1.0 | 0.7 | 1.0 | 1.6 | 1.4 | 0.9 | 0.9 | 0.35 |
| YJL100W | 1.4 | 2.0 | 0.9 | 2.3 | 1.3 | 1.1 | 1.2 | 1.2 | 1.8 | 2.7 | 1.5 | 0.9 | 1.4 | 1.2 | 1.2 | 1.4 | 1.5 | 1.1 | 0.38 |
| YJL169W | 0.8 | 1.1 | 1.5 | 2.8 | 1.4 | 0.8 | 0.9 | 0.7 | 0.3 | 0.5 | 0.4 | 0.5 | 0.7 | 1.0 | 0.9 | 0.6 | 1.1 | 0.9 | 0.35 |
| YJL199C | 1.1 | 0.7 | 0.9 | 4.5 | 1.0 | 1.7 | 1.0 | 1.0 | 1.0 | 1.6 | 1.0 | 0.8 | 0.9 | 1.9 | 0.9 | 3.7 | 1.0 | 1.0 | 0.59 |
| YJR039W | 0.9 | 0.6 | 0.8 | 3.0 | 1.8 | 1.0 | 1.6 | 1.2 | 1.8 | 1.6 | 1.8 | 0.7 | 1.5 | 0.6 | 1.2 | 1.5 | 1.3 | 1.3 | 0.24 |
| YJR101W | 0.8 | 0.6 | 0.7 | 2.9 | 1.8 | 0.9 | 1.5 | 1.1 | 0.4 | 0.8 | 1.2 | 0.5 | 0.5 | 0.9 | 0.5 | 1.2 | 1.0 | 1.1 | 2.04 |
| YKL061W | 1.3 | 0.7 | 0.7 | 1.8 | 0.8 | 1.0 | 0.9 | 1.2 | 0.8 | 1.0 | 1.1 | 0.7 | 0.8 | 1.2 | 0.6 | 1.5 | 1.3 | 1.6 | 0.92 |
| YKL121W | 0.9 | 7.8 | 0.8 | 11.8 | 1.6 | 1.2 | 1.7 | 1.0 | 0.8 | 0.9 | 1.6 | 0.8 | 1.0 | 1.2 | 0.8 | 1.6 | 1.6 | 1.1 | 0.31 |
| YKL160W | 1.5 | 1.0 | 0.4 | 1.8 | 1.1 | 1.5 | 1.5 | 2.1 | 1.3 | 1.9 | 1.4 | 0.9 | 1.6 | 1.5 | 0.8 | 1.6 | 1.5 | 2.2 | 2.19 |
| YLR016C | 0.7 | 1.3 | 0.5 | 2.0 | 1.5 | 0.8 | 1.1 | 1.2 | 0.8 | 1.1 | 1.0 | 0.7 | 0.8 | 0.9 | 0.7 | 1.3 | 1.0 | 1.3 | 1.15 |
| YLR030W | 1.4 | 1.5 | 0.8 | 2.5 | 1.2 | 1.5 | 1.5 | 0.9 | 0.3 | 2.7 | 0.2 | 0.8 | 1.5 | 1.2 | 0.6 | 2.1 | 1.0 | 1.0 | 0.29 |
| YLR036C | 1.3 | 0.8 | 0.7 | 2.0 | 1.5 | 1.3 | 1.7 | 1.2 | 0.4 | 1.6 | 0.8 | 0.7 | 0.7 | 1.3 | 0.6 | 1.4 | 1.0 | 1.4 | 1.07 |
| YLR112W | 1.1 | 0.9 | 1.1 | 1.7 | 1.3 | 1.3 | 1.0 | 0.7 | | 0.7 | 0.5 | 0.5 | 0.7 | 0.8 | 0.4 | 0.4 | 1.0 | 0.8 | 0.39 |
| YLR125W | 1.4 | 2.0 | 0.5 | 3.1 | 1.8 | 0.7 | 1.3 | 1.2 | 0.5 | 1.7 | 1.1 | 0.5 | 0.8 | 1.3 | 0.7 | 1.6 | 1.2 | 1.2 | 0.29 |

| | | | | | | | | | | | | | | | | | | | |
|---|---|---|---|---|---|---|---|---|---|---|---|---|---|---|---|---|---|---|---|
| YLR128W | 1.3 | 1.4 | 0.8 | 1.8 | | 1.3 | 1.3 | 1.1 | 0.8 | 1.7 | 1.4 | 0.7 | 1.2 | 1.4 | 0.9 | 1.5 | 1.2 | 1.2 | 0.25 |
| YLR204W | 1.1 | 0.9 | 0.5 | 2.1 | 2.0 | 1.5 | 2.0 | 1.3 | 1.4 | 1.6 | 1.6 | 0.8 | 1.1 | 1.8 | 1.0 | 2.5 | 1.2 | 1.3 | 1.26 |
| YLR211C | 1.3 | 1.0 | 0.8 | 7.7 | 1.7 | 1.2 | 1.8 | 1.1 | 0.9 | 1.2 | 0.8 | 0.7 | 1.1 | 1.2 | 3.6 | 1.9 | 1.3 | 1.3 | 0.35 |
| YLR257W | 1.5 | 1.0 | 0.7 | 2.4 | 1.4 | 1.2 | 0.8 | 1.3 | 1.0 | 1.1 | 1.2 | 1.0 | 1.0 | 0.9 | 1.0 | 1.7 | 1.8 | 2.6 | 5.20 |
| YLR326W | 1.2 | 1.0 | 1.5 | 2.2 | 1.3 | 0.4 | 1.2 | 0.9 | 0.7 | 1.5 | 6.6 | 0.5 | 0.4 | 1.1 | 0.7 | 0.9 | 0.8 | 1.0 | 0.33 |
| YLR334C | 1.2 | 1.1 | 1.1 | 2.3 | 1.8 | 1.0 | 1.4 | 0.9 | 1.0 | 0.8 | 0.8 | 0.8 | 0.9 | 2.2 | 2.5 | 0.7 | 0.8 | 1.0 | 0.30 |
| YLR408C | 1.3 | 2.3 | 0.6 | 2.3 | 1.4 | 1.0 | 1.0 | 1.4 | 0.9 | 1.1 | 0.7 | 0.8 | 1.1 | 2.1 | 0.9 | 1.1 | 1.2 | 1.2 | 0.79 |
| YLR414C | 1.5 | 1.6 | 1.4 | 4.8 | 1.3 | 0.6 | 0.7 | 0.9 | 0.2 | 1.2 | 1.2 | 0.6 | 1.0 | 1.0 | 2.1 | 0.9 | 3.9 | 3.6 | 2.21 |
| YLR444C | 0.9 | 0.8 | 1.7 | 5.9 | 0.7 | 0.7 | 0.9 | 0.9 | 0.2 | 0.2 | 1.1 | 0.6 | 0.5 | 1.1 | 1.3 | 0.7 | 0.9 | 0.9 | 0.50 |
| YML050W | 1.5 | 1.2 | 1.3 | 2.1 | 0.8 | 0.5 | 0.9 | 1.1 | 1.3 | 1.6 | 1.2 | 0.8 | 0.9 | 1.9 | 1.0 | 1.5 | 0.8 | 1.1 | 0.42 |
| YML107C | 1.1 | 0.7 | 0.4 | 1.8 | 2.2 | 1.1 | 1.5 | 1.3 | 0.6 | 0.9 | 0.9 | 0.7 | 0.6 | 1.3 | 1.0 | 1.3 | 1.2 | 1.5 | 0.67 |
| YMR031C | 0.7 | 0.8 | 1.2 | 2.1 | 0.6 | 0.7 | 0.8 | 0.9 | 1.5 | 1.1 | 0.8 | 0.7 | 1.0 | 1.0 | 0.7 | 1.6 | 0.9 | 1.3 | 1.26 |
| YMR204C | 1.1 | 1.9 | 1.3 | 2.3 | 1.0 | 0.6 | 0.7 | 1.0 | 1.0 | 2.2 | 0.7 | 0.7 | 1.1 | 1.2 | 1.5 | 1.1 | 0.9 | 1.1 | 0.31 |
| YMR206W | 1.4 | 1.4 | 2.3 | 3.9 | 1.1 | 1.0 | 1.3 | 0.9 | 1.0 | 9.0 | 1.8 | 1.0 | 1.2 | 1.4 | 0.3 | 2.2 | 1.0 | 1.2 | 0.17 |
| YNL010W | 1.5 | 1.0 | 0.7 | 1.9 | 1.0 | 1.2 | 1.1 | 1.8 | 0.5 | 0.6 | 1.1 | 0.9 | 0.7 | 1.2 | 0.9 | 1.2 | 1.0 | 1.7 | 4.53 |
| YNL127W | 1.1 | 1.2 | 0.7 | 3.6 | 0.7 | 1.2 | 0.8 | 1.0 | 2.7 | 2.2 | 1.0 | 1.0 | 1.7 | 1.0 | 1.5 | 1.4 | 0.9 | 1.4 | 0.36 |
| YNL217W | 1.1 | 1.2 | 0.5 | 1.7 | 2.1 | 1.5 | 1.3 | 1.4 | 0.3 | 0.9 | 0.7 | 1.0 | 1.0 | 0.9 | 0.5 | 1.0 | 1.3 | 2.1 | 2.35 |
| YOL118C | 2.6 | 2.2 | 1.3 | 2.2 | | 1.5 | 3.2 | 1.1 | | 3.2 | 1.4 | 0.6 | 1.7 | 3.0 | 1.0 | 0.5 | 0.9 | 1.0 | 0.24 |
| YOR053W | 1.0 | 1.1 | 0.5 | 1.7 | 1.7 | 0.9 | 1.6 | 0.9 | 1.3 | 0.8 | 1.3 | 0.6 | 0.9 | 1.2 | 1.0 | 1.3 | 1.2 | 1.2 | 0.77 |
| YOR352W | 1.3 | 0.9 | 1.0 | 2.2 | 1.6 | 0.9 | 1.8 | 1.1 | 1.7 | 2.0 | 1.1 | 1.0 | 1.2 | 1.3 | 0.7 | 2.5 | 1.3 | 1.7 | 0.59 |
| YOR394W | 1.2 | 0.8 | 2.2 | 1.6 | 1.6 | 1.7 | 1.8 | 1.0 | 1.1 | 2.3 | 1.9 | 0.9 | 1.3 | 0.9 | 0.9 | 1.7 | 1.6 | 1.1 | 0.51 |
| YPL033C | 0.8 | 1.0 | 0.9 | 5.1 | 1.3 | 0.7 | 1.0 | 0.7 | 0.4 | 1.6 | 0.7 | 0.6 | 0.7 | 1.1 | 0.8 | 0.4 | 0.9 | 0.8 | 0.23 |
| YPL066W | 0.9 | 1.0 | 1.1 | 2.0 | 1.3 | 1.3 | 1.3 | 0.9 | 0.5 | 0.8 | 1.0 | 1.1 | 0.9 | 0.7 | 0.7 | 1.2 | 1.3 | 1.3 | 0.77 |
| YPR014C | 1.0 | 0.7 | 1.1 | 7.9 | 0.8 | 0.9 | 0.8 | 0.9 | | 0.9 | 0.5 | 0.7 | 0.7 | 1.0 | 0.9 | 0.8 | 0.9 | 1.1 | 0.29 |
| YBR005W | 1.9 | 2.6 | 1.1 | 2.9 | 0.8 | 0.7 | 1.2 | 0.9 | 0.4 | 1.3 | 0.8 | 1.7 | 1.9 | 0.9 | 1.1 | 1.7 | 2.9 | 4.6 | 1.00 |
| YFL027C | 0.8 | 0.8 | 1.1 | 1.3 | 1.1 | 0.3 | 0.4 | 1.1 | 1.8 | 1.3 | 1.3 | 0.6 | 1.1 | 0.3 | 1.4 | 1.2 | 5.1 | 5.2 | 1.74 |
| YGL080W | 1.3 | 1.5 | 1.1 | 1.3 | 1.5 | 1.4 | 1.5 | 1.2 | 1.1 | 1.2 | 0.8 | 1.0 | 0.7 | 1.6 | 0.9 | 1.7 | 1.3 | 2.9 | 1.70 |
| YMR252C | 1.1 | 1.0 | 1.5 | 1.3 | 1.3 | 1.0 | 1.3 | 1.1 | 0.9 | 1.0 | 1.4 | 0.8 | 1.1 | 1.1 | 1.1 | 2.1 | 1.5 | 2.2 | 0.86 |
| YOR385W | 2.8 | 1.2 | 0.2 | 2.5 | 1.5 | 1.0 | 1.2 | 1.0 | 1.5 | 2.2 | 1.2 | 0.6 | 2.8 | 0.8 | 0.8 | 1.2 | 4.6 | 5.9 | 1.17 |
| YAR033W | 1.0 | 0.8 | 1.5 | 1.1 | 1.1 | 1.4 | 2.1 | 1.2 | 1.3 | 1.6 | 1.5 | 0.9 | 1.5 | 1.3 | 0.9 | 2.0 | 1.1 | 3.0 | 1.15 |
| YBR151W | 0.8 | 0.8 | 1.3 | 1.2 | 1.2 | 1.7 | 1.3 | 0.8 | 0.8 | 0.9 | 0.9 | 0.9 | 1.1 | 0.8 | 1.0 | 3.0 | 1.4 | 2.4 | 1.83 |
| YHR162W | 1.2 | 1.2 | 0.9 | 1.8 | 1.2 | 1.3 | 1.7 | 1.4 | 1.0 | 1.7 | 1.3 | 1.2 | 1.5 | 2.0 | 1.1 | 1.4 | 1.3 | 2.7 | 3.64 |

| | | | | | | | | | | | | | | | | | | | |
|---|---|---|---|---|---|---|---|---|---|---|---|---|---|---|---|---|---|---|---|
| YLR165C | 1.4 | 0.8 | 1.3 | 1.1 | 1.1 | 1.5 | 1.4 | 1.1 | 1.7 | 1.0 | 1.4 | 0.9 | 1.2 | 1.4 | 0.5 | 1.6 | 1.1 | 2.1 | 0.78 |
| YNL157W | 1.4 | 1.1 | 0.7 | 1.5 | 1.9 | 0.9 | 1.3 | 1.1 | 1.7 | 1.4 | 1.3 | 0.9 | 1.6 | 1.5 | 1.0 | 1.0 | 1.9 | 2.3 | 1.41 |
| YOL002C | 0.9 | 0.7 | 0.5 | 1.0 | 2.0 | 0.9 | 0.4 | 0.8 | 0.1 | 0.3 | 0.8 | 1.1 | 0.2 | 0.5 | 0.6 | 1.7 | 1.5 | 2.1 | 5.33 |
| YPL233W | 1.2 | 0.9 | 0.5 | 1.1 | 1.4 | 0.6 | 1.0 | 1.0 | 0.3 | 0.6 | 0.9 | 0.6 | 1.0 | 1.0 | 0.4 | 1.0 | 1.9 | 1.9 | 0.48 |
| YGR149W | 1.4 | 1.3 | 1.8 | 1.9 | 1.0 | 1.7 | 1.8 | 0.8 | 0.6 | 0.6 | 1.8 | 1.2 | 2.6 | 1.0 | 2.4 | 1.3 | 2.0 | 1.7 | 0.71 |
| YNL043C | 1.0 | 4.4 | 1.6 | 1.3 | 0.8 | 0.6 | 0.9 | 0.9 | 0.4 | 0.4 | 0.7 | 0.9 | 2.1 | 1.2 | 1.6 | 0.8 | 2.7 | 1.6 | 0.68 |
| YPL067C | 1.2 | 1.6 | 2.8 | 1.4 | 1.0 | 0.8 | 1.2 | 1.2 | | 0.8 | 0.9 | 1.4 | 0.9 | 0.7 | 1.5 | 1.1 | 2.9 | 1.7 | 0.40 |
| YPL170W | 1.2 | 1.3 | 0.9 | 1.1 | 1.9 | 1.1 | 0.6 | 1.1 | 1.4 | 1.0 | 1.1 | 0.9 | 1.1 | 1.4 | 1.2 | 1.6 | 2.2 | 1.4 | 1.05 |
| YGL051W | 1.1 | 1.0 | 1.6 | 1.7 | 1.3 | 1.0 | 1.3 | 0.8 | 1.1 | 1.8 | 1.4 | 1.0 | 1.5 | 1.2 | 0.8 | 2.6 | 1.4 | 1.9 | 1.06 |
| YIL112W | 1.1 | 1.4 | 2.8 | 1.9 | 0.6 | 1.3 | 1.1 | 0.9 | 1.3 | 2.0 | 1.4 | 1.2 | 1.1 | 1.3 | 1.8 | 2.4 | 1.2 | 1.3 | 0.45 |
| YLR052W | 1.0 | 1.5 | 0.8 | 1.3 | 1.0 | 0.8 | 1.0 | 1.2 | 0.4 | 1.1 | 1.1 | 1.0 | 0.8 | 1.2 | 1.0 | 2.4 | 0.7 | 0.9 | 0.77 |
| YNL203C | 0.9 | 1.3 | 0.3 | 3.2 | 1.0 | 1.7 | 1.6 | 0.8 | | 0.3 | 1.1 | 0.9 | 1.2 | 2.7 | 1.2 | 5.6 | 1.3 | 1.7 | 0.21 |
| YNR014W | 1.0 | 0.8 | 1.7 | 1.2 | 1.2 | 0.7 | 1.7 | 0.9 | 0.3 | 2.3 | 4.4 | 0.9 | 1.5 | 4.9 | 1.0 | 2.4 | 1.1 | 1.0 | 0.31 |
| YAL028W | 0.9 | 0.7 | 1.1 | 2.0 | 1.0 | 0.6 | 1.2 | 0.8 | 1.6 | 1.3 | 1.0 | 1.0 | 1.5 | 1.6 | 2.2 | 1.7 | 1.0 | 1.0 | 0.30 |
| YBL095W | 0.8 | 1.4 | 1.2 | 1.4 | 1.1 | 0.9 | 1.1 | 0.9 | 1.0 | 0.6 | 1.2 | 0.8 | 1.0 | 1.0 | 0.4 | 2.2 | 1.3 | 1.9 | 0.72 |
| YBR157C | 0.7 | 1.0 | 0.4 | 1.4 | 1.3 | 1.2 | 1.6 | 0.7 | 0.6 | 0.3 | 0.6 | 0.6 | 0.5 | 0.9 | 0.2 | 1.9 | 1.3 | 0.8 | 0.69 |
| YDL091C | 1.1 | 1.3 | 1.8 | 1.4 | 0.7 | 0.8 | 1.3 | 1.3 | 1.6 | 1.5 | 1.5 | 1.0 | 1.3 | 1.5 | 1.9 | 2.4 | 1.1 | 1.6 | 0.47 |
| YDL216C | 1.1 | 1.1 | 0.7 | 2.0 | 1.6 | 1.6 | 1.2 | 1.6 | 1.3 | 1.3 | 0.7 | 0.9 | 1.2 | 1.1 | 1.0 | 1.8 | 1.0 | 1.6 | 0.42 |
| YDR067C | 1.3 | 1.2 | 0.7 | 2.7 | 0.9 | 1.1 | 1.3 | 1.8 | 1.0 | 1.3 | 0.9 | 0.9 | 1.2 | 1.5 | 1.5 | 2.4 | 1.3 | 1.4 | 0.76 |
| YDR186C | 0.7 | 0.9 | 0.7 | 0.7 | 1.3 | 0.7 | 1.4 | 1.2 | 1.2 | 1.6 | 0.7 | 1.1 | 1.1 | 0.8 | 0.8 | 2.3 | 1.1 | 0.9 | 0.50 |
| YDR196C | 0.9 | 0.9 | 0.7 | 1.1 | 1.0 | 1.7 | 1.7 | 1.5 | 1.6 | 1.1 | 1.3 | 0.7 | 1.2 | 1.0 | 0.7 | 2.7 | 1.2 | 1.9 | 1.54 |
| YDR262W | 1.3 | 1.8 | 0.8 | 1.2 | 1.7 | 1.3 | 1.7 | 1.2 | 0.9 | 1.7 | 1.6 | 0.8 | 0.9 | 1.7 | 1.5 | 3.8 | 1.0 | 1.4 | 1.11 |
| YDR306C | 0.9 | 1.2 | 0.7 | 1.8 | 1.7 | 1.2 | 1.6 | 1.3 | 2.0 | 1.4 | 2.1 | 0.9 | 1.2 | 1.1 | 1.2 | 2.4 | 1.0 | 1.5 | 0.77 |
| YDR319C | 1.3 | 0.9 | 0.8 | 1.1 | 1.2 | 1.2 | 1.7 | 1.3 | 1.1 | 1.1 | 1.3 | 1.0 | | 1.3 | 0.8 | 2.0 | 2.1 | 2.2 | 1.23 |
| YER188W | 1.3 | 0.9 | 1.2 | 1.9 | 0.9 | 1.5 | 1.2 | 0.7 | 0.4 | 0.9 | 1.2 | 0.9 | 1.1 | 1.1 | 0.3 | 2.5 | 1.0 | 1.1 | 0.61 |
| YGL004C | 0.8 | 1.4 | 1.3 | 1.0 | 1.0 | 1.4 | 2.9 | 1.4 | 1.8 | 2.2 | 1.0 | 0.8 | 1.3 | 0.9 | 0.6 | 2.2 | 1.1 | 1.4 | 0.48 |
| YGR141W | 0.8 | 1.6 | 1.6 | 1.7 | 1.1 | 1.5 | 1.5 | 0.9 | 0.3 | 1.0 | 0.6 | 0.6 | 1.0 | 0.7 | 2.6 | 2.1 | 1.1 | 1.3 | 0.65 |
| YHR080C | 0.6 | 1.8 | 1.9 | 0.7 | 0.8 | 1.8 | 1.1 | 1.1 | 1.9 | 1.2 | 0.7 | 1.2 | 2.0 | 0.9 | 0.9 | 2.3 | 1.1 | 1.3 | 0.49 |
| YHR097C | 1.6 | 0.7 | 0.7 | 0.9 | | 0.8 | 0.8 | 0.7 | 0.4 | 0.6 | 0.7 | 0.8 | 1.3 | 0.9 | 2.7 | 2.0 | 1.7 | 1.4 | 0.24 |
| YIL077C | 0.9 | 0.8 | 1.2 | 1.2 | 1.3 | 1.8 | 1.2 | 0.9 | 1.5 | 1.4 | 1.3 | 1.6 | 1.3 | 1.3 | 0.9 | 2.4 | 1.3 | 1.1 | 0.75 |
| YJL046W | 0.9 | 0.9 | 0.6 | 1.8 | 1.0 | 0.7 | 1.5 | 1.1 | 1.8 | 2.7 | 4.2 | 0.9 | 1.3 | 1.3 | 1.0 | 2.5 | 1.0 | 1.3 | 0.68 |
| YLL005C | 1.0 | 1.1 | 1.1 | 1.2 | 1.6 | 1.9 | 1.3 | 1.0 | 0.4 | 1.0 | 0.9 | 0.8 | 1.1 | 1.7 | 1.0 | 2.1 | 1.1 | 1.2 | 0.44 |

| | | | | | | | | | | | | | | | | | | |
|---|---|---|---|---|---|---|---|---|---|---|---|---|---|---|---|---|---|---|
| YLR151C | 1.4 | 2.0 | 1.4 | 1.0 | 1.0 | | 1.4 | 1.4 | 1.5 | 0.9 | 1.1 | 0.5 | 0.7 | 1.3 | 0.7 | 2.4 | 1.0 | 1.4 | 0.30 |
| YLR271W | 1.6 | 1.2 | 0.8 | 1.8 | 1.4 | 1.4 | 1.7 | 1.5 | 1.0 | 1.0 | 1.3 | 0.8 | 1.2 | 1.2 | 1.0 | 3.0 | 1.5 | 1.8 | 0.78 |
| YMR025W | 1.2 | 1.2 | 0.7 | 1.5 | 1.3 | 1.3 | 1.4 | 1.2 | 1.0 | 1.6 | 1.2 | 0.9 | 1.2 | 1.8 | 1.1 | 2.4 | 1.0 | 1.5 | 0.42 |
| YMR135C | 1.0 | 1.0 | 1.1 | 1.9 | 1.8 | 1.0 | 1.0 | 1.0 | 1.1 | 1.4 | 1.1 | 1.0 | 1.3 | 1.0 | 1.4 | 2.3 | 1.5 | 1.3 | 1.06 |
| YMR210W | 1.0 | 1.1 | 1.8 | 1.9 | 0.6 | 0.8 | 1.1 | 1.1 | 1.5 | 2.2 | 0.8 | 0.9 | 1.0 | 1.0 | 1.0 | 2.2 | 0.8 | 1.5 | 0.56 |
| YNR040W | 0.8 | 4.9 | 0.5 | 0.9 | 0.9 | 1.6 | 2.1 | 1.3 | 1.1 | 0.8 | 1.2 | 0.6 | 0.8 | 1.5 | 0.7 | 2.1 | 1.1 | 1.0 | 0.30 |
| YPL039W | 1.2 | 0.9 | 0.6 | 1.3 | 1.4 | 1.5 | 1.5 | 1.4 | 1.1 | 1.0 | 0.9 | 0.9 | 1.1 | 1.1 | 0.6 | 2.4 | 1.1 | 1.1 | 0.52 |
| YPL099C | 1.1 | 1.9 | 0.7 | 2.0 | 0.9 | 1.2 | 1.4 | 1.4 | 2.4 | 1.7 | 0.8 | 0.9 | 1.1 | 1.3 | 0.5 | 2.1 | 1.0 | 1.7 | 0.92 |
| YPL107W | 1.2 | 0.8 | 1.1 | 1.3 | 1.0 | 1.1 | 1.2 | 1.5 | 1.4 | 1.3 | 1.1 | 0.7 | 0.8 | 1.7 | 0.7 | 1.8 | 1.0 | 1.1 | 0.59 |
| YPL138C | 1.1 | 1.7 | 1.1 | 1.8 | 0.7 | 0.5 | 1.1 | 1.3 | 1.1 | 1.5 | 0.5 | 0.9 | 0.6 | 1.3 | 2.7 | 2.6 | 0.8 | 1.1 | 0.38 |

## Table 2 Mitochondria-located protein genes

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YJR048W | 1.3 | 0.9 | 2.2 | 1.4 | 0.8 | 0.5 | 0.3 | 0.9 | 0.5 | 1.5 | 1.0 | 0.8 | 0.4 | 2.5 | 0.6 | 1.5 | 1.2 | 1.2 | 1.19 |
| YOR226C | 1.3 | 2.2 | 1.7 | 1.1 | 1.3 | 1.2 | 1.2 | 1.2 | 1.3 | 5.9 | 1.5 | 0.6 | 0.9 | 2.7 | 1.0 | 0.7 | 1.0 | 0.7 | 0.58 |
| YDL174C | 0.9 | 1.8 | 0.9 | 1.3 | 0.5 | 1.7 | 3.6 | 0.8 | 0.5 | 1.7 | 0.9 | 2.8 | 5.9 | 1.1 | 0.6 | 5.1 | 2.2 | 4.0 | 0.63 |
| YBL022C | 0.4 | 0.8 | 1.2 | 0.7 | 1.2 | 1.2 | 1.0 | 0.4 | 2.8 | 1.2 | 1.1 | 0.9 | 2.6 | 1.0 | 1.5 | 1.0 | 0.9 | 0.7 | 0.76 |
| YCL057W | 0.6 | 1.5 | 2.3 | 0.5 | 1.1 | 1.8 | 1.4 | 1.8 | 4.2 | 2.1 | 1.6 | 1.0 | 3.8 | 1.0 | 2.8 | 1.5 | 0.9 | 1.1 | 0.76 |
| YDR258C | 1.6 | 2.3 | 2.8 | 1.4 | 1.3 | 2.4 | 3.9 | 1.9 | 4.9 | 13.3 | 1.7 | 1.8 | 5.2 | 1.5 | 0.6 | 1.0 | 1.3 | 1.3 | 0.87 |
| YGR028W | 1.4 | 3.5 | 1.0 | 1.8 | 0.9 | 1.5 | 2.1 | 1.4 | 1.7 | 2.0 | 1.1 | 1.5 | 2.6 | 1.2 | 1.0 | 2.2 | 1.5 | 2.6 | 0.91 |
| YGR244C | 1.0 | 1.2 | 1.6 | 1.6 | 0.8 | 2.6 | 3.9 | 1.8 | 1.4 | 1.1 | 1.8 | 1.9 | 2.6 | 1.2 | 2.0 | 2.6 | 1.9 | 3.1 | 1.12 |
| YKL142W | 1.8 | 2.7 | 3.1 | 1.4 | 0.7 | 3.4 | 6.9 | 2.3 | 5.8 | 5.9 | 2.0 | 1.9 | 3.3 | 2.3 | 1.3 | 3.3 | 2.4 | 3.6 | 1.68 |
| YNL055C | 1.1 | 1.8 | 5.5 | 2.0 | 0.9 | 2.6 | 1.2 | 0.7 | 1.1 | 1.0 | 1.9 | 1.7 | 2.4 | 0.9 | 1.1 | 2.6 | 1.2 | 1.7 | 4.10 |
| YNL071W | 0.9 | 0.9 | 1.7 | 1.5 | 0.6 | 1.3 | 1.4 | 0.9 | 1.1 | 1.2 | 1.6 | 1.3 | 2.5 | 0.8 | 1.1 | 1.1 | 1.0 | 1.2 | 1.61 |
| YOR020C | 1.4 | 2.1 | 1.7 | 1.7 | 1.5 | 2.3 | 1.7 | 1.7 | 5.8 | 2.6 | 2.5 | 0.8 | 3.0 | 1.6 | 1.5 | 1.4 | 1.4 | 1.7 | 1.66 |
| YOR037W | 1.0 | 1.5 | 0.6 | 1.4 | 1.5 | 1.6 | 2.5 | 1.5 | 1.3 | 1.0 | 1.2 | 1.2 | 2.1 | 1.0 | 0.9 | 1.4 | 1.2 | 1.6 | 0.61 |
| YDL198C | 1.4 | 1.0 | 2.1 | 1.4 | 0.7 | 0.8 | 1.0 | 1.1 | 1.9 | 2.7 | 2.5 | 1.2 | 2.0 | 1.7 | 1.2 | 1.4 | 1.5 | 1.1 | 1.19 |
| YDR231C | 1.1 | 1.6 | 1.0 | 1.5 | 1.1 | 2.2 | 1.7 | 1.3 | 1.5 | 1.4 | 1.3 | 1.1 | 2.4 | 1.5 | 1.0 | 3.0 | 1.2 | 1.3 | 0.92 |
| YER178W | 0.8 | 0.9 | 3.6 | 1.0 | 0.7 | 2.5 | 1.6 | 0.8 | 1.7 | 1.3 | 2.5 | 1.3 | 2.4 | 0.9 | 1.8 | 1.0 | 1.1 | 0.9 | 2.18 |
| YFL016C | 0.8 | 1.0 | 1.6 | 0.9 | 1.3 | 1.3 | 1.4 | 0.7 | 6.4 | 2.9 | 1.2 | 1.0 | 2.8 | 0.8 | 0.8 | 0.6 | 1.2 | 0.9 | 1.25 |
| YGR008C | 2.1 | 3.0 | 1.7 | 3.2 | 0.9 | 2.9 | 3.7 | 1.9 | 3.1 | 2.4 | 2.6 | 2.0 | 3.4 | 1.3 | 1.5 | 2.3 | 1.7 | 4.2 | 3.03 |
| YIL155C | 1.0 | 0.7 | 1.4 | 3.7 | 1.3 | 1.4 | 2.2 | 1.0 | 2.5 | 2.9 | 1.3 | 1.4 | 2.0 | 1.3 | 1.4 | 3.8 | 1.1 | 1.4 | 0.51 |
| YJL102W | 1.1 | 0.7 | 0.4 | 0.8 | 1.3 | 1.6 | 0.6 | 0.9 | 3.1 | 2.2 | 0.7 | 0.8 | 2.6 | 1.5 | 1.5 | 0.6 | 1.0 | 1.1 | 0.22 |
| YJR045C | 0.5 | 1.9 | 3.9 | | 0.5 | 1.2 | 1.0 | 0.8 | 3.2 | 3.0 | 1.4 | 1.0 | 2.3 | 0.5 | 2.3 | 0.8 | 0.7 | 0.9 | 3.78 |
| YLR259C | 0.7 | 1.0 | 3.1 | 1.6 | 1.1 | 0.9 | 1.8 | 0.8 | 1.8 | 2.6 | 3.0 | 0.7 | 2.5 | 0.8 | 2.1 | 1.3 | 0.8 | 1.0 | 2.09 |
| YLR348C | 1.1 | 4.5 | 1.2 | 1.2 | 0.9 | 1.9 | 0.9 | 0.9 | 2.1 | 1.3 | 1.3 | 0.9 | 1.9 | 1.0 | 2.4 | 1.1 | 1.1 | 1.0 | 0.64 |
| YML054C | 1.5 | 1.8 | 1.3 | 3.4 | 1.3 | 1.8 | 1.2 | 1.5 | 4.1 | 1.8 | 1.4 | 1.8 | 2.8 | 2.1 | 1.1 | 7.8 | 1.1 | 1.6 | 0.25 |
| YMR089C | 0.8 | 1.1 | 0.9 | 0.9 | 0.8 | 1.2 | 1.2 | 1.3 | 5.6 | 2.5 | 1.3 | 0.8 | 2.3 | 0.9 | 1.8 | 1.1 | 0.9 | 1.1 | 0.69 |
| YMR152W | 0.9 | 1.1 | 1.8 | 0.9 | 0.8 | 7.5 | 0.8 | 0.8 | 2.7 | 1.3 | 1.2 | 1.3 | 1.9 | 1.1 | 0.6 | 1.0 | 1.5 | 0.9 | 0.71 |
| YNL104C | 1.1 | 1.3 | 3.0 | 0.9 | 0.8 | 0.8 | 0.6 | 0.9 | 1.1 | 2.0 | 2.1 | 1.1 | 2.1 | 0.9 | 2.5 | 1.5 | 0.9 | 0.9 | 1.69 |
| YOR130C | 0.8 | 1.7 | 1.4 | 1.0 | 1.8 | 1.6 | 1.3 | 0.8 | 2.0 | 1.7 | 0.6 | 0.9 | 2.1 | 1.3 | 1.0 | 0.8 | 1.5 | 1.1 | 0.51 |

| | | | | | | | | | | | | | | | | | | | |
|---|---|---|---|---|---|---|---|---|---|---|---|---|---|---|---|---|---|---|---|
| YPR024W | 0.7 | 1.0 | 1.3 | 1.0 | 1.3 | 1.0 | 1.4 | 0.7 | 3.0 | 1.7 | 0.9 | 0.8 | 2.5 | 0.7 | 1.0 | 1.6 | 1.1 | 0.8 | 0.84 |
| YPR067W | 1.6 | 1.2 | 1.7 | 0.9 | 1.3 | 1.3 | 1.5 | 1.4 | 3.5 | 3.4 | 1.8 | 1.0 | 3.0 | 1.6 | 1.3 | 0.8 | 1.0 | 1.0 | 0.66 |
| YBR029C | 0.7 | 0.2 | 1.8 | 1.0 | 1.4 | 0.8 | 0.4 | 0.7 | 2.0 | 1.1 | 0.7 | 1.3 | 1.7 | 0.9 | 3.5 | 0.9 | 0.6 | 0.8 | 1.56 |
| YCL009C | 0.6 | 0.9 | 2.0 | 0.5 | 0.7 | 1.8 | 1.3 | 1.1 | 0.9 | 1.6 | 1.5 | 1.7 | 1.6 | 0.7 | 6.5 | 0.9 | 1.1 | 0.8 | 0.68 |
| YER026C | 0.8 | 0.6 | 3.3 | 1.0 | 1.2 | 2.5 | 1.6 | 0.8 | 0.9 | 1.5 | 0.9 | 1.7 | 1.9 | 1.1 | 4.0 | 1.4 | 1.7 | 3.2 | 4.48 |
| YLR109W | 0.8 | 2.9 | 6.0 | 0.9 | 1.3 | 2.1 | 2.6 | 0.9 | 2.4 | 2.7 | 1.5 | 1.6 | 3.2 | 2.3 | 3.9 | 1.8 | 1.1 | 1.2 | 3.86 |
| YMR189W | 0.7 | 0.8 | 1.9 | 2.8 | 0.4 | 1.9 | 0.8 | 0.4 | 0.7 | 0.9 | 8.2 | 0.6 | 0.9 | 1.6 | 5.6 | 0.9 | 1.0 | 0.8 | 0.88 |
| YNL169C | 0.7 | 1.4 | 1.4 | 1.2 | 0.7 | 1.4 | 0.7 | 0.8 | 1.1 | 1.1 | 0.8 | 1.0 | 1.7 | 0.9 | 3.4 | 0.8 | 1.1 | 1.3 | 1.05 |
| YER069W | 1.3 | 0.8 | 3.3 | 1.4 | 1.5 | 1.3 | 1.0 | 1.5 | 0.8 | 10.4 | 1.5 | 0.8 | 1.4 | 1.3 | 2.4 | 1.1 | 1.1 | 0.9 | 0.25 |
| YIL022W | 0.8 | 0.7 | 1.1 | 1.6 | 0.8 | 1.2 | 0.7 | 0.8 | 1.1 | 1.3 | 1.0 | 0.9 | 1.2 | 0.6 | 2.9 | 1.1 | 0.8 | 0.7 | 0.52 |
| YBR146W | 0.9 | 0.8 | 0.7 | 1.5 | 1.3 | 2.6 | 1.5 | 1.0 | 0.9 | 1.1 | 1.2 | 1.0 | 1.7 | 1.2 | 1.0 | 2.9 | 1.0 | 1.2 | 1.61 |
| YDR019C | 1.4 | 0.5 | 1.7 | 4.0 | 0.9 | 4.8 | 1.4 | 0.7 | 0.4 | 0.7 | 1.8 | 0.7 | 1.0 | 1.6 | 1.8 | 1.8 | 1.2 | 1.6 | 2.48 |
| YGR207C | 1.5 | 1.1 | 1.0 | 1.1 | 1.5 | 2.8 | 1.7 | 1.9 | 2.1 | 2.6 | 1.4 | 1.1 | 1.0 | 2.2 | 0.6 | 1.8 | 1.2 | 1.8 | 1.49 |
| YMR072W | 1.1 | 1.0 | 1.4 | 1.0 | 1.7 | 2.4 | 1.4 | 1.0 | 3.0 | 1.2 | 1.9 | 0.8 | 2.1 | 1.2 | 0.8 | 1.7 | 0.9 | 1.9 | 2.06 |
| YNL037C | 1.4 | 1.8 | 1.7 | 1.1 | 1.7 | 2.8 | 2.8 | 1.0 | 0.6 | 3.2 | 1.4 | 1.3 | 1.9 | 1.3 | 0.9 | 1.4 | 0.9 | 1.3 | 2.05 |
| YOR136W | 1.0 | 0.9 | 3.5 | 1.0 | 1.5 | 3.9 | 3.2 | 1.1 | 0.3 | 3.4 | 1.4 | 1.2 | 1.5 | 0.9 | 1.2 | 1.3 | 0.8 | 1.3 | 3.20 |
| YPL271W | 1.2 | 3.2 | 4.1 | 1.2 | 1.4 | 3.3 | 1.2 | 0.9 | 0.6 | 1.2 | 1.7 | 0.8 | 1.2 | 1.3 | 0.8 | 1.4 | 1.2 | 1.3 | 1.34 |
| YAL044C | 1.5 | 1.1 | 1.0 | 3.0 | 0.8 | 2.2 | 1.0 | 0.8 | 0.4 | 0.6 | 1.4 | 0.6 | 1.1 | 1.4 | 0.7 | 1.0 | 1.4 | 1.8 | 4.07 |
| YAL054C | 1.2 | 1.1 | 1.4 | 1.6 | 1.1 | 2.2 | 1.1 | 1.1 | 8.9 | 4.1 | 1.3 | 1.0 | 1.8 | 1.3 | 0.9 | 2.5 | 1.5 | 1.4 | 0.24 |
| YBR024W | 1.0 | 1.0 | 1.1 | 2.4 | 1.0 | 2.2 | 1.9 | 1.4 | 1.3 | 1.4 | 1.3 | 1.1 | 1.7 | 1.9 | 0.8 | 2.3 | 1.0 | 1.2 | 1.14 |
| YDR405W | 0.9 | 1.1 | 4.7 | 1.0 | 1.1 | 2.4 | 1.2 | 0.9 | 0.6 | 1.0 | 1.0 | 1.2 | 1.3 | 1.1 | 2.5 | 1.3 | 1.3 | 0.9 | 0.44 |
| YGL068W | 1.0 | 0.6 | 1.1 | 1.4 | 0.9 | 2.6 | 1.5 | 0.9 | 0.7 | 0.8 | 1.0 | 1.1 | 1.0 | 0.9 | 0.6 | 1.8 | 1.8 | 1.0 | 3.35 |
| YGR220C | 1.1 | 0.6 | 0.9 | 1.1 | 1.3 | 2.1 | 1.4 | 1.5 | 0.8 | 1.5 | 1.1 | 0.9 | 1.1 | 1.5 | 1.1 | 2.3 | 0.9 | 1.1 | 1.42 |
| YHR037W | 0.7 | 0.9 | 1.3 | 1.6 | 0.9 | 1.8 | 1.2 | 1.0 | 0.9 | 2.2 | 1.7 | 1.1 | 1.1 | 1.1 | 1.1 | 2.0 | 1.0 | 1.4 | 1.15 |
| YIL070C | 1.2 | 1.0 | 0.8 | 1.4 | 1.3 | 2.3 | 1.6 | 1.1 | 0.3 | 0.6 | 1.0 | 0.8 | 0.7 | 1.3 | 1.0 | 2.4 | 0.7 | 1.1 | 1.69 |
| YIL111W | 1.6 | 1.0 | 1.8 | 2.7 | | 2.6 | 3.2 | 1.2 | 1.6 | 1.1 | 2.1 | 0.9 | 1.7 | 1.5 | 1.1 | 2.5 | 1.9 | 3.9 | 1.52 |
| YKL138C | 1.2 | 0.9 | 0.6 | 0.9 | 1.3 | 2.3 | 1.9 | 1.7 | 1.1 | 2.1 | 1.2 | 0.8 | 0.8 | 1.6 | 0.6 | 2.5 | 1.0 | 1.3 | 1.29 |
| YKL150W | 1.1 | 2.0 | 3.0 | 1.4 | 1.4 | 2.3 | 2.4 | 1.3 | 1.1 | 1.3 | 1.9 | 1.2 | 1.8 | 1.4 | 0.9 | 6.1 | 1.2 | 2.7 | 2.20 |
| YKR006C | 1.0 | 0.7 | 1.2 | 1.2 | 1.4 | 2.4 | 1.6 | 1.2 | 1.0 | 1.5 | 1.2 | 0.8 | 0.9 | 1.6 | 0.9 | 2.3 | 0.8 | 1.1 | 1.39 |
| YLR142W | 4.4 | 2.7 | 1.1 | 6.1 | 1.3 | 3.1 | 1.2 | 2.1 | 3.1 | 4.4 | 3.8 | 0.8 | 1.2 | 2.1 | 1.6 | 3.4 | 1.9 | 3.2 | 0.28 |
| YML110C | 1.1 | 0.8 | 1.9 | 1.6 | 0.8 | 2.6 | 2.0 | 1.4 | 2.7 | 2.2 | 1.7 | 1.1 | 1.8 | 1.4 | 1.3 | 2.3 | 1.1 | 1.6 | 2.01 |

| | | | | | | | | | | | | | | | | | | | |
|---|---|---|---|---|---|---|---|---|---|---|---|---|---|---|---|---|---|---|---|
| YNL284C | 1.1 | 0.7 | 0.3 | 1.4 | 1.8 | 1.0 | 2.8 | 1.6 | 0.9 | 1.9 | 1.3 | 0.7 | 1.0 | 1.5 | 0.8 | 2.4 | 1.0 | 1.5 | 1.26 |
| YDR268W | 0.8 | 1.5 | 0.4 | 1.2 | 2.2 | 1.0 | 2.4 | 1.0 | 1.0 | 2.0 | 1.1 | 0.7 | 1.0 | 1.4 | 1.0 | 1.6 | 1.1 | 1.2 | 0.47 |
| YMR193W | 1.4 | 1.2 | 0.5 | 1.9 | 2.2 | 1.7 | 2.7 | 1.7 | 0.8 | 1.1 | 1.6 | 0.6 | 0.9 | 1.5 | 0.8 | 3.7 | 1.1 | 1.7 | 0.93 |
| YOR374W | 1.3 | 4.5 | 7.3 | 3.9 | 0.9 | 1.4 | 1.9 | 0.9 | 2.3 | 5.9 | 2.6 | 1.5 | 1.7 | 1.1 | 2.5 | 2.5 | 1.2 | 2.4 | 1.24 |
| YER061C | 0.9 | 0.9 | 1.2 | 2.5 | 0.8 | 0.4 | 0.9 | 0.7 | 0.3 | 0.7 | 2.2 | 0.7 | 0.9 | 1.0 | 0.8 | 1.8 | 1.2 | 1.2 | 0.84 |
| YIL136W | 1.1 | 1.0 | 1.2 | 2.5 | 1.0 | 1.0 | 1.8 | 1.1 | 3.4 | 1.7 | 4.8 | 0.9 | 1.7 | 1.7 | 1.5 | 4.5 | 1.3 | 3.2 | 0.95 |
| YLL009C | 1.0 | 1.2 | 0.6 | 1.9 | 1.5 | 1.2 | 1.7 | 1.0 | 1.3 | 1.7 | 3.5 | 0.7 | 0.9 | 1.4 | 1.1 | 2.8 | 0.9 | 1.2 | 1.66 |
| YLR163C | 1.0 | 0.9 | 0.7 | 1.4 | 1.6 | 1.3 | 1.7 | 1.4 | 2.6 | 2.9 | 2.2 | 0.7 | 1.4 | 1.3 | 1.2 | 1.4 | 0.9 | 1.1 | 0.55 |
| YBR291C | 2.0 | 0.9 | 1.0 | 1.5 | 0.9 | 1.1 | 0.9 | 2.2 | 0.9 | 1.0 | 1.1 | 0.9 | 1.0 | 1.7 | 0.5 | 1.5 | 0.9 | 1.3 | 1.19 |
| YIL094C | 1.5 | 0.4 | 0.8 | 0.7 | 1.4 | 1.2 | 1.3 | 3.6 | 0.3 | 0.4 | 1.8 | 1.0 | 0.7 | 1.0 | 0.5 | 0.9 | 0.9 | 1.0 | 2.26 |
| YAL015C | 1.2 | 1.1 | 1.7 | 1.9 | 1.1 | 0.7 | 1.2 | 1.1 | 4.0 | 2.7 | 1.4 | 1.0 | 1.2 | 1.6 | 0.7 | 2.0 | 1.1 | 1.2 | 0.61 |
| YJR095W | 1.2 | 20.5 | 1.9 | 6.7 | 1.2 | 1.5 | 2.0 | 0.9 | 0.5 | 6.3 | 0.6 | 0.7 | 0.8 | 1.3 | 0.8 | 0.8 | 1.3 | 0.9 | 0.23 |
| YKL085W | 1.4 | 2.3 | 1.6 | 1.2 | 1.2 | 1.9 | 1.5 | 1.2 | 1.9 | 3.0 | 1.8 | 0.8 | 1.5 | 1.0 | 0.5 | 1.7 | 0.9 | 1.3 | 2.16 |
| YMR177W | 1.4 | 0.8 | 1.1 | 1.0 | 1.6 | 0.8 | 1.0 | 1.2 | 2.4 | 3.8 | 1.4 | 0.6 | 0.5 | 1.2 | 0.8 | 0.5 | 0.9 | 0.9 | 0.55 |
| YPL224C | 1.0 | 2.2 | 1.3 | 2.5 | 0.6 | 1.3 | 2.2 | 1.3 | 3.2 | 4.2 | 0.8 | 1.1 | 1.3 | 1.4 | 1.0 | 2.5 | 1.3 | 1.9 | 0.64 |
| YER014W | 1.0 | 0.9 | 0.9 | 0.6 | 0.8 | 4.0 | | 1.2 | 3.5 | 1.1 | 0.9 | 1.1 | 1.5 | 1.2 | 2.3 | 0.8 | 1.0 | 0.9 | 0.43 |
| YFR049W | 1.5 | 1.5 | 1.8 | 1.2 | 1.3 | | | 1.3 | 2.6 | 1.5 | 1.7 | 1.2 | 1.4 | 1.2 | 0.7 | 0.9 | 1.3 | 1.0 | 0.83 |
| YGR112W | 1.0 | 2.9 | 1.1 | 1.3 | 0.9 | 1.1 | 1.3 | 1.3 | 2.6 | 1.3 | 1.3 | 0.7 | 1.1 | 1.1 | 1.2 | 2.8 | 0.9 | 1.2 | 0.31 |
| YLL001W | 0.9 | 0.7 | 1.2 | 1.2 | 0.6 | 1.0 | 0.8 | 1.0 | 3.5 | 2.4 | 0.9 | 0.7 | 1.7 | 1.2 | 0.9 | 1.3 | 0.9 | 1.3 | 0.70 |
| YML042W | 0.9 | 1.4 | 1.5 | 2.2 | 1.0 | 0.9 | 0.8 | 1.6 | 3.8 | 2.4 | 2.5 | 1.0 | 1.2 | 1.4 | 1.7 | 4.8 | 1.0 | 0.9 | 0.29 |
| YNL213C | 1.7 | 11.6 | 0.9 | 1.5 | 1.3 | 1.2 | 1.4 | 1.8 | 3.0 | 1.5 | 1.5 | 0.8 | 0.9 | 1.8 | 1.0 | 1.7 | 0.9 | 1.2 | 0.70 |
| YOR386W | 1.0 | 0.8 | 1.3 | 1.5 | 1.2 | 3.3 | 1.8 | 1.0 | 4.1 | 3.0 | 1.4 | 1.2 | 2.4 | 0.9 | 1.3 | 1.2 | 2.1 | 1.7 | 0.46 |
| YBR037C | 0.8 | 0.8 | 1.3 | 2.4 | 0.7 | 1.2 | 1.2 | 1.0 | 3.0 | 1.9 | 1.5 | 0.7 | 1.2 | 1.2 | 1.0 | 2.7 | 1.6 | 1.3 | 0.60 |
| YCR024C | 1.1 | 1.0 | 1.5 | 0.8 | 0.8 | 2.9 | 1.0 | 1.2 | 2.5 | 1.7 | 1.4 | 0.8 | 0.9 | 1.3 | 1.0 | 1.2 | 0.9 | 1.0 | 0.32 |
| YDR194C | 0.7 | 0.9 | 0.5 | 1.0 | 1.0 | 1.0 | 1.3 | 1.4 | 2.8 | 2.2 | 1.2 | 0.6 | 1.2 | 0.8 | 1.1 | 0.8 | 0.6 | 0.8 | 2.19 |
| YER017C | 0.8 | 1.1 | 1.4 | 1.3 | 1.0 | 0.5 | 1.1 | 0.5 | 2.7 | 1.8 | 0.9 | 1.0 | 1.5 | 1.1 | 2.1 | 1.0 | 0.9 | 1.0 | 0.43 |
| YGL125W | 1.0 | 3.2 | 4.3 | 0.7 | 0.9 | | 1.7 | 0.8 | 2.1 | 2.1 | 1.6 | 1.0 | 1.4 | 1.0 | 1.9 | 1.2 | 1.2 | 1.1 | 0.27 |
| YGR029W | 1.1 | 1.4 | 1.0 | 1.0 | 0.8 | 0.6 | 1.6 | 1.7 | 2.7 | 1.1 | 1.2 | 0.6 | 1.0 | 2.0 | 0.4 | 1.1 | 1.1 | 1.3 | 0.71 |
| YHL038C | 1.0 | 1.2 | 1.4 | 0.8 | 1.3 | 0.8 | 1.2 | 1.1 | 4.0 | 1.7 | 0.8 | 1.6 | 1.3 | 0.6 | 0.8 | 0.5 | 0.9 | 0.6 | 1.95 |
| YKL192C | 1.1 | 1.0 | 3.7 | 1.1 | 1.1 | 1.8 | 1.1 | 0.9 | 3.5 | 1.6 | 1.1 | 1.2 | 1.7 | 1.5 | 1.2 | 2.1 | 1.0 | 0.8 | 1.57 |
| YKR052C | 0.9 | 1.1 | 2.6 | 2.6 | 1.0 | 0.5 | 1.9 | 1.1 | 1.9 | 3.6 | 1.3 | 0.9 | 1.2 | 1.1 | 0.5 | 2.5 | 0.8 | 1.5 | 1.15 |

| | | | | | | | | | | | | | | | | | | | |
|---|---|---|---|---|---|---|---|---|---|---|---|---|---|---|---|---|---|---|---|
| YML078W | 1.2 | 1.9 | 2.5 | 1.5 | 0.7 | 1.4 | 1.3 | 1.4 | 2.6 | 2.2 | 2.4 | 1.2 | 1.7 | 1.7 | 1.0 | 1.8 | 1.3 | 1.6 | 1.98 |
| YMR056C | 1.1 | 1.3 | 1.7 | 1.0 | 1.1 | 0.9 | 1.4 | 1.2 | 1.8 | 1.1 | 1.6 | 0.9 | 0.9 | 1.1 | 0.7 | 2.4 | 1.0 | 1.1 | 0.72 |
| YNL005C | 1.1 | 1.0 | 0.6 | 1.2 | 1.2 | 1.7 | 1.3 | 1.5 | 2.0 | 2.0 | 1.2 | 0.9 | 1.5 | 1.3 | 1.0 | 1.6 | 0.9 | 1.5 | 1.52 |
| YPR047W | 1.0 | 1.0 | 0.9 | 1.5 | 0.9 | 1.0 | 1.8 | 1.2 | 2.1 | 1.9 | 1.1 | 0.9 | 1.3 | 1.0 | 0.8 | 2.8 | 1.0 | 1.5 | 0.39 |
| YPR134W | 1.1 | 1.1 | 0.8 | 1.7 | 1.5 | 0.6 | 1.5 | 1.4 | 1.8 | 2.3 | 1.3 | 0.9 | 1.1 | 1.5 | 0.7 | 2.4 | 1.0 | 1.2 | 0.65 |
| YGL191W | 1.7 | 1.6 | 1.4 | 1.8 | 0.9 | 1.6 | 1.0 | 1.6 | 1.1 | 1.3 | 1.6 | 0.9 | 1.1 | 1.6 | 0.7 | 1.8 | 1.2 | 1.7 | 2.35 |
| YLR038C | 2.3 | 1.1 | 0.6 | 2.1 | 1.8 | 1.2 | 1.0 | 1.4 | 0.4 | 0.9 | 1.4 | 0.6 | 0.5 | 1.6 | 0.7 | 2.1 | 1.2 | 2.1 | 1.52 |
| YHR008C | 1.3 | 5.4 | 4.8 | 1.8 | 0.6 | 1.0 | 0.7 | 0.9 | 1.7 | 2.4 | 2.1 | 0.7 | 1.2 | 1.6 | 1.7 | 2.2 | 0.9 | 1.0 | 1.04 |
| YPR037C | 1.2 | 2.3 | 1.6 | 1.3 | 0.8 | 0.9 | 1.2 | 1.1 | 1.5 | 1.3 | 0.9 | 0.9 | 1.0 | 1.6 | 4.1 | 1.8 | 1.2 | 1.7 | 0.80 |
| YAL039C | 1.1 | 2.2 | 1.7 | 1.0 | 1.1 | 0.8 | 1.0 | 1.2 | 1.6 | 2.2 | 1.1 | 1.4 | 1.6 | 1.2 | 3.3 | 2.2 | 1.4 | 1.1 | 0.39 |
| YDL181W | 1.3 | 1.8 | 1.9 | 1.8 | 1.7 | 1.2 | 1.1 | 0.6 | 0.7 | 0.8 | 0.8 | 0.9 | 0.9 | 1.1 | 0.4 | 1.1 | 1.5 | 1.4 | 0.85 |
| YPL215W | 1.1 | 1.9 | 1.0 | 1.5 | 0.8 | 0.7 | 0.7 | 1.1 | 1.7 | 1.2 | 1.1 | 0.7 | 0.7 | 1.2 | 0.6 | 1.4 | 1.0 | 1.1 | 1.10 |
| YPL262W | 1.0 | 1.7 | 3.8 | 1.2 | 0.6 | 1.6 | 1.3 | 1.0 | 1.4 | 5.8 | 1.4 | 1.1 | 1.5 | 1.2 | 0.6 | 1.4 | 1.1 | 1.2 | 0.79 |
| YNL256W | 0.7 | 1.6 | 0.6 | 0.6 | 1.0 | 0.5 | 0.6 | 0.9 | 0.8 | 0.6 | 0.6 | 0.6 | 1.3 | 0.7 | 0.9 | 0.4 | 0.6 | 0.6 | 1.09 |
| YBL030C | 1.0 | 0.9 | 4.5 | 0.9 | 0.7 | 1.1 | 0.9 | 0.8 | 0.4 | 0.9 | 1.1 | 0.9 | 0.5 | 0.8 | 1.8 | 1.0 | 1.0 | 1.1 | 3.12 |
| YBR221C | 0.8 | 0.7 | 3.2 | 1.5 | 1.3 | 1.7 | 1.5 | 0.8 | 1.0 | 1.3 | 1.2 | 1.3 | 1.7 | 0.8 | 1.1 | 1.5 | 0.9 | 1.0 | 3.62 |
| YKL141W | 1.6 | 1.1 | 4.7 | 1.7 | 1.5 | 1.2 | 0.5 | 0.8 | 0.8 | 0.5 | 1.2 | 0.8 | 0.8 | 1.4 | 0.6 | 2.8 | 0.9 | 1.8 | 2.84 |
| YKR066C | 0.9 | 1.4 | 5.5 | 0.7 | 1.0 | 0.9 | 0.5 | 0.7 | 0.7 | 2.4 | 1.3 | 0.9 | 0.6 | 1.5 | 0.6 | 1.3 | 0.9 | 1.1 | 1.25 |
| YMR083W | 1.4 | 1.7 | 2.6 | 1.1 | 1.5 | 1.8 | 1.7 | 1.1 | 0.4 | 1.1 | 1.6 | 0.7 | 0.9 | 0.8 | 1.3 | 1.3 | 0.9 | 1.1 | 2.52 |
| YMR203W | 0.8 | 0.7 | 3.0 | 1.4 | 0.6 | 1.1 | 0.8 | 0.7 | 0.7 | 1.2 | 2.3 | 0.7 | 1.1 | 0.7 | 1.2 | 0.9 | 0.8 | 0.7 | 1.62 |
| YBL099W | 0.8 | 0.9 | 3.1 | 1.4 | 1.0 | 0.9 | 0.9 | 0.8 | 0.9 | 0.7 | 2.1 | 1.0 | 0.9 | 0.6 | 1.0 | 0.9 | 0.7 | 1.2 | 3.49 |
| YDR178W | 2.0 | 2.0 | 3.4 | 2.2 | 0.9 | 2.1 | 0.6 | 0.9 | 0.9 | 0.8 | 2.0 | 1.3 | 1.5 | 1.5 | 0.7 | 3.0 | 1.2 | 2.3 | 2.27 |
| YDR298C | 1.3 | 1.2 | 2.6 | 1.3 | 1.3 | 1.2 | 1.0 | 1.2 | 1.0 | 1.6 | 1.2 | 1.1 | 1.2 | 1.5 | 0.8 | 2.0 | 1.1 | 1.6 | 2.69 |
| YEL024W | 1.3 | 0.9 | 3.8 | 1.1 | 1.3 | 1.1 | 0.7 | 1.0 | 0.7 | 0.6 | 0.9 | 0.8 | 0.6 | 1.1 | 0.6 | 2.1 | 1.1 | 1.5 | 1.59 |
| YGR082W | 1.0 | 0.9 | 2.2 | 1.0 | 0.9 | 1.1 | 0.9 | 0.8 | 0.6 | 0.9 | 1.3 | 0.8 | 0.5 | 0.9 | 1.2 | 1.3 | 0.9 | 0.8 | 1.47 |
| YJL133W | 1.0 | 1.0 | 2.0 | 0.9 | 0.9 | 0.5 | 0.5 | 0.9 | 0.6 | 1.4 | 1.2 | 0.8 | 0.8 | 0.7 | 0.9 | 0.6 | 0.8 | 0.7 | 0.91 |
| YJR077C | 1.1 | 1.1 | 2.0 | 1.6 | 0.9 | 0.9 | 0.7 | 0.8 | 0.4 | 0.7 | 1.6 | 1.0 | 0.9 | 0.7 | 1.5 | 0.7 | 1.0 | 0.8 | 1.79 |
| YJR121W | 0.9 | 1.1 | 3.3 | 1.0 | 0.8 | 1.5 | 1.0 | 0.7 | 0.8 | 1.3 | 0.9 | 0.8 | 1.0 | 0.7 | 0.9 | 1.2 | 1.1 | 1.4 | 3.99 |
| YKL148C | 0.9 | 0.8 | 3.7 | 0.7 | 0.8 | 0.5 | 0.6 | 0.8 | 1.7 | 1.7 | 1.5 | 0.8 | 0.8 | 0.8 | 0.6 | 1.1 | 0.8 | 1.0 | 0.54 |
| YLL041C | 1.1 | 0.5 | 4.6 | 1.0 | 1.6 | 1.2 | 0.3 | 0.8 | 0.7 | 0.8 | 1.3 | 0.8 | 1.0 | 1.2 | 0.4 | 3.1 | 1.1 | 1.7 | 1.75 |
| YLR304C | 0.7 | 0.6 | 5.0 | 0.7 | 0.6 | 1.9 | 0.6 | 0.6 | 0.1 | 2.2 | 1.6 | 1.0 | 0.5 | 0.7 | 1.6 | 1.8 | 0.5 | 0.7 | 2.39 |

| | | | | | | | | | | | | | | | | | | | |
|---|---|---|---|---|---|---|---|---|---|---|---|---|---|---|---|---|---|---|---|
| YOR142W | 1.0 | 1.2 | 3.4 | 1.0 | 1.4 | 1.6 | 1.0 | 0.8 | 1.1 | 1.5 | 1.5 | 0.8 | 1.4 | 0.8 | 1.1 | 0.8 | 0.9 | 1.0 | 1.31 |
| YOR176W | 0.7 | 2.6 | 2.7 | 0.9 | 1.0 | 0.9 | 0.5 | 1.0 | 1.0 | 0.5 | 1.4 | 1.3 | 1.5 | 0.8 | 0.8 | 1.4 | 1.2 | 2.1 | 1.23 |
| YPL135W | 0.9 | 1.2 | 2.5 | 1.2 | 1.6 | 1.3 | 1.2 | 1.0 | 0.5 | 2.8 | 1.3 | 1.1 | 1.2 | 1.1 | 1.5 | 1.2 | 1.5 | 1.7 | 1.50 |
| YDR529C | 1.8 | 1.0 | 0.9 | 3.2 | 1.2 | 0.8 | 0.5 | 1.3 | 0.5 | 0.6 | 0.8 | 0.6 | 0.7 | 1.5 | 0.5 | 1.8 | 1.0 | 2.3 | 3.11 |
| YGL018C | 1.1 | 1.0 | 1.8 | 3.0 | 1.1 | 0.4 | 1.1 | 0.8 | 0.5 | 1.4 | 1.1 | 1.0 | 1.3 | 1.2 | 1.1 | 1.5 | 0.8 | 1.0 | 0.36 |
| YBR003W | 0.9 | 0.8 | 1.0 | 1.9 | 0.7 | 1.2 | 1.1 | 0.9 | 1.0 | 1.3 | 1.3 | 0.8 | 1.2 | 0.8 | 0.7 | 2.0 | 1.1 | 1.3 | 1.06 |
| YBR044C | 0.8 | 1.4 | 0.7 | 1.9 | 1.4 | 1.0 | 1.5 | 1.1 | 0.8 | 1.2 | 1.1 | 0.8 | 1.2 | 0.9 | 0.9 | 1.4 | 0.9 | 1.3 | 0.57 |
| YBR091C | 1.1 | 1.0 | 0.6 | 3.0 | 1.1 | 0.7 | 1.0 | 1.5 | 0.7 | 1.6 | 0.7 | 0.8 | 1.2 | 1.7 | 0.9 | 0.9 | 1.0 | 1.0 | 0.87 |
| YBR185C | 1.2 | 1.0 | 1.3 | 2.0 | 0.8 | 1.2 | 1.2 | 1.1 | 0.9 | 1.6 | 1.2 | 0.7 | 1.0 | 1.7 | 1.0 | 1.7 | 0.8 | 1.3 | 0.89 |
| YBR282W | 1.0 | 1.0 | 0.5 | 2.3 | 1.0 | 1.4 | 1.2 | 1.4 | 1.0 | 0.9 | 0.9 | 0.8 | 1.3 | 1.4 | 1.1 | 1.5 | 0.9 | 1.2 | 1.36 |
| YDR347W | 1.1 | 0.8 | 0.9 | 3.5 | 0.8 | 1.2 | 1.2 | 1.2 | 0.8 | 1.6 | 0.8 | 0.8 | 1.3 | 1.5 | 1.0 | 1.7 | 0.9 | 1.2 | 1.33 |
| YEL039C | 1.1 | 0.7 | 1.3 | 5.1 | 0.9 | 1.5 | 0.6 | 1.1 | 1.2 | 1.2 | 1.7 | 0.6 | 0.5 | 1.4 | 0.4 | 3.5 | 0.7 | 1.1 | 1.59 |
| YGR076C | 1.3 | 1.6 | 0.6 | 2.3 | 0.9 | 1.2 | 1.7 | 1.9 | 1.2 | 1.8 | 1.7 | 1.0 | 1.4 | 2.3 | 0.5 | 2.3 | 0.9 | 1.5 | 0.97 |
| YGR174C | 1.1 | 1.6 | 0.6 | 2.4 | 1.0 | 1.4 | 1.0 | 1.6 | 1.8 | 0.7 | 1.3 | 0.9 | 1.5 | 1.6 | 0.7 | 3.2 | 1.3 | 2.3 | 1.05 |
| YKL003C | 1.1 | 0.7 | 0.8 | 1.8 | 1.0 | 1.0 | 1.4 | 1.2 | 1.5 | 2.2 | 1.7 | 0.7 | 1.3 | 2.0 | 1.0 | 2.3 | 1.0 | 1.2 | 0.90 |
| YKL016C | 1.5 | 1.3 | 0.7 | 1.7 | 1.5 | 0.8 | 1.5 | 1.6 | 1.3 | 1.8 | 1.3 | 1.0 | 1.3 | 1.6 | 0.7 | 1.8 | 1.0 | 1.7 | 2.08 |
| YKL170W | 1.0 | 0.8 | 0.5 | 1.9 | 1.3 | 1.5 | 1.5 | 1.2 | 0.9 | 1.1 | 0.7 | 0.8 | 0.9 | 1.3 | 0.8 | 2.1 | 1.1 | 1.4 | 1.27 |
| YKL194C | 0.9 | 0.7 | 0.8 | 1.6 | 1.8 | 1.4 | 2.1 | 1.1 | 1.3 | 1.4 | 1.6 | 0.6 | 1.1 | 1.4 | 0.8 | 1.2 | 1.1 | 1.1 | 0.52 |
| YLR395C | 1.8 | 1.3 | 2.2 | 1.9 | 0.7 | 0.4 | | 1.0 | 1.0 | 0.9 | 1.5 | 0.6 | 0.5 | 1.7 | 0.4 | 1.2 | 1.2 | 2.3 | 1.76 |
| YML120C | 1.1 | 1.2 | 1.9 | 1.9 | 0.6 | 0.6 | 0.8 | 0.9 | 1.3 | 1.5 | 1.3 | 0.9 | 1.2 | 0.8 | 0.5 | 1.5 | 1.0 | 1.6 | 0.74 |
| YOR100C | 1.3 | 1.4 | 1.6 | 1.7 | 1.0 | 1.2 | 1.6 | 1.0 | 2.0 | 2.6 | 1.4 | 0.8 | 1.0 | 1.4 | 4.3 | 2.8 | 0.9 | 0.8 | 0.32 |
| YOR150W | 1.7 | 1.2 | 1.0 | 1.9 | 0.8 | 0.9 | 1.2 | 1.1 | 0.8 | 1.8 | 1.0 | 0.8 | 1.0 | 1.6 | 0.8 | 1.6 | 0.9 | 1.3 | 1.11 |
| YOR187W | 1.0 | 0.5 | 1.9 | 2.0 | 0.6 | 1.7 | 1.5 | 0.7 | 0.6 | 0.8 | 1.4 | 0.8 | 0.7 | 0.6 | 1.3 | 1.6 | 0.8 | 1.3 | 3.12 |
| YJL166W | 2.1 | 1.5 | 1.6 | 1.3 | 1.6 | 0.7 | 0.8 | 1.4 | 1.3 | 1.2 | 1.4 | 0.7 | 0.8 | 1.9 | 0.5 | 2.3 | 1.7 | 4.0 | 2.31 |
| YMR035W | 1.2 | 1.7 | 1.5 | 1.7 | 0.8 | 1.5 | 2.3 | 1.4 | 1.7 | 1.6 | 1.5 | 0.5 | 1.4 | 1.3 | 1.1 | 1.5 | 2.2 | 2.4 | 0.96 |
| YBL038W | 1.0 | 0.9 | 0.5 | 1.8 | 0.9 | 0.7 | 8.0 | 1.6 | 1.4 | 1.5 | 0.7 | 0.7 | 1.0 | 2.3 | 0.7 | 1.8 | 1.2 | 1.9 | 1.56 |
| YDR377W | 1.3 | 1.1 | 1.3 | 1.4 | 1.9 | 1.3 | 1.1 | 1.3 | 1.0 | 0.9 | 1.4 | 0.9 | 0.7 | 1.4 | 0.6 | 1.8 | 1.2 | 2.5 | 3.00 |
| YGL187C | 1.5 | 0.8 | 1.6 | 1.8 | 1.3 | 1.1 | 0.6 | 0.7 | 0.6 | 0.6 | 0.7 | 0.8 | 0.7 | 0.9 | 0.3 | 1.4 | 0.9 | 2.4 | 2.73 |
| YCR046C | 1.0 | 1.2 | 1.2 | 1.6 | 1.0 | 0.8 | 1.2 | 0.9 | 1.9 | 1.4 | 1.5 | 1.1 | 1.5 | 1.3 | 1.4 | 2.8 | 1.3 | 1.0 | 0.63 |
| YML129C | 1.1 | 0.9 | 0.9 | 1.2 | 1.7 | 1.7 | 1.8 | 1.2 | 1.4 | 2.2 | 1.2 | 0.8 | 1.0 | 1.4 | 1.1 | 2.9 | 1.3 | 1.3 | 1.04 |
| YOL096C | 1.1 | 1.0 | 1.2 | 1.6 | 1.2 | 1.3 | 1.7 | 1.0 | 1.3 | 1.4 | 1.2 | 1.2 | 1.4 | 1.4 | 1.0 | 2.3 | 1.2 | 1.2 | 0.64 |

| | | | | | | | | | | | | | | | | | | |
|---|---|---|---|---|---|---|---|---|---|---|---|---|---|---|---|---|---|---|
| YBR122C | 0.9 | 0.9 | 0.3 | 1.1 | 1.5 | 1.1 | 2.0 | 1.3 | 1.1 | 1.4 | 1.2 | 0.7 | 0.8 | 1.2 | 0.7 | 1.9 | 0.9 | 1.5 | 1.53 |
| YBR251W | 1.0 | 0.9 | 1.0 | 1.2 | 1.3 | 2.0 | 1.4 | 1.3 | 0.5 | 1.1 | 1.5 | 1.1 | 1.0 | 1.6 | 0.8 | 3.0 | 0.9 | 1.4 | 1.13 |
| YCR083W | 1.1 | 2.8 | 1.5 | 1.8 | 1.6 | 2.4 | 1.8 | 1.5 | 1.8 | 1.8 | 1.8 | 1.1 | 1.8 | 2.4 | 1.0 | 3.1 | 1.3 | 1.3 | 0.98 |
| YDL067C | 1.8 | 1.0 | 1.6 | 1.3 | 2.0 | 0.9 | 0.8 | 1.1 | 0.9 | 0.9 | 1.4 | 1.0 | 0.6 | 1.4 | 0.7 | 1.8 | 1.2 | 1.7 | 1.85 |
| YDR079W | 1.3 | 1.1 | 0.8 | 1.1 | 1.2 | 1.7 | 1.3 | 2.6 | 0.9 | 0.8 | 1.3 | 1.1 | 1.3 | 1.5 | 0.6 | 2.6 | 1.1 | 1.3 | 1.06 |
| YGR062C | 0.9 | 1.0 | 0.7 | 1.4 | 0.7 | 0.9 | 1.3 | 0.8 | 1.0 | 1.4 | 1.6 | 1.0 | 1.5 | 1.2 | 0.5 | 1.8 | 0.9 | 1.1 | 0.54 |
| YJL096W | 1.0 | 1.0 | 0.8 | 1.3 | 1.9 | 1.0 | 1.7 | 1.3 | 1.0 | 1.4 | 1.2 | 1.0 | 0.7 | 2.1 | 1.0 | 2.2 | 1.1 | 1.2 | 1.21 |
| YJL180C | 0.9 | 1.6 | 1.1 | 1.3 | 1.5 | 1.3 | 1.6 | 1.0 | 1.4 | 1.4 | 1.4 | 0.8 | 1.2 | 1.4 | 0.6 | 2.0 | 0.9 | 1.4 | 1.08 |
| YLR295C | 1.4 | 1.1 | 1.1 | 1.7 | 2.2 | 1.1 | 1.1 | 1.2 | 1.4 | 1.2 | 1.3 | 0.5 | 0.8 | 1.2 | 0.5 | 2.4 | 0.9 | 1.5 | 1.07 |
| YMR023C | 1.4 | 1.3 | 0.5 | 1.9 | 1.7 | 1.1 | 1.9 | 1.4 | 1.5 | 2.6 | 0.8 | 0.7 | 1.1 | 1.6 | 0.7 | 2.1 | 1.1 | 1.4 | 0.48 |
| YMR267W | 0.8 | 1.8 | 0.7 | 0.9 | 1.0 | 1.4 | 1.2 | 1.5 | 0.4 | 0.8 | 1.0 | 0.8 | 0.7 | 1.0 | 0.9 | 2.6 | 0.9 | 1.3 | 0.94 |
| YNL073W | 0.8 | 1.4 | 0.9 | 1.7 | 0.6 | 0.5 | 1.1 | 1.0 | 0.9 | 1.8 | 1.1 | 0.7 | 1.2 | 0.7 | 0.7 | 1.8 | 0.8 | 1.0 | 0.52 |
| YOR316C | 0.7 | 2.5 | 1.5 | 1.5 | 1.0 | 0.9 | 1.4 | 0.8 | 1.6 | 2.0 | 2.4 | 1.1 | 1.6 | 1.0 | 1.5 | 2.2 | 0.8 | 1.0 | 0.93 |
| YPL040C | 1.1 | 1.0 | 0.8 | 1.7 | 0.8 | 1.1 | 0.8 | 1.1 | 0.2 | 1.2 | 0.9 | 0.9 | 1.0 | 1.1 | 1.1 | 2.7 | 0.8 | 1.1 | 0.33 |
| YPL134C | 1.5 | 1.0 | 1.6 | 1.6 | 1.4 | 1.2 | 1.2 | 1.3 | 0.6 | 0.8 | 1.7 | 0.8 | 1.0 | 1.4 | 1.1 | 3.3 | 1.3 | 1.3 | 0.70 |

**Table 3 DNA repair protein genes**

| yeast genes | The level of expressed mRNA in the presence of chemical substance/ The level of expressed mRNA in the absence of chemical substance | | | | | | | | | | | | | | | | | |
|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YFL014W | 3.4 | 5.1 | 5.7 | 11.0 | 1.0 | 9.3 | 5.5 | 3.4 | 13.1 | 5.8 | 5.0 | 4.3 | 15.2 | 7.3 | 6.3 | 14.2 | 1.5 | 8.8 | 2.14 |
| YGL163C | 0.9 | 0.8 | 0.6 | 4.3 | 1.5 | 0.9 | 1.0 | 1.4 | 5.8 | 2.3 | 0.4 | 0.9 | 3.1 | 1.6 | 1.8 | 1.0 | 0.9 | 0.8 | 0.26 |
| YKL145W | 0.8 | 1.4 | 1.2 | 1.0 | 1.4 | 2.1 | 1.6 | 1.5 | 3.5 | 2.7 | 1.8 | 1.3 | 2.7 | 1.0 | 1.3 | 1.2 | 1.1 | 1.5 | 2.29 |
| YIL153W | 0.8 | 1.4 | 1.3 | 2.9 | 1.0 | 0.9 | 1.6 | 0.5 | 4.3 | 1.1 | 1.0 | 1.0 | 1.2 | 1.0 | 2.8 | 1.3 | 0.9 | 0.7 | 0.32 |
| YIR002C | 0.8 | 1.1 | 0.5 | 0.6 | 1.2 | 2.2 | 1.3 | 1.2 | 1.7 | 1.4 | 0.8 | 1.3 | 1.2 | 1.1 | 1.1 | 1.4 | 1.1 | 1.2 | 0.58 |
| YAL015C | 1.2 | 1.1 | 1.7 | 1.9 | 1.1 | 0.7 | 1.2 | 1.1 | 4.0 | 2.7 | 1.4 | 1.0 | 1.2 | 1.6 | 0.7 | 2.0 | 1.1 | 1.2 | 0.61 |
| YBR073W | 0.7 | 1.0 | 0.9 | 0.7 | 1.2 | 0.7 | 0.6 | 1.1 | 2.5 | 1.8 | 0.5 | 0.9 | 1.4 | 1.0 | 1.3 | 0.8 | 0.7 | 0.8 | 0.63 |
| YDL200C | 1.4 | 2.0 | 1.2 | 1.8 | 1.1 | 0.6 | 1.7 | 1.5 | 2.4 | 1.8 | 1.2 | 1.1 | 1.1 | 1.8 | 0.7 | 1.8 | 0.9 | 1.6 | 0.79 |
| YGL058W | 1.1 | 1.1 | 0.6 | 0.7 | 1.0 | 1.0 | 0.0 | 1.3 | 3.7 | 1.8 | 0.8 | 1.3 | 1.0 | 1.3 | 1.3 | 0.6 | 1.1 | 0.7 | 1.07 |
| YIL143C | 0.9 | 0.8 | 1.1 | 0.9 | 0.8 | 1.4 | 0.8 | 0.9 | 7.1 | 3.5 | 1.5 | 0.9 | 1.7 | 1.0 | 1.3 | 1.1 | 0.9 | 0.9 | 0.56 |
| YML032C | 0.8 | 0.9 | 1.1 | 0.7 | 1.0 | 0.9 | 0.8 | 0.9 | 3.0 | 1.1 | 1.0 | 0.9 | 1.8 | 1.0 | 1.7 | 1.2 | 1.0 | 1.0 | 0.66 |
| YNL250W | 0.9 | 2.2 | 0.8 | 1.2 | 0.8 | 0.8 | 1.2 | 1.3 | 4.2 | 2.3 | 1.0 | 1.4 | 1.3 | 1.6 | 1.3 | 1.4 | 1.0 | 1.0 | 0.31 |
| YOR386W | 1.0 | 0.8 | 1.3 | 1.5 | 1.2 | 3.3 | 1.8 | 1.0 | 4.1 | 3.0 | 1.4 | 1.2 | 2.4 | 0.9 | 1.3 | 1.2 | 2.1 | 1.7 | 0.46 |
| YBL019W | 0.9 | 9.1 | 0.7 | 1.2 | 1.0 | 1.0 | 1.2 | 1.2 | 2.6 | 1.4 | 1.0 | 0.8 | 0.9 | 1.1 | 0.6 | 1.9 | 0.8 | 1.1 | 0.38 |
| YDR369C | 1.1 | 1.1 | 2.4 | 1.1 | 1.0 | 0.6 | 0.4 | 1.0 | 2.4 | 1.8 | 1.1 | 0.9 | 0.1 | 0.7 | 1.2 | 0.6 | 0.8 | 0.7 | 1.88 |
| YEL037C | 0.9 | 2.0 | 0.9 | 1.0 | 0.5 | 0.6 | 0.8 | 0.8 | 2.6 | 1.2 | 1.0 | 1.1 | 2.0 | 1.0 | 1.6 | 0.5 | 1.0 | 0.8 | 0.82 |
| YER162C | 1.0 | 1.2 | 1.0 | 1.1 | 0.9 | 0.7 | 0.5 | 0.9 | 2.8 | 1.6 | 0.5 | 0.4 | 0.8 | 0.8 | 1.0 | 1.0 | 1.2 | 0.9 | 0.45 |
| YGR258C | 0.7 | 0.9 | 0.7 | 0.7 | 1.5 | 2.2 | 1.5 | 0.8 | 2.5 | 1.5 | 0.3 | 0.9 | 1.9 | 1.2 | 1.1 | 1.2 | 1.0 | 0.9 | 0.37 |
| YJR052W | 1.1 | 1.0 | 1.1 | 1.0 | 1.0 | 1.4 | 1.2 | 1.7 | 4.5 | 2.3 | 1.1 | 1.1 | 1.8 | 1.4 | 1.4 | 1.4 | 1.0 | 1.3 | 0.36 |
| YOR005C | 1.2 | 1.8 | 1.0 | 0.8 | 1.3 | 1.3 | 1.4 | 1.3 | 2.4 | 1.5 | 1.2 | 0.9 | 2.1 | 1.2 | 0.8 | 1.4 | 1.1 | 1.0 | 0.29 |
| YPL022W | 0.7 | 0.8 | 0.7 | 1.2 | 0.8 | 5.2 | 0.8 | 1.0 | 2.1 | 1.6 | 0.8 | 0.7 | 1.4 | 0.9 | 0.9 | 1.0 | 0.9 | 0.9 | 0.72 |
| YPL164C | 1.1 | 0.7 | 1.1 | 1.0 | 1.2 | 1.7 | 0.9 | 1.0 | 2.1 | 2.0 | 1.1 | 0.9 | 1.0 | 0.9 | 1.3 | 1.4 | 1.0 | 1.0 | 0.25 |
| YPL194W | 0.9 | 1.4 | 0.4 | 1.1 | 1.3 | 1.1 | 1.5 | 1.3 | 9.6 | 3.1 | 0.9 | 0.9 | 1.4 | 1.1 | 1.4 | 0.9 | 1.0 | 0.8 | 0.22 |
| YPR025C | 1.1 | 0.8 | 0.6 | 0.6 | 1.6 | 0.9 | 1.4 | 1.3 | 1.9 | 1.7 | 0.9 | 0.7 | 1.1 | 1.3 | 1.1 | 1.3 | 1.1 | 1.5 | 0.87 |
| YGR180C | 3.1 | 1.1 | 1.9 | 1.0 | 2.0 | 0.6 | 0.5 | 1.0 | 1.4 | 1.5 | 1.0 | 0.9 | 0.9 | 0.9 | 1.3 | 1.2 | 1.0 | 1.9 | 3.90 |
| YEL019C | 1.1 | 1.6 | 0.3 | 0.7 | 0.9 | 1.5 | 1.2 | 0.9 | 0.7 | 0.7 | 1.2 | 1.0 | 1.0 | 1.7 | 1.5 | 0.3 | 0.8 | 0.9 | 0.27 |
| YLR288C | 1.2 | 0.8 | 1.6 | 1.8 | 1.7 | 1.2 | 1.3 | 1.5 | 0.8 | 1.3 | 1.4 | 0.6 | 0.7 | 1.2 | 0.9 | 1.5 | 1.2 | 1.3 | 0.31 |
| YMR284W | 1.1 | 1.0 | 0.5 | 2.0 | 0.8 | 0.8 | 0.8 | 1.1 | 1.6 | 1.8 | 1.1 | 0.6 | 1.0 | 1.2 | 0.7 | 1.3 | 1.1 | 1.3 | 0.54 |

| | | | | | | | | | | | | | | | | | | |
|---|---|---|---|---|---|---|---|---|---|---|---|---|---|---|---|---|---|---|
| YMR035W | 1.2 | 1.7 | 1.5 | 1.7 | 0.8 | 1.5 | 2.3 | 1.4 | 1.7 | 1.6 | 1.5 | 0.5 | 1.4 | 1.3 | 1.1 | 1.5 | 2.2 | 2.4 | 0.96 |
| YOL043C | 1.1 | 0.9 | 0.8 | 1.7 | 1.2 | 0.8 | 1.0 | 0.9 | 1.0 | 1.8 | 1.2 | 0.8 | 0.9 | 1.5 | 0.6 | 2.5 | 1.0 | 1.0 | 0.38 |
| YGR231C | 1.0 | 1.2 | 0.7 | 1.0 | 1.3 | 1.9 | 2.1 | 1.1 | 3.2 | 2.3 | 1.6 | 1.3 | 3.9 | 2.2 | 1.3 | 2.5 | 1.0 | 1.5 | 1.74 |
| YHR164C | 0.9 | 1.3 | 0.9 | 0.7 | 0.9 | 1.4 | 0.8 | 1.1 | 1.3 | 3.1 | 0.6 | 1.2 | 2.0 | 1.3 | 2.2 | 1.1 | 0.8 | 1.0 | 0.44 |
| YJR046W | 1.0 | 1.2 | 1.2 | 1.5 | 0.8 | 2.0 | 0.9 | 0.9 | 6.0 | 2.4 | 0.6 | 0.9 | 3.0 | 1.0 | 1.1 | 0.8 | 0.7 | 0.7 | 0.45 |
| YLR103C | 0.8 | 1.5 | 1.1 | 0.4 | 1.0 | 1.1 | 1.3 | 1.4 | 0.8 | 0.5 | 0.9 | 1.6 | 3.0 | 1.0 | 0.8 | 1.5 | 1.1 | 1.4 | 1.32 |
| YMR072W | 1.1 | 1.0 | 1.4 | 1.0 | 1.7 | 1.6 | 1.4 | 1.0 | 3.0 | 1.2 | 1.9 | 0.8 | 2.1 | 1.2 | 0.8 | 1.7 | 0.9 | 1.9 | 2.06 |
| YDR054C | 1.3 | 1.0 | 0.7 | 1.4 | 0.7 | 1.9 | 1.1 | 1.5 | 2.9 | 3.2 | 1.9 | 1.0 | 2.5 | 1.1 | 1.6 | 1.4 | 0.8 | 1.4 | 1.18 |
| YAR007C | 0.7 | 1.9 | 0.8 | 0.5 | 1.5 | 1.6 | 0.8 | 1.1 | 4.1 | 1.7 | 0.9 | 0.9 | 1.7 | 1.4 | 1.2 | 0.9 | 0.8 | 1.0 | 1.03 |
| YGL058W | 1.1 | 1.1 | 0.6 | 0.7 | 1.0 | 1.5 | 0.0 | 1.3 | 3.7 | 1.8 | 0.8 | 1.3 | 1.0 | 1.3 | 1.3 | 0.6 | 1.1 | 0.7 | 1.07 |
| YIL036W | 0.9 | 1.3 | 1.3 | 1.8 | 0.9 | 1.8 | 1.4 | 1.1 | 3.6 | 2.7 | 1.2 | 1.2 | 1.9 | 1.1 | 0.8 | 1.3 | 1.1 | 1.3 | 0.66 |
| YNL213C | 1.7 | 11.6 | 0.9 | 1.5 | 1.3 | 1.7 | 1.4 | 1.8 | 3.0 | 1.5 | 1.5 | 0.8 | 0.9 | 1.8 | 1.0 | 1.7 | 0.9 | 1.2 | 0.70 |
| YNL312W | 0.9 | 1.0 | 1.6 | 0.7 | 1.4 | 1.7 | 0.5 | 1.2 | 3.7 | 2.5 | 1.4 | 0.9 | 0.7 | 1.1 | 0.8 | 0.8 | 1.2 | 1.1 | 2.05 |
| YNL261W | 1.0 | 1.3 | 0.6 | 1.3 | 0.7 | 1.3 | 1.0 | 1.1 | 2.3 | 1.5 | 0.8 | 0.8 | 0.8 | 1.4 | 0.7 | 1.1 | 0.8 | 1.0 | 0.91 |
| YGR180C | 3.1 | 1.1 | 1.9 | 1.0 | 2.0 | 1.0 | 0.5 | 1.0 | 1.4 | 1.5 | 1.0 | 0.9 | 0.9 | 0.9 | 1.3 | 1.2 | 1.0 | 1.9 | 3.90 |
| YJL026W | 2.5 | 2.4 | 2.9 | 1.0 | 1.7 | 1.0 | 0.5 | 1.2 | 1.0 | 1.1 | 1.3 | 1.0 | 1.2 | 1.0 | 1.1 | 1.6 | 1.4 | 2.1 | 3.74 |
| YDL017W | 0.8 | 2.3 | 1.2 | 1.3 | 1.2 | 1.1 | 1.1 | 1.2 | 0.8 | 1.8 | 0.8 | 0.8 | 0.9 | 1.3 | 1.3 | 1.0 | 0.8 | 1.0 | 0.38 |
| YML058W | 1.9 | 1.2 | 5.8 | 1.9 | 0.6 | 1.3 | 0.6 | 0.7 | 1.1 | 1.2 | 3.3 | 0.9 | 1.4 | 1.1 | 2.5 | 1.3 | 1.3 | 1.5 | 2.14 |
| YLR233C | 1.1 | 0.9 | 2.0 | 2.0 | 1.2 | 0.8 | 0.8 | 1.1 | 0.6 | 0.5 | 0.8 | 0.9 | 1.0 | 1.1 | 1.1 | 1.1 | 1.0 | 1.2 | 0.43 |
| YMR284W | 1.1 | 1.0 | 0.5 | 2.0 | 0.8 | 1.2 | 0.8 | 1.1 | 1.6 | 1.8 | 1.1 | 0.6 | 1.0 | 1.2 | 0.7 | 1.3 | 1.1 | 1.3 | 0.54 |
| YGL163C | 0.9 | 0.8 | 0.6 | 4.3 | 1.5 | 0.9 | 1.0 | 1.4 | 5.8 | 2.3 | 0.4 | 0.9 | 3.1 | 1.6 | 1.8 | 1.0 | 0.9 | 0.8 | 0.26 |
| YGL127C | 1.0 | 1.9 | 1.3 | 1.7 | 1.3 | 0.6 | 1.0 | 0.9 | 1.2 | 1.5 | 1.0 | 1.4 | 1.7 | 1.0 | 1.5 | 1.3 | 1.0 | 1.2 | 0.54 |
| YMR072W | 1.1 | 1.0 | 1.4 | 1.0 | 1.7 | 2.4 | 1.4 | 1.0 | 3.0 | 1.2 | 1.9 | 0.8 | 2.1 | 1.2 | 0.8 | 1.7 | 0.9 | 1.9 | 2.06 |
| YGL249W | 1.4 | 0.8 | 0.5 | 0.8 | 1.1 | 2.5 | 1.2 | 0.9 | 0.3 | 2.4 | 0.6 | 0.8 | 0.8 | 1.9 | 0.7 | 1.4 | 1.0 | 1.0 | 0.20 |
| YBR272C | 0.8 | 1.8 | 1.3 | 1.0 | 1.1 | | 1.9 | 1.1 | 1.7 | 2.7 | 1.1 | 0.5 | 1.3 | 1.0 | 0.5 | 1.7 | 1.1 | 1.2 | 0.52 |
| YDL059C | 2.6 | 5.1 | 1.4 | 1.7 | 1.2 | 1.2 | 1.1 | 1.2 | 10.5 | 7.8 | 2.0 | 1.3 | 1.7 | 4.7 | 0.7 | 1.5 | 0.8 | 0.9 | 0.63 |
| YAR007C | 0.7 | 1.9 | 0.8 | 0.5 | 1.5 | 0.6 | 0.8 | 1.1 | 4.1 | 1.7 | 0.9 | 0.9 | 1.7 | 1.4 | 1.2 | 0.9 | 0.8 | 1.0 | 1.03 |
| YBR073W | 0.7 | 1.0 | 0.9 | 0.7 | 1.2 | 0.7 | 0.6 | 1.1 | 2.5 | 1.8 | 0.5 | 0.9 | 1.4 | 1.0 | 1.3 | 0.8 | 0.7 | 0.8 | 0.63 |
| YML032C | 0.8 | 0.9 | 1.1 | 0.7 | 1.0 | 0.9 | 0.8 | 0.9 | 3.0 | 1.1 | 1.0 | 0.9 | 1.8 | 1.0 | 1.7 | 1.2 | 1.0 | 1.0 | 0.66 |
| YNL250W | 0.9 | 2.2 | 0.8 | 1.2 | 0.8 | 0.8 | 1.2 | 1.3 | 4.2 | 2.3 | 1.0 | 1.4 | 1.3 | 1.6 | 1.3 | 1.4 | 1.0 | 1.0 | 0.31 |
| YCR014C | 0.8 | 1.0 | 0.8 | 0.6 | 1.2 | 1.1 | 1.1 | 1.0 | 2.4 | 1.0 | 0.8 | 0.7 | 1.2 | 1.0 | 0.7 | 1.5 | 1.0 | 0.9 | 0.34 |

| | | | | | | | | | | | | | | | | | | |
|---|---|---|---|---|---|---|---|---|---|---|---|---|---|---|---|---|---|---|
| YDR369C | 1.1 | 1.1 | 2.4 | 1.1 | 1.0 | 0.6 | 0.4 | 1.0 | 2.4 | 1.8 | 1.1 | 0.9 | 0.1 | 0.7 | 1.2 | 0.6 | 0.8 | 0.7 | 1.88 |
| YIL072W | 0.8 | 3.1 | 1.1 | 2.6 | 1.5 | 1.2 | 1.7 | 1.4 | 2.9 | 2.1 | 1.8 | 1.1 | 1.4 | 2.0 | 1.4 | 1.4 | 1.0 | 1.1 | 0.23 |
| YOR005C | 1.2 | 1.8 | 1.0 | 0.8 | 1.3 | 1.3 | 1.4 | 1.3 | 2.4 | 1.5 | 1.2 | 0.9 | 2.1 | 1.2 | 0.8 | 1.4 | 1.1 | 1.0 | 0.29 |
| YPL164C | 1.1 | 0.7 | 1.1 | 1.0 | 1.2 | 1.7 | 0.9 | 1.0 | 2.1 | 2.0 | 1.1 | 0.9 | 1.0 | 0.9 | 1.3 | 1.4 | 1.0 | 1.0 | 0.25 |
| YPL194W | 0.9 | 1.4 | 0.4 | 1.1 | 1.3 | 1.1 | 1.5 | 1.3 | 9.6 | 3.1 | 0.9 | 0.9 | 1.4 | 1.1 | 1.4 | 0.9 | 1.0 | 0.8 | 0.22 |
| YGR180C | 3.1 | 1.1 | 1.9 | 1.0 | 2.0 | 0.6 | 0.5 | 1.0 | 1.4 | 1.5 | 1.0 | 0.9 | 0.9 | 0.9 | 1.3 | 1.2 | 1.0 | 1.9 | 3.90 |
| YEL019C | 1.1 | 1.6 | 0.3 | 0.7 | 0.9 | 1.5 | 1.2 | 0.9 | 0.7 | 0.7 | 1.2 | 1.0 | 1.0 | 1.7 | 1.5 | 0.3 | 0.8 | 0.9 | 0.27 |
| YML058W | 1.9 | 1.2 | 5.8 | 1.9 | 0.6 | 0.6 | 0.6 | 0.7 | 1.1 | 1.2 | 3.3 | 0.9 | 1.4 | 1.1 | 2.5 | 1.3 | 1.3 | 1.5 | 2.14 |
| YLR288C | 1.2 | 0.8 | 1.6 | 1.8 | 1.7 | 1.2 | 1.3 | 1.5 | 0.8 | 1.3 | 1.4 | 0.6 | 0.7 | 1.2 | 0.9 | 1.5 | 1.2 | 1.3 | 0.31 |
| YMR284W | 1.1 | 1.0 | 0.5 | 2.0 | 0.8 | 0.8 | 0.8 | 1.1 | 1.6 | 1.8 | 1.1 | 0.6 | 1.0 | 1.2 | 0.7 | 1.3 | 1.1 | 1.3 | 0.54 |
| YMR096W | 1.7 | 1.7 | 1.1 | 0.8 | 1.0 | 2.2 | 1.9 | 2.0 | 3.3 | 3.7 | 1.5 | 1.4 | 8.4 | 4.7 | 28.9 | 2.2 | 2.2 | 1.0 | 0.62 |
| YGL091C | 2.0 | 1.8 | 1.0 | 1.7 | 0.8 | 1.6 | 1.3 | 1.8 | 10.8 | 6.0 | 2.1 | 1.2 | 2.5 | 3.1 | 0.7 | 1.0 | 1.4 | 1.8 | 0.96 |

**Table 4 Energy system protein genes**

| yeast genes | The level of expressed mRNA in the presence of chemical substance/ The level of expressed mRNA in the absence of chemical substance | | | | | | | | | | | | | | | | | | |
|---|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YCR107W | 12.1 | 2.6 | 1.8 | 1.6 | 0.7 | 1.5 | 1.5 | 15.6 | 196.6 | 34.0 | 23.0 | 0.9 | 4.0 | 8.3 | 4.8 | 3.1 | 1.3 | 1.1 | 0.59 |
| YDL243C | 14.5 | 2.7 | 2.4 | 1.0 | 1.1 | 2.2 | 1.6 | 11.8 | 64.2 | 29.6 | 19.6 | 1.1 | 4.1 | 12.0 | 4.2 | 3.0 | 1.8 | 1.5 | 0.76 |
| YFL056C | 19.0 | 2.3 | 2.3 | 1.5 | 0.9 | 0.6 | 1.4 | 18.5 | 162.3 | 31.3 | 68.3 | 1.0 | 4.7 | 7.8 | 5.0 | 3.4 | 1.0 | 1.1 | 0.55 |
| YFL057C | 20.9 | 5.9 | 1.5 | 1.8 | 0.9 | 1.2 | 1.8 | 18.0 | 51.8 | 46.1 | 27.7 | 1.0 | 4.1 | 23.4 | 3.1 | 3.9 | 1.6 | 1.3 | 0.71 |
| YJR155W | 7.6 | 3.7 | 1.4 | 2.5 | 0.7 | 1.4 | 1.7 | 10.6 | 38.2 | 18.8 | 15.4 | 1.0 | 5.7 | 9.4 | 2.6 | 5.6 | 1.3 | 1.4 | 0.64 |
| YNL331C | 8.6 | 3.6 | 1.3 | 1.0 | 1.6 | 1.8 | 1.9 | 13.1 | 42.6 | 36.3 | 21.8 | 0.9 | 3.1 | 7.5 | 2.3 | 4.0 | 1.7 | 1.3 | 0.58 |
| YOL165C | 10.1 | 4.5 | 1.8 | 0.9 | 0.9 | 1.7 | 1.4 | 17.8 | 46.9 | 23.3 | 17.6 | 0.8 | 3.7 | 9.1 | 3.0 | 1.8 | 1.6 | 1.0 | 0.69 |
| YPL171C | 15.2 | 4.1 | 3.5 | 2.2 | 1.1 | 1.3 | 1.7 | 20.5 | 60.0 | 50.6 | 37.0 | 1.4 | 2.4 | 9.3 | 1.4 | 1.2 | 2.4 | 1.5 | 0.47 |
| YDL021W | 5.1 | 1.7 | 2.4 | 3.7 | 1.7 | 6.5 | 5.9 | 2.7 | 2.5 | 7.4 | 4.7 | 2.4 | 5.3 | 3.7 | 0.7 | 7.3 | 1.9 | 3.2 | 0.47 |
| YGR043C | 2.6 | 3.7 | 3.2 | 7.9 | 0.9 | 16.3 | 6.5 | 2.6 | 10.9 | 8.4 | 3.6 | 3.3 | 6.9 | 4.1 | 3.0 | 13.7 | 1.6 | 4.8 | 0.66 |
| YHR179W | 3.3 | 2.3 | 2.6 | 0.7 | 1.2 | 3.7 | 1.9 | 3.3 | 3.6 | 5.8 | 2.0 | 2.2 | 2.6 | 5.8 | 2.7 | 1.0 | 1.9 | 1.6 | 2.69 |
| YJR048W | 1.3 | 0.9 | 2.2 | 1.4 | 0.8 | 0.5 | 0.3 | 0.9 | 0.5 | 1.5 | 1.0 | 0.8 | 0.4 | 2.5 | 0.6 | 1.5 | 1.2 | 1.2 | 1.19 |
| YKR097W | 1.6 | 2.4 | 1.3 | 3.3 | 1.1 | 1.7 | 3.7 | 2.5 | 1.9 | 3.3 | 0.8 | 1.8 | 17.5 | 2.1 | 2.5 | 2.2 | 1.1 | 1.5 | 0.16 |
| YML087C | 1.8 | 1.6 | 0.8 | 2.5 | 0.9 | 0.9 | 1.1 | 1.3 | 1.9 | 4.9 | 1.6 | 0.8 | 1.5 | 2.1 | 0.3 | 2.0 | 0.7 | 0.8 | 0.52 |
| YPL088W | 3.3 | 1.3 | 0.6 | 2.5 | 1.5 | 7.1 | 3.7 | 1.9 | 0.6 | 2.8 | 1.1 | 3.6 | 4.8 | 1.6 | 3.2 | 4.3 | 9.1 | 8.3 | 0.69 |
| YDL174C | 0.9 | 1.8 | 0.9 | 1.3 | 0.5 | 1.7 | 3.6 | 0.8 | 0.5 | 1.7 | 0.9 | 2.8 | 5.9 | 1.1 | 0.6 | 5.1 | 2.2 | 4.0 | 0.63 |
| YCR012W | 1.2 | 1.5 | 5.1 | 1.2 | 1.1 | 2.5 | 1.4 | 0.8 | 1.4 | 1.5 | 1.8 | 1.2 | 4.1 | 0.9 | 1.4 | 1.4 | 0.8 | 1.3 | 4.48 |
| YFR053C | 1.8 | 1.4 | 3.0 | 2.2 | 0.9 | 3.2 | 2.1 | 2.8 | 0.6 | 1.1 | 1.5 | 1.7 | 3.0 | 0.6 | 0.6 | 3.0 | 1.4 | 2.2 | 4.17 |
| YGL062W | 0.6 | 1.3 | 1.1 | 0.6 | 0.9 | 1.0 | 1.3 | 0.8 | 1.6 | 2.5 | 0.7 | 1.6 | 4.5 | 1.2 | 2.7 | 1.5 | 1.1 | 0.9 | 0.77 |
| YGR192C | 1.4 | 1.0 | 3.8 | 1.0 | 1.1 | 1.7 | 1.7 | 1.1 | 0.9 | 1.3 | 2.3 | 2.2 | 3.4 | 1.0 | 1.9 | 1.1 | 1.1 | 1.3 | 7.49 |
| YGR244C | 1.0 | 1.2 | 1.6 | 1.6 | 0.8 | 2.6 | 3.9 | 1.8 | 1.4 | 1.1 | 1.8 | 1.9 | 2.6 | 1.2 | 2.0 | 2.6 | 1.9 | 3.1 | 1.12 |
| YGR254W | 1.2 | 1.3 | 3.8 | 1.3 | 1.2 | 1.9 | 1.5 | 1.4 | 0.8 | 1.2 | 1.3 | 1.7 | 3.1 | 0.6 | 2.4 | 1.4 | 1.3 | 1.2 | 7.01 |
| YIL160C | 0.8 | 2.4 | 2.3 | 3.5 | 1.0 | 1.6 | 1.1 | 1.2 | 3.2 | 3.8 | 2.4 | 1.0 | 2.3 | 1.5 | 5.6 | 8.8 | 2.4 | 3.0 | 0.27 |
| YJL052W | 1.6 | 0.9 | 4.0 | 1.8 | 0.7 | 2.4 | 2.1 | 1.5 | 1.6 | 2.0 | 4.3 | 2.4 | 5.6 | 1.3 | 2.3 | 2.3 | 1.2 | 1.9 | 6.19 |
| YJR009C | 1.1 | 1.7 | 5.6 | 1.0 | 1.2 | 1.6 | 1.6 | 1.1 | 1.1 | 1.3 | 1.1 | 1.5 | 2.7 | 1.0 | 2.1 | 1.5 | 1.1 | 1.3 | 5.86 |
| YLR345W | 1.4 | 1.4 | 1.4 | 1.2 | 1.2 | 1.6 | 1.2 | 1.4 | 4.5 | 4.0 | 2.5 | 1.6 | 3.5 | 1.5 | 1.0 | 1.2 | 1.2 | 1.4 | 2.65 |
| YMR118C | 1.3 | 2.9 | 1.4 | 1.6 | 1.2 | 2.0 | 1.0 | 1.0 | 9.3 | 1.4 | 0.7 | 0.8 | 4.2 | 0.9 | 0.9 | 8.1 | 0.8 | 1.0 | 0.38 |
| YNL071W | 0.9 | 0.9 | 1.7 | 1.5 | 0.6 | 1.3 | 1.4 | 0.9 | 1.1 | 1.2 | 1.6 | 1.3 | 2.5 | 0.8 | 1.1 | 1.1 | 1.0 | 1.2 | 1.61 |

| | | | | | | | | | | | | | | | | | | |
|---|---|---|---|---|---|---|---|---|---|---|---|---|---|---|---|---|---|---|
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YPL240C | 0.7 | 1.4 | 2.4 | 0.9 | 1.3 | 1.2 | 1.4 | 1.2 | 3.5 | 2.6 | 1.0 | 0.8 | 2.9 | 0.7 | 1.2 | 0.7 | 0.8 | 1.0 | 4.83 |
| YAL060W | 1.1 | 1.8 | 3.2 | 2.7 | 1.2 | 4.2 | 3.3 | 0.9 | 0.6 | 2.5 | 2.4 | 0.8 | 2.2 | 0.9 | 0.9 | 3.2 | 1.1 | 1.8 | 2.39 |
| YCL040W | 0.9 | 5.6 | 10.1 | 2.0 | 0.5 | 3.5 | 2.9 | 0.7 | 0.9 | 3.0 | 8.2 | 2.3 | 5.6 | 0.7 | 3.4 | 3.1 | 1.4 | 1.7 | 1.98 |
| YDR001C | 1.0 | 2.2 | 2.6 | 1.0 | 1.1 | 2.2 | 1.5 | 0.9 | 2.2 | 3.0 | 0.7 | 1.1 | 2.8 | 1.0 | 4.4 | 1.9 | 1.1 | 1.6 | 0.75 |
| YDR231C | 1.1 | 1.6 | 1.0 | 1.5 | 1.1 | 2.2 | 1.7 | 1.3 | 1.5 | 1.4 | 1.3 | 1.1 | 2.4 | 1.5 | 1.0 | 3.0 | 1.2 | 1.3 | 0.92 |
| YER178W | 0.8 | 0.9 | 3.6 | 1.0 | 0.7 | 2.5 | 1.6 | 0.8 | 1.7 | 1.3 | 2.5 | 1.3 | 2.4 | 0.9 | 1.8 | 1.0 | 1.1 | 0.9 | 2.18 |
| YGR008C | 2.4 | 3.0 | 1.7 | 3.2 | 0.9 | 2.9 | 3.7 | 1.9 | 3.1 | 2.4 | 2.6 | 2.0 | 3.4 | 1.3 | 1.5 | 2.3 | 1.7 | 4.2 | 3.03 |
| YGR256W | 1.2 | 1.5 | 2.3 | 0.8 | 0.9 | 6.2 | 1.4 | 2.2 | 3.4 | 2.5 | 2.7 | 1.5 | 3.9 | 1.1 | 2.7 | 5.3 | 1.1 | 0.8 | 0.94 |
| YHL008C | 0.9 | 0.5 | 1.0 | 1.6 | 0.8 | 0.8 | 0.7 | 0.9 | 1.6 | 1.7 | 1.4 | 0.8 | 1.8 | 1.0 | 1.8 | 0.9 | 0.8 | 0.9 | 0.40 |
| YHR174W | 1.1 | 1.4 | 3.3 | 1.2 | 1.3 | 1.5 | 1.6 | 1.2 | 1.0 | 1.5 | 1.4 | 1.5 | 3.6 | 0.6 | 2.0 | 1.1 | 1.0 | 1.2 | 7.34 |
| YIL045W | 1.7 | 1.4 | 1.9 | 2.2 | 1.3 | 1.7 | 1.6 | 1.1 | 2.1 | 3.2 | 1.2 | 1.5 | 2.0 | 1.6 | 0.6 | 2.9 | 1.1 | 1.8 | 0.37 |
| YKL035W | 1.0 | 0.9 | 4.8 | 1.2 | 0.8 | 1.2 | 0.6 | 1.0 | 0.8 | 1.2 | 2.1 | 1.0 | 2.0 | 0.6 | 1.0 | 1.5 | 1.0 | 1.9 | 2.54 |
| YKL152C | 1.4 | 1.3 | 1.9 | 0.9 | 1.3 | 1.6 | 1.5 | 1.0 | 0.9 | 1.5 | 1.7 | 1.5 | 2.7 | 1.0 | 1.8 | 2.0 | 1.1 | 1.7 | 3.28 |
| YML054C | 1.5 | 1.8 | 1.3 | 3.4 | 1.3 | 1.8 | 1.2 | 1.5 | 4.1 | 1.8 | 1.4 | 1.8 | 2.8 | 2.1 | 1.1 | 7.8 | 1.1 | 1.6 | 0.25 |
| YML100W | 0.8 | 2.7 | 10.6 | 1.4 | 0.9 | 2.8 | 2.2 | 0.7 | 1.7 | 1.4 | 3.2 | 1.2 | 3.8 | 1.2 | 1.8 | 2.2 | 1.0 | 1.5 | 0.88 |
| YML125C | 0.8 | 0.9 | 0.7 | 0.7 | 0.9 | 1.3 | 1.0 | 1.3 | 2.6 | 1.6 | 1.0 | 1.0 | 2.2 | 1.6 | 1.9 | 0.7 | 1.2 | 0.9 | 2.21 |
| YMR089C | 1.0 | 1.1 | 0.9 | 0.9 | 0.8 | 1.2 | 1.2 | 1.3 | 5.6 | 2.5 | 1.3 | 0.8 | 2.3 | 0.9 | 1.8 | 1.1 | 0.9 | 1.1 | 0.69 |
| YMR105C | 2.0 | 3.0 | 5.0 | 4.2 | 0.9 | 2.8 | 2.8 | 1.1 | 0.6 | 2.9 | 3.0 | 1.7 | 3.3 | 1.0 | 0.9 | 2.0 | 1.6 | 2.6 | 1.21 |
| YNL237W | 1.3 | 1.3 | 1.5 | 1.0 | 1.5 | 4.6 | 4.9 | 1.1 | 0.9 | 2.1 | 1.5 | 1.8 | 3.2 | 1.3 | 1.6 | 4.4 | 1.3 | 1.1 | 0.21 |
| YOL126C | 1.1 | 0.8 | 1.0 | 1.6 | 0.7 | 1.5 | 1.4 | 0.9 | 0.8 | 2.4 | 1.8 | 1.1 | 2.6 | 1.2 | 1.4 | 2.5 | 1.6 | 2.4 | 0.59 |
| YOR347C | 0.9 | 0.9 | 2.1 | 2.2 | 0.7 | 0.9 | 0.9 | 0.8 | 0.5 | 0.9 | 1.4 | 1.2 | 1.8 | 0.9 | 2.0 | 1.4 | 1.7 | 1.3 | 1.31 |
| YPR026W | 0.9 | 1.2 | 5.0 | 1.3 | 0.8 | | 1.3 | 1.0 | 0.9 | 3.1 | 1.6 | 1.2 | 2.3 | 1.5 | 3.1 | 2.5 | 0.9 | 1.3 | 0.26 |
| YAL038W | 1.0 | 1.0 | 3.0 | 1.4 | 1.4 | 1.3 | 0.9 | 1.1 | 0.1 | 1.1 | 1.0 | 1.2 | 1.8 | 0.5 | 3.1 | 0.9 | 1.0 | 1.0 | 7.02 |
| YDR380W | 0.8 | 0.8 | 0.8 | 1.7 | 1.1 | 0.4 | 0.5 | 0.2 | 0.2 | 0.3 | 0.4 | 0.4 | 0.4 | 0.6 | 2.4 | 0.2 | 0.8 | 0.8 | 1.04 |
| YLR273C | 1.5 | 1.0 | 1.3 | 2.4 | | 1.2 | 1.2 | 1.3 | 2.8 | 2.4 | 1.4 | 0.9 | 1.7 | 1.3 | 2.6 | 1.5 | 1.0 | 1.0 | 0.20 |
| YGR207C | 1.5 | 1.1 | 1.0 | 1.1 | 1.5 | 2.8 | 1.7 | 1.9 | 2.1 | 2.6 | 1.4 | 1.1 | 1.0 | 2.2 | 0.6 | 1.8 | 1.2 | 1.8 | 1.49 |
| YNL037C | 1.4 | 1.8 | 1.7 | 1.1 | 1.7 | 2.8 | 2.8 | 1.0 | 0.6 | 3.2 | 1.4 | 1.3 | 1.9 | 1.3 | 0.9 | 1.4 | 0.9 | 1.3 | 2.05 |
| YOR136W | 1.0 | 0.9 | 3.5 | 1.0 | 1.5 | 3.9 | 3.2 | 1.1 | 0.3 | 3.4 | 1.4 | 1.2 | 1.5 | 0.9 | 1.2 | 1.3 | 0.8 | 1.3 | 3.20 |
| YPL271W | 1.2 | 3.2 | 4.1 | 1.2 | 1.4 | 3.3 | 1.2 | 0.9 | 0.6 | 1.2 | 1.7 | 0.8 | 1.2 | 1.3 | 0.8 | 1.4 | 1.2 | 1.3 | 1.34 |
| YAL054C | 1.2 | 1.1 | 1.4 | 1.6 | 1.1 | 2.2 | 1.1 | 1.1 | 8.9 | 4.1 | 1.3 | 1.0 | 1.8 | 1.3 | 0.9 | 2.5 | 1.5 | 1.4 | 0.24 |

| | | | | | | | | | | | | | | | | | | | |
|---|---|---|---|---|---|---|---|---|---|---|---|---|---|---|---|---|---|---|---|
| YEL011W | 2.0 | 1.6 | 1.7 | 6.3 | 1.1 | 2.8 | 2.7 | 0.9 | 2.5 | 1.6 | 3.7 | 1.5 | 1.5 | 1.5 | 0.4 | 2.4 | 1.4 | 2.5 | 0.89 |
| YFR015C | 1.2 | 0.8 | 3.2 | 2.1 | 1.2 | 3.0 | 7.3 | 0.7 | 0.3 | 1.8 | 3.9 | 0.4 | 2.5 | 3.1 | 0.4 | 2.4 | 1.3 | 1.2 | 0.66 |
| YGL253W | 0.8 | 1.0 | 3.2 | 0.7 | 1.3 | 2.4 | 1.7 | 1.1 | 0.3 | 1.4 | 1.0 | 1.2 | 1.5 | 0.5 | 0.8 | 0.4 | 0.7 | 0.8 | 4.63 |
| YIL111W | 1.9 | 1.0 | 1.8 | 2.7 | | 2.6 | 3.2 | 1.2 | 1.6 | 1.1 | 2.1 | 0.9 | 1.7 | 1.5 | 1.1 | 2.5 | 1.9 | 3.9 | 1.52 |
| YKL150W | 1.1 | 2.0 | 3.0 | 1.4 | 1.4 | 2.3 | 2.4 | 1.3 | 1.1 | 1.3 | 1.9 | 1.2 | 1.8 | 1.4 | 0.9 | 6.1 | 1.2 | 2.7 | 2.20 |
| YPR006C | 1.9 | 1.5 | 0.5 | 1.9 | 2.0 | 2.4 | 2.8 | 1.5 | 1.5 | 3.9 | 2.4 | 1.1 | 1.8 | 1.2 | 0.7 | 1.6 | 1.8 | 2.1 | 0.46 |
| YBR145W | 1.5 | 0.7 | 2.8 | 0.9 | 1.1 | 11.5 | 58.8 | 1.0 | 0.1 | 1.1 | 1.1 | 1.0 | 2.0 | 2.2 | 1.2 | 3.6 | 1.7 | 2.0 | 2.17 |
| YBR299W | 2.0 | 0.9 | 1.1 | 3.5 | 1.6 | 0.8 | 3.6 | 2.2 | 1.1 | 5.3 | 2.4 | 1.2 | 0.7 | 1.4 | 0.6 | 3.9 | 1.0 | 1.1 | 0.32 |
| YEL020C | 1.0 | 1.5 | 0.8 | 2.9 | 1.5 | 1.3 | 2.4 | 1.2 | 1.4 | 1.1 | 1.3 | 0.8 | 1.2 | 1.0 | 1.4 | 2.1 | 1.2 | 1.3 | 0.31 |
| YGL134W | 1.2 | 1.3 | 0.5 | 0.8 | 1.4 | 1.2 | 2.3 | 1.4 | 1.1 | 1.4 | 1.1 | 0.9 | 0.7 | 1.1 | 0.5 | 1.7 | 0.9 | 1.3 | 0.53 |
| YOL157C | 1.0 | 1.1 | 1.3 | 2.5 | 1.4 | 0.9 | 2.7 | 1.4 | 2.3 | 4.8 | 1.2 | 1.2 | 1.4 | 1.2 | 1.1 | 3.5 | 1.4 | 1.3 | 0.41 |
| YBR126C | 0.8 | 1.9 | 5.6 | 1.2 | 0.7 | 2.9 | 2.3 | 0.6 | 1.7 | | 1.1 | 1.3 | 2.1 | 0.7 | 1.0 | 1.7 | 1.5 | 1.3 | 1.96 |
| YCR005C | 1.2 | 1.9 | 2.0 | 1.2 | 0.9 | 1.6 | 4.4 | 1.2 | 1.5 | 1.5 | 2.1 | 0.5 | 0.7 | 0.8 | 0.7 | 0.7 | 1.6 | 1.7 | 2.38 |
| YIL172C | 1.1 | 1.1 | 1.6 | 1.7 | | 1.3 | 2.5 | 1.6 | 2.8 | 7.1 | 1.0 | 1.4 | 2.0 | 1.3 | 1.4 | 2.8 | 1.1 | 1.2 | 0.42 |
| YOR221C | 0.8 | 1.0 | 0.9 | 1.1 | 1.7 | 0.8 | 2.1 | 0.9 | 1.4 | 1.7 | 1.1 | 0.8 | 1.0 | 1.1 | 1.0 | 1.4 | 0.9 | 1.2 | 0.39 |
| YBR196C | 0.8 | 0.6 | 3.9 | 1.4 | 0.8 | 0.8 | 1.4 | 1.1 | 0.3 | 0.9 | 2.3 | 1.0 | 1.4 | 0.5 | 1.9 | 1.1 | 0.8 | 1.0 | 6.60 |
| YEL047C | 1.3 | 1.9 | 3.2 | 1.2 | 0.8 | 0.6 | 1.3 | 1.0 | 2.7 | 2.3 | 4.2 | 1.2 | 1.6 | 1.1 | 1.0 | 1.3 | 0.9 | 1.0 | 1.02 |
| YMR318C | 1.8 | 2.4 | 2.2 | 0.7 | 1.2 | 2.1 | 0.8 | 3.6 | 2.3 | 4.8 | 3.6 | 0.8 | 1.8 | 1.7 | 1.5 | 1.1 | 1.1 | 1.1 | 3.17 |
| YER061C | 0.9 | 0.9 | 1.2 | 2.5 | 0.8 | 0.4 | 0.9 | 0.7 | 0.3 | 0.7 | 2.2 | 0.7 | 0.9 | 1.0 | 0.8 | 1.8 | 1.2 | 1.2 | 0.84 |
| YJL045W | 1.8 | 2.2 | 1.6 | 5.3 | 0.7 | 0.6 | 0.7 | 0.9 | 9.7 | 1.6 | 2.3 | 1.1 | 1.6 | 1.2 | 3.4 | 2.4 | 0.9 | 0.9 | 0.42 |
| YLL009C | 1.0 | 1.2 | 0.6 | 1.9 | 1.5 | 1.2 | 1.7 | 1.0 | 1.3 | 1.7 | 3.5 | 0.7 | 0.9 | 1.4 | 1.1 | 2.8 | 0.9 | 1.2 | 1.66 |
| YPR160W | 1.4 | 3.8 | 3.6 | 3.3 | 0.7 | 4.5 | 1.8 | 0.9 | 0.9 | 1.3 | 4.4 | 2.1 | 2.2 | 1.1 | 1.4 | 5.3 | 1.0 | 2.9 | 1.42 |
| YDL085W | 1.2 | 1.9 | 1.2 | 2.0 | 1.2 | 1.2 | 1.4 | 1.0 | 7.8 | 3.4 | 2.7 | 0.8 | 1.7 | 1.2 | 0.7 | 4.5 | 0.9 | 0.9 | 0.23 |
| YJL221C | 1.1 | 1.0 | 1.1 | 1.3 | 0.9 | 6.6 | 2.5 | 1.8 | 2.7 | 4.4 | 0.8 | 1.1 | 1.4 | 1.1 | 1.1 | 3.3 | 1.1 | 1.4 | 0.41 |
| YKL085W | 1.5 | 2.3 | 1.6 | 1.2 | 1.2 | 1.9 | 1.5 | 1.2 | 1.9 | 3.0 | 1.8 | 0.8 | 1.5 | 1.0 | 0.5 | 1.7 | 0.9 | 1.3 | 2.16 |
| YLR174W | 1.2 | 1.5 | 1.7 | 2.1 | 0.9 | 0.9 | 1.6 | 1.1 | 2.5 | 8.3 | 1.3 | 1.3 | 1.8 | 1.7 | 0.8 | 4.6 | 0.9 | 1.2 | 0.41 |
| YNL009W | 1.1 | 1.9 | 2.0 | 1.4 | 1.4 | 1.1 | 1.2 | 1.1 | 1.4 | 3.3 | 1.3 | 0.9 | 1.3 | 1.3 | 2.7 | 3.3 | 1.2 | 2.3 | 0.45 |
| YNL117W | 0.9 | 4.7 | 1.7 | 0.8 | 0.8 | | 1.1 | 1.7 | 12.8 | 4.4 | 1.4 | 0.7 | 1.3 | 2.0 | 2.6 | 1.6 | 1.1 | 0.9 | 0.24 |
| YAL061W | 1.7 | 2.4 | 3.3 | 3.8 | 1.0 | 1.0 | 2.0 | 0.8 | 5.5 | 1.4 | 4.1 | 1.1 | 1.4 | 0.7 | 0.6 | 1.1 | 1.4 | 1.2 | 0.88 |
| YBR117C | 0.8 | 1.6 | 1.5 | 1.4 | 0.9 | 2.3 | 1.0 | 0.7 | 5.9 | 1.5 | 0.4 | 0.8 | 2.1 | 0.9 | 1.0 | 12.0 | 0.7 | 0.7 | 0.49 |
| YEL071W | 1.1 | 1.6 | 2.6 | 0.4 | 1.3 | 1.9 | 2.0 | 1.2 | 3.6 | 2.8 | 2.1 | 0.9 | 1.6 | 1.2 | 1.6 | 1.2 | 1.1 | 1.2 | 1.13 |

| | | | | | | | | | | | | | | | | | | |
|---|---|---|---|---|---|---|---|---|---|---|---|---|---|---|---|---|---|---|
| YGR112W | 1.0 | 2.9 | 1.1 | 1.3 | 0.9 | 1.1 | 1.3 | 1.3 | 2.6 | 1.3 | 1.3 | 0.7 | 1.1 | 1.1 | 1.2 | 2.8 | 0.9 | 1.2 | 0.31 |
| YLR164W | 1.0 | 2.4 | 0.3 | 1.0 | 1.2 | 1.2 | 1.1 | 1.2 | 5.3 | 2.0 | 3.2 | 0.7 | 1.0 | 1.5 | 0.7 | 11.0 | 1.5 | 1.2 | 0.31 |
| YNR032W | 1.0 | 1.5 | 1.0 | 2.1 | 1.5 | 1.0 | 1.1 | 1.0 | 6.2 | 2.4 | 1.4 | 0.6 | 1.3 | 1.4 | 0.9 | 1.0 | 1.2 | 1.3 | 0.71 |
| YPL031C | 0.9 | 2.0 | 1.4 | 0.8 | 1.5 | 1.8 | 1.6 | 1.0 | 5.7 | 1.4 | 1.4 | 0.8 | 1.6 | 0.9 | 0.7 | 2.0 | 1.4 | 1.2 | 0.49 |
| YPR048W | 1.3 | 2.0 | 0.9 | 1.1 | 0.9 | 0.6 | 0.9 | 1.5 | 4.0 | 2.1 | 1.0 | 0.8 | 0.6 | 1.2 | 0.6 | 0.8 | 0.7 | 0.8 | 0.75 |
| YBL058W | 0.9 | 1.5 | 0.5 | 1.1 | 1.6 | 0.9 | 1.5 | 1.2 | 2.6 | 2.1 | 1.3 | 0.8 | 1.6 | 1.3 | 1.8 | 1.8 | 1.0 | 1.4 | 1.42 |
| YBR001C | 0.9 | 1.4 | 1.1 | 1.4 | 0.9 | 1.1 | 0.9 | 1.3 | 2.3 | 2.1 | 1.0 | 1.0 | 2.0 | 1.2 | 1.8 | 1.4 | 1.0 | 1.6 | 0.61 |
| YCR105W | 2.2 | 1.2 | 1.2 | 3.0 | 0.9 | 1.0 | 1.0 | 1.4 | 2.0 | 3.9 | 2.3 | 0.9 | 1.9 | 1.9 | 3.1 | 1.3 | 1.3 | 1.1 | 0.36 |
| YIR031C | 0.9 | 1.6 | 0.6 | 0.6 | 1.6 | 1.7 | 1.4 | 1.0 | 2.6 | 1.0 | 1.2 | 0.8 | 1.0 | 1.2 | 0.8 | 0.7 | 0.9 | 0.8 | 0.44 |
| YGL191W | 2.3 | 1.6 | 1.4 | 1.8 | 0.9 | 1.6 | 1.0 | 1.6 | 1.1 | 1.3 | 1.6 | 0.9 | 1.1 | 1.6 | 0.7 | 1.8 | 1.2 | 1.7 | 2.35 |
| YLR038C | 2.6 | 1.1 | 0.6 | 2.1 | 1.8 | 1.2 | 1.0 | 1.4 | 0.4 | 0.9 | 1.4 | 0.6 | 0.5 | 1.6 | 0.7 | 2.1 | 1.2 | 2.1 | 1.52 |
| YGR087C | 1.0 | 15.0 | 1.7 | 0.6 | 1.0 | 1.1 | 1.0 | 0.8 | 0.2 | 0.9 | 0.8 | 1.2 | 1.2 | 0.7 | 3.5 | 0.6 | 1.0 | 0.6 | 1.88 |
| YGL256W | 0.9 | 5.3 | 1.1 | 1.3 | 0.7 | 0.7 | 0.5 | 1.0 | 0.4 | 1.2 | 1.0 | 0.9 | 0.8 | 0.9 | 1.0 | 0.7 | 0.7 | 0.8 | 0.90 |
| YDL181W | 1.4 | 1.8 | 1.9 | 1.8 | 1.7 | 1.2 | 1.1 | 0.6 | 0.7 | 0.8 | 0.8 | 0.9 | 0.9 | 1.1 | 0.4 | 1.1 | 1.5 | 1.4 | 0.85 |
| YPL262W | 1.1 | 1.7 | 3.8 | 1.2 | 0.6 | 1.6 | 1.3 | 1.0 | 1.4 | 5.8 | 1.4 | 1.1 | 1.5 | 1.2 | 0.6 | 1.4 | 1.1 | 1.2 | 0.79 |
| YLR377C | 1.1 | 2.6 | 1.8 | 1.2 | 1.0 | 0.7 | 0.9 | 0.7 | 1.8 | 1.6 | 0.6 | 0.6 | 1.2 | 2.9 | 0.8 | 2.7 | 0.9 | 1.4 | 0.14 |
| YBR221C | 0.8 | 0.7 | 3.2 | 1.5 | 1.3 | 1.7 | 1.5 | 0.8 | 1.0 | 1.3 | 1.2 | 1.3 | 1.7 | 0.8 | 1.1 | 1.5 | 0.9 | 1.0 | 3.62 |
| YKL141W | 1.6 | 1.1 | 4.7 | 1.7 | 1.5 | 1.2 | 0.5 | 0.8 | 0.8 | 0.5 | 1.2 | 0.8 | 0.8 | 1.4 | 0.6 | 2.8 | 0.9 | 1.8 | 2.84 |
| YLR134W | 0.9 | 0.6 | 2.3 | 0.8 | 1.3 | 2.5 | 1.1 | 0.8 | 0.1 | 0.7 | 1.1 | 1.2 | 1.5 | 0.6 | 1.7 | 0.5 | 0.6 | 0.8 | 3.47 |
| YLR258W | 1.7 | 1.0 | 4.2 | 3.5 | 0.9 | 1.8 | 1.8 | 0.9 | 0.8 | 1.3 | 1.2 | 0.9 | 1.4 | 1.2 | 0.6 | 1.7 | 1.0 | 2.0 | 1.36 |
| YOR178C | 1.4 | 1.3 | 4.8 | 2.3 | 1.0 | 2.7 | 0.9 | 0.9 | 0.2 | 1.7 | 4.1 | 1.7 | 1.0 | 1.0 | 1.1 | 1.7 | 1.0 | 1.5 | 0.56 |
| YBL099W | 0.8 | 0.9 | 3.1 | 1.4 | 1.0 | 0.9 | 0.9 | 0.8 | 0.9 | 0.7 | 2.1 | 1.0 | 0.9 | 0.6 | 1.0 | 0.9 | 0.7 | 1.2 | 3.49 |
| YDR050C | 1.9 | 1.3 | 2.3 | 1.8 | 0.9 | 1.7 | 1.5 | 1.3 | 0.4 | 1.3 | 2.0 | 1.4 | 2.0 | 1.2 | 1.9 | 1.3 | 1.1 | 2.3 | 6.26 |
| YDR178W | 2.0 | 2.0 | 3.4 | 2.2 | 0.9 | 2.1 | 0.6 | 0.9 | 0.9 | 0.8 | 2.0 | 1.3 | 1.5 | 1.5 | 0.7 | 3.0 | 1.2 | 2.3 | 2.27 |
| YDR298C | 1.3 | 1.2 | 2.6 | 1.3 | 1.3 | 1.2 | 1.0 | 1.2 | 1.0 | 1.6 | 1.2 | 1.1 | 1.2 | 1.5 | 0.8 | 2.0 | 1.1 | 1.6 | 2.69 |
| YEL024W | 1.5 | 0.9 | 3.8 | 1.1 | 1.3 | 1.1 | 0.7 | 1.0 | 0.7 | 0.6 | 0.9 | 0.8 | 0.6 | 1.1 | 0.6 | 2.1 | 1.1 | 1.5 | 1.59 |
| YJL121C | 1.0 | 0.5 | 1.9 | 0.8 | 1.0 | 1.1 | 0.7 | 1.0 | 0.1 | 0.4 | 1.1 | 0.6 | 0.7 | 0.9 | 1.0 | 0.6 | 1.1 | 1.0 | 1.00 |
| YJR121W | 1.2 | 1.1 | 3.3 | 1.0 | 0.8 | 1.5 | 1.0 | 0.7 | 0.8 | 1.3 | 0.9 | 0.8 | 1.0 | 0.7 | 0.9 | 1.2 | 1.1 | 1.4 | 3.99 |
| YKL060C | 1.7 | 0.8 | 2.5 | 1.0 | 1.6 | 1.3 | 1.2 | 1.1 | 0.3 | 0.8 | 2.1 | 1.3 | 1.2 | 0.8 | 1.6 | 1.5 | 1.2 | 1.7 | 6.01 |
| YKL148C | 0.9 | 0.8 | 3.7 | 0.7 | 0.8 | 0.5 | 0.6 | 0.8 | 1.7 | 1.7 | 1.5 | 0.8 | 0.8 | 0.8 | 0.6 | 1.1 | 0.8 | 1.0 | 0.54 |
| YLL041C | 1.5 | 0.5 | 4.6 | 1.0 | 1.6 | 1.2 | 0.3 | 0.8 | 0.7 | 0.8 | 1.3 | 0.8 | 1.0 | 1.2 | 0.4 | 3.1 | 1.1 | 1.7 | 1.75 |

| | | | | | | | | | | | | | | | | | | |
|---|---|---|---|---|---|---|---|---|---|---|---|---|---|---|---|---|---|---|
| YLR044C | 0.8 | 0.6 | 2.2 | 0.9 | 1.7 | 1.5 | 1.3 | 0.7 | 0.0 | 1.2 | 1.1 | 1.4 | 1.7 | 0.5 | 2.2 | 0.6 | 0.9 | 0.9 | 5.16 |
| YLR284C | 0.9 | 1.0 | 3.8 | 1.9 | 1.6 | 0.9 | 0.8 | 1.2 | 0.9 | 0.9 | 1.5 | 0.6 | 0.8 | 1.3 | 1.0 | 4.5 | 2.9 | 8.1 | 0.84 |
| YLR304C | 0.7 | 0.6 | 5.0 | 0.7 | 0.6 | 1.9 | 0.6 | 0.6 | 0.1 | 2.2 | 1.6 | 1.0 | 0.5 | 0.7 | 1.6 | 1.8 | 0.5 | 0.7 | 2.39 |
| YLR354C | 1.2 | 2.5 | 2.3 | 1.5 | 1.6 | 1.5 | 1.1 | 1.4 | 0.4 | 0.9 | 1.7 | 1.2 | 1.0 | 0.9 | 2.4 | 1.3 | 1.0 | 1.5 | 4.53 |
| YMR205C | 0.5 | 0.7 | 2.3 | 0.8 | 1.2 | 1.2 | 0.9 | 0.7 | 0.3 | 0.8 | 1.0 | 1.1 | 1.3 | 0.5 | 0.9 | 0.9 | 0.6 | 0.5 | 4.75 |
| YMR261C | 0.8 | 1.6 | 3.6 | 0.8 | 0.9 | 0.6 | 1.3 | 0.7 | 1.6 | 1.3 | 0.8 | 0.9 | 1.6 | 0.6 | 0.8 | 1.6 | 0.7 | 1.1 | 0.78 |
| YMR323W | 0.8 | 1.1 | 2.9 | 1.1 | 0.7 | 0.4 | 1.1 | 0.8 | 0.5 | 1.3 | 2.5 | 1.2 | 1.8 | 1.2 | 37.3 | 0.6 | 1.1 | 0.6 | 1.04 |
| YOL086C | 1.1 | 0.5 | 2.2 | 1.1 | 1.9 | 1.7 | 1.9 | 0.8 | 0.1 | 1.2 | 2.6 | 1.3 | 1.7 | 0.6 | 1.6 | 1.4 | 1.1 | 1.3 | 4.19 |
| YOR142W | 1.3 | 1.2 | 3.4 | 1.0 | 1.4 | 1.6 | 1.0 | 0.8 | 1.1 | 1.5 | 1.5 | 0.8 | 1.4 | 0.8 | 1.1 | 0.8 | 0.9 | 1.0 | 1.31 |
| YPL061W | 0.4 | 1.4 | 5.0 | 1.3 | 1.6 | 1.7 | 0.5 | 0.8 | 1.3 | 0.7 | 1.1 | 1.0 | 1.1 | 0.6 | 1.7 | 1.0 | 1.6 | 2.6 | 3.23 |
| YDL107W | 1.3 | 1.3 | 1.0 | 2.2 | 1.0 | 1.3 | 1.8 | 1.8 | 1.2 | 1.8 | 1.0 | 1.0 | 1.2 | 1.5 | 1.0 | 2.0 | 1.1 | 1.5 | 0.51 |
| YDR529C | 1.8 | 1.0 | 0.9 | 3.2 | 1.2 | 0.8 | 0.5 | 1.3 | 0.5 | 0.6 | 0.8 | 0.6 | 0.7 | 1.5 | 0.5 | 1.8 | 1.0 | 2.3 | 3.11 |
| YGL018C | 1.5 | 1.0 | 1.8 | 3.0 | 1.1 | 0.4 | 1.1 | 0.8 | 0.5 | 1.4 | 1.1 | 1.0 | 1.3 | 1.2 | 1.1 | 1.5 | 0.8 | 1.0 | 0.36 |
| YBR185C | 1.5 | 1.0 | 1.3 | 2.0 | 0.8 | 1.2 | 1.2 | 1.1 | 0.9 | 1.6 | 1.2 | 0.7 | 1.0 | 1.7 | 1.0 | 1.7 | 0.8 | 1.3 | 0.89 |
| YEL039C | 1.1 | 0.7 | 1.3 | 5.1 | 0.9 | 1.5 | 0.6 | 1.1 | 1.2 | 1.2 | 1.7 | 0.6 | 0.5 | 1.4 | 0.4 | 3.5 | 0.7 | 1.1 | 1.59 |
| YGR174C | 1.1 | 1.6 | 0.6 | 2.4 | 1.0 | 1.4 | 1.0 | 1.6 | 1.8 | 0.7 | 1.3 | 0.9 | 1.5 | 1.6 | 0.7 | 3.2 | 1.3 | 2.3 | 1.05 |
| YKL016C | 1.5 | 1.3 | 0.7 | 1.7 | 1.5 | 0.8 | 1.5 | 1.6 | 1.3 | 1.8 | 1.3 | 1.0 | 1.3 | 1.6 | 0.7 | 1.8 | 1.0 | 1.7 | 2.08 |
| YLR395C | 1.8 | 1.3 | 2.2 | 1.9 | 0.7 | 0.4 | | 1.0 | 1.0 | 0.9 | 1.5 | 0.6 | 0.5 | 1.7 | 0.4 | 1.2 | 1.2 | 2.3 | 1.76 |
| YML120C | 1.1 | 1.2 | 1.9 | 1.9 | 0.6 | 0.6 | 0.8 | 0.9 | 1.3 | 1.5 | 1.3 | 0.9 | 1.2 | 0.8 | 0.5 | 1.5 | 1.0 | 1.6 | 0.74 |
| YMR073C | 1.1 | 1.1 | 1.2 | 1.5 | 0.8 | 0.9 | 1.0 | 1.1 | 1.2 | 1.2 | 0.9 | 0.8 | 1.2 | 2.0 | 0.9 | 1.5 | 0.8 | 1.2 | 0.69 |
| YOR388C | 1.0 | 1.6 | 1.3 | 4.7 | 1.3 | 1.4 | 1.0 | 0.7 | 0.1 | 1.4 | 1.3 | 0.7 | 1.1 | 1.3 | 4.0 | 0.8 | 1.0 | 0.9 | 0.21 |
| YPL275W | 0.6 | 1.5 | 1.3 | 5.1 | 1.2 | 1.0 | 1.1 | 1.0 | 0.2 | 1.2 | 0.9 | 0.6 | 1.0 | 1.0 | 1.4 | 0.9 | 1.0 | 1.0 | 0.28 |
| YPL276W | 1.2 | 1.5 | 1.6 | 3.0 | 1.5 | 1.1 | 1.2 | 0.9 | -0.2 | 1.8 | -0.4 | 0.6 | 0.9 | 0.9 | 1.0 | 0.6 | 1.1 | 0.8 | 0.22 |
| YGL205W | 1.0 | 0.9 | 0.7 | 0.8 | 1.1 | 1.6 | 0.8 | 1.2 | 0.5 | 0.6 | 0.4 | 1.1 | 1.0 | 1.0 | 2.0 | 4.1 | 3.9 | 9.1 | 0.24 |
| YJL166W | 2.1 | 1.5 | 1.6 | 1.3 | 1.6 | 0.7 | 0.8 | 1.4 | 1.3 | 1.2 | 1.4 | 0.7 | 0.8 | 1.9 | 0.5 | 2.3 | 1.7 | 4.0 | 2.31 |
| YNL202W | 0.7 | 1.3 | 1.2 | 1.7 | 0.9 | 2.0 | 0.9 | 1.2 | 1.3 | 1.1 | 1.2 | 0.9 | 1.8 | 1.0 | 1.4 | 4.2 | 2.4 | 3.3 | 0.47 |
| YDR377W | 1.3 | 1.1 | 1.3 | 1.4 | 1.9 | 1.3 | 1.1 | 1.3 | 1.0 | 0.9 | 1.4 | 0.9 | 0.7 | 1.4 | 0.6 | 1.8 | 1.2 | 2.5 | 3.00 |
| YGL187C | 1.5 | 0.8 | 1.6 | 1.8 | 1.3 | 1.1 | 0.6 | 0.7 | 0.6 | 0.6 | 0.7 | 0.8 | 0.7 | 0.9 | 0.3 | 1.4 | 0.9 | 2.4 | 2.73 |
| YJL216C | 1.1 | 4.7 | 2.2 | 0.9 | 0.8 | | 1.5 | 1.9 | 1.5 | 1.3 | 1.1 | 1.0 | 1.1 | 1.1 | 1.2 | 1.5 | 1.2 | 2.0 | 0.25 |
| YKR009C | 1.0 | 1.2 | 1.5 | 0.9 | 1.0 | 1.8 | 1.0 | 0.9 | 2.4 | 2.1 | 1.2 | 1.0 | 1.8 | 1.0 | 1.0 | 4.6 | 1.9 | 2.5 | 0.27 |
| YCR046C | 1.0 | 1.2 | 1.2 | 1.6 | 1.0 | 0.8 | 1.2 | 0.9 | 1.9 | 1.4 | 1.5 | 1.1 | 1.5 | 1.3 | 1.4 | 2.8 | 1.3 | 1.0 | 0.63 |

| | | | | | | | | | | | | | | | | | | | |
|---|---|---|---|---|---|---|---|---|---|---|---|---|---|---|---|---|---|---|---|
| YML129C | 1.1 | 0.9 | 0.9 | 1.2 | 1.7 | 1.7 | 1.8 | 1.2 | 1.4 | 2.2 | 1.2 | 0.8 | 1.0 | 1.4 | 1.1 | 2.9 | 1.3 | 1.3 | 1.04 |
| YPR184W | 1.2 | 1.4 | 5.7 | 2.2 | 0.9 | 1.5 | 1.3 | 0.7 | 1.9 | 3.2 | 3.6 | 1.4 | 2.5 | 1.3 | 1.5 | 3.1 | 1.0 | 1.7 | 0.37 |
| YDL067C | 1.8 | 1.0 | 1.6 | 1.3 | 2.0 | 0.9 | 0.8 | 1.1 | 0.9 | 0.9 | 1.4 | 1.0 | 0.6 | 1.4 | 0.7 | 1.8 | 1.2 | 1.7 | 1.85 |
| YDL078C | 0.7 | 1.1 | 1.2 | 0.8 | 1.3 | 1.1 | 1.7 | 1.0 | 1.1 | 1.0 | 1.7 | 0.8 | 1.0 | 0.8 | 0.5 | 2.2 | 1.1 | 2.0 | 1.65 |
| YDR079W | 1.3 | 1.1 | 0.8 | 1.1 | 1.2 | 1.7 | 1.3 | 2.6 | 0.9 | 0.8 | 1.3 | 1.1 | 1.3 | 1.5 | 0.6 | 2.6 | 1.1 | 1.3 | 1.06 |
| YGR062C | 0.9 | 1.0 | 0.7 | 1.4 | 0.7 | 0.9 | 1.3 | 0.8 | 1.0 | 1.4 | 1.6 | 1.0 | 1.5 | 1.2 | 0.5 | 1.8 | 0.9 | 1.1 | 0.54 |
| YKR058W | 1.3 | 0.9 | 1.4 | 1.9 | 0.8 | 1.4 | 1.4 | 1.5 | 1.3 | 1.2 | 1.5 | 0.9 | 0.7 | 1.3 | 0.5 | 2.6 | 0.8 | 1.6 | 0.73 |
| YLR295C | 1.5 | 1.1 | 1.1 | 1.7 | 2.2 | 1.1 | 1.1 | 1.2 | 1.4 | 1.2 | 1.3 | 0.5 | 0.8 | 1.2 | 0.5 | 2.4 | 0.9 | 1.5 | 1.07 |
| YMR267W | 0.8 | 1.8 | 0.7 | 0.9 | 1.0 | 1.4 | 1.2 | 1.5 | 0.4 | 0.8 | 1.0 | 0.8 | 0.7 | 1.0 | 0.9 | 2.6 | 0.9 | 1.3 | 0.94 |

**Table 5** Transport facilitation protein genes

| yeast genes | The level of expressed mRNA in the presence of chemical substance/ The level of expressed mRNA in the absence of chemical substance | | | | | | | | | | | | | | | | | |
|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YBR008C | 3.0 | 2.4 | 4.9 | 0.7 | 0.9 | 1.0 | 0.9 | 2.8 | 54.6 | 21.1 | 9.4 | 0.8 | 2.0 | 4.1 | 0.8 | 3.1 | 2.0 | 1.2 | 0.37 |
| YBR296C | 7.0 | 4.3 | 2.2 | 2.5 | 1.4 | 1.4 | 0.2 | 1.1 | 0.4 | 1.8 | 1.5 | 1.6 | 2.0 | 2.3 | 1.7 | 0.3 | 3.8 | 4.0 | 0.54 |
| YDR406W | 0.8 | 1.2 | 3.1 | 1.1 | 1.0 | 5.9 | 1.8 | 0.6 | 1.1 | 1.7 | 1.4 | 3.9 | 7.7 | 0.5 | 0.8 | 5.5 | 2.7 | 5.6 | 0.53 |
| YOR153W | 1.6 | 0.9 | 5.1 | 1.1 | 1.0 | 7.4 | 2.6 | 0.5 | 3.2 | 1.2 | 1.0 | 4.0 | 11.8 | 0.3 | 3.6 | 1.6 | 2.5 | 3.1 | 1.91 |
| YGR281W | 1.2 | 1.0 | 1.3 | 2.2 | 0.6 | 1.9 | 0.9 | 0.7 | 2.7 | 1.4 | 0.7 | 2.4 | 12.9 | 0.9 | 2.1 | 1.1 | 2.8 | 3.1 | 1.04 |
| YHL047C | 0.6 | 4.4 | 1.0 | 1.0 | 1.2 | 8.4 | 16.8 | 1.5 | 1.2 | 11.9 | 1.0 | 2.6 | 3.8 | 0.5 | 1.1 | 2.3 | 1.2 | 1.2 | 0.74 |
| YBR294W | 6.3 | 14.4 | 0.9 | 2.6 | 1.4 | 1.2 | 1.1 | 1.0 | 5.2 | 5.2 | 1.2 | 1.0 | 3.1 | 1.3 | 1.0 | 0.9 | 1.0 | 0.9 | 0.18 |
| YGL006W | 2.5 | 1.3 | 1.3 | 2.6 | 1.0 | 1.0 | 1.3 | 0.9 | 3.0 | 1.2 | 0.8 | 0.9 | 4.5 | 0.9 | 1.1 | 1.7 | 2.4 | 4.1 | 1.37 |
| YGR197C | 1.1 | 2.4 | 2.5 | 1.0 | 0.7 | 0.5 | 1.3 | 0.8 | 16.7 | 3.2 | 1.1 | 1.2 | 2.4 | 1.5 | 1.0 | 1.5 | 1.7 | 1.9 | 0.37 |
| YJL034W | 0.7 | 1.7 | 3.0 | 0.8 | 1.2 | 1.1 | 1.3 | 1.5 | 2.8 | 1.1 | 1.3 | 1.6 | 6.1 | 0.6 | 7.9 | 0.6 | 1.1 | 1.0 | 4.58 |
| YNL055C | 1.1 | 1.8 | 5.5 | 2.0 | 0.9 | 2.6 | 1.2 | 0.7 | 1.1 | 1.0 | 1.9 | 1.7 | 2.4 | 0.9 | 1.1 | 2.6 | 1.2 | 1.7 | 4.10 |
| YBR052C | 1.6 | 1.6 | 1.5 | 2.6 | 1.4 | 2.1 | 3.0 | 1.3 | 2.3 | 2.5 | 1.7 | 1.3 | 2.1 | 1.5 | 0.7 | 3.1 | 1.8 | 3.1 | 2.77 |
| YBR207W | 0.8 | 1.7 | 0.8 | 2.0 | 1.7 | 1.6 | 4.3 | 0.9 | 1.7 | 2.2 | 1.1 | 0.9 | 2.2 | 0.9 | 1.4 | 1.4 | 1.2 | 1.4 | 1.39 |
| YBR293W | 1.9 | 3.1 | 1.0 | 1.8 | 0.8 | 0.9 | 0.9 | 0.9 | 5.5 | 2.4 | 1.1 | 1.0 | 3.0 | 1.4 | 3.1 | 1.6 | 1.0 | 0.9 | 0.94 |
| YDL198C | 1.4 | 1.0 | 2.1 | 1.4 | 0.7 | 0.8 | 1.0 | 1.1 | 1.9 | 2.7 | 2.5 | 1.2 | 2.0 | 1.7 | 1.2 | 1.4 | 1.5 | 1.1 | 1.19 |
| YDL245C | 1.0 | 2.4 | 1.6 | 2.2 | 1.3 | 1.1 | 1.2 | 1.2 | 0.4 | 2.8 | 1.5 | 1.0 | 2.0 | 1.2 | 1.8 | 1.5 | 1.0 | 1.1 | 0.28 |
| YDR497C | 0.7 | 0.5 | 0.6 | 1.3 | 0.7 | 0.6 | 0.5 | 0.6 | 0.4 | 0.8 | 0.7 | 0.9 | 2.7 | 0.6 | 10.1 | | 1.6 | 1.8 | 1.45 |
| YER053C | 1.6 | 1.8 | 1.9 | 1.7 | 0.6 | 2.8 | 2.8 | 1.3 | 1.3 | 3.9 | 2.4 | 1.7 | 4.1 | 1.3 | 1.1 | 2.8 | 1.2 | 2.3 | 1.83 |
| YFL041W | 0.7 | 1.6 | 1.4 | 1.2 | 1.2 | 2.0 | 3.4 | 1.0 | 0.8 | 5.1 | 0.9 | 1.1 | 1.8 | 1.0 | 0.9 | 1.4 | 1.1 | 0.9 | 0.89 |
| YGR055W | 2.5 | 5.7 | 12.0 | 0.7 | 1.3 | 1.1 | 0.6 | 1.1 | 5.0 | 2.4 | 1.5 | 0.9 | 1.9 | 1.2 | 2.1 | 0.6 | 0.7 | 0.7 | 1.42 |
| YJL219W | 1.2 | 2.5 | 3.1 | 1.6 | 1.2 | 1.5 | 0.9 | 1.5 | 4.0 | 2.3 | 2.3 | 1.1 | 4.0 | 1.1 | 2.6 | 1.4 | 1.6 | 1.2 | 0.91 |
| YJR106W | 0.9 | 3.8 | 1.5 | 1.1 | 0.9 | 1.2 | 1.0 | 0.9 | 2.0 | 1.6 | 1.6 | 0.9 | 3.5 | 0.9 | 1.2 | 1.9 | 1.2 | 1.3 | 0.29 |
| YKL146W | 0.8 | 1.3 | 1.3 | 0.9 | 1.0 | 1.3 | 0.9 | 0.8 | 4.6 | 2.8 | 0.9 | 1.1 | 2.7 | 1.0 | 1.5 | 1.3 | 1.0 | 0.9 | 0.42 |
| YLL028W | 0.6 | 0.9 | 4.6 | 0.5 | 0.9 | 1.7 | 1.0 | 0.7 | 1.2 | 2.3 | 0.7 | 1.3 | 4.1 | 0.7 | 3.1 | 0.6 | 1.2 | 1.2 | 1.02 |
| YLR348C | 1.1 | 4.5 | 1.2 | 1.2 | 0.9 | 1.9 | 0.9 | 0.9 | 2.1 | 1.3 | 1.3 | 0.9 | 1.9 | 1.0 | 2.4 | 1.1 | 1.1 | 1.0 | 0.64 |
| YOL119C | 2.6 | 4.8 | 1.5 | 2.3 | 1.5 | 0.9 | 2.2 | 2.0 | 5.0 | 7.4 | 3.4 | 0.9 | 1.6 | 2.4 | 4.7 | 2.8 | 1.4 | 1.0 | 0.37 |
| YOL163W | 1.5 | 3.2 | 2.1 | 3.3 | 0.9 | 0.5 | 0.9 | 1.1 | 7.5 | 6.2 | 1.6 | 1.0 | 2.4 | 1.2 | 1.0 | 1.4 | 0.8 | 0.8 | 0.30 |
| YOR035C | 1.2 | 1.2 | 0.3 | 1.2 | 1.0 | 1.9 | 1.3 | 1.0 | 1.3 | 1.7 | 1.0 | 1.1 | 2.5 | 0.9 | 3.8 | 1.8 | 1.6 | 2.1 | 0.52 |

| | | | | | | | | | | | | | | | | | | |
|---|---|---|---|---|---|---|---|---|---|---|---|---|---|---|---|---|---|---|
| YOR130C | 0.8 | 1.7 | 1.4 | 1.0 | 1.8 | 1.6 | 1.3 | 0.8 | 2.0 | 1.7 | 0.6 | 0.9 | 2.1 | 1.3 | 1.0 | 0.8 | 1.5 | 1.1 | 0.51 |
| YOR273C | 0.7 | 1.0 | 1.7 | 1.0 | 1.1 | 0.4 | 0.4 | 0.6 | 0.6 | 0.5 | 1.5 | 0.8 | 2.6 | 0.5 | 0.8 | 0.6 | 3.2 | 3.1 | 1.20 |
| YOR332W | 1.1 | 1.0 | 1.0 | 1.0 | 0.9 | 1.8 | 0.9 | 1.5 | 1.8 | 1.1 | 1.5 | 1.0 | 2.2 | 1.1 | 1.2 | 1.2 | 1.3 | 1.7 | 3.58 |
| YCR098C | 1.6 | 3.0 | 1.7 | 2.0 | 1.4 | 1.6 | 1.2 | 1.0 | 1.4 | 2.4 | 1.3 | 1.3 | 1.4 | 1.1 | 11.0 | 0.8 | 1.7 | 1.6 | 0.22 |
| YGR138C | 1.1 | 1.6 | 1.0 | 1.2 | 0.8 | 0.8 | 0.7 | 1.0 | 0.5 | 1.0 | 0.7 | 0.6 | 0.6 | 0.9 | 4.5 | 0.7 | 0.7 | 0.7 | 1.24 |
| YBR295W | 1.4 | 1.5 | 3.3 | 1.1 | 0.8 | | 1.6 | 0.9 | 1.2 | 3.3 | 1.1 | 0.9 | 1.8 | 1.1 | 4.8 | 1.4 | 1.3 | 1.2 | 0.27 |
| YGL255W | 0.7 | 0.9 | 0.9 | 0.8 | 1.4 | 1.1 | 1.0 | 0.7 | 0.3 | 5.0 | 0.5 | 0.7 | 0.2 | 1.0 | 2.6 | 0.5 | 0.6 | 0.5 | 1.36 |
| YHL035C | 0.7 | 1.3 | 0.9 | 1.9 | 0.9 | 4.1 | 1.7 | 1.1 | 0.6 | 4.6 | 0.9 | 1.4 | 1.1 | 0.8 | 3.1 | 2.4 | 1.1 | 1.2 | 0.77 |
| YIL022W | 0.8 | 0.7 | 1.1 | 1.6 | 0.8 | 1.2 | 0.7 | 0.8 | 1.1 | 1.3 | 1.0 | 0.9 | 1.2 | 0.6 | 2.9 | 1.1 | 0.8 | 0.7 | 0.52 |
| YLR378C | 0.7 | 0.9 | 2.6 | 0.5 | 1.0 | 1.2 | 0.7 | 0.5 | 1.1 | 0.7 | 1.0 | 1.2 | 1.6 | 0.4 | 5.9 | 0.4 | 0.9 | 0.7 | 1.23 |
| YCL038C | 1.6 | 1.6 | 1.4 | 1.6 | 1.2 | 3.2 | 1.5 | 1.2 | 1.1 | 0.9 | 2.0 | 1.3 | 1.9 | 1.3 | 2.0 | 1.8 | 1.4 | 1.5 | 1.04 |
| YFL054C | 1.6 | 1.1 | 1.4 | 2.2 | 1.3 | 2.4 | 1.1 | 1.5 | 2.0 | 2.3 | 1.7 | 1.2 | 2.3 | 0.9 | 1.4 | 1.3 | 1.7 | 1.4 | 1.06 |
| YHL040C | 1.6 | 4.9 | 1.5 | 0.4 | 1.1 | 7.3 | 4.0 | 1.5 | 1.1 | 11.3 | 1.7 | 2.0 | 2.0 | 1.1 | 3.4 | 1.0 | 1.4 | 1.0 | 0.69 |
| YKR039W | 1.4 | 3.5 | 1.3 | 1.7 | 1.0 | 2.8 | 1.0 | 1.0 | 1.9 | 2.0 | 1.9 | 1.5 | 1.3 | 1.2 | 1.7 | 1.9 | 0.9 | 1.1 | 0.40 |
| YPL271W | 1.2 | 3.2 | 4.1 | 1.2 | 1.4 | 3.3 | 1.2 | 0.9 | 0.6 | 1.2 | 1.7 | 0.8 | 1.2 | 1.3 | 0.8 | 1.4 | 1.2 | 1.3 | 1.34 |
| YBR068C | 1.1 | 1.0 | 1.9 | 2.5 | 1.4 | 1.9 | | 1.0 | 0.8 | 2.5 | 0.8 | 0.8 | 2.2 | 1.8 | 1.6 | 5.5 | 1.1 | 1.3 | 1.99 |
| YCR037C | 0.7 | 1.0 | 0.8 | 0.8 | 1.3 | 1.8 | 1.0 | 0.7 | 0.6 | 0.6 | 0.8 | 0.7 | 1.3 | 0.8 | 1.0 | 1.1 | 1.4 | 0.9 | 0.74 |
| YDL128W | 0.8 | 1.1 | 2.1 | 1.0 | 1.0 | 2.3 | 0.9 | 0.8 | 0.5 | 0.7 | 1.2 | 0.9 | 1.6 | 0.7 | 1.1 | 1.0 | 0.9 | 1.0 | 2.80 |
| YDR270W | 0.7 | 1.5 | 1.4 | 1.3 | 1.0 | 2.2 | 1.7 | 1.2 | 2.9 | 3.6 | 1.0 | 1.0 | 1.8 | 1.0 | 1.4 | 1.1 | 0.9 | 0.7 | 0.38 |
| YEL065W | 0.3 | 3.8 | 1.2 | 0.4 | 1.4 | 2.3 | 4.3 | 0.9 | 0.1 | 4.9 | 0.6 | 1.7 | 0.8 | 0.4 | 2.2 | 2.6 | 0.7 | 0.6 | 2.10 |
| YGL008C | 0.6 | 0.7 | 3.1 | 0.9 | 0.8 | 1.9 | 1.4 | 0.7 | 0.0 | 0.4 | 0.7 | 2.4 | 1.9 | 1.2 | 1.0 | 2.1 | 1.1 | 1.0 | 4.08 |
| YGL104C | 0.8 | 2.0 | 2.1 | 1.8 | 1.0 | 2.1 | 1.2 | 0.8 | 4.6 | 2.1 | 1.8 | 1.1 | 2.0 | 1.0 | 0.9 | 1.7 | 1.3 | 1.3 | 0.58 |
| YGL167C | 0.9 | 0.7 | 1.6 | 1.3 | 1.2 | 2.0 | 1.2 | 0.8 | 1.3 | 1.1 | 0.8 | 1.1 | 1.5 | 0.8 | 1.3 | 1.3 | 1.4 | 1.4 | 1.41 |
| YGR065C | 0.8 | 1.5 | 2.6 | 1.1 | 1.2 | 2.4 | 2.4 | 1.0 | 1.2 | 2.6 | 1.1 | 0.5 | 0.8 | 1.3 | 1.2 | 1.1 | 0.7 | 0.7 | 0.68 |
| YHR092C | 2.3 | 1.0 | 7.5 | 2.5 | 1.4 | 1.7 | 0.5 | 1.5 | 0.2 | 0.9 | 1.5 | 1.0 | 0.5 | 1.6 | 0.2 | 1.2 | 0.9 | 1.3 | 6.09 |
| YIL088C | 0.9 | 2.1 | 2.1 | 1.3 | 1.6 | 2.2 | 1.7 | 0.8 | 1.0 | 1.7 | 1.8 | 0.9 | 1.5 | 1.2 | 1.2 | 1.8 | 1.2 | 1.4 | 1.89 |
| YNL259C | 1.6 | 3.5 | 1.1 | 1.2 | 1.1 | 3.9 | 3.8 | 1.5 | 1.6 | 2.1 | 1.6 | 0.7 | 1.1 | 1.4 | 0.7 | 1.7 | 2.6 | 2.4 | 1.22 |
| YOR270C | 0.7 | 0.5 | 2.0 | 0.8 | 0.8 | 1.8 | 1.0 | 0.6 | 0.4 | 0.9 | 1.5 | 0.7 | 1.3 | 0.7 | 1.1 | 0.8 | 0.9 | 0.9 | 3.49 |
| YPL265W | 0.7 | 0.4 | 2.3 | 1.1 | 1.0 | 2.3 | 8.9 | 2.3 | 0.2 | 1.2 | 1.8 | 0.9 | 1.1 | 1.1 | 1.2 | 2.0 | 0.5 | 0.4 | 1.42 |
| YPR124W | 0.2 | 0.4 | 0.3 | 1.4 | 2.0 | 2.0 | 2.9 | 2.7 | 0.2 | 1.5 | 2.0 | 0.8 | 0.6 | 0.4 | 1.1 | 2.4 | 1.0 | 1.3 | 2.57 |
| YER119C | 0.7 | 2.1 | 1.8 | 0.7 | 0.8 | 1.3 | 0.8 | 0.6 | 5.8 | 1.2 | 2.2 | 0.7 | 1.6 | 1.0 | 2.3 | 0.8 | 1.0 | 0.9 | 0.32 |

| | | | | | | | | | | | | | | | | | | | |
|---|---|---|---|---|---|---|---|---|---|---|---|---|---|---|---|---|---|---|---|
| YFL055W | 2.0 | 6.7 | 1.3 | 2.5 | 1.5 | 1.1 | 1.4 | 1.3 | 23.9 | 6.5 | 2.7 | 0.9 | 1.3 | 1.5 | 0.7 | 1.4 | 1.4 | 1.0 | 0.23 |
| YLL055W | 2.6 | 19.0 | 4.8 | 1.7 | 1.0 | 1.4 | 1.7 | 1.4 | 19.1 | 14.0 | 3.6 | 0.7 | 1.5 | 1.3 | 0.7 | 2.8 | 1.1 | 1.3 | 0.47 |
| YHL036W | 2.2 | 4.8 | 3.4 | 2.0 | 1.3 | 1.3 | 1.0 | 1.2 | 9.0 | 4.4 | 2.3 | 1.2 | 1.5 | 1.2 | 0.9 | 1.1 | 1.0 | 1.0 | 0.60 |
| YHR048W | 2.5 | 1.4 | 1.4 | 1.7 | 1.0 | 0.8 | 0.8 | 1.9 | 4.5 | 2.7 | 2.0 | 0.9 | 1.1 | 1.7 | 0.9 | 1.4 | 0.7 | 0.9 | 0.26 |
| YKL221W | 1.3 | 0.7 | 2.0 | 1.0 | 1.0 | 0.9 | 1.3 | 1.2 | 7.3 | 2.7 | 1.9 | 0.7 | 1.6 | 1.4 | 1.1 | 1.3 | 0.8 | 0.9 | 0.27 |
| YLR092W | 5.0 | 5.6 | 1.4 | 0.6 | 1.3 | 1.0 | 1.3 | 1.5 | 12.7 | 5.8 | 3.1 | 1.0 | 1.8 | 1.4 | 0.9 | 1.2 | 1.0 | 1.1 | 0.24 |
| YML116W | 4.1 | 1.3 | 1.5 | 1.4 | 1.2 | 0.9 | 1.4 | 2.2 | 1.4 | 3.1 | 4.3 | 0.5 | 0.8 | 2.0 | 1.9 | 1.0 | 1.0 | 1.0 | 0.94 |
| YBR291C | 2.0 | 0.9 | 1.0 | 1.5 | 0.9 | 1.1 | 0.9 | 2.2 | 0.9 | 1.0 | 1.1 | 0.9 | 1.0 | 1.7 | 0.5 | 1.5 | 0.9 | 1.3 | 1.19 |
| YCL069W | 0.9 | 4.5 | 0.9 | 0.8 | 0.9 | | 1.4 | 1.3 | 1.2 | 6.9 | 1.0 | 0.9 | 0.8 | 1.3 | 1.4 | 0.9 | 1.0 | 1.0 | 0.25 |
| YJR095W | 1.2 | 20.5 | 1.9 | 6.7 | 1.2 | 1.5 | 2.0 | 0.9 | 0.5 | 6.3 | 0.6 | 0.7 | 0.8 | 1.3 | 0.8 | 0.8 | 1.3 | 0.9 | 0.23 |
| YKL188C | 1.3 | 0.7 | 1.9 | 2.4 | 1.0 | 0.8 | 1.1 | 0.7 | 2.5 | 2.8 | 1.1 | 1.2 | 1.2 | 2.1 | 1.4 | 2.7 | 1.1 | 1.5 | 0.27 |
| YKL217W | 1.8 | 2.4 | 1.0 | 2.1 | 1.1 | 1.2 | 1.6 | 1.1 | 0.9 | 4.1 | 1.6 | 0.8 | 1.2 | 1.2 | 2.2 | 3.0 | 1.7 | 3.3 | 0.29 |
| YKR105C | 0.8 | 0.9 | 0.9 | 1.5 | 1.0 | 1.2 | 1.0 | 1.3 | 1.0 | 5.2 | 0.0 | 0.8 | 2.5 | 1.2 | 1.5 | 0.7 | 0.8 | 1.0 | 0.26 |
| YOL158C | 0.7 | 4.0 | 2.4 | 0.9 | 1.2 | 2.0 | 1.6 | 0.7 | 1.7 | 6.1 | 0.7 | 0.9 | 1.4 | 1.0 | 0.9 | 1.2 | 1.4 | 1.7 | 1.30 |
| YPL224C | 1.0 | 2.2 | 1.3 | 2.5 | 0.6 | 1.3 | 2.2 | 1.3 | 3.2 | 4.2 | 0.8 | 1.1 | 1.3 | 1.4 | 1.0 | 2.5 | 1.3 | 1.9 | 0.64 |
| YPR201W | 33.2 | 0.9 | 1.1 | 1.0 | 1.2 | 1.9 | 0.8 | 0.9 | 8.3 | 5.6 | 2.2 | 0.7 | 1.1 | 1.8 | 0.8 | 0.9 | 0.8 | 0.9 | 0.29 |
| YAL067C | 2.7 | 12.1 | 1.5 | 1.4 | 0.7 | 1.2 | 0.8 | 1.1 | 7.6 | 2.9 | 1.2 | 1.0 | 1.1 | 1.3 | 2.4 | 1.6 | 1.0 | 1.0 | 0.33 |
| YDL149W | 0.8 | 2.0 | 1.2 | 1.6 | 1.5 | 1.0 | 0.7 | 1.2 | 3.2 | 2.0 | 1.2 | 0.7 | 1.5 | 1.2 | 1.8 | 1.4 | 0.8 | 1.0 | 0.33 |
| YJL094C | 1.0 | 1.7 | 1.3 | 2.0 | 1.4 | 1.3 | 1.7 | 0.8 | 5.4 | 2.9 | 1.1 | 1.2 | 2.6 | 1.0 | 1.0 | 1.8 | 2.1 | 2.0 | 0.84 |
| YLL061W | 3.6 | 2.8 | 9.2 | 0.4 | 0.8 | 0.8 | 0.9 | 1.4 | 3.7 | 5.5 | 1.7 | 0.7 | 0.9 | 1.3 | 1.4 | 1.0 | 0.6 | 0.7 | 0.33 |
| YPL274W | 0.8 | 4.2 | 3.8 | 0.8 | 0.6 | 1.1 | 0.7 | 0.9 | 4.1 | 3.6 | 2.0 | 0.5 | 0.6 | 0.9 | 1.5 | 1.1 | 0.7 | 0.6 | 0.41 |
| YBR241C | 0.9 | 9.3 | 2.6 | 0.9 | 0.9 | 1.5 | 1.4 | 0.7 | 29.8 | 2.2 | 1.4 | 1.4 | 1.4 | 0.9 | 1.6 | 0.7 | 1.0 | 1.2 | 1.25 |
| YDL206W | 0.9 | 1.7 | 1.8 | 1.4 | 1.1 | 5.7 | 2.0 | 1.1 | 1.6 | 1.5 | 0.6 | 1.0 | 1.3 | 1.0 | 1.1 | 3.1 | 1.0 | 1.1 | 0.35 |
| YDR040C | 0.7 | 1.4 | 3.7 | 0.8 | 1.0 | 1.7 | 0.7 | 0.8 | 2.0 | 1.2 | 0.8 | 0.9 | 1.8 | 0.9 | 0.8 | 1.4 | 1.4 | 1.6 | 1.25 |
| YFR045W | 1.1 | 1.6 | 1.1 | 1.1 | 1.0 | 1.0 | 0.6 | 1.0 | 2.2 | 1.0 | 1.4 | 1.0 | 1.1 | 1.0 | 1.1 | 0.6 | 0.9 | 0.8 | 0.84 |
| YIL170W | 1.1 | 1.0 | 2.5 | 2.2 | 0.9 | 8.8 | 0.5 | 1.2 | 5.7 | 3.2 | 1.7 | 0.7 | 1.9 | 1.1 | 2.3 | 1.8 | 1.6 | 1.5 | 0.48 |
| YKL192C | 1.1 | 1.0 | 3.7 | 1.1 | 1.1 | 1.8 | 1.1 | 0.9 | 3.5 | 1.6 | 1.1 | 1.2 | 1.7 | 1.5 | 1.2 | 2.1 | 1.0 | 0.8 | 1.57 |
| YKL209C | 1.0 | 1.0 | 1.3 | 0.8 | 1.2 | 3.3 | 0.9 | 1.4 | 2.4 | 1.6 | 0.8 | 0.9 | 1.4 | 1.1 | 1.0 | 1.1 | 1.1 | 1.0 | 0.30 |
| YKR106W | 1.3 | 0.8 | 2.1 | 1.4 | 1.2 | 1.6 | 0.9 | 1.3 | 10.5 | 7.4 | 1.8 | 0.8 | 1.4 | 1.7 | | 2.8 | 0.9 | 0.8 | 0.16 |
| YMR056C | 1.1 | 1.3 | 1.7 | 1.0 | 1.1 | 0.9 | 1.4 | 1.2 | 1.8 | 1.1 | 1.6 | 0.9 | 0.9 | 1.1 | 0.7 | 2.4 | 1.0 | 1.1 | 0.72 |
| YPL147W | 1.0 | 0.8 | 2.3 | 1.2 | 1.2 | 1.1 | 1.0 | 0.9 | 2.4 | 2.4 | 1.3 | 0.9 | 1.5 | 1.0 | 2.4 | 2.5 | 1.5 | 3.6 | 0.33 |

| | | | | | | | | | | | | | | | | | | | |
|---|---|---|---|---|---|---|---|---|---|---|---|---|---|---|---|---|---|---|---|
| YIL166C | 1.9 | 12.5 | 2.1 | 1.2 | 1.0 | 1.9 | | 1.3 | 2.5 | 2.9 | 1.8 | 0.9 | 1.5 | 1.1 | 1.3 | 0.7 | 1.2 | 0.9 | 0.26 |
| YMR058W | 0.3 | 5.1 | 2.1 | 0.3 | 0.8 | 2.6 | 1.8 | 0.8 | 0.2 | 1.4 | 0.8 | 0.8 | 0.4 | 0.3 | 0.5 | 0.4 | 0.6 | 0.5 | 1.66 |
| YNL142W | 0.7 | 2.4 | 2.5 | 0.8 | 1.3 | 0.9 | 1.1 | 0.8 | 0.9 | 0.6 | 0.9 | 1.2 | 0.9 | 0.8 | 0.3 | 1.0 | 1.0 | 0.9 | 0.44 |
| YDL210W | 1.2 | 2.2 | 2.7 | 1.1 | 0.9 | 1.3 | 0.8 | 1.1 | 1.2 | 1.4 | 0.6 | 0.8 | 1.2 | 1.1 | 1.9 | 0.9 | 1.1 | 0.9 | 0.20 |
| YCL025C | 1.1 | 4.6 | 2.5 | 1.7 | 0.9 | 0.9 | 0.7 | 0.7 | 0.3 | 0.3 | 1.0 | 1.1 | 0.5 | 0.7 | 0.8 | 0.6 | 0.6 | 0.6 | 1.98 |
| YJL212C | 0.8 | 3.2 | 3.1 | 0.5 | 1.2 | 1.1 | 0.7 | 0.7 | 1.1 | 1.3 | 0.9 | 0.4 | 1.0 | 0.8 | 0.3 | 0.5 | 0.6 | 0.5 | 0.59 |
| YBR132C | 0.9 | 1.9 | 2.4 | 1.8 | 0.8 | 1.2 | 1.1 | 0.8 | 0.8 | 1.1 | 1.9 | 1.0 | 1.4 | 1.1 | 1.5 | 1.3 | 1.1 | 1.3 | 0.43 |
| YBL030C | 1.0 | 0.9 | 4.5 | 0.9 | 0.7 | 1.1 | 0.9 | 0.8 | 0.4 | 0.9 | 1.1 | 0.9 | 0.5 | 0.8 | 1.8 | 1.0 | 1.0 | 1.1 | 3.12 |
| YCR024C-A | 0.8 | 0.9 | 3.2 | | 0.9 | 1.2 | 0.8 | 0.8 | 0.2 | 0.7 | 1.5 | 1.0 | 0.5 | 0.8 | 1.3 | 1.5 | 0.9 | 1.4 | 4.02 |
| YDR342C | 2.8 | 1.1 | 12.2 | 5.7 | 1.6 | 1.1 | 0.8 | 1.2 | 0.2 | 2.2 | 2.9 | 1.0 | 0.6 | 0.9 | 0.5 | 2.4 | 1.0 | 2.2 | 5.23 |
| YDR343C | 1.2 | 1.0 | 20.6 | 4.6 | 1.3 | 1.3 | 0.7 | 1.2 | 0.3 | 2.1 | 2.3 | 1.0 | 0.8 | 0.8 | 0.5 | 2.8 | 1.1 | 2.3 | 5.81 |
| YEL027W | 1.2 | 0.6 | 3.4 | 1.1 | 1.0 | 1.1 | 0.8 | 0.9 | 0.9 | 1.1 | 0.9 | 0.9 | 0.7 | 1.4 | 1.5 | 1.6 | 1.0 | 1.4 | 4.75 |
| YHR094C | 0.7 | 1.2 | 5.3 | 1.6 | 1.1 | 1.6 | 0.8 | 1.2 | 0.3 | 1.2 | 0.6 | 0.9 | 0.7 | 0.6 | 2.7 | 0.9 | 1.4 | 1.4 | 4.82 |
| YHR175W | 0.8 | 2.0 | 2.4 | 1.5 | 0.9 | 1.0 | 1.6 | 0.9 | 0.6 | 2.0 | 1.1 | 1.4 | 1.2 | 0.7 | 1.3 | 2.3 | 1.3 | 1.2 | 1.01 |
| YIL056W | 1.1 | 0.9 | 6.2 | 1.4 | 1.2 | 1.0 | 0.6 | 0.7 | 1.2 | 1.6 | 0.7 | 1.0 | 1.5 | 1.0 | 0.5 | 0.9 | 1.5 | 1.2 | 0.71 |
| YMR203W | 0.8 | 0.7 | 3.0 | 1.4 | 0.6 | 1.1 | 0.8 | 0.7 | 0.7 | 1.2 | 2.3 | 0.7 | 1.1 | 0.7 | 1.2 | 0.9 | 0.8 | 0.7 | 1.62 |
| YBL099W | 0.8 | 0.9 | 3.1 | 1.4 | 1.0 | 0.9 | 0.9 | 0.8 | 0.9 | 0.7 | 2.1 | 1.0 | 0.9 | 0.6 | 1.0 | 0.9 | 0.7 | 1.2 | 3.49 |
| YBR127C | 0.8 | 0.7 | 2.2 | 1.1 | 1.4 | 1.1 | 0.9 | 1.1 | 0.6 | 1.3 | 1.1 | 0.9 | 0.9 | 0.6 | 1.3 | 1.4 | 0.8 | 1.0 | 4.59 |
| YDR038C | 0.6 | 1.3 | 3.1 | 0.9 | 0.9 | 1.8 | 0.7 | 0.9 | 1.4 | 0.9 | 0.9 | 0.7 | 2.0 | 0.8 | 0.8 | 1.4 | 1.3 | 1.5 | 1.32 |
| YDR039C | 0.6 | 1.6 | 3.2 | 0.9 | 1.3 | 1.1 | 0.8 | 0.6 | 1.8 | 1.0 | 0.9 | 0.8 | 1.8 | 0.9 | 1.2 | 1.4 | 1.1 | 1.6 | 1.39 |
| YDR298C | 1.3 | 1.2 | 2.6 | 1.3 | 1.3 | 1.2 | 1.0 | 1.2 | 1.0 | 1.6 | 1.2 | 1.1 | 1.2 | 1.5 | 0.8 | 2.0 | 1.1 | 1.6 | 2.69 |
| YDR345C | 0.8 | 0.9 | 5.6 | 2.6 | 1.3 | 1.2 | 1.1 | 1.2 | 0.2 | 1.1 | 1.5 | 1.4 | 1.2 | 0.8 | 0.8 | 1.0 | 1.3 | 1.8 | 5.65 |
| YEL063C | 0.7 | 0.8 | 2.4 | 1.3 | 1.2 | 1.3 | 0.7 | 0.6 | 0.8 | 1.7 | 0.8 | 1.1 | 1.1 | 0.8 | 1.3 | 1.0 | 0.7 | 0.7 | 1.12 |
| YFL011W | 1.2 | 0.7 | 3.7 | 3.3 | 1.3 | 1.0 | 0.8 | 0.8 | 0.3 | 1.6 | 2.1 | 0.9 | 0.8 | 0.8 | 1.7 | 1.2 | 0.8 | 1.0 | 1.25 |
| YGR082W | 1.0 | 0.9 | 2.2 | 1.0 | 0.9 | 1.1 | 0.9 | 0.8 | 0.6 | 0.9 | 1.3 | 0.8 | 0.5 | 0.9 | 1.2 | 1.3 | 0.9 | 0.8 | 1.47 |
| YGR191W | 0.8 | 0.7 | 1.6 | 1.5 | 0.8 | 1.1 | 0.7 | 0.7 | 0.1 | 0.7 | 1.6 | 0.7 | 1.0 | 0.7 | 1.5 | 0.7 | 0.8 | 0.8 | 1.58 |
| YGR260W | 0.6 | 0.7 | 3.4 | 0.8 | 0.8 | 0.9 | 0.9 | 0.5 | 0.3 | 0.4 | 1.3 | 0.6 | 0.5 | 0.7 | 1.8 | 1.0 | 0.9 | 0.9 | 1.71 |
| YHR026W | 0.9 | 1.0 | 1.9 | 1.0 | 1.1 | 1.3 | 0.9 | 1.6 | 1.1 | 0.9 | 1.4 | 0.7 | 0.7 | 1.3 | 2.4 | 1.2 | 0.9 | 1.1 | 3.40 |
| YJR077C | 1.1 | 1.1 | 2.0 | 1.6 | 0.9 | 0.9 | 0.7 | 0.8 | 0.4 | 0.7 | 1.6 | 1.0 | 0.9 | 0.7 | 1.5 | 0.7 | 1.0 | 0.8 | 1.79 |
| YJR121W | 0.9 | 1.1 | 3.3 | 1.0 | 0.8 | 1.5 | 1.0 | 0.7 | 0.8 | 1.3 | 0.9 | 0.8 | 1.0 | 0.7 | 0.9 | 1.2 | 1.1 | 1.4 | 3.99 |
| YLR081W | 1.3 | 0.8 | 2.8 | 3.6 | 1.0 | 0.9 | 0.7 | 0.9 | 0.1 | 2.2 | 2.6 | 1.0 | 0.6 | 1.0 | 0.7 | 1.2 | 0.7 | 0.9 | 1.46 |

| | | | | | | | | | | | | | | | | | | | |
|---|---|---|---|---|---|---|---|---|---|---|---|---|---|---|---|---|---|---|---|
| YMR011W | 1.3 | 1.0 | 9.4 | 5.5 | 0.9 | 0.6 | 0.8 | 1.1 | 0.0 | 0.7 | 1.5 | 0.8 | 0.4 | 0.6 | 0.6 | 1.2 | 1.3 | 1.6 | 4.95 |
| YOL156W | 1.1 | 0.7 | 2.5 | 1.1 | 1.2 | 1.2 | 1.0 | 1.0 | 2.3 | 2.1 | 1.1 | 0.9 | 1.7 | 0.9 | 1.9 | 0.9 | 0.9 | 0.9 | 0.53 |
| YPL036W | 0.5 | 0.8 | 2.8 | 0.9 | 1.3 | 0.7 | 0.3 | 0.5 | 0.1 | 0.3 | 0.8 | 0.5 | 0.3 | 0.6 | 1.0 | 0.7 | 0.4 | 0.5 | 3.66 |
| YGR096W | 1.5 | 1.0 | 1.1 | 4.6 | 1.2 | 1.7 | 0.9 | 1.0 | 0.8 | 0.8 | 0.8 | 0.8 | 1.1 | 1.2 | 0.9 | 0.8 | 0.8 | 1.0 | 0.49 |
| YIL006W | 1.0 | 1.1 | 1.4 | 2.9 | 0.8 | 0.3 | 0.8 | 0.6 | 0.9 | 1.1 | 1.0 | 0.9 | 0.8 | 1.0 | 1.5 | 1.7 | 0.8 | 0.9 | 0.28 |
| YKL016C | 1.5 | 1.3 | 0.7 | 1.7 | 1.5 | 0.8 | 1.5 | 1.6 | 1.3 | 1.8 | 1.3 | 1.0 | 1.3 | 1.6 | 0.7 | 1.8 | 1.0 | 1.7 | 2.08 |
| YKR067W | 0.8 | 2.6 | 1.5 | 2.5 | 1.3 | 1.8 | 2.0 | 0.8 | 1.6 | 2.3 | 1.3 | 1.0 | 1.3 | 1.3 | 1.3 | 2.0 | 0.8 | 1.0 | 0.58 |
| YMR162C | 0.8 | 1.1 | 1.1 | 2.0 | 0.9 | 1.0 | 0.8 | 0.8 | 0.7 | 1.0 | 1.7 | 0.7 | 0.9 | 1.0 | 1.0 | 0.8 | 0.8 | 1.1 | 0.32 |
| YOR348C | 1.1 | 0.7 | 1.2 | 2.0 | 0.9 | 0.7 | 0.8 | 0.8 | 0.5 | 0.4 | 1.1 | 0.7 | 1.1 | 1.2 | 1.6 | 1.7 | 0.9 | 0.9 | 0.18 |
| YPR192W | 0.9 | 1.1 | 0.9 | 5.0 | 1.2 | 0.9 | 0.9 | 0.6 | 0.7 | 1.3 | 0.7 | 0.5 | 1.1 | 0.7 | 1.7 | 2.6 | 1.6 | 0.9 | 0.28 |
| YPR194C | 2.2 | 1.4 | 0.5 | 2.9 | 1.3 | 1.5 | 0.7 | 1.0 | 0.5 | 0.6 | 0.7 | 0.7 | 1.0 | 1.0 | 0.4 | 0.8 | 1.0 | 0.9 | 0.27 |
| YDR086C | 1.3 | 1.0 | 1.2 | 1.4 | 1.0 | 0.8 | 2.0 | 1.9 | 0.6 | 0.8 | 1.1 | 0.7 | 0.7 | 1.5 | 2.1 | 1.3 | 1.4 | 1.9 | 2.60 |
| YGR224W | 0.9 | 1.2 | 1.2 | 1.2 | 1.4 | 0.8 | 0.4 | 0.6 | 0.9 | 0.9 | 0.8 | 0.6 | 0.8 | 0.9 | 1.1 | 1.0 | 2.8 | 1.3 | 0.27 |
| YDR387C | 0.8 | 0.9 | 1.2 | 2.2 | 0.9 | 1.2 | 1.3 | 0.7 | 1.4 | 2.0 | 2.0 | 0.9 | 1.3 | 0.9 | 1.4 | 2.7 | 1.2 | 1.1 | 0.86 |
| YFL050C | 0.7 | 1.0 | 1.1 | 2.2 | 1.0 | 0.9 | 0.8 | 0.9 | 0.5 | 1.6 | 1.2 | 0.9 | 1.2 | 1.0 | 1.4 | 2.3 | 0.8 | 1.0 | 0.36 |
| YBR298C | 1.1 | 1.0 | 1.6 | 2.3 | 1.4 | 0.4 | 1.7 | 0.5 | 0.3 | 1.1 | 1.5 | 1.1 | 0.8 | 0.9 | 0.2 | 2.1 | 0.9 | 0.8 | 0.88 |
| YLR295C | 1.4 | 1.1 | 1.1 | 1.7 | 2.2 | 1.1 | 1.1 | 1.2 | 1.4 | 1.2 | 1.3 | 0.5 | 0.8 | 1.2 | 0.5 | 2.4 | 0.9 | 1.5 | 1.07 |
| YNR072W | 1.1 | 1.5 | 0.5 | 0.7 | 1.0 | 0.9 | 1.5 | 0.9 | 0.7 | 1.8 | 1.4 | 1.0 | 1.5 | 1.0 | 1.8 | 2.1 | 1.3 | 1.0 | 0.26 |
| YOR316C | 0.7 | 2.5 | 1.5 | 1.5 | 1.0 | 0.9 | 1.4 | 0.8 | 1.6 | 2.0 | 2.4 | 1.1 | 1.6 | 1.0 | 1.5 | 2.2 | 0.8 | 1.0 | 0.93 |
| YOR328W | 1.0 | 1.0 | | 1.0 | 1.0 | 2.9 | | | | | 1.1 | 1.8 | | 0.8 | 3.3 | 2.1 | | | 0.19 |
| YPL134C | 1.5 | 1.0 | 1.6 | 1.6 | 1.4 | 1.2 | 1.2 | 1.3 | 0.6 | 0.8 | 1.7 | 0.8 | 1.0 | 1.4 | 1.1 | 3.3 | 1.3 | 1.3 | 0.70 |

**Table 6** Stress protein genes

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| | The level of expressed mRNA in the presence of chemical substance/ The level of expressed mRNA in the absence of chemical substance | | | | | | | | | | | | | | | | | | |
| YBR072W | 18.5 | 17.4 | 18.4 | 10.5 | 1.2 | 32.2 | 6.6 | 2.1 | 29.4 | 43.4 | 6.3 | 16.3 | 38.5 | 5.8 | 4.9 | 23.1 | 1.8 | 2.2 | 0.59 |
| YFL014W | 3.4 | 5.1 | 5.7 | 11.0 | 1.0 | 9.3 | 5.5 | 3.4 | 13.1 | 5.8 | 5.0 | 4.3 | 15.2 | 7.3 | 6.3 | 14.2 | 1.5 | 8.8 | 2.14 |
| YLL060C | 12.5 | 4.2 | 2.3 | 2.6 | 0.9 | 1.4 | 2.0 | 5.8 | 13.0 | 23.2 | 14.1 | 1.1 | 1.8 | 10.6 | 1.5 | 3.6 | 1.9 | 2.3 | 0.57 |
| YAL005C | 0.6 | 1.6 | 7.0 | 0.9 | 1.2 | 1.6 | 1.2 | 0.9 | 3.9 | 1.8 | 1.0 | 1.2 | 5.0 | 0.4 | 1.6 | 0.7 | 0.8 | 0.5 | 5.23 |
| YBL075C | 1.3 | 4.3 | 3.1 | 1.6 | 1.1 | 0.8 | 1.3 | 0.8 | 60.3 | 6.7 | 1.6 | 1.1 | 8.0 | 1.1 | 2.6 | 2.2 | 1.2 | 0.7 | 1.17 |
| YBR169C | 1.0 | 2.1 | 3.9 | 1.9 | 0.8 | 4.9 | 2.5 | 0.9 | 7.0 | 4.3 | 2.2 | 1.7 | 7.2 | 1.1 | 1.6 | 2.4 | 1.3 | 1.6 | 1.10 |
| YCL035C | 2.0 | 2.3 | 1.5 | 1.7 | 1.5 | 5.1 | 2.7 | 1.4 | 1.9 | 2.3 | 2.1 | 1.5 | 2.8 | 2.5 | 1.9 | 5.5 | 2.6 | 4.2 | 1.74 |
| YCR060W | | 0.7 | 0.9 | | 0.8 | 3.3 | 2.4 | 1.2 | 0.7 | 1.1 | 1.2 | 1.7 | 8.6 | 1.7 | | 3.3 | 1.2 | 1.7 | 1.04 |
| YDR155C | 1.1 | 1.5 | 3.2 | 1.8 | 0.8 | 1.5 | 1.2 | 1.4 | 1.3 | 1.3 | 3.8 | 1.4 | 3.3 | 1.0 | 2.5 | 1.6 | 1.2 | 2.5 | 4.99 |
| YDR258C | 1.6 | 2.3 | 2.8 | 1.4 | 1.3 | 2.4 | 3.9 | 1.9 | 4.9 | 13.3 | 1.7 | 1.8 | 5.2 | 1.5 | 0.6 | 1.0 | 1.3 | 1.3 | 0.87 |
| YER103W | 0.7 | 2.1 | 5.2 | 2.1 | 1.1 | 1.7 | 1.0 | 0.9 | 20.3 | 9.0 | 1.8 | 1.9 | 9.0 | 0.9 | 3.2 | 1.2 | 1.4 | 0.6 | 2.10 |
| YKL210W | 0.5 | 1.7 | 2.7 | 0.7 | 0.8 | 1.4 | 1.0 | 0.9 | 3.5 | 2.9 | 1.4 | 1.3 | 2.9 | 0.8 | 1.0 | 0.9 | 0.7 | 1.1 | 2.29 |
| YLL024C | 0.5 | 1.6 | 5.3 | 0.8 | 0.7 | 1.0 | 1.1 | 0.7 | 2.8 | 2.4 | 1.6 | 1.0 | 4.8 | 0.4 | 1.3 | 0.5 | 0.5 | 0.6 | 5.12 |
| YLL026W | 1.8 | 2.9 | 7.7 | 1.7 | 0.9 | 1.9 | 2.9 | 1.4 | 11.4 | 6.6 | 1.7 | 1.9 | 8.1 | 0.8 | 0.9 | 1.6 | 1.7 | 2.5 | 2.56 |
| YNL160W | 2.1 | 3.7 | 10.6 | 2.0 | 1.3 | 5.8 | 2.4 | 1.2 | 3.5 | 2.7 | 5.4 | 1.4 | 6.0 | 1.3 | 0.7 | 4.0 | 1.0 | 1.0 | 1.77 |
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YOR027W | 0.7 | 2.4 | 4.4 | 0.8 | 1.0 | 1.6 | 1.3 | 0.9 | 3.5 | 4.0 | 1.3 | 1.3 | 5.1 | 0.8 | 1.4 | 0.6 | 0.9 | 0.8 | 1.52 |
| YPL240C | 0.7 | 1.4 | 2.4 | 0.9 | 1.3 | 1.2 | 1.4 | 1.2 | 3.5 | 2.6 | 1.0 | 0.8 | 2.9 | 0.7 | 1.2 | 0.7 | 0.8 | 1.0 | 4.83 |
| YDR293C | 0.9 | 0.9 | 1.2 | 1.4 | 0.6 | 0.9 | 0.9 | 0.6 | 1.2 | 1.2 | 1.1 | 0.9 | 2.3 | 0.8 | 1.7 | 1.6 | 1.4 | 1.3 | 1.12 |
| YDR436W | 0.9 | 0.9 | 1.1 | 1.4 | 0.7 | 1.1 | 1.2 | 0.9 | 3.5 | 1.1 | 2.0 | 0.9 | 2.7 | 1.2 | 0.8 | 1.4 | 0.9 | 1.2 | 0.53 |
| YDR519W | 1.3 | 3.2 | 1.7 | 1.6 | 0.7 | 1.3 | 1.9 | 1.4 | 0.5 | 1.2 | 1.2 | 1.7 | 2.5 | 1.3 | 2.0 | 1.5 | 1.8 | 2.4 | 2.01 |
| YEL030W | 0.7 | 0.9 | 1.9 | 1.1 | 0.4 | 0.6 | 0.9 | 0.7 | 2.3 | 2.5 | 1.7 | 0.9 | 2.7 | 0.7 | 0.8 | 0.8 | 0.9 | 0.6 | 1.28 |
| YER125W | 0.6 | 1.1 | 1.5 | 0.9 | 1.4 | 0.7 | 0.8 | 0.7 | 0.6 | 1.2 | 1.0 | 0.7 | 1.9 | 0.7 | 0.8 | 1.0 | 1.0 | 1.0 | 1.48 |
| YFL016C | 0.8 | 1.0 | 1.6 | 0.9 | 1.3 | 1.3 | 1.4 | 0.7 | 6.4 | 2.9 | 1.2 | 1.0 | 2.8 | 0.8 | 0.8 | 0.6 | 1.2 | 0.9 | 1.25 |
| YFR052W | 1.1 | 1.6 | 1.3 | 1.5 | 1.4 | 3.2 | 2.2 | 1.5 | 3.7 | 3.0 | 0.9 | 1.8 | 3.8 | 3.3 | 1.4 | 2.7 | 1.2 | 1.9 | 1.62 |
| YHR057C | 1.1 | 1.2 | 0.7 | 0.9 | 1.3 | 1.3 | 1.3 | 1.0 | 2.6 | 1.2 | 1.6 | 1.1 | 1.9 | 1.9 | 1.2 | 2.2 | 1.6 | 1.3 | 1.07 |
| YIR037W | 1.4 | 2.2 | 2.0 | 2.3 | 0.6 | 2.7 | 1.8 | 1.3 | 6.2 | 3.8 | 3.0 | 1.3 | 2.4 | 2.3 | 1.0 | 4.0 | 1.7 | 2.9 | 1.97 |
| YIR038C | 1.3 | 3.5 | 4.6 | 2.0 | 0.7 | 2.5 | 2.0 | 1.3 | 4.6 | 4.5 | 3.4 | 1.1 | 2.5 | 2.5 | 1.2 | 6.0 | 2.8 | 2.0 | 1.11 |
| YJR045C | 0.5 | 1.9 | 3.9 | | 0.5 | 1.2 | 1.0 | 0.8 | 3.2 | 3.0 | 1.4 | 1.0 | 2.3 | 0.5 | 2.3 | 0.8 | 0.7 | 0.9 | 3.78 |

| | | | | | | | | | | | | | | | | | | | |
|---|---|---|---|---|---|---|---|---|---|---|---|---|---|---|---|---|---|---|---|
| YLL039C | 1.1 | 1.0 | 1.7 | 1.2 | 0.9 | 1.5 | 1.1 | 1.1 | 3.4 | 1.5 | 1.1 | 1.0 | 2.2 | 1.4 | 3.0 | 1.1 | 1.6 | 2.1 | 3.60 |
| YLR259C | 0.7 | 1.0 | 3.1 | 1.6 | 1.1 | 0.9 | 1.8 | 0.8 | 1.8 | 2.6 | 3.0 | 0.7 | 2.5 | 0.8 | 2.1 | 1.3 | 0.8 | 1.0 | 2.09 |
| YML070W | 0.9 | 1.7 | 2.5 | 1.9 | 1.4 | 2.2 | 3.1 | 1.3 | 4.5 | 3.9 | 1.5 | 1.3 | 3.3 | 1.3 | 0.9 | 2.6 | 1.2 | 1.3 | 1.03 |
| YPL106C | 0.6 | 1.7 | 2.4 | 1.1 | 1.2 | 1.6 | 1.1 | 1.2 | 4.9 | 1.8 | 1.3 | 1.1 | 3.1 | 0.9 | 1.1 | 0.5 | 0.5 | 0.5 | 3.34 |
| YPR026W | 0.9 | 1.2 | 5.0 | 1.3 | 0.8 | | 1.3 | 1.0 | 0.9 | 3.1 | 1.6 | 1.2 | 2.3 | 1.5 | 3.1 | 2.5 | 0.9 | 1.3 | 0.26 |
| YDR077W | 1.2 | 1.0 | 3.5 | 2.1 | 0.8 | 1.0 | 0.7 | 0.8 | 0.7 | 0.7 | 6.8 | 0.8 | 1.0 | 0.3 | 6.1 | 1.0 | 1.4 | 1.1 | 4.77 |
| YKL163W | 1.1 | 1.4 | 3.4 | 1.9 | 0.6 | 1.1 | 0.9 | 0.8 | 0.7 | 1.6 | 1.3 | 0.9 | 1.5 | 0.5 | 16.8 | 3.0 | 0.8 | 1.0 | 2.01 |
| YLR109W | 0.8 | 2.9 | 6.0 | 0.9 | 1.3 | 2.1 | 2.6 | 0.9 | 2.4 | 2.7 | 1.5 | 1.6 | 3.2 | 2.3 | 3.9 | 1.8 | 1.1 | 1.2 | 3.86 |
| YOR208W | 1.3 | 1.0 | 1.2 | 2.9 | 1.4 | 1.8 | 1.1 | 1.0 | 1.5 | 2.0 | 1.2 | 1.2 | 1.5 | 1.3 | 4.4 | 1.9 | 1.6 | 1.6 | 0.56 |
| YDR098C | 0.8 | 1.4 | 1.0 | 0.8 | 1.5 | 1.1 | 1.5 | 1.2 | 3.7 | 1.5 | 1.4 | 1.3 | 1.2 | 1.5 | 2.2 | 0.8 | 1.0 | 1.2 | 1.59 |
| YHR030C | 2.1 | 0.8 | 0.6 | 2.9 | 1.8 | 0.9 | 1.8 | 1.5 | 0.6 | 2.5 | 1.5 | 0.7 | 1.0 | 1.1 | 3.4 | 1.4 | 2.0 | 1.8 | 1.05 |
| YJL159W | 1.0 | 1.3 | 2.8 | 1.1 | 1.0 | 1.8 | 0.9 | 0.8 | 0.5 | 1.2 | 1.2 | 1.0 | 1.7 | 0.6 | 5.3 | 1.0 | 1.4 | 0.9 | 2.45 |
| YMR173W | 1.5 | 3.6 | 3.0 | 0.9 | 1.1 | 5.0 | 2.4 | 2.3 | 2.7 | 8.2 | 3.2 | 1.4 | 2.0 | 2.3 | 2.5 | 1.2 | 1.9 | 1.8 | 1.37 |
| YCR021C | 5.2 | 1.4 | 4.2 | 8.6 | 0.9 | 2.2 | 0.9 | 0.8 | 2.2 | 1.0 | 0.6 | 0.5 | 0.9 | 1.6 | 0.5 | 0.6 | 0.7 | 0.8 | 1.36 |
| YDL022W | 0.9 | 2.6 | 6.9 | 1.5 | 0.7 | 2.1 | 1.5 | 0.8 | 1.2 | 1.8 | 2.8 | 1.2 | 1.5 | 0.8 | 0.9 | 1.1 | 1.0 | 1.4 | 1.79 |
| YDR033W | 1.1 | 1.3 | 4.6 | 1.5 | 1.1 | 6.7 | 0.8 | 1.0 | 0.3 | 1.0 | 0.7 | 1.0 | 0.7 | 1.0 | 0.8 | 1.0 | 0.7 | 1.0 | 5.78 |
| YFL020C | 1.0 | 1.1 | 1.9 | 1.2 | 1.1 | 2.0 | 1.4 | 0.7 | 1.3 | 1.4 | 1.2 | 1.3 | 1.4 | 1.0 | 1.4 | 0.9 | 1.5 | 0.9 | 0.70 |
| YGL073W | 1.0 | 2.1 | 0.9 | 1.0 | 1.7 | 1.9 | 1.3 | 0.8 | 1.9 | 2.4 | 0.8 | 0.8 | 1.5 | 1.2 | 0.9 | 1.1 | 2.5 | 1.2 | 0.66 |
| YIL033C | 0.8 | 1.3 | 3.3 | 1.3 | 0.7 | 2.0 | 1.1 | 0.7 | 2.4 | 1.3 | 1.8 | 1.1 | 2.5 | 0.7 | 1.0 | 2.0 | 1.0 | 1.2 | 1.18 |
| YMR021C | 1.0 | 0.7 | 0.9 | 1.1 | 1.6 | 2.2 | 2.5 | 1.3 | 0.6 | 1.3 | 0.9 | 0.8 | 1.4 | 1.3 | 1.0 | 2.0 | 3.3 | 3.4 | 1.14 |
| YBR126C | 0.8 | 1.9 | 5.6 | 1.2 | 0.7 | 2.9 | 2.3 | 0.6 | 1.7 | | 1.1 | 1.3 | 2.1 | 0.7 | 1.0 | 1.7 | 1.5 | 1.3 | 1.96 |
| YBR067C | 1.6 | 2.5 | 2.8 | 0.9 | 1.1 | 1.1 | 1.1 | 1.1 | 1.7 | 1.0 | 3.1 | 1.0 | 0.9 | 0.7 | 3.3 | 2.5 | 0.5 | 0.4 | 1.76 |
| YDR513W | 2.2 | 2.5 | 2.3 | 2.6 | 0.9 | 2.1 | 1.6 | 1.6 | 4.6 | 3.1 | 2.0 | 0.9 | 1.8 | 2.0 | 1.3 | 3.8 | 1.3 | 3.2 | 3.10 |
| YGR088W | 1.3 | 1.2 | 7.7 | 2.4 | 1.0 | 1.3 | 0.8 | 0.8 | 1.5 | 3.2 | 3.9 | 1.1 | 1.2 | 1.3 | 0.7 | 5.6 | 0.9 | 2.0 | 0.75 |
| YHR104W | 1.0 | 4.1 | 15.7 | 1.9 | 1.1 | 1.2 | 1.5 | 1.1 | 4.8 | 2.3 | 2.6 | 1.0 | 1.2 | 1.5 | 1.6 | 1.3 | 1.9 | 1.9 | 1.57 |
| YKL161C | 4.2 | 0.8 | 0.9 | 2.2 | 1.4 | 2.0 | 2.0 | 1.9 | 1.4 | 1.7 | 3.1 | 0.9 | 1.3 | 1.4 | 1.3 | 1.1 | 1.4 | 1.9 | 0.45 |
| YPL223C | 4.8 | 20.0 | 1.2 | 5.1 | 0.9 | 1.5 | 1.1 | 1.2 | 29.4 | 16.5 | 1.8 | 0.9 | 2.3 | 2.5 | 1.5 | 19.0 | 1.0 | 1.3 | 0.31 |
| YBR054W | 1.9 | 1.8 | 5.4 | 1.7 | 0.8 | 1.1 | 0.6 | 2.1 | 0.4 | 1.2 | 1.8 | 0.7 | 1.5 | 1.0 | 0.6 | 0.4 | 0.7 | 0.7 | 3.02 |
| YAL015C | 1.2 | 1.1 | 1.7 | 1.9 | 1.1 | 0.7 | 1.2 | 1.1 | 4.0 | 2.7 | 1.4 | 1.0 | 1.2 | 1.6 | 0.7 | 2.0 | 1.1 | 1.2 | 0.61 |
| YDL025C | 1.7 | 5.0 | 3.5 | 1.5 | 0.6 | 0.6 | 1.0 | 1.1 | 3.6 | 4.5 | 1.5 | 0.9 | 1.2 | 1.7 | 1.0 | 1.7 | 1.0 | 1.4 | 0.56 |
| YML007W | 0.9 | 0.8 | 0.9 | 1.3 | 1.0 | 0.5 | 1.1 | 1.9 | 2.1 | 2.5 | 1.3 | 1.1 | 1.7 | 1.2 | 1.0 | 1.4 | 1.0 | 1.4 | 1.54 |
| YER042W | 3.0 | 2.2 | 3.1 | 1.0 | 1.1 | 1.6 | 1.1 | 1.7 | 6.8 | 2.1 | 1.8 | 1.1 | 1.4 | 5.2 | 1.0 | 1.9 | 0.7 | 1.1 | 1.80 |

| | | | | | | | | | | | | | | | | | | | |
|---|---|---|---|---|---|---|---|---|---|---|---|---|---|---|---|---|---|---|---|
| YOL064C | 1.4 | 2.1 | 1.1 | 0.6 | 0.9 | 1.4 | 1.2 | 1.0 | 8.7 | 2.7 | 1.0 | 0.9 | 2.0 | 0.9 | 1.0 | 1.7 | 1.2 | 1.3 | 1.32 |
| YGL181W | 0.9 | 0.9 | 1.2 | 1.1 | 1.1 | 1.9 | 1.3 | 1.2 | 2.7 | 2.3 | 1.2 | 1.0 | 2.8 | 1.0 | 1.5 | 1.3 | 1.0 | 1.3 | 0.98 |
| YJL128C | 0.8 | 1.4 | 0.4 | 0.8 | 1.2 | 1.3 | 1.8 | 0.9 | 2.0 | 1.7 | 0.6 | 1.0 | 1.7 | 0.8 | 1.2 | 0.9 | 1.0 | 1.0 | 0.42 |
| YJL165C | 1.0 | 0.8 | 1.3 | 1.6 | 1.1 | 0.5 | 0.8 | 0.7 | 2.8 | 1.0 | 1.7 | 0.9 | 1.5 | 0.9 | 1.0 | 1.5 | 1.3 | 1.8 | 0.87 |
| YJR090C | 0.8 | 1.0 | 0.6 | 0.8 | 1.2 | 1.2 | 0.8 | 0.8 | 1.9 | 1.2 | 0.4 | 0.8 | 2.2 | 1.0 | 1.6 | 0.8 | 1.1 | 1.0 | 0.60 |
| YMR186W | 0.6 | 1.4 | 2.0 | 0.9 | 1.3 | 1.3 | 1.2 | 0.8 | 2.6 | 1.3 | 1.1 | 1.0 | 2.4 | 0.6 | 1.3 | 0.6 | 0.7 | 1.1 | 6.64 |
| YNL064C | 0.9 | 1.8 | 1.8 | 1.1 | 0.9 | 0.8 | 0.6 | 1.4 | 2.4 | 1.8 | 0.9 | 0.8 | 1.1 | 0.8 | 0.7 | 0.3 | 0.7 | 0.7 | 3.79 |
| YOR008C | 0.7 | 0.8 | 1.4 | 1.1 | 1.4 | 1.1 | 0.8 | 0.8 | 1.9 | 1.0 | 1.0 | 0.7 | 0.9 | 0.9 | 0.9 | 1.2 | 0.8 | 1.3 | 1.75 |
| YPL194W | 0.9 | 1.4 | 0.4 | 1.1 | 1.3 | 1.1 | 1.5 | 1.3 | 9.6 | 3.1 | 0.9 | 0.9 | 1.4 | 1.1 | 1.4 | 0.9 | 1.0 | 0.8 | 0.22 |
| YBL093C | 1.0 | 2.0 | 0.4 | 1.4 | 1.0 | 0.8 | 1.3 | 1.6 | 0.7 | 1.1 | 1.3 | 1.1 | 2.1 | 1.0 | 0.5 | 0.9 | 1.4 | 1.3 | 1.47 |
| YKR053C | 0.9 | 2.0 | 1.0 | 1.2 | 1.0 | 1.1 | 1.7 | 0.9 | 0.8 | 1.8 | 0.5 | 0.7 | 0.7 | 1.3 | 0.3 | 0.9 | 2.8 | 2.4 | 0.35 |
| YJL158C | 0.9 | 0.6 | 2.8 | 1.5 | 1.4 | 0.5 | 0.4 | 0.7 | 0.2 | 0.5 | 1.9 | 1.1 | 0.4 | 0.5 | 1.7 | 0.6 | 1.4 | 1.4 | 3.48 |
| YER057C | 1.2 | 2.0 | 3.0 | 1.8 | 0.8 | 1.6 | 1.4 | 1.0 | 0.6 | 1.4 | 1.8 | 1.5 | 1.0 | 1.4 | 1.7 | 2.8 | 1.1 | 1.8 | 2.03 |
| YHL046C | 1.1 | 1.7 | 2.1 | 1.3 | 0.9 | 0.8 | 1.3 | 1.0 | 1.0 | 2.1 | 0.4 | 1.1 | 1.4 | 1.4 | 1.0 | 1.4 | 0.9 | 0.9 | 0.55 |
| YIL011W | 0.7 | 0.5 | 3.9 | 0.6 | 1.1 | 0.7 | 0.6 | 0.7 | 0.0 | 0.5 | 2.5 | 0.6 | 0.5 | 0.5 | 0.9 | 1.0 | 0.8 | 0.8 | 0.57 |
| YKL164C | 0.6 | 1.1 | 2.5 | 0.9 | 1.1 | 1.4 | 0.8 | 0.8 | 0.4 | 0.7 | 1.0 | 0.8 | 0.9 | 0.3 | 1.2 | 1.1 | 0.8 | 0.8 | 2.75 |
| YNR076W | 1.3 | 1.3 | 2.5 | 1.0 | 1.2 | 1.2 | 1.2 | 1.0 | 1.3 | 2.5 | 1.4 | 0.7 | 0.9 | 0.8 | 1.1 | 0.8 | 1.5 | 0.8 | 0.42 |
| YOL161C | 1.2 | 1.1 | 2.4 | 1.4 | 0.9 | 1.0 | 0.9 | 0.8 | 0.7 | 2.0 | 1.5 | 1.3 | 1.2 | 0.9 | 1.1 | 1.6 | 1.1 | 1.0 | 0.89 |
| YOR009W | 0.9 | 0.8 | 3.2 | 0.7 | 0.8 | 0.9 | 0.6 | 0.7 | 0.4 | 0.5 | 1.4 | 0.4 | 0.4 | 0.7 | 0.6 | 0.8 | 0.9 | 0.8 | 0.36 |
| YOR010C | 0.7 | 0.7 | 3.2 | 0.6 | 1.2 | 0.5 | 0.4 | 0.5 | 0.6 | 0.7 | 1.2 | 0.5 | 0.5 | 0.4 | 0.6 | 1.1 | 1.4 | 0.6 | 0.61 |
| YPL059W | 1.2 | 0.9 | 2.6 | 1.3 | 1.2 | 1.3 | 0.7 | 1.4 | 2.0 | 1.3 | 1.6 | 0.8 | 0.7 | 1.3 | 1.0 | 2.1 | 1.8 | 1.3 | 1.35 |
| YBR044C | 0.8 | 1.4 | 0.7 | 1.9 | 1.4 | 1.0 | 1.5 | 1.1 | 0.8 | 1.2 | 1.1 | 0.8 | 1.2 | 0.9 | 0.9 | 1.4 | 0.9 | 1.3 | 0.57 |
| YEL039C | 1.1 | 0.7 | 1.3 | 5.1 | 0.9 | 1.5 | 0.6 | 1.1 | 1.2 | 1.2 | 1.7 | 0.6 | 0.5 | 1.4 | 0.4 | 3.5 | 0.7 | 1.1 | 1.59 |
| YGL115W | 0.9 | 0.6 | 1.2 | 2.0 | 1.2 | 1.2 | 1.4 | 1.0 | 1.2 | 1.2 | 1.0 | 0.9 | 0.7 | 1.2 | 0.7 | 1.7 | 0.8 | 1.2 | 2.46 |
| YLR006C | 0.7 | 0.7 | 0.9 | 6.2 | 1.6 | 0.9 | 1.1 | 0.9 | 1.1 | 1.1 | 1.5 | 0.7 | 0.8 | 1.0 | 0.9 | 0.8 | 1.6 | 1.0 | 0.40 |
| YPR005C | 1.3 | 0.8 | 1.5 | 3.8 | 1.0 | 0.8 | 1.2 | 1.2 | 2.8 | 2.0 | 0.4 | 0.6 | 0.7 | 1.3 | 2.7 | 0.8 | 1.0 | 1.1 | 0.24 |
| YHL028W | 0.7 | 0.8 | 1.4 | 0.5 | 1.1 | 1.8 | 2.1 | 0.4 | 0.2 | 0.4 | 0.7 | 1.5 | 2.1 | 1.3 | 0.6 | 2.6 | 1.6 | 2.5 | 0.62 |
| YCR083W | 1.1 | 2.8 | 1.5 | 1.8 | 1.6 | 2.4 | 1.8 | 1.5 | 1.8 | 1.8 | 1.8 | 1.1 | 1.8 | 2.4 | 1.0 | 3.1 | 1.3 | 1.3 | 0.98 |
| YPL140C | 0.9 | 1.3 | 0.9 | 1.0 | 0.8 | 0.3 | 1.4 | 1.1 | 0.9 | 0.9 | 0.8 | 0.8 | 0.9 | 1.1 | 0.7 | 1.8 | 0.9 | 1.4 | 0.46 |

**Table 7 Metabolism protein genes**

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YCR107W | 12.0 | 2.6 | 1.8 | 1.6 | 0.7 | 1.5 | 1.5 | 15.6 | 196.6 | 34.0 | 23.0 | 0.9 | 4.0 | 8.3 | 4.8 | 3.1 | 1.3 | 1.1 | 0.59 |
| YDL243C | 14.4 | 2.7 | 2.4 | 1.0 | 1.1 | 2.2 | 1.6 | 11.8 | 64.2 | 29.6 | 19.6 | 1.1 | 4.1 | 12.0 | 4.2 | 3.0 | 1.8 | 1.5 | 0.76 |
| YFL014W | 3.4 | 7.2 | 5.7 | 11.0 | 1.0 | 9.3 | 5.5 | 3.4 | 13.1 | 5.8 | 5.0 | 4.3 | 15.2 | 7.3 | 6.3 | 14.2 | 1.5 | 8.8 | 2.14 |
| YFL056C | 19.0 | 2.3 | 2.3 | 1.5 | 0.9 | 0.6 | 1.4 | 18.5 | 162.3 | 31.3 | 68.3 | 1.0 | 4.7 | 7.8 | 5.0 | 3.4 | 1.0 | 1.1 | 0.55 |
| YFL057C | 20.9 | 5.8 | 1.5 | 1.8 | 0.9 | 1.2 | 1.8 | 18.0 | 51.8 | 46.1 | 27.7 | 1.0 | 4.1 | 23.4 | 3.1 | 3.9 | 1.6 | 1.3 | 0.71 |
| YJR155W | 10.6 | 3.7 | 1.4 | 2.5 | 0.7 | 1.4 | 1.7 | 10.6 | 38.2 | 18.8 | 15.4 | 1.0 | 5.7 | 9.4 | 2.6 | 5.6 | 1.3 | 1.4 | 0.64 |
| YNL331C | 8.6 | 3.6 | 1.3 | 1.0 | 1.6 | 1.8 | 1.9 | 13.1 | 42.6 | 36.3 | 21.8 | 0.9 | 3.1 | 7.5 | 2.3 | 4.0 | 1.7 | 1.3 | 0.58 |
| YNL332W | 1.1 | 1.5 | 1.1 | 2.0 | 1.6 | 1.1 | 2.1 | 1.5 | 3.3 | 2.1 | 2.6 | 1.2 | 2.1 | 3.0 | 5.4 | 2.2 | 1.0 | 1.0 | 0.24 |
| YOL165C | 10.1 | 4.5 | 1.8 | 0.9 | 0.9 | 1.7 | 1.4 | 17.8 | 46.9 | 23.3 | 17.6 | 0.8 | 3.7 | 9.1 | 3.0 | 1.8 | 1.6 | 1.0 | 0.69 |
| YPR167C | 5.3 | 5.8 | 1.9 | 0.8 | 1.4 | 2.6 | 1.2 | 5.5 | 76.6 | 9.0 | 1.9 | 1.3 | 1.9 | 4.2 | 0.9 | 0.9 | 1.7 | 1.4 | 0.54 |
| YBR256C | 3.0 | 1.4 | 0.6 | 1.3 | 1.6 | 2.0 | 2.1 | 2.3 | 18.1 | 6.8 | 3.2 | 1.7 | 3.5 | 5.6 | 1.6 | 3.0 | 2.6 | 3.3 | 1.97 |
| YBR296C | 7.0 | 11.2 | 2.2 | 2.5 | 1.4 | 1.4 | 0.2 | 1.1 | 0.4 | 1.8 | 1.5 | 1.6 | 2.0 | 2.3 | 1.7 | 0.3 | 3.8 | 4.0 | 0.54 |
| YDL021W | 4.8 | 1.7 | 2.4 | 3.7 | 1.7 | 6.5 | 5.9 | 2.7 | 2.5 | 7.4 | 4.7 | 2.4 | 5.3 | 3.7 | 0.7 | 7.3 | 1.9 | 3.2 | 0.47 |
| YFL061W | 1.5 | 1.4 | 2.1 | 3.0 | 1.0 | 0.5 | 1.2 | 1.2 | 3.2 | 9.9 | 1.1 | 0.9 | 1.2 | 6.0 | 2.1 | 1.0 | 1.2 | 0.9 | 0.31 |
| YGR043C | 2.5 | 4.7 | 3.2 | 7.9 | 0.9 | 16.3 | 6.5 | 2.6 | 10.9 | 8.4 | 3.6 | 3.3 | 6.9 | 4.1 | 3.0 | 13.7 | 1.6 | 4.8 | 0.66 |
| YHR112C | 1.8 | 3.1 | 0.7 | 1.4 | 1.3 | 2.9 | 3.4 | 1.7 | 10.5 | 4.4 | 1.5 | 1.9 | 3.9 | 2.4 | 2.0 | 2.7 | 1.6 | 1.8 | 0.55 |
| YIR030C | 1.4 | 1.0 | 0.9 | 1.3 | 0.9 | 0.5 | 1.2 | 1.4 | 5.4 | 2.1 | 1.4 | 0.9 | 1.3 | 2.9 | 0.7 | 0.8 | 0.9 | 1.0 | 0.42 |
| YJR010W | 9.1 | 7.3 | 4.9 | 0.8 | 1.1 | 2.5 | 1.4 | 3.1 | 30.0 | 11.1 | 2.9 | 1.4 | 3.2 | 5.4 | 1.7 | 1.7 | 1.2 | 1.0 | 0.56 |
| YKL001C | 6.8 | 21.4 | 5.6 | 1.8 | 0.8 | 0.9 | 1.1 | 3.4 | 10.3 | 3.4 | 2.6 | 2.1 | 1.6 | 6.2 | 1.1 | 1.8 | 2.7 | 1.7 | 0.91 |
| YKR097W | 1.3 | 2.4 | 1.3 | 3.3 | 1.1 | 1.7 | 3.7 | 2.5 | 1.9 | 3.3 | 0.8 | 1.8 | 17.5 | 2.1 | 2.5 | 2.2 | 1.1 | 1.5 | 0.16 |
| YLR303W | 7.3 | 9.8 | 12.1 | 1.1 | 1.1 | 3.0 | 3.6 | 5.3 | 18.6 | 5.6 | 9.8 | 4.3 | 9.9 | 3.8 | 5.8 | 3.4 | 1.5 | 1.4 | 1.42 |
| YNL274C | 1.4 | 18.7 | 2.6 | 1.9 | 0.7 | 4.2 | 2.0 | 2.3 | 6.3 | 5.3 | 4.0 | 1.1 | 2.3 | 3.3 | 2.5 | 6.4 | 1.4 | 2.6 | 0.85 |
| YOL151W | 6.7 | 3.8 | 9.1 | 1.3 | 1.3 | 8.4 | 4.4 | 6.7 | 22.8 | 17.0 | 9.0 | 4.6 | 16.9 | 4.0 | 2.8 | 3.7 | 4.0 | 5.6 | 1.12 |
| YOR226C | 1.3 | 2.2 | 1.7 | 1.1 | 1.3 | 1.2 | 1.2 | 1.2 | 1.3 | 5.9 | 1.5 | 0.6 | 0.9 | 2.7 | 1.0 | 0.7 | 1.0 | 0.7 | 0.58 |
| YJL153C | 1.3 | 1.7 | 1.7 | 17.6 | 0.7 | 0.8 | 2.3 | 1.3 | 3.9 | 2.5 | 0.7 | 10.2 | 38.0 | 1.4 | 37.0 | 1.0 | 6.5 | 1.8 | 0.27 |
| YOR153W | 1.6 | 0.9 | 5.1 | 1.1 | 1.0 | 7.4 | 2.6 | 0.5 | 3.2 | 1.2 | 1.0 | 4.0 | 11.8 | 0.3 | 3.6 | 1.6 | 2.5 | 3.1 | 1.91 |
| YPL088W | 3.3 | 1.3 | 0.6 | 2.5 | 1.5 | 7.1 | 3.7 | 1.9 | 0.6 | 2.8 | 1.1 | 3.6 | 4.8 | 1.6 | 3.2 | 4.3 | 9.1 | 8.3 | 0.69 |
| YDL124W | 2.1 | 8.5 | 5.7 | 1.7 | 0.7 | 3.4 | 2.9 | 2.1 | 3.3 | 5.3 | 3.4 | 2.4 | 6.0 | 1.6 | 3.4 | 3.6 | 2.2 | 2.6 | 2.43 |

| | | | | | | | | | | | | | | | | | | | |
|---|---|---|---|---|---|---|---|---|---|---|---|---|---|---|---|---|---|---|---|
| YDL174C | 0.9 | 1.8 | 0.9 | 1.3 | 0.5 | 1.7 | 3.6 | 0.8 | 0.5 | 1.7 | 0.9 | 2.8 | 5.9 | 1.1 | 0.6 | 5.1 | 2.2 | 4.0 | 0.63 |
| YGL156W | 1.3 | 2.5 | 1.8 | 2.2 | 1.4 | 2.1 | 2.1 | 1.4 | 5.6 | 2.4 | 2.1 | 2.6 | 6.0 | 1.6 | 3.0 | 4.1 | 1.4 | 1.9 | 0.29 |
| YGR157W | 1.0 | 0.9 | 5.2 | 0.8 | 1.2 | 1.0 | 0.8 | 0.7 | 3.3 | 2.0 | 1.0 | 1.8 | 4.8 | 0.7 | 4.6 | 1.0 | 0.8 | 1.3 | 2.10 |
| YHR043C | 1.3 | 1.8 | 1.2 | 1.7 | 0.9 | 2.2 | 2.9 | 3.0 | 1.2 | 1.0 | 1.6 | 2.2 | 2.7 | 1.3 | 2.1 | 2.2 | 1.4 | 1.7 | 1.38 |
| YHR044C | 1.3 | 1.3 | 1.7 | 1.5 | 1.2 | 3.3 | 2.6 | 2.0 | 1.1 | 1.1 | 1.7 | 2.3 | 4.3 | 1.3 | 1.5 | 2.4 | 1.3 | 1.4 | 1.12 |
| YJR073C | 1.1 | 2.4 | 2.7 | 2.7 | 0.6 | 2.2 | 2.9 | 1.0 | 6.8 | 2.8 | 2.8 | 4.1 | 10.0 | 2.3 | 28.9 | 3.7 | 2.9 | 2.6 | 0.99 |
| YOR303W | 1.1 | 2.2 | 0.5 | 1.0 | 1.2 | 1.8 | 1.2 | 1.6 | 0.9 | 5.2 | 0.9 | 1.9 | 3.3 | 1.6 | 4.1 | 1.2 | 1.2 | 0.8 | 1.47 |
| YBR294W | 6.3 | 12.0 | 0.9 | 2.6 | 1.4 | 1.2 | 1.1 | 1.0 | 5.2 | 5.2 | 1.2 | 1.0 | 3.1 | 1.3 | 1.0 | 0.9 | 1.0 | 0.9 | 0.18 |
| YCL043C | 0.6 | 3.7 | 2.5 | 0.7 | 0.9 | 1.3 | 1.0 | 0.8 | 1.6 | 1.2 | 1.7 | 1.5 | 4.1 | 0.6 | 5.7 | 0.8 | 0.9 | 1.0 | 2.37 |
| YCL050C | 1.0 | 1.1 | 1.9 | 0.7 | 1.4 | 1.2 | 1.2 | 1.2 | 3.0 | 2.9 | 1.3 | 1.2 | 2.8 | 1.2 | 0.8 | 0.7 | 1.0 | 1.0 | 2.20 |
| YCR012W | 1.1 | 1.5 | 5.1 | 1.2 | 1.1 | 2.5 | 1.4 | 0.8 | 1.4 | 1.5 | 1.8 | 1.2 | 4.1 | 0.9 | 1.4 | 1.4 | 0.8 | 1.3 | 4.48 |
| YDR158W | 1.1 | 0.5 | 1.8 | 0.8 | 1.3 | 1.0 | 0.8 | 1.2 | 1.1 | 1.7 | 1.6 | 1.0 | 2.6 | 0.9 | 1.0 | 0.6 | 1.0 | 0.9 | 3.54 |
| YDR204W | 0.9 | 2.3 | 3.0 | 1.4 | 1.0 | 2.2 | 2.2 | 1.1 | 5.7 | 3.7 | 2.2 | 1.2 | 2.9 | 1.1 | 0.6 | 2.8 | 1.1 | 1.7 | 0.62 |
| YDR313C | 1.2 | 1.2 | 1.2 | 1.4 | 1.3 | 1.4 | 1.5 | 1.2 | 4.7 | 2.6 | 1.5 | 1.2 | 2.3 | 1.3 | 0.8 | 2.9 | 1.1 | 1.3 | 0.84 |
| YDR368W | 2.0 | 2.2 | 2.2 | 1.8 | 1.0 | 3.0 | 2.1 | 1.7 | 3.8 | 3.8 | 1.8 | 1.4 | 2.6 | 2.0 | 1.2 | 2.3 | 1.1 | 2.0 | 1.70 |
| YER091C | 3.6 | 3.9 | 30.7 | 1.3 | 0.9 | 2.3 | 2.1 | 0.9 | 5.8 | 1.4 | 3.2 | 2.0 | 5.4 | 1.2 | 10.2 | 2.7 | 1.2 | 1.0 | 1.23 |
| YFR053C | 1.7 | 1.4 | 3.0 | 2.2 | 0.9 | 3.2 | 2.1 | 2.8 | 0.6 | 1.1 | 1.5 | 1.7 | 3.0 | 0.6 | 0.6 | 3.0 | 1.4 | 2.2 | 4.17 |
| YGL062W | 0.6 | 1.3 | 1.1 | 0.6 | 0.9 | 1.0 | 1.3 | 0.8 | 1.6 | 2.5 | 0.7 | 1.6 | 4.5 | 1.2 | 2.7 | 1.5 | 1.1 | 0.9 | 0.77 |
| YGL157W | 0.7 | 0.9 | 1.8 | 0.9 | 1.5 | 5.4 | 3.3 | 1.4 | 0.6 | 1.8 | 1.4 | 1.5 | 4.8 | 1.2 | 5.1 | 1.7 | 1.8 | 2.3 | 1.69 |
| YGL184C | 4.6 | 15.9 | 57.4 | 1.3 | 1.1 | 1.0 | 1.2 | 1.9 | 37.9 | 66.8 | 1.7 | 2.6 | 7.4 | 1.4 | 0.4 | 1.5 | 1.0 | 0.9 | 0.16 |
| YGR032W | 1.0 | 1.4 | 1.0 | 1.3 | 0.7 | 1.9 | 0.8 | 0.6 | 0.5 | 0.7 | 1.5 | 1.5 | 3.9 | 1.1 | 5.4 | 1.5 | 3.2 | 2.8 | 1.18 |
| YGR124W | 0.5 | 0.6 | 1.7 | 0.7 | 1.2 | 1.1 | 0.7 | 1.0 | 2.2 | 1.3 | 1.1 | 1.1 | 2.3 | 0.5 | 1.5 | 0.3 | 0.7 | 0.6 | 3.56 |
| YGR192C | 1.4 | 1.0 | 3.8 | 1.0 | 1.1 | 1.7 | 1.7 | 1.1 | 0.9 | 1.3 | 2.3 | 2.2 | 3.4 | 1.0 | 1.9 | 1.1 | 1.1 | 1.3 | 7.49 |
| YGR244C | 1.0 | 1.2 | 1.6 | 1.6 | 0.8 | 2.6 | 3.9 | 1.8 | 1.4 | 1.1 | 1.8 | 1.9 | 2.6 | 1.2 | 2.0 | 2.6 | 1.9 | 3.1 | 1.12 |
| YGR254W | 1.2 | 1.3 | 3.8 | 1.3 | 1.2 | 1.9 | 1.5 | 1.4 | 0.8 | 1.2 | 1.3 | 1.7 | 3.1 | 0.6 | 2.4 | 1.4 | 1.3 | 1.2 | 7.01 |
| YHR018C | 1.0 | 1.0 | 1.5 | 1.4 | 1.7 | 1.1 | 0.8 | 1.0 | 1.4 | 4.4 | 1.4 | 1.4 | 3.3 | 1.0 | 4.4 | 1.0 | 1.9 | 1.2 | 1.21 |
| YIL160C | 0.8 | 2.4 | 2.3 | 3.5 | 1.0 | 1.6 | 1.1 | 1.2 | 3.2 | 3.8 | 2.4 | 1.0 | 2.3 | 1.5 | 5.6 | 8.8 | 2.4 | 3.0 | 0.27 |
| YIR017C | 3.5 | 8.6 | 2.5 | 1.9 | 0.8 | 1.9 | 1.5 | 1.2 | 5.2 | 3.4 | 4.2 | 1.2 | 3.4 | 1.9 | 2.3 | 3.0 | 1.5 | 1.3 | 0.36 |
| YJL052W | 1.5 | 0.9 | 4.0 | 1.8 | 0.7 | 2.4 | 2.1 | 1.5 | 1.6 | 2.0 | 4.3 | 2.4 | 5.6 | 1.3 | 2.3 | 2.3 | 1.2 | 1.9 | 6.19 |
| YJR009C | 1.1 | 1.7 | 5.6 | 1.0 | 1.2 | 1.6 | 1.6 | 1.1 | 1.1 | 1.3 | 1.1 | 1.5 | 2.7 | 1.0 | 2.1 | 1.5 | 1.1 | 1.3 | 5.86 |
| YJR130C | 1.1 | 0.6 | 0.8 | 0.9 | 1.4 | 2.0 | 1.3 | 1.3 | 5.2 | 3.8 | 1.7 | 1.0 | 2.9 | 1.1 | 0.9 | 1.5 | 1.1 | 1.3 | 0.62 |

| | | | | | | | | | | | | | | | | | | | |
|---|---|---|---|---|---|---|---|---|---|---|---|---|---|---|---|---|---|---|---|
| YJR149W | 1.0 | 1.8 | 1.4 | 2.3 | 0.8 | 1.0 | 1.7 | 1.0 | 1.4 | 1.0 | 0.7 | 1.0 | 3.5 | 1.4 | 1.4 | 2.4 | 1.1 | 1.2 | 0.34 |
| YKL218C | 2.1 | 1.0 | 2.2 | 1.8 | 1.2 | 3.6 | 1.6 | 1.7 | 3.4 | 2.7 | 1.7 | 1.2 | 5.1 | 1.4 | 0.8 | 3.1 | 2.5 | 2.6 | 0.60 |
| YLR027C | 1.0 | 1.5 | 2.3 | 0.8 | 0.9 | 2.0 | 1.4 | 0.7 | 1.6 | 2.7 | 1.6 | 1.3 | 2.5 | 1.0 | 1.4 | 0.9 | 1.3 | 1.3 | 1.39 |
| YLR133W | 0.9 | 1.6 | 1.4 | 0.7 | 1.0 | 2.2 | 1.3 | 0.7 | 6.1 | 2.3 | 0.6 | 1.5 | 3.3 | 1.1 | 1.9 | 1.0 | 1.1 | 1.3 | 0.36 |
| YLR155C | 1.4 | 1.1 | 2.4 | 1.8 | 1.7 | 1.6 | 2.0 | 1.0 | 3.0 | 1.8 | 2.0 | 1.1 | 3.3 | 1.1 | 1.2 | 2.2 | 1.4 | 2.2 | 1.63 |
| YLR158C | 1.4 | 1.2 | 2.1 | 1.7 | 1.7 | 1.5 | 2.0 | 1.1 | 2.7 | s | 2.4 | 1.1 | 3.2 | 1.2 | 1.1 | 2.1 | 1.4 | 2.0 | 1.57 |
| YLR195C | 0.8 | 0.9 | 1.0 | 0.8 | 1.0 | 0.8 | 1.1 | 1.0 | 0.6 | 0.7 | 0.8 | 1.3 | 2.3 | 0.7 | 0.9 | 0.8 | 0.9 | 1.0 | 1.55 |
| YLR345W | 1.4 | 1.4 | 1.4 | 1.2 | 1.2 | 1.6 | 1.2 | 1.4 | 4.5 | 4.0 | 2.5 | 1.6 | 3.5 | 1.5 | 1.0 | 1.2 | 1.2 | 1.4 | 2.65 |
| YML004C | 0.8 | 1.6 | 2.5 | 1.0 | 0.7 | 1.7 | 2.1 | 1.9 | 3.9 | 2.2 | 2.2 | 1.1 | 2.4 | 1.4 | 2.1 | 4.5 | 1.5 | 2.4 | 2.41 |
| YML131W | 11.2 | 4.3 | 7.7 | 1.9 | 0.7 | 2.0 | 3.0 | 6.1 | 11.1 | 17.7 | 14.4 | 1.1 | 2.4 | 2.7 | 2.2 | 1.6 | 2.5 | 3.0 | 1.15 |
| YNL071W | 0.9 | 0.9 | 1.7 | 1.5 | 0.6 | 1.3 | 1.4 | 0.9 | 1.1 | 1.2 | 1.6 | 1.3 | 2.5 | 0.8 | 1.1 | 1.1 | 1.0 | 1.2 | 1.61 |
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YOR120W | 1.8 | 5.4 | 2.8 | 3.2 | 1.4 | 3.4 | 2.7 | 2.0 | 3.3 | 3.0 | 3.0 | 1.2 | 3.6 | 2.4 | 1.5 | 3.9 | 1.3 | 2.6 | 1.06 |
| YAL023C | 0.4 | 1.5 | 1.0 | 0.7 | 1.0 | 0.6 | 0.8 | 0.6 | 0.3 | 0.4 | 1.7 | 1.2 | 2.3 | 0.4 | 2.5 | 0.4 | 1.0 | 0.7 | 1.49 |
| YAL060W | 1.1 | 1.8 | 3.2 | 2.7 | 1.2 | 4.2 | 3.3 | 0.9 | 0.6 | 2.5 | 2.4 | 0.8 | 2.2 | 0.9 | 0.9 | 3.2 | 1.1 | 1.8 | 2.39 |
| YAL062W | 0.8 | 1.5 | 1.4 | 1.1 | 1.0 | 1.2 | 1.1 | 0.8 | 1.4 | 0.7 | 1.2 | 1.0 | 2.1 | 0.8 | 1.1 | 1.3 | 1.0 | 0.8 | 0.86 |
| YBR006W | 1.3 | 1.6 | 2.6 | 1.5 | 1.3 | 3.1 | 3.3 | 1.0 | 4.5 | 2.7 | 1.9 | 1.1 | 2.2 | 1.1 | 1.1 | 3.0 | 1.2 | 1.8 | 0.62 |
| YBR056W | 1.4 | 1.7 | 1.4 | 2.3 | 1.4 | 2.3 | 5.5 | 1.4 | 3.0 | 3.3 | 1.8 | 1.4 | 3.0 | 1.0 | 0.7 | 2.8 | 2.4 | 3.4 | 1.13 |
| YBR149W | 1.1 | 2.0 | 2.7 | 1.7 | 1.4 | 2.8 | 3.1 | 1.5 | 1.7 | 1.9 | 1.9 | 1.4 | 2.2 | 1.2 | 0.7 | 2.7 | 2.1 | 3.4 | 2.72 |
| YBR284W | 0.9 | 1.5 | 2.8 | 4.8 | 1.3 | 1.9 | 1.0 | 0.9 | 6.5 | 3.2 | 1.4 | 1.2 | 2.8 | 1.6 | 0.8 | 0.8 | 1.1 | 1.0 | 0.25 |
| YCL018W | 1.2 | 2.7 | 2.4 | 1.4 | 0.8 | 2.3 | 2.3 | 1.3 | 2.1 | 4.3 | 3.7 | 1.6 | 3.2 | 1.0 | 0.6 | 2.3 | 1.3 | 1.0 | 0.99 |
| YCL040W | 0.9 | 7.1 | 10.1 | 2.0 | 0.5 | 3.5 | 2.9 | 0.7 | 0.9 | 3.0 | 8.2 | 2.3 | 5.6 | 0.7 | 3.4 | 3.1 | 1.4 | 1.7 | 1.98 |
| YDL010W | 1.7 | 0.8 | 0.6 | 0.7 | 1.2 | 1.1 | 1.9 | 1.1 | 2.2 | 1.3 | 1.3 | 0.9 | 2.0 | 2.2 | 1.4 | 1.4 | 1.5 | 2.4 | 0.93 |
| YDL024C | 1.6 | 1.5 | 1.2 | 2.2 | 1.0 | 1.7 | 0.9 | 1.7 | 4.2 | 3.4 | 1.2 | 1.7 | 2.6 | 1.9 | 2.1 | 2.8 | 0.9 | 1.1 | 0.40 |
| YDL095W | 0.5 | 1.3 | 1.0 | 0.8 | 0.8 | 1.0 | 0.8 | 0.7 | 1.2 | 0.7 | 1.0 | 1.1 | 2.2 | 0.5 | 2.0 | 0.5 | 0.7 | 0.9 | 1.57 |
| YDL245C | 1.0 | 2.4 | 1.6 | 2.2 | 1.3 | 1.1 | 1.2 | 1.2 | 0.4 | 2.8 | 1.5 | 1.0 | 2.0 | 1.2 | 1.8 | 1.5 | 1.0 | 1.1 | 0.28 |
| YDL246C | 1.2 | 1.5 | 1.3 | 1.1 | 1.1 | 1.4 | 2.1 | 2.4 | 4.4 | 3.6 | 2.6 | 1.2 | 2.9 | 1.7 | 2.6 | 1.7 | 1.8 | 1.8 | 0.43 |
| YDR001C | 0.8 | 2.2 | 2.6 | 1.0 | 1.1 | 2.2 | 1.5 | 0.9 | 2.2 | 3.0 | 0.7 | 1.1 | 2.8 | 1.0 | 4.4 | 1.9 | 1.1 | 1.6 | 0.75 |
| YDR058C | 1.2 | 2.2 | 0.8 | 2.7 | 0.7 | 1.0 | 1.4 | 1.9 | 2.4 | 1.7 | 1.7 | 1.2 | 2.5 | 1.8 | 1.2 | 1.9 | 1.1 | 1.6 | 0.56 |
| YDR072C | 1.3 | 1.3 | 1.7 | 1.2 | 0.9 | 1.5 | 0.9 | 0.7 | 0.6 | 0.7 | 0.7 | 0.9 | 1.9 | 0.6 | 0.4 | 0.7 | 1.9 | 1.9 | 2.81 |
| YDR127W | 0.8 | 0.9 | 1.5 | 0.7 | 1.2 | 0.8 | 0.9 | 0.7 | 0.6 | 1.3 | 1.3 | 0.8 | 1.7 | 1.0 | 1.3 | 0.6 | 0.9 | 0.7 | 0.78 |

| | | | | | | | | | | | | | | | | | | |
|---|---|---|---|---|---|---|---|---|---|---|---|---|---|---|---|---|---|---|
| YDR261C | 1.2 | 1.2 | 1.9 | 0.7 | 1.3 | 1.4 | 0.8 | 0.9 | 1.7 | 1.0 | 1.2 | 1.0 | 1.9 | 0.9 | 2.3 | 0.6 | 0.9 | 0.8 | 0.68 |
| YDR272W | 1.0 | 4.0 | 0.8 | 1.8 | 0.7 | 2.3 | 1.7 | 2.1 | 2.5 | 1.9 | 2.1 | 1.2 | 2.4 | 1.4 | 1.1 | 2.1 | 1.4 | 2.3 | 1.51 |
| YDR497C | 0.7 | 0.5 | 0.6 | 1.3 | 0.7 | 0.6 | 0.5 | 0.6 | 0.4 | 0.8 | 0.7 | 0.9 | 2.7 | 0.6 | 10.1 | | 1.6 | 1.8 | 1.45 |
| YDR516C | 1.2 | 1.5 | 7.8 | 2.1 | 1.3 | 1.8 | 3.9 | 1.3 | 1.8 | 5.2 | 2.8 | 1.1 | 2.5 | 0.7 | 0.4 | 1.7 | 1.1 | 1.2 | 1.66 |
| YER053C | 1.6 | 1.8 | 1.9 | 1.7 | 0.6 | 2.8 | 2.8 | 1.3 | 1.3 | 3.9 | 2.4 | 1.7 | 4.1 | 1.3 | 1.1 | 2.8 | 1.2 | 2.3 | 1.83 |
| YER096W | 1.1 | 2.3 | 2.0 | 2.0 | 1.3 | 1.2 | 0.9 | 1.0 | 7.5 | 3.5 | 1.1 | 0.9 | 2.3 | 3.1 | 1.4 | 2.0 | 1.0 | 1.2 | 0.22 |
| YER178W | 0.8 | 0.9 | 3.6 | 1.0 | 0.7 | 2.5 | 1.6 | 0.8 | 1.7 | 1.3 | 2.5 | 1.3 | 2.4 | 0.9 | 1.8 | 1.0 | 1.1 | 0.9 | 2.18 |
| YFL030W | 1.6 | 6.0 | 3.1 | 2.0 | 1.1 | 1.3 | 0.6 | 1.6 | 24.5 | 4.9 | 5.4 | 1.2 | 2.2 | 0.8 | 1.1 | 0.6 | 1.0 | 0.8 | 0.50 |
| YFL031W | 0.5 | 1.3 | 2.1 | 0.7 | 1.0 | 0.3 | 0.9 | 0.7 | 1.0 | 0.7 | 1.2 | 1.0 | 2.6 | 0.4 | 2.7 | 0.9 | 1.2 | 1.1 | 3.16 |
| YFR047C | 1.3 | 4.0 | 2.7 | 2.6 | 1.2 | 2.0 | 2.0 | 1.0 | 3.0 | 1.5 | 2.3 | 1.7 | 2.7 | 1.9 | 0.6 | 3.6 | 1.5 | 1.6 | 1.57 |
| YGL248W | 1.3 | 1.3 | 0.9 | 0.7 | 1.3 | 2.7 | 1.6 | 0.8 | 0.2 | 2.9 | 1.0 | 0.8 | 2.0 | 1.3 | 1.8 | 2.9 | 1.1 | 1.2 | 0.22 |
| YGR037C | 1.0 | 1.2 | 2.2 | 0.8 | 1.0 | 1.1 | 1.4 | 1.4 | 1.7 | 1.4 | 1.4 | 1.5 | 2.7 | 1.5 | 1.5 | 2.6 | 1.4 | 2.0 | 3.40 |
| YGR055W | 2.5 | 5.7 | 12.0 | 0.7 | 1.3 | 1.1 | 0.6 | 1.1 | 5.0 | 2.4 | 1.5 | 0.9 | 1.9 | 1.2 | 2.1 | 0.6 | 0.7 | 0.7 | 1.42 |
| YGR194C | 0.9 | 1.0 | 1.3 | 2.1 | 0.8 | 1.9 | 2.2 | 1.2 | 2.4 | 2.1 | 0.7 | 0.8 | 2.2 | 1.0 | 0.6 | 2.6 | 1.1 | 2.0 | 0.63 |
| YGR256W | 1.2 | 1.5 | 2.3 | 0.8 | 0.9 | 6.2 | 1.4 | 2.2 | 3.4 | 2.5 | 2.7 | 1.5 | 3.9 | 1.1 | 2.7 | 5.3 | 1.1 | 0.8 | 0.94 |
| YHR111W | 1.4 | 1.3 | 0.8 | 1.9 | 1.5 | 1.3 | 1.6 | 1.3 | 4.5 | 4.2 | 1.9 | 0.9 | 2.2 | 1.7 | 1.1 | 1.3 | 1.1 | 1.2 | 0.44 |
| YHR174W | 1.1 | 1.4 | 3.3 | 1.2 | 1.3 | 1.5 | 1.6 | 1.2 | 1.0 | 1.5 | 1.4 | 1.5 | 3.6 | 0.6 | 2.0 | 1.1 | 1.0 | 1.2 | 7.34 |
| YHR176W | 1.8 | 2.0 | 1.1 | 1.4 | 1.0 | 2.3 | 1.4 | 1.3 | 6.3 | 6.4 | 1.2 | 1.2 | 1.9 | 1.3 | 1.5 | 1.8 | 1.4 | 1.6 | 0.27 |
| YIL045W | 1.7 | 1.4 | 1.9 | 2.2 | 1.3 | 1.7 | 1.6 | 1.1 | 2.1 | 3.2 | 1.2 | 1.5 | 2.0 | 1.6 | 0.6 | 2.9 | 1.1 | 1.8 | 0.37 |
| YIL107C | 1.5 | 0.8 | 1.1 | 1.5 | 0.8 | 1.0 | 1.6 | 1.3 | 3.5 | 3.5 | 0.9 | 1.1 | 1.9 | 1.6 | 1.0 | 2.7 | 1.2 | 2.0 | 0.58 |
| YIL155C | 1.0 | 0.7 | 1.4 | 3.7 | 1.3 | 1.4 | 2.2 | 1.0 | 2.5 | 2.9 | 1.3 | 1.4 | 2.0 | 1.3 | 1.4 | 3.8 | 1.1 | 1.4 | 0.51 |
| YIR034C | 1.2 | 1.1 | 2.5 | 0.6 | 1.1 | 1.3 | 0.8 | 1.7 | 3.2 | 3.5 | 3.0 | 1.4 | 2.6 | 1.2 | 1.9 | 1.3 | 1.0 | 1.0 | 0.92 |
| YIR036C | 1.0 | 1.7 | 2.6 | 1.8 | 0.9 | 0.7 | 1.0 | 1.0 | 3.3 | 1.9 | 2.8 | 1.2 | 1.9 | 1.2 | 1.1 | 4.0 | 1.5 | 1.3 | 0.64 |
| YJL031C | 1.6 | 1.2 | 0.5 | 1.3 | 1.7 | 1.6 | 2.3 | 1.8 | 4.7 | 3.6 | 1.9 | 1.3 | 2.6 | 3.0 | 2.0 | 1.9 | 1.2 | 1.9 | 1.02 |
| YJL068C | 1.2 | 2.7 | 3.4 | 1.6 | 0.7 | 1.1 | 1.2 | 1.1 | 6.5 | 3.1 | 3.2 | 1.7 | 2.7 | 1.5 | 0.5 | 2.6 | 1.4 | 1.5 | 0.95 |
| YJL099W | 1.2 | 1.2 | 0.3 | 1.3 | 1.9 | 2.0 | 1.8 | 1.0 | 3.1 | 3.1 | 0.9 | 1.0 | 2.4 | 1.8 | 1.4 | 1.7 | 1.2 | 1.6 | 0.55 |
| YJL172W | 0.8 | 2.1 | 1.8 | 1.5 | 0.9 | 3.8 | 1.0 | 0.7 | 0.7 | 1.9 | 1.4 | 1.1 | 3.1 | 0.9 | 1.2 | 0.5 | 1.8 | 1.8 | 0.62 |
| YJL219W | 1.2 | 2.5 | 3.1 | 1.6 | 1.2 | 1.5 | 0.9 | 1.5 | 4.0 | 2.3 | 2.3 | 1.1 | 4.0 | 1.1 | 2.6 | 1.4 | 1.6 | 1.2 | 0.91 |
| YJR137C | 2.7 | 2.9 | 4.6 | 0.8 | 0.6 | 1.5 | 0.8 | 1.6 | 9.0 | 4.8 | 2.0 | 1.4 | 2.1 | 1.2 | 1.0 | 1.3 | 0.9 | 0.8 | 0.46 |
| YKL035W | 1.0 | 0.9 | 4.8 | 1.2 | 0.8 | 1.2 | 0.6 | 1.0 | 0.8 | 1.2 | 2.1 | 1.0 | 2.0 | 0.6 | 1.0 | 1.5 | 1.0 | 1.9 | 2.54 |
| YKL091C | 1.2 | 1.3 | 1.4 | 2.6 | 1.0 | 2.2 | 1.9 | 1.3 | 2.3 | 3.2 | 1.7 | 1.3 | 2.0 | 1.5 | 1.1 | 4.6 | 1.4 | 2.0 | 0.83 |

| | | | | | | | | | | | | | | | | | | | |
|---|---|---|---|---|---|---|---|---|---|---|---|---|---|---|---|---|---|---|---|
| YKL104C | 1.0 | 1.0 | 1.6 | 1.2 | 1.4 | 0.9 | 2.3 | 1.1 | 1.4 | 1.8 | 2.2 | 1.6 | 2.2 | 0.8 | 3.3 | 0.6 | 1.0 | 1.1 | 1.25 |
| YKL152C | 1.3 | 1.3 | 1.9 | 0.9 | 1.3 | 1.6 | 1.5 | 1.0 | 0.9 | 1.5 | 1.7 | 1.5 | 2.7 | 1.0 | 1.8 | 2.0 | 1.1 | 1.7 | 3.28 |
| YKL213C | 0.9 | 0.8 | 1.0 | 1.3 | 0.7 | 1.0 | 1.0 | 1.1 | 4.8 | 2.1 | 1.3 | 0.9 | 2.1 | 0.9 | 1.1 | 1.5 | 1.4 | 1.6 | 0.77 |
| YKL215C | 0.6 | 1.1 | 1.3 | 0.8 | 1.0 | 2.1 | 1.1 | 1.1 | 1.3 | 1.9 | 0.5 | 1.0 | 2.6 | 1.0 | 0.9 | 0.8 | 0.8 | 0.9 | 0.38 |
| YLL058W | 1.0 | 3.8 | 1.4 | 0.9 | 0.8 | 1.4 | 1.5 | 0.9 | 3.5 | 3.6 | 2.0 | 1.0 | 2.3 | 0.8 | 1.9 | 1.1 | 1.4 | 1.3 | 0.39 |
| YLR299W | 1.2 | 1.9 | 1.5 | 1.3 | 0.6 | 0.9 | 0.8 | 1.0 | 4.9 | 2.3 | 1.7 | 1.0 | 2.9 | 1.3 | 1.5 | 0.9 | 1.3 | 1.4 | 0.57 |
| YLR348C | 1.1 | 4.5 | 1.2 | 1.2 | 0.9 | 1.9 | 0.9 | 0.9 | 2.1 | 1.3 | 1.3 | 0.9 | 1.9 | 1.0 | 2.4 | 1.1 | 1.1 | 1.0 | 0.64 |
| YML054C | 1.5 | 1.8 | 1.3 | 3.4 | 1.3 | 1.8 | 1.2 | 1.5 | 4.1 | 1.8 | 1.4 | 1.8 | 2.8 | 2.1 | 1.1 | 7.8 | 1.1 | 1.6 | 0.25 |
| YML070W | 0.9 | 1.7 | 2.5 | 1.9 | 1.4 | 2.2 | 3.1 | 1.3 | 4.5 | 3.9 | 1.5 | 1.3 | 3.3 | 1.3 | 0.9 | 2.6 | 1.2 | 1.3 | 1.03 |
| YML100W | 0.8 | 2.7 | 10.6 | 1.4 | 0.9 | 2.8 | 2.2 | 0.7 | 1.7 | 1.4 | 3.2 | 1.2 | 3.8 | 1.2 | 1.8 | 2.2 | 1.0 | 1.5 | 0.88 |
| YMR008C | 0.7 | 0.9 | 1.4 | 1.0 | 1.1 | 1.7 | 0.9 | 0.5 | 0.3 | 0.6 | 1.3 | 1.1 | 3.7 | 0.7 | 2.0 | 1.2 | 3.0 | 1.9 | 1.59 |
| YMR020W | 1.3 | 1.0 | 0.9 | 1.7 | 1.4 | 1.6 | 2.7 | 1.2 | 2.3 | 2.9 | 1.5 | 1.1 | 3.0 | 1.1 | 1.8 | 2.8 | 2.3 | 2.2 | 0.81 |
| YMR105C | 1.9 | 3.0 | 5.0 | 4.2 | 0.9 | 2.8 | 2.8 | 1.1 | 0.6 | 2.9 | 3.0 | 1.7 | 3.3 | 1.0 | 0.9 | 2.0 | 1.6 | 2.6 | 1.21 |
| YMR271C | 1.6 | 1.6 | 1.3 | 2.0 | 1.1 | 4.2 | 3.4 | 1.8 | 8.0 | 4.4 | 1.7 | 1.3 | 3.6 | 1.8 | 0.6 | 5.7 | 1.3 | 2.2 | 0.70 |
| YNL012W | 1.3 | 1.3 | 1.8 | 0.8 | 1.3 | 1.7 | 2.0 | 1.8 | 3.3 | 3.2 | 1.6 | 1.3 | 2.9 | 1.0 | 1.6 | 1.2 | 1.3 | 1.5 | 0.24 |
| YNL045W | 0.8 | 1.3 | 2.0 | 1.3 | 1.1 | 2.2 | 1.8 | 0.7 | 1.3 | 1.1 | 2.1 | 0.9 | 2.6 | 0.8 | 0.8 | 2.4 | 1.1 | 1.1 | 0.80 |
| YNL104C | 1.1 | 1.3 | 3.0 | 0.9 | 0.8 | 0.8 | 0.6 | 0.9 | 1.1 | 2.0 | 2.1 | 1.1 | 2.1 | 0.9 | 2.5 | 1.5 | 0.9 | 0.9 | 1.69 |
| YNL231C | 1.5 | 0.8 | 0.8 | 0.5 | 2.1 | 1.8 | 2.5 | 1.5 | 2.2 | 1.3 | 1.3 | 1.8 | 2.9 | 0.9 | 0.9 | 0.7 | 3.1 | 4.0 | 2.13 |
| YNR019W | 1.0 | 1.3 | 1.6 | 1.2 | 0.8 | 1.3 | 0.8 | 0.7 | 1.8 | 1.9 | 0.7 | 1.0 | 2.1 | 1.0 | 1.6 | 0.8 | 2.4 | 2.0 | 0.37 |
| YNR033W | 0.6 | 0.8 | 0.8 | 1.0 | 1.3 | 1.6 | 1.2 | 1.1 | 3.6 | 4.2 | 0.4 | 1.1 | 3.8 | 0.9 | 1.3 | 2.2 | 0.9 | 1.1 | 0.72 |
| YNR059W | 1.1 | 0.9 | 0.2 | 1.4 | 1.4 | 0.8 | 1.6 | 1.3 | 1.8 | 1.4 | 1.0 | 0.8 | 2.4 | 1.0 | 1.0 | 2.0 | 2.6 | 2.2 | 0.45 |
| YOL126C | 1.0 | 0.8 | 1.0 | 1.6 | 0.7 | 1.5 | 1.4 | 0.9 | 0.8 | 2.4 | 1.8 | 1.1 | 2.6 | 1.2 | 1.4 | 2.5 | 1.6 | 2.4 | 0.59 |
| YOL153C | 1.0 | 1.2 | 2.1 | 3.9 | 1.2 | 1.9 | 3.3 | 1.0 | 2.5 | 3.2 | 2.4 | 1.9 | 2.9 | 1.5 | 1.2 | 5.4 | 1.1 | 1.7 | 0.37 |
| YOR099W | 0.8 | 1.3 | 2.1 | 1.3 | 1.5 | 0.9 | 1.0 | 0.9 | 0.6 | 0.7 | 1.2 | 1.1 | 1.6 | 0.7 | 1.5 | 1.1 | 1.2 | 2.3 | 3.94 |
| YOR130C | 0.8 | 1.7 | 1.4 | 1.0 | 1.8 | 1.6 | 1.3 | 0.8 | 2.0 | 1.7 | 0.6 | 0.9 | 2.1 | 1.3 | 1.0 | 0.8 | 1.5 | 1.1 | 0.51 |
| YOR336W | 0.7 | 1.4 | 0.7 | 1.0 | 1.3 | 1.7 | 1.2 | 0.8 | 1.7 | 1.5 | 1.4 | 1.0 | 2.1 | 1.1 | 0.6 | 0.9 | 1.0 | 1.0 | 0.44 |
| YOR347C | 0.9 | 0.9 | 2.1 | 2.2 | 0.7 | 0.9 | 0.9 | 0.8 | 0.5 | 0.9 | 1.4 | 1.2 | 1.8 | 0.9 | 2.0 | 1.4 | 1.7 | 1.3 | 1.31 |
| YPL017C | 1.0 | 2.5 | 0.8 | 1.0 | 1.3 | 1.5 | 1.4 | 1.3 | 3.0 | 6.7 | 0.4 | 1.0 | 1.9 | 1.6 | 1.0 | 1.3 | 1.3 | 0.8 | 0.19 |
| YPR026W | 0.9 | 1.2 | 5.0 | 1.3 | 0.8 | | 1.3 | 1.0 | 0.9 | 3.1 | 1.6 | 1.2 | 2.3 | 1.5 | 3.1 | 2.5 | 0.9 | 1.3 | 0.26 |
| YAL012W | 1.1 | 6.5 | 10.4 | 0.8 | 0.6 | 1.6 | 0.6 | 0.7 | 4.7 | 3.9 | 4.0 | 1.1 | 1.1 | 0.9 | 3.1 | 1.0 | 0.9 | 0.8 | 0.96 |
| YBR029C | 0.7 | 0.2 | 1.8 | 1.0 | 1.4 | 0.8 | 0.4 | 0.7 | 2.0 | 1.1 | 0.7 | 1.3 | 1.7 | 0.9 | 3.5 | 0.9 | 0.6 | 0.8 | 1.56 |

| | | | | | | | | | | | | | | | | | | |
|---|---|---|---|---|---|---|---|---|---|---|---|---|---|---|---|---|---|---|
| YBR222C | 0.6 | 1.2 | 1.3 | 0.8 | 0.7 | 1.3 | 1.0 | 0.6 | 0.7 | 1.0 | 1.3 | 0.9 | 1.6 | 0.5 | 4.3 | 2.1 | 0.8 | 0.9 | 0.52 |
| YCL009C | 0.6 | 0.9 | 2.0 | 0.5 | 0.7 | 1.8 | 1.3 | 1.1 | 0.9 | 1.6 | 1.5 | 1.7 | 1.6 | 0.7 | 6.5 | 0.9 | 1.1 | 0.8 | 0.68 |
| YCL064C | 0.6 | 0.6 | 0.8 | 0.8 | 1.4 | 3.3 | 13.4 | 2.4 | 1.7 | 5.2 | 1.8 | 0.7 | 1.6 | 0.8 | 21.4 | 1.0 | 0.3 | 0.3 | 1.42 |
| YCR098C | 1.6 | 3.0 | 1.7 | 2.0 | 1.4 | 1.6 | 1.2 | 1.0 | 1.4 | 2.4 | 1.3 | 1.3 | 1.4 | 1.1 | 11.0 | 0.8 | 1.7 | 1.6 | 0.22 |
| YDR502C | 1.2 | 2.8 | 2.4 | 1.2 | 1.5 | 1.5 | 0.9 | 0.9 | 1.6 | 0.9 | 1.3 | 1.3 | 1.2 | 0.8 | 8.2 | 1.0 | 0.9 | 0.9 | 2.75 |
| YER026C | 0.8 | 0.6 | 3.3 | 1.0 | 1.2 | 2.5 | 1.6 | 0.8 | 0.9 | 1.5 | 0.9 | 1.7 | 1.9 | 1.1 | 4.0 | 1.4 | 1.7 | 3.2 | 4.48 |
| YHR137W | 0.7 | 1.0 | 2.4 | 1.7 | 1.0 | 0.7 | 0.4 | 0.4 | 0.2 | 0.9 | 0.6 | 0.6 | 0.6 | 0.8 | 2.8 | 0.4 | 0.8 | 1.3 | 1.40 |
| YMR189W | 0.7 | 0.8 | 1.9 | 2.8 | 0.4 | 1.9 | 0.8 | 0.4 | 0.7 | 0.9 | 8.2 | 0.6 | 0.9 | 1.6 | 5.6 | 0.9 | 1.0 | 0.8 | 0.88 |
| YNL106C | 1.0 | 1.4 | 0.8 | 1.5 | 0.8 | 1.0 | 0.9 | 0.8 | 0.7 | 1.4 | 0.9 | 0.8 | 0.9 | 1.1 | 3.3 | 1.2 | 1.1 | 0.9 | 0.37 |
| YNL169C | 0.7 | 1.4 | 1.4 | 1.2 | 0.7 | 1.4 | 0.7 | 0.8 | 1.1 | 1.1 | 0.8 | 1.0 | 1.7 | 0.9 | 3.4 | 0.8 | 1.1 | 1.3 | 1.05 |
| YNL322C | 0.8 | 0.9 | 1.9 | 0.8 | 1.4 | 1.1 | 0.9 | 0.7 | 0.7 | 0.8 | 0.6 | 0.7 | 0.8 | 1.2 | 3.7 | 0.9 | 0.9 | 1.0 | 0.75 |
| YAL038W | 1.0 | 1.0 | 3.0 | 1.4 | 1.4 | 1.3 | 0.9 | 1.1 | 0.1 | 1.1 | 1.0 | 1.2 | 1.8 | 0.5 | 3.1 | 0.9 | 1.0 | 1.0 | 7.02 |
| YBR023C | 0.5 | 0.8 | 1.3 | 1.1 | 0.8 | 0.5 | 0.7 | 0.5 | 0.3 | 0.4 | 0.9 | 0.8 | 0.5 | 0.8 | 2.8 | 0.7 | 0.7 | 0.9 | 0.93 |
| YCR048W | 0.5 | 1.1 | 1.0 | 0.6 | 0.9 | 0.2 | 0.9 | 0.8 | 0.8 | 0.5 | 1.2 | 0.9 | 1.3 | 0.9 | 3.1 | 1.1 | 1.2 | 0.8 | 0.29 |
| YDR380W | 0.8 | 0.8 | 0.8 | 1.7 | 1.1 | 0.4 | 0.5 | 0.2 | 0.2 | 0.3 | 0.4 | 0.4 | 0.4 | 0.6 | 2.4 | 0.2 | 0.8 | 0.8 | 1.04 |
| YER069W | 1.3 | 0.8 | 3.3 | 1.4 | 1.5 | 1.3 | 1.0 | 1.5 | 0.8 | 10.4 | 1.5 | 0.8 | 1.4 | 1.3 | 2.4 | 1.1 | 1.1 | 0.9 | 0.25 |
| YGL022W | 0.5 | 0.8 | 1.4 | 0.3 | 1.0 | 0.9 | 0.7 | 0.4 | 0.9 | 0.4 | 0.8 | 0.7 | 1.0 | 0.5 | 4.0 | 0.3 | 0.9 | 0.7 | 0.91 |
| YGL126W | 0.6 | 1.3 | 1.4 | 0.8 | 1.1 | 1.6 | | 0.7 | 1.6 | 0.7 | 1.0 | 0.6 | | | 2.8 | | 0.9 | 0.9 | 0.34 |
| YGL209W | 1.2 | 1.6 | 1.1 | 2.6 | 0.7 | 1.0 | 1.0 | 1.1 | 0.6 | 0.4 | 0.8 | 1.3 | 1.9 | 1.6 | 3.2 | 2.8 | 1.1 | 1.5 | 0.63 |
| YGR282C | 1.3 | 0.7 | 4.6 | 1.9 | 1.0 | 2.2 | 1.1 | 1.0 | 0.8 | 0.8 | 1.5 | 1.3 | 1.2 | 1.0 | 2.9 | 1.4 | 1.1 | 1.5 | 5.04 |
| YIL154C | 0.8 | 1.3 | 1.9 | 0.8 | 0.9 | 1.1 | 1.3 | 0.6 | 2.4 | 1.0 | 1.2 | 1.1 | 1.7 | 0.9 | 4.3 | 1.2 | 1.2 | 1.0 | 0.39 |
| YJL088W | 0.9 | 0.8 | 2.2 | 0.5 | 0.9 | 2.8 | | 1.3 | 3.2 | 4.1 | 0.9 | 0.7 | 1.0 | 2.0 | 5.7 | 0.7 | 1.4 | 0.9 | 0.27 |
| YJR148W | 0.7 | 1.4 | 2.0 | 3.5 | 0.6 | 2.0 | 2.1 | 0.6 | 0.7 | 3.0 | 0.6 | 0.8 | 1.1 | 1.3 | 3.2 | 1.3 | 1.3 | 1.7 | 1.50 |
| YLR180W | 1.3 | 1.4 | 2.4 | 1.0 | 0.8 | 1.2 | 0.6 | 0.9 | 0.8 | 0.9 | 1.7 | 0.6 | 0.6 | 0.6 | 2.5 | 0.5 | 0.8 | 0.7 | 3.23 |
| YLR273C | 1.4 | 1.0 | 1.3 | 2.4 | | 1.2 | 1.2 | 1.3 | 2.8 | 2.4 | 1.4 | 0.9 | 1.7 | 1.3 | 2.6 | 1.5 | 1.0 | 1.0 | 0.20 |
| YLR300W | 0.9 | 0.8 | 0.8 | 1.0 | 0.8 | 1.1 | 0.5 | 0.8 | 0.0 | 0.5 | 1.0 | 0.5 | 0.2 | 0.6 | 2.7 | 0.3 | 0.7 | 0.5 | 5.14 |
| YLR307W | 0.9 | 0.4 | 0.7 | 3.3 | 1.4 | 1.2 | 1.5 | 1.2 | 0.6 | 0.7 | 0.8 | 0.7 | 1.0 | 1.9 | 3.9 | 0.9 | 1.1 | 1.0 | 0.16 |
| YMR296C | 0.6 | 0.7 | 1.2 | 2.6 | 0.4 | 0.4 | 0.6 | 0.5 | 0.4 | 0.6 | 0.8 | 0.8 | 0.9 | 0.7 | 6.7 | 0.5 | 0.8 | 0.7 | 0.52 |
| YOL058W | 0.8 | 0.5 | 1.7 | 0.9 | 1.2 | 0.8 | 0.4 | 1.6 | 0.1 | 15.7 | 2.3 | 1.3 | 1.2 | 1.5 | 2.7 | 0.9 | 1.2 | 0.8 | 0.82 |
| YBR183W | 1.8 | 1.5 | 2.0 | 2.9 | 0.9 | 2.5 | 1.7 | 1.0 | 0.4 | 2.0 | 1.9 | 1.2 | 1.4 | 1.3 | 1.1 | 2.1 | 1.4 | 2.4 | 1.47 |
| YDR019C | 1.4 | 0.5 | 1.7 | 4.0 | 0.9 | 4.8 | 1.4 | 0.7 | 0.4 | 0.7 | 1.8 | 0.7 | 1.0 | 1.6 | 1.8 | 1.8 | 1.2 | 1.6 | 2.48 |

| | | | | | | | | | | | | | | | | | | | |
|---|---|---|---|---|---|---|---|---|---|---|---|---|---|---|---|---|---|---|---|
| YIL167W | 3.3 | 1.6 | 2.0 | 1.0 | 1.0 | 7.7 | 1.2 | 1.9 | 6.9 | 12.7 | 1.9 | 0.7 | 1.2 | 1.4 | 0.6 | 2.3 | 0.7 | 0.8 | 2.11 |
| YKR039W | 1.4 | 3.5 | 1.3 | 1.7 | 1.0 | 2.8 | 1.0 | 1.0 | 1.9 | 2.0 | 1.9 | 1.5 | 1.3 | 1.2 | 1.7 | 1.9 | 0.9 | 1.1 | 0.40 |
| YNL037C | 1.4 | 1.8 | 1.7 | 1.1 | 1.7 | 2.8 | 2.8 | 1.0 | 0.6 | 3.2 | 1.4 | 1.3 | 1.9 | 1.3 | 0.9 | 1.4 | 0.9 | 1.3 | 2.05 |
| YNR002C | 1.3 | 1.6 | 1.0 | 2.0 | 1.2 | 2.3 | 1.2 | 1.0 | 3.9 | 1.6 | 1.6 | 1.2 | 2.1 | 1.2 | 1.0 | 2.4 | 1.5 | 2.1 | 0.29 |
| YOL143C | 1.0 | 1.4 | 1.2 | 1.2 | 0.9 | 3.5 | 1.3 | 1.2 | 0.8 | 1.0 | 2.0 | 1.2 | 1.2 | 0.7 | 1.0 | 2.2 | 1.8 | 1.4 | 2.30 |
| YOR136W | 1.0 | 0.9 | 3.5 | 1.0 | 1.5 | 3.9 | 3.2 | 1.1 | 0.3 | 3.4 | 1.4 | 1.2 | 1.5 | 0.9 | 1.2 | 1.3 | 0.8 | 1.3 | 3.20 |
| YAL044C | 1.5 | 1.1 | 1.0 | 3.0 | 0.8 | 2.2 | 1.0 | 0.8 | 0.4 | 0.6 | 1.4 | 0.6 | 1.1 | 1.4 | 0.7 | 1.0 | 1.4 | 1.8 | 4.07 |
| YAL054C | 1.2 | 1.1 | 1.4 | 1.6 | 1.1 | 2.2 | 1.1 | 1.1 | 8.9 | 4.1 | 1.3 | 1.0 | 1.8 | 1.3 | 0.9 | 2.5 | 1.5 | 1.4 | 0.24 |
| YAR071W | 1.6 | 0.8 | 1.0 | 0.7 | 1.6 | 2.0 | 0.3 | 1.4 | 0.3 | 0.3 | 1.1 | 2.0 | 0.3 | 1.1 | 1.1 | 0.3 | 0.7 | 1.6 | 3.21 |
| YBL001C | 1.1 | 1.5 | 1.0 | 1.6 | 1.0 | 2.4 | 1.3 | 1.0 | 1.5 | 0.9 | 2.3 | 0.7 | 1.0 | 1.5 | 1.0 | 1.7 | 1.6 | 1.8 | 2.38 |
| YBR014C | 1.1 | 1.2 | 0.8 | 0.9 | 1.4 | 2.0 | 1.4 | 1.2 | 1.3 | 0.8 | 1.8 | 0.6 | 0.9 | 1.2 | 1.2 | 1.5 | 1.0 | 1.6 | 1.29 |
| YBR035C | 1.1 | 1.0 | 1.7 | 1.1 | 1.3 | 2.3 | 1.5 | 1.5 | 2.2 | 1.5 | 2.0 | 1.2 | 1.8 | 1.3 | 0.7 | 2.1 | 1.5 | 1.9 | 1.89 |
| YBR068C | 1.1 | 1.0 | 1.9 | 2.5 | 1.4 | 1.9 | | 1.0 | 0.8 | 2.5 | 0.8 | 0.8 | 2.2 | 1.8 | 1.6 | 5.5 | 1.1 | 1.3 | 1.99 |
| YBR111C | 1.0 | 0.9 | 1.0 | 1.7 | 1.3 | 3.0 | 2.2 | 1.7 | 1.9 | 1.9 | 1.5 | 1.2 | 1.6 | 1.6 | 0.7 | 3.5 | 1.4 | 2.4 | 3.20 |
| YCR037C | 0.7 | 1.0 | 0.8 | 0.8 | 1.3 | 1.8 | 1.0 | 0.7 | 0.6 | 0.6 | 0.8 | 0.7 | 1.3 | 0.8 | 1.0 | 1.1 | 1.4 | 0.9 | 0.74 |
| YDL022W | 0.9 | 2.6 | 6.9 | 1.5 | 0.7 | 2.1 | 1.5 | 0.8 | 1.2 | 1.8 | 2.8 | 1.2 | 1.5 | 0.8 | 0.9 | 1.1 | 1.0 | 1.4 | 1.79 |
| YDR009W | 1.4 | 0.8 | 1.5 | 1.0 | 1.2 | 2.4 | 1.4 | 1.2 | 1.3 | 2.0 | 0.1 | 1.0 | 1.2 | 1.5 | 3.2 | 1.0 | 1.2 | 1.0 | 0.21 |
| YDR410C | 0.8 | 2.0 | 1.3 | 0.9 | 1.1 | 2.5 | 1.4 | 0.9 | 1.2 | 1.5 | 1.6 | 0.9 | 1.2 | 1.3 | 1.2 | 0.8 | 1.0 | 1.0 | 1.33 |
| YDR487C | 2.2 | 0.9 | 1.3 | 1.1 | 1.3 | 2.3 | 1.3 | 1.8 | 2.7 | 2.3 | 2.1 | 1.3 | 1.5 | 2.5 | 1.0 | 1.5 | 1.5 | 1.9 | 2.61 |
| YEL011W | 2.0 | 1.6 | 1.7 | 6.3 | 1.1 | 2.8 | 2.7 | 0.9 | 2.5 | 1.6 | 3.7 | 1.5 | 1.5 | 1.5 | 0.4 | 2.4 | 1.4 | 2.5 | 0.89 |
| YFR015C | 1.2 | 0.8 | 3.2 | 2.1 | 1.2 | 3.0 | 7.3 | 0.7 | 0.3 | 1.8 | 3.9 | 0.4 | 2.5 | 3.1 | 0.4 | 2.4 | 1.3 | 1.2 | 0.66 |
| YGL001C | 1.0 | 1.0 | 1.4 | 1.1 | 1.5 | 2.8 | 1.4 | 1.1 | 0.8 | 0.8 | 1.6 | 0.9 | 1.0 | 1.1 | 0.9 | 2.2 | 1.9 | 2.1 | 2.53 |
| YGL104C | 0.8 | 2.0 | 2.1 | 1.8 | 1.0 | 2.1 | 1.2 | 0.8 | 4.6 | 2.1 | 1.8 | 1.1 | 2.0 | 1.0 | 0.9 | 1.7 | 1.3 | 1.3 | 0.58 |
| YGL154C | 1.3 | 1.4 | 1.5 | 0.9 | 1.0 | 1.7 | 0.7 | 1.2 | 2.0 | 1.4 | 0.9 | 0.9 | 2.0 | 1.1 | 0.8 | 0.7 | 1.0 | 1.1 | 0.57 |
| YGL253W | 0.8 | 1.0 | 3.2 | 0.7 | 1.3 | 2.4 | 1.7 | 1.1 | 0.3 | 1.4 | 1.0 | 1.2 | 1.5 | 0.5 | 0.8 | 0.4 | 0.7 | 0.8 | 4.63 |
| YGR060W | 0.7 | 0.6 | 0.9 | 0.7 | 1.9 | 2.5 | 0.5 | 1.0 | 0.5 | 0.4 | 0.8 | 1.0 | 0.6 | 0.8 | 1.4 | 2.1 | 1.3 | 1.6 | 4.19 |
| YHR037W | 0.7 | 0.9 | 1.3 | 1.6 | 0.9 | 1.8 | 1.2 | 1.0 | 0.9 | 2.2 | 1.7 | 1.1 | 1.1 | 1.1 | 1.1 | 2.0 | 1.0 | 1.4 | 1.15 |
| YHR092C | 2.3 | 1.0 | 7.5 | 2.5 | 1.4 | 1.7 | 0.5 | 1.5 | 0.2 | 0.9 | 1.5 | 1.0 | 0.5 | 1.6 | 0.2 | 1.2 | 0.9 | 1.3 | 6.09 |
| YHR190W | 0.8 | 3.9 | 1.2 | 1.2 | 1.8 | 1.7 | 1.9 | 1.2 | 2.4 | 1.9 | 1.3 | 1.1 | 1.7 | 1.3 | 1.3 | 1.8 | 1.3 | 1.8 | 2.15 |
| YIL033C | 0.8 | 1.3 | 3.3 | 1.3 | 0.7 | 2.0 | 1.1 | 0.7 | 2.4 | 1.3 | 1.8 | 1.1 | 2.5 | 0.7 | 1.0 | 2.0 | 1.0 | 1.2 | 1.18 |
| YIR035C | 1.4 | 1.8 | 0.9 | 1.6 | 0.7 | 2.1 | 1.3 | 1.3 | 0.7 | 1.2 | 1.3 | 0.9 | 1.1 | 1.1 | 1.4 | 1.0 | 1.1 | 0.9 | 1.51 |

| | | | | | | | | | | | | | | | | | | | |
|---|---|---|---|---|---|---|---|---|---|---|---|---|---|---|---|---|---|---|---|
| YJL132W | 1.0 | 0.7 | 0.8 | 1.6 | 1.3 | 2.0 | 1.7 | 1.0 | 1.6 | 1.4 | 1.4 | 1.0 | 1.3 | 1.4 | 1.1 | 2.1 | 1.3 | 1.5 | 0.37 |
| YJL196C | 0.9 | 0.9 | 2.5 | 1.3 | 1.1 | 2.0 | 0.7 | 0.7 | 1.1 | 0.9 | 2.3 | 1.1 | 0.6 | 0.8 | 0.6 | 1.7 | 0.9 | 1.9 | 2.85 |
| YJR142W | 1.2 | 0.6 | 1.1 | 1.1 | 1.3 | 2.4 | 1.7 | 1.0 | 0.6 | 0.8 | 0.9 | 0.8 | 1.6 | 1.3 | 0.6 | 2.0 | 1.2 | 1.4 | 0.94 |
| YKL067W | 1.1 | 1.9 | 2.1 | 1.4 | 1.9 | 2.2 | 2.6 | 1.2 | 0.9 | 1.6 | 1.8 | 0.9 | 1.7 | 1.2 | 1.1 | 2.4 | 1.4 | 3.2 | 3.29 |
| YLR142W | 4.4 | 2.7 | 1.1 | 6.1 | 1.3 | 3.1 | 1.2 | 2.1 | 3.1 | 4.4 | 3.8 | 0.8 | 1.2 | 2.1 | 1.6 | 3.4 | 1.9 | 3.2 | 0.28 |
| YML110C | 1.1 | 0.8 | 1.9 | 1.6 | 0.8 | 2.6 | 2.0 | 1.4 | 2.7 | 2.2 | 1.7 | 1.1 | 1.8 | 1.4 | 1.3 | 2.3 | 1.1 | 1.6 | 2.01 |
| YMR272C | 0.7 | 1.0 | 1.6 | 0.7 | 1.1 | 2.6 | 1.0 | 0.8 | 0.4 | 0.6 | 0.8 | 1.2 | 1.2 | 0.8 | 1.1 | 1.5 | 1.3 | 1.4 | 1.66 |
| YNL130C | 0.6 | 0.7 | 2.4 | 0.7 | 0.8 | 2.4 | 1.3 | 0.8 | 1.7 | 2.4 | 1.5 | 0.9 | 1.9 | 1.0 | 2.5 | 0.7 | 0.7 | 1.2 | 1.18 |
| YPR006C | 1.9 | 1.5 | 0.5 | 1.9 | 2.0 | 2.4 | 2.8 | 1.5 | 1.5 | 3.9 | 2.4 | 1.1 | 1.8 | 1.2 | 0.7 | 1.6 | 1.8 | 2.1 | 0.46 |
| YBR050C | 2.4 | 1.9 | 1.9 | 3.1 | 1.0 | 4.1 | 2.6 | 1.7 | 5.8 | 3.2 | 1.1 | 1.0 | 1.3 | 1.3 | 0.5 | 1.7 | 1.5 | 1.8 | 0.41 |
| YBR145W | 1.5 | 0.7 | 2.8 | 0.9 | 1.1 | 11.5 | 58.8 | 1.0 | 0.1 | 1.1 | 1.1 | 1.0 | 2.0 | 2.2 | 1.2 | 3.6 | 1.7 | 2.0 | 2.17 |
| YBR299W | 1.8 | 0.9 | 1.1 | 3.5 | 1.6 | 0.8 | 3.6 | 2.2 | 1.1 | 5.3 | 2.4 | 1.2 | 0.7 | 1.4 | 0.6 | 3.9 | 1.0 | 1.1 | 0.32 |
| YEL020C | 1.0 | 1.5 | 0.8 | 2.9 | 1.5 | 1.3 | 2.4 | 1.2 | 1.4 | 1.1 | 1.3 | 0.8 | 1.2 | 1.0 | 1.4 | 2.1 | 1.2 | 1.3 | 0.31 |
| YGL039W | 0.8 | 1.4 | 2.1 | 1.3 | 1.3 | 1.7 | 4.2 | 1.3 | 0.7 | 2.8 | 1.1 | 1.3 | 1.5 | 0.9 | 1.5 | 1.5 | 0.6 | 1.0 | 1.09 |
| YGL134W | 0.9 | 1.3 | 0.5 | 0.8 | 1.4 | 1.2 | 2.3 | 1.4 | 1.1 | 1.4 | 1.1 | 0.9 | 0.7 | 1.1 | 0.5 | 1.7 | 0.9 | 1.3 | 0.53 |
| YJR159W | 1.4 | 2.3 | 1.3 | 1.7 | 0.9 | 1.4 | 2.3 | 2.7 | 5.2 | 2.8 | 2.2 | 1.5 | 2.1 | 2.0 | 2.3 | 5.2 | 1.7 | 2.3 | 0.30 |
| YOL157C | 1.0 | 1.1 | 1.3 | 2.5 | 1.4 | 0.9 | 2.7 | 1.4 | 2.3 | 4.8 | 1.2 | 1.2 | 1.4 | 1.2 | 1.1 | 3.5 | 1.4 | 1.3 | 0.41 |
| YOR344C | 1.1 | 1.1 | 0.4 | 0.7 | 1.9 | 2.0 | 2.4 | 1.4 | 0.3 | 0.9 | 0.6 | 1.3 | 1.8 | 0.6 | 0.7 | 0.6 | 1.3 | 1.3 | 2.11 |
| YPL265W | 0.7 | 0.4 | 2.3 | 1.1 | 1.0 | 2.3 | 8.9 | 2.3 | 0.2 | 1.2 | 1.8 | 0.9 | 1.1 | 1.1 | 1.2 | 2.0 | 0.5 | 0.4 | 1.42 |
| YBR126C | 0.8 | 1.9 | 5.6 | 1.2 | 0.7 | 2.9 | 2.3 | 0.6 | 1.7 | | 1.1 | 1.3 | 2.1 | 0.7 | 1.0 | 1.7 | 1.5 | 1.3 | 1.96 |
| YCR005C | 1.1 | 1.9 | 2.0 | 1.2 | 0.9 | 1.6 | 4.4 | 1.2 | 1.5 | 1.5 | 2.1 | 0.5 | 0.7 | 0.8 | 0.7 | 0.7 | 1.6 | 1.7 | 2.38 |
| YDR452W | 1.1 | 1.1 | 1.3 | 0.8 | 1.2 | 2.0 | 1.9 | 1.0 | 1.4 | 1.3 | 1.5 | 0.9 | 1.4 | 1.2 | 1.3 | 1.3 | 1.9 | 2.2 | 1.54 |
| YGR019W | 1.2 | 0.8 | 1.4 | 1.7 | 0.8 | 2.4 | 2.4 | 1.2 | 2.9 | | 1.1 | 1.2 | 2.1 | 1.3 | 2.2 | 2.4 | 1.3 | 2.0 | 0.79 |
| YGR255C | 0.9 | 1.4 | 1.4 | 1.5 | 1.3 | 3.2 | 1.8 | 1.0 | 2.5 | 1.8 | 2.0 | 1.3 | 2.5 | 0.9 | 1.0 | 1.1 | 1.1 | 1.1 | 0.81 |
| YIL098C | 1.4 | 1.6 | 0.5 | 2.0 | 1.7 | 1.7 | 2.2 | 1.6 | 1.9 | 1.9 | 1.4 | 0.8 | 1.3 | 1.9 | 0.7 | 2.0 | 1.2 | 1.6 | 0.75 |
| YIL172C | 1.1 | 1.1 | 1.6 | 1.7 | | 1.3 | 2.5 | 1.6 | 2.8 | 7.1 | 1.0 | 1.4 | 2.0 | 1.3 | 1.4 | 2.8 | 1.1 | 1.2 | 0.42 |
| YLR100W | 0.8 | 1.4 | 1.3 | 1.9 | 1.8 | 1.8 | 2.3 | 1.0 | 0.9 | 1.0 | 0.9 | 1.1 | 1.6 | 1.1 | 0.6 | 1.9 | 1.5 | 2.1 | 1.88 |
| YOR221C | 0.8 | 1.0 | 0.9 | 1.1 | 1.7 | 0.8 | 2.1 | 0.9 | 1.4 | 1.7 | 1.1 | 0.8 | 1.0 | 1.1 | 1.0 | 1.4 | 0.9 | 1.2 | 0.39 |
| YPL123C | 1.2 | 0.8 | 1.8 | 3.0 | 0.8 | 1.9 | 2.7 | 1.2 | 4.2 | 2.3 | 1.9 | 1.0 | 2.2 | 1.6 | 1.4 | 2.6 | 1.6 | 3.2 | 0.71 |
| YBR093C | 2.9 | 1.2 | 1.7 | 1.3 | 1.1 | 0.6 | 0.3 | 1.7 | 0.1 | 0.5 | 3.1 | 2.0 | 0.2 | 1.3 | 2.7 | 0.4 | 0.8 | 1.9 | 3.33 |
| YBR196C | 0.8 | 0.6 | 3.9 | 1.4 | 0.8 | 0.8 | 1.4 | 1.1 | 0.3 | 0.9 | 2.3 | 1.0 | 1.4 | 0.5 | 1.9 | 1.1 | 0.8 | 1.0 | 6.60 |

| | | | | | | | | | | | | | | | | | | | |
|---|---|---|---|---|---|---|---|---|---|---|---|---|---|---|---|---|---|---|---|
| YER023W | 0.8 | 0.6 | 1.2 | 1.2 | 0.8 | 1.3 | 0.7 | 0.9 | 1.1 | 0.6 | 2.1 | 0.8 | 1.2 | 0.7 | 1.2 | 1.6 | 1.5 | 1.4 | 1.77 |
| YFL055W | 2.0 | 6.7 | 1.3 | 2.5 | 1.5 | 1.1 | 1.4 | 1.3 | 23.9 | 6.5 | 2.7 | 0.9 | 1.3 | 1.5 | 0.7 | 1.4 | 1.4 | 1.0 | 0.23 |
| YIL124W | 0.9 | 0.8 | 2.8 | 1.6 | 0.9 | 1.2 | 1.3 | 0.9 | 1.0 | 0.7 | 2.8 | 1.1 | 0.9 | 1.1 | 0.6 | 3.1 | 1.2 | 2.1 | 2.39 |
| YMR318C | 1.4 | 2.4 | 2.2 | 0.7 | 1.2 | 2.1 | 0.8 | 3.6 | 2.3 | 4.8 | 3.6 | 0.8 | 1.8 | 1.7 | 1.5 | 1.1 | 1.1 | 1.1 | 3.17 |
| YBR067C | 1.6 | 2.5 | 2.8 | 0.9 | 1.1 | 1.1 | 1.1 | 1.1 | 1.7 | 1.0 | 3.1 | 1.0 | 0.9 | 0.7 | 3.3 | 2.5 | 0.5 | 0.4 | 1.76 |
| YBR115C | 0.6 | 0.7 | 1.4 | 0.4 | 0.7 | 0.9 | 1.1 | 1.3 | 0.8 | 1.6 | 1.8 | 0.9 | 2.3 | 0.7 | 1.1 | 0.5 | 0.6 | 0.6 | 0.64 |
| YDL131W | 1.1 | 0.9 | 1.8 | 1.0 | 1.0 | 0.9 | 0.7 | 2.2 | 0.8 | 2.7 | 2.2 | 1.2 | 1.0 | 0.9 | 1.1 | 0.9 | 0.7 | 0.6 | 1.81 |
| YDL168W | 2.3 | 2.0 | 2.1 | 0.9 | 1.2 | 1.4 | 1.1 | 1.7 | 8.2 | 4.7 | 1.9 | 0.6 | 1.3 | 1.6 | 1.2 | 1.1 | 0.9 | 0.8 | 1.08 |
| YDR216W | 1.3 | 1.1 | 2.0 | 2.6 | 0.8 | 1.3 | 1.0 | 1.0 | 2.5 | 2.7 | 2.1 | 1.0 | 2.4 | 0.9 | 2.5 | 1.6 | 1.0 | 1.8 | 0.44 |
| YDR253C | 5.7 | 6.4 | 1.4 | 3.2 | 0.9 | 0.8 | 1.0 | 1.7 | 14.8 | 6.3 | 2.3 | 1.0 | 2.2 | 3.6 | 1.2 | 1.6 | 1.0 | 1.0 | 0.38 |
| YDR513W | 2.2 | 2.5 | 2.3 | 2.6 | 0.9 | 2.1 | 1.6 | 1.6 | 4.6 | 3.1 | 2.0 | 0.9 | 1.8 | 2.0 | 1.3 | 3.8 | 1.3 | 3.2 | 3.10 |
| YER061C | 0.9 | 0.9 | 1.2 | 2.5 | 0.8 | 0.4 | 0.9 | 0.7 | 0.3 | 0.7 | 2.2 | 0.7 | 0.9 | 1.0 | 0.8 | 1.8 | 1.2 | 1.2 | 0.84 |
| YFL052W | 1.3 | 0.8 | 0.7 | 2.3 | 1.5 | 2.1 | 1.4 | 1.3 | 0.6 | 1.2 | 2.3 | 0.8 | 1.0 | 1.6 | 0.4 | 1.0 | 1.1 | 0.9 | 0.32 |
| YFL058W | 1.2 | 1.7 | 1.1 | 1.4 | 1.1 | 2.0 | 0.9 | 1.7 | 3.2 | 2.4 | 2.4 | 1.2 | 1.2 | 1.0 | 1.7 | 0.7 | 1.1 | 0.9 | 0.40 |
| YFR030W | 3.1 | 5.5 | 8.5 | 0.5 | 0.8 | 1.8 | 1.1 | 1.7 | 24.1 | 9.0 | 2.4 | 2.4 | 4.1 | 1.3 | 1.7 | 1.6 | 1.3 | 0.9 | 0.30 |
| YGL202W | 0.8 | 0.7 | 2.2 | 0.7 | 0.7 | 0.7 | 0.6 | 1.0 | 1.3 | 1.7 | 2.4 | 0.9 | 1.1 | 0.8 | 2.2 | 0.6 | 1.3 | 0.9 | 1.70 |
| YGR070W | 1.1 | 1.0 | 2.0 | 1.5 | 1.4 | | 1.4 | 1.8 | 1.1 | 1.5 | 1.8 | 0.6 | 1.4 | 1.0 | 2.6 | 2.3 | 1.2 | 1.3 | 0.40 |
| YHL036W | 2.2 | 4.8 | 3.4 | 2.0 | 1.3 | 1.3 | 1.0 | 1.2 | 9.0 | 4.4 | 2.3 | 1.2 | 1.5 | 1.2 | 0.9 | 1.1 | 1.0 | 1.0 | 0.60 |
| YHR104W | 1.0 | 4.1 | 15.7 | 1.9 | 1.1 | 1.2 | 1.5 | 1.1 | 4.8 | 2.3 | 2.6 | 1.0 | 1.2 | 1.5 | 1.6 | 1.3 | 1.9 | 1.9 | 1.57 |
| YJL045W | 1.8 | 2.2 | 1.6 | 5.3 | 0.7 | 0.6 | 0.7 | 0.9 | 9.7 | 1.6 | 2.3 | 1.1 | 1.6 | 1.2 | 3.4 | 2.4 | 0.9 | 0.9 | 0.42 |
| YJL060W | 1.8 | 2.6 | 9.6 | 1.0 | 1.4 | 1.3 | 1.3 | 0.8 | 5.3 | 2.8 | 1.6 | 1.5 | 2.1 | 1.7 | 1.4 | 1.5 | 1.2 | 1.1 | 0.95 |
| YJL155C | 2.1 | 5.0 | 0.8 | 2.4 | 0.8 | 0.8 | 2.0 | 1.5 | 3.8 | 2.8 | 2.4 | 1.0 | 6.6 | 1.2 | 1.2 | 2.3 | 1.8 | 3.1 | 0.60 |
| YJR109C | 0.8 | 0.7 | 1.4 | 0.9 | 0.7 | 0.9 | 0.8 | 1.0 | 1.8 | 4.3 | 1.5 | 1.4 | 3.1 | 0.9 | 1.3 | 0.5 | 1.1 | 0.6 | 0.84 |
| YJR156C | 1.6 | 3.2 | 2.4 | 1.8 | 0.9 | 0.9 | 1.3 | 1.5 | 3.4 | 2.8 | 3.0 | 1.1 | 1.6 | 2.4 | 3.0 | 2.3 | 0.8 | 1.1 | 0.24 |
| YLR092W | 5.0 | 7.5 | 1.4 | 0.6 | 1.3 | 1.0 | 1.3 | 1.5 | 12.7 | 5.8 | 3.1 | 1.0 | 1.8 | 1.4 | 0.9 | 1.2 | 1.0 | 1.1 | 0.24 |
| YMR081C | 3.5 | 1.4 | 2.0 | 5.8 | 1.7 | 1.1 | 0.9 | 1.7 | 0.3 | 2.2 | 2.5 | 1.1 | 0.7 | 1.8 | 0.2 | 3.0 | 1.9 | 3.0 | 0.62 |
| YMR250W | 0.9 | 5.1 | 5.6 | 1.3 | 0.6 | 4.5 | 3.0 | 0.9 | 6.1 | 2.4 | 4.3 | 1.2 | 2.6 | 1.1 | 2.0 | 5.0 | 1.2 | 3.0 | 0.84 |
| YNL277W | 5.7 | 10.3 | 5.3 | 0.6 | 1.1 | 1.7 | 1.4 | 2.2 | 55.0 | 10.9 | 3.2 | 1.3 | 1.8 | 1.5 | 0.6 | 1.3 | 0.9 | 0.7 | 0.27 |
| YOR184W | 1.1 | 0.9 | 2.1 | 2.1 | 1.0 | 0.6 | 1.2 | 0.7 | 0.9 | 0.7 | 2.7 | 0.7 | 0.6 | 0.7 | 1.0 | 0.9 | 1.2 | 1.2 | 3.19 |
| YPR160W | 1.4 | 3.8 | 3.6 | 3.3 | 0.7 | 4.5 | 1.8 | 0.9 | 0.9 | 1.3 | 4.4 | 2.1 | 2.2 | 1.1 | 1.4 | 5.3 | 1.0 | 2.9 | 1.42 |
| YDL182W | 0.8 | 0.7 | 1.3 | 0.8 | 1.3 | 1.0 | 1.0 | 2.9 | 0.8 | 2.4 | 2.0 | 1.4 | 1.5 | 0.8 | 0.7 | 1.1 | 0.6 | 0.6 | 2.31 |

| | | | | | | | | | | | | | | | | | | | |
|---|---|---|---|---|---|---|---|---|---|---|---|---|---|---|---|---|---|---|---|
| YBR291C | 2.0 | 0.9 | 1.0 | 1.5 | 0.9 | 1.1 | 0.9 | 2.2 | 0.9 | 1.0 | 1.1 | 0.9 | 1.0 | 1.7 | 0.5 | 1.5 | 0.9 | 1.3 | 1.19 |
| YIL094C | 1.5 | 0.4 | 0.8 | 0.7 | 1.4 | 1.2 | 1.3 | 3.6 | 0.3 | 0.4 | 1.8 | 1.0 | 0.7 | 1.0 | 0.5 | 0.9 | 0.9 | 1.0 | 2.26 |
| YNR050C | 1.7 | 0.5 | 1.2 | 0.6 | 1.3 | 0.9 | 1.0 | 2.2 | 0.4 | 0.7 | 1.5 | 1.0 | 1.1 | 0.6 | 0.3 | 1.4 | 0.6 | 0.7 | 2.13 |
| YDL244W | 1.1 | 3.5 | 1.8 | 1.6 | 0.9 | 1.3 | 1.7 | 1.8 | 2.5 | 3.7 | 1.2 | 1.2 | 2.0 | 2.0 | 2.9 | 1.5 | 1.1 | 1.0 | 0.25 |
| YDR054C | 1.3 | 1.0 | 0.7 | 1.4 | 0.7 | 1.0 | 1.1 | 1.5 | 2.9 | 3.2 | 1.9 | 1.0 | 2.5 | 1.1 | 1.6 | 1.4 | 0.8 | 1.4 | 1.18 |
| YDR353W | 0.9 | 1.6 | 3.4 | 1.6 | 0.6 | 0.9 | 1.0 | 1.1 | 2.0 | 5.1 | 1.4 | 0.8 | 0.7 | 1.6 | 1.3 | 1.0 | 0.9 | 1.3 | 3.20 |
| YEL070W | 1.4 | 0.9 | 1.6 | 3.4 | 1.3 | 1.2 | 1.3 | 1.2 | 1.9 | 13.0 | 1.1 | 1.1 | 1.1 | 2.1 | 3.2 | 1.9 | 0.9 | 1.0 | 0.27 |
| YIL168W | 2.8 | 1.4 | 1.0 | 1.2 | 1.4 | 0.7 | 1.3 | 1.6 | 7.8 | 19.3 | 1.9 | 0.8 | 0.9 | 1.5 | 1.2 | 1.2 | 1.0 | 1.1 | 0.39 |
| YJL221C | 1.1 | 1.0 | 1.1 | 1.3 | 0.9 | 6.6 | 2.5 | 1.8 | 2.7 | 4.4 | 0.8 | 1.1 | 1.4 | 1.1 | 1.1 | 3.3 | 1.1 | 1.4 | 0.41 |
| YJR095W | 1.2 | 20.5 | 1.9 | 6.7 | 1.2 | 1.5 | 2.0 | 0.9 | 0.5 | 6.3 | 0.6 | 0.7 | 0.8 | 1.3 | 0.8 | 0.8 | 1.3 | 0.9 | 0.23 |
| YKL085W | 1.4 | 2.3 | 1.6 | 1.2 | 1.2 | 1.9 | 1.5 | 1.2 | 1.9 | 3.0 | 1.8 | 0.8 | 1.5 | 1.0 | 0.5 | 1.7 | 0.9 | 1.3 | 2.16 |
| YKL188C | 1.3 | 0.7 | 1.9 | 2.4 | 1.0 | 0.8 | 1.1 | 0.7 | 2.5 | 2.8 | 1.1 | 1.2 | 1.2 | 2.1 | 1.4 | 2.7 | 1.1 | 1.5 | 0.27 |
| YKL217W | 1.8 | 2.4 | 1.0 | 2.1 | 1.1 | 1.2 | 1.6 | 1.1 | 0.9 | 4.1 | 1.6 | 0.8 | 1.2 | 1.2 | 2.2 | 3.0 | 1.7 | 3.3 | 0.29 |
| YKR061W | 2.0 | 0.9 | 0.5 | 1.9 | 1.2 | 1.2 | 1.4 | 1.5 | 1.5 | 2.6 | 1.1 | 0.9 | 0.9 | 1.6 | 0.9 | 0.8 | 1.5 | 1.6 | 0.70 |
| YLR174W | 1.2 | 1.5 | 1.7 | 2.1 | 0.9 | 0.9 | 1.6 | 1.1 | 2.5 | 8.3 | 1.3 | 1.3 | 1.8 | 1.7 | 0.8 | 4.6 | 0.9 | 1.2 | 0.41 |
| YLR260W | 1.1 | 1.5 | 1.5 | 2.4 | 1.2 | 1.0 | 1.2 | 1.2 | 2.6 | 3.2 | 0.6 | 0.8 | 1.9 | 1.2 | 1.0 | 1.0 | 1.1 | 1.2 | 0.44 |
| YNL009W | 1.1 | 1.9 | 2.0 | 1.4 | 1.4 | 1.1 | 1.2 | 1.1 | 1.4 | 3.3 | 1.3 | 0.9 | 1.3 | 1.3 | 2.7 | 3.3 | 1.2 | 2.3 | 0.45 |
| YNL117W | 0.9 | 4.7 | 1.7 | 0.8 | 0.8 | | 1.1 | 1.7 | 12.8 | 4.4 | 1.4 | 0.7 | 1.3 | 2.0 | 2.6 | 1.6 | 1.1 | 0.9 | 0.24 |
| YNL183C | 0.9 | 3.5 | 6.4 | 1.3 | 0.7 | 1.2 | 1.3 | 0.9 | 4.8 | 4.3 | 1.3 | 1.1 | 2.3 | 1.1 | 1.1 | 1.2 | 0.8 | 1.0 | 0.47 |
| YNR073C | 1.1 | 1.4 | 0.8 | 2.7 | 1.4 | 1.7 | 2.2 | 1.5 | 2.5 | 16.8 | 2.2 | 1.1 | 1.2 | 2.3 | 1.7 | 1.6 | 1.0 | 0.9 | 0.18 |
| YPL161C | 1.0 | 1.7 | 1.1 | 1.1 | 0.8 | 0.7 | 1.3 | 1.8 | 1.2 | 2.7 | 1.0 | 1.0 | 1.2 | 1.1 | 0.8 | 1.4 | 1.3 | 1.3 | 0.41 |
| YAL061W | 1.7 | 2.4 | 3.3 | 3.8 | 1.0 | 1.0 | 2.0 | 0.8 | 5.5 | 1.4 | 4.1 | 1.1 | 1.4 | 0.7 | 0.6 | 1.1 | 1.4 | 1.2 | 0.88 |
| YAL067C | 2.7 | 7.5 | 1.5 | 1.4 | 0.7 | 1.2 | 0.8 | 1.1 | 7.6 | 2.9 | 1.2 | 1.0 | 1.1 | 1.3 | 2.4 | 1.6 | 1.0 | 1.0 | 0.33 |
| YBL033C | 1.3 | 1.9 | 2.4 | 1.0 | 1.1 | 0.4 | 0.9 | 1.4 | 6.6 | 4.2 | 1.1 | 0.9 | 1.8 | 1.4 | 1.0 | 1.2 | 1.2 | 1.0 | 0.53 |
| YBL086C | 0.8 | 0.5 | 1.0 | 0.6 | 1.3 | 0.7 | 1.0 | 1.1 | 3.3 | 1.4 | 1.3 | 0.5 | 0.9 | 0.8 | 0.5 | 1.4 | 0.9 | 1.0 | 0.43 |
| YBR117C | 0.8 | 1.6 | 1.5 | 1.4 | 0.9 | 2.3 | 1.0 | 0.7 | 5.9 | 1.5 | 0.4 | 0.8 | 2.1 | 0.9 | 1.0 | 12.0 | 0.7 | 0.7 | 0.49 |
| YBR213W | 1.0 | 1.6 | 1.0 | 0.5 | 1.1 | 2.4 | 1.3 | 1.3 | 11.4 | 2.3 | 0.5 | 1.2 | 1.4 | 1.3 | 1.7 | 0.8 | 0.9 | 0.9 | 0.23 |
| YCR036W | 1.3 | 1.2 | 1.3 | 1.3 | 1.5 | 1.4 | 1.5 | 1.1 | 2.6 | 1.5 | 1.0 | 1.3 | 1.8 | 1.5 | 1.7 | 1.6 | 1.6 | 1.7 | 1.35 |
| YDL132W | 0.8 | 1.2 | 1.1 | 0.7 | 1.3 | 0.9 | 1.4 | 1.0 | 3.8 | 2.4 | 1.3 | 0.7 | 2.0 | 0.9 | 1.7 | 1.0 | 0.9 | 1.1 | 0.74 |
| YER014W | 1.0 | 0.9 | 0.9 | 0.6 | 0.8 | 4.0 | | 1.2 | 3.5 | 1.1 | 0.9 | 1.1 | 1.5 | 1.2 | 2.3 | 0.8 | 1.0 | 0.9 | 0.43 |
| YER042W | 3.0 | 2.2 | 3.1 | 1.0 | 1.1 | 1.6 | 1.1 | 1.7 | 6.8 | 2.1 | 1.8 | 1.1 | 1.4 | 5.2 | 1.0 | 1.9 | 0.7 | 1.1 | 1.80 |

| | | | | | | | | | | | | | | | | | | | |
|---|---|---|---|---|---|---|---|---|---|---|---|---|---|---|---|---|---|---|---|
| YER090W | 1.0 | 1.1 | 2.0 | 0.7 | 1.3 | 0.7 | 0.9 | 1.4 | 3.7 | 2.5 | 1.8 | 1.3 | 1.2 | 1.2 | 1.5 | 0.8 | 1.1 | 0.9 | 0.97 |
| YGL026C | 0.7 | 9.3 | 1.9 | 0.6 | 1.2 | 1.7 | 0.9 | 0.8 | 3.7 | 1.5 | 3.4 | 1.3 | 1.8 | 0.9 | 2.4 | 0.7 | 1.5 | 0.8 | 0.87 |
| YGL252C | 1.1 | 2.4 | 1.4 | 0.7 | 1.3 | 0.8 | 1.4 | 1.2 | 2.7 | 1.7 | 0.9 | 0.9 | 1.2 | 1.0 | 1.3 | 1.2 | 1.0 | 1.3 | 0.91 |
| YGL254W | 1.2 | 1.1 | 0.8 | 0.6 | 1.1 | 0.7 | 1.3 | 1.4 | 3.1 | 1.8 | 1.1 | 1.2 | 2.4 | 1.3 | 1.1 | 1.4 | 0.8 | 1.2 | 0.53 |
| YGR276C | 0.9 | 0.8 | 0.3 | 1.3 | 1.5 | 0.7 | 1.9 | 1.5 | 4.5 | 2.2 | 0.9 | 1.0 | 1.8 | 1.0 | 0.8 | 1.3 | 0.9 | 1.1 | 0.97 |
| YHR106W | 0.9 | 1.7 | 1.7 | 1.5 | 1.7 | 1.3 | 1.2 | 1.0 | 2.8 | 2.5 | 1.4 | 0.7 | 1.0 | 1.2 | 0.9 | 2.4 | 1.3 | 1.1 | 1.73 |
| YIL046W | 1.5 | 2.7 | 1.6 | 0.7 | 1.4 | 0.9 | 1.0 | 1.0 | 10.7 | 2.5 | 1.2 | 1.0 | 1.7 | 1.1 | 1.3 | 1.8 | 9.0 | 5.8 | 0.68 |
| YJL071W | 1.0 | 1.5 | 1.3 | 1.6 | 1.4 | 1.0 | 1.3 | 1.1 | 2.8 | 2.2 | 1.3 | 1.1 | 1.0 | 1.0 | 1.5 | 1.2 | 0.9 | 0.9 | 0.41 |
| YJR139C | 1.4 | 2.6 | 2.9 | 1.1 | 1.2 | 1.8 | 1.3 | 1.1 | 4.1 | 1.9 | 1.5 | 1.0 | 1.5 | 1.4 | 0.7 | 1.2 | 1.0 | 1.3 | 4.47 |
| YKR069W | 3.4 | 6.3 | 5.0 | 3.1 | 0.7 | 0.5 | 1.0 | 1.2 | 10.9 | 9.0 | 2.0 | 1.4 | 1.4 | 1.7 | 1.1 | 1.8 | 1.0 | 1.0 | 0.25 |
| YLL061W | 3.6 | 2.8 | 9.2 | 0.4 | 0.8 | 0.8 | 0.9 | 1.4 | 3.7 | 5.5 | 1.7 | 0.7 | 0.9 | 1.3 | 1.4 | 1.0 | 0.6 | 0.7 | 0.33 |
| YLR070C | 1.0 | 1.3 | 1.4 | 1.1 | 0.7 | 1.1 | 1.3 | 1.0 | 3.5 | 1.6 | 0.4 | 0.7 | 2.0 | 1.3 | 0.5 | 2.1 | 1.0 | 0.9 | 0.31 |
| YLR099C | 2.0 | 2.2 | 1.4 | 0.8 | 0.9 | 0.4 | 0.2 | 1.2 | 3.6 | 1.1 | 1.2 | 0.9 | 0.8 | 0.7 | 0.9 | 0.7 | 0.7 | 0.5 | 0.92 |
| YLR157C | 1.1 | 1.1 | 3.4 | 1.1 | 1.3 | 1.9 | 1.9 | 1.5 | 5.1 | 1.8 | 1.9 | 1.2 | 1.8 | 1.1 | 1.1 | 2.1 | 1.6 | 2.1 | 1.70 |
| YLR160C | 1.2 | 1.4 | 3.9 | 1.1 | 1.3 | 1.8 | 1.9 | 2.0 | 4.8 | 1.8 | 1.6 | 1.2 | 2.0 | 1.2 | 1.0 | 2.0 | 1.6 | 2.2 | 1.59 |
| YLR164W | 1.0 | 2.4 | 0.3 | 1.0 | 1.2 | 1.2 | 1.1 | 1.2 | 5.3 | 2.0 | 3.2 | 0.7 | 1.0 | 1.5 | 0.7 | 11.0 | 1.5 | 1.2 | 0.31 |
| YML042W | 0.9 | 1.4 | 1.5 | 2.2 | 1.0 | 0.9 | 0.8 | 1.6 | 3.8 | 2.4 | 2.5 | 1.0 | 1.2 | 1.4 | 1.7 | 4.8 | 1.0 | 0.9 | 0.29 |
| YOL049W | 0.9 | 1.1 | 1.3 | 0.9 | 1.4 | 0.6 | 1.1 | 1.0 | 3.0 | 1.9 | 1.8 | 0.8 | 0.9 | 0.9 | 0.7 | 1.3 | 1.0 | 1.3 | 1.61 |
| YOL064C | 1.4 | 2.1 | 1.1 | 0.6 | 0.9 | 1.4 | 1.2 | 1.0 | 8.7 | 2.7 | 1.0 | 0.9 | 2.0 | 0.9 | 1.0 | 1.7 | 1.2 | 1.3 | 1.32 |
| YOR377W | 0.6 | 0.6 | 0.6 | 1.0 | 1.4 | 1.6 | 1.6 | 1.0 | 3.8 | 1.1 | 1.2 | 0.9 | 1.3 | 0.9 | 1.2 | 1.3 | 1.1 | 1.1 | 0.46 |
| YPL031C | 0.9 | 2.0 | 1.4 | 0.8 | 1.5 | 1.8 | 1.6 | 1.0 | 5.7 | 1.4 | 1.4 | 0.8 | 1.6 | 0.9 | 0.7 | 2.0 | 1.4 | 1.2 | 0.49 |
| YPL113C | 1.4 | 1.7 | 0.4 | 2.4 | 1.3 | 1.0 | 1.9 | 1.4 | 5.5 | 2.0 | 1.5 | 1.1 | 1.7 | 1.6 | 0.9 | 2.5 | 1.2 | 1.7 | 0.28 |
| YPL274W | 0.8 | 4.2 | 3.8 | 0.8 | 0.6 | 1.1 | 0.7 | 0.9 | 4.1 | 3.6 | 2.0 | 0.5 | 0.6 | 0.9 | 1.5 | 1.1 | 0.7 | 0.6 | 0.41 |
| YPR048W | 1.3 | 2.0 | 0.9 | 1.1 | 0.9 | 0.6 | 0.9 | 1.5 | 4.0 | 2.1 | 1.0 | 0.8 | 0.6 | 1.2 | 0.6 | 0.8 | 0.7 | 0.8 | 0.75 |
| YBR001C | 0.9 | 1.4 | 1.1 | 1.4 | 0.9 | 1.1 | 0.9 | 1.3 | 2.3 | 2.1 | 1.0 | 1.0 | 2.0 | 1.2 | 1.8 | 1.4 | 1.0 | 1.6 | 0.61 |
| YBR018C | 0.9 | 1.1 | 1.9 | 2.9 | 0.9 | 0.7 | 1.4 | 0.6 | 1.3 | 1.7 | 5.8 | 1.0 | 1.0 | 2.4 | 1.3 | 6.2 | 0.8 | 0.9 | 0.20 |
| YBR204C | 1.0 | 1.0 | 1.5 | 1.8 | 1.1 | 0.9 | 1.7 | 1.1 | 1.8 | 1.9 | 2.2 | 1.0 | 1.7 | 1.4 | 1.2 | 2.3 | 1.1 | 1.5 | 0.84 |
| YBR241C | 0.9 | 9.3 | 2.6 | 0.9 | 0.9 | 1.5 | 1.4 | 0.7 | 29.8 | 2.2 | 1.4 | 1.4 | 1.4 | 0.9 | 1.6 | 0.7 | 1.0 | 1.2 | 1.25 |
| YCR105W | 2.2 | 1.2 | 1.2 | 3.0 | 0.9 | 1.0 | 1.0 | 1.4 | 2.0 | 3.9 | 2.3 | 0.9 | 1.9 | 1.9 | 3.1 | 1.3 | 1.3 | 1.1 | 0.36 |
| YDR287W | 1.1 | 8.4 | 1.9 | 1.2 | 1.2 | 1.6 | 1.2 | 1.2 | 2.5 | 1.8 | 1.5 | 1.1 | 1.5 | 1.2 | 1.6 | 2.7 | 1.5 | 1.3 | 0.52 |
| YDR294C | 0.7 | 1.1 | 1.9 | 1.2 | 0.9 | 0.7 | 1.0 | 1.0 | 2.0 | 2.9 | 1.2 | 1.1 | 1.6 | 1.0 | 2.0 | 1.7 | 0.9 | 1.1 | 1.02 |

| | | | | | | | | | | | | | | | | | | |
|---|---|---|---|---|---|---|---|---|---|---|---|---|---|---|---|---|---|---|
| YER052C | 1.1 | 1.0 | 3.6 | 1.1 | 0.6 | 0.6 | 0.5 | 1.0 | 2.7 | 2.4 | 1.1 | 0.9 | 2.1 | 0.7 | 1.2 | 0.2 | 0.8 | 0.4 | 1.54 |
| YFL021W | 0.8 | 1.4 | 2.4 | 1.7 | 1.1 | 0.8 | 0.8 | 0.6 | 2.7 | 1.2 | 1.3 | 0.9 | 1.5 | 1.1 | 1.3 | 1.0 | 0.8 | 0.5 | 0.40 |
| YGL040C | 0.7 | 1.2 | 1.4 | 0.9 | 1.3 | 2.0 | 1.1 | 1.0 | 2.1 | 1.9 | 0.9 | 0.6 | 1.6 | 1.3 | 0.7 | 1.5 | 0.7 | 1.0 | 1.77 |
| YGL125W | 1.0 | 3.2 | 4.3 | 0.7 | 0.9 | | 1.7 | 0.8 | 2.1 | 2.1 | 1.6 | 1.0 | 1.4 | 1.0 | 1.9 | 1.2 | 1.2 | 1.1 | 0.27 |
| YGR007W | 1.3 | 1.7 | 0.7 | 1.6 | 0.6 | 0.8 | 1.2 | 1.1 | 2.2 | 2.3 | 0.9 | 0.8 | 1.4 | 1.8 | 1.9 | 1.3 | 1.1 | 1.4 | 0.74 |
| YGR155W | 1.3 | 4.4 | 1.2 | 0.6 | 1.3 | 1.6 | 1.1 | 1.4 | 2.4 | 1.7 | 1.3 | 1.0 | 1.4 | 0.7 | 0.5 | 1.0 | 0.5 | 0.6 | 4.15 |
| YIL099W | 0.9 | 1.6 | 1.5 | 0.4 | 0.8 | | 101.4 | 1.0 | 2.2 | 1.8 | 0.9 | 0.9 | 1.4 | 1.6 | 5.5 | 1.2 | 1.0 | 1.1 | 0.18 |
| YIL170W | 1.1 | 1.0 | 2.5 | 2.2 | 0.9 | 8.8 | 0.5 | 1.2 | 5.7 | 3.2 | 1.7 | 0.7 | 1.9 | 1.1 | 2.3 | 1.8 | 1.6 | 1.5 | 0.48 |
| YIR031C | 0.8 | 1.6 | 0.6 | 0.6 | 1.6 | 1.7 | 1.4 | 1.0 | 2.6 | 1.0 | 1.2 | 0.8 | 1.0 | 1.2 | 0.8 | 0.7 | 0.9 | 0.8 | 0.44 |
| YIR032C | 1.1 | 1.9 | 1.3 | 2.6 | 1.0 | 0.6 | 1.0 | 1.2 | 2.6 | 2.3 | 1.4 | 1.0 | 1.4 | 1.6 | 0.6 | 1.6 | 0.9 | 1.1 | 0.40 |
| YJL128C | 0.8 | 1.4 | 0.4 | 0.8 | 1.2 | 1.3 | 1.8 | 0.9 | 2.0 | 1.7 | 0.6 | 1.0 | 1.7 | 0.8 | 1.2 | 0.9 | 1.0 | 1.0 | 0.42 |
| YJR090C | 0.8 | 1.0 | 0.6 | 0.8 | 1.2 | 1.2 | 0.8 | 0.8 | 1.9 | 1.2 | 0.4 | 0.8 | 2.2 | 1.0 | 1.6 | 0.8 | 1.1 | 1.0 | 0.60 |
| YJR103W | 1.1 | 3.8 | 1.2 | 1.6 | 0.7 | 0.6 | 0.6 | 0.8 | 1.8 | 3.0 | 1.1 | 0.6 | 0.7 | 0.7 | 3.2 | 2.3 | 0.9 | 1.0 | 0.74 |
| YJR153W | 1.1 | 1.7 | 1.1 | 0.8 | 0.9 | 0.4 | 1.3 | 0.9 | 2.4 | 1.6 | 2.0 | 0.7 | 1.3 | 1.7 | 2.9 | 1.6 | 0.8 | 1.0 | 0.18 |
| YKL192C | 1.1 | 1.0 | 3.7 | 1.1 | 1.1 | 1.8 | 1.1 | 0.9 | 3.5 | 1.6 | 1.1 | 1.2 | 1.7 | 1.5 | 1.2 | 2.1 | 1.0 | 0.8 | 1.57 |
| YLR025W | 1.2 | 5.1 | 0.7 | 1.1 | 1.9 | 1.5 | 1.7 | 1.5 | 2.4 | 1.5 | 1.1 | 0.9 | 1.0 | 1.7 | 1.5 | 2.2 | 1.2 | 1.3 | 1.15 |
| YML051W | 1.0 | 1.4 | 1.8 | 0.9 | 1.0 | 0.7 | 0.9 | 0.9 | 2.0 | 1.5 | 1.4 | 0.7 | 1.2 | 1.1 | 1.4 | 1.1 | 0.7 | 1.0 | 0.57 |
| YML099C | 0.6 | 1.0 | 1.1 | 1.3 | 1.0 | 0.5 | 1.0 | 0.7 | 2.2 | 1.7 | 1.6 | 1.1 | 1.3 | 0.9 | 1.0 | 1.6 | 0.7 | 0.8 | 0.48 |
| YMR056C | 1.1 | 1.3 | 1.7 | 1.0 | 1.1 | 0.9 | 1.4 | 1.2 | 1.8 | 1.1 | 1.6 | 0.9 | 0.9 | 1.1 | 0.7 | 2.4 | 1.0 | 1.1 | 0.72 |
| YNL257C | 0.7 | 1.0 | 0.9 | 0.8 | 0.9 | 2.0 | 1.1 | 1.1 | 2.1 | 1.4 | 1.0 | 1.1 | 1.7 | 0.8 | 1.0 | 1.4 | 0.9 | 1.3 | 0.63 |
| YNL264C | 1.0 | 2.4 | 1.3 | 1.0 | 0.8 | 1.4 | 1.5 | 1.1 | 3.3 | 1.3 | 1.0 | 1.1 | 1.5 | 1.2 | 1.1 | 1.3 | 0.7 | 1.1 | 0.48 |
| YNR071C | 1.4 | 1.0 | 0.9 | 3.2 | 1.1 | 0.9 | 1.4 | 1.2 | 3.1 | 7.0 | 1.3 | 0.6 | 1.7 | 1.1 | 1.8 | 1.0 | 1.0 | 0.9 | 0.16 |
| YOL065C | 1.0 | 1.7 | 1.6 | 1.3 | 0.8 | 1.3 | 1.9 | 1.0 | 2.1 | 1.8 | 1.0 | 0.8 | 1.3 | 1.1 | 0.7 | 2.6 | 1.1 | 1.5 | 0.43 |
| YOL067C | 0.8 | 0.9 | 0.9 | 1.4 | 0.7 | 0.3 | 0.7 | 1.0 | 2.1 | 1.3 | 1.3 | 0.7 | 1.7 | 0.7 | 1.2 | 1.1 | 1.4 | 0.9 | 0.55 |
| YPL147W | 1.0 | 0.8 | 2.3 | 1.2 | 1.2 | 1.1 | 1.0 | 0.9 | 2.4 | 2.4 | 1.3 | 0.9 | 1.5 | 1.0 | 2.4 | 2.5 | 1.5 | 3.6 | 0.33 |
| YDR043C | 2.8 | 1.1 | 1.2 | 6.9 | 1.7 | 1.4 | 1.3 | 1.2 | 1.0 | 2.7 | 1.3 | 0.9 | 0.7 | 1.8 | 1.2 | 1.4 | 1.9 | 1.2 | 0.66 |
| YGR180C | 3.1 | 1.1 | 1.9 | 1.0 | 2.0 | 0.6 | 0.5 | 1.0 | 1.4 | 1.5 | 1.0 | 0.9 | 0.9 | 0.9 | 1.3 | 1.2 | 1.0 | 1.9 | 3.90 |
| YJL026W | 2.5 | 2.4 | 2.9 | 1.0 | 1.7 | 1.4 | 0.5 | 1.2 | 1.0 | 1.1 | 1.3 | 1.0 | 1.2 | 1.0 | 1.1 | 1.6 | 1.4 | 2.1 | 3.74 |
| YGR087C | 1.0 | 15.0 | 1.7 | 0.6 | 1.0 | 1.1 | 1.0 | 0.8 | 0.2 | 0.9 | 0.8 | 1.2 | 1.2 | 0.7 | 3.5 | 0.6 | 1.0 | 0.6 | 1.88 |
| YGL256W | 0.9 | 5.3 | 1.1 | 1.3 | 0.7 | 0.7 | 0.5 | 1.0 | 0.4 | 1.2 | 1.0 | 0.9 | 0.8 | 0.9 | 1.0 | 0.7 | 0.7 | 0.8 | 0.90 |
| YAL039C | 1.1 | 2.2 | 1.7 | 1.0 | 1.1 | 0.8 | 1.0 | 1.2 | 1.6 | 2.2 | 1.1 | 1.4 | 1.6 | 1.2 | 3.3 | 2.2 | 1.4 | 1.1 | 0.39 |

| | | | | | | | | | | | | | | | | | | | |
|---|---|---|---|---|---|---|---|---|---|---|---|---|---|---|---|---|---|---|---|
| YJR107W | 1.0 | 3.1 | 1.5 | 1.8 | 0.7 | 1.5 | 1.3 | 1.3 | 1.4 | 0.9 | 1.3 | 1.1 | 1.7 | 1.4 | 1.1 | 1.1 | 1.4 | 1.3 | 0.41 |
| YNL142W | 0.7 | 2.4 | 2.5 | 0.8 | 1.3 | 0.9 | 1.1 | 0.8 | 0.9 | 0.6 | 0.9 | 1.2 | 0.9 | 0.8 | 0.3 | 1.0 | 1.0 | 0.9 | 0.44 |
| YDL210W | 1.2 | 2.2 | 2.7 | 1.1 | 0.9 | 1.3 | 0.8 | 1.1 | 1.2 | 1.4 | 0.6 | 0.8 | 1.2 | 1.1 | 1.9 | 0.9 | 1.1 | 0.9 | 0.20 |
| YGL055W | 1.1 | 1.8 | 0.3 | 2.5 | 1.5 | 1.6 | 0.6 | 1.4 | 0.2 | 0.7 | 0.9 | 1.3 | 0.2 | 0.7 | 0.5 | 3.0 | 1.0 | 1.5 | 5.60 |
| YCL025C | 1.1 | 4.6 | 2.5 | 1.7 | 0.9 | 0.9 | 0.7 | 0.7 | 0.3 | 0.3 | 1.0 | 1.1 | 0.5 | 0.7 | 0.8 | 0.6 | 0.6 | 0.6 | 1.98 |
| YBR132C | 0.9 | 1.9 | 2.4 | 1.8 | 0.8 | 1.2 | 1.1 | 0.8 | 0.8 | 1.1 | 1.9 | 1.0 | 1.4 | 1.1 | 1.5 | 1.3 | 1.1 | 1.3 | 0.43 |
| YHL018W | 0.8 | 1.5 | 0.5 | 0.5 | 0.9 | 0.4 | 1.2 | 0.8 | 0.8 | 0.6 | 1.2 | 0.7 | 0.8 | 1.0 | 0.9 | 0.9 | 1.0 | 0.9 | 0.37 |
| YPL038W | 0.8 | 1.5 | 0.2 | 1.1 | 1.4 | 0.5 | 1.0 | 1.2 | 0.9 | 0.7 | 1.1 | 0.6 | 0.6 | 0.9 | 0.6 | 0.8 | 0.9 | 0.9 | 0.64 |
| YKR053C | 0.9 | 2.0 | 1.0 | 1.2 | 1.0 | 1.1 | 1.7 | 0.9 | 0.8 | 1.8 | 0.5 | 0.7 | 0.7 | 1.3 | 0.3 | 0.9 | 2.8 | 2.4 | 0.35 |
| YNL256W | 0.7 | 1.6 | 0.6 | 0.6 | 1.0 | 0.5 | 0.6 | 0.9 | 0.8 | 0.6 | 0.6 | 0.6 | 1.3 | 0.7 | 0.9 | 0.4 | 0.6 | 0.6 | 1.09 |
| YLR377C | 1.1 | 2.6 | 1.8 | 1.2 | 1.0 | 0.7 | 0.9 | 0.7 | 1.8 | 1.6 | 0.6 | 0.6 | 1.2 | 2.9 | 0.8 | 2.7 | 0.9 | 1.4 | 0.14 |
| YBR253W | 1.3 | 4.4 | 0.4 | 1.1 | 1.4 | 1.3 | 1.4 | 1.8 | 1.8 | 1.5 | 1.1 | 1.0 | 0.8 | 1.2 | 0.9 | 1.3 | 1.3 | 1.1 | 0.96 |
| YBL030C | 1.0 | 0.9 | 4.5 | 0.9 | 0.7 | 1.1 | 0.9 | 0.8 | 0.4 | 0.9 | 1.1 | 0.9 | 0.5 | 0.8 | 1.8 | 1.0 | 1.0 | 1.1 | 3.12 |
| YBR221C | 0.8 | 0.7 | 3.2 | 1.5 | 1.3 | 1.7 | 1.5 | 0.8 | 1.0 | 1.3 | 1.2 | 1.3 | 1.7 | 0.8 | 1.1 | 1.5 | 0.9 | 1.0 | 3.62 |
| YDR342C | 2.8 | 1.1 | 12.2 | 5.7 | 1.6 | 1.1 | 0.8 | 1.2 | 0.2 | 2.2 | 2.9 | 1.0 | 0.6 | 0.9 | 0.5 | 2.4 | 1.0 | 2.2 | 5.23 |
| YDR343C | 1.2 | 1.0 | 20.6 | 4.6 | 1.3 | 1.3 | 0.7 | 1.2 | 0.3 | 2.1 | 2.3 | 1.0 | 0.8 | 0.8 | 0.5 | 2.8 | 1.1 | 2.3 | 5.81 |
| YEL034W | 0.9 | 0.6 | 3.3 | 0.9 | 1.4 | 0.9 | 0.4 | 0.9 | 0.8 | 1.0 | 1.1 | 0.9 | 0.8 | 0.9 | 0.8 | 0.9 | 0.9 | 0.9 | 5.44 |
| YHR094C | 0.7 | 1.2 | 5.3 | 1.6 | 1.1 | 1.6 | 0.8 | 1.2 | 0.3 | 1.2 | 0.6 | 0.9 | 0.7 | 0.6 | 2.7 | 0.9 | 1.4 | 1.4 | 4.82 |
| YIL162W | 1.3 | 1.5 | 6.8 | 2.4 | 1.2 | 1.0 | 1.2 | 1.6 | 0.8 | 3.7 | 2.8 | 2.0 | 1.0 | 1.0 | 1.5 | 1.7 | 0.9 | 1.4 | 1.22 |
| YJR105W | 0.6 | 0.7 | 3.0 | 0.8 | 0.7 | 0.3 | 0.5 | 0.8 | 0.9 | 0.5 | 1.0 | 0.8 | 0.8 | 0.5 | 1.2 | 0.7 | 1.1 | 0.9 | 3.75 |
| YLR134W | 0.8 | 0.6 | 2.3 | 0.8 | 1.3 | 2.5 | 1.1 | 0.8 | 0.1 | 0.7 | 1.1 | 1.2 | 1.5 | 0.6 | 1.7 | 0.5 | 0.6 | 0.8 | 3.47 |
| YLR258W | 1.5 | 1.0 | 4.2 | 3.5 | 0.9 | 1.8 | 1.8 | 0.9 | 0.8 | 1.3 | 1.2 | 0.9 | 1.4 | 1.2 | 0.6 | 1.7 | 1.0 | 2.0 | 1.36 |
| YML058W | 1.9 | 1.2 | 5.8 | 1.9 | 0.6 | 0.6 | 0.6 | 0.7 | 1.1 | 1.2 | 3.3 | 0.9 | 1.4 | 1.1 | 2.5 | 1.3 | 1.3 | 1.5 | 2.14 |
| YMR083W | 1.4 | 1.7 | 2.6 | 1.1 | 1.5 | 1.8 | 1.7 | 1.1 | 0.4 | 1.1 | 1.6 | 0.7 | 0.9 | 0.8 | 1.3 | 1.3 | 0.9 | 1.1 | 2.52 |
| YOR178C | 1.4 | 1.3 | 4.8 | 2.3 | 1.0 | 2.7 | 0.9 | 0.9 | 0.2 | 1.7 | 4.1 | 1.7 | 1.0 | 1.0 | 1.1 | 1.7 | 1.0 | 1.5 | 0.56 |
| YPL028W | 0.7 | 0.9 | 2.6 | 0.9 | 1.0 | 1.2 | 1.3 | 0.9 | 1.2 | 0.8 | 1.2 | 1.4 | 0.9 | 0.8 | 0.6 | 2.2 | 1.4 | 1.8 | 4.35 |
| YPR113W | 1.1 | 0.8 | 3.7 | 1.3 | 2.0 | 1.1 | 1.2 | 0.9 | 0.4 | 0.8 | 2.0 | 0.7 | 0.7 | 1.3 | 1.0 | 2.3 | 1.2 | 1.9 | 2.85 |
| YPR183W | 0.9 | 1.5 | 3.5 | 0.8 | 0.9 | 1.2 | 1.2 | 1.0 | 0.9 | 0.6 | 2.5 | 1.2 | 1.6 | 0.7 | 0.9 | 0.8 | 1.2 | 1.0 | 1.17 |
| YBR011C | 1.3 | 1.8 | 2.5 | 1.1 | 0.9 | 2.0 | 1.1 | 1.3 | 2.2 | 1.0 | 1.7 | 1.1 | 1.5 | 0.9 | 2.1 | 1.7 | 0.8 | 1.1 | 3.68 |
| YCR034W | 0.8 | 0.4 | 2.5 | 0.9 | 0.9 | 0.3 | 0.3 | 0.9 | 0.1 | 0.2 | 0.8 | 0.5 | 0.2 | 0.6 | 0.9 | 0.3 | 0.9 | 0.7 | 3.77 |
| YDR050C | 1.6 | 1.3 | 2.3 | 1.8 | 0.9 | 1.7 | 1.5 | 1.3 | 0.4 | 1.3 | 2.0 | 1.4 | 2.0 | 1.2 | 1.9 | 1.3 | 1.1 | 2.3 | 6.26 |

| | | | | | | | | | | | | | | | | | | | |
|---|---|---|---|---|---|---|---|---|---|---|---|---|---|---|---|---|---|---|---|
| YDR178W | 2.0 | 2.0 | 3.4 | 2.2 | 0.9 | 2.1 | 0.6 | 0.9 | 0.9 | 0.8 | 2.0 | 1.3 | 1.5 | 1.5 | 0.7 | 3.0 | 1.2 | 2.3 | 2.27 |
| YDR284C | 0.8 | 1.5 | 3.0 | 2.9 | 0.8 | 1.8 | 1.4 | 0.9 | 0.9 | 1.0 | 1.3 | 0.9 | 1.2 | 1.3 | 0.9 | 1.7 | 1.5 | 1.5 | 1.44 |
| YDR345C | 0.8 | 0.9 | 5.6 | 2.6 | 1.3 | 1.2 | 1.1 | 1.2 | 0.2 | 1.1 | 1.5 | 1.4 | 1.2 | 0.8 | 0.8 | 1.0 | 1.3 | 1.8 | 5.65 |
| YDR400W | 1.0 | 2.0 | 2.2 | 0.7 | 1.0 | 1.1 | 0.6 | 1.4 | 1.1 | 1.4 | 1.3 | 0.4 | 0.7 | 0.9 | 0.8 | 1.1 | 1.1 | 0.8 | 0.56 |
| YEL063C | 0.7 | 0.8 | 2.4 | 1.3 | 1.2 | 1.3 | 0.7 | 0.6 | 0.8 | 1.7 | 0.8 | 1.1 | 1.1 | 0.8 | 1.3 | 1.0 | 0.7 | 0.7 | 1.12 |
| YER081W | 1.6 | 1.9 | 2.7 | 1.4 | 1.1 | 1.1 | 0.5 | 0.6 | 1.8 | 1.1 | 1.1 | 1.0 | 0.6 | 1.6 | 0.5 | 0.8 | 1.1 | 1.7 | 2.31 |
| YER120W | 0.9 | 1.1 | 2.5 | 0.8 | 0.8 | 1.5 | 0.9 | 0.6 | 0.8 | 0.9 | 1.1 | 1.3 | 1.0 | 0.9 | 1.5 | 1.0 | 1.2 | 1.0 | 1.52 |
| YFL011W | 1.2 | 0.7 | 3.7 | 3.3 | 1.3 | 1.0 | 0.8 | 0.8 | 0.3 | 1.6 | 2.1 | 0.9 | 0.8 | 0.8 | 1.7 | 1.2 | 0.8 | 1.0 | 1.25 |
| YGL012W | 1.0 | 0.8 | 3.0 | 1.2 | 1.5 | 1.6 | 0.9 | 0.8 | 0.3 | 0.9 | 0.6 | 0.6 | 0.3 | 0.8 | 0.6 | 1.2 | 0.9 | 1.1 | 4.88 |
| YGR191W | 0.8 | 0.7 | 1.6 | 1.5 | 0.8 | 1.1 | 0.7 | 0.7 | 0.1 | 0.7 | 1.6 | 0.7 | 1.0 | 0.7 | 1.5 | 0.7 | 0.8 | 0.8 | 1.58 |
| YGR204W | 0.5 | 1.1 | 2.7 | 1.3 | 0.6 | 0.6 | 0.8 | 0.4 | 0.9 | 2.3 | 2.1 | 1.0 | 1.3 | 0.5 | 1.6 | 0.6 | 1.0 | 0.7 | 1.49 |
| YHR025W | 0.6 | 0.8 | 2.4 | 0.6 | 0.9 | 0.8 | 0.8 | 0.8 | 0.2 | 0.4 | 2.1 | 0.9 | 0.7 | 0.6 | 2.1 | 0.5 | 1.0 | 0.6 | 0.68 |
| YHR123W | 0.7 | 1.2 | 1.8 | 1.0 | | 1.0 | 0.9 | 0.8 | 0.8 | 1.1 | 1.2 | 0.7 | 1.5 | 0.8 | 2.3 | 0.5 | 1.1 | 1.0 | 0.85 |
| YJL121C | 1.0 | 0.5 | 1.9 | 0.8 | 1.0 | 1.1 | 0.7 | 1.0 | 0.1 | 0.4 | 1.1 | 0.6 | 0.7 | 0.9 | 1.0 | 0.6 | 1.1 | 1.0 | 1.00 |
| YJR077C | 1.1 | 1.1 | 2.0 | 1.6 | 0.9 | 0.9 | 0.7 | 0.8 | 0.4 | 0.7 | 1.6 | 1.0 | 0.9 | 0.7 | 1.5 | 0.7 | 1.0 | 0.8 | 1.79 |
| YJR143C | 0.6 | 0.6 | 2.1 | 0.8 | 1.6 | 0.6 | 0.5 | 0.7 | 0.1 | 0.3 | 1.3 | 0.6 | 0.3 | 0.6 | 1.1 | 0.5 | 0.7 | 0.8 | 3.24 |
| YKL060C | 1.6 | 0.8 | 2.5 | 1.0 | 1.6 | 1.3 | 1.2 | 1.1 | 0.3 | 0.8 | 2.1 | 1.3 | 1.2 | 0.8 | 1.6 | 1.5 | 1.2 | 1.7 | 6.01 |
| YKL148C | 0.9 | 0.8 | 3.7 | 0.7 | 0.8 | 0.5 | 0.6 | 0.8 | 1.7 | 1.7 | 1.5 | 0.8 | 0.8 | 0.8 | 0.6 | 1.1 | 0.8 | 1.0 | 0.54 |
| YKL157W | 0.7 | 1.0 | 2.3 | 1.3 | 1.5 | 1.5 | 1.9 | 1.1 | 1.7 | 1.9 | 1.5 | 1.0 | 2.3 | 0.8 | 1.2 | 1.7 | 1.2 | 1.5 | 1.22 |
| YLR044C | 0.8 | 0.6 | 2.2 | 0.9 | 1.7 | 1.5 | 1.3 | 0.7 | 0.0 | 1.2 | 1.1 | 1.4 | 1.7 | 0.5 | 2.2 | 0.6 | 0.9 | 0.9 | 5.16 |
| YLR056W | 1.0 | 0.6 | 2.5 | 0.8 | 1.6 | 1.2 | 0.8 | 0.9 | 0.0 | 0.5 | 0.8 | 0.9 | 0.2 | 0.7 | 0.9 | 2.1 | 1.1 | 1.1 | 3.61 |
| YLR058C | 0.9 | 0.8 | 5.5 | 2.6 | 0.7 | 0.9 | 0.5 | 0.3 | 0.2 | 0.1 | 2.6 | 0.4 | 0.3 | 0.7 | 1.5 | 0.4 | 0.8 | 0.8 | 2.71 |
| YLR081W | 1.3 | 0.8 | 2.8 | 3.6 | 1.0 | 0.9 | 0.7 | 0.9 | 0.1 | 2.2 | 2.6 | 1.0 | 0.6 | 1.0 | 0.7 | 1.2 | 0.7 | 0.9 | 1.46 |
| YLR089C | 0.9 | 1.2 | 2.1 | 0.9 | 0.8 | 1.5 | 1.6 | 0.7 | 0.5 | 0.8 | 1.7 | 0.6 | 1.0 | 0.7 | 1.7 | 1.4 | 1.2 | 1.3 | 1.27 |
| YLR284C | 0.9 | 1.0 | 3.8 | 1.9 | 1.6 | 0.9 | 0.8 | 1.2 | 0.9 | 0.9 | 1.5 | 0.6 | 0.8 | 1.3 | 1.0 | 4.5 | 2.9 | 8.1 | 0.84 |
| YLR304C | 0.7 | 0.6 | 5.0 | 0.7 | 0.6 | 1.9 | 0.6 | 0.6 | 0.1 | 2.2 | 1.6 | 1.0 | 0.5 | 0.7 | 1.6 | 1.8 | 0.5 | 0.7 | 2.39 |
| YLR354C | 1.1 | 2.5 | 2.3 | 1.5 | 1.6 | 1.5 | 1.1 | 1.4 | 0.4 | 0.9 | 1.7 | 1.2 | 1.0 | 0.9 | 2.4 | 1.3 | 1.0 | 1.5 | 4.53 |
| YLR372W | 0.7 | 0.3 | 1.7 | 0.8 | 1.4 | 0.5 | 0.2 | 0.9 | 0.0 | 0.1 | 0.9 | 0.6 | 0.1 | 0.6 | 0.7 | 0.2 | 0.5 | 0.6 | 4.46 |
| YML022W | 0.9 | 0.6 | 2.0 | 1.1 | 1.3 | 0.8 | 0.6 | 1.1 | 0.3 | 0.6 | 1.2 | 0.6 | 0.2 | 0.9 | 0.6 | 0.6 | 0.5 | 0.7 | 4.93 |
| YMR011W | 1.3 | 1.0 | 9.4 | 5.5 | 0.9 | 0.6 | 0.8 | 1.1 | 0.0 | 0.7 | 1.5 | 0.8 | 0.4 | 0.6 | 0.6 | 1.2 | 1.3 | 1.6 | 4.95 |
| YMR015C | 0.7 | 0.7 | 1.9 | 0.9 | 1.7 | 1.3 | 0.4 | 0.7 | 0.1 | 0.3 | 1.0 | 0.9 | 0.4 | 0.8 | 1.2 | 1.5 | 1.0 | 1.0 | 2.51 |

| | | | | | | | | | | | | | | | | | | | |
|---|---|---|---|---|---|---|---|---|---|---|---|---|---|---|---|---|---|---|---|
| YMR205C | 0.5 | 0.7 | 2.3 | 0.8 | 1.2 | 1.2 | 0.9 | 0.7 | 0.3 | 0.8 | 1.0 | 1.1 | 1.3 | 0.5 | 0.9 | 0.9 | 0.6 | 0.5 | 4.75 |
| YMR261C | 0.7 | 1.6 | 3.6 | 0.8 | 0.9 | 0.6 | 1.3 | 0.7 | 1.6 | 1.3 | 0.8 | 0.9 | 1.6 | 0.6 | 0.8 | 1.6 | 0.7 | 1.1 | 0.78 |
| YMR323W | 0.8 | 1.1 | 2.9 | 1.1 | 0.7 | 0.4 | 1.1 | 0.8 | 0.5 | 1.3 | 2.5 | 1.2 | 1.8 | 1.2 | 37.3 | 0.6 | 1.1 | 0.6 | 1.04 |
| YOL086C | 1.1 | 0.5 | 2.2 | 1.1 | 1.9 | 1.7 | 1.9 | 0.8 | 0.1 | 1.2 | 2.6 | 1.3 | 1.7 | 0.6 | 1.6 | 1.4 | 1.1 | 1.3 | 4.19 |
| YOL156W | 1.1 | 0.7 | 2.5 | 1.1 | 1.2 | 1.2 | 1.0 | 1.0 | 2.3 | 2.1 | 1.1 | 0.9 | 1.7 | 0.9 | 1.9 | 0.9 | 0.9 | 0.9 | 0.53 |
| YOR002W | 0.8 | 1.1 | 1.9 | 0.9 | 1.1 | 1.1 | 1.0 | 0.9 | 0.8 | 0.9 | 1.2 | 0.5 | 0.6 | 0.9 | 1.5 | 0.8 | 0.9 | 1.1 | 1.49 |
| YOR085W | 0.7 | 0.8 | 1.9 | 1.0 | 1.0 | 0.6 | 0.6 | 0.7 | 0.5 | 0.6 | 1.1 | 0.8 | 1.0 | 0.7 | 1.8 | 0.8 | 0.9 | 0.9 | 1.86 |
| YOR108W | 0.8 | 0.9 | 2.3 | 0.6 | 1.1 | 1.6 | 1.2 | 0.9 | 0.4 | 1.1 | 1.4 | 0.7 | 1.3 | 0.9 | 0.7 | 1.0 | 1.1 | 1.1 | 1.07 |
| YOR128C | 0.9 | 0.7 | 2.1 | 2.1 | 1.1 | 1.0 | 0.7 | 0.6 | 0.3 | 0.4 | 0.8 | 0.5 | 0.4 | 0.7 | 1.6 | 0.5 | 1.2 | 1.2 | 2.14 |
| YOR142W | 1.0 | 1.2 | 3.4 | 1.0 | 1.4 | 1.6 | 1.0 | 0.8 | 1.1 | 1.5 | 1.5 | 0.8 | 1.4 | 0.8 | 1.1 | 0.8 | 0.9 | 1.0 | 1.31 |
| YOR176W | 0.7 | 2.6 | 2.7 | 0.9 | 1.0 | 0.9 | 0.5 | 1.0 | 1.0 | 0.5 | 1.4 | 1.3 | 1.5 | 0.8 | 0.8 | 1.4 | 1.2 | 2.1 | 1.23 |
| YPL057C | 1.9 | 1.1 | 3.2 | 3.1 | 1.9 | 0.6 | 0.6 | 1.0 | 0.4 | 1.4 | 1.6 | 1.0 | 1.3 | 0.7 | 1.1 | 2.5 | 2.2 | 2.0 | 2.08 |
| YPL135W | 0.9 | 1.2 | 2.5 | 1.2 | 1.6 | 1.3 | 1.2 | 1.0 | 0.5 | 2.8 | 1.3 | 1.1 | 1.2 | 1.1 | 1.5 | 1.2 | 1.5 | 1.7 | 1.50 |
| YCR010C | 1.6 | 1.6 | 1.6 | 4.2 | 1.7 | 1.3 | 1.3 | 0.8 | 0.9 | 4.7 | 1.1 | 0.8 | 1.0 | 1.2 | 1.2 | 2.8 | 1.9 | 1.5 | 0.26 |
| YBR003W | 0.9 | 0.8 | 1.0 | 1.9 | 0.7 | 1.2 | 1.1 | 0.9 | 1.0 | 1.3 | 1.3 | 0.8 | 1.2 | 0.8 | 0.7 | 2.0 | 1.1 | 1.3 | 1.06 |
| YBR020W | 1.0 | 0.5 | 1.9 | 1.9 | 1.0 | 0.8 | 0.7 | 1.0 | -0.4 | 1.1 | 1.0 | 0.8 | 1.1 | 1.0 | 1.3 | 0.7 | 0.8 | 0.9 | 0.27 |
| YDR123C | 1.2 | 0.8 | 0.4 | 2.1 | 1.6 | 0.7 | 0.9 | 1.3 | 0.5 | 0.6 | 0.6 | 0.5 | 0.9 | 0.7 | 1.2 | 0.8 | 1.3 | 1.0 | 0.30 |
| YDR277C | 1.6 | 1.2 | 1.0 | 3.0 | 0.8 | 1.1 | 1.2 | 1.7 | 0.2 | 0.9 | 1.2 | 0.9 | 1.2 | 1.3 | 0.4 | 2.3 | 1.2 | 1.7 | 0.89 |
| YDR408C | 1.0 | 1.0 | 1.4 | 4.1 | 0.9 | 0.9 | 0.8 | 0.7 | 0.3 | 0.5 | 1.7 | 0.6 | 0.5 | 0.9 | 1.0 | 1.0 | 1.1 | 1.1 | 2.16 |
| YDR483W | 1.1 | 1.0 | 1.3 | 2.0 | 0.7 | 1.5 | 0.8 | 1.0 | 0.4 | 1.1 | 1.4 | 0.8 | 1.3 | 0.8 | 1.5 | 1.2 | 1.2 | 1.4 | 3.24 |
| YGL115W | 0.9 | 0.6 | 1.2 | 2.0 | 1.2 | 1.2 | 1.4 | 1.0 | 1.2 | 1.2 | 1.0 | 0.9 | 0.7 | 1.2 | 0.7 | 1.7 | 0.8 | 1.2 | 2.46 |
| YGR096W | 1.5 | 1.0 | 1.1 | 4.6 | 1.2 | 1.7 | 0.9 | 1.0 | 0.8 | 0.8 | 0.8 | 0.8 | 1.1 | 1.2 | 0.9 | 0.8 | 0.8 | 1.0 | 0.49 |
| YGR288W | 0.9 | 0.5 | 1.0 | 2.6 | 0.8 | | 1.4 | 1.0 | 1.6 | 2.6 | 1.3 | 0.9 | 1.3 | 1.1 | 0.8 | 1.7 | 1.2 | 1.3 | 0.41 |
| YHR210C | 1.3 | 1.0 | 0.6 | 5.2 | 1.8 | 0.7 | 1.9 | 1.3 | -0.1 | 0.9 | 1.5 | 0.5 | 0.4 | 1.0 | 0.5 | 1.5 | 1.4 | 1.2 | 0.33 |
| YIL006W | 1.0 | 1.1 | 1.4 | 2.9 | 0.8 | 0.3 | 0.8 | 0.6 | 0.9 | 1.1 | 1.0 | 0.9 | 0.8 | 1.0 | 1.5 | 1.7 | 0.8 | 0.9 | 0.28 |
| YKR034W | 0.9 | 1.5 | 1.1 | 4.7 | 0.9 | 0.9 | 0.7 | 1.5 | 0.5 | 0.6 | 0.0 | 0.7 | 1.2 | 1.1 | 1.7 | 0.6 | 0.8 | 1.0 | 0.26 |
| YLR006C | 0.7 | 0.7 | 0.9 | 6.2 | 1.6 | 0.9 | 1.1 | 0.9 | 1.1 | 1.1 | 1.5 | 0.7 | 0.8 | 1.0 | 0.9 | 0.8 | 1.6 | 1.0 | 0.40 |
| YNL025C | 0.9 | 1.4 | 1.4 | 12.8 | 0.7 | 0.5 | 1.2 | 1.0 | 1.1 | 1.8 | 1.1 | 0.8 | 1.3 | 1.2 | 1.4 | 2.9 | 0.7 | 1.3 | 0.37 |
| YOL116W | 1.1 | 1.0 | 0.9 | 2.8 | 1.7 | 0.7 | 1.5 | 1.3 | 0.6 | 1.8 | 1.0 | 0.6 | 0.8 | 0.9 | 0.7 | 1.9 | 1.1 | 1.4 | 0.47 |
| YOR103C | 1.1 | 0.8 | 1.3 | 1.8 | 1.5 | 1.0 | 1.2 | 1.0 | 0.8 | 0.9 | 1.0 | 1.2 | 0.8 | 1.2 | 1.4 | 1.3 | 1.1 | 1.5 | 2.84 |
| YOR251C | 1.1 | 1.0 | 1.3 | 1.8 | 0.8 | 0.9 | 1.0 | 1.1 | 0.8 | 0.8 | 1.0 | 0.9 | 1.0 | 1.1 | 0.6 | 1.2 | 0.9 | 1.3 | 1.34 |

| | | | | | | | | | | | | | | | | | | | |
|---|---|---|---|---|---|---|---|---|---|---|---|---|---|---|---|---|---|---|---|
| YOR348C | 1.1 | 0.7 | 1.2 | 2.0 | 0.9 | 0.7 | 0.8 | 0.8 | 0.5 | 0.4 | 1.1 | 0.7 | 1.1 | 1.2 | 1.6 | 1.7 | 0.9 | 0.9 | 0.18 |
| YPL148C | 1.2 | 0.8 | 0.9 | 2.3 | 1.1 | 0.6 | 1.0 | 0.9 | 0.7 | 1.5 | 3.5 | 0.7 | 0.6 | 1.6 | 0.6 | 1.6 | 1.0 | 1.1 | 0.48 |
| YGL205W | 0.9 | 0.9 | 0.7 | 0.8 | 1.1 | 1.6 | 0.8 | 1.2 | 0.5 | 0.6 | 0.4 | 1.1 | 1.0 | 1.0 | 2.0 | 4.1 | 3.9 | 9.1 | 0.24 |
| YNL192W | 1.1 | 1.2 | 1.5 | 1.6 | 0.9 | 0.6 | 1.0 | 0.8 | 0.6 | 1.8 | 1.1 | 1.1 | 2.7 | 0.7 | 2.1 | 0.8 | 2.3 | 2.8 | 1.31 |
| YOL108C | 1.4 | 1.5 | 0.4 | 1.9 | 2.1 | 0.9 | 1.1 | 1.3 | 1.6 | 0.9 | 1.1 | 1.0 | 1.3 | 0.9 | 1.5 | 1.8 | 1.5 | 2.7 | 0.87 |
| YPR165W | 1.3 | 0.7 | 1.6 | 1.0 | 1.5 | 1.8 | 1.0 | 1.0 | 0.9 | 0.7 | 1.0 | 0.8 | 1.0 | 1.0 | 0.9 | 1.0 | 2.3 | 2.7 | 3.64 |
| YDR073W | 1.7 | 0.9 | 0.7 | 1.4 | 1.2 | 1.3 | 1.2 | 1.3 | 0.5 | 1.2 | 1.0 | 0.8 | 0.6 | 1.2 | 0.8 | 1.6 | 1.8 | 2.3 | 1.27 |
| YER015W | 0.7 | 0.9 | 1.1 | 1.5 | 1.3 | 0.9 | 1.2 | 1.1 | 0.4 | 0.9 | 1.0 | 0.9 | 0.8 | 1.1 | 0.8 | 3.0 | 1.6 | 2.6 | 0.41 |
| YJL167W | 0.9 | 1.4 | 1.4 | 1.1 | 0.7 | 1.3 | 1.5 | 1.5 | 0.9 | 0.9 | 1.2 | 1.3 | 0.9 | 0.9 | 1.6 | 1.4 | 1.4 | 2.1 | 2.94 |
| YJL216C | 0.8 | 4.7 | 2.2 | 0.9 | 0.8 | | 1.5 | 1.9 | 1.5 | 1.3 | 1.1 | 1.0 | 1.1 | 1.1 | 1.2 | 1.5 | 1.2 | 2.0 | 0.25 |
| YKR009C | 1.0 | 1.2 | 1.5 | 0.9 | 1.0 | 1.8 | 1.0 | 0.9 | 2.4 | 2.1 | 1.2 | 1.0 | 1.8 | 1.0 | 1.0 | 4.6 | 1.9 | 2.5 | 0.27 |
| YOR180C | 0.8 | 1.1 | 1.0 | 1.0 | 1.0 | 0.7 | 0.8 | 0.9 | 0.3 | 0.4 | 0.6 | 0.8 | 0.9 | 1.0 | 1.1 | 2.4 | 0.9 | 2.3 | 0.55 |
| YBR036C | 0.8 | 1.7 | 1.8 | 1.2 | 1.2 | 1.3 | 1.3 | 0.7 | 1.0 | 1.0 | 1.5 | 1.0 | 1.9 | 0.8 | 1.5 | 1.6 | 2.1 | 1.8 | 2.05 |
| YDR297W | 1.5 | 0.8 | 0.8 | 1.1 | 0.9 | 1.4 | 0.6 | 1.5 | 0.3 | 0.7 | 1.4 | 1.0 | 0.8 | 0.9 | 1.4 | 1.4 | 1.9 | 1.9 | 1.73 |
| YDR387C | 0.8 | 0.9 | 1.2 | 2.2 | 0.9 | 1.2 | 1.3 | 0.7 | 1.4 | 2.0 | 2.0 | 0.9 | 1.3 | 0.9 | 1.4 | 2.7 | 1.2 | 1.1 | 0.86 |
| YOL096C | 1.1 | 1.0 | 1.2 | 1.6 | 1.2 | 1.3 | 1.7 | 1.0 | 1.3 | 1.4 | 1.2 | 1.2 | 1.4 | 1.4 | 1.0 | 2.3 | 1.2 | 1.2 | 0.64 |
| YPR184W | 1.2 | 1.4 | 5.7 | 2.2 | 0.9 | 1.5 | 1.3 | 0.7 | 1.9 | 3.2 | 3.6 | 1.4 | 2.5 | 1.3 | 1.5 | 3.1 | 1.0 | 1.7 | 0.37 |
| YBR298C | 1.1 | 1.0 | 1.6 | 2.3 | 1.4 | 0.4 | 1.7 | 0.5 | 0.3 | 1.1 | 1.5 | 1.1 | 0.8 | 0.9 | 0.2 | 2.1 | 0.9 | 0.8 | 0.88 |
| YDL078C | 0.7 | 1.1 | 1.2 | 0.8 | 1.3 | 1.1 | 1.7 | 1.0 | 1.1 | 1.0 | 1.7 | 0.8 | 1.0 | 0.8 | 0.5 | 2.2 | 1.1 | 2.0 | 1.65 |
| YDL215C | 1.0 | 1.5 | 1.0 | 1.6 | 1.5 | 0.9 | 1.9 | 1.1 | 1.7 | 2.4 | 0.8 | 0.8 | 1.5 | 1.0 | 1.1 | 2.2 | 1.1 | 1.6 | 0.91 |
| YGL035C | 0.7 | 1.4 | 4.3 | 1.4 | 1.1 | 0.6 | 1.2 | 1.3 | 0.9 | 1.5 | 1.3 | 0.9 | 1.1 | 1.1 | 1.0 | 2.3 | 0.9 | 1.2 | 0.61 |
| YGR287C | 1.3 | 1.0 | 1.3 | 1.5 | 0.8 | 0.8 | 2.2 | 1.3 | 2.6 | 4.2 | 1.5 | 1.1 | 1.7 | 1.1 | 2.3 | 3.0 | 1.0 | 1.1 | 0.38 |
| YJL070C | 1.3 | 0.9 | 1.1 | 1.2 | 1.3 | 2.0 | 1.4 | 1.1 | 1.0 | 1.1 | 1.3 | 1.0 | 1.8 | 1.3 | 3.5 | 2.2 | 1.1 | 1.2 | 0.34 |
| YLR351C | 1.0 | 1.4 | 1.3 | 1.5 | 1.3 | 1.4 | 1.4 | 1.2 | 0.8 | 1.2 | 1.1 | 0.9 | 0.9 | 1.4 | 1.2 | 2.0 | 1.3 | 1.8 | 1.75 |
| YLR375W | 0.9 | 1.5 | 0.9 | 0.9 | 1.0 | 1.3 | 0.9 | 1.0 | 1.6 | 1.2 | 1.6 | 0.9 | 1.6 | 0.9 | 1.3 | 2.2 | 1.0 | 1.0 | 1.05 |
| YMR267W | 0.8 | 1.8 | 0.7 | 0.9 | 1.0 | 1.4 | 1.2 | 1.5 | 0.4 | 0.8 | 1.0 | 0.8 | 0.7 | 1.0 | 0.9 | 2.6 | 0.9 | 1.3 | 0.94 |
| YMR278W | 0.7 | 1.7 | 1.4 | 1.0 | 1.2 | 1.3 | 1.8 | 1.1 | 1.4 | 1.7 | 1.1 | 1.0 | 2.5 | 0.8 | 1.8 | 2.5 | 1.0 | 1.5 | 0.62 |
| YMR293C | 0.9 | 1.5 | 1.6 | 2.2 | 0.6 | 0.5 | 1.2 | 1.0 | 0.7 | 1.3 | 0.4 | 0.9 | 0.8 | 1.3 | 0.7 | 2.2 | 0.8 | 1.0 | 0.37 |
| YNR072W | 1.1 | 1.5 | 0.5 | 0.7 | 1.0 | 0.9 | 1.5 | 0.9 | 0.7 | 1.8 | 1.4 | 1.0 | 1.5 | 1.0 | 1.8 | 2.1 | 1.3 | 1.0 | 0.26 |
| YOR363C | 0.8 | 2.0 | 0.9 | 1.3 | 0.7 | 1.2 | 0.7 | 1.1 | 1.2 | 1.6 | 1.2 | 0.9 | 1.3 | 0.9 | 1.1 | 2.1 | 1.3 | 1.8 | 0.40 |

## Table 8 Detoxification protein genes

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YDR453C | 1.1 | 2.7 | 4.4 | 3.0 | 0.7 | 1.7 | 1.0 | 1.3 | 4.5 | 6.1 | 2.3 | 0.9 | 1.4 | 3.8 | 2.3 | 5.6 | 1.0 | 1.5 | 1.53 |
| YLL060C | 12.5 | 4.2 | 2.3 | 2.6 | 0.9 | 1.4 | 2.0 | 5.8 | 13.0 | 23.2 | 14.1 | 1.1 | 1.8 | 10.6 | 1.5 | 3.6 | 1.9 | 2.3 | 0.57 |
| YBL064C | 2.6 | 2.8 | 3.9 | 1.7 | 0.8 | 5.2 | 2.5 | 2.4 | 4.5 | 3.8 | 2.9 | 1.7 | 4.1 | 5.0 | 1.3 | 5.0 | 1.2 | 2.3 | 1.67 |
| YBR008C | 3.0 | 2.4 | 4.9 | 0.7 | 0.9 | 1.0 | 0.9 | 2.8 | 54.6 | 21.1 | 9.4 | 0.8 | 2.0 | 4.1 | 0.8 | 3.1 | 2.0 | 1.2 | 0.37 |
| YOR153W | 1.6 | 0.9 | 5.1 | 1.1 | 1.0 | 7.4 | 2.6 | 0.5 | 3.2 | 1.2 | 1.0 | 4.0 | 11.8 | 0.3 | 3.6 | 1.6 | 2.5 | 3.1 | 1.91 |
| YHL047C | 0.6 | 4.4 | 1.0 | 1.0 | 1.2 | 8.4 | 16.8 | 1.5 | 1.2 | 11.9 | 1.0 | 2.6 | 3.8 | 0.5 | 1.1 | 2.3 | 1.2 | 1.2 | 0.74 |
| YCL035C | 2.0 | 2.3 | 1.5 | 1.7 | 1.5 | 5.1 | 2.7 | 1.4 | 1.9 | 2.3 | 2.1 | 1.5 | 2.8 | 2.5 | 1.9 | 5.5 | 2.6 | 4.2 | 1.74 |
| YGR197C | 1.1 | 2.4 | 2.5 | 1.0 | 0.7 | 0.5 | 1.3 | 0.8 | 16.7 | 3.2 | 1.1 | 1.2 | 2.4 | 1.5 | 1.0 | 1.5 | 1.7 | 1.9 | 0.37 |
| YHR055C | 4.4 | 1.8 | 0.8 | 1.2 | 2.7 | 1.6 | 1.1 | 2.1 | 3.9 | 3.3 | 1.8 | 1.2 | 3.0 | 1.5 | 0.5 | 0.8 | 0.9 | 0.6 | 3.96 |
| YNL239W | 1.0 | 2.4 | 2.4 | 1.2 | 1.2 | 1.8 | 2.1 | 1.3 | 2.5 | 6.0 | 1.9 | 1.5 | 3.6 | 1.3 | 1.7 | 1.5 | 1.1 | 1.4 | 0.68 |
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YBR293W | 1.9 | 3.1 | 1.0 | 1.8 | 0.8 | 0.9 | 0.9 | 0.9 | 5.5 | 2.4 | 1.1 | 1.0 | 3.0 | 1.4 | 3.1 | 1.6 | 1.0 | 0.9 | 0.94 |
| YDL100C | 1.0 | 1.2 | 1.1 | 0.8 | 1.0 | 2.1 | 1.6 | 1.7 | 5.4 | 3.2 | 2.8 | 1.5 | 2.5 | 1.4 | 1.0 | 1.3 | 1.2 | 1.4 | 2.60 |
| YER185W | 2.0 | 3.1 | 2.1 | 7.0 | 1.6 | 1.2 | 1.1 | 1.0 | 2.9 | 1.2 | 1.6 | 1.1 | 2.1 | 1.6 | 1.7 | 0.6 | 2.6 | 1.1 | 0.26 |
| YGL013C | 0.9 | 1.2 | 0.8 | 0.7 | 1.1 | 2.3 | 0.9 | 1.4 | 3.0 | 1.5 | 0.9 | 1.0 | 2.3 | 0.8 | 1.0 | 0.9 | 1.1 | 1.0 | 0.43 |
| YHR053C | 3.5 | 2.1 | 0.7 | 1.1 | 2.9 | 1.3 | 1.2 | 2.2 | 4.5 | 2.9 | 1.4 | 1.1 | 1.9 | 1.5 | 0.3 | 0.7 | 1.0 | 0.5 | 3.99 |
| YIR038C | 1.3 | 3.5 | 4.6 | 2.0 | 0.7 | 2.5 | 2.0 | 1.3 | 4.6 | 4.5 | 3.4 | 1.1 | 2.5 | 2.5 | 1.2 | 6.0 | 2.8 | 2.0 | 1.11 |
| YKL026C | 2.7 | 2.0 | 1.2 | 4.9 | 1.0 | 1.8 | 2.5 | 1.8 | 7.5 | 3.0 | 3.0 | 1.0 | 2.3 | 1.8 | 0.8 | 6.6 | 1.6 | 3.3 | 0.61 |
| YLL028W | 0.6 | 0.9 | 4.6 | 0.5 | 0.9 | 1.7 | 1.0 | 0.7 | 1.2 | 2.3 | 0.7 | 1.3 | 4.1 | 0.7 | 3.1 | 0.6 | 1.2 | 1.2 | 1.02 |
| YOR273C | 0.7 | 1.0 | 1.7 | 1.0 | 1.1 | 0.4 | 0.4 | 0.6 | 0.6 | 0.5 | 1.5 | 0.8 | 2.6 | 0.5 | 0.8 | 0.6 | 3.2 | 3.1 | 1.20 |
| YGR138C | 1.1 | 1.6 | 1.0 | 1.2 | 0.8 | 0.8 | 0.7 | 1.0 | 0.5 | 1.0 | 0.7 | 0.6 | 0.6 | 0.9 | 4.5 | 0.7 | 0.7 | 0.7 | 1.24 |
| YOR247W | 1.7 | 0.6 | 3.6 | 1.4 | 0.6 | 0.2 | 0.5 | 0.7 | 0.1 | 0.4 | 2.9 | 0.6 | 0.3 | 0.4 | 7.3 | 0.2 | 1.2 | 0.9 | 2.23 |
| YPL163C | 0.6 | 0.7 | 1.4 | 0.9 | 0.6 | 0.6 | 0.4 | 0.5 | 0.1 | 0.4 | 0.5 | 0.9 | 0.4 | 0.6 | 5.3 | 0.6 | 1.4 | 1.4 | 1.05 |
| YHL040C | 1.6 | 4.4 | 1.5 | 0.4 | 1.1 | 7.3 | 4.0 | 1.5 | 1.1 | 11.3 | 1.7 | 2.0 | 2.0 | 1.1 | 3.4 | 1.0 | 1.4 | 1.0 | 0.69 |
| YEL065W | 0.3 | 3.8 | 1.2 | 0.4 | 1.4 | 2.3 | 4.3 | 0.9 | 0.1 | 4.9 | 0.6 | 1.7 | 0.8 | 0.4 | 2.2 | 2.6 | 0.7 | 0.6 | 2.10 |
| YIR002C | 0.8 | 1.1 | 0.5 | 0.6 | 1.2 | 2.2 | 1.3 | 1.2 | 1.7 | 1.4 | 0.8 | 1.3 | 1.2 | 1.1 | 1.1 | 1.4 | 1.1 | 1.2 | 0.58 |
| YNL259C | 1.6 | 4.5 | 1.1 | 1.2 | 1.1 | 3.9 | 3.8 | 1.5 | 1.6 | 2.1 | 1.6 | 0.7 | 1.1 | 1.4 | 0.7 | 1.7 | 2.6 | 2.4 | 1.22 |
| YBR145W | 1.5 | 0.7 | 2.8 | 0.9 | 1.1 | 11.5 | 58.8 | 1.0 | 0.1 | 1.1 | 1.1 | 1.0 | 2.0 | 2.2 | 1.2 | 3.6 | 1.7 | 2.0 | 2.17 |
| YLR043C | 1.4 | 1.4 | 2.0 | 1.8 | 2.0 | 1.4 | 2.6 | 1.1 | 2.8 | 2.5 | 1.4 | 0.8 | 2.0 | 2.1 | 0.5 | 1.8 | 1.5 | 2.2 | 2.12 |

| | | | | | | | | | | | | | | | | | | | |
|---|---|---|---|---|---|---|---|---|---|---|---|---|---|---|---|---|---|---|---|
| YGR209C | 1.6 | 3.1 | 3.5 | 1.5 | 1.9 | 1.9 | 1.7 | 1.6 | 2.4 | 6.3 | 3.1 | 0.9 | 1.4 | 5.0 | 1.3 | 1.9 | 1.3 | 2.4 | 3.17 |
| YDL168W | 2.3 | 2.0 | 2.1 | 0.9 | 1.2 | 1.4 | 1.1 | 1.7 | 8.2 | 4.7 | 1.9 | 0.6 | 1.3 | 1.6 | 1.2 | 1.1 | 0.9 | 0.8 | 1.08 |
| YDR513W | 2.2 | 2.5 | 2.3 | 2.6 | 0.9 | 2.1 | 1.6 | 1.6 | 4.6 | 3.1 | 2.0 | 0.9 | 1.8 | 2.0 | 1.3 | 3.8 | 1.3 | 3.2 | 3.10 |
| YGR088W | 1.3 | 1.2 | 7.7 | 2.4 | 1.0 | 1.3 | 0.8 | 0.8 | 1.5 | 3.2 | 3.9 | 1.1 | 1.2 | 1.3 | 0.7 | 5.6 | 0.9 | 2.0 | 0.75 |
| YHR048W | 2.5 | 1.4 | 1.4 | 1.7 | 1.0 | 0.8 | 0.8 | 1.9 | 4.5 | 2.7 | 2.0 | 0.9 | 1.1 | 1.7 | 0.9 | 1.4 | 0.7 | 0.9 | 0.26 |
| YJL101C | 2.1 | 1.9 | 2.1 | 1.2 | 1.4 | 1.0 | 1.0 | 1.3 | 3.1 | 3.6 | 2.7 | 0.7 | 1.0 | 1.0 | 0.5 | 1.3 | 0.9 | 1.0 | 1.13 |
| YML116W | 4.1 | 1.3 | 1.5 | 1.4 | 1.2 | 0.9 | 1.4 | 2.2 | 1.4 | 3.1 | 4.3 | 0.5 | 0.8 | 2.0 | 1.9 | 1.0 | 1.0 | 1.0 | 0.94 |
| YMR038C | 1.9 | 1.7 | 1.8 | 0.8 | 1.5 | 1.0 | 1.1 | 1.9 | 2.4 | 2.6 | 2.7 | 0.9 | 1.7 | 1.1 | 0.6 | 1.5 | 1.2 | 1.3 | 1.76 |
| YBR244W | 0.6 | 1.4 | 2.0 | 0.9 | 1.7 | 1.0 | 0.6 | 1.0 | 1.4 | 3.0 | 1.5 | 0.5 | 0.4 | 3.7 | 0.4 | 0.7 | 1.2 | 1.2 | 3.42 |
| YCL069W | 0.9 | 15.7 | 0.9 | 0.8 | 0.9 | | 1.4 | 1.3 | 1.2 | 6.9 | 1.0 | 0.9 | 0.8 | 1.3 | 1.4 | 0.9 | 1.0 | 1.0 | 0.25 |
| YKR105C | 0.8 | 0.9 | 0.9 | 1.5 | 1.0 | 1.2 | 1.0 | 1.3 | 1.0 | 5.2 | 0.0 | 0.8 | 2.5 | 1.2 | 1.5 | 0.7 | 0.8 | 1.0 | 0.26 |
| YOL158C | 0.7 | 4.0 | 2.4 | 0.9 | 1.2 | 2.0 | 1.6 | 0.7 | 1.7 | 6.1 | 0.7 | 0.9 | 1.4 | 1.0 | 0.9 | 1.2 | 1.4 | 1.7 | 1.30 |
| YDR256C | 0.8 | 1.4 | 0.7 | 1.3 | 1.2 | 0.5 | 0.6 | 0.8 | 5.2 | 2.0 | 1.4 | 0.8 | 1.1 | 1.6 | 1.0 | 4.3 | 1.0 | 2.0 | 0.30 |
| YGL254W | 1.2 | 1.1 | 0.8 | 0.6 | 1.1 | 0.7 | 1.3 | 1.4 | 3.1 | 1.8 | 1.1 | 1.2 | 2.4 | 1.3 | 1.1 | 1.4 | 0.8 | 1.2 | 0.53 |
| YKL064W | 0.6 | 0.9 | 0.4 | 0.8 | 1.2 | 1.5 | 1.1 | 0.9 | 2.4 | 1.6 | 0.7 | 0.7 | 1.6 | 1.1 | 0.7 | 1.4 | 1.3 | 1.3 | 0.60 |
| YLL057C | 2.2 | 59.9 | 1.8 | 0.9 | 1.1 | 1.0 | 1.6 | 1.4 | 121.8 | 2.9 | 1.3 | 0.7 | 1.4 | 1.1 | 1.4 | 0.9 | 3.0 | 1.3 | 0.19 |
| YPR200C | 47.7 | 1.7 | 0.8 | 5.0 | 1.6 | 0.8 | 1.3 | 1.4 | 4.7 | 3.8 | 2.0 | 0.4 | 0.6 | 2.0 | 0.7 | 1.2 | 1.1 | 1.0 | 0.27 |
| YJR104C | 1.4 | 1.8 | 2.9 | 1.2 | 1.5 | 1.4 | 1.1 | 1.5 | 1.9 | 2.8 | 2.6 | 0.9 | 0.9 | 2.0 | 1.3 | 2.9 | 0.9 | 1.1 | 3.50 |
| YKR106W | 1.3 | 0.8 | 2.1 | 1.4 | 1.2 | 1.6 | 0.9 | 1.3 | 10.5 | 7.4 | 1.8 | 0.8 | 1.4 | 1.7 | | 2.8 | 0.9 | 0.8 | 0.16 |
| YOR031W | 2.6 | 1.7 | 1.3 | 3.4 | 1.4 | 0.9 | 1.3 | 0.9 | 0.7 | 1.3 | 1.2 | 0.8 | 1.8 | 1.5 | 1.4 | 6.2 | 1.7 | 2.4 | 0.52 |
| YHR008C | 1.3 | 6.9 | 4.8 | 1.8 | 0.6 | 1.0 | 0.7 | 0.9 | 1.7 | 2.4 | 2.1 | 0.7 | 1.2 | 1.6 | 1.7 | 2.2 | 0.9 | 1.0 | 1.04 |
| YML028W | 1.0 | 2.2 | 4.4 | 1.3 | 1.6 | 1.2 | 0.8 | 0.8 | 0.8 | 1.8 | 1.0 | 1.6 | 1.6 | 2.0 | 2.2 | 1.3 | 1.1 | 1.5 | 4.85 |
| YEL027W | 1.2 | 0.6 | 3.4 | 1.1 | 1.0 | 1.1 | 0.8 | 0.9 | 0.9 | 1.1 | 0.9 | 0.9 | 0.7 | 1.4 | 1.5 | 1.6 | 1.0 | 1.4 | 4.75 |
| YKR066C | 0.9 | 1.4 | 5.5 | 0.7 | 1.0 | 0.9 | 0.5 | 0.7 | 0.7 | 2.4 | 1.3 | 0.9 | 0.6 | 1.5 | 0.6 | 1.3 | 0.9 | 1.1 | 1.25 |
| YDR538W | 0.9 | 1.1 | 2.4 | 1.2 | 1.0 | 1.0 | 1.2 | 1.0 | 0.3 | 0.8 | 0.9 | 0.7 | 0.7 | 0.9 | 1.0 | 0.9 | 1.0 | 0.9 | 0.53 |
| YMR015C | 0.7 | 0.7 | 1.9 | 0.9 | 1.7 | 1.3 | 0.4 | 0.7 | 0.1 | 0.3 | 1.0 | 0.9 | 0.4 | 0.8 | 1.2 | 1.5 | 1.0 | 1.0 | 2.51 |
| YOR251C | 1.1 | 1.0 | 1.3 | 1.8 | 0.8 | 0.9 | 1.0 | 1.1 | 0.8 | 0.8 | 1.0 | 0.9 | 1.0 | 1.1 | 0.6 | 1.2 | 0.9 | 1.3 | 1.34 |
| YLR046C | 0.7 | 1.7 | 0.9 | 0.8 | 1.0 | 3.8 | 1.5 | 0.5 | 1.0 | 1.4 | 0.9 | 0.8 | 1.2 | 1.3 | 0.8 | 1.3 | 5.2 | 5.4 | 0.86 |
| YGR224W | 0.9 | 1.2 | 1.2 | 1.2 | 1.4 | 0.8 | 0.4 | 0.6 | 0.9 | 0.9 | 0.8 | 0.6 | 0.8 | 0.9 | 1.1 | 1.0 | 2.8 | 1.3 | 0.27 |
| YFL050C | 0.7 | 1.0 | 1.1 | 2.2 | 1.0 | 0.9 | 0.8 | 0.9 | 0.5 | 1.6 | 1.2 | 0.9 | 1.2 | 1.0 | 1.4 | 2.3 | 0.8 | 1.0 | 0.36 |
| YCR083W | 1.1 | 2.8 | 1.5 | 1.8 | 1.6 | 2.4 | 1.8 | 1.5 | 1.8 | 1.8 | 1.8 | 1.1 | 1.8 | 2.4 | 1.0 | 3.1 | 1.3 | 1.3 | 0.98 |

## Table 9 Genes belonging to other category

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YKR076W | 5.0 | 2.6 | 3.5 | 2.2 | 1.0 | 4.7 | 4.9 | 6.2 | 43.4 | 43.1 | 18.6 | 1.5 | 5.1 | 14.7 | 3.2 | 9.6 | 1.8 | 2.7 | 0.27 |
| YNL335W | 1.2 | 0.7 | 1.5 | 2.3 | 1.5 | 1.6 | 1.3 | 1.7 | 1.6 | 5.3 | 1.1 | 0.8 | 0.9 | 4.6 | 1.7 | 1.0 | 1.2 | 1.1 | 0.22 |
| YBR173C | 1.1 | 2.4 | 3.4 | 1.6 | 0.7 | 1.3 | 1.6 | 1.2 | 6.0 | 5.1 | 2.5 | 1.7 | 3.8 | 3.0 | 15.4 | 2.1 | 0.9 | 1.6 | 1.15 |
| YFL022C | 0.8 | 0.9 | 1.5 | 0.6 | 0.7 | 0.9 | 0.3 | 0.8 | 0.5 | 0.5 | 1.2 | 0.6 | 0.6 | 3.1 | 1.1 | 1.3 | 0.9 | 0.8 | 1.07 |
| YGL158W | 1.1 | 2.8 | 1.9 | 12.9 | 1.0 | 0.4 | 1.3 | 1.3 | -0.4 | 0.9 | 0.2 | 1.3 | 1.0 | 2.3 | 2.3 | 8.2 | 0.8 | 1.1 | 0.18 |
| YHR139C | 3.0 | 4.6 | 6.8 | 9.3 | 1.3 | 1.9 | 1.5 | 1.3 | 107.2 | 46.6 | 3.5 | 1.6 | 7.0 | 2.5 | 1.9 | 17.1 | 1.4 | 1.3 | 0.34 |
| YGR213C | 5.4 | 2.9 | 1.0 | 6.8 | 1.2 | 4.1 | 7.0 | 2.0 | 7.5 | 6.9 | 1.6 | 10.6 | 30.4 | 1.0 | 6.5 | 5.2 | 12.8 | 8.5 | 0.22 |
| YKL165C | 0.5 | 2.0 | 1.1 | 0.6 | 0.9 | 0.6 | 2.1 | 1.5 | 0.4 | 0.4 | 0.3 | 2.4 | 3.2 | 0.8 | 2.1 | 1.2 | 0.9 | 1.0 | 1.08 |
| YBL005W-A | 1.0 | 0.9 | 0.5 | 0.9 | 1.3 | 1.2 | 1.4 | 1.0 | 0.8 | 1.5 | 0.8 | 1.6 | 2.9 | 1.4 | 0.9 | 1.1 | 1.3 | 1.0 | 0.48 |
| YBL041W | 1.2 | 1.4 | 0.9 | 1.0 | 1.6 | 1.7 | 1.6 | 1.7 | 2.8 | 2.6 | 1.6 | 1.6 | 2.6 | 2.0 | 1.3 | 2.3 | 1.3 | 2.0 | 3.03 |
| YBL078C | 2.3 | 2.7 | 1.3 | 2.6 | 0.7 | 2.4 | 3.1 | 1.6 | 12.3 | 15.2 | 4.6 | 3.1 | 5.0 | 3.6 | 2.5 | 4.4 | 1.8 | 2.8 | 0.65 |
| YCL020W | 1.1 | 2.5 | 0.5 | 1.1 | 0.5 | 1.0 | 1.5 | 1.7 | 0.9 | 2.4 | 1.3 | 1.5 | 3.0 | 1.7 | 2.5 | 1.3 | 0.9 | 1.3 | 1.46 |
| YDL007W | 0.8 | 1.4 | 1.3 | 0.9 | 1.0 | 1.1 | 1.2 | 1.4 | 3.8 | 2.4 | 1.3 | 1.1 | 2.4 | 1.4 | 1.4 | 0.8 | 0.8 | 1.5 | 2.43 |
| YDL097C | 0.9 | 1.7 | 0.9 | 1.2 | 0.8 | 1.3 | 1.6 | 1.5 | 4.5 | 2.3 | 1.5 | 1.3 | 4.0 | 1.4 | 1.1 | 1.1 | 1.0 | 1.3 | 2.06 |
| YDL126C | 0.5 | 1.4 | 2.3 | 0.7 | 0.9 | 1.2 | 1.5 | 0.8 | 2.7 | 2.0 | 1.4 | 1.3 | 4.3 | 0.6 | 1.7 | 0.7 | 0.9 | 0.9 | 3.24 |
| YER012W | 1.4 | 1.2 | 1.2 | 1.0 | 1.9 | 2.4 | 2.4 | 1.8 | 6.7 | 2.7 | 1.9 | 1.5 | 2.9 | 4.6 | 1.7 | 2.2 | 1.5 | 1.3 | 1.84 |
| YFR010W | 1.1 | 1.1 | 0.8 | 1.2 | 0.9 | 1.3 | 1.4 | 1.6 | 4.3 | 3.4 | 1.5 | 1.2 | 3.7 | 1.1 | 1.5 | 1.3 | 0.9 | 1.4 | 1.89 |
| YFR024C | 0.8 | 25.0 | 2.3 | 1.1 | 0.7 | 1.4 | 3.0 | 0.9 | 5.3 | 4.1 | 1.9 | 1.5 | 2.8 | 1.2 | 0.9 | 1.9 | 1.0 | 1.4 | 1.06 |
| YGL048C | 1.0 | 1.3 | 0.7 | 1.3 | 1.8 | 1.5 | 2.0 | 1.5 | 2.4 | 2.1 | 1.5 | 1.3 | 2.5 | 1.2 | 1.0 | 1.6 | 1.4 | 2.0 | 3.07 |
| YGL141W | 0.8 | 1.2 | 1.1 | 0.7 | 1.4 | 3.0 | 1.7 | 0.8 | 4.5 | 1.9 | 0.6 | 1.4 | 3.6 | 1.2 | 1.5 | 1.2 | 1.4 | 1.2 | 0.44 |
| YGL180W | 1.3 | 1.3 | 1.7 | 0.6 | 1.2 | 1.1 | 2.4 | 1.2 | 7.2 | 3.7 | 1.3 | 1.4 | 2.6 | 1.0 | 1.3 | 1.7 | 1.0 | 1.1 | 0.28 |
| YGR048W | 0.7 | 2.7 | 1.4 | 1.3 | 1.0 | 1.0 | 1.9 | 1.4 | 4.4 | 3.1 | 1.1 | 1.1 | 2.5 | 1.3 | 0.8 | 1.1 | 0.9 | 1.2 | 0.93 |
| YGR135W | 1.0 | 1.0 | 1.0 | 1.1 | 1.6 | 1.5 | 2.2 | 1.8 | 1.5 | 2.3 | 1.6 | 1.2 | 2.8 | 2.6 | 1.5 | 1.5 | 1.0 | 1.9 | 3.10 |
| YGR201C | 1.7 | 11.9 | 2.4 | 2.0 | 0.8 | 3.1 | 2.6 | 1.6 | 5.4 | 3.5 | 1.6 | 1.9 | 8.6 | 1.6 | 0.5 | 14.4 | 1.4 | 2.1 | 0.60 |
| YHL030W | 0.5 | 0.8 | 1.0 | 0.7 | 0.7 | 1.9 | 0.9 | 0.8 | 2.7 | 2.5 | 1.1 | 1.1 | 3.8 | 1.0 | 2.0 | 0.5 | 0.8 | 0.9 | 0.66 |
| YHR166C | 0.9 | 1.1 | 1.0 | 0.7 | 1.0 | 1.8 | 1.5 | 1.0 | 2.6 | 1.2 | 0.6 | 1.7 | 6.9 | 1.2 | 0.9 | 1.1 | 1.4 | 1.5 | 1.35 |
| YJR069C | 0.8 | 0.8 | 0.9 | 0.6 | 0.9 | 1.2 | 1.3 | 0.9 | 0.7 | 0.6 | 0.9 | 1.1 | 3.9 | 0.6 | 0.4 | 0.8 | 1.0 | 0.9 | 1.73 |
| YKL073W | 0.6 | 1.4 | 0.5 | 0.6 | 1.2 | 0.5 | 2.1 | 1.6 | 1.3 | 1.1 | 0.5 | 1.5 | 3.3 | 0.7 | 1.6 | 1.0 | 1.2 | 1.5 | 1.29 |
| YKL103C | 1.6 | 4.1 | 2.5 | 2.1 | 0.9 | 2.7 | 2.8 | 1.6 | 8.3 | 9.9 | 2.8 | 1.3 | 3.8 | 2.0 | 2.1 | 1.2 | 1.9 | 2.2 | 0.56 |

| | | | | | | | | | | | | | | | | | | | |
|---|---|---|---|---|---|---|---|---|---|---|---|---|---|---|---|---|---|---|---|
| YLR080W | 1.0 | 1.4 | 1.2 | 1.7 | 1.1 | 2.5 | 2.8 | 1.3 | 2.3 | 1.5 | 1.9 | 0.8 | 3.6 | 1.2 | 0.7 | 3.0 | 1.6 | 2.2 | 0.41 |
| YLR107W | 1.2 | 0.9 | 0.5 | 1.2 | 1.7 | 2.3 | 1.6 | 1.1 | 1.8 | 1.2 | 0.8 | 1.0 | 2.9 | 1.6 | 1.1 | 1.6 | 1.8 | 2.4 | 0.94 |
| YLR121C | 1.7 | 1.7 | 1.1 | 1.2 | 1.0 | 1.6 | | 1.3 | 2.2 | 2.0 | 2.2 | 1.1 | 4.0 | 1.2 | 10.7 | 1.5 | 2.7 | 1.4 | 0.30 |
| YLR336C | 0.6 | 0.8 | 0.7 | 0.4 | 0.9 | 0.8 | 1.8 | 1.0 | -0.3 | 0.5 | 0.5 | 1.9 | 4.6 | 0.6 | 0.8 | 1.3 | 1.5 | 1.0 | 0.71 |
| YLR370C | 1.1 | 1.0 | 1.1 | 1.4 | 1.4 | 4.1 | 2.1 | 0.8 | 1.5 | 1.9 | 1.0 | 1.2 | 3.1 | 1.9 | 1.5 | 1.6 | 1.6 | 2.0 | 1.41 |
| YLR423C | 1.2 | 1.2 | 0.7 | 1.9 | 1.5 | 1.2 | 3.8 | 2.0 | 1.2 | 1.0 | 1.0 | 1.7 | 3.9 | 1.2 | 1.3 | 1.9 | 1.2 | 1.6 | 0.34 |
| YML092C | 1.1 | 3.1 | 2.4 | 1.1 | 0.9 | 1.9 | 1.2 | 1.2 | 6.4 | 4.0 | 2.5 | 1.2 | 2.3 | 1.6 | 1.4 | 2.2 | 1.3 | 1.5 | 1.72 |
| YML130C | 1.5 | 2.7 | 5.3 | 1.2 | 0.9 | 0.6 | 2.9 | 2.7 | 7.8 | 4.2 | 1.9 | 2.3 | 5.8 | 1.3 | 2.4 | 0.8 | 1.2 | 1.2 | 1.59 |
| YMR214W | 0.8 | 3.8 | 1.0 | 1.4 | 0.8 | 0.9 | 1.0 | 1.1 | 2.1 | 1.0 | 0.7 | 1.0 | 2.4 | 1.1 | 4.8 | 0.7 | 1.1 | 1.0 | 0.88 |
| YMR297W | 0.7 | 2.0 | 3.6 | 1.9 | 0.6 | 1.8 | 1.2 | 0.8 | 1.9 | 1.0 | 2.5 | 1.2 | 4.2 | 0.5 | 2.4 | 1.9 | 1.1 | 1.4 | 4.10 |
| YNL036W | 2.0 | 1.9 | 3.2 | 1.0 | 1.6 | 1.1 | 2.0 | 2.1 | 6.6 | 5.0 | 1.7 | 1.4 | 3.3 | 5.1 | 1.6 | 1.3 | 1.4 | 1.9 | 2.04 |
| YOL005C | 1.4 | 1.4 | 1.1 | 0.8 | 1.6 | 1.1 | 1.1 | 1.0 | 0.9 | 1.0 | 1.6 | 1.2 | 2.9 | 1.2 | 0.9 | 1.0 | 1.2 | 1.2 | 1.71 |
| YOR134W | 2.0 | 3.6 | 1.0 | 9.5 | 0.9 | 1.4 | 1.1 | 1.5 | 1.5 | 8.2 | 1.9 | 2.8 | 4.4 | 1.3 | 1.6 | 7.7 | 2.2 | 4.6 | 0.28 |
| YOR362C | 1.0 | 1.7 | 1.0 | 0.8 | 1.7 | 1.4 | 1.6 | 1.4 | 4.3 | 3.0 | 1.3 | 1.3 | 2.5 | 1.4 | 1.0 | 1.5 | 1.1 | 1.8 | 2.55 |
| YAR009C | 0.8 | 1.3 | 0.6 | 1.0 | 1.4 | 1.6 | 2.7 | 1.4 | 0.9 | 1.4 | 0.9 | 1.3 | 2.0 | 1.0 | 2.1 | 0.6 | 0.8 | 1.2 | 7.11 |
| YBL101C | 1.0 | 1.1 | 1.9 | 0.9 | 1.0 | 0.9 | 0.8 | 0.8 | 3.0 | 1.4 | 0.8 | 1.1 | 3.5 | 0.9 | 2.7 | 1.4 | 1.0 | 1.1 | 0.39 |
| YBR046C | 1.4 | 1.4 | 1.7 | 1.8 | 1.3 | 2.6 | 3.9 | 1.6 | 4.6 | 6.2 | 2.9 | 1.2 | 2.0 | 1.3 | 2.1 | 3.0 | 1.2 | 2.0 | 0.63 |
| YBR139W | 1.2 | 1.4 | 3.1 | 1.4 | 0.8 | 2.4 | 1.9 | 0.7 | 2.3 | 1.6 | 2.3 | 1.2 | 2.7 | 1.1 | 5.6 | 2.3 | 1.2 | 2.0 | 1.21 |
| YBR170C | 0.8 | 1.2 | 0.6 | 1.4 | 1.5 | 1.4 | 1.9 | 1.3 | 6.1 | 4.2 | 1.5 | 1.2 | 2.7 | 1.3 | 1.9 | 1.3 | 1.1 | 1.4 | 0.72 |
| YBR177C | 0.8 | 1.1 | 4.0 | 1.2 | 1.2 | 2.3 | 0.9 | 0.9 | 2.1 | 1.7 | 0.6 | 1.2 | 3.1 | 1.3 | 1.4 | 0.4 | 0.8 | 0.9 | 0.76 |
| YBR212W | 0.7 | 1.2 | 2.6 | 1.2 | 1.0 | 1.4 | 0.9 | 0.6 | 2.7 | 2.3 | 1.1 | 0.9 | 2.2 | 0.7 | 1.0 | 1.5 | 1.1 | 1.0 | 0.95 |
| YBR239C | 1.0 | 1.3 | 0.9 | 1.5 | 0.8 | 1.5 | 1.0 | 1.3 | 0.4 | 1.2 | 0.7 | 1.1 | 1.9 | 1.1 | 1.0 | 2.7 | 1.0 | 0.9 | 0.31 |
| YCL033C | 1.1 | 2.0 | 1.7 | 1.7 | 1.2 | 4.6 | 2.4 | 1.1 | 2.3 | 1.4 | 1.9 | 1.6 | 2.0 | 1.8 | 0.8 | 3.6 | 2.0 | 2.0 | 1.14 |
| YCR062W | 0.8 | 1.9 | 2.7 | 2.8 | 1.4 | 1.9 | 0.9 | 0.7 | 0.7 | 1.3 | 1.2 | 1.3 | 2.2 | 1.0 | 2.3 | 1.1 | 1.1 | 1.0 | 0.41 |
| YCR067C | 0.9 | 0.7 | 1.2 | 1.3 | 0.7 | 0.8 | 0.8 | 0.7 | 1.8 | 1.6 | 1.2 | 0.9 | 2.0 | 0.9 | 2.1 | 1.4 | 1.1 | 0.9 | 0.62 |
| YDL020C | 2.3 | 1.2 | 3.4 | 1.1 | 1.2 | 2.5 | 1.0 | 2.2 | 5.6 | 2.9 | 1.9 | 1.1 | 3.4 | 1.2 | 1.5 | 1.8 | 1.5 | 1.7 | 1.36 |
| YDR168W | 1.2 | 1.4 | 1.0 | 0.8 | 1.5 | 1.3 | 2.4 | 2.1 | 3.7 | 2.3 | 1.5 | 1.2 | 2.4 | 1.4 | 1.9 | 1.4 | 1.2 | 1.4 | 1.28 |
| YDR169C | 1.0 | 1.4 | 1.1 | 1.3 | 1.3 | 1.2 | 1.1 | 1.0 | 1.6 | 1.4 | 0.9 | 0.9 | 1.9 | 0.9 | 1.4 | 0.9 | 1.4 | 1.1 | 0.40 |
| YDR188W | 0.8 | 1.2 | 1.0 | 0.8 | 0.8 | 0.8 | 0.9 | 1.0 | 2.9 | 1.6 | 0.9 | 0.9 | 1.7 | 0.7 | 1.3 | 0.9 | 0.7 | 0.8 | 1.78 |
| YDR264C | 0.5 | 2.6 | 0.9 | 1.4 | 0.9 | 1.8 | 3.2 | 0.8 | 1.1 | 3.4 | 0.9 | 1.1 | 2.1 | 0.7 | 2.3 | 1.5 | 1.2 | 1.4 | 1.63 |
| YDR304C | 1.0 | 1.8 | 2.7 | 1.3 | 1.5 | 2.1 | 2.0 | 1.3 | 3.9 | 1.9 | 1.2 | 1.3 | 2.3 | 1.7 | 1.0 | 4.0 | 1.6 | 1.8 | 2.23 |
| YDR403W | 1.0 | 1.2 | 1.6 | 1.0 | 1.2 | 1.1 | 1.2 | 1.2 | 4.1 | 3.6 | 1.0 | 1.0 | 2.0 | 1.1 | 0.9 | 0.9 | 0.9 | 1.0 | 0.35 |

| | | | | | | | | | | | | | | | | | | | |
|---|---|---|---|---|---|---|---|---|---|---|---|---|---|---|---|---|---|---|---|
| YDR427W | 0.8 | 1.1 | 0.8 | 0.8 | 1.5 | 1.9 | 1.4 | 1.2 | 2.3 | 2.0 | 1.2 | 1.1 | 2.1 | 1.0 | 1.3 | 1.1 | 1.0 | 1.5 | 2.37 |
| YEL012W | 1.3 | 1.8 | 1.0 | 1.8 | 1.7 | 1.6 | 4.7 | 1.4 | 4.6 | 4.2 | 2.2 | 1.2 | 2.2 | 1.9 | 1.1 | 2.5 | 1.5 | 2.2 | 0.74 |
| YER009W | 1.3 | 0.6 | 2.4 | 1.2 | 1.2 | 2.0 | 2.8 | 1.0 | 0.7 | 1.0 | 1.4 | 1.1 | 2.0 | 1.6 | 0.9 | 1.5 | 2.0 | 2.1 | 3.35 |
| YER021W | 0.8 | 1.0 | 0.9 | 0.9 | 1.4 | 1.1 | 1.5 | 1.2 | 3.0 | 2.2 | 1.3 | 1.0 | 1.8 | 1.3 | 0.8 | 1.4 | 0.8 | 1.5 | 2.02 |
| YER094C | 1.0 | 1.3 | 0.9 | 1.1 | 1.9 | 1.1 | 1.8 | 1.3 | 2.3 | 1.7 | 1.3 | 1.1 | 2.1 | 1.5 | 0.8 | 1.5 | 1.3 | 1.8 | 3.01 |
| YER177W | 1.4 | 1.5 | 2.1 | 1.5 | 0.9 | 1.6 | 1.2 | 1.1 | 1.8 | 1.7 | 1.9 | 1.1 | 2.2 | 0.9 | 1.4 | 1.0 | 0.9 | 2.0 | 5.81 |
| YFL029C | 1.0 | 1.2 | 1.2 | 1.8 | 1.7 | 1.0 | 1.6 | 1.5 | 4.3 | 2.9 | 2.1 | 1.1 | 1.8 | 1.9 | 1.3 | 1.2 | 1.2 | 1.4 | 0.49 |
| YFL038C | 1.0 | 1.2 | 0.7 | 1.3 | 1.5 | 1.3 | 1.7 | 1.4 | 2.1 | 1.9 | 1.7 | 1.1 | 2.1 | 1.5 | 1.5 | 2.1 | 1.2 | 1.6 | 2.31 |
| YFR004W | 1.0 | 1.5 | 0.8 | 1.4 | 1.0 | 1.5 | 1.3 | 1.4 | 3.6 | 2.6 | 1.3 | 1.2 | 2.0 | 2.1 | 1.1 | 1.3 | 1.0 | 1.4 | 2.44 |
| YFR050C | 0.9 | 1.2 | 1.1 | 1.3 | 1.7 | 1.5 | 1.4 | 1.4 | 3.1 | 2.1 | 1.4 | 1.5 | 2.1 | 1.6 | 1.5 | 1.5 | 1.1 | 1.7 | 1.70 |
| YGL011C | 1.2 | 1.5 | 1.1 | 1.1 | 1.8 | 1.6 | 1.5 | 1.3 | 2.0 | 2.2 | 1.5 | 1.0 | 2.1 | 3.0 | 1.3 | 1.7 | 1.3 | 1.5 | 1.78 |
| YGL094C | 0.8 | 1.3 | 0.3 | 0.9 | 1.3 | 1.0 | 0.8 | 1.0 | 1.4 | 1.1 | 0.6 | 0.8 | 2.3 | 0.9 | 1.9 | 1.0 | 1.0 | 0.9 | 0.51 |
| YGL150C | 0.7 | 0.8 | 1.2 | 1.0 | 0.8 | 1.1 | 0.8 | 0.8 | 1.4 | 0.9 | 0.4 | 0.8 | 2.4 | 1.0 | 1.3 | 0.9 | 0.9 | 0.8 | 0.62 |
| YGL207W | 0.5 | 0.6 | 0.9 | 0.6 | 1.3 | 0.9 | 0.6 | 1.0 | 1.4 | 1.1 | 0.7 | 0.7 | 2.5 | 0.8 | 1.2 | 0.7 | 0.9 | 0.8 | 0.88 |
| YGR232W | 1.2 | 1.2 | 1.2 | 1.7 | 1.5 | 1.9 | 2.0 | 1.1 | 3.4 | 2.2 | 1.3 | 1.1 | 2.4 | 2.1 | 0.9 | 1.8 | 1.3 | 1.2 | 0.96 |
| YGR248W | 1.3 | 1.7 | 3.2 | 4.0 | 0.5 | 2.3 | 1.1 | 1.2 | 2.8 | 3.2 | 2.3 | 1.1 | 2.0 | 1.5 | 1.5 | 7.9 | 1.0 | 1.5 | 0.52 |
| YGR253C | 1.3 | 1.4 | 0.7 | 1.3 | 2.0 | 1.4 | 1.6 | 2.0 | 4.0 | 3.1 | 2.4 | 1.1 | 3.4 | 1.8 | 1.1 | 1.8 | 1.5 | 2.1 | 2.01 |
| YHR027C | 0.5 | 0.8 | 1.2 | 0.6 | 1.1 | 0.4 | 0.9 | 0.6 | 3.6 | 2.2 | 1.1 | 1.0 | 2.1 | 0.7 | 1.5 | 0.9 | 0.8 | 1.0 | 2.06 |
| YHR161C | 0.6 | 1.1 | 1.3 | 2.1 | 1.5 | 1.2 | 1.8 | 0.7 | 2.5 | 2.0 | 1.2 | 1.0 | 1.8 | 1.0 | 1.4 | 1.7 | 1.4 | 1.5 | 0.86 |
| YHR169W | 0.6 | 0.5 | 0.4 | 0.4 | 1.1 | 1.2 | 0.9 | 0.9 | 0.0 | 0.2 | 0.4 | 1.1 | 3.3 | 1.1 | 0.6 | 1.0 | 1.6 | 1.3 | 0.81 |
| YIL010W | 1.0 | 1.2 | 1.4 | 0.8 | 1.2 | 4.4 | 1.5 | 0.9 | 1.4 | 2.2 | 1.4 | 1.4 | 2.3 | 1.7 | 1.1 | 1.7 | 1.5 | 1.5 | 0.70 |
| YIL034C | 1.0 | 0.8 | 1.5 | 3.0 | 0.7 | 1.7 | 1.2 | 0.9 | 2.1 | 1.3 | 1.6 | 1.0 | 2.6 | 1.1 | 1.0 | 1.7 | 1.2 | 1.5 | 0.99 |
| YIL142W | 0.7 | 1.1 | 0.9 | 1.1 | 0.8 | 1.1 | 0.8 | 1.1 | 4.4 | 3.0 | 1.7 | 1.1 | 1.8 | 1.0 | 1.3 | 0.7 | 0.9 | 0.8 | 1.84 |
| YIR039C | 1.4 | 1.8 | 4.3 | 4.2 | 1.5 | 3.2 | 4.1 | 1.1 | 4.4 | 4.1 | 3.8 | 1.4 | 2.9 | 1.9 | 2.0 | 4.2 | 1.2 | 1.1 | 0.45 |
| YJL001W | 0.9 | 1.7 | 1.2 | 2.0 | | 1.7 | 1.6 | 1.3 | 3.1 | 2.8 | 1.5 | 1.6 | 2.9 | 2.5 | 1.2 | 1.8 | 1.0 | 1.6 | 3.00 |
| YJL035C | 0.9 | 1.3 | 0.8 | 0.6 | 1.4 | 0.7 | 1.3 | 1.5 | 1.7 | 1.4 | 0.8 | 1.1 | 2.4 | 1.6 | 0.7 | 0.6 | 1.0 | 1.0 | 0.82 |
| YJL053W | 1.1 | 0.9 | 1.7 | 1.3 | 1.1 | 2.0 | 1.7 | 1.3 | 2.2 | 2.2 | 1.6 | 1.1 | 1.9 | 1.3 | 1.1 | 1.7 | 1.3 | 1.6 | 0.80 |
| YJL164C | 1.1 | 1.1 | 1.0 | 1.9 | 1.1 | 2.7 | 3.1 | 0.9 | 2.1 | 0.8 | 1.5 | 1.0 | 2.1 | 0.9 | 0.8 | 2.1 | 1.3 | 1.7 | 1.04 |
| YJL210W | 1.2 | 1.4 | 1.6 | 2.0 | 0.6 | 0.6 | 1.0 | 1.0 | 2.0 | 0.9 | 2.4 | 1.0 | 2.2 | 0.9 | 0.8 | 2.3 | 0.9 | 1.0 | 0.70 |
| YJR117W | 1.0 | 2.1 | 2.3 | 0.7 | 1.2 | 1.3 | 1.0 | 0.8 | 4.7 | 2.3 | 1.0 | 1.2 | 2.8 | 0.8 | 2.2 | 0.7 | 0.8 | 0.9 | 1.34 |
| YKL007W | 1.2 | 1.6 | 1.2 | 0.9 | 1.0 | 0.9 | 1.5 | 1.2 | 2.3 | 2.4 | 1.8 | 1.3 | 2.5 | 0.7 | 1.0 | 1.0 | 1.4 | 1.3 | 1.13 |
| YKL117W | 1.0 | 1.3 | 0.8 | 1.0 | 1.8 | 1.0 | 1.3 | 1.5 | 2.9 | 2.1 | 1.6 | 1.0 | 2.4 | 1.6 | 1.6 | 1.8 | 1.2 | 1.8 | 3.73 |

| | | | | | | | | | | | | | | | | | | | |
|---|---|---|---|---|---|---|---|---|---|---|---|---|---|---|---|---|---|---|---|
| YKL193C | 1.3 | 0.8 | 1.1 | 1.4 | 1.1 | 0.9 | 2.1 | 1.4 | 1.0 | 1.6 | 1.7 | 0.9 | 2.1 | 1.2 | 1.2 | 2.4 | 1.2 | 2.3 | 0.71 |
| YLR120C | 1.2 | 1.9 | 2.4 | 1.6 | 1.1 | 1.9 | 1.5 | 0.9 | 1.8 | 1.6 | 1.2 | 1.6 | 4.8 | 0.8 | 2.7 | 1.7 | 3.5 | 2.9 | 1.53 |
| YLR136C | 1.3 | 9.0 | 0.6 | 3.3 | 1.6 | 1.7 | 3.9 | 1.1 | 0.9 | 6.0 | 1.2 | 1.3 | 2.9 | 0.7 | 2.1 | 1.2 | 1.8 | 1.4 | 0.41 |
| YLR178C | 1.7 | 6.9 | 8.8 | 4.6 | 1.0 | 4.2 | 3.3 | 0.9 | 4.3 | 4.1 | 3.7 | 2.2 | 7.5 | 2.8 | 2.3 | 10.6 | 2.0 | 3.6 | 1.03 |
| YLR327C | 5.5 | 3.3 | 14.4 | 8.2 | 1.3 | 5.1 | 5.7 | 2.2 | 3.0 | 8.3 | 5.0 | 1.4 | 2.8 | 1.5 | 0.8 | 2.8 | 3.3 | 5.3 | 2.23 |
| YLR356W | 1.0 | 2.9 | 4.9 | 1.2 | 1.1 | 3.4 | 1.1 | 0.7 | 2.3 | 1.6 | 3.1 | 1.5 | 2.5 | 2.2 | 2.8 | 0.9 | 1.1 | 1.0 | 0.41 |
| YLR362W | 1.1 | 1.2 | 1.0 | 3.0 | 1.5 | 0.8 | 1.9 | 1.4 | 3.5 | 3.3 | 1.5 | 0.8 | 2.4 | 1.2 | 1.3 | 1.9 | 1.2 | 1.3 | 0.35 |
| YLR429W | 0.7 | 1.0 | 0.6 | 0.9 | 1.4 | 0.8 | 1.3 | 1.0 | 0.9 | 1.3 | 0.9 | 1.1 | 2.3 | 0.7 | 1.5 | 1.0 | 1.0 | 0.9 | 0.86 |
| YMR004W | 0.9 | 0.9 | 1.3 | 1.3 | 1.0 | 0.5 | 1.2 | 1.0 | 94.2 | 3.4 | 2.5 | 1.0 | 2.2 | 2.2 | 1.3 | 1.3 | 0.9 | 1.3 | 0.52 |
| YMR219W | 0.7 | 1.1 | 1.0 | 0.7 | 1.6 | 0.8 | | 1.1 | 2.3 | 0.9 | 1.5 | 1.5 | 2.1 | 0.5 | 1.7 | 0.8 | 1.1 | 0.9 | 0.23 |
| YMR275C | 0.7 | 0.7 | 1.2 | 0.6 | 0.7 | 1.4 | 0.8 | 0.8 | 2.4 | 2.1 | 1.1 | 0.7 | 2.1 | 0.8 | 1.7 | 1.0 | 0.9 | 0.7 | 0.79 |
| YMR314W | | 1.2 | 0.9 | 1.3 | | 1.3 | 1.1 | 1.2 | 5.9 | 2.7 | 1.8 | 1.0 | 2.6 | 1.0 | 1.2 | 1.6 | 1.3 | 1.6 | 1.67 |
| YNL006W | 1.1 | 2.1 | 1.3 | 1.5 | 0.9 | 1.0 | 1.6 | 1.1 | 4.0 | 4.0 | 1.1 | 0.7 | 2.3 | 0.8 | 0.7 | 0.9 | 1.0 | 1.3 | 1.20 |
| YNL007C | 0.7 | 2.4 | 5.1 | 0.8 | 1.2 | 0.6 | 1.2 | 0.8 | 3.0 | 1.7 | 1.3 | 0.7 | 2.1 | 0.4 | 0.8 | 0.5 | 1.1 | 1.0 | 3.62 |
| YNL093W | 2.1 | 6.4 | 0.4 | 4.5 | 1.2 | 1.6 | 1.4 | 0.8 | 1.7 | 2.0 | 1.6 | 1.1 | 2.2 | 1.1 | 0.6 | 4.9 | 3.0 | 3.1 | 0.32 |
| YNL333W | 1.0 | 1.4 | 1.7 | 2.3 | 1.5 | 1.5 | 2.9 | 2.1 | 2.3 | 2.1 | 2.0 | 1.0 | 2.2 | 1.9 | 1.0 | 1.4 | 1.4 | 1.1 | 0.49 |
| YNR010W | 1.3 | 1.2 | 0.5 | 2.8 | 1.8 | 1.3 | 1.5 | 1.1 | 1.7 | 0.9 | 1.4 | 0.8 | 2.8 | 1.3 | 0.8 | 1.2 | 1.7 | 2.3 | 0.37 |
| YNR069C | 1.5 | 1.3 | 0.7 | 0.4 | 1.4 | 1.7 | 1.4 | 1.4 | 5.5 | 5.8 | 2.5 | 1.2 | 3.4 | 2.1 | 1.0 | 0.8 | 1.1 | 0.9 | 0.18 |
| YOL164W | 0.9 | 4.4 | 1.0 | 1.5 | 1.6 | 1.2 | 2.2 | 1.0 | 4.9 | 3.6 | 1.0 | 1.1 | 3.4 | 1.3 | 1.0 | 1.1 | 1.1 | 1.3 | 0.45 |
| YOR036W | 1.8 | 0.9 | 1.4 | 1.6 | 1.1 | 1.7 | 1.8 | 1.4 | 1.2 | 1.9 | 1.3 | 1.2 | 2.1 | 1.2 | 1.2 | 1.3 | 1.6 | 1.7 | 0.98 |
| YOR117W | 0.8 | 1.3 | 1.7 | 0.8 | 1.2 | 1.1 | 3.3 | 1.0 | 3.5 | 1.8 | 1.2 | 2.5 | 2.3 | 1.0 | 1.4 | 1.2 | 1.4 | 0.9 | 1.42 |
| YOR124C | 0.6 | 1.1 | 1.3 | 0.8 | 1.1 | 0.9 | 1.1 | 1.3 | 2.0 | 1.9 | 1.2 | 0.9 | 2.1 | 0.8 | 1.3 | 0.9 | 1.0 | 1.0 | 0.65 |
| YOR132W | 0.9 | 1.6 | 0.8 | 2.8 | 1.1 | 1.6 | 1.4 | 1.2 | 2.8 | 1.6 | 1.3 | 0.7 | 2.5 | 0.7 | 1.5 | 1.6 | 1.2 | 1.6 | 0.46 |
| YOR157C | 1.0 | 1.3 | 1.2 | 1.3 | 0.7 | 1.2 | 1.2 | 1.2 | 5.7 | 2.6 | 1.6 | 1.3 | 2.4 | 2.2 | 1.9 | 0.9 | 1.2 | 1.2 | 1.23 |
| YOR185C | 1.1 | 1.3 | 1.9 | 1.1 | 1.2 | 3.0 | 1.3 | 1.4 | 1.9 | 2.9 | 1.2 | 1.5 | 2.2 | 2.1 | 1.0 | 2.2 | 0.9 | 1.1 | 1.67 |
| YOR259C | 0.8 | 1.1 | 0.8 | 0.9 | 2.0 | 0.8 | 1.3 | 1.1 | 2.7 | 1.5 | 1.0 | 0.8 | 1.8 | 1.2 | 1.3 | 1.4 | 0.9 | 1.5 | 2.33 |
| YOR261C | 1.0 | 1.4 | 0.6 | 0.9 | 1.5 | 1.4 | 1.9 | 2.6 | 5.5 | 2.5 | 2.1 | 0.9 | 2.5 | 1.8 | 1.3 | 2.0 | 1.0 | 1.9 | 2.80 |
| YOR288C | 0.9 | 1.3 | 1.1 | 1.1 | 1.1 | 1.0 | 2.0 | 1.9 | 0.9 | 1.0 | 1.4 | 1.4 | 2.4 | 0.8 | 1.8 | 1.4 | 1.5 | 1.6 | 0.79 |
| YPL109C | 1.1 | 2.5 | 0.5 | 1.2 | 1.2 | 1.2 | 1.8 | 1.1 | 1.2 | 1.5 | -0.2 | 0.9 | 1.8 | 1.3 | 1.3 | 1.9 | 1.2 | 1.2 | 0.21 |
| YPL149W | 1.5 | 1.5 | 0.6 | 2.8 | 0.8 | 0.9 | 1.2 | 1.1 | 5.6 | 3.7 | 1.2 | 0.9 | 2.3 | 1.0 | 0.8 | 1.2 | 2.2 | 2.9 | 1.39 |
| YPL154C | 0.7 | 3.3 | 4.2 | 1.2 | 1.2 | 3.5 | 1.5 | 0.8 | 2.8 | 1.7 | 1.8 | 1.5 | 3.1 | 0.8 | 4.0 | 1.8 | 1.6 | 2.0 | 3.78 |
| YPR103W | 0.7 | 1.1 | 1.3 | 0.7 | 1.7 | 1.2 | 1.6 | 1.0 | 5.5 | 2.0 | 0.9 | 1.2 | 2.8 | 1.8 | 1.0 | 1.1 | 1.2 | 1.5 | 1.93 |

| | | | | | | | | | | | | | | | | | | | |
|---|---|---|---|---|---|---|---|---|---|---|---|---|---|---|---|---|---|---|---|
| YPR108W | 0.8 | 1.0 | 1.3 | 0.6 | 1.3 | 1.5 | 1.6 | 1.6 | 3.7 | 2.8 | 1.8 | 1.0 | 2.6 | 1.0 | 1.3 | 1.2 | 1.0 | 1.3 | 2.25 |
| YCL027W | 0.7 | 2.0 | 1.0 | 1.0 | 1.2 | 1.3 | 1.0 | 0.6 | 0.7 | 16.3 | 0.9 | 0.8 | 1.1 | 1.2 | 3.6 | 1.1 | 0.7 | 0.8 | 0.30 |
| YDR055W | 1.7 | 2.1 | 2.2 | 2.8 | 1.7 | 3.9 | 1.5 | 1.6 | 0.8 | 2.4 | 2.2 | 1.4 | 3.1 | 0.9 | 5.7 | 3.4 | 2.0 | 1.9 | 1.55 |
| YEL042W | 0.6 | 0.9 | 1.7 | 0.9 | 0.7 | 0.5 | 0.5 | 0.7 | 0.2 | 0.3 | 1.6 | 1.0 | 0.4 | 0.6 | 4.8 | 0.6 | 0.7 | 0.8 | 1.21 |
| YGR136W | 0.9 | 1.2 | 1.3 | 0.7 | 1.2 | 1.1 | 1.5 | 1.3 | 2.1 | 2.2 | 1.3 | 1.2 | 1.7 | 1.1 | 2.6 | 2.2 | 1.3 | 1.8 | 1.42 |
| YHR142W | 1.5 | 0.8 | 2.1 | 0.8 | 1.3 | 1.2 | 0.9 | 1.3 | 0.4 | 0.9 | 1.1 | 1.3 | 1.2 | 1.2 | 4.8 | 1.0 | 1.3 | 1.3 | 1.17 |
| YJL073W | 0.8 | 0.8 | 2.5 | 0.7 | 1.2 | 2.0 | | 1.0 | 1.7 | 0.7 | 0.5 | 1.0 | 1.6 | 1.1 | 2.6 | 0.8 | 1.1 | 1.0 | 0.43 |
| YJR004C | 0.2 | 0.8 | 3.4 | 0.6 | 1.1 | 1.2 | 0.4 | 0.4 | 0.3 | 0.7 | 0.7 | 0.8 | 0.3 | 0.7 | 3.2 | 0.3 | 0.4 | 0.4 | 2.12 |
| YKL039W | 0.8 | 1.1 | 2.4 | 0.8 | 0.7 | 4.9 | 2.0 | 0.8 | 1.3 | 3.3 | 1.0 | 0.9 | 1.9 | 0.8 | 3.1 | 1.5 | 1.2 | 1.3 | 1.13 |
| YLR250W | 1.5 | 1.0 | 1.0 | 1.4 | 1.4 | 1.1 | 1.9 | 1.3 | 2.7 | 2.4 | 1.4 | 1.0 | 1.2 | 2.5 | 2.6 | 2.1 | 1.4 | 2.2 | 2.90 |
| YOR181W | 1.0 | 0.9 | 1.1 | 1.4 | 0.7 | 0.9 | 0.7 | 0.7 | 0.6 | 0.5 | 1.0 | 0.8 | 1.2 | 0.9 | 3.4 | 1.0 | 1.1 | 0.6 | 0.39 |
| YOR198C | 1.0 | 0.7 | 1.4 | 1.0 | 0.8 | 0.9 | 1.6 | 1.3 | 2.4 | 1.4 | 1.4 | 1.5 | 1.8 | 1.1 | 3.8 | 0.8 | 1.1 | 1.5 | 1.76 |
| YPL089C | 1.2 | 1.0 | 0.5 | 1.8 | 1.4 | 0.8 | 0.9 | 0.8 | 0.4 | 2.5 | 0.9 | 0.7 | 1.3 | 0.8 | 2.6 | 0.9 | 1.5 | 1.1 | 0.37 |
| YBR214W | 0.9 | 2.1 | 5.7 | 2.0 | 1.1 | 0.8 | 1.1 | 0.7 | 2.2 | 3.5 | 3.2 | 1.2 | 2.1 | 0.9 | 5.1 | 1.3 | 1.1 | 1.2 | 0.51 |
| YDR085C | 1.9 | 1.8 | 1.6 | 3.4 | 0.9 | 2.1 | 4.1 | 2.0 | 0.4 | 2.3 | 1.7 | 1.6 | 1.1 | 1.6 | 3.1 | 2.3 | 1.3 | 1.8 | 0.37 |
| YDR259C | 1.4 | 1.0 | 0.4 | 1.4 | 1.0 | 0.5 | 2.8 | 1.4 | 1.0 | 1.8 | 1.2 | 1.0 | 2.0 | 1.5 | 2.9 | 0.9 | 1.0 | 0.8 | 0.28 |
| YDR388W | 0.7 | 0.7 | 1.2 | 1.0 | 0.7 | 1.0 | 1.1 | 0.8 | 1.4 | 0.9 | 1.6 | 0.8 | 1.7 | 0.6 | 2.6 | 1.0 | 1.2 | 1.3 | 0.99 |
| YDR432W | 0.5 | 0.8 | 1.0 | 1.4 | 1.6 | 0.6 | 0.5 | 0.5 | 0.7 | 0.8 | 0.6 | 0.7 | 1.4 | 0.5 | 2.6 | 0.6 | 0.7 | 0.7 | 2.38 |
| YDR481C | 0.7 | 2.3 | 1.3 | 0.7 | 1.0 | 0.9 | 0.7 | 1.0 | 1.9 | 1.6 | 1.2 | 0.9 | 1.5 | 0.9 | 3.5 | 1.3 | 1.2 | 1.4 | 1.43 |
| YDR510W | 1.0 | 1.3 | 1.5 | 1.3 | 0.7 | 1.0 | 1.6 | 1.5 | 2.7 | 2.5 | 1.6 | 1.4 | 2.0 | 1.9 | 2.2 | 1.5 | 1.1 | 1.5 | 1.57 |
| YGR189C | 1.1 | 0.9 | 1.5 | 3.5 | 0.7 | 0.5 | 0.6 | 1.0 | 0.3 | 0.9 | 1.5 | 1.3 | 1.2 | 0.9 | 9.3 | 0.6 | 2.3 | 1.7 | 1.51 |
| YIL123W | 1.0 | 0.5 | 1.2 | 1.5 | 0.6 | 0.5 | 0.5 | 0.6 | 0.1 | 0.2 | 1.2 | 0.8 | 0.4 | 0.3 | 4.8 | 0.7 | 1.3 | 1.0 | 1.67 |
| YIL140W | 0.7 | 1.0 | 1.5 | 1.4 | 0.8 | 0.5 | 0.5 | 0.7 | 0.7 | 0.6 | 0.9 | 1.0 | 0.5 | 0.9 | 3.3 | 0.6 | 0.7 | 1.0 | 0.62 |
| YKL096W | 1.4 | 0.6 | 8.1 | 1.6 | 0.6 | 0.6 | 0.2 | 0.5 | 0.0 | 0.5 | 3.5 | 0.3 | 0.1 | 0.5 | 2.3 | 0.4 | 0.5 | 0.7 | 2.04 |
| YLR391W | | 0.7 | 2.9 | 0.8 | 1.2 | 1.5 | 0.7 | 0.7 | 0.4 | 0.7 | 1.4 | 1.0 | 0.9 | 0.5 | 3.9 | 1.3 | 1.4 | 0.9 | 1.72 |
| YMR094W | 1.0 | 0.5 | 1.3 | 1.7 | 0.9 | 0.5 | 0.8 | 1.0 | -0.1 | 0.9 | 1.0 | 0.9 | 1.4 | 2.8 | 6.6 | 1.0 | 1.0 | 1.0 | 0.23 |
| YMR104C | 1.9 | 14.3 | 2.2 | 2.6 | 0.7 | 1.9 | 1.5 | 0.9 | 0.6 | 3.2 | 1.1 | 1.0 | 1.5 | 1.1 | 2.5 | 2.3 | 2.8 | 1.9 | 0.51 |
| YMR276W | 0.6 | 1.4 | 1.5 | 0.7 | 1.3 | 0.8 | 1.1 | 0.8 | 1.8 | 1.7 | 1.9 | 1.2 | 1.9 | 1.2 | 3.3 | 0.9 | 1.2 | 0.7 | 0.47 |
| YOL013C | 0.9 | 1.0 | 1.0 | 1.5 | 0.7 | 1.1 | 1.7 | 1.0 | 0.8 | 1.0 | 1.4 | 1.1 | 1.8 | 0.9 | 2.7 | 1.3 | 1.1 | 1.4 | 0.65 |
| YOR355W | 0.7 | 0.6 | 1.1 | 0.8 | 1.0 | 1.6 | 1.1 | 0.8 | 0.1 | 0.8 | 1.2 | 0.9 | 1.1 | 0.6 | 3.2 | 0.8 | 0.8 | 0.9 | 0.98 |
| YCR071C | 1.1 | 0.7 | 0.9 | 0.8 | 1.2 | 2.5 | 1.4 | 0.9 | 0.6 | 1.2 | 1.1 | 1.1 | 1.3 | 1.7 | 1.1 | 1.5 | 1.2 | 1.3 | 0.96 |
| YDL008W | 1.1 | 1.0 | 1.0 | 1.1 | 1.8 | 2.7 | 2.0 | 1.2 | 2.3 | 3.0 | 0.9 | 1.2 | 2.0 | 2.2 | 0.7 | 1.3 | 1.5 | 1.6 | 0.95 |

| | | | | | | | | | | | | | | | | | | | |
|---|---|---|---|---|---|---|---|---|---|---|---|---|---|---|---|---|---|---|---|
| YDR115W | 1.3 | 0.9 | 0.6 | 1.5 | 1.4 | 2.3 | 1.6 | 1.3 | 1.1 | 1.0 | 1.2 | 0.9 | 1.1 | 1.5 | 0.8 | 2.2 | 1.2 | 1.4 | 1.50 |
| YER130C | 0.9 | 1.6 | 0.5 | 0.8 | 1.7 | 2.7 | 1.5 | 1.2 | 0.5 | 1.3 | 0.9 | 1.0 | 1.6 | 0.8 | 1.1 | 0.7 | 3.0 | 2.5 | 1.36 |
| YMR226C | 1.1 | 1.8 | 1.4 | 1.6 | 0.9 | 2.6 | 1.8 | 1.6 | 2.6 | 2.2 | 1.4 | 1.1 | 1.6 | 1.6 | 2.0 | 1.7 | 1.6 | 2.0 | 2.03 |
| YOR383C | 0.9 | 4.3 | 2.6 | 0.9 | 0.9 | 6.8 | 8.0 | 1.0 | 1.2 | 4.5 | 1.4 | 1.1 | 1.2 | 0.6 | 1.1 | 3.0 | 1.4 | 1.1 | 0.68 |
| YAR010C | 0.8 | 1.2 | 1.2 | 0.8 | 1.0 | 2.3 | 1.9 | 1.3 | 1.2 | 1.1 | 0.9 | 1.0 | 1.4 | 1.0 | 1.8 | 0.7 | 1.5 | 1.0 | 4.69 |
| YBL043W | 1.2 | 1.2 | 1.1 | 1.3 | 1.1 | 26.9 | 1.5 | 2.1 | 0.5 | 14.7 | 1.5 | 0.9 | 1.8 | 1.9 | 1.8 | 2.3 | 2.3 | 1.8 | 0.47 |
| YCR004C | 1.7 | 1.0 | 12.0 | | 1.2 | 1.8 | 1.4 | 1.2 | 4.7 | 2.1 | 1.9 | 0.8 | 1.3 | 3.7 | 0.8 | 4.9 | 1.4 | 3.5 | 3.02 |
| YCR088W | 0.9 | 1.1 | 1.2 | 0.9 | 1.1 | 2.0 | 1.2 | 0.5 | 0.8 | 0.9 | 1.0 | 1.3 | 1.9 | 0.9 | 2.8 | 0.7 | 1.4 | 0.9 | 0.47 |
| YDL238C | 0.7 | 1.0 | 1.1 | 1.7 | 0.9 | 2.1 | 1.4 | 5.8 | 1.7 | 2.7 | 1.1 | 0.9 | 2.0 | 1.2 | 1.3 | 1.7 | 1.1 | 1.3 | 0.35 |
| YDR084C | 0.9 | 0.9 | 1.5 | 1.1 | 1.2 | 2.1 | 0.9 | 0.7 | 0.6 | 0.6 | 0.7 | 0.7 | 1.0 | 1.0 | 3.9 | 1.0 | 2.0 | 1.3 | 1.12 |
| YDR104C | 0.8 | 1.1 | 1.2 | 1.2 | 1.3 | 1.7 | 1.0 | 1.3 | 2.8 | 2.7 | 1.0 | 1.1 | 1.6 | 1.4 | 1.4 | 0.9 | 0.9 | 1.0 | 0.34 |
| YDR315C | 1.0 | 1.4 | 1.0 | 1.3 | 0.9 | 9.4 | 1.4 | 1.4 | 1.1 | 1.6 | 1.1 | 0.6 | 1.6 | 1.4 | 1.5 | 1.5 | 1.2 | 1.2 | 0.48 |
| YDR358W | 0.7 | 1.1 | 0.7 | 1.1 | 1.4 | 2.5 | 2.8 | 1.2 | 2.9 | 3.2 | 1.0 | 1.1 | 3.2 | 1.2 | 0.9 | 2.2 | 1.9 | 1.9 | 0.50 |
| YEL066W | 2.0 | 2.9 | 1.5 | 2.2 | 0.8 | 2.2 | 1.8 | 1.4 | 1.3 | 2.4 | 1.9 | 0.8 | 1.6 | 1.9 | 1.0 | 1.9 | 1.0 | 1.1 | 0.64 |
| YER039C | 1.0 | 1.2 | 1.0 | 4.1 | 1.5 | 1.6 | 1.3 | 0.7 | 1.2 | 0.8 | 1.2 | 0.9 | 2.1 | 1.2 | 1.2 | 1.8 | 1.7 | 1.1 | 0.42 |
| YER107C | 0.9 | 0.5 | 1.0 | 0.6 | 1.3 | 2.5 | 1.0 | 0.8 | 0.4 | 0.5 | 0.7 | 0.7 | 0.9 | 1.1 | 0.7 | 0.9 | 1.1 | 1.2 | 0.92 |
| YFL028C | 1.2 | 1.1 | 0.7 | 1.5 | 1.7 | 1.7 | 2.0 | 1.5 | 2.7 | 1.8 | 1.3 | 1.1 | 1.4 | 1.6 | 0.8 | 1.9 | 1.2 | 1.5 | 1.28 |
| YFL043C | 1.0 | 1.2 | 0.6 | 1.2 | 1.2 | 1.8 | 1.8 | 1.0 | 2.2 | 3.8 | 1.1 | 0.9 | 1.5 | 1.5 | 1.1 | 2.0 | 1.5 | 1.2 | 0.38 |
| YGL229C | 0.6 | 0.7 | 0.9 | 0.8 | 1.0 | 2.2 | 2.2 | 0.7 | 1.8 | 1.7 | 1.1 | 1.1 | 1.7 | 1.0 | 0.2 | 1.8 | 1.1 | 0.9 | 0.44 |
| YGR257C | 0.7 | 0.8 | 0.9 | 1.1 | 1.3 | 2.1 | 2.4 | 1.0 | 1.5 | 1.9 | 1.2 | 0.9 | 2.0 | 0.9 | 1.3 | 1.3 | 1.1 | 1.3 | 0.84 |
| YHR004C | 0.9 | 1.1 | 0.8 | 1.1 | 1.5 | 2.0 | 1.5 | 0.9 | 1.1 | 0.6 | 0.8 | 0.9 | 1.1 | 0.8 | 1.2 | 2.4 | 1.4 | 1.6 | 0.84 |
| YHR071W | 1.5 | 1.1 | 0.6 | 1.6 | 1.5 | 2.0 | 1.0 | 1.7 | 1.0 | 7.2 | 1.8 | 0.8 | 2.3 | 1.6 | 3.0 | 1.7 | 2.6 | 1.0 | 0.79 |
| YJL089W | 1.1 | 1.5 | 1.0 | 0.8 | 1.1 | 1.6 | 1.1 | 1.1 | 1.6 | 3.9 | 0.6 | 1.2 | 1.8 | 1.8 | 6.4 | 1.2 | 1.2 | 1.1 | 0.21 |
| YJL116C | 0.9 | 1.8 | 1.3 | 1.9 | 1.3 | 3.4 | 0.8 | 1.8 | 2.4 | 2.6 | 2.9 | 1.1 | 4.3 | 2.6 | 1.0 | 1.3 | 1.2 | 0.7 | 1.01 |
| YJR086W | 1.2 | 1.1 | 0.8 | 1.4 | 1.3 | 1.9 | 1.7 | 1.1 | 1.4 | 1.1 | 1.0 | 0.8 | 0.9 | 2.2 | 2.0 | 1.4 | 1.4 | 1.6 | 1.24 |
| YKL008C | 1.2 | 0.6 | 2.0 | 0.8 | 0.9 | 3.8 | 1.4 | 1.2 | 0.3 | 0.4 | 1.5 | 1.5 | 1.7 | 0.8 | 1.3 | 2.1 | 1.4 | 1.9 | 2.11 |
| YKL013C | 1.4 | 0.9 | 1.4 | 1.1 | 1.9 | 1.9 | 1.8 | 0.9 | 1.5 | 1.4 | 1.1 | 0.7 | 1.3 | 1.4 | 1.2 | 1.5 | 1.6 | 1.7 | 1.55 |
| YKL041W | 1.2 | 0.8 | 1.1 | 1.0 | 1.3 | 2.2 | 1.0 | 1.6 | 1.6 | 1.5 | 1.2 | 0.8 | 1.3 | 1.3 | 1.0 | 2.5 | 1.1 | 1.3 | 0.91 |
| YKL139W | 0.9 | 0.7 | 1.4 | 0.8 | 2.8 | 3.5 | 2.6 | 1.2 | 1.6 | 1.5 | 1.4 | 0.8 | 1.6 | 1.0 | 1.0 | 1.3 | 1.9 | 0.8 | 0.62 |
| YKR014C | 1.1 | 1.3 | 0.7 | 1.1 | 1.5 | 2.1 | 1.7 | 1.6 | 2.4 | 2.0 | 1.1 | 1.1 | 1.5 | 1.8 | 1.4 | 2.4 | 1.0 | 1.7 | 2.05 |
| YLR093C | 1.3 | 0.8 | 0.7 | 1.2 | 1.4 | 2.9 | 1.9 | 1.2 | 1.5 | 1.3 | 1.0 | 1.0 | 1.3 | 1.3 | 0.9 | 2.4 | 1.7 | 3.2 | 1.90 |
| YLR118C | 1.0 | 1.1 | 1.6 | 1.0 | 1.0 | 2.5 | 1.5 | 1.0 | 1.1 | 1.3 | 1.2 | 1.0 | 1.4 | 1.5 | 0.8 | 3.5 | 1.3 | 1.9 | 1.17 |

| | | | | | | | | | | | | | | | | | | | |
|---|---|---|---|---|---|---|---|---|---|---|---|---|---|---|---|---|---|---|---|
| YLR251W | 1.8 | 0.8 | 4.2 | 4.6 | 1.6 | 2.1 | 2.6 | 1.2 | 2.1 | 1.3 | 4.1 | 0.8 | 1.7 | 1.3 | 0.8 | 2.5 | 2.1 | 3.8 | 0.78 |
| YMR027W | 0.8 | 2.6 | 0.7 | 0.8 | 1.1 | 2.1 | 2.0 | 0.9 | 1.1 | 1.4 | 1.1 | 0.9 | 2.4 | 0.9 | 4.0 | 0.8 | 1.3 | 1.2 | 4.05 |
| YMR262W | 1.0 | 1.3 | 1.9 | 1.1 | 1.2 | 2.0 | 1.9 | 1.0 | 3.0 | 1.2 | 1.3 | 0.8 | 1.7 | 0.9 | 0.3 | 1.0 | 1.0 | 1.2 | 0.65 |
| YNL214W | 1.2 | 0.9 | 1.1 | 1.1 | 1.4 | 2.3 | 1.3 | 1.1 | 1.5 | 1.5 | 0.6 | 0.8 | 1.2 | 1.7 | 0.9 | 2.0 | 1.1 | 1.2 | 0.47 |
| YOR149C | 0.7 | 0.9 | 1.0 | 0.8 | 1.1 | 2.3 | 1.0 | 0.7 | 0.4 | 1.0 | 1.0 | 0.9 | 1.3 | 0.9 | 1.3 | 1.1 | 1.0 | 1.2 | 0.76 |
| YOR165W | 0.9 | 1.5 | 0.7 | 0.8 | 0.8 | 2.2 | 0.9 | 1.1 | 0.8 | 1.3 | 1.3 | 0.7 | 0.9 | 0.8 | 0.8 | 1.0 | 0.9 | 1.0 | 1.03 |
| YOR285W | 1.4 | 6.0 | 3.5 | 1.9 | 1.7 | 2.1 | 4.0 | 1.2 | 4.0 | 2.9 | 2.3 | 0.9 | 2.1 | 1.6 | 2.0 | 2.9 | 2.3 | 3.3 | 2.62 |
| YOR367W | 1.0 | 0.9 | 1.7 | 0.9 | 1.1 | 3.3 | 1.9 | 1.1 | 1.1 | 1.1 | 1.4 | 0.5 | 0.8 | 1.5 | 1.1 | 1.3 | 1.4 | 1.4 | 0.60 |
| YPL018W | 1.0 | 1.4 | 1.0 | 0.5 | 1.8 | 2.1 | 0.9 | 1.1 | 1.4 | 1.2 | 1.3 | 1.1 | 2.2 | 1.3 | 0.9 | 0.8 | 1.3 | 0.8 | 0.22 |
| YPL203W | 1.3 | 1.0 | 2.2 | 1.0 | 2.1 | 1.8 | 2.1 | 1.1 | 1.8 | 2.1 | 1.5 | 0.9 | 1.5 | 1.0 | 0.6 | 2.3 | 1.3 | 1.7 | 0.79 |
| YPL255W | 0.8 | 0.3 | 1.1 | 0.6 | 0.9 | 2.0 | 0.6 | 0.9 | 0.1 | 0.1 | 0.7 | 0.6 | 0.6 | 0.7 | 0.6 | 0.7 | 0.8 | 0.7 | 0.55 |
| YPR073C | 1.2 | 1.9 | 0.5 | 1.4 | 1.8 | 2.0 | 2.5 | 1.5 | 1.3 | 2.6 | 0.9 | 0.8 | 1.4 | 1.5 | 0.8 | 1.8 | 1.4 | 1.8 | 1.55 |
| YDR518W | 0.8 | 1.7 | 1.1 | 1.2 | 1.6 | 1.2 | 2.7 | 1.1 | 0.2 | 1.2 | 1.0 | 1.5 | 1.8 | 0.8 | 2.5 | 1.7 | 1.4 | 1.6 | 0.93 |
| YOR381W | 0.8 | 2.0 | 0.8 | 1.8 | 1.0 | 1.0 | 3.6 | 0.8 | 1.2 | 3.5 | 2.6 | 1.1 | 1.7 | 0.8 | 0.6 | 2.5 | 0.8 | 0.9 | 0.38 |
| YBR109C | 1.6 | 1.3 | 2.4 | 1.1 | 1.0 | 1.4 | 2.4 | 1.7 | 3.8 | 3.3 | 1.5 | 1.4 | 2.0 | 2.0 | 1.4 | 1.9 | 1.4 | 1.9 | 2.15 |
| YBR201W | 1.2 | 4.3 | 1.2 | 2.4 | 1.2 | 1.0 | 3.4 | 2.2 | 0.8 | 1.0 | 1.1 | 1.7 | 1.9 | 1.1 | 1.6 | 1.5 | 1.8 | 1.6 | 0.75 |
| YDR041W | 1.2 | 1.1 | 0.6 | 1.8 | 2.0 | 1.6 | 2.4 | 1.2 | 1.3 | 1.3 | 1.2 | 0.8 | 0.9 | 1.4 | 1.0 | 2.4 | 1.5 | 2.5 | 1.85 |
| YER136W | 0.9 | 1.1 | 1.1 | 1.4 | 1.6 | 1.3 | 2.1 | 1.3 | 1.4 | 1.6 | 1.7 | 0.9 | 1.3 | 0.8 | 1.2 | 1.6 | 1.3 | 1.7 | 2.31 |
| YER159C | 1.1 | 0.9 | 0.2 | 1.6 | 2.1 | 1.3 | 2.0 | 1.4 | 0.2 | 0.9 | 1.6 | 0.9 | 0.9 | 1.1 | 2.2 | 1.1 | 1.6 | 1.9 | 0.99 |
| YJL030W | 1.6 | 1.4 | 0.5 | 1.0 | 1.3 | 1.1 | 1.9 | 1.6 | 2.3 | 1.9 | 1.4 | 1.0 | 1.4 | 2.1 | 1.5 | 1.9 | 1.3 | 1.5 | 0.87 |
| YJR029W | 0.8 | 1.3 | 0.6 | 0.8 | | 1.0 | 2.4 | 0.9 | 1.1 | 1.5 | 0.6 | 1.0 | 2.1 | 1.1 | 1.8 | 0.7 | 0.8 | 1.1 | 5.84 |
| YJR099W | 1.4 | 0.9 | 1.1 | 1.3 | 1.5 | 2.1 | 2.0 | 1.4 | 1.4 | 2.4 | 1.3 | 0.7 | 1.5 | 2.1 | 0.5 | 2.0 | 1.1 | 1.7 | 0.94 |
| YJR122W | 2.0 | 1.4 | 2.5 | 1.1 | 1.3 | 1.4 | 1.8 | 1.4 | 5.3 | 6.1 | 2.4 | 0.9 | 1.3 | 1.4 | 0.8 | 1.9 | 1.0 | 1.1 | 0.43 |
| YJR125C | 1.0 | 1.0 | 1.2 | 1.0 | 1.2 | 1.3 | 1.9 | 0.9 | 1.8 | 1.3 | 1.1 | 0.9 | 1.5 | 1.1 | 1.3 | 2.0 | 1.2 | 1.4 | 0.90 |
| YKL190W | 1.1 | 1.3 | 0.4 | 0.9 | | 1.5 | 2.0 | 1.1 | 1.8 | 1.9 | 1.5 | 0.9 | 1.3 | 1.7 | 1.4 | 1.8 | 1.3 | 1.3 | 1.14 |
| YLL051C | 0.8 | 1.1 | 1.0 | 1.5 | 1.4 | 1.2 | 2.0 | 0.9 | 0.9 | 1.8 | 0.7 | 1.1 | 1.3 | 0.8 | 1.2 | 0.8 | 1.0 | 1.0 | 0.81 |
| YLR090W | 0.7 | 1.1 | 0.8 | 1.3 | 1.2 | 1.3 | 2.2 | 0.9 | 1.8 | 1.4 | 1.0 | 0.8 | 1.7 | 1.0 | 0.8 | 1.9 | 0.8 | 1.0 | 0.67 |
| YMR051C | 0.9 | 2.8 | 0.8 | 1.3 | 1.7 | 1.2 | 2.6 | 0.9 | 1.0 | 1.4 | 1.1 | 0.9 | 1.3 | 1.2 | 1.7 | 1.0 | 1.8 | 1.2 | 3.91 |
| YMR139W | 1.0 | 3.4 | 2.5 | 1.5 | 1.2 | 1.5 | 2.2 | 0.9 | 2.8 | 2.4 | 1.3 | 0.9 | 1.6 | 1.3 | 0.9 | 2.3 | 1.1 | 2.0 | 0.89 |
| YNL015W | 2.3 | 6.7 | 1.8 | 2.4 | 1.8 | 1.7 | 3.5 | 2.2 | 2.1 | 4.3 | 2.1 | 2.8 | 3.9 | 1.6 | 1.4 | 5.3 | 3.0 | 5.2 | 1.03 |
| YNL079C | 1.1 | 1.0 | 0.4 | 1.0 | 1.7 | 1.1 | 1.9 | 1.6 | 1.5 | 1.2 | 1.3 | 1.2 | 1.8 | 1.0 | 1.5 | 1.4 | 1.0 | 1.9 | 3.26 |
| YNL223W | 1.1 | 1.2 | 0.6 | 1.7 | 1.0 | 1.8 | 2.2 | 1.4 | 3.3 | 1.9 | 1.3 | 1.3 | 2.0 | 1.2 | 1.0 | 2.1 | 1.0 | 1.5 | 0.34 |

| | | | | | | | | | | | | | | | | | | |
|---|---|---|---|---|---|---|---|---|---|---|---|---|---|---|---|---|---|---|
| YNR007C | 1.2 | 1.8 | 0.7 | 1.0 | 1.5 | 1.3 | 1.9 | 1.2 | 2.5 | 2.0 | 0.7 | 0.9 | 1.5 | 1.5 | 0.6 | 2.0 | 1.4 | 1.6 | 0.49 |
| YNR035C | 0.8 | 1.2 | 1.7 | 1.0 | 0.8 | 1.1 | 2.6 | 1.1 | 2.9 | 1.5 | 1.5 | 1.1 | 1.7 | 1.0 | 1.3 | 1.6 | 1.4 | 1.8 | 1.60 |
| YOL016C | 1.3 | 2.0 | 0.4 | 2.0 | 1.6 | 0.9 | 3.0 | 1.3 | 1.9 | 1.6 | 1.3 | 1.4 | 3.0 | 0.7 | 0.5 | 1.6 | 4.3 | 3.8 | 2.10 |
| YOL104C | 1.0 | 1.7 | 0.2 | 1.8 | 1.9 | 1.4 | 2.6 | 1.4 | 0.2 | 1.7 | 1.2 | 0.8 | 0.6 | 2.2 | 1.3 | 0.5 | 1.1 | 0.9 | 0.32 |
| YPR107C | 1.4 | 1.0 | 1.2 | 1.9 | 1.5 | 1.3 | 2.0 | 1.3 | 2.4 | 2.4 | 1.2 | 0.7 | 1.4 | 1.7 | 1.0 | 1.4 | 1.2 | 1.5 | 0.81 |
| YOL152W | 0.2 | 0.6 | 1.0 | 0.8 | 0.7 | 1.8 | 2.4 | 4.1 | 0.9 | 1.2 | 5.9 | 0.8 | 0.5 | 0.4 | 0.8 | 1.2 | 1.1 | 1.2 | 0.91 |
| YAL007C | 1.3 | 0.9 | 0.9 | 1.5 | 0.6 | 0.7 | 1.5 | 1.7 | 1.1 | 0.9 | 2.0 | 1.3 | 2.0 | 1.3 | 2.5 | 0.8 | 1.2 | 1.5 | 1.74 |
| YDL043C | 1.0 | 0.8 | 0.8 | 1.2 | 1.1 | 0.5 | 0.9 | 1.3 | 0.8 | 0.8 | 3.9 | 0.9 | 0.8 | 1.2 | 0.8 | 1.2 | 0.8 | 0.8 | 0.78 |
| YDL212W | 1.0 | 1.2 | 1.6 | 1.2 | 1.1 | 0.4 | 0.7 | 0.9 | 0.4 | 0.6 | 2.0 | 0.8 | 0.5 | 1.0 | 1.1 | 0.9 | 1.1 | 1.7 | 4.27 |
| YDR183W | 1.2 | 0.7 | 0.9 | 1.4 | 1.5 | 1.4 | 1.8 | 1.8 | 2.5 | 2.9 | 1.8 | 1.1 | 1.4 | 1.9 | 1.4 | 1.3 | 0.8 | 1.0 | 0.61 |
| YGL089C | 0.4 | 0.7 | 2.0 | 1.0 | 1.5 | 0.5 | 0.4 | 1.0 | 0.0 | 1.9 | 2.3 | 0.4 | 0.2 | 1.4 | 0.9 | 0.2 | 0.3 | 0.6 | 3.00 |
| YGL096W | 1.9 | 2.9 | 1.2 | 2.5 | 0.9 | 0.5 | 1.8 | 1.7 | 2.1 | 3.6 | 1.7 | 1.1 | 1.2 | 1.4 | 0.8 | 1.7 | 1.1 | 1.1 | 0.54 |
| YGR006W | 1.2 | 1.1 | 0.9 | 2.9 | 0.5 | 0.5 | 1.0 | 1.2 | 0.7 | 0.9 | 1.6 | 0.8 | 1.4 | 1.1 | 1.2 | 1.5 | 0.9 | 1.0 | 0.40 |
| YHL034C | 0.7 | 1.0 | 0.7 | 0.8 | 1.9 | 1.1 | 1.4 | 1.4 | 1.9 | 1.9 | 2.2 | 0.8 | 1.6 | 1.0 | 1.4 | 1.6 | 1.2 | 1.5 | 2.11 |
| YHR163W | 0.9 | 1.2 | 1.3 | 1.2 | 0.8 | 1.9 | 0.9 | 1.3 | 1.4 | 1.1 | 2.9 | 0.7 | 1.1 | 1.5 | 0.7 | 1.2 | 1.1 | 1.2 | 1.71 |
| YIR024C | 1.5 | 0.9 | 0.9 | 2.0 | 1.1 | 0.8 | 1.5 | 1.4 | 5.6 | 2.9 | 6.3 | 1.1 | 1.6 | 2.6 | 0.9 | 2.2 | 0.9 | 1.4 | 0.75 |
| YKL070W | 1.0 | 0.8 | 1.7 | 0.9 | 1.3 | 1.1 | 1.1 | 1.5 | 15.0 | 11.0 | 10.4 | 0.6 | 1.0 | 2.2 | 0.8 | 0.9 | 1.0 | 0.9 | 0.29 |
| YLL050C | 1.4 | 1.0 | 1.2 | 1.7 | | 1.9 | 1.4 | 1.3 | 1.0 | 1.0 | 2.3 | 0.9 | 1.8 | 1.2 | 1.6 | 1.3 | 1.2 | 2.1 | 3.43 |
| YLR220W | 0.9 | 0.6 | 3.0 | 1.0 | 0.9 | 0.3 | 0.4 | 1.0 | 0.4 | 0.4 | 2.4 | 0.7 | 0.5 | 1.4 | 0.7 | 1.1 | 0.8 | 0.8 | 2.28 |
| YLR390W | 0.9 | 0.9 | 1.2 | 1.7 | 1.2 | 0.7 | 1.5 | 0.8 | 1.7 | 1.7 | 1.9 | 1.0 | 1.1 | 1.1 | 0.5 | 2.0 | 1.0 | 0.9 | 0.51 |
| YOL044W | 1.0 | 1.4 | 1.0 | 1.3 | 1.0 | 1.2 | 1.2 | 1.1 | 1.6 | 1.4 | 2.1 | 0.7 | 1.3 | 1.1 | 0.6 | 1.3 | 1.6 | 1.9 | 0.65 |
| YOL147C | 1.6 | 1.1 | 2.4 | 1.5 | 0.7 | 0.9 | 0.6 | 1.5 | 0.5 | 0.7 | 2.8 | 0.7 | 0.8 | 0.9 | 0.8 | 2.4 | 2.0 | 1.7 | 1.09 |
| YDR069C | 1.4 | 0.8 | 3.0 | 1.5 | 1.6 | 0.7 | 1.0 | 1.8 | 1.8 | 4.5 | 0.8 | 0.7 | 1.3 | 1.2 | 1.0 | 1.3 | 1.0 | 1.3 | 0.33 |
| YER131W | 1.5 | 0.3 | 0.9 | 0.7 | 1.6 | 0.9 | 0.6 | 2.0 | 0.0 | 0.6 | 1.0 | 0.9 | 0.2 | 0.8 | 0.9 | 0.4 | 0.9 | 0.9 | 5.32 |
| YGR044C | 0.8 | 1.3 | 0.7 | 1.2 | 1.1 | 1.4 | 1.3 | 1.8 | 0.9 | 0.8 | 2.1 | 0.8 | 1.4 | 1.0 | 0.5 | 3.3 | 1.1 | 2.6 | 1.43 |
| YMR240C | 1.1 | 0.6 | 2.5 | 0.7 | 1.4 | 0.8 | 1.1 | 2.3 | 0.5 | 1.1 | 1.4 | 0.6 | 0.8 | 1.1 | 1.0 | 1.0 | 1.1 | 1.1 | 0.39 |
| YBR105C | 1.7 | 1.5 | 0.9 | 2.0 | 0.8 | 1.8 | 2.0 | 1.2 | 1.1 | 3.3 | 1.1 | 1.2 | 1.1 | 1.7 | 1.4 | 1.3 | 1.3 | 1.1 | 0.96 |
| YBR182C | 2.0 | 1.9 | 1.0 | 2.4 | 1.4 | 1.0 | 1.7 | 1.3 | 0.6 | 3.2 | 1.5 | 1.3 | 1.2 | 1.2 | 3.1 | 1.1 | 3.4 | 1.6 | 0.41 |
| YBR186W | 1.0 | 1.0 | 1.1 | 1.7 | 1.0 | 0.5 | 0.9 | 0.9 | 1.0 | 2.4 | 0.9 | 0.8 | 0.7 | 1.1 | 0.9 | 1.2 | 0.7 | 1.0 | 0.31 |
| YEL052W | 1.1 | 1.0 | 2.0 | 2.0 | 1.3 | 1.2 | 1.2 | 1.2 | 3.0 | 3.4 | 1.7 | 1.1 | 1.5 | 1.1 | 0.7 | 2.1 | 0.9 | 1.0 | 0.90 |
| YER098W | 0.9 | 0.9 | 0.5 | 1.1 | 1.4 | 1.6 | 1.3 | 1.7 | 2.8 | 3.3 | 1.4 | 1.0 | 1.1 | 1.3 | 0.9 | 1.7 | 1.2 | 1.5 | 0.35 |
| YGL240W | 0.9 | 1.2 | 0.8 | 1.8 | 1.2 | 1.1 | 1.2 | 0.8 | 2.0 | 3.4 | 0.9 | 0.8 | 1.7 | 1.3 | 1.1 | 1.0 | 1.2 | 0.8 | 0.48 |

| | | | | | | | | | | | | | | | | | | | |
|---|---|---|---|---|---|---|---|---|---|---|---|---|---|---|---|---|---|---|---|
| YGR067C | 2.2 | 1.4 | 1.2 | 4.6 | 1.6 | 1.3 | 1.5 | 1.5 | 0.7 | 6.7 | 1.0 | 0.7 | 1.0 | 0.9 | 1.2 | 1.7 | 1.2 | 1.0 | 0.11 |
| YGR133W | 1.0 | 1.1 | 0.7 | 0.6 | 1.3 | 1.2 | 1.2 | 1.2 | 1.3 | 3.6 | 1.6 | 0.9 | 1.2 | 1.4 | 1.0 | 1.5 | 1.0 | 1.0 | 0.35 |
| YHR124W | 1.1 | 1.1 | 1.5 | 2.0 | 0.9 | 0.8 | 1.2 | 1.1 | 3.0 | 6.8 | 1.1 | 1.2 | 1.7 | 2.7 | 0.8 | 1.8 | 1.3 | 1.1 | 0.24 |
| YJL103C | 1.3 | 1.8 | 3.8 | 1.3 | 1.1 | 1.0 | 0.7 | 0.8 | 2.3 | 2.4 | 1.5 | 0.8 | 1.4 | 1.0 | 1.3 | 0.9 | 1.4 | 1.2 | 0.44 |
| YJR036C | 1.2 | 1.2 | 1.0 | 4.3 | 1.5 | 0.9 | 1.0 | 1.0 | 1.4 | 3.0 | 1.1 | 0.9 | 1.1 | 1.3 | 1.8 | 2.0 | 0.9 | 1.2 | 0.31 |
| YLR216C | 1.2 | 2.3 | 3.3 | 1.1 | 1.2 | 1.2 | 5.2 | 1.3 | 14.1 | 4.1 | 1.8 | 0.7 | 1.2 | 1.1 | 1.2 | 1.5 | 1.2 | 1.3 | 1.44 |
| YLR389C | 0.8 | 6.4 | 2.3 | 0.6 | 0.7 | 0.2 | 1.0 | 1.0 | 2.4 | 2.8 | 0.9 | 0.8 | 1.9 | 0.8 | 1.0 | 1.4 | 1.0 | 1.0 | 0.60 |
| YNL128W | 1.3 | 4.6 | 1.1 | 0.8 | 1.2 | 1.1 | 1.0 | 1.3 | 2.9 | 2.4 | 0.9 | 0.9 | 1.4 | 1.4 | 1.1 | 0.6 | 0.9 | 0.9 | 0.21 |
| YOL133W | 1.0 | 1.6 | 1.4 | 1.2 | 0.9 | 1.6 | 1.8 | 1.0 | 4.7 | 3.8 | 1.5 | 0.6 | 1.0 | 1.4 | 1.3 | 2.0 | 1.3 | 1.3 | 0.87 |
| YOR133W | 1.1 | 1.3 | 1.6 | 1.3 | 1.3 | 0.9 | 1.1 | 0.8 | 3.1 | 4.9 | 1.3 | 0.8 | 1.3 | 1.1 | 1.8 | 1.3 | 1.2 | 1.2 | 0.26 |
| YOR227W | 1.1 | 2.4 | 3.2 | 0.9 | 0.5 | 1.0 | 1.0 | 0.7 | 2.9 | 3.0 | 1.2 | 1.0 | 1.7 | 1.1 | 3.7 | 2.2 | 0.8 | 1.3 | 0.35 |
| YPR015C | 1.3 | 1.3 | 1.5 | 1.7 | 1.1 | 0.7 | 1.3 | 1.2 | 5.6 | 5.3 | 1.0 | 0.7 | 1.5 | 1.5 | 1.6 | 2.0 | 1.0 | 1.0 | 0.32 |
| YPR086W | 1.0 | 1.4 | 0.7 | 0.7 | 1.2 | 1.1 | 1.6 | 1.3 | 3.0 | 2.9 | 1.3 | 0.8 | 1.0 | 1.0 | 1.3 | 1.4 | 1.2 | 1.2 | 1.37 |
| YBL056W | 0.7 | 0.7 | 1.1 | 1.2 | 1.8 | 1.6 | 1.4 | 1.3 | 3.6 | 2.2 | 0.7 | 0.9 | 2.7 | 0.9 | 1.0 | 1.7 | 1.1 | 1.5 | 2.49 |
| YBR026C | 1.1 | 1.7 | 2.8 | 0.9 | 1.2 | 0.1 | | 1.1 | 3.4 | 1.6 | 1.6 | 1.2 | 1.5 | 1.5 | 0.7 | 0.5 | 0.8 | 0.9 | 0.59 |
| YBR123C | 0.8 | 0.7 | 1.6 | 1.8 | 1.1 | 1.3 | 0.9 | 0.9 | 3.2 | 2.5 | 1.1 | 1.0 | 1.4 | 1.1 | 1.0 | 1.2 | 0.8 | 1.0 | 0.62 |
| YDR099W | 0.9 | 1.0 | 2.0 | 1.3 | 1.1 | 2.2 | 1.3 | 1.1 | 2.4 | 1.3 | 1.1 | 0.9 | 1.5 | 0.9 | 1.9 | 1.4 | 1.2 | 1.7 | 3.51 |
| YDR177W | 1.2 | 1.7 | 1.0 | 1.0 | 1.0 | 1.4 | 1.3 | 1.6 | 3.5 | 1.9 | 1.3 | 1.2 | 1.7 | 2.4 | 0.8 | 1.3 | 1.3 | 1.5 | 1.77 |
| YDR392W | 0.9 | 2.9 | 1.1 | 1.3 | 1.2 | 0.6 | 1.1 | 1.1 | 4.3 | 1.5 | 1.2 | 0.8 | 1.4 | 1.3 | 1.1 | 1.4 | 1.0 | 1.2 | 0.63 |
| YDR394W | 0.9 | 1.2 | 1.3 | 0.8 | 1.2 | 1.1 | 1.2 | 1.3 | 3.8 | 2.0 | 1.8 | 0.6 | 1.0 | 1.2 | 1.1 | 1.0 | 1.1 | 0.8 | 0.97 |
| YER184C | 2.3 | 1.6 | 1.1 | 2.3 | 1.0 | 0.6 | 0.9 | 1.1 | 4.6 | 3.2 | 1.3 | 0.8 | 1.6 | 1.4 | 1.4 | 1.2 | 1.6 | 2.3 | 0.29 |
| YFL059W | 1.2 | 1.4 | 1.2 | 2.2 | 1.1 | 1.0 | 1.3 | 1.5 | 4.1 | 4.2 | 1.5 | 1.2 | 2.3 | 2.4 | 0.9 | 1.6 | 1.3 | 1.1 | 0.57 |
| YGL185C | 1.3 | 1.9 | 1.9 | 1.9 | 0.8 | 0.8 | 1.9 | 1.5 | 4.1 | 2.3 | 1.5 | 1.5 | 1.7 | 1.5 | 0.9 | 15.8 | 1.3 | 1.6 | 0.23 |
| YHL019C | 1.1 | 0.8 | 1.2 | 0.6 | 1.0 | 1.2 | 1.1 | 1.0 | 2.1 | 1.4 | 1.3 | 0.8 | 0.8 | 0.8 | 1.2 | 1.3 | 1.0 | 1.0 | 0.55 |
| YHR012W | 1.1 | 1.0 | 1.2 | 1.0 | | 1.6 | 1.2 | 1.2 | 4.9 | 2.2 | 1.5 | 0.9 | 1.7 | 1.7 | 1.0 | 1.0 | 1.2 | 1.4 | 1.11 |
| YHR028C | 0.8 | 1.2 | 1.4 | 0.8 | 1.0 | 0.9 | 1.2 | 0.8 | 3.7 | 2.2 | 1.4 | 0.7 | 0.6 | 1.1 | 1.1 | 0.7 | 1.0 | 1.0 | 0.92 |
| YHR109W | 0.8 | 1.0 | 0.0 | 0.7 | 1.3 | 1.4 | 1.4 | 1.2 | 2.4 | 1.8 | 0.7 | 1.0 | 1.4 | 1.5 | 0.9 | 1.3 | 0.9 | 1.1 | 0.22 |
| YHR156C | 1.3 | 1.3 | 0.9 | 2.1 | 1.9 | 1.0 | 1.2 | 1.2 | 3.6 | 1.9 | 0.6 | 0.7 | 1.0 | 1.9 | 1.2 | 1.4 | 0.9 | 1.3 | 0.47 |
| YIL159W | 1.1 | 1.6 | 1.3 | 0.7 | 1.2 | 2.3 | 0.6 | 1.1 | 5.9 | 1.6 | 0.8 | 0.9 | 1.5 | 2.0 | 1.0 | 0.7 | 0.9 | 0.8 | 0.29 |
| YJL154C | 0.6 | 2.7 | 0.7 | 0.6 | 1.2 | 1.3 | 1.5 | 1.0 | 2.3 | 2.0 | 0.5 | 0.9 | 1.6 | 0.8 | 1.2 | 1.2 | 1.2 | 1.1 | 0.62 |
| YJR110W | 1.0 | 0.6 | 1.0 | 1.4 | 1.3 | 0.9 | 0.9 | 1.1 | 4.9 | 2.3 | 1.2 | 0.7 | 0.9 | 1.1 | 1.2 | 1.1 | 0.8 | 1.0 | 0.58 |
| YKL025C | 0.7 | 1.1 | 1.1 | 0.9 | 1.1 | 0.9 | 0.9 | 0.9 | 2.8 | 1.7 | 1.1 | 0.8 | 1.3 | 0.9 | 1.2 | 1.1 | 0.9 | 1.0 | 0.54 |

| | | | | | | | | | | | | | | | | | | | |
|---|---|---|---|---|---|---|---|---|---|---|---|---|---|---|---|---|---|---|---|
| YKL171W | 0.9 | 1.3 | 1.2 | 2.1 | 0.9 | 0.4 | 0.9 | 0.8 | 8.2 | 1.8 | 2.8 | 1.1 | 1.5 | 1.1 | 1.2 | 2.1 | 0.9 | 1.2 | 0.45 |
| YKL196C | 1.2 | 1.1 | 0.5 | 0.9 | 1.3 | 0.9 | 1.5 | 1.3 | 4.3 | 2.0 | 1.6 | 1.0 | 1.8 | 2.5 | 1.9 | 1.9 | 1.3 | 2.0 | 2.41 |
| YKR068C | 1.2 | 1.5 | 1.0 | 1.8 | 1.1 | 1.4 | 1.4 | 1.3 | 2.4 | 1.9 | 1.0 | 1.0 | 1.1 | 1.6 | 0.8 | 1.4 | 1.4 | 1.7 | 1.72 |
| YLR144C | 0.8 | 1.5 | 4.0 | 1.3 | 0.7 | 0.9 | 0.7 | 1.1 | 2.8 | 1.5 | 1.0 | 0.7 | 2.3 | 1.2 | 1.9 | 1.7 | 1.0 | 1.0 | 0.44 |
| YML112W | 1.0 | 1.0 | 1.1 | 2.0 | 1.0 | 1.4 | 0.9 | 1.3 | 3.2 | 2.4 | 1.0 | 1.0 | 1.3 | 1.8 | 0.8 | 0.9 | 0.8 | 0.9 | 0.49 |
| YOL038W | 0.9 | 1.0 | 0.4 | 1.6 | 1.8 | 1.2 | 1.4 | 1.4 | 3.5 | 2.7 | 2.0 | 0.9 | 1.8 | 2.1 | 1.7 | 1.6 | 1.1 | 1.4 | 1.29 |
| YOR257W | 1.1 | 2.5 | 0.8 | 1.1 | 1.1 | 0.8 | 1.3 | 1.5 | 3.4 | 1.4 | 1.0 | 0.7 | 0.8 | 1.5 | 0.7 | 1.6 | 1.1 | 1.6 | 0.68 |
| YOR265W | 1.4 | 1.0 | 0.8 | 1.0 | 1.2 | 1.4 | 1.3 | 1.4 | 3.5 | 2.2 | 1.3 | 0.8 | 1.8 | 2.5 | 1.2 | 1.1 | 1.0 | 1.2 | 0.93 |
| YPL124W | 1.3 | 1.3 | 0.7 | 0.7 | 1.6 | 0.8 | 0.9 | 1.8 | 2.2 | 1.0 | 0.9 | 0.7 | 0.9 | 1.6 | 0.6 | 0.8 | 1.3 | 1.2 | 0.41 |
| YPR125W | 0.6 | 1.3 | 0.9 | | 1.2 | 0.7 | 1.3 | 1.2 | 4.1 | 1.8 | 0.9 | 1.1 | 1.9 | 1.6 | 2.0 | 0.6 | 0.8 | 0.7 | 0.73 |
| YPR168W | 0.9 | 1.0 | 0.2 | 0.5 | 1.3 | 0.4 | 1.1 | 1.1 | 4.7 | 2.7 | 1.2 | 0.5 | 1.0 | 1.1 | 1.2 | 1.3 | 0.9 | 0.8 | 0.29 |
| YPR180W | 0.9 | 1.2 | 1.6 | 1.0 | 1.0 | 0.8 | 2.4 | 1.3 | 2.3 | 1.1 | 0.5 | 0.7 | 1.3 | 1.2 | 1.3 | 1.0 | 1.0 | 1.4 | 0.61 |
| YPR193C | 1.0 | 2.2 | 0.6 | 1.1 | 1.4 | 1.3 | 1.4 | 1.1 | 3.5 | 4.1 | 0.1 | 0.9 | 1.6 | 1.6 | 0.9 | 1.6 | 1.1 | 0.9 | 0.17 |
| YBR045C | 0.9 | 1.0 | 2.2 | 0.7 | 1.0 | 0.8 | 0.8 | 1.0 | 3.6 | 1.1 | 0.4 | 1.3 | 1.3 | 1.0 | 2.0 | 0.8 | 1.1 | 0.9 | 1.29 |
| YBR128C | 1.0 | 1.1 | 2.1 | 1.0 | 1.1 | 1.5 | 2.1 | 1.2 | 2.8 | 1.2 | 1.3 | 0.7 | 1.3 | 1.4 | 1.4 | 2.0 | 1.2 | 1.8 | 0.44 |
| YCL055W | 0.7 | 2.2 | 0.7 | 1.3 | 1.2 | 1.4 | 1.0 | 1.3 | 2.1 | 1.5 | 1.4 | 0.9 | 0.9 | 1.8 | 1.5 | 0.7 | 0.8 | 1.1 | 0.66 |
| YCR019W | 1.0 | 1.6 | 1.0 | 0.8 | 1.4 | 1.5 | 1.1 | 1.5 | 2.8 | 1.6 | 2.0 | 1.0 | 1.6 | 1.2 | 0.7 | 1.4 | 1.0 | 1.0 | 0.45 |
| YDL065C | 1.2 | 0.9 | 1.0 | 0.7 | 1.4 | 1.1 | 1.3 | 1.4 | 2.4 | 1.5 | 1.4 | 0.6 | 0.7 | 1.4 | 1.3 | 1.5 | 1.1 | 1.2 | 1.08 |
| YDL143W | 0.8 | 0.8 | 1.2 | 1.1 | 1.0 | 1.0 | 0.9 | 1.2 | 2.1 | 1.8 | 1.3 | 1.1 | 1.9 | 0.9 | 1.4 | 0.9 | 0.8 | 0.8 | 1.86 |
| YDL197C | 1.0 | 1.1 | 0.9 | 2.1 | 1.5 | 1.2 | 1.0 | 1.0 | 2.6 | 1.5 | 1.1 | 0.8 | 1.4 | 1.1 | 1.3 | 0.9 | 1.3 | 0.8 | 0.31 |
| YDL230W | 1.0 | 0.8 | 1.2 | 1.3 | 1.4 | 0.9 | 1.1 | 0.9 | 2.6 | 1.2 | 0.9 | 0.6 | 1.3 | 1.0 | 1.4 | 1.7 | 1.0 | 1.2 | 0.42 |
| YDR212W | 0.8 | 1.2 | 1.3 | 0.8 | 1.4 | 1.4 | 1.0 | 1.3 | 3.5 | 1.7 | 1.8 | 0.8 | 2.0 | 1.0 | 1.2 | 0.7 | 0.6 | 1.0 | 2.13 |
| YDR257C | 0.8 | 5.3 | 0.6 | 0.8 | 1.6 | 1.1 | 1.0 | 1.0 | 3.0 | 1.6 | 1.3 | 0.7 | 1.0 | 1.0 | 1.3 | 1.0 | 1.1 | 0.8 | 0.62 |
| YDR329C | 0.9 | 0.8 | 1.0 | 1.7 | 1.9 | 1.2 | 1.6 | 1.2 | 2.5 | 1.0 | 1.0 | 0.8 | 1.1 | 1.1 | 1.0 | 1.6 | 1.2 | 1.6 | 0.95 |
| YDR488C | 1.0 | 0.3 | 0.9 | 0.9 | 0.9 | 0.9 | 0.7 | 0.9 | 2.1 | 0.4 | 0.4 | 0.6 | 1.3 | 0.8 | 3.5 | 0.7 | 0.9 | 0.9 | 0.32 |
| YDR505C | 0.8 | 1.0 | 1.0 | 1.4 | 1.2 | 1.1 | 1.2 | 0.8 | 2.5 | 1.0 | 1.0 | 0.7 | 1.6 | 0.9 | 1.1 | 1.6 | 1.0 | 1.5 | 0.84 |
| YDR506C | 0.9 | 0.7 | 1.3 | 0.7 | 0.9 | 0.9 | 0.6 | 1.0 | 1.9 | 1.4 | 1.1 | 0.9 | 0.3 | 0.5 | 0.9 | 0.4 | 0.6 | 0.5 | 1.90 |
| YDR515W | 0.9 | 1.1 | 0.8 | 0.8 | 1.5 | 0.8 | 0.6 | 1.4 | 5.8 | 2.7 | 1.0 | 0.8 | 0.7 | 0.8 | 1.3 | 0.5 | 0.7 | 0.5 | 1.02 |
| YEL005C | 1.1 | 1.3 | 0.8 | 1.1 | 1.6 | 1.9 | 2.0 | 1.2 | 2.3 | 1.6 | 1.6 | 0.8 | 1.2 | 1.7 | 0.8 | 3.4 | 1.1 | 1.4 | 0.45 |
| YER048C | 0.8 | 1.2 | 0.4 | 1.4 | 1.8 | 1.9 | 1.9 | 1.6 | 2.3 | 1.1 | 1.6 | 0.8 | 1.1 | 1.0 | 0.6 | 1.8 | 1.3 | 1.6 | 1.08 |
| YER078C | 0.9 | 0.8 | 1.8 | 0.8 | 0.9 | 1.4 | 1.3 | 1.1 | 2.3 | 1.5 | 1.3 | 1.0 | 1.5 | 1.0 | 0.9 | 1.0 | 0.9 | 0.9 | 0.49 |
| YER089C | 0.5 | 0.5 | 1.4 | 1.1 | 0.8 | 0.6 | 0.9 | 0.5 | 2.0 | 2.3 | 1.2 | 0.7 | 1.3 | 0.6 | 1.0 | 0.8 | 0.7 | 0.6 | 0.75 |

| | | | | | | | | | | | | | | | | | | | |
|---|---|---|---|---|---|---|---|---|---|---|---|---|---|---|---|---|---|---|---|
| YER100W | 1.1 | 1.1 | 0.7 | 0.7 | 0.9 | 0.5 | 0.6 | 1.2 | 2.3 | 1.6 | 1.1 | 0.8 | 0.7 | 0.8 | 1.0 | 1.1 | 1.1 | 0.7 | 0.95 |
| YFR051C | 0.5 | 1.1 | 2.2 | 0.5 | 0.5 | 0.2 | 1.0 | 0.8 | 2.5 | 1.4 | 1.3 | 1.2 | 1.5 | 0.8 | 3.0 | 0.8 | 1.1 | 0.9 | 0.53 |
| YGL093W | 0.8 | 0.9 | 1.2 | 0.6 | 0.9 | 1.0 | 0.8 | 0.8 | 4.5 | 1.6 | 1.1 | 0.6 | 0.7 | 0.8 | 1.4 | 1.0 | 0.9 | 1.2 | 0.56 |
| YGL105W | 0.9 | 0.8 | 1.1 | 0.8 | 1.3 | 1.5 | 0.7 | 1.2 | 1.8 | 1.4 | 1.4 | 1.1 | 1.0 | 1.1 | 0.9 | 1.0 | 0.8 | 0.9 | 3.01 |
| YGL166W | 1.0 | 1.4 | 0.6 | 1.5 | 1.3 | 1.0 | 1.5 | 1.2 | 1.8 | 2.4 | 1.0 | 1.1 | 1.9 | 1.1 | 0.9 | 1.2 | 2.1 | 1.9 | 0.73 |
| YGL215W | 0.7 | 1.2 | 1.2 | 0.9 | 1.0 | 0.9 | 1.0 | 0.7 | 2.2 | 1.8 | 0.8 | 0.7 | 1.0 | 0.8 | 1.0 | 1.2 | 0.7 | 0.9 | 1.31 |
| YGL216W | 0.7 | 1.3 | 0.8 | 1.0 | 1.2 | 1.5 | 1.0 | 1.0 | 2.6 | 0.5 | 0.5 | 1.0 | 1.4 | 1.0 | 1.1 | 0.8 | 1.1 | 0.9 | 0.37 |
| YGL221C | 1.1 | 1.2 | 1.0 | 1.1 | 0.9 | 2.2 | 1.3 | 1.6 | 3.4 | 1.9 | 1.4 | 1.1 | 1.7 | 1.7 | 0.7 | 1.3 | 1.1 | 1.4 | 1.64 |
| YGR186W | 0.9 | 0.7 | 0.4 | 1.1 | 1.5 | 0.7 | 1.3 | 1.3 | 2.6 | 1.9 | 1.0 | 0.6 | 1.1 | 1.1 | 1.3 | 1.4 | 1.3 | 1.2 | 0.79 |
| YGR270W | 0.9 | 1.0 | 0.8 | 0.8 | 0.7 | 0.9 | 0.9 | 0.9 | 2.3 | 1.3 | 0.7 | 0.8 | 1.3 | 0.9 | 1.6 | 1.0 | 0.9 | 0.9 | 0.51 |
| YGR274C | 0.7 | 0.5 | 0.3 | 0.9 | 1.8 | 0.7 | 1.3 | 1.2 | 2.2 | 1.8 | 0.9 | 0.7 | 1.1 | 0.8 | 1.2 | 0.7 | 1.1 | 1.0 | 0.62 |
| YHR082C | 0.7 | 1.1 | 1.6 | 0.9 | 1.6 | 0.8 | 1.1 | 0.8 | 2.6 | 1.7 | 0.9 | 0.7 | 1.7 | 0.9 | 1.4 | 0.7 | 1.1 | 0.9 | 0.60 |
| YHR160C | 1.0 | 0.5 | 1.6 | 1.6 | 1.0 | 1.0 | 1.1 | 1.2 | 2.3 | 1.6 | -0.2 | 0.8 | 1.8 | 1.2 | 2.1 | 4.4 | 1.0 | 1.5 | 0.32 |
| YHR171W | 1.2 | 0.8 | 1.1 | 1.2 | 1.3 | 2.0 | 1.9 | 0.9 | 2.1 | 1.6 | 1.2 | 0.8 | 1.3 | 1.0 | 0.7 | 1.6 | 1.2 | 1.5 | 0.41 |
| YHR205W | 0.7 | 1.3 | 0.6 | 0.8 | 1.3 | 1.0 | 0.7 | 0.5 | 2.0 | 0.6 | 0.9 | 0.9 | 1.7 | 0.9 | 1.5 | 0.8 | 0.9 | 0.8 | 0.34 |
| YIL062C | 1.1 | 1.0 | 1.3 | 1.2 | 1.2 | 0.9 | 0.4 | 1.1 | 2.8 | 2.3 | 1.7 | 0.8 | 1.1 | 2.0 | 0.9 | 0.4 | 1.1 | 1.1 | 1.72 |
| YIL075C | 0.5 | 0.7 | 1.5 | 0.6 | 1.1 | 0.8 | 0.8 | 0.7 | 1.9 | 2.3 | 0.8 | 0.8 | 0.7 | 0.8 | 1.6 | 0.8 | 0.8 | 0.8 | 1.54 |
| YIR009W | 0.8 | 0.8 | 0.9 | 2.8 | 1.5 | 1.4 | 1.3 | 1.3 | 2.6 | 1.5 | 1.1 | 0.7 | 0.8 | 1.6 | 1.1 | 1.0 | 1.7 | 1.0 | 0.48 |
| YIR018W | 1.2 | 1.0 | 0.9 | 1.1 | 1.4 | 2.0 | 1.5 | 1.5 | 4.0 | 2.1 | 1.2 | 1.1 | 1.5 | 1.2 | 1.5 | 1.0 | 1.6 | 1.1 | 0.44 |
| YJR091C | 0.8 | 1.2 | 2.6 | 1.4 | 1.4 | 0.8 | 1.1 | 0.8 | 2.4 | 2.3 | 1.8 | 0.8 | 1.4 | 0.8 | 1.4 | 0.7 | 1.2 | 0.9 | 0.57 |
| YKL079W | 0.9 | 0.8 | 1.5 | 1.0 | 1.4 | 0.8 | 1.7 | 1.2 | 2.3 | 2.0 | 1.3 | 0.9 | 1.2 | 0.9 | 1.1 | 1.6 | 1.1 | 1.2 | 0.64 |
| YKR102W | 0.8 | 1.6 | 1.4 | 0.8 | 0.9 | | | 0.7 | 2.4 | 1.4 | 3.3 | 0.8 | 2.7 | 1.8 | 1.1 | 1.5 | 1.5 | 0.9 | 0.19 |
| YLL054C | 1.2 | 0.6 | 0.5 | 1.2 | 1.4 | 1.9 | 1.1 | 1.1 | 2.4 | 1.4 | 0.5 | 0.7 | 1.7 | 1.2 | 0.9 | 0.9 | 1.0 | 1.0 | 0.33 |
| YLR200W | 1.4 | 0.9 | 0.5 | 1.2 | 1.7 | 1.2 | 1.9 | 1.4 | 2.2 | 2.6 | 1.1 | 0.9 | 1.3 | 2.3 | 0.9 | 1.2 | 1.1 | 1.5 | 1.12 |
| YLR248W | 0.9 | 0.7 | 1.1 | 1.4 | 1.9 | 1.2 | 1.2 | 1.1 | 2.3 | 1.1 | 1.0 | 0.7 | 1.2 | 0.9 | 1.2 | 1.7 | 1.1 | 1.7 | 1.58 |
| YLR266C | 0.9 | 1.3 | 0.7 | 1.5 | 0.7 | 1.0 | 1.6 | 1.1 | 2.2 | 2.6 | 1.1 | 1.0 | 1.3 | 1.0 | 0.8 | 1.7 | 0.8 | 1.4 | 0.74 |
| YML088W | 0.9 | 1.6 | 0.3 | 0.5 | 1.5 | 0.7 | 1.1 | 1.1 | 4.0 | 2.2 | 0.5 | 1.0 | 1.3 | 1.1 | 1.4 | 0.8 | 0.8 | 0.8 | 0.58 |
| YMR091C | 1.0 | 0.7 | 0.8 | 1.0 | 0.7 | 0.8 | 0.8 | 1.3 | 2.1 | 1.3 | 0.9 | 0.8 | 0.9 | 1.5 | 0.9 | 1.3 | 0.9 | 1.2 | 1.03 |
| YMR110C | 1.0 | 1.4 | 1.8 | 1.9 | 1.3 | 2.1 | 1.9 | 0.9 | 2.3 | 1.6 | 1.6 | 0.9 | 1.8 | 0.8 | 0.9 | 2.7 | 1.5 | 2.5 | 1.63 |
| YMR255W | 1.2 | 1.4 | 0.7 | 1.8 | 1.3 | 0.9 | 1.5 | 1.4 | 2.8 | 2.4 | 1.2 | 0.9 | 1.1 | 1.6 | 1.0 | 1.3 | 1.5 | 2.0 | 0.97 |
| YNL039W | 0.7 | 0.8 | 0.3 | 0.4 | 2.1 | 0.8 | 1.1 | 1.2 | 2.1 | 1.8 | 0.4 | 0.9 | 0.8 | 0.8 | 1.3 | 1.3 | 0.9 | 1.1 | 0.61 |
| YNL077W | 1.5 | 1.1 | 2.3 | 1.2 | 1.5 | 0.9 | 1.2 | 1.0 | 4.4 | 5.1 | 1.0 | 0.7 | 1.5 | 0.5 | 0.3 | 0.6 | 1.3 | 1.0 | 0.78 |

| | | | | | | | | | | | | | | | | | | |
|---|---|---|---|---|---|---|---|---|---|---|---|---|---|---|---|---|---|---|
| YNL083W | 0.8 | 1.4 | 1.3 | 1.2 | 0.8 | 0.7 | 0.9 | 1.1 | 3.1 | 1.6 | 0.9 | 0.9 | 1.2 | 0.9 | 0.8 | 1.8 | 0.8 | 1.1 | 0.37 |
| YNL147W | 1.3 | 2.4 | 0.6 | 0.7 | 2.0 | 1.5 | 1.4 | 1.8 | 2.1 | 2.9 | 1.2 | 0.8 | 1.0 | 2.0 | 1.2 | 1.7 | 1.0 | 1.6 | 1.32 |
| YNR006W | 1.1 | 1.8 | 0.5 | 0.9 | 1.5 | 0.9 | 1.5 | 0.9 | 2.2 | 2.3 | 0.8 | 0.9 | 1.6 | 0.9 | 1.2 | 1.1 | 1.8 | 1.2 | 0.40 |
| YNR034W | 0.9 | 1.4 | 5.3 | 1.6 | 1.0 | 0.9 | 1.3 | 1.0 | 3.0 | 2.1 | 1.3 | 0.9 | 1.4 | 0.9 | 1.1 | 2.3 | 0.7 | 1.1 | 0.64 |
| YNR047W | 0.7 | 1.0 | 1.7 | 0.5 | 1.1 | 1.0 | 1.0 | 0.8 | 2.0 | 1.2 | 0.6 | 0.9 | 1.5 | 0.9 | 1.2 | 1.5 | 0.9 | 1.0 | 0.44 |
| YOR023C | 0.7 | 1.2 | 1.3 | 0.8 | 1.2 | 1.8 | 1.1 | 0.6 | 3.5 | 1.3 | 0.9 | 1.1 | 1.9 | 0.9 | 1.6 | 0.8 | 1.1 | 0.8 | 0.37 |
| YOR058C | 2.0 | 1.4 | 2.0 | 0.6 | 0.9 | 0.9 | 0.8 | 1.7 | 6.4 | 4.5 | 1.4 | 0.7 | 1.7 | 1.7 | 1.1 | 1.6 | 0.9 | 1.0 | 0.31 |
| YOR069W | 0.8 | 1.6 | 0.7 | 1.3 | 1.0 | 0.5 | 1.2 | 1.1 | 1.9 | 1.4 | 2.8 | 0.9 | 1.1 | 1.3 | 1.4 | 1.5 | 0.8 | 1.1 | 0.69 |
| YOR229W | 0.9 | 0.9 | 1.0 | 0.8 | 1.5 | 1.3 | 1.1 | 0.8 | 2.5 | 0.8 | 0.7 | 0.8 | 1.3 | 1.0 | 1.2 | 0.9 | 0.9 | 1.0 | 0.58 |
| YOR256C | 1.0 | 0.8 | 0.9 | 1.5 | 1.7 | 0.9 | 1.4 | 1.1 | 2.7 | 1.8 | 1.7 | 0.7 | 1.6 | 1.1 | 1.1 | 1.5 | 1.2 | 1.5 | 0.53 |
| YPL020C | 1.1 | 0.7 | 0.7 | 1.3 | 0.8 | 0.8 | 1.1 | 1.1 | 2.3 | 1.6 | 1.1 | 0.9 | 1.1 | 1.1 | 1.6 | 1.2 | 1.3 | 1.4 | 0.91 |
| YPL105C | 0.6 | 0.8 | 0.6 | 0.7 | 1.1 | 1.2 | 0.7 | 1.0 | 3.4 | 1.4 | 0.6 | 0.7 | 1.0 | 0.6 | 1.1 | 0.5 | 0.8 | 0.6 | 0.80 |
| YPR066W | 1.2 | 2.2 | 1.1 | 2.2 | 1.3 | 0.7 | 1.3 | 0.8 | 2.3 | 1.9 | 0.9 | 0.8 | 1.4 | 1.2 | 0.7 | 1.6 | 1.1 | 1.2 | 0.41 |
| YPR081C | 0.9 | 1.0 | 1.0 | 1.1 | 0.8 | 1.1 | 1.1 | 1.0 | 4.2 | 2.6 | 0.9 | 1.0 | 1.4 | 1.1 | 1.3 | 1.8 | 0.9 | 1.3 | 0.34 |
| YPR140W | 0.9 | 1.2 | 0.8 | 1.8 | 0.9 | 3.0 | 1.9 | 1.1 | 2.1 | 1.3 | 0.8 | 0.8 | 1.3 | 1.4 | 1.7 | 2.4 | 0.8 | 1.3 | 0.47 |
| YPR155C | 0.9 | 2.5 | 1.5 | 1.4 | 0.9 | 0.9 | 1.4 | 1.0 | 2.0 | 1.3 | 0.9 | 0.9 | 1.3 | 1.2 | 0.5 | 3.4 | 0.8 | 1.6 | 0.40 |
| YPR185W | 0.7 | 0.9 | 0.3 | 0.4 | 1.1 | 0.9 | 1.2 | 1.0 | 2.5 | 1.7 | 0.9 | 0.8 | 1.7 | 1.0 | 1.1 | 1.3 | 1.3 | 1.1 | 0.68 |
| YBR076W | 2.3 | 1.1 | 1.4 | 5.0 | 0.9 | 0.8 | 1.0 | 0.7 | 0.4 | 1.8 | 0.8 | 0.9 | 1.9 | 1.2 | 1.1 | 0.5 | 1.4 | 1.2 | 0.36 |
| YDR373W | 1.7 | 0.8 | 0.5 | 1.3 | 1.8 | 0.9 | 1.4 | 1.9 | 2.5 | 2.8 | 1.7 | 0.5 | 1.0 | 2.1 | 1.6 | 1.4 | 1.2 | 1.6 | 1.08 |
| YFR014C | 2.1 | 2.4 | 0.8 | 2.6 | 0.7 | 0.8 | 1.4 | 1.3 | 1.0 | 1.6 | 1.8 | 0.9 | 1.3 | 1.0 | 0.6 | 2.3 | 1.2 | 2.9 | 0.94 |
| YHR136C | 2.4 | 13.6 | 1.4 | 2.1 | 2.4 | 1.4 | 0.5 | 1.3 | 0.1 | 2.0 | 2.0 | 1.2 | 0.3 | 1.7 | 0.5 | 1.3 | 1.6 | 2.5 | 1.33 |
| YIL129C | 0.8 | 5.8 | 1.0 | 1.2 | | 0.3 | | | 2.1 | 1.8 | 1.5 | | 1.9 | 0.9 | 1.0 | 1.0 | 1.3 | 0.8 | 0.25 |
| YMR077C | 1.1 | 3.9 | 1.1 | 1.8 | 1.0 | 0.5 | 1.4 | 1.7 | 1.9 | 2.5 | 1.5 | 0.9 | 0.8 | 1.6 | 1.7 | 2.3 | 0.9 | 1.3 | 0.59 |
| YBR264C | 1.2 | 3.7 | 1.5 | 1.5 | 0.7 | 0.9 | 1.3 | 1.2 | 0.9 | 1.1 | 1.0 | 1.0 | 0.9 | 1.6 | 1.1 | 1.8 | 0.9 | 1.3 | 0.61 |
| YPL177C | 1.2 | 3.7 | 0.9 | 1.3 | 0.7 | 0.8 | 0.8 | 1.3 | 0.5 | 1.0 | 1.4 | 0.8 | 0.8 | 1.3 | 0.6 | 1.9 | 1.2 | 1.1 | 0.75 |
| YGL056C | 0.7 | 3.5 | 1.3 | 1.0 | 0.6 | 0.4 | 1.0 | 0.9 | 0.4 | 0.6 | 0.0 | 1.1 | 0.8 | 1.1 | 1.2 | 1.5 | 0.9 | 1.1 | 0.47 |
| YBL101W-A | 1.0 | 5.7 | 0.7 | 0.6 | 1.0 | 1.4 | 1.3 | 1.5 | 1.2 | 2.1 | 1.2 | 1.1 | 2.0 | 1.5 | 2.1 | 1.1 | 1.3 | 1.2 | 1.24 |
| YER072W | 1.6 | 2.3 | 1.6 | 1.3 | 1.2 | 1.5 | 0.7 | 1.2 | 0.5 | 1.1 | 1.7 | 1.1 | 0.6 | 1.5 | 0.9 | 1.1 | 1.7 | 1.7 | 1.69 |
| YMR112C | 1.2 | 4.2 | 0.9 | 1.2 | 1.0 | 0.7 | 1.5 | 1.6 | 1.4 | 1.3 | 0.9 | 1.0 | 0.9 | 1.8 | 0.9 | 1.4 | 1.1 | 1.6 | 0.57 |
| YJR058C | 1.3 | 2.0 | 1.3 | 1.5 | 1.1 | 1.6 | 1.3 | 1.2 | 1.9 | 2.6 | 1.2 | 0.7 | 0.8 | 1.8 | 0.8 | 1.2 | 1.3 | 1.2 | 1.00 |
| YML055W | 1.7 | 2.3 | 0.6 | 2.0 | 0.9 | 1.2 | 1.7 | 1.7 | 1.0 | 1.4 | 1.4 | 1.0 | 1.7 | 1.9 | 0.6 | 1.4 | 1.2 | 1.5 | 1.08 |
| YDR080W | 0.9 | 1.8 | 0.5 | 0.6 | 1.2 | 1.5 | 1.6 | 1.2 | 1.7 | 2.6 | 1.2 | 1.0 | 2.0 | 1.1 | 1.2 | 1.7 | 1.2 | 1.3 | 0.68 |

| | | | | | | | | | | | | | | | | | | | |
|---|---|---|---|---|---|---|---|---|---|---|---|---|---|---|---|---|---|---|---|
| YNR030W | 0.6 | 2.1 | 2.2 | 0.7 | 0.8 | 1.2 | 1.0 | 0.6 | 1.0 | 1.4 | 1.2 | 0.8 | 1.5 | 0.6 | 1.1 | 0.4 | 0.9 | 0.8 | 1.19 |
| YNL196C | 1.1 | 1.8 | 1.4 | 1.3 | 0.9 | 1.2 | 1.0 | 0.7 | 0.5 | 2.2 | 0.7 | 1.0 | 2.2 | 1.2 | 0.8 | 2.1 | 1.1 | 1.2 | 0.50 |
| YOR148C | 1.4 | 1.4 | 0.5 | 1.8 | 0.9 | 0.9 | 1.2 | 1.5 | 0.8 | 0.8 | 1.3 | 0.9 | 1.4 | 1.4 | 1.0 | 1.3 | 1.0 | 1.3 | 1.08 |
| YER029C | 1.0 | 1.6 | 0.4 | 1.9 | 1.7 | 1.2 | 1.1 | 1.5 | 1.4 | 1.1 | 0.8 | 0.9 | 0.9 | 1.4 | 1.1 | 1.7 | 0.9 | 1.3 | 1.20 |
| YLR360W | 0.9 | 2.0 | 0.5 | 0.7 | 1.9 | 1.4 | 1.7 | 1.3 | 0.9 | 1.3 | 0.9 | 0.9 | 0.7 | 1.1 | 0.8 | 2.1 | 1.2 | 1.4 | 0.53 |
| YGR239C | 1.3 | 1.3 | 0.7 | 1.3 | 1.2 | 2.3 | 1.2 | 1.5 | 0.5 | 3.8 | 1.4 | 1.0 | 1.0 | 1.4 | 1.3 | 1.1 | 1.1 | 1.3 | 0.49 |
| YDL229W | 0.9 | 1.9 | 0.4 | 0.6 | 1.2 | 1.0 | 1.3 | 1.2 | 1.4 | 0.9 | 1.6 | 0.9 | 1.0 | 1.3 | 1.2 | 1.0 | 1.2 | 1.1 | 0.76 |
| YJR027W | 0.8 | 1.9 | 0.8 | 0.7 | | 1.5 | 2.0 | 1.0 | 1.2 | 1.3 | 1.1 | 0.8 | 1.3 | 1.1 | 2.3 | 0.7 | 0.7 | 1.1 | 4.88 |
| YKL198C | 1.2 | 1.4 | 0.8 | 0.2 | 0.9 | 1.0 | 1.2 | 0.6 | 0.0 | 0.8 | 1.4 | 0.7 | 1.9 | 6.6 | 2.6 | 0.7 | 1.0 | 0.8 | 0.17 |
| YBR031W | 1.1 | 0.8 | 4.1 | 1.4 | 1.4 | 0.7 | 0.5 | 1.0 | 0.1 | 1.0 | 0.9 | 1.0 | 0.3 | 0.4 | 0.9 | 0.7 | 1.0 | 0.8 | 7.58 |
| YBR118W | 1.3 | 0.8 | 2.9 | 1.2 | 1.0 | 1.3 | 1.0 | 1.3 | 0.5 | 0.9 | 1.0 | 0.8 | 0.8 | 0.7 | 1.6 | 0.6 | 1.2 | 0.9 | 8.91 |
| YCR106W | 0.9 | 1.1 | 2.8 | 1.1 | 0.8 | 1.4 | 0.9 | 0.8 | 2.0 | 1.2 | 1.2 | 0.8 | 1.2 | 0.8 | 1.3 | 1.2 | 1.1 | 1.2 | 0.76 |
| YDR012W | 0.9 | 0.8 | 3.9 | 1.2 | 1.1 | 0.9 | 0.5 | 1.1 | 0.3 | 0.7 | 0.9 | 1.2 | 0.2 | 0.4 | 0.8 | 0.7 | 1.0 | 0.7 | 7.07 |
| YDR134C | 1.0 | 0.7 | 3.9 | 1.9 | 0.8 | 1.1 | 0.4 | 0.6 | 0.1 | 0.5 | 1.9 | 0.6 | 0.4 | 0.5 | 0.8 | 0.9 | 0.7 | 1.1 | 5.84 |
| YDR276C | 1.5 | 1.6 | 6.1 | 1.4 | 0.7 | 1.1 | 1.0 | 1.1 | 1.1 | 2.7 | 1.4 | 0.9 | 1.1 | 1.3 | 2.2 | 3.6 | 1.3 | 2.4 | 2.48 |
| YGR279C | 0.9 | 0.7 | 3.6 | 1.0 | 1.2 | 1.6 | 0.8 | 0.6 | 0.3 | 0.7 | 0.9 | 0.7 | 0.3 | 0.4 | 1.3 | 0.8 | 1.0 | 0.8 | 3.77 |
| YJL059W | 1.0 | 1.7 | 3.1 | 0.8 | 0.9 | 1.6 | 1.1 | 0.8 | 1.7 | 1.6 | 1.8 | 1.0 | 1.6 | 1.3 | 0.9 | 0.8 | 1.1 | 1.0 | 0.39 |
| YKL056C | 1.3 | 0.7 | 2.6 | 1.4 | 1.7 | 0.9 | 0.5 | 1.0 | 0.1 | 0.7 | 1.1 | 0.8 | 0.1 | 0.7 | 0.9 | 0.6 | 0.8 | 1.2 | 6.68 |
| YKL097W-A | 1.5 | 1.3 | 4.2 | 1.3 | 1.2 | 0.9 | 0.2 | 0.7 | 0.0 | 0.4 | 1.6 | 0.7 | 0.2 | 0.6 | 2.0 | 0.8 | 1.1 | 1.4 | 4.01 |
| YNL209W | 0.9 | 0.9 | 2.7 | 0.9 | 1.3 | 0.9 | 0.5 | 1.1 | 0.2 | 0.8 | 0.9 | 0.9 | 0.5 | 0.6 | 0.9 | 0.3 | 0.6 | 0.6 | 8.06 |
| YNL307C | 0.8 | 0.6 | 3.1 | 1.3 | 1.0 | 1.9 | 0.9 | 0.9 | 1.0 | 0.9 | 1.0 | 1.0 | 1.0 | 0.7 | 0.6 | 1.0 | 1.0 | 1.3 | 2.79 |
| YPR028W | 1.0 | 1.1 | 4.0 | 1.7 | 1.0 | 1.4 | 0.8 | 0.7 | 0.5 | 0.8 | 1.4 | 0.6 | 1.7 | 0.8 | 0.9 | 1.3 | 1.2 | 1.7 | 4.06 |
| YPR149W | 1.0 | 1.1 | 5.9 | 1.7 | 1.7 | 1.6 | 2.1 | 0.6 | 1.2 | 1.8 | 1.1 | 0.7 | 1.4 | 1.3 | 1.0 | 2.2 | 1.1 | 1.1 | 2.65 |
| YAL016W | 0.8 | 1.0 | 2.8 | 0.9 | 0.9 | 0.9 | 1.1 | 1.0 | 1.2 | 1.9 | 0.9 | 0.8 | 1.4 | 0.9 | 1.1 | 1.1 | 0.8 | 1.3 | 1.00 |
| YBR283C | 0.6 | 0.7 | 1.9 | 1.2 | 1.4 | 2.2 | 0.8 | 0.8 | 1.5 | 1.2 | 2.0 | 0.8 | 0.8 | 0.6 | 1.4 | 0.9 | 0.9 | 0.9 | 2.62 |
| YBR286W | 0.7 | 1.4 | 3.8 | 1.2 | 1.3 | 1.5 | 1.3 | 0.9 | 1.2 | 1.6 | 1.0 | 1.1 | 1.8 | 1.5 | 1.3 | 2.6 | 0.8 | 1.6 | 2.98 |
| YCL008C | 0.8 | 1.1 | 1.8 | 1.2 | 1.2 | 0.9 | 0.9 | 0.7 | 0.9 | 1.4 | 0.9 | 0.9 | 1.0 | 0.9 | 1.4 | 0.6 | 0.9 | 0.9 | 0.47 |
| YCR069W | 1.0 | 1.2 | 2.0 | 0.9 | 1.1 | 1.4 | 1.1 | 0.7 | 0.8 | 0.7 | 1.3 | 1.4 | 1.6 | 1.0 | 2.4 | 1.2 | 1.2 | 1.3 | 0.97 |
| YDL061C | 1.4 | 0.5 | 2.9 | 0.9 | 1.3 | 1.1 | 0.5 | 1.0 | 0.0 | 0.5 | 1.4 | 1.0 | 0.1 | 0.7 | 1.0 | 0.6 | 1.1 | 1.0 | 4.40 |
| YDR151C | 1.3 | 1.0 | 2.3 | 1.8 | 0.8 | 0.7 | 0.8 | 1.5 | 2.6 | 2.8 | 2.1 | 0.9 | 2.8 | 1.1 | 0.6 | 1.2 | 0.8 | 1.1 | 0.96 |
| YDR382W | 1.3 | 0.5 | 2.4 | 1.2 | 1.1 | 0.6 | 0.4 | 1.2 | 0.0 | 0.4 | 1.2 | 0.7 | 0.1 | 0.7 | 1.1 | 0.4 | 1.0 | 1.0 | 6.40 |
| YDR385W | 0.6 | 0.4 | 3.5 | 0.9 | 1.1 | 0.5 | 0.4 | 0.7 | 0.2 | 0.5 | 0.9 | 0.9 | 0.3 | 0.5 | 0.8 | 0.3 | 0.6 | 0.4 | 7.27 |

| | | | | | | | | | | | | | | | | | | |
|---|---|---|---|---|---|---|---|---|---|---|---|---|---|---|---|---|---|---|
| YDR407C | 0.7 | 1.0 | 2.0 | 0.7 | 0.8 | 1.2 | 1.4 | 0.9 | 1.5 | 1.7 | 1.2 | 1.0 | 1.3 | 0.8 | 1.3 | 1.0 | 0.9 | 0.9 | 0.52 |
| YGL206C | 0.4 | 3.5 | 2.0 | 0.5 | 0.6 | 0.8 | 0.8 | 0.5 | 0.7 | 0.7 | 0.9 | 0.7 | 1.1 | 0.8 | 1.3 | 0.6 | 0.8 | 1.0 | 1.04 |
| YGR172C | 0.6 | 1.3 | 2.5 | 1.3 | 0.6 | 0.9 | 1.0 | 0.8 | 0.4 | 1.1 | 0.8 | 0.8 | 0.7 | 1.1 | 1.9 | 1.0 | 1.1 | 1.1 | 0.94 |
| YIL015W | 1.1 | 1.7 | 2.0 | 2.5 | 0.9 | 0.4 | 0.9 | 0.9 | 0.3 | 1.1 | 0.8 | 0.9 | 0.8 | 5.9 | 1.1 | 0.8 | 0.8 | 1.0 | 0.17 |
| YIL018W | 1.0 | 0.4 | 2.3 | 1.4 | | 0.9 | 0.4 | 0.8 | 0.0 | 0.3 | 1.2 | 0.6 | 0.0 | 0.6 | 0.6 | 0.4 | 0.8 | 0.8 | 6.27 |
| YJL138C | 1.0 | 0.7 | 2.3 | 1.4 | 0.9 | 0.8 | 0.8 | 1.2 | 0.2 | 0.7 | 1.2 | 0.8 | 0.3 | 0.7 | 0.9 | 0.6 | 0.9 | 1.0 | 7.38 |
| YJL191W | 1.0 | 0.4 | 2.2 | 0.7 | | 1.3 | 1.0 | 0.8 | 0.0 | 0.4 | 1.1 | 1.0 | 0.9 | 1.1 | 0.7 | 2.2 | 1.0 | 1.0 | 1.86 |
| YJR047C | 1.1 | 0.8 | 2.6 | 1.1 | 1.1 | 1.1 | 0.4 | 0.9 | 0.7 | 0.5 | 1.1 | 0.9 | 0.9 | 0.9 | 0.6 | 0.8 | 0.8 | 0.9 | 5.29 |
| YJR119C | 0.9 | 1.0 | 2.0 | 1.0 | 0.9 | 1.4 | 1.1 | 1.6 | 0.5 | 1.3 | 1.1 | 0.9 | 1.2 | 1.3 | 1.5 | 1.0 | 0.7 | 0.9 | 0.30 |
| YJR123W | 1.2 | 0.6 | 2.2 | 1.0 | 1.3 | 1.0 | 0.4 | 1.2 | 0.0 | 0.5 | 0.9 | 0.8 | 0.1 | 0.6 | 1.1 | 0.4 | 0.8 | 0.9 | 7.84 |
| YJR145C | 0.8 | 0.7 | 2.3 | 1.3 | | 0.4 | 0.4 | 1.0 | 0.0 | 0.4 | 0.8 | 0.6 | 0.0 | 0.6 | 0.8 | 0.5 | 0.8 | 0.9 | 4.82 |
| YLR110C | 1.4 | 0.8 | 2.7 | 1.2 | 1.7 | 0.9 | 0.4 | 0.8 | 0.3 | 0.8 | 3.2 | 0.7 | 0.5 | 0.5 | 1.7 | 0.8 | 0.9 | 1.1 | 3.99 |
| YLR264W | 1.1 | 0.4 | 2.2 | 1.0 | 1.1 | 0.7 | 0.4 | 1.0 | 0.0 | 0.3 | 0.8 | 1.1 | 0.3 | 1.2 | 1.2 | 0.5 | 1.0 | 1.1 | 3.91 |
| YLR340W | 0.8 | 0.4 | 2.6 | 1.0 | 0.6 | 0.7 | 0.3 | 0.9 | 0.0 | 0.5 | 1.4 | 0.9 | 0.1 | 0.5 | 1.3 | 0.2 | 0.9 | 0.7 | 6.55 |
| YLR388W | 1.6 | 0.4 | 1.9 | 1.0 | 2.5 | 0.7 | 0.5 | 1.2 | 0.2 | 0.7 | 1.0 | 0.6 | 0.1 | 0.8 | 0.6 | 0.5 | 0.8 | 1.0 | 4.48 |
| YMR092C | 0.6 | 1.2 | 1.9 | 1.3 | 0.8 | 0.9 | 1.2 | 0.9 | 1.5 | 1.7 | 1.2 | 0.9 | 1.7 | 0.8 | 1.1 | 1.2 | 0.9 | 1.3 | 1.43 |
| YMR101C | 1.1 | 1.8 | 1.9 | 2.9 | 0.9 | 0.4 | 0.9 | 0.8 | -4.0 | 1.6 | -0.3 | 0.7 | 0.8 | 1.1 | 1.0 | 0.9 | 0.7 | 0.8 | 0.16 |
| YNL069C | 1.2 | 0.3 | 2.8 | 2.3 | 0.7 | 0.4 | 0.3 | 1.0 | 0.0 | 0.4 | 1.0 | 0.6 | 0.1 | 0.9 | 1.0 | 0.3 | 0.8 | 0.6 | 4.03 |
| YNL135C | 1.1 | 0.9 | 2.0 | 1.4 | 1.7 | 0.9 | 0.8 | 0.5 | 0.7 | 0.5 | 0.7 | 0.8 | 0.9 | 0.5 | 1.0 | 1.4 | 1.2 | 1.7 | 3.05 |
| YOL039W | 1.2 | 0.3 | 3.9 | 0.7 | 1.3 | 0.4 | 0.3 | 1.0 | 0.0 | 0.9 | 1.1 | 0.7 | 0.1 | 0.5 | 0.8 | 0.4 | 0.7 | 0.7 | 4.94 |
| YOL120C | 1.0 | 0.3 | 2.3 | 0.8 | 1.5 | 0.3 | 0.4 | 0.8 | 0.0 | 0.3 | 1.0 | 0.7 | 0.1 | 0.6 | 0.6 | 0.3 | 0.6 | 0.8 | 4.42 |
| YOR230W | 1.2 | 2.3 | 3.0 | 1.0 | 0.9 | 1.1 | 0.6 | 0.6 | 0.6 | 0.7 | 1.6 | 1.4 | 1.5 | 0.7 | 0.6 | 1.6 | 1.2 | 1.6 | 1.76 |
| YOR298W | 0.9 | 0.9 | 3.2 | 0.5 | 1.5 | 0.9 | 0.6 | 0.7 | 0.6 | 1.0 | 0.1 | 0.7 | 1.1 | 2.1 | 1.2 | 0.5 | 1.0 | 0.8 | 0.41 |
| YPL048W | 0.7 | 0.7 | 2.6 | 1.2 | 0.7 | 0.6 | 0.6 | 0.8 | 1.6 | 0.6 | 1.7 | 1.0 | 0.9 | 0.6 | 1.5 | 1.3 | 1.2 | 1.0 | 2.11 |
| YPL179W | 1.0 | 1.5 | 2.4 | 1.0 | 1.1 | 1.6 | 1.2 | 0.8 | 1.2 | 1.1 | 1.1 | 1.0 | 1.3 | 0.8 | 1.2 | 1.4 | 1.3 | 1.3 | 1.38 |
| YPL218W | 1.5 | 0.9 | 2.5 | 1.7 | 1.1 | 1.5 | 1.3 | 1.4 | 1.1 | 1.2 | 1.1 | 1.2 | 1.0 | 1.5 | 1.1 | 1.7 | 1.1 | 1.8 | 2.98 |
| YPL220W | 1.3 | 0.9 | 2.3 | 1.4 | 1.1 | 1.3 | 0.6 | 1.2 | 0.0 | 0.5 | 1.3 | 0.5 | 0.1 | 0.6 | 0.6 | 0.5 | 1.1 | 1.1 | 8.33 |
| YPR080W | 1.3 | 1.3 | 2.8 | 1.2 | 1.4 | 1.4 | 0.9 | 1.3 | 0.7 | 1.1 | 1.0 | 1.0 | 1.3 | 0.6 | 1.5 | 0.6 | 1.1 | 1.0 | 8.03 |
| YPR181C | 0.5 | 0.8 | 2.3 | 0.6 | 1.0 | 2.0 | 1.3 | 0.5 | 1.0 | 0.7 | 1.3 | 1.0 | 1.7 | 2.4 | 1.5 | 1.6 | 1.8 | 1.6 | 1.84 |
| YBR290W | 1.3 | 1.4 | 0.8 | 3.0 | 1.0 | 1.1 | 1.4 | 1.5 | 2.0 | 1.8 | 1.2 | 1.0 | 1.7 | 1.4 | 0.9 | 1.3 | 1.2 | 2.1 | 1.34 |
| YCR091W | 1.3 | 1.1 | 0.6 | 2.2 | 1.3 | 1.3 | 1.3 | 1.1 | 0.8 | 1.2 | 0.8 | 1.1 | 1.3 | 1.3 | 0.7 | 2.7 | 1.3 | 1.4 | 0.22 |
| YFL026W | 1.0 | 0.8 | 1.2 | 5.0 | 1.4 | 1.0 | 1.2 | 1.3 | 0.6 | 1.5 | 1.3 | 0.8 | 1.1 | 1.1 | 1.0 | 1.0 | 1.0 | 1.0 | 0.27 |

| | | | | | | | | | | | | | | | | | | | |
|---|---|---|---|---|---|---|---|---|---|---|---|---|---|---|---|---|---|---|---|
| YOR003W | 0.9 | 1.1 | 1.3 | 3.3 | 1.6 | 1.1 | 1.1 | 0.9 | 1.6 | 1.3 | 0.7 | 0.8 | 1.0 | 1.2 | 2.8 | 1.3 | 1.0 | 1.1 | 0.30 |
| YCR038C | 0.9 | 1.1 | 0.9 | 2.5 | 0.9 | 0.9 | 0.9 | 0.9 | 0.7 | 0.7 | 1.1 | 0.8 | 1.0 | 1.1 | 0.7 | 1.0 | 0.9 | 1.1 | 0.31 |
| YDL119C | 1.0 | 0.8 | 0.6 | 2.2 | 0.9 | 0.6 | 1.3 | 1.1 | 1.3 | 1.1 | 1.5 | 0.9 | 1.1 | 1.2 | 0.5 | 1.2 | 1.0 | 1.0 | 0.77 |
| YDL220C | 0.9 | 1.3 | 1.1 | 1.6 | 1.2 | 1.8 | 1.1 | 0.8 | 0.2 | 1.3 | 0.8 | 1.0 | 1.6 | 0.9 | 1.6 | 0.8 | 1.1 | 0.9 | 0.24 |
| YDR125C | 1.1 | 0.9 | 1.0 | 1.7 | 1.1 | 0.8 | 1.0 | 1.2 | 0.7 | 1.9 | 1.0 | 0.8 | 0.9 | 1.0 | 0.8 | 1.7 | 0.9 | 1.2 | 0.35 |
| YDR225W | 1.0 | 0.6 | 0.6 | 1.6 | 1.9 | 0.5 | 0.4 | 0.8 | 0.3 | 0.7 | 2.7 | 0.4 | 0.2 | 0.8 | 0.1 | 0.8 | 1.1 | 1.8 | 5.54 |
| YER066W | 0.9 | 0.7 | 0.9 | 2.1 | 1.5 | 1.3 | 1.3 | 0.7 | 1.7 | 1.4 | 1.5 | 0.7 | 0.9 | 1.1 | 0.5 | 3.3 | 1.1 | 1.1 | 0.66 |
| YER076C | 0.8 | 0.9 | 1.2 | 1.9 | 1.1 | 0.6 | 0.8 | 0.7 | 0.2 | 1.2 | 0.7 | 0.7 | 1.1 | 0.9 | 0.8 | 1.3 | 1.0 | 1.0 | 0.33 |
| YFR006W | 0.9 | 1.8 | 1.1 | 3.4 | 0.7 | 0.8 | 1.4 | 1.0 | -1.6 | 0.7 | 1.2 | 1.0 | 0.9 | 0.9 | 0.8 | 1.2 | 0.9 | 1.3 | 1.54 |
| YGL208W | 0.9 | 1.8 | 1.1 | 2.2 | 0.7 | 0.6 | 1.1 | 0.7 | 1.4 | 1.3 | 1.3 | 0.8 | 1.5 | 0.9 | 1.8 | 3.1 | 0.8 | 1.3 | 0.32 |
| YGR023W | 1.0 | 0.9 | 1.1 | 4.2 | 1.0 | 1.2 | 1.0 | 1.2 | 1.2 | 3.2 | 2.4 | 0.9 | 1.4 | 1.3 | 1.1 | 0.9 | 1.2 | 1.5 | 0.69 |
| YGR108W | 0.9 | 3.9 | 0.3 | 3.6 | 0.8 | 0.1 | 0.3 | 0.5 | 0.0 | 0.1 | 0.5 | 0.4 | 0.3 | 0.8 | 0.2 | 0.4 | 0.7 | 1.0 | 0.93 |
| YHR195W | 1.1 | 1.5 | 0.6 | 2.4 | 1.5 | 1.0 | 1.3 | 1.6 | 1.8 | 0.8 | 1.4 | 0.9 | 1.2 | 1.2 | 0.7 | 3.7 | 1.0 | 1.5 | 1.12 |
| YIL050W | 1.0 | 1.3 | 1.3 | 2.4 | 0.9 | 0.7 | 1.1 | 1.2 | 1.3 | 1.9 | 0.9 | 1.0 | 1.0 | 1.3 | 0.6 | 2.7 | 1.0 | 1.1 | 0.52 |
| YJR050W | 1.0 | 0.6 | 0.7 | 2.1 | 1.2 | 1.4 | 1.1 | 1.8 | 2.0 | 1.3 | 0.8 | 0.8 | 1.9 | 1.3 | 0.9 | 1.5 | 0.9 | 1.1 | 0.85 |
| YKL093W | 1.0 | 1.6 | 1.4 | 3.6 | 0.8 | 0.8 | 0.7 | 0.9 | 1.5 | 1.6 | 0.9 | 0.9 | 1.2 | 1.1 | 0.8 | 2.9 | 0.8 | 1.0 | 0.38 |
| YMR053C | 1.1 | 1.0 | 0.8 | 2.1 | 0.9 | 1.3 | 0.9 | 1.0 | 2.2 | 1.4 | 1.3 | 0.9 | 1.5 | 1.3 | 2.4 | 2.3 | 1.1 | 1.6 | 0.31 |
| YNL139C | 0.8 | 1.1 | 0.8 | 2.0 | 1.9 | 1.0 | 1.0 | 0.9 | 0.6 | 0.7 | 0.8 | 0.5 | 1.2 | 0.7 | 1.4 | 1.1 | 1.1 | 1.0 | 0.47 |
| YOR122C | 1.0 | 0.7 | 1.8 | 1.9 | 1.7 | 1.3 | 1.2 | 1.0 | 1.1 | 0.9 | 1.5 | 0.8 | 1.0 | 1.2 | 1.6 | 1.5 | 1.0 | 2.2 | 3.06 |
| YOR312C | 1.2 | 0.4 | 1.0 | 1.9 | 0.7 | 0.6 | 0.6 | 1.2 | 0.0 | 0.3 | 0.8 | 0.7 | 0.1 | 0.7 | 0.7 | 0.5 | 1.2 | 1.0 | 5.24 |
| YOR327C | 2.3 | 1.8 | 0.5 | 1.9 | 1.0 | 1.4 | 1.4 | 1.9 | 1.4 | 2.2 | 2.1 | 1.0 | 2.1 | 1.5 | 0.9 | 1.5 | 2.2 | 2.1 | 1.46 |
| YPL001W | 0.9 | 0.9 | 1.0 | 2.5 | 1.7 | 0.7 | 1.0 | 1.2 | 0.7 | 1.0 | 1.3 | 0.7 | 0.8 | 1.3 | 1.3 | 0.8 | 0.9 | 1.2 | 0.74 |
| YPL230W | 1.2 | 1.7 | 1.8 | 4.0 | 0.8 | 1.0 | 1.1 | 1.0 | 0.9 | 2.7 | 3.1 | 1.0 | 2.0 | 1.3 | 0.7 | 2.0 | 1.0 | 1.6 | 0.47 |
| YER025W | 0.6 | 0.7 | 0.6 | 0.6 | 1.5 | 1.0 | 1.5 | 1.1 | 0.4 | 0.6 | 1.2 | 0.7 | 0.6 | 2.1 | 0.8 | 2.6 | 1.8 | 2.3 | 1.96 |
| YHR185C | 0.9 | 1.1 | 1.4 | 0.7 | 1.4 | 1.0 | 1.3 | 0.8 | -0.6 | 0.9 | 0.7 | 0.8 | 0.4 | 1.7 | 1.3 | 3.2 | 1.4 | 3.0 | 1.64 |
| YIL076W | 1.3 | 0.6 | 1.1 | 1.0 | 1.8 | 0.6 | 0.5 | 1.3 | 1.0 | 1.4 | 1.1 | 0.8 | 0.8 | 1.4 | 1.0 | 0.8 | 1.2 | 2.5 | 3.73 |
| YMR238W | 1.9 | 1.3 | 1.1 | 1.3 | 1.5 | 1.3 | 1.2 | 0.9 | 1.3 | 1.4 | 1.2 | 1.0 | 1.5 | 1.2 | 2.3 | 1.4 | 2.2 | 2.4 | 1.36 |
| YBR009C | 1.6 | 0.8 | 0.4 | 1.7 | 1.4 | 1.0 | 0.5 | 1.2 | 0.6 | 0.6 | 0.8 | 0.5 | 0.4 | 1.1 | 0.2 | 0.4 | 1.0 | 2.4 | 7.00 |
| YBR010W | 1.0 | 0.8 | 0.5 | 2.0 | 0.8 | 1.1 | 0.8 | 1.3 | 0.5 | 0.7 | 1.0 | 0.7 | 0.4 | 1.0 | 0.3 | 0.8 | 1.1 | 2.3 | 7.25 |
| YCL067C | 1.2 | 1.1 | 0.3 | 1.7 | 1.1 | 1.7 | 1.3 | 1.7 | 1.4 | 1.9 | 1.6 | 1.0 | 1.4 | 2.1 | 2.2 | 1.7 | 0.8 | 2.2 | 3.15 |
| YCR096C | 1.1 | 0.9 | 0.4 | 1.6 | 2.1 | 1.2 | 1.7 | 1.5 | 1.2 | 1.6 | 1.4 | 1.0 | 1.0 | 2.1 | 2.1 | 1.8 | 1.0 | 2.1 | 2.34 |
| YDL137W | 1.4 | 1.1 | 1.2 | 1.2 | 1.8 | 1.0 | 2.0 | 1.1 | 1.1 | 1.3 | 1.5 | 1.6 | 1.7 | 1.4 | 1.3 | 2.3 | 1.3 | 2.2 | 4.95 |

| | | | | | | | | | | | | | | | | | | | |
|---|---|---|---|---|---|---|---|---|---|---|---|---|---|---|---|---|---|---|---|
| YDL192W | 1.4 | 1.0 | 0.6 | 1.2 | 1.4 | 1.0 | 1.7 | 1.5 | 0.8 | 0.6 | 1.3 | 1.1 | 0.9 | 1.2 | 1.3 | 1.3 | 1.1 | 2.3 | 5.57 |
| YDR224C | 1.1 | 0.7 | 0.8 | 0.9 | 1.2 | 1.0 | 0.8 | 1.4 | 1.1 | 1.3 | 1.1 | 0.7 | 0.5 | 1.7 | 0.3 | 1.1 | 1.2 | 2.2 | 5.62 |
| YDR378C | 1.3 | 1.3 | 1.4 | 1.3 | 1.0 | 1.0 | 1.4 | 1.5 | 0.5 | 1.3 | 0.8 | 0.9 | 0.8 | 1.4 | 0.8 | 1.0 | 1.7 | 2.2 | 2.40 |
| YMR197C | 1.3 | 1.6 | 1.4 | 1.9 | 1.1 | 1.6 | 1.5 | 1.4 | 1.9 | 2.5 | 1.0 | 1.1 | 1.5 | 4.1 | 1.7 | 1.9 | 1.2 | 2.2 | 1.20 |
| YOL109W | 2.0 | 2.0 | 1.5 | 1.8 | 1.0 | 1.3 | 1.2 | 1.5 | 0.1 | 1.1 | 1.0 | 0.8 | 0.5 | 1.4 | 1.0 | 0.8 | 1.6 | 2.2 | 4.13 |
| YPL010W | 1.0 | 1.1 | 0.9 | 1.0 | 1.4 | 1.0 | 1.9 | 1.5 | 1.7 | 1.5 | 1.3 | 1.1 | 1.1 | 1.3 | 1.2 | 1.3 | 1.3 | 2.0 | 3.25 |
| YHR132C | 0.8 | 0.7 | 1.7 | 1.4 | 1.0 | 0.7 | 0.8 | 0.8 | 0.9 | 1.1 | 1.1 | 0.8 | 1.2 | 0.9 | 1.4 | 2.3 | 0.8 | 1.3 | 1.52 |
| YJL141C | 1.0 | 0.8 | 1.0 | 1.6 | 1.5 | 1.1 | 1.1 | 0.9 | 1.2 | 1.3 | 1.2 | 0.8 | 1.4 | 0.8 | 0.8 | 2.2 | 1.4 | 1.6 | 0.94 |
| YKR098C | 1.0 | 1.1 | 1.2 | 1.7 | 1.1 | 1.8 | 1.7 | 1.3 | 1.4 | 2.7 | 1.9 | 1.1 | 1.4 | 1.4 | 0.9 | 3.9 | 1.1 | 1.6 | 0.55 |
| YLR206W | 0.7 | 1.6 | 1.4 | 1.1 | 1.1 | 0.5 | 0.7 | 0.8 | 1.5 | 1.0 | 1.3 | 0.8 | 1.4 | 0.8 | 2.4 | 2.3 | 1.1 | 1.2 | 0.72 |
| YAL055W | 1.2 | 1.0 | 0.9 | 1.0 | 1.4 | 1.3 | 1.5 | 1.9 | 1.0 | 1.4 | 1.3 | 1.2 | 1.5 | 1.6 | 1.1 | 2.6 | 1.3 | 1.3 | 0.62 |
| YAR062W | 1.2 | 1.8 | 1.4 | 0.7 | 1.0 | 0.6 | 1.3 | 0.9 | 0.4 | 0.9 | -1.1 | 0.8 | 1.2 | 1.1 | 1.3 | 2.4 | 0.9 | 1.0 | 0.32 |
| YBL102W | 0.8 | 1.0 | 1.4 | 1.2 | 0.9 | 2.5 | 1.6 | 0.8 | 2.3 | 1.1 | 1.2 | 0.9 | 1.7 | 1.1 | 1.1 | 2.0 | 2.4 | 1.5 | 1.26 |
| YBR161W | 1.2 | 0.8 | 2.3 | 0.7 | 1.1 | | 3.0 | 0.9 | 0.4 | 0.4 | 1.1 | 0.6 | 1.0 | 0.9 | 1.5 | 2.1 | 1.5 | 2.2 | 0.72 |
| YCR039C | 1.9 | 2.1 | 0.3 | 0.9 | 1.9 | 1.5 | 1.4 | 1.6 | 1.8 | 2.2 | 1.2 | 0.8 | 1.0 | 1.7 | 1.9 | 2.1 | 1.0 | 2.1 | 1.73 |
| YDL018C | 1.3 | 0.9 | 0.9 | 1.1 | 1.8 | 1.4 | 1.2 | 1.3 | 1.0 | 1.0 | 0.9 | 0.7 | 1.0 | 1.5 | 1.1 | 3.1 | 1.5 | 1.7 | 0.84 |
| YDR022C | 1.1 | 1.2 | 0.8 | 0.9 | 1.5 | 2.2 | 1.8 | 1.4 | 0.9 | 1.4 | 0.9 | 0.7 | 1.3 | 2.1 | 1.0 | 2.1 | 1.3 | 1.2 | 0.47 |
| YDR181C | 1.1 | 1.2 | 0.8 | 1.4 | 0.9 | 0.7 | 1.0 | 1.2 | 2.0 | 0.9 | 0.8 | 0.7 | 0.7 | 1.3 | 1.2 | 2.0 | 0.8 | 1.2 | 0.48 |
| YGR036C | 0.9 | 0.8 | 0.9 | 1.1 | 1.3 | 1.3 | 1.0 | 0.8 | 0.9 | 0.9 | 0.9 | 1.0 | 1.3 | 1.0 | 0.7 | 2.0 | 1.3 | 1.1 | 1.12 |
| YGR120C | 1.2 | 1.8 | 0.5 | 1.0 | 1.8 | 1.5 | 1.6 | 1.3 | 0.3 | 1.2 | 1.1 | 0.7 | 0.5 | 1.0 | 1.2 | 1.9 | 1.1 | 1.2 | 0.36 |
| YGR131W | 0.8 | 2.1 | 1.9 | 1.1 | 0.9 | 1.9 | 1.1 | 1.1 | 1.9 | 5.8 | 1.1 | 0.5 | 1.4 | 2.0 | 0.8 | 2.6 | 1.3 | 1.0 | 0.41 |
| YGR167W | 1.1 | 1.1 | 0.6 | 1.5 | 0.9 | 1.7 | 1.4 | 1.4 | 1.4 | 1.9 | 1.1 | 1.1 | 1.6 | 1.7 | 1.6 | 2.8 | 1.3 | 1.7 | 1.79 |
| YHL024W | 1.2 | 0.8 | 1.6 | 1.1 | 1.5 | 1.2 | 1.3 | 0.8 | 2.1 | 3.6 | 1.1 | 1.6 | 1.6 | 2.1 | 2.9 | 3.8 | 1.4 | 1.4 | 0.58 |
| YJL113W | 1.2 | 1.9 | 1.2 | 1.2 | | 0.7 | 1.2 | 1.3 | 1.3 | 2.1 | 1.2 | 0.7 | 0.9 | 1.3 | 1.7 | 2.1 | 0.9 | 1.5 | 0.41 |
| YJL146W | 1.3 | 1.9 | 1.2 | 2.0 | 0.8 | 0.7 | 1.3 | 1.0 | 1.0 | 1.7 | -1.1 | 1.1 | 0.8 | 1.5 | 1.2 | 1.8 | 1.0 | 1.3 | 0.30 |
| YJR019C | 0.8 | 1.8 | 1.9 | 1.6 | 1.1 | 0.8 | 0.9 | 0.7 | 1.1 | 0.6 | 2.4 | 1.1 | 1.4 | 0.8 | 1.1 | 2.6 | 1.8 | 1.4 | 0.33 |
| YJR049C | 1.0 | 1.2 | 1.0 | 1.0 | 0.7 | 0.6 | 1.0 | 1.2 | 1.4 | 1.4 | 1.6 | 1.0 | 2.1 | 1.2 | 1.2 | 1.7 | 0.9 | 1.2 | 0.44 |
| YLR078C | 0.9 | 1.1 | 0.7 | 1.4 | | 0.8 | 1.2 | 1.1 | 0.8 | 1.1 | 2.9 | 1.0 | 0.8 | 1.2 | 0.6 | 2.2 | 1.2 | 1.2 | 1.02 |
| YNR037C | 0.9 | 0.9 | 0.9 | 1.7 | 1.2 | 0.7 | 1.7 | 1.1 | 1.3 | 1.7 | 1.5 | 0.7 | 2.0 | 1.2 | 0.8 | 1.9 | 1.4 | 1.6 | 1.34 |
| YOR028C | 1.4 | 0.7 | 1.1 | 2.3 | 1.6 | 1.0 | 1.2 | 1.5 | 1.2 | 2.0 | 1.5 | 0.6 | 0.8 | 1.3 | 1.1 | 3.7 | 0.8 | 1.7 | 0.78 |

The tables show that the expressed mRNA of about 700 of 2400 unknown yeast genes was induced by any one of chemical substances such as heavy metals, agricultural chemicals, surfactants (Table 1), as well as the expressed mRNA of 167 mitochondria-located genes (Table 2), 52 DNA repair genes (Table 3), 161 energy genes (Table 4), 142 transport facilitation protein genes (Table 5), 90 stress protein genes (Table 6), 142 metabolism genes (Table 7), 60 detoxification genes (Table 8), and 507 genes belonging to other category (Table 9). Here, when the value of the following is 2 or more, then it is considered significant:

$$\frac{\text{The level of expressed mRNA in the presence of chemical substance}}{\text{The level of expressed mRNA in the absence of chemical substance.}}$$

The inventors of the present application understood that the induction of certain yeast genes by toxic substances is caused by the activation of the promoters of the genes by the toxic substances. Thus, the inventors prepared a vector that comprised a polynucleotide sequence comprising the promoter of the yeast gene, which sequence is operably linked to a polynucleotide encoding a marker protein; and transformed yeast cells with the vector. Such cells enable us to detect readily toxic substances by detecting the expressed marker proteins (hereinafter, such detection may be referred to as "promoter assay".). The working examples hereinafter illustrate the preparation of such vectors, transformation of yeast cells by use of said vectors, and the detection of toxic substances by use of the transformed cells.
Promoter assay is a method for determining variations of the level of intracellular genes without destroying cells on the basis of the level of expressed marker genes instead of the expressed mRNA. The gene selected to detect a chemical substance is expressed in the absence of chemical substances, and thus a marker protein also occurs in the absence of chemical substances. In the method according to the present invention, the behavior of yeast genes on the addition of test materials is determined on the basis of the level of expressed marker protein so that the presence or the absence, and the kind of toxic chemical substances are predicted. Thus, it is desired that the production of marker proteins is lower in the absence of chemical substances, and the production of marker proteins is higher in the presence of chemical substances. Under the circumstance, yeast gene as used in promoter assay is selected, of which intensity (level of expressed gene in control cells/average level of expressed whole genes) is preferably 1.5 or less, more preferably 1 or less, even more preferably 0.5 or less, and of which expression magnification (expressed mRNA in the presence of chemical substance/expressed mRNA in the absence of chemical substance) is preferably 3 or more, more preferably 10 or more, even more preferably 20 or more.

Example 2

Primers for PCR to amplify the polynucleotide comprising the promoter of yeast gene YKL071w were prepared. Primers were designed using a primer design software, Oligo4.0-S, Sequencher I Mackintosh version. The base sequence of the upper primer was:

CGCAATAATACTGGAAACATCAA (SEQ ID No: 7),

whereas the base sequence of the lower primer was:

ATCGACTTTGTTTGCTTAGAAT (SEQ ID No: 8).

For PCR, yeast chromosome (*Saccharomyces cerevisiae* S288C, Cat.40802, Research Genetics, Inc.) was used as template, and the commercially available kit (KOD DNA Polymerase; Code KOD-101, Toyobo) was used as reagents.
Type YEp shuttle vector pYES2 (pYES2, Cat no: V825-20, Invirtogen Corporation, USA), which can be replicated both in *E.coli*. and yeast was used as vector (R. W. Old, S. B. Primrose Principles of Gene Manipulation 5th Ed., BAIFUKAN CO., LTD, pp138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YKL071w (SEQ ID No: 1) to give an intended plasmid vector. Suitable restriction enzymes were selected, and the insertion of the polynucleotide comprising GFP and the promoter sequence was conducted.
Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 µL of 2.5M lithium acetate;
4) Dispending each 300 µl, adding 10µl of above plasmid vector thereto, then incubating the suspensions at 30 ° C for 30 minutes;
5) Adding 700 µl of 50% PEG4000, and incubating the mixture under shaking at 30 ° C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YKL071w-pQBI has been deposited as the International Deposition at National Institute of Advanced Industrial Science and Technology, International Patent Organism Depositary of Tsukuba Central 6, 1-1-1 Higashi, Tsukuba, Ibaraki, Japan under Accession No. FERM BP-8161 on August 19, 2002.

Example 3

Primers for PCR to amplify the polynucleotide comprising the promoter of yeast gene YCR102c were prepared. Primers were designed using a primer design software, Oligo4.0-S, Sequencher I Mackintosh version. The base sequence of the upper primer was:

AGGTGCGATAGTGGGGAATAAGA (SEQ ID No: 9),

whereas the base sequence of the lower primer was:

GGTTTCTGGAATTGCAACTTGC (SEQ ID No: 10).

For PCR, yeast chromosome (*Saccharomyces cerevisiae* S288C, Cat.40802, Research Genetics, Inc.) was used as template, and the commercially available kit (KOD DNA Polymerase; Code KOD-101, Toyobo) was used as reagents.

Type YEp shuttle vector pYES2 (pYES2, Cat no: V825-20, Invirtogen Corporation, USA), which can be replicated both in *E.coli.* and yeast was used as vector (R. W. Old, S. B. Primrose Principles of Gene Manipulation 5th Ed., BAIFUKAN CO., LTD, pp138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YCR102c (SEQ ID No: 2) to give an intended plasmid vector. Suitable restriction enzymes were selected, and the insertion of the polynucleotide comprising GFP and the promoter sequence was conducted.

Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 µL of 2.5M lithium acetate;
4) Dispending each 300 µl, adding 10µl of above plasmid vector thereto, then incubating the suspensions at 30°C for 30 minutes;
5) Adding 700 µl of 50% PEG4000, and incubating the mixture under shaking at 30 °C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YCR102c-pQBI has been deposited as the International Deposition at National Institute of Advanced Industrial Science and Technology, International Patent Organism Depositary of Tsukuba Central 6, 1-1-1 Higashi, Tsukuba, Ibaraki, Japan under Accession No. FERM BP-8159 on August 19, 2002.

Example 4

Primers for PCR to amplify the polynucleotide comprising the promoter of yeast gene YOR382w were prepared. Primers were designed using a primer design software, Oligo4.0-S, Sequencher I Mackintosh version. The base sequence of the upper primer was:

GCTTTTCTCGCTTCGTTATCACC (SEQ ID No: 11),

whereas the base sequence of the lower primer was:

TATTATTGTTTTGTGATGGCTT (SEQ ID No: 12).

For PCR, yeast chromosome (*Saccharomyces cerevisiae* S288C, Cat.40802, Research Genetics, Inc.) was used as template, and the commercially available kit (KOD DNA Polymerase; Code KOD-101, Toyobo) was used as reagents.

Type YEp shuttle vector pYES2 (pYES2, Cat no: V825-20, Invirtogen Corporation, USA), which can be replicated both in E. coli. and yeast was used as vector (R. W. Old, S. B. Primrose Principles of Gene Manipulation 5th Ed., BAIFUKAN CO., LTD, pp 138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YOR382w (SEQ ID No: 3) to give an intended plasmid vector. Suitable restriction enzymes were selected, and the insertion of the polynucleotide comprising GFP and the promoter sequence was conducted.

Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 µL of 2.5M lithium acetate;
4) Dispending each 300 µl, adding 10µl of above plasmid vector thereto, then incubating the suspensions at 30°C for 30 minutes;
5) Adding 700 µl of 50% PEG4000, and incubating the mixture under shaking at 30 °C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YOR382W-pQBI has been deposited as the International Deposition at National Institute of Advanced Industrial Science and Technology, International Patent Organism Depositary of Tsukuba Central 6, 1-1-1 Higashi, Tsukuba, Ibaraki, Japan under Accession No. FERM BP-8160 on August 19, 2002.

Example 5

Primers for PCR to amplify the polynucleotide comprising the promoter of yeast gene YLL057c were prepared. Primers were designed using a primer design software, Oligo4.0-S, Sequencher I Mackintosh version. The base sequence of the upper primer was:

GCTAACGAACAGGATGGTATTGA (SEQ ID No: 13),

whereas the base sequence of the lower primer was:

ATTTTAACTGGGTTACTGTGCT (SEQ ID No: 14).

For PCR, yeast chromosome (*Saccharomyces cerevisiae* S288C, Cat.40802, Research Genetics, Inc.) was used as template, and the commercially available kit (KOD DNA Polymerase; Code KOD-101, Toyobo) was used as reagents.

Type YEp shuttle vector pYES2 (pYES2, Cat no: V825-20, Invirtogen Corporation, USA), which can be replicated both in *E.coli*. and yeast was used as vector (R. W. Old, S. B. Primrose Principles of Gene Manipulation 5th Ed., BAIFUKAN CO., LTD, pp 138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YLL057c (SEQ ID No: 4) to give an intended plasmid vector. Suitable restriction enzymes were selected, and the insertion of the polynucleotide comprising GFP and the promoter sequence was conducted.

Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 μL of 2.5M lithium acetate;
4) Dispending each 300 μl, adding 10μl of above plasmid vector thereto, then incubating the suspensions at 30°C for 30 minutes;
5) Adding 700 μl of 50% PEG4000, and incubating the mixture under shaking at 30 °C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YLL057C-pQBI was deposited at National Institute of Advanced Industrial Science and Technology, International Patent Organism Depositary of Tsukuba Central 6, 1-1-1 Higashi, Tsukuba, Ibaraki, Japan under Accession No. PERM P-18439 on July 27, 2001, and then transferred into the International Deposition under Budapest Treaty as PERM BP-8158 on August 19, 2002.

Example 6

Primers for PCR to amplify the polynucleotide comprising the promoter of yeast gene YLR303w were prepared. Primers were designed using a primer design software, Oligo4.0-S, Sequencher I Mackintosh version. The base sequence of the upper primer was:

TCGTTTTCTACTTTCTTCTGCTG (SEQ ID No: 15),

whereas the base sequence of the lower primer was:

TGTATGGATGGGGGTAATAGAA (SEQ ID No: 16).

For PCR, yeast chromosome (*Saccharomyces cerevisiae* S288C, Cat.40802, Research Genetics, Inc.) was used as template, and the commercially available kit (KOD DNA Polymerase; Code KOD-101, Toyobo) was used as reagents.

Type YEp shuttle vector pYES2 (pYES2, Cat no: V825-20, Invirtogen Corporation, USA), which can be replicated both in *E.coli*. and yeast was used as vector (R. W. Old, S. B. Primrose Principles of Gene Manipulation 5th Ed., BAIFUKAN CO., LTD, pp138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YLR303w (SEQ ID No: 5) to give an intended plasmid vector. Suitable restriction enzymes were selected, and the insertion of the polynucleotide comprising GFP and the promoter sequence was conducted.

Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 μL of 2.5M lithium acetate;
4) Dispending each 300 μl, adding 10μl of above plasmid vector thereto, then incubating the suspensions at 30°C for 30 minutes;
5) Adding 700 μl of 50% PEG4000, and incubating the mixture under shaking at 30 ° C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YLR303W-pQBI was deposited at National Institute of Advanced Industrial Science and Technology, International Patent Organism Depositary of Tsukuba Central 6, 1-1-1 Higashi, Tsukuba, Ibaraki, Japan under Accession No. FERM P-18438 on July 27, 2001, and then transferred into the International Deposition under Budapest Treaty as FERM BP-8157 on August 19, 2002.

Example 7

The cells of SC-YKL071W-pQBI as prepared in Example 2 were contacted to one of the following compounds. The yeast cells SC-YKL071W-pQBI were incubated at 25 °C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 10.

Table 10

| Chemical substances | | Concentrations | Fluorescens |
|---|---|---|---|
| (1) | benzo(a)pyrene | 0.2 mM | - |
| (2) | bisphenol-A | 0.4 mM | - |
| (3) | (2-ethylhexyl)phthalate | 83.3 mM | - |
| (4) | 2,5-dichlorophenol | 0.3 mM | - |
| (5) | 2,4-dichlorophenoxyacetic acid | 0.3 mM | - |
| (6) | formaldehyde | 0.2 mM | - |

Table 10 (continued)

| Chemical substances | | Concentrations | Fluorescens |
|---|---|---|---|
| (7) | methylmercury chloride | 0.2 µM | - |
| (8) | 4-nitroquinolin-N-oxide | 0.6 µM | - |
| (9) | p-nonylphenol | 10 µM | - |
| (10) | pentachlorophenol | 50 µM | - |
| (11) | sodium arsenite | 0.3 mM | - |
| (12) | Tetramethylthiuram disulfide | 20 µM | + |
| (13) | tributyltin chloride | 0.4 µM | - |
| (14) | 2,4,5-trichlorophenol | 30 mM | - |
| (15) | Trp-P-2 (acetate) | 0.2 mM | - |
| (16) | paraquat | 16.7 mM | - |
| (17) | cadmium chloride | 40 µM | - |
| (18) | γ-hexachlorocyclohexane | 6.7 mM | - |
| (19) | malathon | 22.2 mM | - |
| (20) | manganese ethylenebis (dithiocarbamate) | 0.8 mM | - |
| (21) | nickel (II) chloride | 3.3 mM | - |
| (22) | potassium bichromate | 0.3 mM | - |
| (23) | triphenyltin chloride | 10 µM | - |
| (24) | phenol | 5.6 mM | - |
| (25) | S-4-Chlorobenzyl-N,N-diethylthiocarbamate | 0.7 mM | - |
| (26) | hexachlorophene | 30 µM | - |
| (27) | triclosan | 730 µM | - |
| (28) | mercury(II) chloride | 50 µM | - |
| (29) | copper sulfate (II) | 3.3 mM | - |
| (30) | potassium cyanide | 16.7 mM | - |
| (31) | dimethylsulfoxide | 3.7 % | - |

Table 10 shows that tetramethylthiuram disulfide induced the expression of GFP.

Example 8

The cells of SC-YCR102C-pQBI as prepared in Example 3 were contacted to one of the following compounds. The yeast cells SC-YCR102C-pQBI were incubated at 25 °C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 11.

Table 11

| Chemical substances | | Concentrations | Fluorescence |
|---|---|---|---|
| (1) | benzo(a)pyrene | 0.2 mM | - |
| (2) | bisphenol-A | 0.4 mM | - |
| (3) | (2-ethylhexyl)phthalate | 83.3 mM | - |
| (4) | 2,5-dichlorophenol | 0.3 mM | - |
| (5) | 2,4-dichlorophenoxyacetic acid | 0.3 mM | - |
| (6) | formaldehyde | 0.2 mM | - |
| (7) | methylmercury chloride | 0.2 μM | - |
| (8) | 4-nitroquinolin-N-oxide | 0.6 μM | - |
| (9) | p-nonylphenol | 10 μM | - |
| (10) | pentachlorophenol | 50 μM | - |
| (11) | sodium arsenite | 0.3 mM | - |
| (12) | Tetramethylthiuram disulfide | 20 μM | + |
| (13) | tributyltin chloride | 0.4 μM | - |
| (14) | 2,4,5-trichlorophenol | 30 mM | - |
| (15) | Trp-P-2 (acetate) | 0.2 mM | - |
| (16) | paraquat | 16.7 mM | - |
| (17) | cadmium chloride | 40 μM | - |
| (18) | γ-hexachlorocyclohexane | 6.7 mM | - |
| (19) | malathon | 22.2 mM | - |
| (20) | manganese ethylenebis (dithiocarbamate) | 0.8 mM | - |
| (21) | nickel (II) chloride | 3.3 mM | - |
| (22) | potassium bichromate | 0.3 mM | - |
| (23) | triphenyltin chloride | 10 μM | - |
| (24) | phenol | 5.6 mM | - |
| (25) | S-4-Chlorobenzyl-N,N-diethylthiocarbamate | 0.7 mM | - |
| (26) | hexachlorophene | 30 μM | - |
| (27) | triclosan | 730 μM | - |
| (28) | mercury(II) chloride | 50 μM | - |
| (29) | copper sulfate (II) | 3.3 mM | - |
| (30) | potassium cyanide | 16.7 mM | - |
| (31) | dimethylsulfoxide | 3.7 % | - |

Table 11 shows that tetramethylthiuram disulfide induced the expression of GFP.

Example 9

The cells of SC-YOR382W-pQBI as prepared in Example 4 were contacted to one of the following compounds. The yeast cells SC-YOR382W-pQBI were incubated at 25 ° C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, ( 14) 2,4,5-trichlorophenol, (15) Trp-P-2 (acetate), (16) paraquat, (17) cadmium chloride , (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.
After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 12.

Table 12

| Chemical sbstances | | Concentrations | Fluorescence |
|---|---|---|---|
| (1) | benzo(a)pyrene | 0.2 mM | - |
| (2) | bisphenol-A | 0.4 mM | - |
| (3) | (2-ethylhexyl)phthalate | 83.3 mM | - |
| (4) | 2,5-dichlorophenol | 0.3 mM | ++ |
| (5) | 2,4-dichlorophenoxyacetic acid | 0.3 mM | - |
| (6) | formaldehyde | 0.2 mM | - |
| (7) | methylmercury chloride | 0.2 μM | - |
| (8) | 4-nitroquinolin-N-oxide | 0.6 μM | ++ |
| (9) | p-nonylphenol | 10 μM | ++ |
| (10) | pentachlorophenol | 50 μM | - |
| (11) | sodium arsenite | 0.3 mM | - |
| (12) | Tetramethylthiuram disulfide | 20 μM | - |
| (13) | tributyltin chloride | 0.4 μM | - |
| (14) | 2,4,5-trichlorophenol | 30 mM | ++ |
| (15) | Trp-P-2 (acetate) | 0.2 mM | ++ |
| (16) | paraquat | 16.7 mM | - |
| ( 17) | cadmium chloride | 40 μM | - |
| (18) | γ-hexachlorocyclohexane | 6.7 mM | - |
| (19) | malathon | 22.2 mM | ++ |
| (20) | manganese ethylenebis (dithiocarbamate) | 0.8 mM | ++ |
| (21) | nickel (II) chloride | 3.3 mM | ++ |
| (22) | potassium bichromate | 0.3 mM | ++ |
| (23) | triphenyltin chloride | 10 μM | - |
| (24) | phenol | 5.6 mM | ++ |
| (25) | S-4-Chlorobenzyl-N,N-diethylthiocarbamate | 0.7 mM | - |
| (26) | hexachlorophene | 30 μM | - |
| (27) | triclosan | 730 μM | - |
| (28) | mercury(II) chloride | 50 μM | - |
| (29) | copper sulfate (II) | 3.3 mM | - |
| (30) | potassium cyanide | 16.7 mM | - |
| (31) | dimethylsulfoxide | 3.7 % | ++ |

Table 12 shows that 2,4-dichlorophenoxyacetic acid, 4-nitroquinolin-N-oxide, p-nonylphenol, 2,4,5-trichlorophenol, Trp-P-2 (acetate) , malathon, manganese ethylenebis(dithiocarbamate), nickel (II) chloride, potassium bichromate, phenol, and dimethylsulfoxide induced the expression of GFP.

Example 10

The cells of SC-YLL057C-pQBI as prepared in Example 5 were contacted to one of the following compounds. The yeast cells SC-YLL057C-pQBI were incubated at 25 °C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 13.

Table 13

| Chemical substances | | Concentrations | Fluorescence |
|---|---|---|---|
| (1) | benzo(a)pyrene | 0.2 mM | - |
| (2) | bisphenol-A | 0.4 mM | - |
| (3) | (2-ethylhexyl)phthalate | 83.3 mM | - |
| (4) | 2,5-dichlorophenol | 0.3 mM | - |
| (5) | 2,4-dichlorophenoxyacetic acid | 0.3 mM | ++ |
| (6) | formaldehyde | 0.2 mM | - |
| (7) | methylmercury chloride | 0.2 µM | - |
| (8) | 4-nitroquinolin-N-oxide | 0.6 µM | - |
| (9) | p-nonylphenol | 10 µM | - |
| (10) | pentachlorophenol | 50 µM | - |
| (11) | sodium arsenite | 0.3 mM | ++ |
| (12) | Tetramethylthiuram disulfide | 20 µM | - |
| (13) | tributyltin chloride | 0.4 µM | - |
| (14) | 2,4,5-trichlorophenol | 30 mM | - |
| (15) | Trp-P-2 (acetate) | 0.2 mM | - |
| (16) | paraquat | 16.7 mM | - |
| (17) | cadmium chloride | 40 µM | ++ |
| (18) | γ-hexachlorocyclohexane | 6.7 mM | - |
| (19) | malathon | 22.2 mM | - |
| (20) | manganese ethylenebis (dithiocarbamate) | 0.8 mM | - |
| (21) | nickel (II) chloride | 3.3 mM | - |
| (22) | potassium bichromate | 0.3 mM | - |
| (23) | triphenyltin chloride | 10 µM | - |
| (24) | phenol | 5.6 mM | - |
| (25) | S-4-Chlorobenzyl-N,N-diethylthiocarbamate | 0.7 mM | - |
| (26) | hexachlorophene | 30 µM | - |
| | (27) triclosan | 730 µM | - |
| (28) | mercury(II) chloride | 50 µM | - |
| (29) | copper sulfate (II) | 3.3 mM | - |
| (30) | potassium cyanide | 16.7 mM | ++ |
| (31) | dimethylsulfoxide | 3.7 % | - |

Table 13 shows that 2,4-dichlorophenoxyacetic acid, sodium arsenite, cadmium chloride, and potassium cyanide induced the expression of GFP.

Example 11

The cells of SC-YCR303W-pQBI as prepared in Example 6 were contacted to one of the following compounds. The yeast cells SC-YCR303W-pQBI were incubated at 25 °C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 14.

Table 14

| Chemical substances | | Concentrations | Fluorescence |
|---|---|---|---|
| (1) | benzo(a)pyrene | 0.2 mM | ++ |
| (2) | bisphenol-A | 0.4 mM | - |
| (3) | (2-ethylhexyl)phthalate | 83.3 mM | - |
| (4) | 2,5-dichlorophenol | 0.3 mM | - |
| (5) | 2,4-dichlorophenoxyacetic acid | 0.3 mM | ++ |
| (6) | formaldehyde | 0.2 mM | ++ |
| (7) | methylmercury chloride | 0.2 μM | - |
| (8) | 4-nitroquinolin-N-oxide | 0.6 μM | - |
| (9) | p-nonylphenol | 10 μM | - |
| (10) | pentachlorophenol | 50 μM | - |
| (11) | sodium arsenite | 0.3 mM | ++ |
| (12) | Tetramethylthiuram disulfide | 20 μM | - |
| (13) | tributyltin chloride | 0.4 μM | - |
| (14) | 2,4,5-trichlorophenol | 30 mM | - |
| (15) | Trp-P-2 (acetate) | 0.2 mM | - |
| (16) | paraquat | 16.7 mM | - |
| (17) | cadmium chloride | 40 μM | ++ |
| (18) | γ-hexachlorocyclohexane | 6.7 mM | - |
| (19) | malathon | 22.2 mM | - |
| (20) | manganese ethylenebis (dithiocarbamate) | 0.8 mM | + |
| (21) | nickel (II) chloride | 3.3 mM | - |
| (22) | potassium bichromate | 0.3 mM | - |
| (23) | triphenyltin chloride | 10 μM | - |
| (24) | phenol | 5.6 mM | - |
| (25) | S-4-Chlorobenzyl-N,N-diethylthiocarbamate | 0.7 mM | - |
| (26) | hexachlorophene | 30 μM | - |
| (27) | triclosan | 730 μM | - |
| (28) | mercury(II) chloride | 50 μM | ++ |
| (29) | copper sulfate (II) | 3.3 mM | - |
| (30) | potassium cyanide | 16.7 mM | ++ |
| (31) | dimethylsulfoxide | 3.7 % | - |

Table 14 shows that benzo(a)pyrene, 2,4-dichlorophenoxyacetic acid, formaldehyde, sodium arsenite, cadmium chloride, manganese ethylenebis(dithiocarbamate), mercury(II) chloride, and potassium cyanide induced the expression of GFP.

## Claims

1. A polynucleotide which comprises a polynucleotide sequence operably linked to a polynucleotide encoding a marker protein, wherein the polynucleotide sequence comprises a promoter from yeast genes that is selected from a group consisting of the following, or a promoter from a gene that is homologous to the yeast genes and is derived from other species:
YBR072W, YCR102C, YCR107W, YDL218W, YDL243C, YDR453C, YDR533C, YFL014W, YFL056C, YFL057C, YGR110W, YJR155W, YKL071W, YKR076W, YLL060C, YLR460C, YMR090W, YNL331C, YNL332W, YNL335W, YOL150C, YOL165C, YPL171C, YPR167C, YBL048W, YBL064C, YBL107C, YBR008C, YBR173C, YBR256C, YBR296C, YDL021W, YFL022C, YFL024C, YFL061W, YGL121C, YGL158W, YGR043C, YHR029C, YHR112C, YHR139C, YHR179W, YHR209W, YIR030C, YJR010W, YJR048W, YKL001C, YKL107W, YKR075C, YKR097W, YLL056C, YLR297W, YLR303W, YML087C, YMR096W, YNL274C, YOL151W, YOR226C, YOR338W, YOR391C, YPL280W, YDR406W, YJL153C, YLR346C, YOR049C, YOR153W, YPL088W, YAL034C, YDL124W, YDL174C, YDR476C, YGL156W, YGR035C, YGR157W, YGR213C, YGR281W, YGR284C, YHL047C, YHR043C, YHR044C, YHR054C, YJR073C, YKL165C, YLR008C, YMR315W, YNL211C, YOL031C, YOL101C, YOR303W, YAL005C, YAR031W, YBL005W-A, YBL022C, YBL041W, YBL049W, YBL075C, YBL078C, YBR062C, YBR169C, YBR294W, YCL020W, YCL035C, YCL043C, YCL050C, YCL057W, YCR012W, YCR013C, YCR060W, YDL007W, YDL027C, YDL097C, YDL110C, YDL126C, YDL169C, YDR070C, YDR155C, YDR158W, YDR204W, YDR210W, YDR214W, YDR258C, YDR313C, YDR368W, YDR435C, YER012W, YER037W, YER091C, YER103W, YFL044C, YFR003C, YFR010W, YFR020W, YFR024C, YFR044C, YFR053C, YGL006W, YGL048C, YGL062W, YGL141W, YGL157W, YGL163C, YGL180W, YGL184C, YGR010W, YGR028W, YGR032W, YGR048W, YGR124W, YGR135W, YGR142W, YGR161C, YGR192C, YGR197C, YGR201C, YGR212W, YGR231C, YGR244C, YGR254W, YGR268C, YHL030W, YHR016C, YHR018C, YHR055C, YHR087W, YHR166C, YIL160C, YIR017C, YJL034W, YJL048C, YJL052W, YJL144W, YJL163C, YJR009C, YJR069C, YJR074W, YJR130C, YJR149W, YKL065C, YKL073W, YKL103C, YKL142W, YKL210W, YKL218C, YKR011C, YKR018C, YKR046C, YKR049C, YLL024C, YLL026W, YLR027C, YLR080W, YLR107W, YLR121C, YLR132C, YLR133W, YLR155C, YLR158C, YLR161W, YLR195C, YLR217W, YLR328W, YLR336C, YLR345W, YLR370C, YLR423C, YML004C, YML092C, YML128C, YML130C, YML131W, YMR040W, YMR118C, YMR214W, YMR251W, YMR297W, YMR322C, YNL036W, YNL055C, YNL071W, YNL094W, YNL134C, YNL155W, YNL160W, YNL239W, YNL241C, YOL005C, YOR020C, YOR027W, YOR037W, YOR059C, YOR120W, YOR134W, YOR152C, YOR173W, YOR289W, YOR362C, YPL240C, YPR030W, YAL008W, YAL023C, YAL060W, YAL062W, YAR009C, YBL101C, YBR006W, YBR046C, YBR052C, YBR053C, YBR056W, YBR099C, YBR137W, YBR139W, YBR149W, YBR170C, YBR177C, YBR203W, YBR207W, YBR212W, YBR239C, YBR284W, YBR293W, YCL018W, YCL033C, YCL040W, YCL049C, YCR062W, YCR067C, YCR082W, YDL010W, YDL020C, YDL024C, YDL054C, YDL095W, YDL100C, YDL115C, YDL144C, YDL198C, YDL223C, YDL245C, YDL246C, YDR001C, YDR032C, YDR058C, YDR072C, YDR127W, YDR168W, YDR169C, YDR188W, YDR231C, YDR261C, YDR264C, YDR272W, YDR293C, YDR304C, YDR330W, YDR403W, YDR411C, YDR427W, YDR436W, YDR497C, YDR511W, YDR516C, YDR519W, YDR545W, YEL012W, YEL030W, YER004W, YER009W, YER021W, YER035W, YER053C, YER079W, YER094C, YER096W, YER125W, YER158C, YER163C, YER175C, YER177W, YER178W, YER185W, YFL006W, YFL010C, YFL016C, YFL029C, YFL030W, YFL031W, YFL032W, YFL038C, YFL041W, YFR004W, YFR047C, YFR050C, YFR052W, YGL011C, YGL013C, YGL037C, YGL047W, YGL053W, YGL091C, YGL094C, YGL127C, YGL150C, YGL199C, YGL207W, YGL248W, YGR008C, YGR037C, YGR055W, YGR101W, YGR130C, YGR154C, YGR194C, YGR221C, YGR232W, YGR248W, YGR253C, YGR256W, YHL008C, YHR027C, YHR053C, YHR057C, YHR111W, YHR138C, YHR161C, YHR164C, YHR169W, YHR174W, YHR176W, YHR199C, YIL010W, YIL034C, YIL041W, YIL045W, YIL087C, YIL107C, YIL142W, YIL155C, YIR034C, YIR036C, YIR037W, YIR038C, YIR039C, YJL001W, YJL031C, YJL035C, YJL053W, YJL057C, YJL066C, YJL068C, YJL082W, YJL099W, YJL102W, YJL151C, YJL152W, YJL161W, YJL164C, YJL171C, YJL172W, YJL210W, YJL219W, YJR008W, YJR045C, YJR046W, YJR106W, YJR117W, YJR137C, YKL007W, YKL026C, YKL035W, YKL091C, YKL104C, YKL117W, YKL145W, YKL146W, YKL151C, YKL152C, YKL153W, YKL193C, YKL195W, YKL213C, YKL215C, YLL028W, YLL039C, YLL058W, YLR054C, YLR103C, YLR120C, YLR136C, YLR149C, YLR152C, YLR178C, YLR259C, YLR299W, YLR324W, YLR327C, YLR348C, YLR350W, YLR356W, YLR362W, YLR387C, YLR429W, YML054C, YML070W, YML100W, YML117W, YML125C, YMR004W, YMR008C, YMR009W, YMR020W, YMR067C, YMR089C, YMR097C, YMR102C, YMR105C, YMR107W, YMR152W, YMR180C, YMR184W, YMR191W, YMR219W, YMR271C, YMR275C, YMR295C, YMR314W, YMR316W, YNL006W, YNL007C, YNL012W, YNL044W, YNL045W, YNL074C, YNL092W, YNL093W, YNL104C, YNL115C, YNL156C, YNL231C, YNL234W, YNL237W, YNL281W, YNL305C, YNL333W, YNR010W, YNR019W, YNR033W, YNR059W, YNR068C, YNR069C, YOL032W, YOL036W, YOL047C, YOL071W, YOL082W, YOL083W, YOL117W, YOL119C, YOL126C, YOL131W, YOL153C, YOL162W, YOL163W, YOL164W, YOR019W, YOR035C, YOR036W, YOR099W, YOR117W, YOR124C, YOR130C, YOR132W, YOR157C, YOR185C, YOR197W, YOR259C, YOR261C, YOR273C, YOR288C, YOR332W, YOR336W, YOR347C, YPL017C, YPL087W, YPL106C, YPL109C, YPL149W, YPL154C, YPL196W, YPL206C, YPL222W, YPR023C, YPR024W, YPR026W, YPR067W, YPR103W, YPR108W, YPR151C, YAL012W, YBR029C, YBR222C, YCL009C, YCL027W, YCL064C, YCR098C, YDL222C, YDR055W, YDR077W, YDR502C, YEL001C, YEL042W, YER026C, YER106W, YGR136W, YGR138C, YHR137W, YHR142W, YIL023C, YIL153W, YJL073W, YJR004C, YJR054W, YKL039W, YKL086W, YKL163W, YKR091W, YLR109W, YLR194C, YLR250W, YMR095C, YMR189W, YNL106C, YNL169C, YNL322C, YOR181W, YOR198C, YOR208W, YOR247W, YPL089C, YAL038W, YAL053W, YBR023C, YBR214W, YBR295W, YCR048W, YDL072C, YDL204W, YDR085C, YDR098C, YDR259C, YDR380W, YDR388W, YDR391C, YDR432W, YDR481C, YDR510W, YER069W, YGL022W, YGL126W, YGL209W, YGL255W, YGR189C, YGR282C, YHL035C, YHR030C, YIL022W, YIL024C, YIL117C, YIL123W, YIL140W, YIL154C, YJL088W, YJL108C, YJL149W, YJL159W, YJL186W, YJR148W, YKL096W, YLR180W, YLR273C, YLR300W, YLR307W, YLR378C, YLR391W, YMR094W, YMR104C, YMR276W, YMR296C, YNL190W, YNL208W, YNL300W, YNR064C, YOL013C, YOL058W, YOR248W, YOR355W, YPL052W, YPL163C, YPR079W, YAR028W, YBR146W, YBR183W, YCL038C, YCR071C, YDL008W, YDR019C, YDR031W, YDR115W, YDR486C, YER038C, YER130C, YFL054C, YGL136C, YGR146C, YGR207C, YHL040C, YIL167W, YJL020C, YKR039W, YLR031W, YLR205C, YMR072W, YMR140W, YMR173W, YMR195W, YMR226C, YNL037C, YNR002C, YOL143C, YOR136W, YOR215C, YOR382W, YOR383C, YPL054W, YPL271W, YPR127W, YAL044C, YAL054C, YAR010C, YAR027W, YAR071W, YBL001C, YBL043W, YBL057C, YBR014C, YBR024W, YBR035C, YBR068C, YBR111C, YBR116C, YBR147W, YBR168W, YBR246W, YBR273C, YCR004C, YCR021C, YCR037C, YCR088W, YDL022W, YDL128W, YDL238C, YDR003W, YDR009W, YDR033W, YDR084C, YDR104C, YDR270W, YDR315C, YDR340W, YDR357C, YDR358W, YDR396W, YDR405W, YDR410C, YDR434W, YDR482C, YDR487C, YDR520C, YDR534C, YDR539W, YEL011W, YEL065W, YEL066W, YER039C, YER044C, YER067W, YER080W, YER107C, YFL020C, YFL028C, YFL043C, YFR015C, YGL001C, YGL008C, YGL068W, YGL073W, YGL104C, YGL113W, YGL154C, YGL167C, YGL229C, YGL242C, YGL249W, YGL253W, YGR052W, YGR060W, YGR065C, YGR106C, YGR111W, YGR220C, YGR257C, YHL023C, YHL048W, YHR004C, YHR037W, YHR071W, YHR092C, YHR190W, YIL007C, YIL033C, YIL070C, YIL088C, YIL111W, YIR002C, YIR016W, YIR035C, YIR043C, YJL012C, YJL083W, YJL089W, YJL116C, YJL131C, YJL132W, YJL196C, YJR061W, YJR086W, YJR142W, YJR161C, YKL008C, YKL013C, YKL041W, YKL067W, YKL138C, YKL139W, YKL150W, YKL175W, YKR006C, YKR014C, YKR070W, YLL023C, YLR023C, YLR093C, YLR118C, YLR142W, YLR225C, YLR241W, YLR251W, YLR252W, YLR270W, YML030W, YML110C, YMR021C, YMR027W, YMR148W, YMR181C, YMR262W, YMR272C, YMR298W, YNL011C, YNL130C, YNL214W, YNL259C, YOL129W, YOR042W, YOR052C, YOR137C, YOR149C, YOR165W, YOR270C, YOR285W, YOR367W, YPL018W, YPL156C, YPL186C, YPL203W, YPL216W, YPL255W, YPR006C, YPR073C, YPR098C, YBR050C, YBR145W, YBR299W, YDR518W, YEL020C, YFL062W, YGL039W, YGL134W, YJL217W, YJR159W, YLR126C, YNL249C, YNL284C, YNL336W, YOL157C, YOR344C, YOR381W, YPL265W, YPR124W, YBR074W, YBR109C, YBR126C, YBR201W, YCR005C, YDL248W, YDR041W, YDR105C, YDR268W, YDR452W, YEL075C, YER046W, YER050C, YER136W, YER159C, YGL250W, YGR019W, YGR042W, YGR053C, YGR066C, YGR247W, YGR255C, YGR295C, YHL044W, YHR145C, YIL058W, YIL065C, YIL083C, YIL098C, YIL172C, YJL030W, YJL185C, YJL213W, YJR029W, YJR099W, YJR122W, YJR125C, YKL190W, YKR020W, YLL025W, YLL051C, YLR043C, YLR090W, YLR100W, YLR108C, YLR290C, YML068W, YMR051C, YMR139W, YMR178W, YMR193W, YNL015W, YNL079C, YNL122C, YNL223W, YNL285W, YNL293W, YNR007C, YNR035C, YNR061C, YOL016C, YOL104C, YOR220W, YOR221C, YOR374W, YPL123C, YPR077C, YPR107C, YPR147C, YBR093C, YBR196C, YEL041W, YEL047C, YER023W, YER119C, YFL055W, YGR209C, YIL124W, YKL187C, YLL055W, YMR318C, YOL152W, YAL007C, YBR067C, YBR115C, YBR285W, YBR292C, YDL043C, YDL123W, YDL131W, YDL168W, YDL212W, YDR056C, YDR132C, YDR154C, YDR183W, YDR216W, YDR253C, YDR295C, YDR494W, YDR513W, YEL072W, YER045C, YER061C, YER181C, YFL052W, YFL058W, YFR030W, YGL089C, YGL096W, YGL114W, YGL193C, YGL202W, YGL204C, YGL259W, YGR006W, YGR070W, YGR088W, YHL034C, YHL036W, YHR048W, YHR104W, YHR163W, YIL060W, YIL136W, YIR024C, YJL036W, YJL045W, YJL060W, YJL101C, YJL155C, YJR085C, YJR109C, YJR156C, YKL070W, YKL161C, YKL221W, YKR071C, YLL009C, YLL050C, YLR092W, YLR145W, YLR156W, YLR163C, YLR220W, YLR280C, YLR311C, YLR390W, YML116W, YMR034C, YMR038C, YMR081C, YMR250W, YNL240C, YNL260C, YNL277W, YNR074C, YOL044W, YOL084W, YOL147C, YOL159C, YOR184W, YOR228C, YOR255W, YPL223C, YPR160W, YDL182W, YBR047W, YBR054W, YBR291C, YDR069C, YER124C, YER131W, YGR044C, YIL094C, YKR007W, YMR240C, YNR050C, YOR007C, YAL015C, YBL065W, YBR105C, YBR182C, YBR186W, YBR244W, YBR272C, YCL069W, YDL025C, YDL059C, YDL085W, YDL113C, YDL244W, YDR018C, YDR054C, YDR202C, YDR223W, YDR350C, YDR353W, YDR374C, YDR512C, YEL052W, YEL070W, YER098W, YFR017C, YGL046W, YGL067W, YGL098W, YGL117W, YGL146C, YGL240W, YGR011W, YGR067C, YGR133W, YGR153W, YGR223C, YHR116W, YHR124W, YIL097W, YIL168W, YJL103C, YJL221C, YJR036C, YJR095W, YKL085W, YKL133C, YKL162C, YKL188C, YKL217W, YKR061W, YKR105C, YLL062C, YLR174W, YLR216C, YLR247C, YLR260W, YLR267W, YLR389C, YML007W, YMR041C, YMR177W, YMR253C, YNL009W, YNL117W, YNL128W, YNL183C, YNR073C, YOL133W, YOL158C, YOR133W, YOR225W, YOR227W, YPL161C, YPL166W, YPL202C, YPL224C, YPR015C, YPR086W, YPR201W, YAL061W, YAL067C, YAR007C, YBL033C, YBL056W, YBL086C, YBR026C, YBR073W, YBR101C, YBR117C, YBR123C, YBR213W, YBR269C, YBR280C, YCR036W, YDL132W, YDL149W, YDL200C, YDL234C, YDL242W, YDR099W, YDR177W, YDR256C, YDR392W, YDR394W, YDR531W, YEL071W, YER014W, YER042W, YER090W, YER184C, YFL059W, YFR042W, YFR046C, YFR049W, YGL026C, YGL058W, YGL185C, YGL227W, YGL252C, YGL254W, YGR089W, YGR112W, YGR134W, YGR276C, YHL019C, YHR012W, YHR017W, YHR028C, YHR106W, YHR109W, YHR156C, YIL036W, YIL046W, YIL143C, YIL152W, YIL159W, YIL164C, YJL071W, YJL094C, YJL154C, YJR056C, YJR072C, YJR110W, YJR139C, YKL025C, YKL034W, YKL064W, YKL171W, YKL196C, YKR012C, YKR068C, YKR069W, YLL001W, YLL057C, YLL061W, YLR064W, YLR070C, YLR099C, YLR144C, YLR157C, YLR160C, YLR164W, YLR364W, YLR421C, YML032C, YML042W, YML112W, YML118W, YMR114C, YMR115W, YMR258C, YNL181W, YNL191W, YNL212W, YNL213C, YNL250W, YNL265C, YNL312W, YNR032W, YOL038W, YOL049W, YOL064C, YOR088W, YOR155C, YOR257W, YOR265W, YOR377W, YOR386W, YPL031C, YPL113C, YPL124W, YPL151C, YPL249C, YPL260W, YPL274W, YPR048W, YPR061C, YPR093C, YPR125W, YPR158W, YPR168W, YPR169W, YPR174C, YPR180W, YPR193C, YPR200C, YAL014C, YAL017W, YAL049C, YBL019W, YBL058W, YBR001C, YBR013C, YBR018C, YBR037C, YBR045C, YBR051W, YBR063C, YBR128C, YBR129C, YBR204C, YBR241C, YBR255W, YBR281C, YCL044C, YCL055W, YCR014C, YCR019W, YCR024C, YCR105W, YDL065C, YDL089W, YDL143W, YDL173W, YDL193W, YDL197C, YDL206W, YDL230W, YDL233W, YDR040C, YDR071C, YDR078C, YDR109C, YDR140W, YDR194C, YDR212W, YDR221W, YDR257C, YDR271C, YDR287W, YDR294C, YDR316W, YDR329C, YDR338C, YDR369C, YDR421W, YDR425W, YDR485C, YDR488C, YDR504C, YDR505C, YDR506C, YDR515W, YEL005C, YEL037C, YEL044W, YER017C, YER048C, YER052C, YER078C, YER089C, YER092W, YER100W, YER162C, YER182W, YFL021W, YFL042C, YFR045W, YFR051C, YFR056C, YGL040C, YGL041C, YGL045W, YGL057C, YGL093W, YGL105W, YGL125W, YGL166W, YGL181W, YGL183C, YGL215W, YGL216W, YGL221C, YGL223C, YGR007W, YGR029W, YGR155W, YGR156W, YGR186W, YGR198W, YGR210C, YGR211W, YGR237C, YGR250C, YGR258C, YGR266W, YGR270W, YGR274C, YGR277C, YHL021C, YHL037C, YHL038C, YHR082C, YHR083W, YHR134W, YHR160C, YHR171W, YHR180W, YHR205W, YIL062C, YIL072W, YIL075C, YIL099W, YIL108W, YIL165C, YIL170W, YIR009W, YIR018W, YIR031C, YIR032C, YJL032W, YJL049W, YJL128C, YJL165C, YJR044C, YJR052W, YJR090C, YJR091C, YJR103W, YJR104C, YJR153W, YKL059C, YKL079W, YKL090W, YKL094W, YKL192C, YKL209C, YKR052C, YKR102W, YKR106W, YLL054C, YLR025W, YLR097C, YLR200W, YLR226W, YLR248W, YLR266C, YLR392C, YLR427W, YML013W, YML029W, YML041C, YML051W, YML078W, YML079W, YML088W, YML099C, YMR056C, YMR068W, YMR091C, YMR110C, YMR160W, YMR186W, YMR255W, YNL005C, YNL026W, YNL039W, YNL063W, YNL064C, YNL077W, YNL083W, YNL147W, YNL176C, YNL194C, YNL253W, YNL257C, YNL261W, YNL264C, YNL276C, YNR006W, YNR034W, YNR047W, YNR051C, YNR071C, YOL065C, YOL067C, YOR005C, YOR008C, YOR022C, YOR023C, YOR058C, YOR069W, YOR087W, YOR138C, YOR229W, YOR256C, YOR267C, YPL005W, YPL020C, YPL022W, YPL105C, YPL147W, YPL150W, YPL152W, YPL164C, YPL168W, YPL180W, YPL188W, YPL194W, YPR025C, YPR047W, YPR049C, YPR066W, YPR081C, YPR134W, YPR140W, YPR148C, YPR155C, YPR172W, YPR185W, YAL018C, YAR064W, YBR012C, YBR076W, YBR287W, YDR043C, YDR250C, YDR373W, YFR014C, YGL191W, YGR180C, YHR136C, YJL026W, YJL037W, YLR038C, YNL058C, YOR031W, YGR087C, YIL166C, YHR008C, YIL129C, YGL256W, YJR030C, YMR077C, YBR264C, YPL177C, YKR040C, YGL056C, YDR128W, YGR139W, YBL101W-A, YOR253W, YOL026C, YDR278C, YHR095W, YCL042W, YNL200C, YPL221W, YLR415C, YMR058W, YPR037C, YER072W, YML028W, YOR325W, YAL039C, YMR112C, YJR107W, YGL088W, YJR058C, YNL142W, YDR090C, YMR071C, YBL093C, YGR293C, YML055W, YDL017W, YDL210W, YGL055W, YCL025C, YDR080W, YDL181W, YNR030W, YJL017W, YIL127C, YDR281C, YDR366C, YFR026C, YJL212C, YPL215W, YEL019C, YBR132C, YHL018W, YNL196C, YPL038W, YAR047C, YPL262W, YHL006C, YPL225W, YBR124W, YOR148C, YKR053C, YBL044W, YER029C, YLR360W, YCL056C, YCR007C, YGR239C, YNL256W, YPR146C, YLR377C, YKL097C, YBR066C, YLR338W, YDL229W, YBR253W, YJR027W, YKL198C, YBL030C, YBR031W, YBR118W, YBR162C, YBR221C, YCR024C-A, YCR106W, YDL046W, YDR012W, YDR133C, YDR134C, YDR276C, YDR342C, YDR343C, YEL027W, YEL034W, YGR038W, YGR243W, YGR279C, YHR094C, YHR105W, YHR175W, YHR181W, YIL056W, YIL162W, YJL059W, YJL097W, YJL158C, YJR105W, YKL051W, YKL056C, YKL097W-A, YKL100C, YKL141W, YKR066C, YLR134W, YLR258W, YLR339C, YML058W, YMR083W, YMR203W, YNL209W, YNL307C, YOL030W, YOR178C, YPL028W, YPR028W, YPR113W, YPR149W, YPR150W, YPR183W, YAL016W, YBL099W, YBL100C, YBR011C, YBR096W, YBR100W, YBR127C, YBR283C, YBR286W, YCL008C, YCL058C, YCR030C, YCR034W, YCR069W, YDL015C, YDL023C, YDL061C, YDL086W, YDR038C, YDR039C, YDR050C, YDR151C, YDR178W, YDR233C, YDR284C, YDR298C, YDR345C, YDR359C, YDR382W, YDR385W, YDR400W, YDR407C, YDR538W, YEL024W, YEL033W, YEL063C, YER057C, YER081W, YER120W, YFL011W, YGL012W, YGL206C, YGR022C, YGR026W, YGR082W, YGR107W, YGR172C, YGR191W, YGR204W, YGR260W, YHL005C, YHL046C, YHR025W, YHR026W, YHR123W, YHR126C, YHR143W, YIL011W, YIL015W, YIL018W, YIL157C, YIR041W, YJL016W, YJL121C, YJL133W, YJL138C, YJL191W, YJR018W, YJR047C, YJR077C, YJR119C, YJR121W, YJR123W, YJR143C, YJR145C, YKL060C, YKL147C, YKL148C, YKL157W, YKL164C, YKL169C, YKR033C, YLL041C, YLL064C, YLR041W, YLR044C, YLR056W, YLR058C, YLR081W, YLR089C, YLR110C, YLR177W, YLR264W, YLR284C, YLR304C, YLR340W, YLR354C, YLR372W, YLR388W, YML022W, YMR007W, YMR011W, YMR015C, YMR092C, YMR101C, YMR156C, YMR205C, YMR215W, YMR261C, YMR323W, YNL069C, YNL135C, YNL195C, YNR076W, YOL039W, YOL073C, YOL086C, YOL120C, YOL156W, YOL161C, YOR002W, YOR009W, YOR010C, YOR085W, YOR108W, YOR128C, YOR129C, YOR142W, YOR161C, YOR176W, YOR230W, YOR298W, YPL004C, YPL036W, YPL048W, YPL057C, YPL059W, YPL061W, YPL135W, YPL179W, YPL218W, YPL220W, YPL246C, YPL272C, YPR063C, YPR080W, YPR181C, YBR290W, YCR010C, YCR091W, YDL107W, YDL129W, YDR066C, YDR529C, YFL026W, YGL018C, YGL059W, YNL144C, YOR003W, YAL037W, YAR023C, YBR003W, YBR020W, YBR044C, YBR091C, YBR185C, YBR282W, YCR015C, YCR038C, YCR043C, YDL119C, YDL146W, YDL220C, YDR057W, YDR123C, YDR125C, YDR222W, YDR225W, YDR277C, YDR286C, YDR347W, YDR408C, YDR438W, YDR479C, YDR483W, YEL039C, YEL057C, YEL073C, YER066W, YER076C, YER084W, YER121W, YER189W, YFL017C, YFL046W, YFR006W, YFR008W, YGL115W, YGL208W, YGL214W, YGL218W, YGR021W, YGR023W, YGR024C, YGR064W, YGR076C, YGR096W, YGR108W, YGR174C, YGR182C, YGR236C, YGR288W, YHL042W, YHR195W, YHR210C, YIL006W, YIL012W, YIL028W, YIL050W, YIL057C, YIL089W, YIL102C, YIL113W, YIL122W, YJL100W, YJL169W, YJL199C, YJR039W, YJR050W, YJR101W, YKL003C, YKL016C, YKL061W, YKL093W, YKL121W, YKL160W, YKL170W, YKL194C, YKR034W, YKR067W, YLR006C, YLR016C, YLR030W, YLR036C, YLR112W, YLR125W, YLR128W, YLR204W, YLR211C, YLR233C, YLR257W, YLR288C, YLR326W, YLR334C, YLR395C, YLR408C, YLR414C, YLR444C, YML050W, YML107C, YML120C, YMR031C, YMR053C, YMR073C, YMR162C, YMR204C, YMR206W, YMR284W, YNL010W, YNL025C, YNL127W, YNL139C, YNL217W, YOL116W, YOL118C, YOR053W, YOR100C, YOR103C, YOR122C, YOR150W, YOR187W, YOR251C, YOR312C, YOR327C, YOR348C, YOR352W, YOR388C, YOR394W, YPL001W, YPL033C, YPL066W, YPL148C, YPL230W, YPL275W, YPL276W, YPR005C, YPR014C, YPR192W, YPR194C, YBR005W, YER025W, YFL027C, YGL080W, YGL205W, YHL028W, YHR185C, YIL076W, YJL166W, YLR046C, YMR035W, YMR238W, YMR252C, YNL192W, YNL202W, YOL108C, YOR385W, YPR165W, YAR033W, YBL038W, YBR009C, YBR010W, YBR151W, YCL067C, YCR096C, YDL137W, YDL192W, YDR073W, YDR086C, YDR224C, YDR377W, YDR378C, YER015W, YGL187C, YHR162W, YJL167W, YJL216C, YKR009C, YLR165C, YMR197C, YNL157W, YOL002C, YOL109W, YOR180C, YPL010W, YPL233W, YBR036C, YDR297W, YGR149W, YGR224W, YNL043C, YPL067C, YPL170W, YCR046C, YDR387C, YFL050C, YGL051W, YHR132C, YIL112W, YJL141C, YKR098C, YLR052W, YLR206W, YML129C, YNL203C, YNR014W, YOL043C, YOL096C, YPR184W, YAL028W, YAL055W, YAR062W, YBL095W, YBL102W, YBR122C, YBR157C, YBR161W, YBR251W, YBR298C, YCR039C, YCR083W, YDL018C, YDL067C, YDL078C, YDL091C, YDL215C, YDL216C, YDR022C, YDR067C, YDR079W, YDR181C, YDR186C, YDR196C, YDR262W, YDR306C, YDR319C, YER188W, YGL004C, YGL035C, YGR036C, YGR062C, YGR120C, YGR131W, YGR141W, YGR167W, YGR287C, YHL024W, YHR080C, YHR097C, YIL077C, YJL046W, YJL070C, YJL096W, YJL113W, YJL146W, YJL180C, YJR019C, YJR049C, YKR058W, YLL005C, YLR078C, YLR151C, YLR271W, YLR295C, YLR351C, YLR375W, YMR023C, YMR025W, YMR135C, YMR210W, YMR267W, YMR278W, YMR293C, YNL073W, YNR037C, YNR040W, YNR072W, YOR028C, YOR316C, YOR328W, YOR363C, YPL039W, YPL040C, YPL099C, YPL107W, YPL134C, YPL138C, and YPL140C.

**2.** The polynucleotide according to claim 1, wherein the yeast gene belongs to a category of unknown yeast genes.

**3.** The polynucleotide according to claim 1, wherein the yeast gene belongs to a category of mitochondria genes.

**4.** The polynucleotide according to claim 1, wherein the yeast gene belongs to a category of DNA repair genes.

**5.** The polynucleotide according to claim 1, wherein the yeast gene belongs to a category of energy genes.

**6.** The polynucleotide according to claim 1, wherein the yeast gene belongs to a category of transport facilitation genes.

**7.** The polynucleotide according to claim 1, wherein the yeast gene belongs to a category of stress protein genes.

**8.** The polynucleotide according to claim 1, wherein the yeast gene belongs to a category of metabolism genes.

**9.** The polynucleotide according to claim 1, wherein the yeast gene belongs to a category of detoxification genes.

**10.** The polynucleotide according to claim 2, wherein the unknown yeast gene is YKL071W.

**11.** The polynucleotide according to claim 2, wherein the unknown yeast gene is YCR102C.

**12.** The polynucleotide according to claim 2, wherein the unknown yeast gene is YOR382W.

**13.** The polynucleotide according to claim 8, wherein the metabolism genes is YLR303W.

**14.** The polynucleotide according to claim 9, wherein the detoxification genes is YLL057C.

**15.** A vector that comprises the polynucleotide according to any one of claims 1 to 14.

**16.** A cell that is transformed with the polynucleotide according to any one of claims 1 to 14 or the vector according to claim 15.

**17.** A process for detecting a toxic compound in a test material, which comprises:

(1) contacting the test material to the cells according to claim 16, and
(2) detecting the expression of mRNA encoding a marker protein.

**18.** The process according to claim 17, wherein the expression of mRNA is confirmed by the expression of a marker protein.

**19.** The process according to claim 17, wherein the expression of mRNA is detected by northern blotting.

**20.** The process according to claim 17, wherein mRNA is amplified by reverse transcription-PCR (RT-PCR), and the expression of mRNA is detected.

**21.** A process of identifying a toxic compound, which comprises conducting the process according to any one of claims 17 to 20 against each of two or more of the cell according to claim 16.

**22.** The process according to claim 21, wherein two or more of the cell according to claim 16 is selected from a group consisting of YLL057C, YLR303W, YKL071W, YCR102C, and YOR382W.

## INTERNATIONAL SEARCH REPORT

International application No.
PCT/JP02/08495

A. CLASSIFICATION OF SUBJECT MATTER
Int.Cl[7] C12N15/00, C12Q1/68

According to International Patent Classification (IPC) or to both national classification and IPC

B. FIELDS SEARCHED

Minimum documentation searched (classification system followed by classification symbols)
Int.Cl[7] C12N15/00, C12Q1/68

Documentation searched other than minimum documentation to the extent that such documents are included in the fields searched

Electronic data base consulted during the international search (name of data base and, where practicable, search terms used)
CAPLUS/MEDLINE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| E,X | JP 2001-286281 A (Director General of National Institute of Advanced Industrial Science and Technology), 16 October, 2001 (16.10.01), Full text (Family: none) | 1-9,15-21 |
| X<br>Y | Prein, B. et al., "A novel strategy for constructing N-terminal chromosomal fusions to green fluorescent protein in the yeast Saccharomyces cerevisiae", FEBS Letters, 2000, Vol.485, No.1, pages 29 to 34, abstract, Materials and Methods | 1-9,15<br>16-21 |
| X<br>Y | WO 00/58520 A1 (Rosetta Inpharmatics Inc.), 05 October, 2000 (05.10.00), Claims; pages 100 to 102; table 1 & AU 2000039313 A | 1-9,15<br>16-21 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 November, 2002 (26.11.02) | 10 December, 2002 (10.12.02) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/08495

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | Casalone, E. et al., "Disruption and phenotypic analysis of six novel genes from chromosome IV of Saccharomyces cerevisiae revieal YDL060w as an essential gene for vegetative growth", Yeast, 1999, Vol.15, No.15, pages 1691 to 1701, abstract; table 1 | 1-9,15<br>16-21 |
| X<br>Y | Belli, G. et al., "Functional analysis of yeast essential genes using a promoter-substitution cassette and the tetracycline-regulatable dual expression system", Yeast, 1998, Vol.14, No.12, pages 1127 to 1138, abstract; table 3 | 1-9,15<br>16-21 |
| X<br>Y | Huang, M.E., "Disruption of six novel yeast genes reveals three genes essential for vegetative growth and one required for growth at low temperature", Yeast, 1997, Vol.13, No.12, pages 1181 to 1194, abstract; table 2 | 1-9,15<br>16-21 |
| X<br>Y | Sartori, G. et al., "Inactivation of six genes from chromosomes VII and XIV of Saccharomyces cerevisiae and basic phenotypic analysis of the mutant strains", Yeast, 2000, Vol.16, No.3, pages 255 to 265, abstract; table 1 | 1-9,15<br>16-21 |
| Y | FUJITA, K. et al., "Hsp104 expression and morphological changes associated with disinfectants in Saccharomyces cerevisiae: Environmental bioassay using stress response", Water Science and Technology, 1998, Vol.38, No.7, pages 237 to 243, abstract | 1-9,15-21 |
| Y | MIURA, S. et al., "Screening of genes involved in isooctane tolerance in Saccharomyces cerevisiae by using mRNA differential display", Applied and Environmental Microbiology, 2000, Vol.66, No.11, pages 4883 to 4889, abstract | 1-9,15-21 |
| Y | Parry, J.M., "The use of yeast cultures for the detection of environmental mutagens using a fluctuation test", Mutation Research, 1977, Vol.46, No.3, pages 165 to 175, abstract | 1-9,15-21 |
| Y | Alberts, B. et al., "Essential Cell Biology", New York: Garland Publishing, Inc., 1998, page 323, Fig. 10-9 | 1-9,15-21 |
| Y | Lashkari, D.A. et al., "Yeast microarrays for genome wide parallel genetic and gene expression analysis", Proc.Natl.Acad.Sci.USA, 1997, Vol.94, pages 13057 to 13062, abstract; table 1 | 1-9,15-21 |

Form PCT/ISA/210 (continuation of second sheet) (July 1998)

## INTERNATIONAL SEARCH REPORT

International application No.
PCT/JP02/08495

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
(See extra sheet.)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: Parts of claims 1 to 9 and 15 to 21.

**Remark on Protest** ☐ The additional search fees were accompanied by the applicant's protest.
☐ No protest accompanied the payment of additional search fees.

Form PCT/ISA/210 (continuation of first sheet (1)) (July 1998)

INTERNATIONAL SEARCH REPORT

International application No.
PCT/JP02/08495

Continuation of Box No.II of continuation of first sheet(1)

Requirement of unity of invention in international application (PCT Rule 13.1) is not fulfilled unless there is a technical relationship in a group of claimed inventions involving one or more of the same or corresponding special technical features. The expression "special technical feature" means a technical feature that defines a contribution which each of the claimed inventions, considered as a whole, makes over the prior art (PCT Rule 13.2). The determination of unity of invention is made without regard to whether the inventions are claimed in separate claims or as alternatives within a single claims (PCT Rule 13.3).

Concerning the claims in the present case, a large number of polynucleotides, among those wherein a polynucleotide encoding a marker protein is ligated in an operable manner to a polynucleotide sequence containing the promoter of any of the 2053 publicly known yeast genes as set forth in claim 1, had been constructed by cloning these genes and thus publicly known, or have been publicly known as replacement cassettes to be used in short-flanking homology (SFH) gene replacement techniques (see, for example, Casalone, E., et al.; "Disruption and phenotypic analysis of six novel genes from chromosome IV of Saccharomyces cerevisiae reveal YDL060w as an essential gene for vegetative growth" YEAST, Vol. 15, No. 15, 1999, Nov., pp. 1691-1701) (in the above document, for example, the promoters of YDL065C and YDL110C are ligated to kanMX4 marker).

Thus, it can be said that there is no "special technical feature" common to the inventions relating to the respective polynucleotides with the use of the promoters of the above-described genes presented in the claims.

Such being the case, the claims involve 2053 inventions, which are different from each other, corresponding respectively to the promoters with the use of the 2053 genes.

Form PCT/ISA/210 (extra sheet) (July 1998)

SEQUENCE LISTING

<110> National Institute of Advanced Industrial Science and Technology

<110> DAIKIN INDUSTRIES, LTD.

<120> Processes for Detecting Toxic Substances

<130> 664319

<160> 16

<210> 1

<211> 1682

<212> DNA

<213> Saccharomyces cerevisiae

<400> 1

```
cgcaataata ctggaaacat caacgaatgc tataataatg cgcatttact ttgggcaaaa   60
gaactattta aaattacgca ttacgtagtt tattttgaca ttatgatcat aaacatgcgc  120
aaatcctaag atcgtgaatt acctctactg cgagaataca aattataaca tatgactaac  180
gttacacaac attgtattac atttctgact aatttgaagt ctgggagtta attgtagtag  240
cctactaaat agcgcggtta tttataaagc taacattacc atgttacatg acatgctatt  300
tctagcgagg taaaagcatt gctccggctt ttcgaagata agtattcctt caatttccaa  360
atgtcacaac ataaaatttt gatttgcgtt atctctcgtt ctctttaatc actacaatag  420
catcaaccaa gtcaataaaa catgttgtga agagactacg cagacgtgac ggtcatctag  480
caactctcca tcttttgatg cagccatcca cagtttatcg tgagttatgg caaaatccaa  540
aaatgtaccg taccatttcc tctttaatcc tgtattcaat aagcaagttc cccatttcaa  600
ttgatctaca catcactgcg cgttaaagcg cacctaattt attcggtagg aaacagaaat  660
agtctgcaaa gtcattcaag cacctttctt taccaaatga aaggatttaa tagtacctat  720
```

gaaacaatag ttcgaacttt tgccatttcc cggttttttt ggccagcttg tataaaagtg 780

caccttaccc ttatattggg ctcttattga atgccttccg aagaactgac tattcaaaaa 840

atagaaacaa gtacgtcaat aaaaaatttt gcaattctac gaataattat tcctgtttct 900

ttaacctggt aaaaaaaagt acaaacactt aagctttttg aaacagcttt attttgcttc 960

attaaatagc taggataaga aatccctcat ccgaaaggtt ttgtatctaa ctaccctaga 1020

gaacatttgt cctgatcagg ttcatttgga gtttatattt tttagaagct caaagtttgt 1080

tggactcatt accatggaag aaaaaaagaa gatactacga aatattggtt tctcaggtta 1040

aataagggac accattttcc tattaggcta gtcgagctta gttcttctaa tttcttcaga 1200

tcttctataa tttcctatct tctacctgat gtgtgcatga tatatctatg agctcctgat 1260

attgcttgtt ttactttagc ttgcatgact tgcaataatc taatcatata tgttcccgat 1320

taatatactg tgcacaaatt gcaggacata taatttttcc gtggattata tcttcgatta 1380

acgtccgcgg gtctcataaa aagcaaacca acttcgcaat tccctagaaa tacctcaata 1440

gaaagttatt tgtaatgaga ttagtaatga gattagcaat gagattagta atgagattag 1500

taatgagatt agtaatgtga ttagtaatgc atagcggtat aaatggtagt actaataagt 1560

aagatagtat accagttata ataaataggc ggcgatgctt caaaactaat ttttgacgtt 1620

tttaagaata aagcctttac cagtggcata aatcagtaga attctaagca aacaaagtcg 1680

at 1682

〈210〉 2

〈211〉 1072

〈212〉 DNA

〈213〉 Saccharomyces cerevisiae

〈400〉 2

aggtgcgata gtggggaata agaattggaa atccgcttga taatcaggga aaacttaaag 60

aacggggtgt taaaagtctg tgaagtatag atcaaactcg cactagctag caatactcat 120

gagaacctag gtgagtgtaa agcagtgaac cttctttaga aagcagagaa aaatcctcca 180

agttaacagt cttccatact tactcagtat ggatcaagtt ccacagtttg tccacttctg 240

tagaataaac ttgtagtgct tttccgatga aaagggatag agtctttaaa agacttgatc 300

ttggcatttt tatcctcctt caagcagaat ggctccagta gagttgttct gaacgcttct 360
gggcagtgag cagcattgaa gaaaaagtac tcagcattcc aagctttgtc ttcatataat 420
tataaattaa aactcatagc caccaatttc caggttttgg tcgcaatatg ctgcagaatc 480
aatggcttgt ttcgttacta tactggtccg acggagttta cttactctaa atttgctgaa 540
atagcaaggc cacacgccat acaattaaca atagttcatt cagttgaaag gtttccgaga 600
tagaaagtgc ggtagtagag aagaacaata aacgtatcaa actttgcgca gtcatggaat 660
ttccaggaaa catttttagg ttttcatatg acagcagttc ctgtccgaga gctgtgcctt 720
cttgtctcag ctgactaaga tgtctcggat ccgcccggct ggcgcgcggc tccgtctagt 780
gggataggct ttcaacacat aggaaaccct tcagatgatg agaacacatc gaatcccaga 840
gagtaatatc accaagaata aagcaaccat gatagacaat attactaagt gttcctcaac 900
agatattgag aaaagttctt ataaagcggg caggcttttt tctatttttt tctttttctt 960
tatctagctt aagatccttc taaccatatt ctataaaatg gcaagctgaa tacagattat 1020
caacgacaat tacatataac ctacaaacag gcaagttgca attccagaaa cc 1072

<210> 3
<211> 1477
<212> DNA
<213> Saccharomyces cerevisiae
<400> 3

gcttttctcg cttcgttatc acctaaccac taagaacata ctaataatac aaaattttta 60
tccacttttt accaacagat tttcatgaaa tatccatctt ctttccgaat tctgtataaa 120
atgaaagaga gatttttttgc tctcaatcca ggctcaaggc tcttgagaac aggtaattgt 180
tgtttagcac gttttcaaca catttatcag aaaacgacag ttaaaaataa gacgagcggc 240
gttatcatcc tgatagtcac taaatgaaaa gcaggtactt ttgaagagca catgtaatat 300
ttgctcaact gataatacct tcattttata cacacttcta attttttttt taaaatcaga 360
tacgcattga tgttcaatgt agcattacca tcacaagaca aaaaatcaga attttacaag 420
caaaagcgct tatctttaag gaccaacata ttagatgaat tcttaagggt tgccaaaacg 480
caagacaaca ggcaaaataa tttcgtttct cagtaccgaa atgacgaaat atactgaggc 540
aaatgcgatc atcatgcctt tgcgccaaga aactcccttg tgaagaactt caaaccgaaa 600

tgggaaaact ttgagttatt gacagggaat acggagggga agatcacact taaatccgta 660
tgagccgcgc acataatggt attcaaatac acaagaacat tcatgagcta tttttcatcc 720
gtgcaaacga atttactaca attggaccag agggcaccat aactggagac tttgctactg 780
actcaacgtt gatgatgcga gtagtgggtg tactgtgatt tgctcatttt ttttttata 840
gaaagattcg attaatgaaa gtcacaggag acatttttac atagacattc cgtatatgtt 900
gcgggtatcg cggatgcgga ttagtgatgc ctttaactac atttcataga tttctgtata 960
ccaattgaaa tgagtgaagt aagctcctac agtgaaatat ctgggtgcta ctgacgccaa 1020
gccctacagc gatcggaatg cgggaacgga agttaacggg gcttccagaa cggcggaagc 1080
gaattgaacg aggacggcaa acaaaaacac ccaaaatttc attacttaga atgaccctca 1140
agagcagggt gcaatttatc aagcgatcat tgaactaact aagttcatat cctgtatagg 1200
atttaaaaca atgcacccta agttcaaatg caccccccct cgccccgcag cggacccttg 1260
aacagagaac tgtttcgagg ttcacccaat tggatcactt gtataatttg taatcgagtt 1320
cggataagat gtatacgaat ctaactgggt gcagtataat tagcatttta tattacctag 1380
caatatatgt ataaaacagg aatgtgtgcg tgcttcaggc agaattttac ggtccttgta 1440
aaaaagtcta tcataaagcc atcacaaaac aataata 1477

〈210〉 4
〈211〉 948
〈212〉 DNA
〈213〉 Saccharomyces cerevisiae

〈400〉 4
gctaacgaac aggatggtat tgatgaagat tccaaaattt tgcgtgctgt cactgaacag 60
gttgccttga gttacaaaga caaaggtgtt tcagcaagaa ttgtcagact cccattctcg 120
gttcatggca agggggacaa ggcttttgta ccaatattaa tgaatattgc caaagctgcc 180
ggaaaatctg gctatgtcgg acaaggcaca aacgcttggg cggctgtaca tcgtttggat 240
acggctcctc tgttcagact tgttttagag aaaggaaaaa caggacaagt gtatcattgc 300
gttggtgaac aaggtatacc attcaaagat attgcgcgtg tgattggaga aattttgaat 360
gttcccgtgg cctctatccc tgttgatgac gcggaaagtc attttggctt cctcacttgt 420

tttgtcacta gagatggccc agtttcaagc gaaggtacca gaaaagagct gggatggcag 480
ccacaacaaa tcggtcttct tgaagatatc cgtgcgaact atagcttaaa ctgacttgaa 540
gcttatatta tgcgtttttt ctaagagcgc catagcatgt aacgtttttt acatactagt 600
tagctttatc tatatagtct actgtgcagc ttaaaatacc caactcatgc gtctcattgg 660
acgagctctt ggcccttgga aaggtgctat attagtatat aggggaatga tgacaaaagc 720
ctcaatgtgg cttgagtcga tttcttattt ggcgccacag ggcacatgga gtttatttat 780
catactacta acataaagaa ggtatgtagg caatacaaca agaatgctgg aaaagttaag 840
gagtcagtaa cagttcttga tgacagaaac atataaaaag gtgtactatt gctgtataat 900
cggccctttc atacttgtac aaacatagca cagtaaccca gttaaaat 948

〈210〉 5
〈211〉 968
〈212〉 DNA
〈213〉 Saccharomyces cerevisiae

〈400〉 5
tcgttttcta ctttcttctg ctgctataat aagcacctat gggatctata tagtattttt 60
ataacgatag actttataaa agaaaatacc taagtgaaaa tttggtgaat tttgagataa 120
ttgttgggat tccattttta ataaggcaat aatattaggt atgtagaata tactagaagt 180
tctcctcgag gatttaggaa tccataaaag ggaatctgca attctacaca attctataaa 240
tattattatc atcgttttat atgttaatat tcattgatcc tattacatta tcaatccttg 300
cgtttcagct tccactaatt tagatgacta tttctcatca tttgcgtcat cttctaacac 360
cgtatatgat aatatactag taacgtaaat actagttagt agatgatagt tgatttttat 420
tccaacacta agaaataatt tcgccatttc ttgaatgtat ttaaagatat ttaatgctat 480
aatagacatt taaatccaat tcttccaaca tacaatggga gtttggccga gtggtttaag 540
gcgtcagatt taggtggatt taacctctaa aatctctgat atcttcggat gcaagggttc 600
gaatccctta gctctcatta ttttttgctt tttctcttga ggtcacatga tcgcaaaatg 660
gcaaatggca cgtgaagctg tcgatattgg ggaactgtgg tggttggcaa atgactaatt 720
aagttagtca aggcgccatc ctcatgaaaa ctgtgtaaca taataaccga agtgtcgaaa 780

aggtggcacc ttgtccaatt gaacacgctc gatgaaaaaa ataagatata tataaggtta 840
agtaaagcgt ctgttagaaa ggaagttttt cctttttctt gctctcttgt cttttcatct 900
actatttcct tcgtgtaata cagggtcgtc agatacatag atacaattct attacccccca 960
tccataca 968

〈210〉 6
〈211〉 720
〈212〉 DNA
〈213〉 Aequorea victoria

〈400〉 6
tcagttgtac agttcatcca tgccatgtgt aatcccagca gctgttacaa actcaagaag 60
gaccatgtgg tctctctttt cgttgggatc tttcgaaagg gcagattgtg tggacaggta 120
atggttgtct ggtaaaagga cagggccatc gccaattgga gtattttgtt gataatggtc 180
tgctagttga acgcttccat cttcaatgtt gtggcgggtc ttgaagttca ctttgattcc 240
attcttttgt ttgtctgcca tgatgtatac attgtgtgag ttatagttgt attccaattt 300
gtgtcccaga atgttgccat cttccttgaa gtcaatacct tttaactcga ttctattaac 360
aagggtatca ccttcaaact tgacttcagc acgtgtcttg tagttgccgt catctttgaa 420
gaagatggtc ctttcctgta cataaccttc gggcatggca ctcttgaaaa agtcatgccg 480
tttcatatga tccgggtatc ttgaaaagca ttgaacacca tagcacagag tagtgactag 540
tgttggccat ggaacaggca gtttgccagt agtgcagatg aacttcaggg taagttttcc 600
gtatgttgca tcaccttcac cctctccact gacagagaac ttgtggccgt taacatcacc 660
atctaattca acaagaattg ggacaactcc agtgaagagt tcttctcctt tgctagccat 720

〈210〉 7
〈211〉 23
〈212〉 DNA
〈213〉 Saccharomyces cerevisiae

⟨400⟩ 7

cgcaataata ctggaaacat caa

⟨210⟩ 8

⟨211⟩ 22

⟨212⟩ DNA

⟨213⟩ Saccharomyces cerevisiae

⟨400⟩ 8

atcgactttg tttgcttaga at

⟨210⟩ 9

⟨211⟩ 23

⟨212⟩ DNA

⟨213⟩ Saccharomyces cerevisiae

⟨400⟩ 9

aggtgcgata gtggggaata aga

⟨210⟩ 10

⟨211⟩ 22

⟨212⟩ DNA

⟨213⟩ Saccharomyces cerevisiae

⟨400⟩ 10

ggtttctgga attgcaactt gc

⟨210⟩ 11

⟨211⟩ 23

〈212〉 DNA

〈213〉 Saccharomyces cerevisiae

〈400〉 11

gcttttctcg cttcgttatc acc

〈210〉 12

〈211〉 22

〈212〉 DNA

〈213〉 Saccharomyces cerevisiae

〈400〉 12

tattattgtt ttgtgatggc tt

〈210〉 13

〈211〉 23

〈212〉 DNA

〈213〉 Saccharomyces cerevisiae

〈400〉 13

gctaacgaac aggatggtat tga

〈210〉 14

〈211〉 22

〈212〉 DNA

〈213〉 Saccharomyces cerevisiae

〈400〉 14

attttaactg ggttactgtg ct

<210> 15

<211> 23

<212> DNA

<213> Saccharomyces cerevisiae

<400> 15

tcgttttcta ctttcttctg ctg

<210> 16

<211> 22

<212> DNA

<213> Saccharomyces cerevisiae

<400> 16

tgtatggatg ggggtaatag aa